# EUROPEAN PATENT SPECIFICATION

(11) **EP 3 847 279 B1**
(45) Date of publication and mention of the grant of the patent: **06.03.2024**
(21) Application number: 19765703.4
(22) Date of filing: 05.09.2019
(51) Int. Cl.: C12Q 1/6883

(54) **LONG NON-CODING RNAS (LNCRNAS) FOR THE DIAGNOSIS AND THERAPEUTICS OF BRAIN DISORDERS, IN PARTICULAR COGNITIVE DISORDERS**
LANGE NICHT-CODIERENDE RNAS (LNCRNAS) ZUR DIAGNOSE UND THERAPIE VON HIRNERKRANKUNGEN, INSBESONDERE KOGNITIVEN STÖRUNGEN
LONGS ARN NON CODANTS (ARNNC) POUR LE DIAGNOSTIC ET LA THÉRAPIE DES MALADIES CÉRÉBRALES, EN PARTICULIER DE TROUBLES COGNITIFS

(30) Priority: 05.09.2018 US 201862727210 P
(43) Date of publication of application: 14.07.2021
(73) Proprietor: Amoneta Diagnostics SAS, 68330 Huningue (FR)
(72) Inventor: MOUSSAOUI, Saliha, 68870 Bartenheim (FR); FIRAT, Hueseyin, 68330 Huningue (FR); SCHORDAN, Eric, 68220 Wentzwiller (FR)
(74) Representative: Pharma Patents International AG
(86) International application number: PCT/EP2019/073775
(87) International publication number: WO 2020/049135

(56) References cited:
- EP-A2- 1 716 227
- EP-A2- 1 716 227
- WO-A1-2015/031958
- ZHIFENG ZHANG ET AL: "Preparation and application of streptavidin magnetic particles", SCIENCE IN CHINA SERIES B ; CHEMISTRY, SCIENCE IN CHINA PRESS, BE, vol. 50, no. 1, 1 February 2007 (2007-02-01), pages 127-134, XP019485648, ISSN: 1862-2771, DOI: 10.1007/S11426-007-2031-3
- NI YAOHUI ET AL: "Investigation of Long Non-coding RNA Expression Profiles in the Substantia Nigra of Parkinson's Disease", CELLULAR AND MOLECULAR NEUROBIOLOGY, SPRINGER NEW YORK, US, vol. 37, no. 2, 5 May 2016 (2016-05-05), pages 329-338, XP036160130, ISSN: 0272-4340, DOI: 10.1007/S10571-016-0373-0 [retrieved on 2016-05-05]
- YANG BO ET AL: "Distinct Hippocampal Expression Profiles of Long Non-coding RNAs in an Alzheimer's Disease Model", MOLECULAR NEUROBIOLOGY, SPRINGER US, NEW YORK, vol. 54, no. 7, 8 August 2016 (2016-08-08) , pages 4833-4846, XP036287447, ISSN: 0893-7648, DOI: 10.1007/S12035-016-0038-5 [retrieved on 2016-08-08]
- RAMA SHANKAR ET AL: "Transcriptome analysis in different rice cultivars provides novel insights into desiccation and salinity stress responses", SCIENTIFIC REPORTS, vol. 6, no. 1, 31 March 2016 (2016-03-31), XP055636000, DOI: 10.1038/srep23719
- Yaxing Gui ET AL: "Altered microRNA profiles in cerebrospinal fluid exosome in Parkinson disease and Alzheimer disease", Oncotarget, 10 November 2015 (2015-11-10), page 37043, XP055278437, United States DOI: 10.18632/oncotarget.6158 Retrieved from the Internet: URL:https://www.ncbi.nlm.nih.gov/pmc/artic les/PMC4741914/pdf/oncotarget-06-37043.pdf
- Yanyao Deng ET AL: "Plasma long noncoding RNA 51A as a stable biomarker of Alzheimer's disease", Int J Clin Exp Pathol, 1 April 2017 (2017-04-01), pages 4694-4699, XP055636042, Retrieved from the Internet: URL:http://www.ijcep.com/files/ijcep004780 7.pdf [retrieved on 2019-10-25]
- LILACH SOREQ ET AL: "Long Non-Coding RNA and Alternative Splicing Modulations in Parkinson's Leukocytes Identified by RNA Sequencing", PLOS COMPUTATIONAL BIOLOGY, vol. 10, no. 3, 20 March 2014 (2014-03-20) , page e1003517, XP055334570, DOI: 10.1371/journal.pcbi.1003517
- LIANG FENG ET AL: "Plasma long non-coding RNA BACE1 as a novel biomarker for diagnosis of Alzheimer disease", BMC NEUROLOGY, vol. 18, no. 1, 9 January 2018 (2018-01-09), XP055636641, DOI: 10.1186/s12883-017-1008-x
- ZHIFENG ZHANG ET AL: "Preparation and application of streptavidin magnetic particles", SCIENCE IN CHINA SERIES B ; CHEMISTRY, SCIENCE IN CHINA PRESS, BE, vol. 50, no. 1, 1 February 2007 (2007-02-01), pages 127-134, XP019485648, ISSN: 1862-2771, DOI: 10.1007/S11426-007-2031-3
- NI YAOHUI ET AL: "Investigation of Long Non-coding RNA Expression Profiles in the Substantia Nigra of Parkinson's Disease", CELLULAR AND MOLECULAR NEUROBIOLOGY, SPRINGER NEW YORK, US, vol. 37, no. 2, 5 May 2016 (2016-05-05), pages 329-338, XP036160130, ISSN: 0272-4340, DOI: 10.1007/S10571-016-0373-0 [retrieved on 2016-05-05]
- YANG BO ET AL: "Distinct Hippocampal Expression Profiles of Long Non-coding RNAs in an Alzheimer's Disease Model", MOLECULAR NEUROBIOLOGY, SPRINGER US, NEW YORK, vol. 54, no. 7, 8 August 2016 (2016-08-08) , pages 4833-4846, XP036287447, ISSN: 0893-7648, DOI: 10.1007/S12035-016-0038-5 [retrieved on 2016-08-08]

## Description

### CROSS REFERENCE TO RELATED APPLICATIONS

This application claims the benefit and priority of United States provisional application 62/727,210, filed on September 5, 2018.

### FIELD OF THE INVENTION

The present invention relates to the field of human medicine and specifically to the diagnostic and therapeutic of brain disorders and particularly cognitive disorders including those with mild cognitive impairment, Alzheimer disease, dementia with Lewy body and frontotemporal dementia. This invention relates to a biomarker for brain disorders, such as cognitive disorders, consisting in long non-coding RNAs (IncRNAs) in a peripheral circulating body fluid such as plasma or blood and/or expressed in brain tissue and/or whole blood and representing a therapeutic target for brain disorders, or non-invasive method for diagnosing brain disorders such as cognitive disorders, in particular Alzheimer's disease or for monitoring its development or progression using this biomarker. It further relates to associated kits, methods, protocols and transmittable forms of information for diagnosis purposes and/or for other human medicine applications including the patient stratification in clinical trials and/or monitoring the efficacy of therapeutic strategies using this biomarker and peripheral body fluids or whole blood from patients. The invention further consists in brain specific IncRNAs altered in patient with brain disease in particular cognitive disorder and representing a diagnostic or therapeutic candidate for their treatment. The present invention will thus have a strong impact on the quality of life of patients and a significant impact on the healthcare system and economy.

### BACKGROUND ART

Alzheimer's disease (AD) is a chronic neurodegenerative disease characterized by progressive loss of cognitive function. Mild cognitive impairment (MCI) is a major first symptom and evolves progressively into mild, moderate and severe AD stages. Early-onset AD occurs between a 30s to mid-60s and represents less than 10% of all people with AD and is caused by a mutation in APP or PSEN1 or PSEN2 genes. Most people with AD have the late-onset form with onset of symptoms in the mid-60s and later. The causes of late-onset AD are not yet completely understood, but most documented genetic risk factor is the apolipoprotein E (APOE) gene allele ε4. All AD forms, whether early-onset familial or sporadic forms, are pathologically characterized by two typical hallmarks, extracellular deposition of amyloid-beta peptide (Aβ) (a metabolite of APP protein encoded by APP gene) in amyloid plaques and intracellular deposition of hyper-phosphorylated tau protein (encoded by MAPT gene) in neurofibrillary tangles in the brain, associated with progressive neuronal degeneration (Marcus et al. J Neurogenet. 2011; 25(4):127-33; Gotz et al, Br J Pharmacol. 2012; 165(5):1246-59).

AD is the most common form of dementia. Approximately 46.8 million people worldwide currently live with AD or other type of dementia. With an ageing population, this number is estimated to increase to 131.5 million by 2050 (World Alzheimer Report, 2015). As such, AD is becoming an increasingly important burden on the affected individuals and their families as well as economic and social costs on medical and healthcare resources in both developed and emerging countries.

Dementia with Lewy bodies (DLB) and Parkinson's disease dementia (PDD), both called Lewy body dementias, are the second most common type of degenerative dementia in patients older than 65 years and their brain pathological hallmarks are α-synuclein neuronal inclusions (Lewy bodies, and Lewy neurites), accompanied by neuronal loss (Lancet. 2015 Oct 24;386(10004): 1683-97). Variants in three genes *APOE, SNCA,* and *GBA* have been associated with an increased risk of dementia with Lewy bodies, but in most cases, the cause is unknown.

Frontotemporal dementia (FTD) is the third most common dementia across all age groups and the 1^{st} or the 2^{nd} (after AD) prevalent dementia in the 45-64 years age group. The most common form is known as behavioral variant frontotemporal dementia (bvFTD), which is characterized (in its early stages) by changes in personality, behavior, and judgment. Other disorders under the "frontotemporal disorders" umbrella include Pick's disease, primary progressive aphasia, primary non-fluent aphasia, semantic dementia, corticobasal degeneration (CBD) syndrome, progressive supranuclear palsy (PSP), frontotemporal dementia (FTD) with parkinsonism, and FTD with amyotrophic lateral sclerosis (ALS).

There are overlapping clinical features between the classic clinical phenotypes of FTD spectrum disorders, thus complicating the clinical diagnostic picture. FTD spectrum disorders also share common underlying molecular pathology with findings of frontotemporal lobar degeneration, with neuronal and astrocytic inclusions of tau protein, TAR DNA-binding protein 43 (TDP-43), and rarely RNA-binding protein fused in sarcoma (FUS). bvFTD and PPA_language variants are associated with tau, TDP-43 or FUS proteinopathies, and PSP syndrome is often associated with tau proteinopathy. While CBD syndrome pathology comprises tauopathy in the form of CBD or PSP, but also beta-amyloidopathy/tauopathy (typical of AD), prionopathy, TDP-43 proteinopathy and alpha-synucleinopathy. As many as 40% of patients with FTD have a family history of the disease. 10%-20% of all cases attributed to mutations in or near the 3 genes: *C9orf72* (encoding protein C9orf72), *GRN* (encoding progranulin) and *MAPT* (encoding tau). More rare, other causing genetic mutations have been identified in familial FTD: *VCP, TARDBP, TIA1, TBK1* and *CCNF* genes for cases of FTD due to TDP proteinopathy, and in *CHMP2B* and *FUS* for cases of FTD due to tau-negative, TDP-negative, ubiquitin-positive pathology (Lancet. 2015 Oct 24;386(10004):1672-82; Desmarais P, et al. J Neurol Neurosurg Psychiatry 2019;90:412-423).

Currently, there is no cure for AD, nor for any of the other non-AD dementia types. Symptomatic treatments exist for these diseases, all trying to counterbalance neurotransmitter's disturbance. Although a number of new potential disease-modifying therapeutic candidates are currently being studied in clinical trials in AD, none has been approved yet. From 2002 to 2012, there was a failure of 99.6% of AD clinical trials that were 289 Phases 2 and 3 trials on symptomatic agents (36.6%), disease-modifying small molecules (35.1%) and disease-modifying immunotherapies (18%) (Cummings et al., Expert Rev Clin Pharmacol. 2014;7(2):161-5). Among the strategies proposed by worldwide experts in the AD field and by pharmaceutical industries to improve the success rate for AD drug development, are: a) intervening earlier in the disease process before neurodegeneration begins, b) identifying and developing accurate biomarkers for early diagnosis, non-invasive and suitable for stratification of subject populations and for longitudinal monitoring of drug efficacy in clinical trials.

The AD pathological features are defined in post-mortem histopathological analysis. The presence of both types of lesions (amyloid plaques and tangles) in the brain remains an absolutely required feature for the definitive diagnosis of AD.

Current diagnosis of the disease remains uncertain (Dubois et al Lancet Neurol. 2014;13(6):614-29) and it is based on combination of battery of clinical and neuropsychological tests and neuroimaging, such as structural imaging using Magnetic Resonance Imaging (MRI) and/or glucose metabolism using ositron emission tomography (PET) of fluorodeoxyglucose F18 (¹⁸F-FDG), with an accuracy of less than 70%-85% depending on the method and the severity stage of the disease (Frisoni et al., Nat Rev Neurol. 2010; 6(2): 67-77; Tahmasian et al., J Nucl Med. 2016 ;57(3):410-5).

Sometimes, detection of AD-associated biomarkers Aβ42 and tau or phosphorylated-tau in cerebrospinal fluid (CSF) is additionally performed (Sunderland et al., JAMA. 2003;289(16):2094-2103), but CSF tests request a lumbar puncture, which is an invasive procedure and often requires hospitalization of subjects and therefore used only in a small proportion of subjects and at a too-late-stage of AD.

Longitudinal confirmatory diagnosis test is essential for applications to early detection of the preclinical stages of AD and mild cognitive impairment (MCI; defined as an early stage during which the first subtle symptoms manifest) and thus detecting early and calculating the risk of conversion of MCI into AD. Due to its limits (invasive), a CSF test is not routinely used as a biomarker for early diagnosis in preclinical AD stages before symptoms appear, nor it is suitable as a companion biomarker repeatedly practiced at several time points in same subjects recruited in longitudinal clinical trials.

Recently, specific neuroimaging methods relevant for AD pathology are being developed, notably with radioactive ligands for in vivo amyloid-beta (Aβ) ligands for Positron Emission Tomography (PET) neuroimaging including F-18 florbetapir and F-18 flutemetamol which have been approved by FDA and/or EMA (Choi et al., 2009 and Wong et al., 2010). However, their practice is costly and very limited (available only in some hospitals of large cities, still mainly for clinical research purposes as not reimbursed by health insurances) and their diagnosis accuracy is still under studies for further understanding. Thus, the use of these Aβ PET neuroimaging methods remains very limited and the vast majority of patients do not profit from such tests even in rich countries.

There is a need for better definition of AD patient population to be included for the drug-development trials: PET imaging using Aβ ligands showed that up to 30% of subjects diagnosed with AD show a negative Aβ scan and that up to 35% of subjects with normal cognition status show a positive Aβ scan (Gaël Chételat et al, Neurolmage Clinical 2013; 2:356-65). Thus, PET Aβ scan is being used to guide for recruitment of subjects in the desired cohorts in some clinical trials by the large pharmaceutical companies. Anti-Aβ antibody e.g. Solanezumab tested in patients with mixed mild to moderate AD failed to show clear efficacy. In mild AD patients selected based on CSF biomarker profile, the results showed a first promising efficacy (Carlson et al., Alzheimers Dement (Amst). 2016; 2:75-85; Siemers et al., Alzheimers Dement. 2016; 12(2): 110-20). However, the use of CSF biomarker is invasive, dramatically limiting its use as a biomarker for drug development. For example, it is not suitable for repeated use to monitor the stability and/or the progression of the disease, nor it is suitable to monitor in clinical longitudinal trials the efficacy of disease-modifying therapeutic drug candidates or preventive strategies in routine clinical practice use.

Overall, the existing tests either lack an easy accessibility and simplicity for use for diagnosis of the large AD population and/or lack accuracy (sensitivity and specificity). This represents a major impediment and bottleneck to develop reliable and rapid diagnosis test for AD. Another impediment is the identification of a biomarker that does not require invasive sample collecting, such as a spinal tap. The lack of such an accessible, sensitive and specific biomarker that could be validated by cellular, animal model, pre-clinical models, and human testing impedes the development of therapies and drugs for AD or for the studies on pathological processes triggering AD or involved in the progression of AD.

Today, clearly there is a high unmet medical need for efficient preventive or disease-modifying therapeutic treatment, as well as for an accurate and non-invasive peripheral biomarker test for early diagnosis of AD including preclinical and early MCI and for applications in drug development (patient stratification and repeated monitoring drug efficacy in clinical trials) and to monitor the efficacy and adjust the dosing novel future therapies once approved.

Likewise, for non-AD dementia, including DLB and for many FTD related neurodegenerative disorders, there is a high unmet medical need for efficient preventive or disease-modifying therapeutic treatment, and for an accurate and non-invasive peripheral biomarker test for diagnosis in particular of their early forms.

IncRNAs are typically defined as transcripts longer than 200 nucleotides and among the RNA families, IncRNAs seem to be the most tissue-specifically expressed. In the brain, IncRNAs can regulate gene expression at epigenetic, transcriptional, and posttranscriptional levels of proteins with diverse functions including neuronal transmission and synaptic plasticity. Recent studies identified a few IncRNA candidates in AD postmortem brain tissue; some IncRNA candidates have been shown in *in vitro* experiments or animal models to directly or indirectly regulate the formation of the neurotoxic Aβ, synaptic activity or the neuronal DNA repair (for review: Luo K and Chen Y, Clin Interv Aging 2016; 11: 867-872).

We previously showed that the IncRNAs expressed or highly enriched in tissues such as cardiac tissue or brain tissue, can be released into the peripheral circulation and be easily quantifiable by classical RT-PCR in different peripheral samples (PCT/EP2018/065492). In addition of the RT-PCR, we performed also total RNA sequencing on peripheral samples and quantified significant proportion of IncRNAs in the peripheral samples such as serum, plasma and Paxgene-RNA-tube collected whole blood (PCT/EP2018/065492).

Yaxing Gui ET AL ,Oncotarget, 10 November 2015 (2015-11-10), p. 37043, relates to altered microRNA profiles in cerebrospinal fluid exosome in Parkinson disease and Alzheimer disease.

According to the invention, new IncRNA signatures specific of brain diseases, in particular mild cognitive impairment (MCI) and Alzheimer or another cognitive disorder have been identified, using a method set up combining most recent technologies and samples collected in non-invasively manner, including Paxgene whole blood tube, serum and plasma.

### SUMMARY OF THE INVENTION

The present invention is defined in the appended claims. In the present disclosure, 1091 novel brain IncRNAs have been sequenced and identified for the first time in human postmortem brain tissue, in particular meditotemporal cortex of AD patients and cognitively intact healthy controls. Brain IncRNAs including novel brain IncRNAs with differential expression in the brain of AD patients when compared to the brain of healthy controls have been identified. Furthermore, new IncRNA signatures comprising the already sequenced and newly sequenced have been identified as differentially expressed in samples collected in non-invasively manner, including whole blood collected in Paxgene RNA tube and plasma derived from blood of patients suffering MCI, AD, DLB or FTD when compared to healthy controls, as well as panels that detect specifically MCI and/or one of the dementia type (AD or DLB or FTD) when compared to the other dementia types studied. Furthermore, brain-enriched IncRNAs including novel brain-enriched IncRNAs have been identified. These brain-enriched IncRNAs include circulating IncRNAs detectable in the peripheral body fluids plasma and whole blood Paxgene RNA samples.

In an aspect, the IncRNAs including novel IncRNAs identified as having deficient expression in AD brain represent novel therapeutic candidates for development as IncRNA molecule-enhancing therapeutic approaches, and the novel brain IncRNAs identified as having an over expression in AD brain represent novel therapeutic candidates for development as IncRNA molecule-silencing therapeutic approaches, both for treatment of brain disorders in particular Alzheimer's disease and other cognitive disorders.

In another aspect, the plasma and whole blood IncRNAs panels, identified as differentially expressed in patients suffering cognitive disorders are potential therapeutic targets and/or biomarkers for the prediction and/or the diagnosis and/or the differential diagnosis of brain disorders, in particular cognitive disorders in a subject at risk to develop AD, FTD, DLB and/or another cognitive disorder or dementia type.

In another aspect, the brain-enriched IncRNAs panels identified represent therapeutic and diagnostic targets for brain disorders, including but not limited to cognitive disorders such as MCI, AD, FTD, DLB.

The novel IncRNAs, the newly identified brain-enriched IncRNAs panels we called BrainLinc, the new IncRNAs panels identified as differentially expressed in the peripheral body fluids including the common to brain and plasma (or to brain and blood) IncRNAs identified can be quantified in the peripheral samples of patients using the innovative Celemics technology with significantly higher sensitivity, precision and/or accuracy that would not have been realized using other currently existing technologies. Monitoring of large number of IncRNAs targets by Cemelics and for quantification of small number of IncRNAs by qPCR and Quantamatrix are preferred methods to other existing methods such as NGS and HTG.

### BRIEF DESCRIPTION OF THE FIGURES

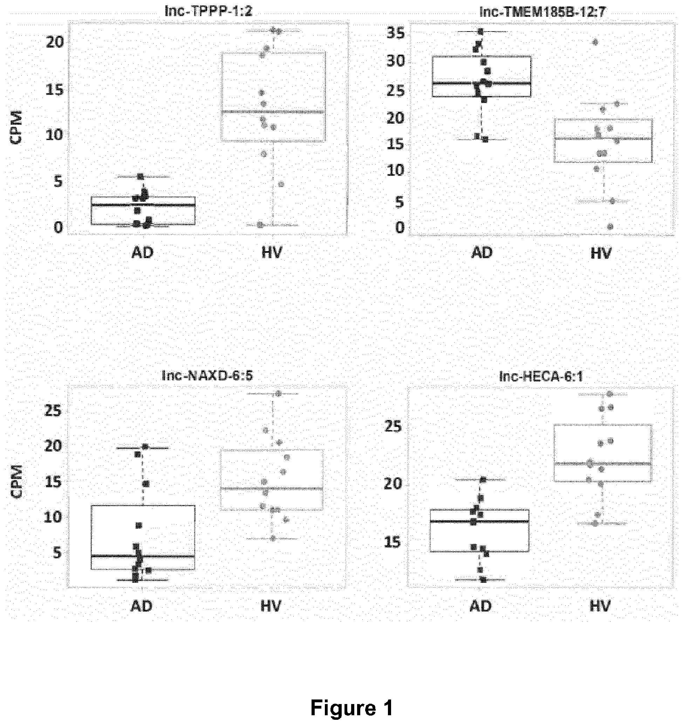

Figure 1 shows the expression level of IncRNA candidates Inc-TPPP-1:2, Inc-TMEM185B-12:7, Inc-NAXD-6:5 and Inc-HECA-6:1 on human serum samples from patients with early to moderate Alzheimer (AD) and age-matched healthy controls. Y axis: mean +/standard error of the mean of concentration of the IncRNAs in CPM (count per million). X axis: AD: Alzheimer patient group, HV: Healthy control group.

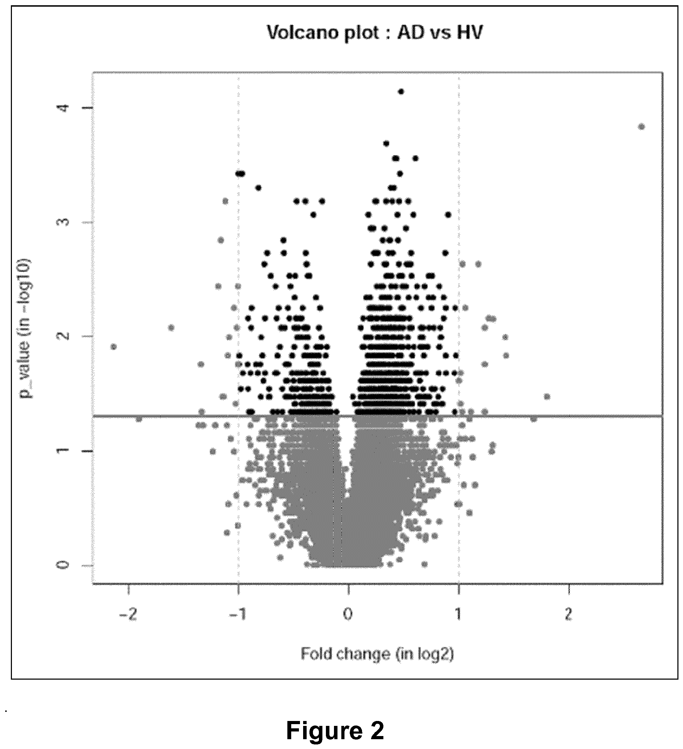

Figure 2 shows Volcano plot of the comparison. The volcano plot shows the serum 1008 IncRNAs that are differentially expressed in AD group versus HV (healthy control) group with statistically significance (p<0.05) above the line. 33 IncRNAs show both p value of <0.05 and fold change of ≥2 or <0.5. The IncRNAs that are differentially expressed but do not reach a statistically significance are shown below the line.

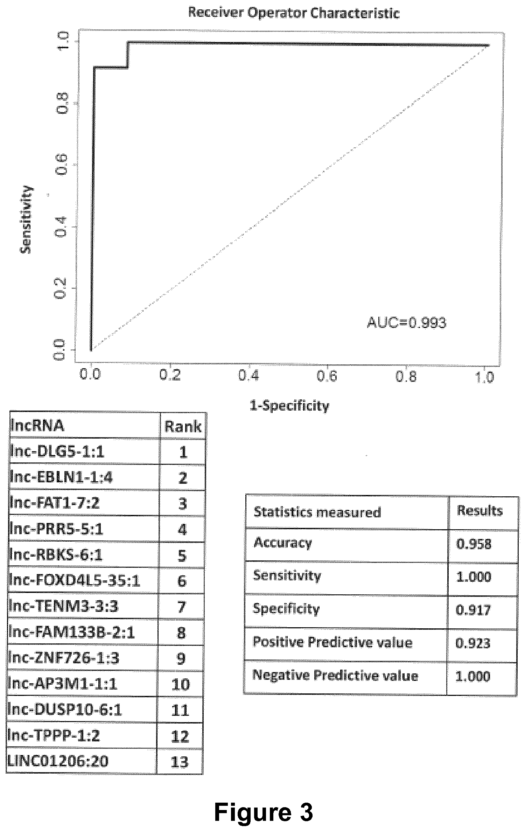

Figure 3 shows a predictive modelling based on the random forest algorithm enabling the identification of the signature of the 13-top ranked serum IncRNAs candidates selected out of 19867 IncRNAs.

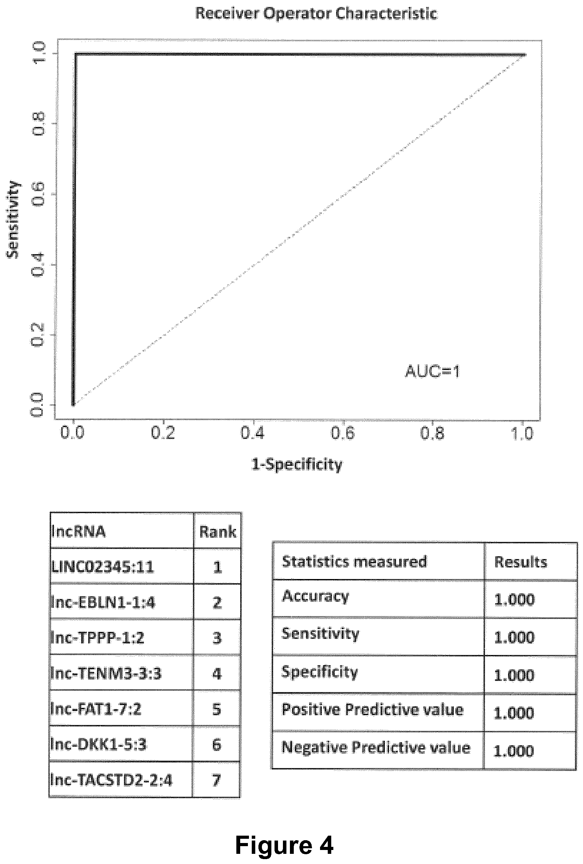

Figure 4 shows a predictive modelling based on the random forest algorithm enabling the identification of the signature of the 7-top ranked serum IncRNAs candidates out of 90 IncRNAs with a p value of <0.05 and fold change of ≥ 2 or ≤ 0.5 or an AUC of ≥ 0.85 or ≤ 0.15.

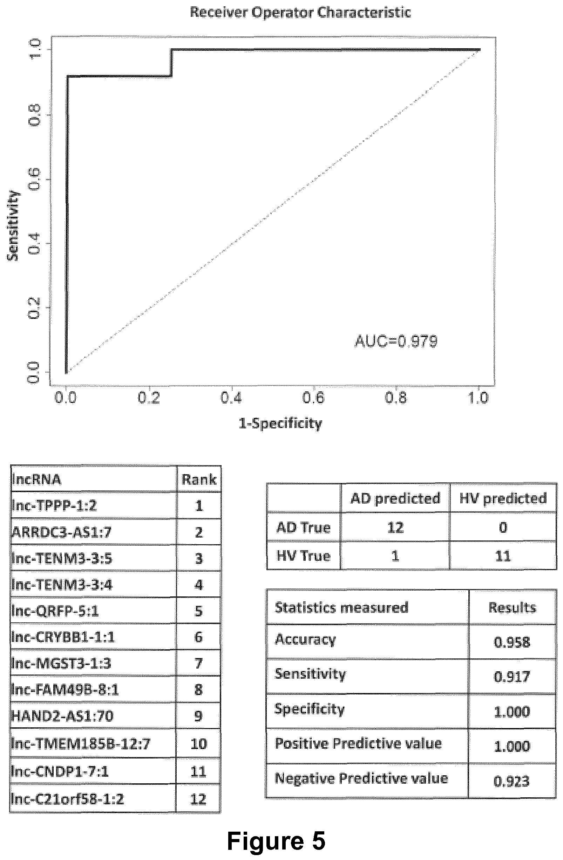

Figure 5 shows a predictive modelling based on the random forest algorithm enabling identification of the signature of the 12 top serum IncRNAs candidates selected out of the 90 IncRNAs (with a p value of <0.05 and_fold change of ≥ 1,6 or ≤ 0.6 and an AUC of ≥ 0.85 or ≤ 0.15.

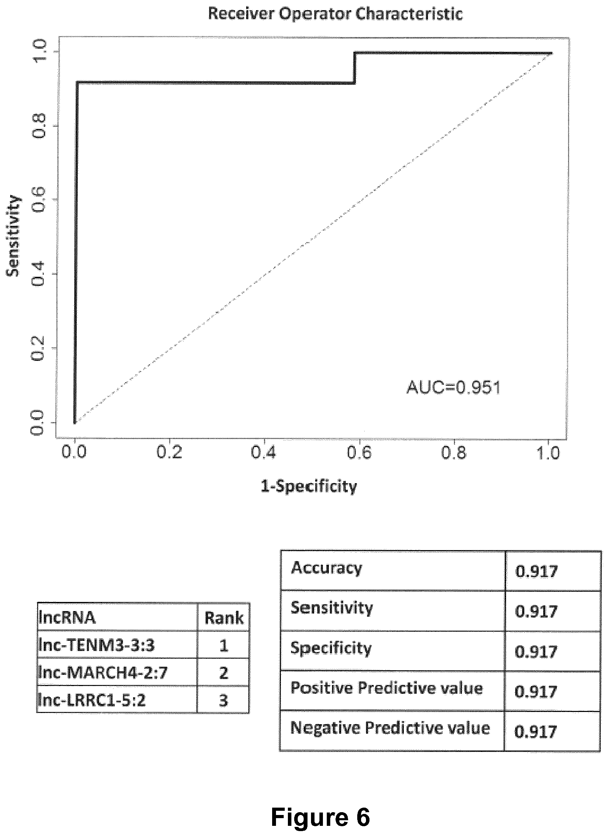

Figure 6 shows a predictive modelling based on the random forest algorithm enabling identification of the signature of the 3 top serum IncRNA candidates selected out of the IncRNAs with a p value of <0.05 and a good correlation (Pearson) with scores of neurocognitive tests including MMSE and/or MoCA out of 7 neuropsychological tests performed.

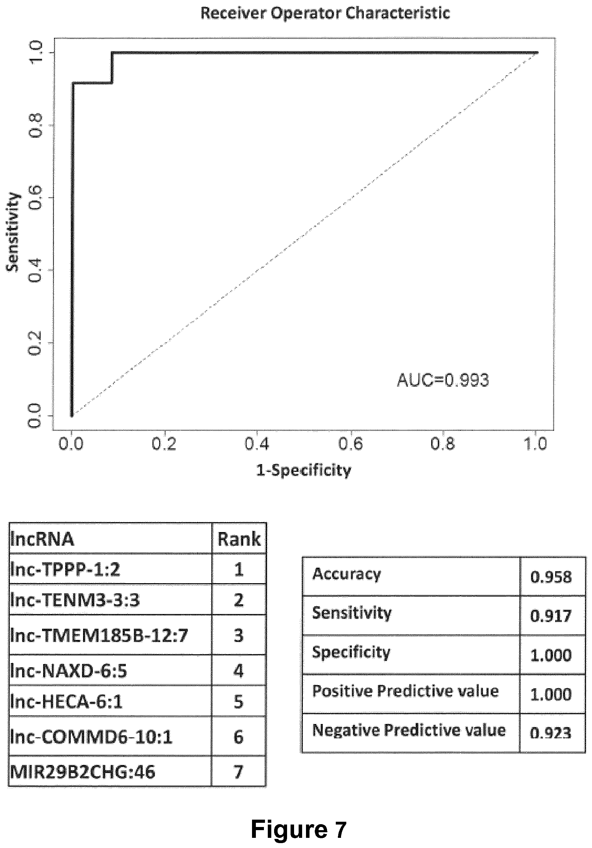

Figure 7 shows a predictive modelling based on the random forest algorithm enabling the identification of the signature of the 7 top IncRNA candidates selected out of serum IncRNAs with a p value of <0.05 and a good correlation (Pearson) with neuroimaging scores (volume of brain structures of relevance for cognition and memory such as the mediotemporal area, left and right hippocampus, left and right amygdala, entorhinal cortex out of more than 120 structures measured.

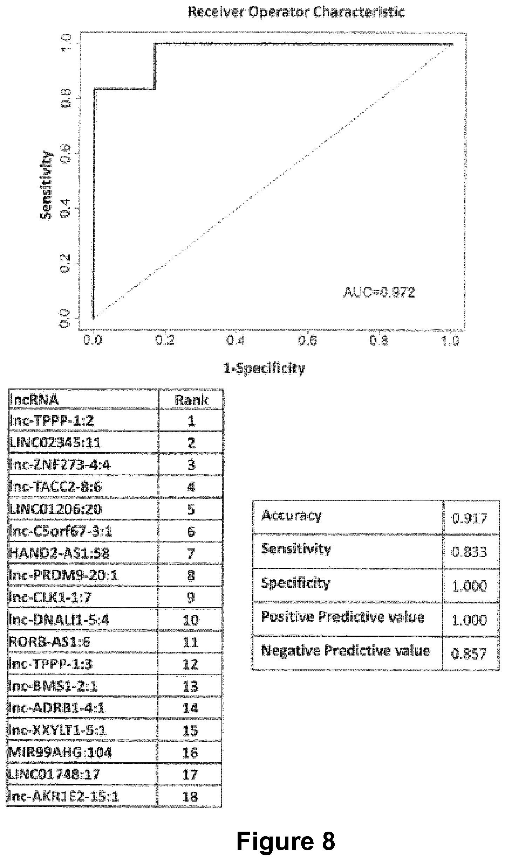

Figure 8 shows a predictive modelling based on the random forest algorithm enabling the identification of the signature of the 18 top serum IncRNA candidates selected out of IncRNAs that show a p value of <0.05 and a good correlation (Pearson) with CSF biomarkers Aβ42 and tau (total tau or phosphorylated tau).

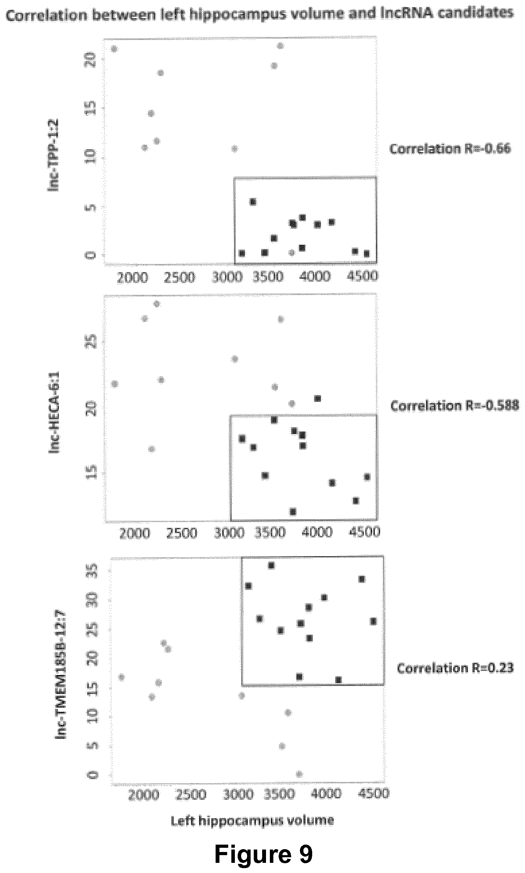

Figure 9 shows examples of results on correlation between the level of serum IncRNAs and the volumetric neuroimaging score of brain structures implicated in cognition such as the mediotemporal area and the hippocampus.

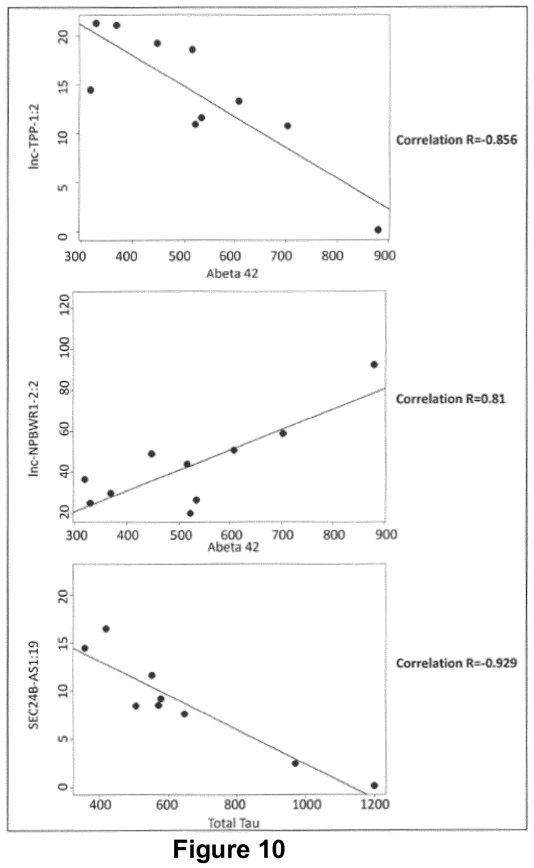

Figure 10 shows examples of results on correlation between serum IncRNAs and CSF biomarkers Aβ42 or Tau.

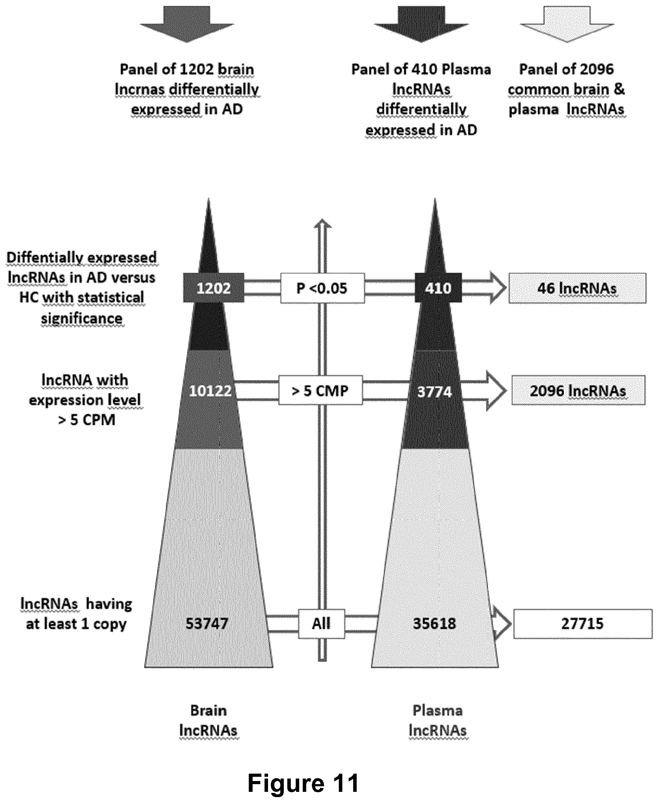

Figure 11 shows the scheme of identifying brain and plasma IncRNAs.

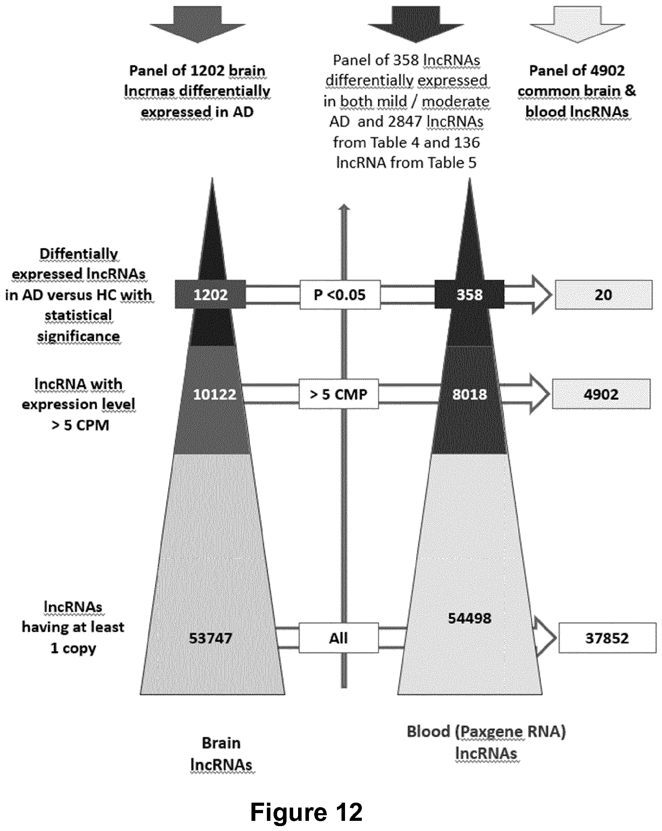

Figure 12 shows the scheme of identifying brain and blood (Paxgene RNA) IncRNAs.

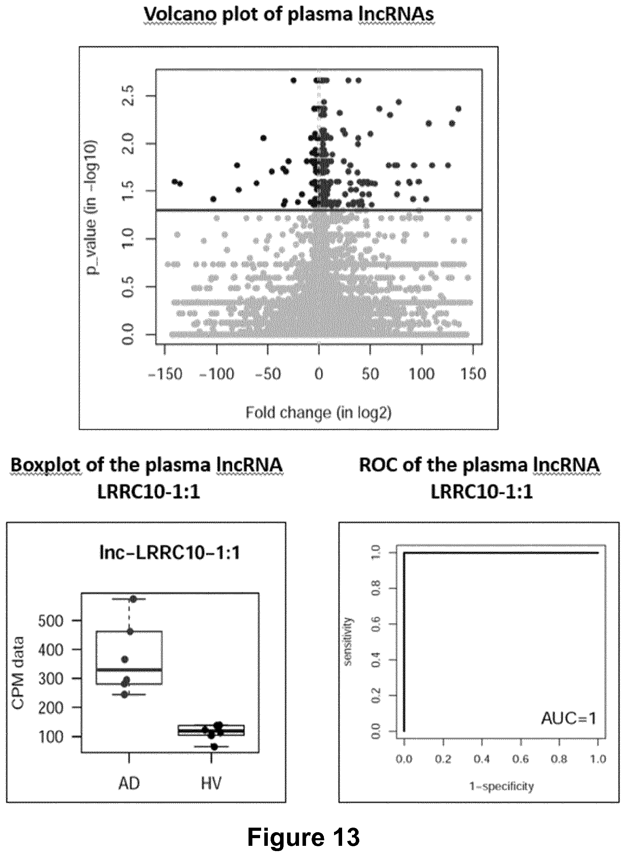

Figure 13 shows Volcano plot of plasma IncRNAs obtained when comparing 6 Alzheimer's patients (AD) and 6 healthy control subjects (HC) and boxplot of plasma IncRNA LRRC10-1:1 and and its ROC curve showing its AUC=1. The volcano plot shows the IncRNAs that are differentially expressed with statistically significance (p < 0.05, Wilcoxon test) above the line. The IncRNAs that are differentially expressed but did not reach a statistically significance, are shown below the line.

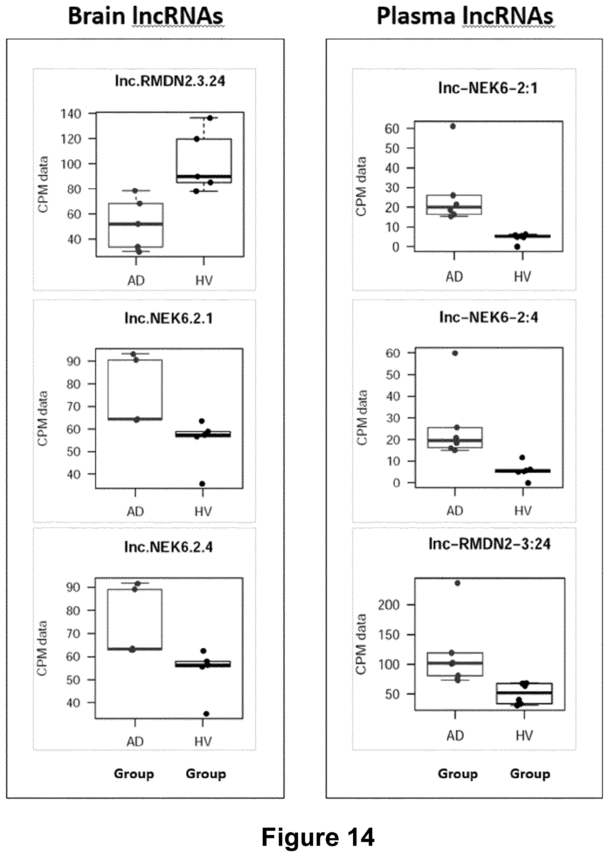

Figure 14 shows example of 3 brain IncRNAs differentially expressed in the brain of 5 Alzheimer's patients as compared to brain of 5 healthy control subjects that are also identified as circulating plasma IncRNAs and as differentially expressed candidates in plasma of 6 Alzheimer patients as compared to 6 healthy control subjects.

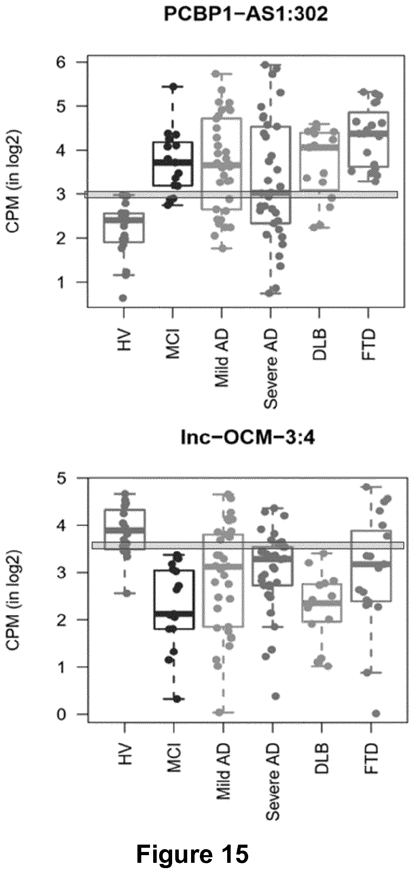

Figure 15 shows boxplots of 2 whole blood IncRNAs: one overexpressed, the other down- expressed in MCI group and dementia groups (AD, DLB, FTD) when compared to healthy control group.

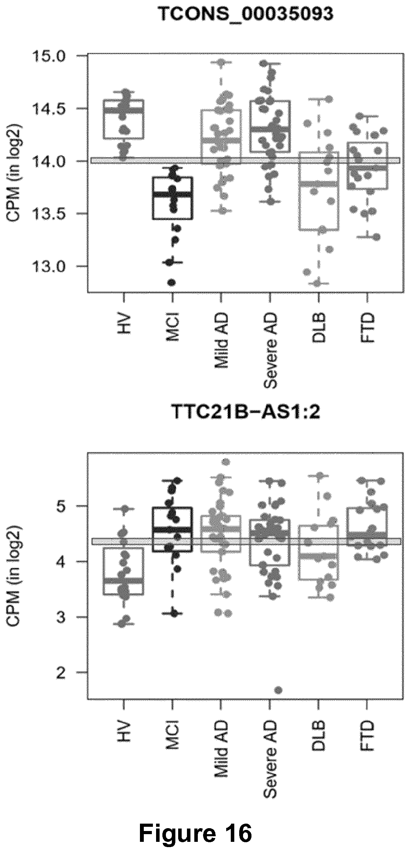

Figure 16 shows boxplots of 2 whole blood IncRNAs differentially expressed in at least one of the patient groups as compared to healthy control group. The IncRNA TCONS_00035093 shows decreased expression in all MCI patients as compared to healthy controls. The IncRNA TTC21B-AS1:2 shows increased expression in most patients with MCI and AD patients.

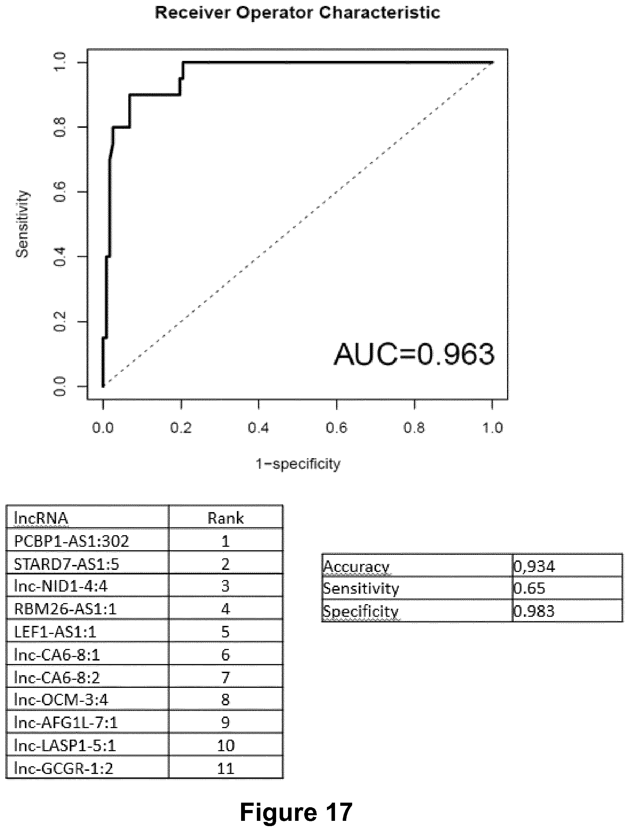

Figure 17 shows an example of a panel of whole blood IncRNAs discriminating between HC group versus all the other groups with an accuracy 93.4%.

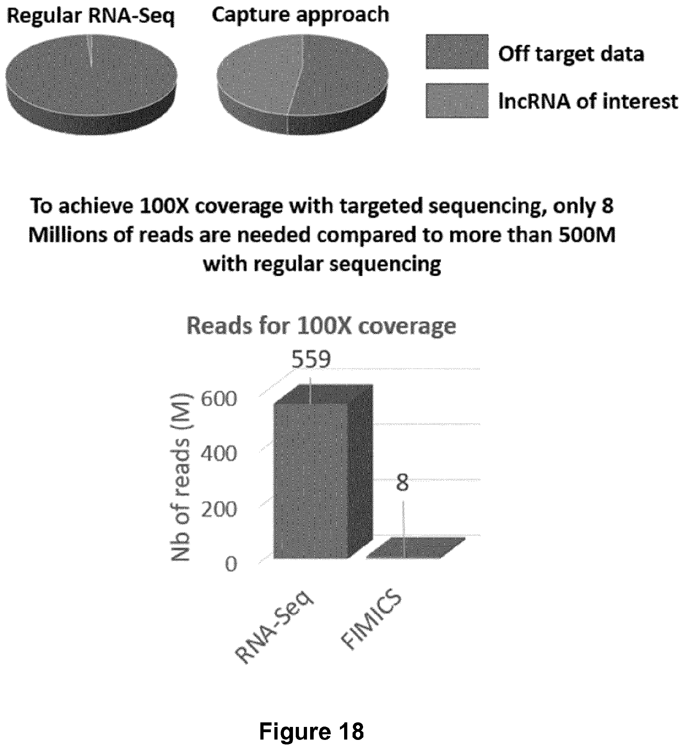

Figure 18 shows targeted sequencing approach which shows a better efficiency to quantify precisely the selected IncRNAs. As an example, the use of FIMICS show an enrichment of 60X of the sequence of interest, representing less than 1 % of the total sequences in global sequencing giving more difficult results to analyze. Only 8M of reads were needed to achieve a coverage of 100X allowing higher precision that regular RNA-Seq experiments.

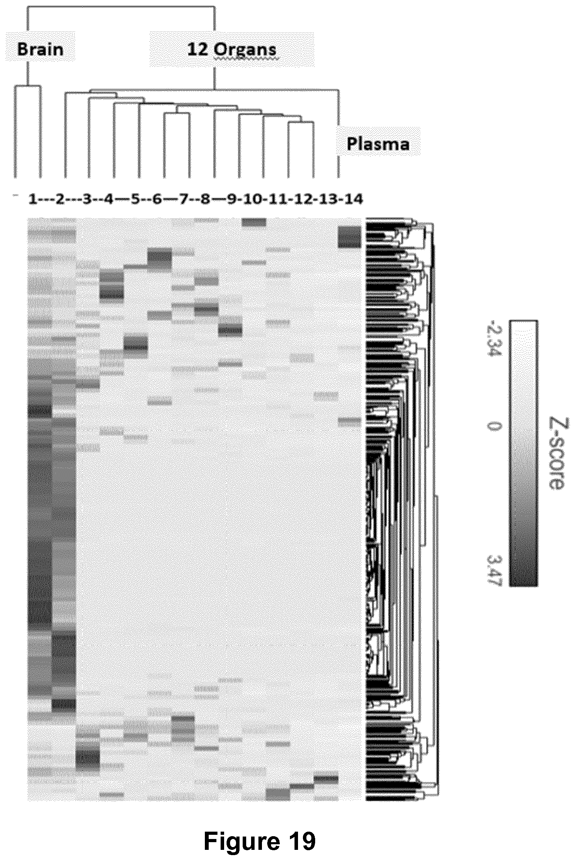

Figure 19 shows Heat map showing that many identified brain-enriched IncRNAs that are expressed in the brain of AD and/or non-AD human subjects (columns 1,2) but not or poorly expressed in the organs tested (columns 2 to 13).Interestingly out of these brain-enriched IncRNAs, some are circulating brain-enriched IncRNAs as expressed in the plasma. 1: AD brain; 2: Non-AD brain; 3: Lung; 4: Ovary; 5: Colon; 6: Prostate; 7: Breast; 8: Liver; 9: Bladder; 10: Kidney; 11: Skin; 12: Heart; 13: Muscle; 14: Plasma.

### DESCRIPTION OF THE INVENTION

IncRNAs, including the 1091 novel IncRNAs identified for the first time in the present application, that are disclosed in this disclosure may be involved in the pathogenesis of primary and secondary brain disorders including but not limited to neurodegenerative diseases, in particular Alzheimer's disease and cognitive disorders, neuropsychiatric disorders such as depression and anxiety, autism, neurological disorders such as multiple sclerosis, motoneuron disorders, stroke or implicated in systemic disorders, cardiovascular metabolic disorders indirectly involving the nervous system. Thus, these IncRNAs with altered expression represent novel targets for therapeutic and diagnostic applications. The novel brain IncRNAs identified as having an altered expression in AD brain may represent novel therapeutic candidates for development as IncRNA molecule-silencing therapeutic approaches for treatment of brain disorders in particular Alzheimer's disease and other cognitive disorders.

In addition, drug candidates or other medicinal products may induce cerebral toxicities such as autism or other syndromes. The IncRNAs identified in the present application might be used to diagnose, predict or monitor their toxicity or efficacy.

In an aspect, disclosed, but not part of the claimed invention, is IncRNAs for use in the treatment of brain diseases, in particular mild cognitive impairment (MCI) and Alzheimer or another cognitive disorder in a subject at risk of having or developing mild cognitive impairment or a cognitive disorder, wherein the treatment comprises administering to the subject an effective amount of a pharmaceutical agent modulating (inducing or inhibiting) the expression of the IncRNAs in the brain and/or in the periphery. The pharmaceutical agents may be specifically designed molecules such as small chemical entities or biological medicinal compounds, including but not limited to monoclonal antibodies, recombinant proteins, recombinant vector driven-therapeutics including but not limited to AV or AAV recombinant vectors, other RNA-related therapeutic means. In some embodiments, the pharmaceutical agent is administered to the subject with at least one pharmaceutically acceptable excipient. In some embodiments, the pharmaceutical agent is single or repeatedly administered to the subject as an oral, intravenous or subcutaneous or intra-cerebral treatment.

In some embodiments, the IncRNAs for use in the treatment of brain diseases are at least one novel IncRNA identified for the first time in the present application (1091 novel IncRNAs listed in Table 7). In some embodiments, the IncRNAs are at least one novel IncRNA selected from 922 IncRNAs (430 IncRNAs with Fold change<0.9 and 492 IncRNAs with Fold change >1.1 listed in Table 7). In a preferred embodiment, the IncRNAs are at least one IncRNAs selected from the below group, which is referred as "IncRNA signature 12" in this disclosure:

| | | | |
|---|---|---|---|
| **Novel IncRNA** | TCONS_00033653 | TCONS_00023309 | TCONS_00023401 |
| TCONS_00050539 | TCONS_00040878 | TCONS_00027765 | TCONS_00066358 |
| TCONS_00062555 | TCONS_00000634 | TCONS_00062602 | TCONS_00062306 |
| TCONS_00024916 | TCONS_00050178 | TCONS_00005325 | TCONS_00027438 |
| TCONS_00059198 | TCONS_00055496 | TCONS_00011974 | |
| TCONS_00066462 | TCONS_00033736 | TCONS_00023240 | |

In some embodiments, the IncRNAs for use in the treatment of brain diseases are at least one LINCipedia-based IncRNAs that are sequenced in the brain and identified by the present application to be differentially deficient or overexpressed in Alzheimer or dementia brain as compared to healthy control brain. In some embodiments, the IncRNAs are at least one IncRNAs selected from 681 IncRNAs with decreased level of expression in AD brain and 521 IncRNAs overexpressed in AD brain listed in Table 8. In a preferred embodiment, the IncRNAs are at least one IncRNAs selected from the below group, which is referred as "IncRNA signature 13" in this disclosure:

| | | |
|---|---|---|
| IncRNA | Inc-PMM2-6:2 | |
| LINC-PINT:16 | MALAT1:23 | Inc-LTBP3-2:3 |
| Inc-ATP6V1C1-12:1 | MALAT1:48 | MALAT1:11 |
| Inc-GABBR1-1:2 | Inc-LTBP3-2:6 | Inc-LTBP3-2:2 |

In another embodiment, the IncRNAs are at least one IncRNAs selected from the below group, which is referred as "IncRNA signature 42" in this disclosure:

| | |
|---|---|
| **LncRNAs** | Inc-FEM1B-4:1 |
| Inc-NEK6-2:1 | Inc-GNA14-3:1 |
| Inc-PMM2-6:4 | Inc-PINK1-2:1 |
| UGDH-AS1:10 | Inc-PRSS27-4:24 |
| Inc-RMDN2-3:24 | Inc-SLC25A3-7:1 |
| Inc-EIF1AD-1:1 | Inc-FEM1B-4:1 |
| Inc-FANCL-4:1 | NORAD:2 |
| Inc-FAAP100-2:1 | NORAD:8 |
| TCONS_00011974 | CACTIN-AS1:5 |

In some embodiments, the IncRNAs for use in the treatment of brain diseases are at least one brain-enriched IncRNAs identified as detectable in the brain while not expressed or at least 2-fold less expressed in peripheral organ biopsies: lung, ovary, colon, prostate, breast, liver, bladder, kidney, skin, heart, muscle. In some embodiments, the IncRNAs are at least one IncRNAs selected from 860 IncRNAs listed in Table 12. In a preferred embodiment, the IncRNAs are at least one IncRNAs selected from the below group, which is referred as "IncRNA signature 14" in this disclosure:

| | | |
|---|---|---|
| TCONS_00035022 | TCONS_00035024 | Inc-ENC1-5:1 |
| TCONS_00035023 | TCONS_00035091 | Inc-SNAP25-3:1 |
| TCONS_00035094 | TCONS_00012059 | Inc-HMGA1-2:4 |
| TCONS_00035093 | TCONS_00035092 | PEG3-AS1:1 |
| TCONS_00035021 | TCONS_00012060 | Inc-UNC80-1:1 |
| TCONS_00035090 | TCONS_00050014 | |

In some embodiments, the IncRNAs for use in the treatment of brain diseases are at least one IncRNA selected from a IncRNA signature selected from IncRNA signatures 1 to 42. IncRNA signatures 1 to 42 are defined below in this disclosure.

In an aspect, the invention relates to IncRNA signatures for use in the diagnosis of brain diseases, in particular mild cognitive impairment (MCI) and Alzheimer or another cognitive disorder or for use in the diagnosis and differential diagnosis for brain disorders types, in particular dementia types, including but not limited to Alzheimer, dementia with Lewy bodies and frontotemporal dementia, as defined in present claim 1.

IncRNA signatures comprising the already sequenced IncRNAs and newly sequenced IncRNAs that are differentially expressed in samples collected in non-invasively manner, including whole blood collected in Paxgene RNA tube and plasma derived from blood in patients suffering MCI, AD, DLB and/or FTD when compared to healthy controls, as well as panels that detect specifically MCI and one of the dementia type (AD or DLB or FTD) when compared to the other dementia types studied may be used. In some embodiments, the IncRNA signature is selected from the below group:

| **IncRNA signature** | **IncRNAs included in the IncRNA signature** |
|---|---|
| IncRNA signature 1 | 68 IncRNAs (TCONS_00050539, TCONS_00062555, TCONS_00024916, TCONS_00059198, TCONS_00066462, TCONS_00033653, LINC-PINT:16, Inc-ATP6V1C1-12:1, Inc-GABBR1-1:2, Inc-PMM2-6:2, MALAT1:23, MALAT1:48, Inc-LTBP3-2:6, Inc-LTBP3-2:3,Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, lnc-ZC3H12B-1:5, TCONS_00019798, TSPOAP1-AS1:39,TCONS_00035093, Inc-OCM-3:4, Inc-KIF14-1:2, PCBP1-AS1 :302, Inc-CA6-8:1, NEAT1:22, Inc-CA6-8:2, STARD7-AS1 :5, Inc-IL31 RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2) |
| IncRNA signature 2 | 54 IncRNAs (Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, Inc-ZC3H12B-1:5, TCONS_00019798, TSPOAP1-AS1:39,TCONS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2) |
| IncRNA signature 3 | 6 IncRNAs (Inc-ANAPC11-2:8, Inc-CLLU1-2:6, Inc-KLHL36-2:2, Inc-LRRC10-1:1, Inc-RMDN2-3:24, Inc-ZC3H12B-1:5) |
| IncRNA signature 4 | 11 IncRNAs (Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, Inc-ZC3H12B-1:5, TCONS_00019798, TSPOAP1-AS1:39) |
| IncRNA signature 5 | 43 IncRNAs (TCONS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2) |
| IncRNA signature 6 | 6 IncRNAs (TCONS_00045364, TCONS_00035023, TCONS_00035091,TCONS_00035022, TCONS_00035092, TCONS_00035093) |
| IncRNA signature 7 | 14 IncRNAs (TCONS_00050539, TCONS_00062555, TCONS_00024916, TCONS_00059198, TCONS_00066462, TCONS_00033653, LINC-PINT:16, Inc-ATP6V1C1-12:1, Inc-GABBR1-1:2, Inc-PMM2-6:2, MALAT1:23, MALAT1:48, Inc-LTBP3-2:6, Inc-LTBP3-2:3) |
| IncRNA signature 8 | 2 IncRNAs (TCONS_00035093, Inc-OCM-3:4) |
| IncRNA signature 9 | 7 IncRNAs (Inc-KIF14-1:2, PCBP1-AS1 :302, Inc-CA6-8:1, NEAT1:22, Inc-CA6-8:2, STARD7-AS1 :5, Inc-IL31 RA-1:1) |
| IncRNA signature 10 | 6 IncRNAs (Inc-CA6-8:2, Inc-ANKRD36-1:4, Inc-ATP6AP2-13:1, Inc-SOCS6-10:1, Inc-CDIPT-2:1, Inc-USP47-2:1) |
| IncRNA signature 11 | 7 IncRNAs (Inc-CA6-8:2, Inc-CA6-8:1, RBM26-AS1:1, PCBP1-AS1:302, MIR181A2HG:1, TTC21B-AS1:2, Inc-SOCS6-10:1) |
| IncRNA signature 12 | 21 IncRNAs (TCONS_00050539, TCONS_00062555, TCONS_00024916, TCONS_00059198, TCONS_00066462, TCONS_00033653, TCONS_00040878, TCONS_00000634, TCONS_00050178, TCONS_00055496, TCONS_00033736, TCONS_00023309, TCONS_00027765, TCONS_00062602, TCONS_00005325, TCONS_00011974, TCONS_00023240, TCONS_00023401, TCONS_00066358, TCONS_00062306, TCONS_00027438) |
| IncRNA signature 13 | 10 IncRNAs (LINC-PINT:16, Inc-ATP6V1C1-12:1, Inc-GABBR1-1:2, Inc-PMM2-6:2, MALAT1:23, MALAT1:48, Inc-LTBP3-2:6, Inc-LTBP3-2:3, MALAT1:11, Inc-LTBP3-2:2) |
| IncRNA signature 14 | 17 IncRNAs (TCONS_00035022, TCONS_00035023, TCONS_00035094, TCONS_00035093, TCONS_00035021, TCONS_00035090, TCONS_00035024, TCONS_00035091, TCONS_00012059, TCONS_00035092, TCONS_00012060, TCONS_00050014, Inc-ENC1-5:1, Inc-SNAP25-3:1, Inc-HMGA1-2:4, PEG3-AS1:1, Inc-UNC80-1:1) |
| IncRNA signature 15 | 2 IncRNAs (Inc-OCM-3:4, Inc-SLC35E3-8:1) |
| IncRNA signature 16 | 3 IncRNAs (Inc-ZCCHC13-4:1, PCBP1-AS1:302, Inc-NEMF-1:4) |
| IncRNA signature 17 | 6 IncRNAs (Inc-STAT3-1 :2, Inc-NEMF-1 :4, Inc-EPN2-3:2, Inc-SLC25A39-2:1, Inc-ARF6-1:1, Inc-CCNYL1-2:1) |
| IncRNA signature 18 | 5 IncRNAs (ANKRD44-IT1:1, APOBEC3B-AS1:2, ADAMTSL4-AS1:2, AGAP2-AS1:2, A1BG-AS1:14) |
| IncRNA signature 19 | 3 IncRNAs (LEF1-AS1:1, PCBP1-AS1:302, Inc-CA6-8:2) |
| IncRNA signature 20 | 5 IncRNAs (Inc-CGREF1-2:1, PSMB8-AS1:14, Inc-STX10-2:1, Inc-TCFL5-6:1, ZC3H12B-11:1) |
| IncRNA signature 21 | 3 IncRNAs (TCONS_00011994, Inc-EVX1-15:1, Inc-REC8-2:1) |
| IncRNA signature 22 | 3 IncRNAs (OIP5-AS1:36, MYLK-AS1:13, Inc-PCP4L1-3:2) |
| IncRNA signature 23 | 11 IncRNAs (PCBP1-AS1:302, STARD7-AS1:5, Inc-NID1-4:4, RBM26-AS1:1, LEF1-AS1:1, Inc-CA6-8:1, Inc-CA6-8:2, Inc-OCM-3:4, Inc-AFG1L-7:1, Inc-LASP1-5:1, Inc-GCGR-1 :2) |
| IncRNA signature 24 | 7 IncRNAs (Inc-CA6-8:2, Inc-ANKRD36-1:4, Inc-ATP6AP2-13:1, Inc-SOCS6-10:1, Inc-CDIPT-2:1, Inc-USP47-2:1, Inc-UXS1-3:1) |
| IncRNA signature 25 | 3 IncRNAs (Inc-OCM-3:4, Inc-SLC35E3-8:1, LINC-PINT:11) |
| IncRNA signature 26 | 2 IncRNAs (Inc-ZCCHC13-4:1, PCBP1-AS1:302) |
| IncRNA signature 27 | 5 IncRNAs (Inc-JUNB-1:1, Inc-RARB-4:1, Inc-ZNF284-1:1, TCONS_00035093, Inc-TELO2-3:3) |
| IncRNA signature 28 | 5 IncRNAs (Inc-CGREF1-2:1 PSMB8-AS1:14 Inc-STX10-2:1 Inc-TCFL5-6:1 Inc-ZC3H12B-11:1) |
| IncRNA signature 29 | 13 IncRNAs (Inc-DLG5-1:1, Inc-EBLN1-1 :4, Inc-FAT1-7:2, Inc-PRR5-5:1, Inc-RBKS-6:1, Inc-FOXD4L5-35:1, Inc-TENM3-3:3, Inc-FAM133B-2:1, Inc-ZNF726-1:3, Inc-AP3M1-1:1, Inc-DUSP10-6:1, Inc-TPPP-1:2, LINC01206:20) |
| IncRNA signature 30 | 7 IncRNAs (LINC02345:11, Inc-EBLN1-1 :4, Inc-TPPP-1 :2, Inc-TENM3-3:3, Inc-FAT1-7:2, Inc-DKK1-5:3, Inc-TACSTD2-2:4) |
| IncRNA signature 31 | 12 Inc RNAs (Inc-TPPP-1 :2, ARRDC3-AS1 :7, Inc-TENM3-3:5, Inc-TENM3-3:4, Inc-QRFP-5:1, Inc-CRYBB1-1:1, Inc-MGST3-1:3, Inc-FAM49B-8:1, HAND2-AS1:70, Inc-TMEM185B-12:7, Inc-CNDP1-7:1, Inc-C21orf58-1:2) |
| IncRNA signature 32 | 3 IncRNAs (Inc-TENM3-3:3, Inc-MARCH4-2:7, and Inc-LRRC1-5:2) |
| IncRNA signature 33 | 7 IncRNAs (Inc-TPPP-1:2, Inc-TENM3-3:3, Inc-TMEM185B-12:7, Inc-NAXD-6:5, Inc-HECA-6:1, Inc-COMMD6-10:1, and MIR29B2CHG:46) |
| IncRNA signature 34 | 18 IncRNAs (Inc-TPPP-1 :2, LINC02345:11, Inc-ZNF273-4:4, Inc-TACC2-8:6, LINC01206:20, Inc-C5orf67-3:1, HAND2-AS1:58, Inc-PRDM9-20:1, Inc-CLK1-1:7, Inc-DNALI1-5:4, RORB-AS1 :6, Inc-TPPP-1 :3, Inc-BMS1-2:1, Inc-ADRB1-4:1, Inc-XXYLT1-5:1, MIR99AHG:104, LINC01748:17, Inc-AKR1E2-15:1) |
| IncRNA signature 35 | 922 IncRNAs (430 IncRNAs with Fold change<0.9 and 492 IncRNAs with Fold change >1.1 listed in Table 7) |
| IncRNA signature 36 | 681 IncRNAs with decreased level of expression in AD brain and 521 IncRNAs overexpressed in AD brain listed in Table 8 |
| IncRNA signature 37 | 860 IncRNAs listed in Table 12 |
| IncRNA signature 38 | 410 IncRNAs listed in Table 9 |
| IncRNA signature 39 | 2847 IncRNAs listed in Table 10 and 136 IncRNAs listed in Table 11 |
| IncRNA signature 40 | 410 IncRNAs listed in Table 9, 2847 IncRNAs listed in Table 10 and 136 IncRNAs listed in Table 11 |
| IncRNA signature 41 | IncRNA signature 6 plus 8 IncRNAs listed in both Tables 9 and 12 or 55 IncRNAs listed in both Table 10 or 11 and Table 12 |
| IncRNA signature 42 | 17 IncRNAs (Inc-NEK6-2:1, Inc-PMM2-6:4, UGDH-AS1:10, Inc-RMDN2-3:24, Inc-EIF1AD-1:1, Inc-FANCL-4:1, Inc-FAAP100-2:1, TCONS_00011974, Inc-FEM1B-4:1, Inc-GNA14-3:1, Inc-PINK1-2:1, Inc-PRSS27-4:24, Inc-SLC25A3-7:1, Inc-FEM1B-4:1, NORAD:2, NORAD:8, CACTIN-AS1:5) |

The above 42 IncRNA signatures can be used in all methods or uses disclosed in this disclosure. However, the claimed invention only relates to signature 8.

In an aspect, the invention relates to a method for the diagnosis of a brain disorder including but not limited to cognitive disorder such as mild cognitive impairment (MCI), Alzheimer disease, frontotemporal dementia and/or dementia with Lewy body in a subject at risk of having or developing mild cognitive impairment or a cognitive disorder, comprising:
(a) isolating a biological sample from the subject;
(b) detecting a level of expression in a IncRNA signature, the IncRNA signature being signature number 8, in the biological sample from said subject;
(c) comparing the level of expression of IncRNA in the sample to a level of expression in a reference,
wherein an increased or decreased level of expression in the sample compared to the level in the reference identifies the subject having a cognitive disorder or being at risk of developing a cognitive disorder.

In some embodiments, the method further comprises a step of designing the therapeutic treatment according to the said identified brain disorder such as a cognitive disorder after step (c). In some embodiments, the biological sample is whole blood, serum or plasma.

In some embodiments, the IncRNA signature comprises or consist of at least one IncRNA selected from IncRNA signature 6 plus 8 IncRNAs listed in both Tables 9 and 12 or 55 IncRNAs listed in both Table 10 or 11 and Table 12 ("IncRNA signature 41"). In some embodiments, the IncRNA signature comprises or consist of at least one IncRNA selected from brain-enriched IncRNAs circulating in plasma sample including novel brain-enriched IncRNAs circulating in plasma. In some embodiments, the IncRNA signature comprises or consist of at least one IncRNA selected from 6 IncRNAs (TCONS_00045364, TCONS_00035023, TCONS_00035091, TCONS_00035022, TCONS_00035092, TCONS_00035093) ("IncRNA signature 6"). In some embodiments, the IncRNA signature is or comprises IncRNA signature 6.

In some embodiments, the IncRNA signature comprises or consist of at least one IncRNA selected from 410 IncRNAs listed in Table 9, 2847 IncRNAs listed in Table 10 and 136 IncRNAs listed in Table 11 ("IncRNA signature 40"). In some embodiments, the IncRNA signature comprises or consist of at least one IncRNA selected from 54 IncRNAs (Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, lnc-ZC3H12B-1:5, TCONS_00019798, TSPOAP1-AS1:39,TCONS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2)("IncRNA signature 2"). In some embodiments, the IncRNA signature is or comprises IncRNA signature 2.

In some embodiments, the IncRNA signature comprises or consist of at least one IncRNA selected from a IncRNA signature selected from IncRNA signatures 1 to 42. In some embodiments, the IncRNA signature is or comprises a IncRNA signature selected from IncRNA signatures 1 to 42.

Disclosed, but not part of the claimed invention, is , a method for the diagnosis of a brain disorder including but not limited to cognitive disorder such as mild cognitive impairment (MCI), Alzheimer disease, frontotemporal dementia and/or dementia with Lewy body in a subject at risk of having or developing mild cognitive impairment or a cognitive disorder, comprising:
(a) isolating a biological sample from the subject, wherein the biological sample is plasma;
(b) detecting a level of expression in a IncRNA signature, the IncRNA signature comprising or consisting of at least one IncRNA selected from 410 plasma IncRNAs listed in Table 9 ("IncRNA signature 38"), in the biological sample from said subject;
(c) comparing the level of expression of IncRNA in the sample to a level of expression in a reference,
wherein an increased or decreased level of expression in the sample compared to the level in the reference identifies the subject having a cognitive disorder or being at risk of developing a cognitive disorder.

In some embodiments, the method further comprises a step of designing the therapeutic treatment according to the said identified brain disorder such as a cognitive disorder after step (c).

In some embodiments, the IncRNA signature comprises or consist of at least one IncRNA selected from 6 IncRNAs (Inc-ANAPC11-2:8, Inc-CLLU1-2:6, Inc-KLHL36-2:2, Inc-LRRC10-1:1, Inc-RMDN2-3:24, Inc-ZC3H12B-1:5) "IncRNA signature 3". In a preferred embodiment, the IncRNA signature is or comprises IncRNA signature 3.

In some embodiments, the IncRNA signature comprises or consist of at least one IncRNA selected from 11 IncRNAs (Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, Inc-ZC3H12B-1:5, TCONS_00019798, TSPOAP1-AS1:39)) "IncRNA signature 4". In a preferred embodiment, the IncRNA signature is or comprises IncRNA signature 4.

Disclosed, but not part of the claimed invention, is a method for the diagnosis of a brain disorder including but not limited to cognitive disorder such as mild cognitive impairment (MCI), Alzheimer disease, frontotemporal dementia and/or dementia with Lewy body in a subject at risk of having or developing mild cognitive impairment or a cognitive disorder, comprising:
(a) isolating a biological sample from the subject, wherein the biological sample is whole blood;
(b) detecting a level of expression in a IncRNA signature, the IncRNA signature comprising or consisting of at least one IncRNA selected from 2847 IncRNAs listed in Table 10 and 136 IncRNAs listed in Table 11 ("IncRNA signature 39"), in the biological sample from said subject;
(c) comparing the level of expression of IncRNA in the sample to a level of expression in a reference,
wherein an increased or decreased level of expression in the sample compared to the level in the reference identifies the subject having a cognitive disorder or being at risk of developing a cognitive disorder.

In some embodiments, the method further comprises a step of designing the therapeutic treatment according to the said identified brain disorder such as a cognitive disorder after step (c).

In some embodiments, the IncRNA signature comprises or consist of at least one IncRNA selected from 43 IncRNAs (TCONS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31 RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2) ("IncRNA signature 5"). In a preferred embodiment, the IncRNA signature is or comprises IncRNA signature 5.

Disclosed, but not part of the claimed invention, is a method for the diagnosis of a brain disorder including but not limited to cognitive disorder such as mild cognitive impairment (MCI), Alzheimer disease, frontotemporal dementia and/or dementia with Lewy body in a subject at risk of having or developing mild cognitive impairment or a cognitive disorder, comprising:
(a) isolating two or more biological samples (such as both plasma and whole blood) from the subject;
(b) detecting a level of expression in a IncRNA signature, the IncRNA signature comprising or consisting of at least one IncRNA selected from 410 IncRNAs listed in Table 9, 2847 IncRNAs listed in Table 10 and 136 IncRNAs listed in Table 11 ("IncRNA signature 40"), in the biological samples from said subject;
(c) comparing the level of expression of IncRNA in the sample to a level of expression in a reference,
wherein an increased or decreased level of expression in the sample compared to the level in the reference identifies the subject having a cognitive disorder or being at risk of developing a cognitive disorder.

In some embodiments, the method further comprises a step of designing the therapeutic treatment according to the said identified brain disorder such as a cognitive disorder after step (c). In some embodiments, the two or more biological samples are plasma and whole blood.

In some embodiments, the IncRNA signature comprises or consist of at least one IncRNA selected from 54 IncRNAs (Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, Inc-ZC3H12B-1:5, TCONS_00019798, TSPOAP1-AS1:39,TCONS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2)("IncRNA signature 2"). In some embodiments, the IncRNA signature is or comprises IncRNA signature 2.

Disclosed, but not part of the claimed invention, is a method for diagnosis and differential diagnosis for brain disorders types, in particular dementia types, including but not limited to Alzheimer, dementia with Lewy bodies and frontotemporal dementia in a subject suffering from a brain disorder such as loss or impairment of cognitive function or dementia of subtype to be diagnosed, said method comprising :
(a) isolating two or more biological samples (such as both plasma and whole blood) from the subject;
(b) detecting a level of expression in a IncRNA signature, the IncRNA signature comprising or consisting of at least one IncRNA selected 410 IncRNAs listed in Table 9, 2847 IncRNAs listed in Table 10 and 136 IncRNAs listed in Table 11 ("IncRNA signature 40"), in the biological samples from said subject;
(c) comparing the level of expression of IncRNA in the sample to a level of expression in a reference,
wherein an increased or decreased level of expression in the sample compared to the level in the reference identifies the subject having a cognitive disorder or being at risk of developing a cognitive disorder.

In some embodiments, the method further comprises a step of designing the therapeutic treatment according to the said identified brain disorder such as a cognitive disorder after step (c). In some embodiments, the biological sample is plasma or whole blood.

In some embodiments, the IncRNA signature comprises or consist of at least one IncRNA selected from 54 IncRNAs (Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, Inc-ZC3H12B-1:5, TCONS_00019798, TSPOAP1-AS1:39,TCONS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2)("IncRNA signature 2"). In some embodiments, the IncRNA signature is or comprises IncRNA signature 2.

Disclosed, but not part of the claimed invention, is a method for monitoring the development or progression of brain disorders including but not limited to cognitive disorder such as mild cognitive impairment, Alzheimer disease, dementia with Lewy bodies frontotemporal dementia in a subject suffering from a brain disorder including loss or impairment of cognitive function or dementia, said method comprising the following steps:
(a) isolating a biological sample from the subject;
(b) detecting a level of expression in a IncRNA signature, the IncRNA signature comprising or consisting of at least one IncRNA selected from 410 IncRNAs listed in Table 9, 2847 IncRNAs listed in Table 10 and 136 IncRNAs listed in Table 11 ("IncRNA signature 40"), in the biological sample from said subject;
(c) comparing the level of expression of IncRNA in the sample to a level of expression in a reference,
wherein an increased or decreased level of expression in the sample compared to the level in the reference identifies the subject having a cognitive disorder or being at risk of developing a cognitive disorder.

In some embodiments, the method further comprises a step of designing the therapeutic treatment according to the said identified brain disorder such as a cognitive disorder after step (c). In some embodiments, the biological sample is plasma or whole blood.

In some embodiments, the IncRNA signature comprises or consist of at least one IncRNA selected from 54 IncRNAs (Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, Inc-ZC3H12B-1:5, TCONS_00019798, TSPOAP1-AS1:39,TCONS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2)("IncRNA signature 2"). In some embodiments, the IncRNA signature is or comprises IncRNA signature 2.

A biological sample may be a small part of a subject obtained from a body fluid, e.g. blood, plasma, serum, saliva, urine, tear or cerebrospinal fluid or organ tissue. Suitably the biological sample are plasma or serum or whole blood (Paxgene-RNA-tube).

By brain disorder is meant e.g. a cognitive disorder such as a dementia type, a neurodegenerative disease, neurological or neuropsychatric disorder or systemic disorder involving the brain from the below example of brain disease list):
**Dementia types** and subtypes are numerous and include e.g.
   Alzheimer (AD) and MCI of AD type
   Frontotemporal dementia (FTD) and MCI of FTD type
   Progressive supranuclear palsy (PSP)
   Corticobasal degeneration (CBD)
   Dementia with Lewy body (DLB) and MCI of DLB type
   Parkinson's disease dementia (PDD) and MCI of PDD type
   Vascular dementia and related MCI
   Stroke-related MCI and dementia
   Other dementia types,
**Neurodegenerative and neurological and neuropsychiatric disorders** with a cognitive impairment often occurring early or at later stages of the disease progression:
   Parkinson disease
   Huntington disease
   Autism
   Amyotrophic lateral sclerosis
   Motoneuron diseases
   Multiple sclerosis
   Neuropathies
   Major depressive disorder
   Anxiety
**Systemic disorders with brain involvement** and negative impact on the brain health such as brain cognitive impairment, including metabolic disorders such as diabetes, cardiovascular such as stroke, autoimmune and inflammatory disorders.

### For diagnosis of brain disorder including cognitive impairment or dementia:

The "reference" may be suitable control sample such as for example a sample from a normal, healthy subject having no brain disorder or cognitive impairment symptoms and no abnormal neuroimaging findings and being age-matched to the patient to be diagnosed with the method of the present invention. The reference may be a sample from the same subject prior to demonstration of disorder or disease symptoms or prior to diagnosis of the brain disorder or the impairment or loss of cognitive function or dementia. The reference may be a standardized sample, e.g. a sample comprising material or data from several samples of healthy subjects who have no brain or cognitive impairment symptoms and no abnormal neuroimaging findings.

### For therapeutics, diagnosis or differential diagnosis of a specific dementia type or its mild cognitive impairment (MCI) versus other dementia types and their MCI:

Brain disorders and subtypes are numerous and include cognitive disorders with dementia types and subtypes:
**Dementia types** and subtypes are numerous and include e.g.
   Alzheimer (AD) and MCI of AD type
   Frontotemporal dementia (FTD) and MCI of FTD type
   Progressive supranuclear palsy (PSP)
   Corticobasal degeneration (CBD)
   Dementia with Lewy body (DLB) and MCI of DLB type
   Parkinson's disease dementia (PDD) and MCI of PDD type
   Vascular dementia and related MCI
   Stroke-related MCI and dementia
   Other dementia types,
   **Neurodegenerative and neurological and neuropsychiatric disorders** with a cognitive impairment often occurring early or at later stages of the disease progression:
      Parkinson disease
      Huntington disease
      Amyotrophic lateral sclerosis
      Autism Multiple sclerosis
      Motoneuron diseases
      Neuropathies
      Major depressive disorder
      Anxiety
**Systemic disorders with brain involvement** and negative impact on the brain health such as brain cognitive impairment, including metabolic disorders such as diabetes, cardiovascular such as stroke, autoimmune and inflammatory disorders.

For differential diagnosis of one brain disorder the reference sample may be sample(s) from patient(s) suffering another brain disorder of the same class of disorders (for example for differential diagnosis of a dementia subtype, the reference sample may be sample(s) from patient(s) suffering dementia of other type(s)). and/or to reference value obtained on samples from subject(s) who may suffer another disease but have no symptom similar to the targeted brain disorder (for example for differential diagnosis of a dementia, reference sample(s) may be from a patient having no cognitive impairment or dementia).

The invention also relates to a method of diagnosing a brain disorder including but not limited to cognitive disorder such as Alzheimer disease, frontotemporal dementia, dementia with Lewy bodies and monitoring its development or progression in a subject suffering from progressive impairment or loss of cognitive function or dementia, comprising:
(a) determining the presence of a brain disorder such as a cognitive disorder using a biological sample of the said subject using a method according to the invention, and
(b) adapting a therapeutic treatment in function of the results of step (a).

The invention also relates to a method of diagnosing a brain disorder including but not limited to cognitive disorder such early MCI and very mild Alzheimer disease in a subject with no apparent and/or no measurable symptoms when using the available methods to detect clinical symptoms (such as MMSE, MoCA and other neurocognitive tests for cognitive disorder), but with abnormal changes of biomarkers of the present invention preceding the symptoms, comprising:
(a) determining the risk of developing a cognitive disorder using a biological sample of the said subject using a method according to the invention, and
(b) adapting a therapeutic treatment in function of the results of step (a).

The invention also relates to a method of future improved diagnosing a brain disorder including but not limited to a cognitive disorder such as early MCI and very mild Alzheimer disease in a subject by measuring the biomarkers of the present invention in combination with risk factors, such as genotypes including but not limited to ApoE gene alleles, cardiovascular, metabolic, inflammatory biomarkers, comprising:
(a) determining the risk of developing a cognitive disorder using a biological sample of the said subject using a method according to the invention in combination with one of the above-mentioned risk factors, and
(b) adapting a therapeutic treatment in function of the results of step (a).

The invention further relates to a method of predictive diagnosing a brain disorder including but not limited to a cognitive disorder such as early MCI and early stages of Alzheimer disease or frontotemporal dementia or dementia with Lewy bodies in a subject with no apparent and/or no measurable abnormality by neuroimaging methods as MRI and/or CT scans, comprising:
(a) determining the risk of developing a cognitive disorder using a biological sample of the said subject using a method according to the invention, and
(b) adapting a therapeutic treatment in function of the results of step (a).

The invention further relates to a method of predictive diagnosing a cognitive disorder including but not limited to early MCI and early stages of Alzheimer disease or frontotemporal dementia or dementia with Lewy bodies in a subject with no apparent and/or no measurable abnormality by molecular neuroimaging methods, such as but not limited to amyloid or tau or dopaminergic or synucleinergic PET scan, comprising:
(a) determining the risk of developing a cognitive disorder using a biological sample of the said subject using a method according to the invention, and
(b) adapting a therapeutic treatment in function of the results of step (a).

The invention further relates to a method of diagnosing or differential diagnosing or monitoring a cognitive disorder including but not limited to early MCI and early stages of Alzheimer disease or frontotemporal dementia or dementia with Lewy bodies in a subject using combination(s) of biomarkers of the present invention with a neuroimaging method including molecular neuroimaging methods, comprising:
(a) determining the risk of developing a cognitive disorder using a biological sample of the said subject using a method according to the invention, and
(b) adapting a therapeutic treatment in function of the results of step (a).

Disclosed, but not part of the claimed invention, is a method for treating a subject suffering from a progressive impairment or loss of cognitive function or dementia, said method comprising:
(a) diagnosing the cognitive disorder associated to the progressive impairment or loss of cognitive function or dementia in said patient using the method according to the invention, and
(b) adapting the therapeutic treatment to the results obtained in step (a).

Examples of therapeutic treatment of cognitive disorders may comprise the administration of:
- a drug already approved for treatment of brain disorder
   ∘ for example, for a drug already approved for treatment of Alzheimer's disease, among cholinesterase inhibitors, donenepezil, rivastigmine or galantamine, or a NMDA receptor antagonist, memantine, or a combination of, e.g. donepezil and memantine, and/or
- a therapeutic candidate or combination under clinical development
   ∘ for example as currently tested in Alzheimer patients in the anti-amyloid field, e.g. beta-secretase inhibitors and anti-beta-amyloid monoclonal antibodies, and anti-tau approaches, or as currently tested in the anti-tau field, e.g. modulators of kinases or phosphatases that regulate tau phosphorylation status and anti-tau antibodies, drug and drug candidates modulating the molecular pathways of neurodegeneration, oxidative stress, autophagy, mitochondrial dysfunction and immuno-inflammation, and/or
- a novel therapeutic approach derived from the present invention.

Disclosed, but not part of the claimed invention, is a kit for diagnosing and/or monitoring a brain disorder including but not limited to cognitive disorders such as MCI, AD, FTD, DLB, comprising at least one reagent for the determination of a IncRNA expression profile comprising or consisting of at least one IncRNA selected from 410 IncRNAs listed in Table 9, 2847 IncRNAs listed in Table 10 and 136 IncRNAs listed in Table 11 ("IncRNA signature 40"). In some embodiments, the IncRNA expression profile comprises or consist of at least one IncRNA selected from 54 IncRNAs (Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, Inc-ZC3H12B-1:5, TCONS_00019798, TSPOAP1-AS1:39,TCONS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2)("IncRNA signature 2"). In some embodiments, the IncRNA expression profile comprises or consist of at least one IncRNA selected from a IncRNA signature selected from IncRNA signatures 1 to 42. In some embodiments, the IncRNA expression profile comprises or consists of a IncRNA signature selected from IncRNA signatures 1 to 42. In some embodiments, the IncRNA expression profile is or comprises IncRNA signature 2. Claimed is a kit with oligonucleotides for signature 8.

The kit allows performing the measurement of the IncRNA signature of the invention, wherein the kit comprises at least one reagent for measuring at least one IncRNA selected from the IncRNAs as indicated above.

By "reagent" is also meant a reagent which specifically allows the determination of the IncRNA expression profile, i.e. a reagent specifically intended for the specific determination of the expression level of the IncRNA present in the IncRNA expression profile. Examples include e.g. amplification primer pairs (forward and reward) and/or probes specific for the IncRNA present in the IncRNA expression profile. This definition excludes generic reagents useful for the determination of the expression level of any other IncRNA.

In some embodiments, the kit for diagnosing and/or monitoring a brain disorder including but not limited to cognitive disorders such as MCI, AD, FTD, DLB comprises one or more oligonucleotide probes specific for IncRNAs of interest and a reagent for purifying the probe-target nucleic acid complexes. The oligonucleotide probes comprise a sequence complementary to a region of the IncRNAs of interest. The oligonucleotide probes may be DNA or RNA. The oligonucleotide probes are preferably DNA. The length of oligonucleotide probes specific for IncRNAs may be from 30 to 80 nucleotides, e.g., from 40 to 70, from 40 to 60, or about 50 nucleotides. In a preferred embodiment, the oligonucleotide probes are biotinylated and the reagent for purifying the probe-target complexes is a streptavidin-coated substrate, e.g, a streptavidin-coated magnetic particle, e.g., T1 streptavidin coated magnetic bead.

Disclosed, but not part of the claimed invention, is a targeted sequencing panel for next generation sequencing, comprising nucleic acids specific for at least one IncRNA selected from 68 IncRNAs (TCONS_00050539, TCONS_00062555, TCONS_00024916, TCONS_00059198, TCONS_00066462, TCONS_00033653, LINC-PINT:16, Inc-A TP6V1 C1-12:1, Inc-GABBR1-1:2, Inc-PMM2-6:2, MALAT1:23, MALAT1:48, Inc-LTBP3-2:6, Inc-LTBP3-2:3,lnc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, lnc-ZC3H12B-1:5, TCONS_00019798, TSPOAP1-AS1:39,TCONS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2) ("IncRNA signature 1"). In some embodiments, the at least one IncRNA is selected from a IncRNA signature selected from IncRNA signatures 1 to 42. In some embodiments, the at least one IncRNA comprises or consists of a IncRNA signature selected from IncRNA signatures 1 to 42. In a preferred embodiment, the at least one IncRNA is or comprises IncRNA signature Claimed is a targeted sequencing panel for signature 8. 1.

Disclosed, but not part of the claimed invention, are applications of the method for development of new therapeutic strategies (drug candidates) tested in clinical trials for the treatment of a brain disorder including but not limited to a cognitive disorder such as Alzheimer disease, dementia with lewy bodies, frontotemporal dementia wherein the method can be used (a) before starting the treatment for the selection of patients who would then be recruited in clinical trial; thus the test will enhance the likelihood for treating patient population that benefits from the tested therapeutic strategy(ies) while avoiding recruitment of patient subpopulation patients who do not benefit from this tested new drug candidate(s) and/or (b) for monitoring the response(s) including efficacy of the tested therapeutic strategy(ies) once treatment with the new tested drug candidate starts.

Disclosed, but not part of the claimed invention, are methods of detection. For example, the invention contemplates a method for the detection of one or more IncRNAs. In one aspect the method comprises:
(a) isolating a biological sample from the subject;
(b) detecting a level of expression in a IncRNA signature, the IncRNA signature comprising or consisting of at least one IncRNA selected from 68 IncRNAs (TCONS_00050539, TCONS_00062555, TCONS_00024916, TCONS_00059198, TCONS_00066462, TCONS_00033653, LINC-PINT:16, Inc-ATP6V1 C1-12:1, Inc-GABBR1-1:2, Inc-PMM2-6:2, MALAT1:23, MALAT1:48, Inc-LTBP3-2:6, Inc-LTBP3-2:3,Inc-ANAPC11-2:8, Inc-CLLU1-2:6, Inc-KLHL36-2:2, Inc-LRRC10-1:1, Inc-RMDN2-3:24, Inc-SMN2-4:2, Inc-SPRYD3-1:17, Inc-USP47-2:1, lnc-ZC3H12B-1:5, TCONS_00019798, TSPOAP1-AS1:39,TCGNS_00035093, Inc-OCM-3:4, Inc-KIF14-1:2, PCBP1-AS1:302, Inc-CA6-8:1, NEAT1:22, Inc-CA6-8:2, STARD7-AS1:5, Inc-IL31RA-1:1, Inc-ANKRD36-1:4, Inc-ATP6AP2-13:1, Inc-SOCS6-10:1, Inc-CDIPT-2:1, Inc-USP47-2:1, RBM26-AS1:1, MIR181A2HG:1, TTC21B-AS1:2, LEF1-AS1:1, Inc-SLC35E3-8:1, Inc-ZCCHC13-4:1, Inc-NEMF-1:4, TCONS_00011994, Inc-EVX1-15:1, Inc-REC8-2:1, OIP5-AS1:36, MYLK-AS1:13, Inc-PCP4L1-3:2, Inc-CGREF1-2:1, ZC3H12B-11:1, PSMB8-AS1:14, Inc-STX10-2:1, Inc-TCFL5-6:1, Inc-UQCC3-2:1, Inc-ZNF516-14:1, Inc-PDGFA-6:2, Inc-FOXD4L5-16:1, Inc-SAMD11-12:4, Inc-ADAD1-3:1, Inc-SLC35E3-8:2, Inc-NID1-4:4, FTX:19, TCONS_00017372, Inc-GRAP-1:2).

In some embodiments, the IncRNA signature comprises or consist of at least one IncRNA selected from a IncRNA signature selected from IncRNA signatures 1 to 42. In some embodiments, the IncRNA signature is or comprises a IncRNA signature selected from IncRNA signatures 1 to 42. In a preferred embodiment, the IncRNA signature is or comprises IncRNA signature 1.

The expression level of IncRNAs may be determined by any technology known by a man skilled in the art. In particular, the expression level of IncRNAs is determined by measuring the amount of nucleic acid transcripts of each IncRNAs. The amount of nucleic acid transcripts can be measured by any technology known by a man skilled in the art. The measure may be carried out directly on an extracted RNA sample or on retrotranscribed complementary DNA (cDNA) prepared from extracted RNA by technologies well-known in the art. From the RNA or cDNA sample, the amount of nucleic acid transcripts may be measured using any technology known by a man skilled in the art, including nucleic acid microarrays, quantitative PCR, sequencing (e.g., next generation sequencing), FIMAP quantification, and hybridization with a labeled probe.

In some embodiments, the expression level of IncRNAs is determined using sequencing, e.g., next generation sequencing. Sequencing may be carried out after converting extracted RNA to cDNA using reverse transcriptase or RNA molecules may be directly sequenced. In a particular embodiment, which should not be considered as limiting the scope of the invention, the measurement of the expression level using next generation sequencing may be performed as follows. Briefly, RNA is extracted from a sample (e.g., blood sample). After removing rRNA, RNA samples are then reverse transcribed into cDNA. To ensure strand specificity, single stranded cDNA is first synthetized using Super-Script II reverse transcriptase and random primers in the presence of Actinomycin D, and then converted to double stranded cDNA with the second strand marking mix that incorporates dUTP in place of dTTP. Resulting blunt ended cDNA are purified using AMPure XP magnetic beads. After a 3'end adenylation step, adaptor is attached to cDNA. So obtained cDNA (sequencing library) may be amplified by PCR. The sequencing libraries can be sequenced by any next generation sequencing technology known by a man skilled in the art.

In some embodiments, the measurement of the expression level of IncRNAs, e.g., by sequencing (e.g., next generation sequencing), is facilitated by capturing and enriching nucleic acids (RNA or cDNA) corresponding to ncRNAs of interest prior to the measurement. As used herein, enrichment refers to increasing the percentage of the nucleic acids of interest in the sample relative to the initial sample by selectively purifying the nucleic acids of interest. The enrichment of nucleic acids corresponding to IncRNAs of interest can be carried out on extracted RNA sample or cDNA sample prepared from extracted RNA. In some embodiments, nucleic acids corresponding to IncRNAs of interest are captured and enriched by hybridizing RNA or cDNA sample to oligonucleotide probes specific for IncRNAs of interest (e.g. oligonucleotide probes comprising a sequence complementary to a region of IncRNAs of interest) under conditions allowing for hybridization of the probes and target nucleic acids to form probe-target nucleic acid complexes. Probes may be DNA or RNA, preferably DNA. The length of probes specific for IncRNAs may be from 30 to 80 nucleotides, e.g., from 40 to 70, from 40 to 60, or about 50 nucleotides.The probe-target nucleic acid complexes can be purified by any technology known by a man skilled in the art. In a preferred embodiment, probes are biotinylated. The biotinylated probe-target nucleic acid complexes can be purified by using a streptavidin-coated substrate, e.g, a streptavidin-coated magnetic particle, e.g., T1 streptavidin coated magnetic bead.

In some embodiments, the expression level of IncRNAs may be determined using quantitative PCR. Quantitative, or real-time, PCR is a well known and easily available technology for those skilled in the art and does not need a precise description. In a particular embodiment, which should not be considered as limiting the scope of the invention, the determination of the expression profile using quantitative PCR may be performed as follows. Briefly, the real-time PCR reactions are carried out using the TaqMan Universal PCR Master Mix (Applied Biosystems). 6 µl cDNA is added to a 9 µl PCR mixture containing 7.5 µl TaqMan Universal PCR Master Mix, 0.75 µl of a 20X mixture of probe and primers and 0.75µl water. The reaction consists of one initiating step of 2 min at 50 deg. C, followed by 10 min at 95 deg. C, and 40 cycles of amplification including 15 sec at 95 deg. C and 1 min at 60 deg. C. The reaction and data acquisition can be performed using the ABI 7900HT Fast Real-Time PCR System (Applied Biosystems). The number of template transcript molecules in a sample is determined by recording the amplification cycle in the exponential phase (cycle threshold or C_{Q} or C_{T}), at which time the fluorescence signal can be detected above background fluorescence. Thus, the starting number of template transcript molecules is inversely related to Cr.

In some embodiments, the expression level of IncRNAs may be determined by the use of a nucleic acid microarray. A nucleic acid microarray consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"). To determine the expression profile of a target nucleic acid sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art.

In some embodiments, the expression level of IncRNAs may be determined by FIMAP quantification. Briefly, IncRNAs are amplified by PCR using primers with the same sequences as for qPCR that are chemically modified. Forward primers are phosphorylated in 5' and Reverse primers biotinylated in 5'. PCR products are digested with exonuclease to eliminate the phosphorylated strand and keep only the biotinylated ones. Biotinylated PCR products are incubated with coded silica microdisks coated with oligos complementary to the RNAs of interest (one code per target oligo), and hybridized products are revealed by addition of a fluorescent streptavidin conjugate. The expression level of IncRNAs is determined by measuring fluorescence signal.

In a still further aspect, the method of detection contemplates the use of a reagent to measure or detect the one or more IncRNA. By "reagent" is meant a reagent that specifically allows the determination of the IncRNA expression profile, i.e. a reagent specifically intended for the specific determination of the expression level of the IncRNA present in the IncRNA expression profile. Examples include e.g. amplification primer pairs (forward and reward) and/or or the IncRNA present in the IncRNA expression profile. This definition excludes generic reagents useful for the determination of the expression level of any other IncRNA.

In yet a further aspect, the method of detection comprises detecting one or more IncRNA from a biological sample, wherein the biological sample is selected from blood, plasma, serum, urine, cerebrospinal fluid, tear, saliva. In some embodiments, the biological sample is blood.

By "treating" or "treatment" of a subject being at risk to develop or having a cognitive disorder, particularly a progressive impairment or loss of cognitive function or dementia, e.g. AD, FTD, DLB, is meant administering or administration of a regimen to the subject in need thereof such that at least one symptom of the disorder or disease is cured, alleviated, remedied or improved. According to the present invention, depending on the diagnosing results, the subject is submitted to an adapted therapeutic treatment and is further monitored. The monitoring of the subject based on the IncRNA signature of the invention allows to further adapt or modify the therapeutic treatment, e.g. increasing the drug amount, administering a combination of drugs to obtain a synergy effect, or replacing the drug by an effective amount of another drug.

The invention also relates to the modification of a therapeutic strategy in subjects suffering from brain disorder such as a cognitive disorder who have been diagnosed and/or monitored using a method for (in vitro) diagnosis or monitoring of a progressive brain function such as impairment or loss of cognitive function or dementia according to the invention. According to the invention, the IncRNAs of the invention may be used in combination with one or more biomarkers currently used for diagnosing a cognitive disorder including but not limited to MCI, AD, FTD, DLB. Examples of such biomarkers include without any limitation the biomarkers as disclosed in USP 9,377,472, USP 7,897,786 and in USP 6,703,212, PCT/EP2018/073905/PCT/EP2018/073905), the contents thereof being included herein by reference. The method of the invention combining the IncRNAs of the invention with one or more known biomarkers may allow enhanced accuracy of diagnosis; or differential diagnosis; and a more efficient and successful drug development, e.g. enhanced accuracy for patient stratification before recruitment in clinical trials and monitoring of the efficacy of approved therapies or novel drugs under development.

According to the invention, the IncRNAs of the invention may be used in combination with one or more tests currently used for diagnosing a cognitive disorder including but not limited to AD, FTD, DLB and/or for differential diagnostic purposes. Examples of such tests include without any limitation the neuropsychological tests, such as MMSE, MoCA, FCSRT tests, and the neuroimaging methods, in particular volumetric MRI and molecular neuroimaging PET methods using a ligand specific to amyloid, Tau, alpha-synucelin or other molecules to visualize in the brain a proteinopathy . The method of the invention combining the IncRNAs of the invention with one or more known biomarkers may allow enhanced accuracy of diagnosis; or differential diagnosis; and a more efficient and successful drug development, e.g. enhanced accuracy for patient stratification before recruitment in clinical trials and monitoring of the efficacy of approved therapies or novel drugs under development.

According to the invention, novel therapeutic approaches consisting in normalizing the expression and/or amount of the IncRNAs identified for the first time by the invention in the brain may be used as treatment (alone or in combination with existing and novel other therapies) of brain disorders characterized by an altered expression or amount of such novel IncRNAs identified by the invention.

According to the invention, the identified brain-enriched IncRNAs, the identified circulating IncRNAs in the plasma, serum, blood or another body fluid may be used for the design and development of novel therapeutic treatment and for diagnosis applications for brain disorders characterized by an altered expression or amount of such circulating IncRNAs identified by the invention.

According to the invention, the altered IncRNAs identified by the invention as differentially expressed in a brain disorder such as a cognitive disorder (MCI, AD, DLB and/or FTD) in the brain or in peripheral body fluids may be used for the design and development of novel therapeutic treatment and for diagnosis applications for brain disorders characterized by an altered expression or amount of such IncRNAs identified by the invention as differentially expressed in the brain or peripheral body fluids.

To identify IncRNA signatures involved specifically in the pathogenesis of AD and mild cognitive impairment (MCI) of AD type, a total of 127802 IncRNAs in samples from different groups, including a group of patients with AD or MCI of AD type and group of cognitively intact healthy controls with no brain imaging abnormalities, have been screened. Subsequently IncRNA profiling in all samples, 19867 IncRNAs have been detected above the threshold. By comparison of IncRNA-expression levels measured in the samples of the different groups, the IncRNA differentially expressed in the samples of the control group as compared to the expression level in the samples of patient group diagnosed as having AD or cognitive impairment of AD type (at the Neurology department of clinical sites, based on neurocognitive and neuropsychological tests and neuroimaging tests and on cerebrospinal fluid biomarkers: beta-amyloid peptide 1-42 (Aβ42) and tau (total and/or phosphorylated) were identified as IncRNA biomarker candidates.

Expression levels were analyzed using a two-tailed Welch t test and/or Wilcoxon Mann-Whitney test between two groups. Significant differential expression was identified as p<0.05. Fold change and AUC (Area Under the Curve) are calculated for each IncRNA and for each tested condition.

Serum IncRNA candidates were also selected when differential expression was determined based on fold-changes ≥ 1.6 (or ≤ 0.6) and/or an AUC ≥ 0.80 (or <0.20), are indicated in Tables 2 and 3.

Random Forest algorithm (Breimann 2001, Breiman and Cutler 2001) was used to build the model and also used to select the top IncRNAs. A predictive model based on the combination of the top 2-20 IncRNAs enables to predict the disease with an accuracy of ≥ 80%.

Out of the 127802 serum IncRNAs sequenced, 19867 IncRNAs were selected based on their threshold expression level for statistical analysis. The comparison of the AD patient with healthy control populations showed that 1008 IncRNAs are differentially expressed with a statistical significance (p value <0.05, Wilcoxon test) (Table 1). The sequences of these 1008 IncRNAs are shown in the sequence listing included in this application.

**Table 1: The Sequence number, p value, mean +/- SD, fold change and AUC of the 1008 serum IncRNAs with differential expression in AD group versus healthy control group (p value <0.05, Wilcoxon test)**

| **IncRNA** | **SEQ** | **p value** | **Fold change** | **AUC** | **IncRNA** | **SEQ** | **p value** | **Fold change** | **AUC** |
|---|---|---|---|---|---|---|---|---|---|
| ADAMTS9-AS2:12 | SEQ0397 | 0,02049 | 0,852 | 0,778 | Inc-LMX1A-2:1 | SEQ0708 | 0,03324 | 0,786 | 0,757 |
| ADNP-AS1:12 | SEQ0340 | 0,01727 | 0,809 | 0,785 | Inc-LONP2-6:10 | SEQ0280 | 0,01209 | 0,912 | 0,799 |
| ADPGK-AS1:7 | SEQ0858 | 0,0449 | 1,225 | 0,257 | Inc-LRCH1-1:1 | SEQ0437 | 0,02049 | 0,793 | 0,778 |
| APTR:17 | SEQ0260 | 0,01209 | 1,147 | 0,201 | Inc-LRP12-4:3 | SEQ0939 | 0, 0449 | 1,165 | 0,257 |
| ARF4-AS1:8 | SEQ0859 | 0, 0449 | 1,143 | 0,257 | Inc-LRP5L-2:14 | SEQ0940 | 0, 0449 | 1,203 | 0,257 |
| ARRDC3-AS1:7 | SEQ0050 | 0,00232 | 0,441 | 0,854 | Inc-LRR1-1:2 | SEQ0813 | 0,03872 | 0,729 | 0,75 |
| BISPR:24 | SEQ0566 | 0,02842 | 0,579 | 0,764 | Inc-LRR1-1:3 | SEQ0814 | 0,03872 | 0,725 | 0,75 |
| BLACAT1:3 | SEQ0300 | 0,01449 | 1,717 | 0,208 | Inc-LRRC1-5:2 | SEQ0087 | 0,00364 | 0,649 | 0,84 |
| BLACAT1:5 | SEQ0341 | 0,01727 | 0,424 | 0,785 | Inc-LRRC3B-1:3 | SEQ0941 | 0, 0449 | 0,463 | 0,743 |
| CALML3-AS1:9 | SEQ0136 | 0,00681 | 1,567 | 0,181 | Inc-LRRC41-3:2 | SEQ0942 | 0, 0449 | 1,272 | 0,257 |
| CASC15:51 | SEQ0567 | 0,02842 | 1,273 | 0,236 | Inc-LRRC4C-7:1 | SEQ0815 | 0,03872 | 0,818 | 0,75 |
| CASC20:2 | SEQ0860 | 0, 0449 | 0,765 | 0,743 | Inc-LRRFIP2-3:4 | SEQ0617 | 0,02842 | 0,74 | 0,764 |
| CERS3-AS1:5 | SEQ0301 | 0,01449 | 1,251 | 0,208 | Inc-LRRK1-3:4 | SEQ0242 | 0,01004 | 0,79 | 0,806 |
| CFAP58-AS1:4 | SEQ0109 | 0,00556 | 1,464 | 0,174 | Inc-LRRK2-1:10 | SEQ0369 | 0,01727 | 0,86 | 0,785 |
| CLRN1-AS1:1 | SEQ0475 | 0,02418 | 0,793 | 0,771 | Inc-LRRK2-1:9 | SEQ0370 | 0,01727 | 0,86 | 0,785 |
| CPB2-AS1:18 | SEQ0476 | 0,02418 | 0,682 | 0,771 | Inc-LRRTM4-3:2 | SEQ0618 | 0,02842 | 0,865 | 0,764 |
| CYTOR:18 | SEQ0062 | 0,00291 | 1,278 | 0,153 | Inc-LY9-3:1 | SEQ0207 | 0,00829 | 1,317 | 0,188 |
| DARS-AS1:47 | SEQ0178 | 0,00829 | 2,015 | 0,188 | Inc-LYN-8:1 | SEQ0619 | 0,02842 | 1,516 | 0,236 |
| DDX11-AS1:5 | SEQ0861 | 0, 0449 | 1,256 | 0,257 | Inc-MAFB-1:4 | SEQ0525 | 0,02418 | 0,839 | 0,771 |
| DLEU2:26 | SEQ0862 | 0, 0449 | 1,076 | 0,257 | Inc-MAMDC2-1:1 | SEQ0526 | 0,02418 | 0,828 | 0,771 |
| DLEU2:45 | SEQ0863 | 0, 0449 | 0,882 | 0,743 | Inc-MAML3-2:1 | SEQ0438 | 0,02049 | 0,768 | 0,778 |
| DLGAP2-AS1:18 | SEQ0656 | 0,03324 | 0,789 | 0,757 | Inc-MAN1A1-1:3 | SEQ0158 | 0,00681 | 0,722 | 0,819 |
| DPH6-AS1:3 | SEQ0342 | 0,01727 | 2,004 | 0,215 | Inc-MAP9-6:1 | SEQ0281 | 0,01209 | 0,741 | 0,799 |
| EGFR-AS1:4 | SEQ0093 | 0,00451 | 0,81 | 0,833 | Inc-MARCH4-2:7 | SEQ0088 | 0,00364 | 2 | 0,16 |
| EGOT:11 | SEQ0110 | 0,00556 | 1,195 | 0,174 | Inc-MARCKS-1:9 | SEQ0527 | 0,02418 | 0,717 | 0,771 |
| EIF3J-AS1:21 | SEQ0261 | 0,01209 | 0,87 | 0,799 | Inc-MASTL-2:1 | SEQ0943 | 0, 0449 | 0,756 | 0,743 |
| ERICH3-AS1:4 | SEQ0864 | 0, 0449 | 0,888 | 0,743 | Inc-MB-3:1 | SEQ0944 | 0, 0449 | 1,302 | 0,257 |
| EXTL3-AS1:16 | SEQ0568 | 0,02842 | 0,739 | 0,764 | Inc-MBP-16:2 | SEQ0208 | 0,00829 | 1,204 | 0,188 |
| FAM66B:14 | SEQ0063 | 0,00291 | 0,604 | 0,847 | Inc-MC5R-5:1 | SEQ0945 | 0, 0449 | 0,742 | 0,743 |
| FLG-AS1:14 | SEQ0752 | 0,03872 | 1,18 | 0,25 | Inc-MC5R-6:2 | SEQ0243 | 0,01004 | 0,626 | 0,806 |
| FLVCR1-AS1:13 | SEQ0137 | 0,00681 | 0,413 | 0,819 | Inc-MDM4-8:1 | SEQ0816 | 0,03872 | 0,791 | 0,75 |
| FRG1-DT:6 | SEQ0865 | 0, 0449 | 0,873 | 0,743 | Inc-ME3-1:1 | SEQ0946 | 0, 0449 | 1,353 | 0,257 |
| GAS1RR:11 | SEQ0302 | 0,01449 | 0,848 | 0,792 | Inc-MED10-23:1 | SEQ0209 | 0,00829 | 1,295 | 0,188 |
| GAS6-AS2:9 | SEQ0303 | 0,01449 | 0,787 | 0,792 | Inc-MED15-1:2 | SEQ0620 | 0,02842 | 1,191 | 0,236 |
| GPC5-AS1:7 | SEQ0753 | 0,03872 | 0,623 | 0,75 | Inc-MESD-6:1 | SEQ0947 | 0, 0449 | 0,762 | 0,743 |
| GPR158-AS1:1 | SEQ0398 | 0,02049 | 0,775 | 0,778 | Inc-MEST-6:1 | SEQ0124 | 0,00556 | 0,817 | 0,826 |
| GRM5-AS1:1 | SEQ0138 | 0,00681 | 0,83 | 0,819 | Inc-METTL22-11:1 | SEQ0709 | 0,03324 | 0,747 | 0,757 |
| HAND2-AS1:58 | SEQ0094 | 0,00451 | 0,587 | 0,833 | Inc-MFSD8-6:3 | SEQ0528 | 0,02418 | 0,845 | 0,771 |
| HAND2-AS1:59 | SEQ0111 | 0,00556 | 0,592 | 0,826 | Inc-MFSD8-6:8 | SEQ0371 | 0,01727 | 0,794 | 0,785 |
| HAND2-AS1:70 | SEQ0039 | 0,00183 | 0,543 | 0,861 | Inc-MGST3-1:3 | SEQ0043 | 0,00183 | 1,664 | 0,139 |
| HAND2-AS1:71 | SEQ0076 | 0,00364 | 0,565 | 0,84 | Inc-MIB1-1:1 | SEQ0159 | 0,00681 | 0,68 | 0,819 |
| HCG23:5 | SEQ0754 | 0,03872 | 0,813 | 0,75 | Inc-MNX1-10:1 | SEQ0322 | 0,01449 | 1,204 | 0,208 |
| KAZN-AS1:4 | SEQ0399 | 0,02049 | 0,732 | 0,778 | Inc-MOGAT1-3:2 | SEQ0948 | 0, 0449 | 0,703 | 0,743 |
| KCNQ1-AS1:3 | SEQ0569 | 0,02842 | 0,848 | 0,764 | Inc-MPLKIP-3:1 | SEQ0710 | 0,03324 | 0,867 | 0,757 |
| KCNQ1OT1:8 | SEQ0139 | 0,00681 | 0,908 | 0,819 | Inc-MPP4-3:1 | SEQ0817 | 0,03872 | 0,813 | 0,75 |
| LACTB2-AS1:1 | SEQ0755 | 0,03872 | 1,206 | 0,25 | Inc-MRC2-2:1 | SEQ0818 | 0,03872 | 0,835 | 0,75 |
| LINC00158:5 | SEQ0570 | 0,02842 | 0,748 | 0,764 | Inc-MRGPRO-2: 1 | SEQ0244 | 0,01004 | 0,79 | 0,806 |
| LINC00200:6 | SEQ0064 | 0,00291 | 1,29 | 0,153 | Inc-MRGPRF-4:4 | SEQ0105 | 0,00451 | 0,604 | 0,833 |
| LINC00276:2 | SEQ0866 | 0, 0449 | 0,846 | 0,743 | Inc-MROH7-2:1 | SEQ0529 | 0,02418 | 0,844 | 0,771 |
| LINC00354:4 | SEQ0867 | 0, 0449 | 1,284 | 0,257 | Inc-MRPL57-5:8 | SEQ0621 | 0,02842 | 0,855 | 0,764 |
| LINC00458:19 | SEQ0571 | 0,02842 | 0,789 | 0,764 | Inc-MRPS30-13:1 | SEQ0819 | 0,03872 | 0,814 | 0,75 |
| LINC00460:13 | SEQ0868 | 0, 0449 | 0,752 | 0,743 | Inc-MSH2-3:2 | SEQ0711 | 0,03324 | 1,401 | 0,243 |
| LINC00472:21 | SEQ0140 | 0,00681 | 1,389 | 0,181 | Inc-MTRNR2L1-3:1 | SEQ0530 | 0,02418 | 1,325 | 0,229 |
| LINC00554:2 | SEQ0756 | 0,03872 | 0,766 | 0,75 | Inc-MVB12B-6:1 | SEQ0531 | 0,02418 | 0,811 | 0,771 |
| LINC00554:4 | SEQ0757 | 0,03872 | 0,766 | 0,75 | Inc-MYC-12:1 | SEQ0622 | 0,02842 | 1,418 | 0,236 |
| LINC00574:13 | SEQ0112 | 0,00556 | 0,771 | 0,826 | Inc-MYO18B-2:3 | SEQ0323 | 0,01449 | 0,833 | 0,792 |
| LINC00589:3 | SEQ0869 | 0, 0449 | 0,781 | 0,743 | Inc-MYO18B-3:3 | SEQ0160 | 0,00681 | 0,762 | 0,819 |
| LINC00649:23 | SEQ0077 | 0,00364 | 0,619 | 0,84 | Inc-MYOCOS-2:1 | SEQ0439 | 0,02049 | 0,891 | 0,778 |
| LINC00698:2 | SEQ0657 | 0,03324 | 0,85 | 0,757 | Inc-NAA38-3:1 | SEQ0532 | 0,02418 | 0,854 | 0,771 |
| LINC00707:9 | SEQ0572 | 0,02842 | 1,304 | 0,236 | lnc-NAALADL2-8:1 | SEQ0324 | 0,01449 | 0,864 | 0,792 |
| LINC00839:18 | SEQ0113 | 0,00556 | 0,874 | 0,826 | Inc-NANOS1-3:1 | SEQ0712 | 0,03324 | 1,277 | 0,243 |
| LINC00882:70 | SEQ0028 | 0,00111 | 0,867 | 0,875 | Inc-NAXD-6:5 | SEQ0125 | 0,00556 | 0,479 | 0,826 |
| LINC00882:71 | SEQ0029 | 0,00111 | 0,867 | 0,875 | Inc-NBPF14-1:2 | SEQ0089 | 0,00364 | 0,815 | 0,84 |
| LINC00887:15 | SEQ0870 | 0, 0449 | 0,925 | 0,743 | Inc-NBPF14-3:1 | SEQ0623 | 0,02842 | 0,889 | 0,764 |
| LINC00895:3 | SEQ0179 | 0,00829 | 1,491 | 0,188 | Inc-NCR3LG1-3:1 | SEQ0018 | 0,00066 | 0,837 | 0,889 |
| LINC00927:21 | SEQ0658 | 0,03324 | 0,619 | 0,757 | Inc-NDFIP2-7:13 | SEQ0820 | 0,03872 | 0,775 | 0,75 |
| LINC00927:22 | SEQ0659 | 0,03324 | 0,619 | 0,757 | Inc-NDFIP2-7:14 | SEQ0440 | 0,02049 | 0,77 | 0,778 |
| LINC00927:24 | SEQ0343 | 0,01727 | 0,772 | 0,785 | Inc-NORG2-5:1 | SEQ0949 | 0, 0449 | 0,773 | 0,743 |
| LINC00938:6 | SEQ0262 | 0,01209 | 0,811 | 0,799 | Inc-NDUFA10-6:1 | SEQ0161 | 0,00681 | 0,712 | 0,819 |
| LINC00963:67 | SEQ0573 | 0,02842 | 1,379 | 0,236 | Inc-NDUFB9-2:2 | SEQ0713 | 0,03324 | 1,345 | 0,243 |
| LINC01050:6 | SEQ0114 | 0,00556 | 0,82 | 0,826 | Inc-NDUFS6-15:1 | SEQ0950 | 0, 0449 | 0,805 | 0,743 |
| LINC01058:3 | SEQ0871 | 0, 0449 | 1,225 | 0,257 | Inc-NEUROD2-4:1 | SEQ0071 | 0,00291 | 0,772 | 0,847 |
| LINC01087:1 | SEQ0141 | 0,00681 | 0,764 | 0,819 | Inc-NKAIN2-5:1 | SEQ0821 | 0,03872 | 0,794 | 0,75 |
| LINC01088:18 | SEQ0660 | 0,03324 | 0,757 | 0,757 | Inc-NKX6-1-2:1 | SEQ0282 | 0,01209 | 0,773 | 0,799 |
| LINC01107:1 | SEQ0304 | 0,01449 | 0,768 | 0,792 | Inc-NOC2L-1:21 | SEQ0245 | 0,01004 | 1,382 | 0,194 |
| LINC01122:27 | SEQ0574 | 0,02842 | 1,263 | 0,236 | Inc-NOC2L-12:1 | SEQ0372 | 0,01727 | 1,479 | 0,215 |
| LINC01146:14 | SEQ0758 | 0,03872 | 0,774 | 0,75 | Inc-NOS2-7:1 | SEQ0951 | 0, 0449 | 1,203 | 0,257 |
| LINC01153:1 | SEQ0661 | 0,03324 | 1,169 | 0,243 | Inc-NPBWR1-2:2 | SEQ0952 | 0, 0449 | 1,517 | 0,257 |
| LINC01185:7 | SEQ0575 | 0,02842 | 0,793 | 0,764 | Inc-NPIPB12-1:1 | SEQ0822 | 0,03872 | 1,13 | 0,25 |
| LINC01197:26 | SEQ0759 | 0,03872 | 0,847 | 0,75 | Inc-NPY5R-4:1 | SEQ0823 | 0,03872 | 0,817 | 0,75 |
| LINC01206:11 | SEQ0662 | 0,03324 | 0,612 | 0,757 | Inc-NR2E1-4:4 | SEQ0714 | 0,03324 | 0,688 | 0,757 |
| LINC01206:20 | SEQ0400 | 0,02049 | 1,94 | 0,222 | Inc-NR5A2-1:13 | SEQ0441 | 0,02049 | 0,908 | 0,778 |
| LINC01226:8 | SEQ0663 | 0,03324 | 0, 902 | 0,757 | Inc-NRP1-4:1 | SEQ0953 | 0,0449 | 1,258 | 0,257 |
| LINC01270:15 | SEQ0477 | 0,02418 | 1,703 | 0,229 | Inc-NUB1-4:1 | SEQ0126 | 0,00556 | 0,809 | 0,826 |
| LINC01320:22 | SEQ0576 | 0,02842 | 0,785 | 0,764 | Inc-NUP35-4:2 | SEQ0715 | 0,03324 | 0,858 | 0,757 |
| LINC01322:20 | SEQ0223 | 0,01004 | 0,561 | 0,806 | Inc-NUTM1-14:4 | SEQ0716 | 0,03324 | 0,638 | 0,757 |
| LINC01355:9 | SEQ0664 | 0,03324 | 0,854 | 0,757 | Inc-NUTM2B-3:1 | SEQ0533 | 0,02418 | 0,801 | 0,771 |
| LINC01376:10 | SEQ0760 | 0,03872 | 0,934 | 0,75 | Inc-NUTM2E-1:5 | SEQ0127 | 0,00556 | 0,849 | 0,826 |
| LINC01394:15 | SEQ0401 | 0,02049 | 1,238 | 0,222 | Inc-ODF1-5:2 | SEQ0325 | 0,01449 | 0,847 | 0,792 |
| LINC01394:26 | SEQ0402 | 0,02049 | 1,238 | 0,222 | Inc-OOF1-9:1 | SEQ0824 | 0,03872 | 0,806 | 0,75 |
| LINC01394:50 | SEQ0095 | 0,00451 | 0,797 | 0,833 | Inc-OPRK1-2:1 | SEQ0717 | 0,03324 | 0,826 | 0,757 |
| LINC01410:11 | SEQ0023 | 0,00086 | 0,734 | 0,882 | Inc-OR10AD1-1:1 | SEQ0624 | 0,02842 | 0,826 | 0,764 |
| LINC01419:6 | SEQ0872 | 0, 0449 | 0,663 | 0,743 | Inc-OR13C4-8:1 | SEQ0625 | 0,02842 | 0,806 | 0,764 |
| LINC01426:10 | SEQ0577 | 0,02842 | 0,871 | 0,764 | Inc-OR4F21-7:9 | SEQ0283 | 0,01209 | 0,761 | 0,799 |
| LINC01481:8 | SEQ0478 | 0,02418 | 0,9 | 0,771 | Inc-OR4F29-3:11 | SEQ0044 | 0,00183 | 0,85 | 0,861 |
| LINC01519:3 | SEQ0305 | 0,01449 | 0,805 | 0,792 | Inc-OR4F29-8:4 | SEQ0210 | 0,00829 | 0,633 | 0,813 |
| LINC01527:1 | SEQ0578 | 0,02842 | 0,781 | 0,764 | Inc-OR5H2-1:2 | SEQ0326 | 0,01449 | 0,768 | 0,792 |
| LINC01568:12 | SEQ0479 | 0,02418 | 0,68 | 0,771 | lnc-OR6S1-2:1 | SEQ0373 | 0,01727 | 0,857 | 0,785 |
| LINC01629:11 | SEQ0579 | 0,02842 | 0,59 | 0,764 | Inc-OR8G5-7:2 | SEQ0626 | 0,02842 | 0,763 | 0,764 |
| LINC01653:2 | SEQ0580 | 0,02842 | 0,792 | 0,764 | Inc-ORAOV1-3:3 | SEQ0442 | 0,02049 | 0,895 | 0,778 |
| LINC01684:16 | SEQ0480 | 0,02418 | 0,907 | 0,771 | Inc-OSBPL6-1:1 | SEQ0443 | 0,02049 | 1,366 | 0,222 |
| LINC01697:9 | SEQ0873 | 0, 0449 | 0,874 | 0,743 | Inc-OSBPL7-3:3 | SEQ0534 | 0,02418 | 0,855 | 0,771 |
| LINC01719:19 | SEQ0761 | 0,03872 | 0,855 | 0,75 | Inc-OSBPL7-3:4 | SEQ0535 | 0,02418 | 0,855 | 0,771 |
| LINC01748:17 | SEQ0481 | 0,02418 | 0,791 | 0,771 | Inc-OSBPL7-3:6 | SEQ0536 | 0,02418 | 0,855 | 0,771 |
| LINC01762:4 | SEQ0874 | 0, 0449 | 0, 941 | 0,743 | Inc-OSBPL7-5:1 | SEQ0162 | 0,00681 | 1,685 | 0,181 |
| LINC01793:2 | SEQ0344 | 0,01727 | 0,743 | 0,785 | Inc-OST4-6:2 | SEQ0954 | 0, 0449 | 0,774 | 0,743 |
| LINC01798:4 | SEQ0482 | 0,02418 | 0,791 | 0,771 | Inc-OXGR1-6:2 | SEQ0374 | 0,01727 | 1,987 | 0,215 |
| LINC01845:3 | SEQ0115 | 0,00556 | 1,55 | 0,174 | Inc-OXGR1-6:3 | SEQ0163 | 0,00681 | 0,861 | 0,819 |
| LINC01856:2 | SEQ0762 | 0,03872 | 1,176 | 0,25 | Inc-OXR1-1:1 | SEQ0627 | 0,02842 | 0,752 | 0,764 |
| LINC01903:2 | SEQ0345 | 0,01727 | 0,774 | 0,785 | Inc-P2RY2-11:1 | SEQ0537 | 0,02418 | 0,86 | 0,771 |
| LINC01918:13 | SEQ0763 | 0,03872 | 1,138 | 0,25 | Inc-PABPC1-3:1 | SEQ0444 | 0,02049 | 0,814 | 0,778 |
| LINC02104:7 | SEQ0306 | 0,01449 | 0,82 | 0,792 | Inc-PABPC4L-21:3 | SEQ0375 | 0,01727 | 1,485 | 0,215 |
| LINC02177:6 | SEQ0403 | 0,02049 | 0,768 | 0,778 | Inc-PABPC4L-5:7 | SEQ0284 | 0,01209 | 0,626 | 0,799 |
| LINC02177:8 | SEQ0764 | 0,03872 | 0,494 | 0,75 | Inc-PACRGL-5:1 | SEQ0718 | 0,03324 | 0,64 | 0,757 |
| LINC02197:6 | SEQ0483 | 0,02418 | 0,76 | 0,771 | Inc-PAGR1-2:4 | SEQ0719 | 0,03324 | 1,176 | 0,243 |
| LINC02204:2 | SEQ0142 | 0,00681 | 0,78 | 0,819 | Inc-PALLD-3: 1 | SEQ0164 | 0,00681 | 0,741 | 0,819 |
| LINC02204:3 | SEQ0143 | 0,00681 | 0,78 | 0,819 | Inc-PAN3-3:1 | SEQ0955 | 0, 0449 | 0,758 | 0,743 |
| LINC02232:10 | SEQ0665 | 0,03324 | 0,646 | 0,757 | Inc-PANK1-7:1 | SEQ0211 | 0,00829 | 0,723 | 0,813 |
| LINC02246:11 | SEQ0346 | 0,01727 | 0,74 | 0,785 | Inc-PAPD7-2:3 | SEQ0956 | 0, 0449 | 0,786 | 0,743 |
| LINC02252:3 | SEQ0765 | 0,03872 | 0,881 | 0,75 | Inc-PAPPA-1:3 | SEQ0720 | 0,03324 | 2,192 | 0,243 |
| LINC02323:8 | SEQ0307 | 0,01449 | 0,622 | 0,792 | Inc-PAPPA-1:4 | SEQ0721 | 0,03324 | 0,814 | 0,757 |
| LINC02334:6 | SEQ0581 | 0,02842 | 0,737 | 0,764 | Inc-PAPPA2-1:10 | SEQ0246 | 0,01004 | 0,753 | 0,806 |
| LINC02345:11 | SEQ0013 | 0,00066 | 1,181 | 0,111 | Inc-PAPPA2-7:1 | SEQ0165 | 0,00681 | 0,839 | 0,819 |
| LINC02432:9 | SEQ0180 | 0,00829 | 0,776 | 0,813 | Inc-PATE2-1:1 | SEQ0247 | 0,01004 | 0,728 | 0,806 |
| LINC02473:3 | SEQ0065 | 0,00291 | 0,786 | 0,847 | Inc-PAX8-6:2 | SEQ0538 | 0,02418 | 1,413 | 0,229 |
| LINC02519:7 | SEQ0875 | 0,0449 | 0,682 | 0,743 | Inc-PAXIP1-8:1 | SEQ0957 | 0,0449 | 0,731 | 0,743 |
| LINC02554:5 | SEQ0404 | 0,02049 | 0,744 | 0,778 | Inc-PCDH10-11:1 | SEQ0958 | 0, 0449 | 0,719 | 0,743 |
| LINC02580:5 | SEQ0484 | 0,02418 | 0,767 | 0,771 | Inc-PCDH8-12:1 | SEQ0166 | 0,00681 | 0,742 | 0,819 |
| Inc-AASDHPPT-3:1 | SEQ0409 | 0,02049 | 0,715 | 0,778 | Inc-PCOLCE2-1:1 | SEQ0959 | 0, 0449 | 0,909 | 0,743 |
| Inc-ABCA1-8:9 | SEQ0589 | 0,02842 | 0, 592 | 0,764 | Inc-PCSK9-4:6 | SEQ0327 | 0,01449 | 1,154 | 0,208 |
| Inc-ABCA5-14:2 | SEQ0881 | 0, 0449 | 1,164 | 0,257 | Inc-PCSK9-4:9 | SEQ0328 | 0,01449 | 1,154 | 0,208 |
| Inc-ABCA5-7:1 | SEQ0014 | 0,00066 | 1,309 | 0,111 | Inc-PDLIM1-3:1 | SEQ0960 | 0, 0449 | 0,837 | 0,743 |
| Inc-ABCG2-3:5 | SEQ0667 | 0,03324 | 0,758 | 0,757 | Inc-PFKP-16:15 | SEQ0628 | 0,02842 | 0,662 | 0,764 |
| Inc-ACO1-1:1 | SEQ0590 | 0,02842 | 0,759 | 0,764 | Inc-PFKP-17:1 | SEQ0445 | 0,02049 | 0,834 | 0,778 |
| Inc-ACOT12-9:1 | SEQ0053 | 0,00232 | 0,727 | 0,854 | Inc-PHF14-14:19 | SEQ0825 | 0,03872 | 0,678 | 0,75 |
| Inc-ACTL7B-8:1 | SEQ0493 | 0,02418 | 0,697 | 0,771 | Inc-PIGB-1:5 | SEQ0539 | 0,02418 | 0,691 | 0,771 |
| Inc-ADAD1-3:1 | SEQ0774 | 0,03872 | 0,828 | 0,75 | Inc-PIGM-4:1 | SEQ0248 | 0,01004 | 0,715 | 0,806 |
| Inc-ADAMTS20-3:1 | SEQ0494 | 0,02418 | 1,219 | 0,229 | Inc-PINX1-7:1 | SEQ0329 | 0,01449 | 1,307 | 0,208 |
| Inc-ADAMTS5-1:1 | SEQ0882 | 0, 0449 | 0,851 | 0,743 | Inc-PLA2G2F-1:2 | SEQ0285 | 0,01209 | 1,224 | 0,201 |
| Inc-ADAT1-1:1 | SEQ0495 | 0,02418 | 0,877 | 0,771 | Inc-PLA2G4A-7:5 | SEQ0446 | 0,02049 | 0,865 | 0,778 |
| Inc-ADRA2A-4:1 | SEQ0883 | 0, 0449 | 0,729 | 0,743 | Inc-PLA2G4A-7:8 | SEQ0826 | 0,03872 | 0,74 | 0,75 |
| Inc-AORB1-4:1 | SEQ0118 | 0,00556 | 0,858 | 0,826 | Inc-PLAT-1:3 | SEQ0961 | 0, 0449 | 0,738 | 0,743 |
| Inc-AFG1L-5:1 | SEQ0263 | 0,01209 | 1,407 | 0,201 | Inc-PLCB1-7:2 | SEQ0827 | 0,03872 | 0,854 | 0,75 |
| Inc-AGO2-2:2 | SEQ0119 | 0,00556 | 0,511 | 0,826 | Inc-PLEKHA8-3:5 | SEQ0962 | 0, 0449 | 0,793 | 0,743 |
| Inc-AGO2-2:3 | SEQ0668 | 0,03324 | 1,442 | 0,243 | Inc-PLK1-1:6 | SEQ0963 | 0, 0449 | 0,663 | 0,743 |
| Inc-AGR3-6:1 | SEQ0884 | 0, 0449 | 1,156 | 0,257 | Inc-PLN-2:1 | SEQ0722 | 0,03324 | 0,781 | 0,757 |
| Inc-AHR-5:1 | SEQ0264 | 0,01209 | 0,792 | 0,799 | Inc-PLSCR2-2:1 | SEQ0964 | 0, 0449 | 0,813 | 0,743 |
| Inc-AIG1-5:1 | SEQ0496 | 0,02418 | 0,862 | 0,771 | Inc-POC5-3:1 | SEQ0629 | 0,02842 | 0,747 | 0,764 |
| Inc-AKAP9-1:2 | SEQ0775 | 0,03872 | 0,704 | 0,75 | Inc-POLE4-3:1 | SEQ0330 | 0,01449 | 0,798 | 0,792 |
| Inc-AKIRIN1-1:11 | SEQ0591 | 0,02842 | 0, 553 | 0,764 | Inc-POU2AF1-1:2 | SEQ0447 | 0,02049 | 0,874 | 0,778 |
| Inc-AKR1 C2-3:17 | SEQ0097 | 0,00451 | 0,768 | 0,833 | Inc-PPIAL4F-3:2 | SEQ0828 | 0,03872 | 1,302 | 0,25 |
| Inc-AKR1 D1-5:2 | SEQ0098 | 0,00451 | 0,891 | 0,833 | Inc-PPM1D-1:8 | SEQ0540 | 0,02418 | 0,8 | 0,771 |
| Inc-AKR1 D1-8:3 | SEQ0885 | 0, 0449 | 0,797 | 0,743 | Inc-PPP2R3C-4:1 | SEQ0025 | 0,00086 | 0,879 | 0,882 |
| Inc-AKR1 E2-15:1 | SEQ0410 | 0,02049 | 1,303 | 0,222 | Inc-PPP5C-4:1 | SEQ0723 | 0,03324 | 0,757 | 0,757 |
| Inc-AKR7 A2-2: 1 | SEQ0145 | 0,00681 | 1,339 | 0,181 | Inc-PROM9-19:2 | SEQ0829 | 0,03872 | 0,709 | 0,75 |
| Inc-AKT1-1:15 | SEQ0411 | 0,02049 | 0,825 | 0,778 | Inc-PROM9-20: 1 | SEQ0376 | 0,01727 | 0,678 | 0,785 |
| Inc-ALB-1:12 | SEQ0146 | 0,00681 | 1,448 | 0,181 | Inc-PRELID2-1:2 | SEQ0541 | 0,02418 | 1,232 | 0,229 |
| Inc-ALB-1:6 | SEQ0886 | 0, 0449 | 0,881 | 0,743 | Inc-PRKACG-1:2 | SEQ0724 | 0,03324 | 0,738 | 0,757 |
| Inc-ALDH3B2-3:4 | SEQ0412 | 0,02049 | 0,89 | 0,778 | Inc-PRKACG-2:1 | SEQ0630 | 0,02842 | 1,186 | 0,236 |
| Inc-ALG14-5:2 | SEQ0350 | 0,01727 | 0,697 | 0,785 | Inc-PRKCH-1:1 | SEQ0286 | 0,01209 | 0,904 | 0,799 |
| Inc-ALS2CR12-1:2 | SEQ0351 | 0,01727 | 0,729 | 0,785 | Inc-PRKN-8:1 | SEQ0212 | 0,00829 | 1,368 | 0,188 |
| Inc-ANAPC11-2:6 | SEQ0887 | 0,0449 | 1,836 | 0,257 | Inc-PRND-2:1 | SEQ0287 | 0,01209 | 0,798 | 0,799 |
| Inc-ANKRD1-1:6 | SEQ0888 | 0, 0449 | 0,77 | 0,743 | Inc-PRR11-1:4 | SEQ0288 | 0,01209 | 0,862 | 0,799 |
| Inc-ANKRD26-1:3 | SEQ0497 | 0,02418 | 0,852 | 0,771 | Inc-PRR5-5:1 | SEQ0167 | 0,00681 | 0,703 | 0,819 |
| Inc-ANKRD30BL-2:2 | SEQ0413 | 0,02049 | 0,747 | 0,778 | Inc-PRSS54-2:2 | SEQ0168 | 0,00681 | 0,769 | 0,819 |
| Inc-ANKRD46-1:3 | SEQ0669 | 0,03324 | 0,687 | 0,757 | Inc-PSMB1-6:4 | SEQ0331 | 0,01449 | 2,134 | 0,208 |
| Inc-ANXA3-8:1 | SEQ0670 | 0,03324 | 0,838 | 0,757 | Inc-PTDSS1-1:2 | SEQ0448 | 0,02049 | 1,246 | 0,222 |
| Inc-APBA1-5:1 | SEQ0592 | 0,02842 | 0,781 | 0,764 | Inc-PTP4A2-1:2 | SEQ0725 | 0,03324 | 1,223 | 0,243 |
| Inc-APIP-1:13 | SEQ0776 | 0,03872 | 0,807 | 0,75 | Inc-PTPN14-11:1 | SEQ0449 | 0,02049 | 1,341 | 0,222 |
| Inc-APLP2-4:1 | SEQ0054 | 0,00232 | 0,786 | 0,854 | Inc-PTPN4-1:1 | SEQ0631 | 0,02842 | 0,755 | 0,764 |
| Inc-APOB-1:2 | SEQ0777 | 0,03872 | 0,583 | 0,75 | Inc-QRFP-5:1 | SEQ0019 | 0,00066 | 2,17 | 0,111 |
| Inc-APPL2-1:2 | SEQ0498 | 0,02418 | 1,133 | 0,229 | Inc-RAB3B-1:1 | SEQ0965 | 0, 0449 | 0,843 | 0,743 |
| Inc-AQP8-2:7 | SEQ0352 | 0,01727 | 1,254 | 0,215 | Inc-RAB6C-3:1 | SEQ0966 | 0, 0449 | 0,836 | 0,743 |
| Inc-ARAP2-9:1 | SEQ0147 | 0,00681 | 0,795 | 0,819 | Inc-RAI14-3:1 | SEQ0542 | 0,02418 | 0,835 | 0,771 |
| Inc-ARHGAP15-17:1 | SEQ0353 | 0,01727 | 0,857 | 0,785 | Inc-RALGAPA1-1:2 | SEQ0726 | 0,03324 | 0,86 | 0,757 |
| Inc-ARHGAP15-22:1 | SEQ0593 | 0,02842 | 0,846 | 0,764 | Inc-RALGAPA2-2:4 | SEQ0967 | 0, 0449 | 0,805 | 0,743 |
| Inc-ARHGAP21-1:2 | SEQ0309 | 0,01449 | 0,723 | 0,792 | Inc-RARRES1-3:2 | SEQ0632 | 0,02842 | 0,84 | 0,764 |
| Inc-ARHGAP26-4:11 | SEQ0189 | 0,00829 | 0,923 | 0,813 | Inc-RASGRP1-3:3 | SEQ0968 | 0, 0449 | 0,796 | 0,743 |
| Inc-ARHGAP26-4:33 | SEQ0778 | 0,03872 | 0,71 | 0,75 | Inc-RBFOX1-2:1 | SEQ0377 | 0,01727 | 1,269 | 0,215 |
| Inc-ARHGAP26-4:39 | SEQ0889 | 0, 0449 | 1,383 | 0,257 | Inc-RBKS-6:1 | SEQ0128 | 0,00556 | 0,802 | 0,826 |
| Inc-ARHGEF26-2:1 | SEQ0067 | 0,00291 | 1,399 | 0,153 | Inc-RBM11-11:1 | SEQ0450 | 0,02049 | 0,714 | 0,778 |
| Inc-ARHGEF5-5:1 | SEQ0354 | 0,01727 | 0,712 | 0,785 | Inc-RBM25-1:1 | SEQ0969 | 0, 0449 | 0,89 | 0,743 |
| I n c-ARI D2-7:1 | SEQ0671 | 0,03324 | 0,924 | 0,757 | Inc-RBM33-3:1 | SEQ0727 | 0,03324 | 0,762 | 0,757 |
| Inc-ARNTL-2:1 | SEQ0265 | 0,01209 | 0,61 | 0,799 | Inc-RBM45-7:1 | SEQ0830 | 0,03872 | 0,792 | 0,75 |
| Inc-ARROC4-7: 1 | SEQ0779 | 0,03872 | 0,876 | 0,75 | Inc-RBMS1-7:1 | SEQ0249 | 0,01004 | 0,778 | 0,806 |
| Inc-ART5-2:1 | SEQ0414 | 0,02049 | 0,787 | 0,778 | Inc-RCSD1-4:1 | SEQ0831 | 0,03872 | 0,831 | 0,75 |
| lnc-ATAD1-5:2 | SEQ0148 | 0,00681 | 0,738 | 0,819 | Inc-RDH13-1:2 | SEQ0250 | 0,01004 | 0,635 | 0,806 |
| Inc-ATIC-2:8 | SEQ0780 | 0,03872 | 0, 554 | 0,75 | Inc-RGMA-28:2 | SEQ0378 | 0,01727 | 0,875 | 0,785 |
| Inc-ATP12A-3:1 | SEQ0499 | 0,02418 | 0,763 | 0,771 | Inc-RGS9-15:6 | SEQ0970 | 0, 0449 | 0,705 | 0,743 |
| Inc-ATP1 3A4-2:4 | SEQ0672 | 0,03324 | 1,64 | 0,243 | Inc-RHNO1-1:1 | SEQ0026 | 0,00086 | 1,248 | 0,118 |
| Inc-ATP5O-3:1 | SEQ0224 | 0,01004 | 1,358 | 0,194 | Inc-RHOB-1:3 | SEQ0451 | 0,02049 | 0,612 | 0,778 |
| Inc-ATP6V0E2-7:1 | SEQ0500 | 0,02418 | 1,361 | 0,229 | Inc-RHOB-21:1 | SEQ0971 | 0, 0449 | 0,817 | 0,743 |
| Inc-ATP6V1B2-2:6 | SEQ0594 | 0,02842 | 1,132 | 0,236 | Inc-RHOBTB2-4:1 | SEQ0213 | 0,00829 | 0,839 | 0,813 |
| Inc-ATP6V1B2-2:7 | SEQ0595 | 0,02842 | 1,132 | 0,236 | Inc-RIPPLY3-1:3 | SEQ0832 | 0,03872 | 0,889 | 0,75 |
| Inc-ATP8A2-1:1 | SEQ0355 | 0,01727 | 1,477 | 0,215 | Inc-RIT2-5:1 | SEQ0289 | 0,01209 | 1,302 | 0,201 |
| Inc-ATXN2-1:1 | SEQ0099 | 0,00451 | 0,792 | 0,833 | Inc-RNF6-2:1 | SEQ0059 | 0,00232 | 0,692 | 0,854 |
| Inc-ATXN7-11:1 | SEQ0501 | 0,02418 | 1,319 | 0,229 | Inc-RNFT2-1:5 | SEQ0379 | 0,01727 | 0,8 | 0,785 |
| Inc-ATXN7L1-1:1 | SEQ0890 | 0,0449 | 1,32 | 0,257 | Inc-RNLS-1:1 | SEQ0972 | 0, 0449 | 0,829 | 0,743 |
| Inc-AUH-2:7 | SEQ0190 | 0,00829 | 0,584 | 0,813 | Inc-ROBO2-16:1 | SEQ0251 | 0,01004 | 0,799 | 0,806 |
| Inc-AUH-2:9 | SEQ0673 | 0,03324 | 1,35 | 0,243 | Inc-RPE65-4:2 | SEQ0129 | 0,00556 | 0,857 | 0,826 |
| Inc-AUH-4:1 | SEQ0596 | 0,02842 | 0,79 | 0,764 | Inc-RPIA-25:1 | SEQ0130 | 0,00556 | 0,866 | 0,826 |
| Inc-BAG3-4:4 | SEQ0891 | 0, 0449 | 1,314 | 0,257 | Inc-RPL10L-5:1 | SEQ0452 | 0,02049 | 0,77 | 0,778 |
| Inc-BARHL2-4:4 | SEQ0100 | 0,00451 | 0,736 | 0,833 | Inc-RPL24-6:1 | SEQ0728 | 0,03324 | 1,245 | 0,243 |
| Inc-BCHE-1:1 | SEQ0674 | 0,03324 | 0,741 | 0,757 | Inc-RPL35-2:1 | SEQ0543 | 0,02418 | 0,812 | 0,771 |
| Inc-BCL6-9:1 | SEQ0310 | 0,01449 | 0,885 | 0,792 | Inc-RPL37-2:1 | SEQ0214 | 0,00829 | 1,309 | 0,188 |
| Inc-BIRC2-5:5 | SEQ0225 | 0,01004 | 0,831 | 0,806 | Inc-RPRM-7:1 | SEQ0380 | 0,01727 | 0,679 | 0,785 |
| Inc-BIRC6-1:4 | SEQ0892 | 0, 0449 | 0,829 | 0,743 | Inc-RPS12-4:1 | SEQ0633 | 0,02842 | 0,806 | 0,764 |
| Inc-BMS1-2:1 | SEQ0675 | 0,03324 | 1,124 | 0,243 | Inc-RPS21-4:2 | SEQ0027 | 0,00086 | 0,664 | 0,882 |
| Inc-BNC2-5:1 | SEQ0191 | 0,00829 | 1,299 | 0,188 | Inc-RRM1-2:5 | SEQ0973 | 0, 0449 | 0, 934 | 0,743 |
| Inc-BORA-31:1 | SEQ0192 | 0,00829 | 0,67 | 0,813 | Inc-RSL1D1-2:1 | SEQ0215 | 0,00829 | 0,762 | 0,813 |
| Inc-BRD1-17:1 | SEQ0311 | 0,01449 | 0,669 | 0,792 | Inc-RTL1-3:9 | SEQ0544 | 0,02418 | 0,848 | 0,771 |
| Inc-BRINP1-3:1 | SEQ0893 | 0, 0449 | 0,894 | 0,743 | Inc-RUBCN-1:1 | SEQ0833 | 0,03872 | 0,788 | 0,75 |
| Inc-BRINP2-3:1 | SEQ0597 | 0,02842 | 0,878 | 0,764 | Inc-S1PR1-13:1 | SEQ0381 | 0,01727 | 0,686 | 0,785 |
| Inc-C10orf90-2:2 | SEQ0415 | 0,02049 | 1,687 | 0,222 | Inc-SAMD11-1:1 | SEQ0729 | 0,03324 | 1,247 | 0,243 |
| Inc-C12orf40-3:3 | SEQ0894 | 0, 0449 | 1,434 | 0,257 | Inc-SAMD5-1:10 | SEQ0090 | 0,00364 | 0,708 | 0,84 |
| Inc-C12orf42-3:6 | SEQ0598 | 0,02842 | 1,303 | 0,236 | Inc-SC5D-4:1 | SEQ0730 | 0,03324 | 1,249 | 0,243 |
| Inc-C15orf41-18:5 | SEQ0676 | 0,03324 | 1,649 | 0,243 | Inc-SCO-7: 1 | SEQ0974 | 0,0449 | 0,731 | 0,743 |
| Inc-C15orf41-18:6 | SEQ0895 | 0, 0449 | 1,561 | 0,257 | Inc-SCGB1C2-8:1 | SEQ0975 | 0, 0449 | 0,776 | 0,743 |
| Inc-C19orf57-1:1 | SEQ0896 | 0, 0449 | 1,361 | 0,257 | Inc-SCNN1B-3:1 | SEQ0976 | 0, 0449 | 1,155 | 0,257 |
| Inc-C1QTNF9-4:1 | SEQ0781 | 0,03872 | 1,282 | 0,25 | Inc-SCTR-2:4 | SEQ0634 | 0,02842 | 0,852 | 0,764 |
| Inc-C21orf58-1:2 | SEQ0033 | 0,00143 | 2,231 | 0,132 | Inc-SEPT14-6:1 | SEQ0545 | 0,02418 | 0,878 | 0,771 |
| Inc-C2CD4B-6:4 | SEQ0193 | 0,00829 | 1,39 | 0,188 | Inc-SERHL2-1:8 | SEQ0546 | 0,02418 | 1,483 | 0,229 |
| Inc-C2orf42-10:1 | SEQ0502 | 0,02418 | 1,28 | 0,229 | Inc-SERINC1-8:3 | SEQ0731 | 0,03324 | 0,827 | 0,757 |
| Inc-C3orf58-7:1 | SEQ0599 | 0,02842 | 0,725 | 0,764 | Inc-SERP1-4:6 | SEQ0072 | 0,00291 | 0,719 | 0,847 |
| Inc-C5orf30-10:1 | SEQ0266 | 0,01209 | 0,708 | 0,799 | Inc-SERP1-4:8 | SEQ0977 | 0, 0449 | 0,831 | 0,743 |
| Inc-C5orf30-10:2 | SEQ0600 | 0,02842 | 0,891 | 0,764 | Inc-SERPINI1-14:1 | SEQ0978 | 0, 0449 | 0,85 | 0,743 |
| Inc-C5orf67-3:1 | SEQ0149 | 0,00681 | 0,876 | 0,819 | Inc-SERTM1-1:1 | SEQ0290 | 0,01209 | 0,817 | 0,799 |
| Inc-C7orf57-4:1 | SEQ0356 | 0,01727 | 1,461 | 0,215 | Inc-SFPQ-2:1 | SEQ0169 | 0,00681 | 0,72 | 0,819 |
| Inc-C9orf3-5:1 | SEQ0120 | 0,00556 | 0,782 | 0,826 | Inc-SGCG-7:2 | SEQ0453 | 0,02049 | 0,733 | 0,778 |
| Inc-CA7-2:2 | SEQ0677 | 0,03324 | 0,817 | 0,757 | Inc-SGK1-3:14 | SEQ0979 | 0, 0449 | 1,836 | 0,257 |
| Inc-CAB39L-1:4 | SEQ0267 | 0,01209 | 0,769 | 0,799 | Inc-SGMS1-4:1 | SEQ0834 | 0,03872 | 0,793 | 0,75 |
| Inc-CAB39L-4:2 | SEQ0601 | 0,02842 | 1,181 | 0,236 | Inc-SH3BGRL2-4:1 | SEQ0980 | 0, 0449 | 1,174 | 0,257 |
| Inc-CACNA1I-1:1 | SEQ0503 | 0,02418 | 0,813 | 0,771 | Inc-SKIL-2:3 | SEQ0981 | 0, 0449 | 1,222 | 0,257 |
| Inc-CACNA2D1-1:1 | SEQ0897 | 0, 0449 | 0,82 | 0,743 | Inc-SLC1A3-1:1 | SEQ0547 | 0,02418 | 0,791 | 0,771 |
| Inc-CACNG1-1:1 | SEQ0602 | 0,02842 | 0,652 | 0,764 | Inc-SLC22A23-11:2 | SEQ0732 | 0,03324 | 1,239 | 0,243 |
| Inc-CALML6-1:10 | SEQ0898 | 0, 0449 | 0,577 | 0,743 | Inc-SLC25A21-1:1 | SEQ0733 | 0,03324 | 0,77 | 0,757 |
| Inc-CAPS2-1:1 | SEQ0357 | 0,01727 | 0,708 | 0,785 | Inc-SLC25A24-2:1 | SEQ0548 | 0,02418 | 0,883 | 0,771 |
| Inc-CASC10-3:1 | SEQ0899 | 0, 0449 | 0,744 | 0,743 | Inc-SLC25A30-2:4 | SEQ0291 | 0,01209 | 1,539 | 0,201 |
| Inc-CASP9-1:1 | SEQ0782 | 0,03872 | 1,246 | 0,25 | Inc-SLC2A 10-1: 1 | SEQ0549 | 0,02418 | 0,788 | 0,771 |
| Inc-CAVIN2-2:1 | SEQ0416 | 0,02049 | 1,425 | 0,222 | Inc-SLC38A2-1:11 | SEQ0252 | 0,01004 | 0,892 | 0,806 |
| Inc-CBLB-9:1 | SEQ0900 | 0, 0449 | 0,788 | 0,743 | Inc-SLC38A2-1:15 | SEQ0332 | 0,01449 | 1,425 | 0,208 |
| Inc-CCDC102B-7:1 | SEQ0901 | 0, 0449 | 0,816 | 0,743 | Inc-SLC39A11-10:11 | SEQ0292 | 0,01209 | 4,38 | 0,201 |
| Inc-CCDC167-5:1 | SEQ0417 | 0,02049 | 1,25 | 0,222 | Inc-SLC46A3-7:1 | SEQ0550 | 0,02418 | 0,858 | 0,771 |
| Inc-CCDC177-6:1 | SEQ0783 | 0,03872 | 1,157 | 0,25 | Inc-SLCO6A1-2:1 | SEQ0635 | 0,02842 | 0,775 | 0,764 |
| Inc-CCDC192-3:1 | SEQ0902 | 0,0449 | 0,8 | 0,743 | Inc-SLITRK5-17:1 | SEQ0293 | 0,01209 | 0,636 | 0,799 |
| Inc-CCDC197-2:1 | SEQ0068 | 0,00291 | 0,672 | 0,847 | Inc-SLTM-1:2 | SEQ0982 | 0, 0449 | 0,9 | 0,743 |
| Inc-CCDC61-4:1 | SEQ0194 | 0,00829 | 0,561 | 0,813 | Inc-SMARCA5-4:18 | SEQ0253 | 0,01004 | 0,825 | 0,806 |
| Inc-CCOC7-17:1 | SEQ0226 | 0,01004 | 1,341 | 0,194 | Inc-SMIM14-4:1 | SEQ0454 | 0,02049 | 0,788 | 0,778 |
| Inc-CCOC93-10:2 | SEQ0603 | 0,02842 | 0,87 | 0,764 | Inc-SMIM17-5:4 | SEQ0734 | 0,03324 | 0,715 | 0,757 |
| Inc-CCL1-10:1 | SEQ0227 | 0,01004 | 1,307 | 0,194 | Inc-SNAPC3-12:5 | SEQ0455 | 0,02049 | 1,387 | 0,222 |
| Inc-CCNB1IP1-1:2 | SEQ0903 | 0, 0449 | 0,512 | 0,743 | Inc-SNCA-3:1 | SEQ0551 | 0,02418 | 0,498 | 0,771 |
| Inc-CCR8-2:1 | SEQ0904 | 0, 0449 | 1,409 | 0,257 | Inc-SNRPB2-2:4 | SEQ0254 | 0,01004 | 1,727 | 0,194 |
| Inc-CCSER1-2:1 | SEQ0678 | 0,03324 | 0,791 | 0,757 | Inc-SNX10-6:1 | SEQ0636 | 0,02842 | 0,764 | 0,764 |
| Inc-CCT8L2-28:1 | SEQ0418 | 0,02049 | 0,812 | 0,778 | Inc-SNX13-2:6 | SEQ0456 | 0,02049 | 1,576 | 0,222 |
| Inc-CD47-11:2 | SEQ0905 | 0,0449 | 1,317 | 0,257 | Inc-SNX16-6:1 | SEQ0983 | 0,0449 | 0,83 | 0,743 |
| Inc-CD47-11:4 | SEQ0906 | 0,0449 | 1,139 | 0,257 | Inc-SNX17-1:13 | SEQ0333 | 0,01449 | 1,985 | 0,208 |
| lnc-CDADC1-1:1 | SEQ0907 | 0,0449 | 1,18 | 0,257 | Inc-SNX17-1:8 | SEQ0984 | 0, 0449 | 0,754 | 0,743 |
| Inc-CDH23-2:1 | SEQ0908 | 0, 0449 | 0,757 | 0,743 | Inc-SNX19-6:1 | SEQ0835 | 0,03872 | 1,249 | 0,25 |
| Inc-CDK20-14:1 | SEQ0358 | 0,01727 | 1,274 | 0,215 | Inc-SNX19-9:1 | SEQ0836 | 0,03872 | 0,837 | 0,75 |
| Inc-CDK2AP1-1:8 | SEQ0909 | 0, 0449 | 0,84 | 0,743 | Inc-SNX20-8:5 | SEQ0985 | 0,0449 | 0,933 | 0,743 |
| Inc-CEACAM16-2:1 | SEQ0195 | 0,00829 | 0,708 | 0,813 | Inc-SOX11-5:1 | SEQ0382 | 0,01727 | 1,216 | 0,215 |
| Inc-CEBPD-11:2 | SEQ0784 | 0,03872 | 0,763 | 0,75 | Inc-SOX14-2:1 | SEQ0045 | 0,00183 | 0,837 | 0,861 |
| Inc-CELF4-15:1 | SEQ0785 | 0,03872 | 0,89 | 0,75 | Inc-SPAG9-2:1 | SEQ0383 | 0,01727 | 0,851 | 0,785 |
| Inc-CELSR1-2:3 | SEQ0268 | 0,01209 | 0,762 | 0,799 | Inc-SPAG9-2:2 | SEQ0384 | 0,01727 | 0,851 | 0,785 |
| Inc-CEP170-9:2 | SEQ0042 | 0,00183 | 0,828 | 0,861 | Inc-SPATA31A6-10:1 | SEQ0837 | 0,03872 | 0,804 | 0,75 |
| lnc-CFAP36-3:2 | SEQ0786 | 0,03872 | 0,69 | 0,75 | Inc-SPATA31D4-1:6 | SEQ0838 | 0,03872 | 0,718 | 0,75 |
| Inc-CHD1L-5:13 | SEQ0312 | 0,01449 | 0,37 | 0,792 | Inc-SPP1-1:1 | SEQ0046 | 0,00183 | 0,846 | 0,861 |
| Inc-CHMP2B-1:11 | SEQ0679 | 0,03324 | 0,858 | 0,757 | Inc-SPRY1-9:1 | SEQ0839 | 0,03872 | 0,767 | 0,75 |
| Inc-CHN1-5:11 | SEQ0419 | 0,02049 | 0,739 | 0,778 | Inc-SPTSSA-5:2 | SEQ0552 | 0,02418 | 1,31 | 0,229 |
| Inc-CHRAC1-1:1 | SEQ0680 | 0,03324 | 0,747 | 0,757 | Inc-SRCIN1-1:18 | SEQ0553 | 0,02418 | 0,798 | 0,771 |
| Inc-CHRAC1-6:1 | SEQ0420 | 0,02049 | 1,247 | 0,222 | Inc-SRSF2-2:5 | SEQ0735 | 0,03324 | 0,633 | 0,757 |
| Inc-CHRM2-1:1 | SEQ0121 | 0,00556 | 0,833 | 0,826 | Inc-ST8SIA4-3:5 | SEQ0457 | 0,02049 | 1,584 | 0,222 |
| Inc-CHRM3-1:5 | SEQ0681 | 0,03324 | 0,714 | 0,757 | Inc-STARD10-1:6 | SEQ0840 | 0,03872 | 1,156 | 0,25 |
| Inc-CHST2-6:2 | SEQ0196 | 0,00829 | 0,763 | 0,813 | Inc-STAT1-2:3 | SEQ0986 | 0, 0449 | 0,832 | 0,743 |
| Inc-CLON 10-5:1 | SEQ0313 | 0,01449 | 0,717 | 0,792 | Inc-STK32B-2:1 | SEQ0170 | 0,00681 | 0,807 | 0,819 |
| Inc-CLEC19A-3:1 | SEQ0787 | 0,03872 | 1,284 | 0,25 | Inc-STOML3-6:1 | SEQ0038 | 0,00143 | 0,731 | 0,868 |
| Inc-CLK1-1:7 | SEQ0197 | 0,00829 | 0,602 | 0,813 | Inc-STPG1-1:1 | SEQ0637 | 0,02842 | 0,82 | 0,764 |
| Inc-CLVS2-2:5 | SEQ0359 | 0,01727 | 0,818 | 0,785 | Inc-STRADB-6:2 | SEQ0987 | 0, 0449 | 0,723 | 0,743 |
| Inc-CMPK2-34:4 | SEQ0101 | 0,00451 | 0,651 | 0,833 | Inc-SUCLA2-13:2 | SEQ0736 | 0,03324 | 1,22 | 0,243 |
| Inc-CMTM7-2:2 | SEQ0269 | 0,01209 | 0,838 | 0,799 | Inc-SUCLA2-13:3 | SEQ0737 | 0,03324 | 1,22 | 0,243 |
| Inc-CMTR1-10:1 | SEQ0788 | 0,03872 | 0,868 | 0,75 | Inc-SUGT1-3:1 | SEQ0131 | 0,00556 | 2,047 | 0,174 |
| Inc-CNBD1-4:13 | SEQ0682 | 0,03324 | 0,568 | 0,757 | Inc-SULT1A4-1:27 | SEQ0841 | 0,03872 | 1,274 | 0,25 |
| Inc-CNOP1-7:1 | SEQ0024 | 0,00086 | 0, 534 | 0,882 | Inc-SULT1C2-3:1 | SEQ0842 | 0,03872 | 1,396 | 0,25 |
| Inc-CNOT6-10:1 | SEQ0270 | 0,01209 | 1,19 | 0,201 | Inc-SUSD1-1:5 | SEQ0638 | 0,02842 | 0,798 | 0,764 |
| Inc-COL6A6-2:1 | SEQ0910 | 0, 0449 | 0,717 | 0,743 | Inc-SYCP1-4:1 | SEQ0843 | 0,03872 | 1,204 | 0,25 |
| Inc-COMMD6-10:1 | SEQ0079 | 0,00364 | 1,58 | 0,16 | Inc-TAAR9-3:2 | SEQ0738 | 0,03324 | 0,806 | 0,757 |
| Inc-COX 10-9:2 | SEQ0911 | 0, 0449 | 0,849 | 0,743 | Inc-TAB2-1:4 | SEQ0988 | 0, 0449 | 0,846 | 0,743 |
| Inc-CPEB3-2:1 | SEQ0789 | 0,03872 | 0,78 | 0,75 | Inc-TACC2-8:6 | SEQ0171 | 0,00681 | 0,869 | 0,819 |
| Inc-CPM-2:11 | SEQ0912 | 0, 0449 | 0,424 | 0,743 | I n c-TACST D2-2:4 | SEQ0020 | 0,00066 | 0,757 | 0,889 |
| Inc-CPM-3:1 | SEQ0228 | 0,01004 | 0,831 | 0,806 | Inc-TADA2B-6:1 | SEQ0554 | 0,02418 | 1,467 | 0,229 |
| Inc-CRIPT-1:3 | SEQ0790 | 0,03872 | 0,802 | 0,75 | Inc-TAF9-10:1 | SEQ0639 | 0,02842 | 0,825 | 0,764 |
| Inc-CRISP1-1:2 | SEQ0271 | 0,01209 | 0,685 | 0,799 | Inc-TASP1-11:1 | SEQ0989 | 0, 0449 | 1,217 | 0,257 |
| Inc-CRYBA1-4:1 | SEQ0421 | 0,02049 | 0,693 | 0,778 | Inc-TBC1D22A-4:12 | SEQ0739 | 0,03324 | 0,969 | 0,757 |
| Inc-CRYBB1-1:1 | SEQ0055 | 0,00232 | 0,487 | 0,854 | Inc-TBC1 D3H-1:1 | SEQ0334 | 0,01449 | 0,729 | 0,792 |
| Inc-CSGALNACT2-2:3 | SEQ0791 | 0,03872 | 0,781 | 0,75 | Inc-TBC1D3H-3:1 | SEQ0294 | 0,01209 | 0,72 | 0,799 |
| Inc-CSNK1A1-6:1 | SEQ0030 | 0,00111 | 0,696 | 0,875 | Inc-TBL 1XR1-7:1 | SEQ0458 | 0,02049 | 0,754 | 0,778 |
| Inc-CTIF-9:2 | SEQ0504 | 0,02418 | 1,28 | 0,229 | Inc-TCEANC2-3:1 | SEQ0106 | 0,00451 | 0,549 | 0,833 |
| Inc-CTNNA2-3:11 | SEQ0198 | 0,00829 | 0,812 | 0,813 | Inc-TCEANC2-3:2 | SEQ0385 | 0,01727 | 1,882 | 0,215 |
| Inc-CTNNA3-1:2 | SEQ0683 | 0,03324 | 0,82 | 0,757 | Inc-TCF19-1:80 | SEQ0740 | 0,03324 | 1,351 | 0,243 |
| I n c-CTN N D2-3:1 | SEQ0913 | 0, 0449 | 0,797 | 0,743 | Inc-TCF7-1:3 | SEQ0990 | 0, 0449 | 0,866 | 0,743 |
| Inc-CTR9-7:1 | SEQ0914 | 0, 0449 | 0,763 | 0,743 | Inc-TCP10-2:1 | SEQ0991 | 0, 0449 | 0,876 | 0,743 |
| Inc-CYB5R2-3:13 | SEQ0199 | 0,00829 | 0,749 | 0,813 | Inc-TCP11-2:3 | SEQ0640 | 0,02842 | 0,67 | 0,764 |
| Inc-CYBA-4:3 | SEQ0150 | 0,00681 | 0,784 | 0,819 | Inc-TCP11L2-1:4 | SEQ0741 | 0,03324 | 0,74 | 0,757 |
| Inc-CYP2E1-1:1 | SEQ0684 | 0,03324 | 1,733 | 0,243 | Inc-TDO2-6:1 | SEQ0641 | 0,02842 | 0,758 | 0,764 |
| Inc-CYTIP-2:1 | SEQ0200 | 0,00829 | 0,727 | 0,813 | Inc-TOP2-1:1 | SEQ0335 | 0,01449 | 1,29 | 0,208 |
| Inc-OAO-3:1 | SEQ0685 | 0,03324 | 1,109 | 0,243 | Inc-TEAD4-1:1 | SEQ0992 | 0,0449 | 1,868 | 0,257 |
| Inc-DAPP1-2:11 | SEQ0505 | 0,02418 | 0,822 | 0,771 | Inc-TEFM-10:2 | SEQ0993 | 0,0449 | 0,736 | 0,743 |
| Inc-DAZAP2-3:1 | SEQ0056 | 0,00232 | 0,797 | 0,854 | Inc-TEKT3-3:1 | SEQ0844 | 0,03872 | 0,744 | 0,75 |
| Inc-DDX1-3:1 | SEQ0915 | 0,0449 | 1,251 | 0,257 | Inc-TEKT4-4:1 | SEQ0132 | 0,00556 | 1,332 | 0,174 |
| Inc-DDX18-1:1 | SEQ0506 | 0,02418 | 0,721 | 0,771 | Inc-TENM3-3:3 | SEQ0001 | 0,00007 | 0,717 | 0, 938 |
| Inc-DDX18-1:7 | SEQ0314 | 0,01449 | 0,755 | 0,792 | Inc-TENM3-3:4 | SEQ0008 | 0,00037 | 1,947 | 0,097 |
| Inc-DEFB112-3:4 | SEQ0422 | 0,02049 | 0,565 | 0,778 | Inc-TENM3-3:5 | SEQ0009 | 0,00037 | 1,993 | 0,097 |
| Inc-DEK-4:1 | SEQ0507 | 0,02418 | 0,715 | 0,771 | Inc-TENM4-4:1 | SEQ0742 | 0,03324 | 2,209 | 0,243 |
| Inc-DEPTOR-5:3 | SEQ0360 | 0,01727 | 0,846 | 0,785 | Inc-TEX10-1:1 | SEQ0459 | 0,02049 | 0,814 | 0,778 |
| Inc-OGCR2-5: 1 | SEQ0423 | 0,02049 | 0,872 | 0,778 | Inc-TEX29-3:1 | SEQ0172 | 0,00681 | 0,767 | 0,819 |
| Inc-DGCR6-7:26 | SEQ0792 | 0,03872 | 0,736 | 0,75 | Inc-TEX49-4:1 | SEQ0994 | 0,0449 | 0,789 | 0,743 |
| Inc-DHX37-18:1 | SEQ0229 | 0,01004 | 0,751 | 0,806 | Inc-TF-4:1 | SEQ0743 | 0,03324 | 0,898 | 0,757 |
| Inc-DHX38-25:1 | SEQ0230 | 0,01004 | 0,894 | 0,806 | Inc-TFCP2L1-6:1 | SEQ0460 | 0,02049 | 0,743 | 0,778 |
| Inc-DKK1-5:3 | SEQ0005 | 0,00027 | 0,739 | 0,91 | Inc-TGM6-2:1 | SEQ0216 | 0,00829 | 0,741 | 0,813 |
| Inc-DKK1-5:4 | SEQ0793 | 0,03872 | 0,893 | 0,75 | Inc-THAP12-1:5 | SEQ0642 | 0,02842 | 1,427 | 0,236 |
| Inc-DLG5-1:1 | SEQ0034 | 0,00143 | 0,773 | 0,868 | Inc-THOC5-3:1 | SEQ0133 | 0,00556 | 0,717 | 0,826 |
| Inc-DLX2-12:1 | SEQ0151 | 0,00681 | 0,747 | 0,819 | Inc-THY1-3:1 | SEQ0995 | 0, 0449 | 0,788 | 0,743 |
| Inc-DMRTA1-17:1 | SEQ0201 | 0,00829 | 0,79 | 0,813 | Inc-THYN1-1:1 | SEQ0461 | 0,02049 | 1,173 | 0,222 |
| Inc-DNAH9-1:1 | SEQ0508 | 0,02418 | 0,812 | 0,771 | Inc-TLDC2-4:1 | SEQ0555 | 0,02418 | 1,214 | 0,229 |
| Inc-DNALI1-5:4 | SEQ0102 | 0,00451 | 0,776 | 0,833 | Inc-TLE4-7:1 | SEQ0255 | 0,01004 | 1,184 | 0,194 |
| Inc-DOCK7-7:1 | SEQ0015 | 0,00066 | 0,843 | 0,889 | Inc-TLK1-1:2 | SEQ0556 | 0,02418 | 0,76 | 0,771 |
| Inc-DTWD2-14:1 | SEQ0361 | 0,01727 | 0,811 | 0,785 | Inc-TLNRD1-3:1 | SEQ0462 | 0,02049 | 0,829 | 0,778 |
| Inc-DUSP10-6:1 | SEQ0057 | 0,00232 | 0,675 | 0,854 | Inc-TMEM126B-2:3 | SEQ0336 | 0,01449 | 0,508 | 0,792 |
| Inc-DUSP26-3:2 | SEQ0031 | 0,00111 | 0,796 | 0,875 | Inc-TMEM126B-2:4 | SEQ0643 | 0,02842 | 1,964 | 0,236 |
| Inc-DYNAP-1:1 | SEQ0604 | 0,02842 | 0,761 | 0,764 | Inc-TMEM132C-6:5 | SEQ0996 | 0,0449 | 1,222 | 0,257 |
| Inc-EAF1-2:1 | SEQ0509 | 0,02418 | 0,737 | 0,771 | Inc-TMEM168-1:1 | SEQ0073 | 0,00291 | 0,761 | 0,847 |
| Inc-EBF3-1:6 | SEQ0686 | 0,03324 | 1,241 | 0,243 | Inc-TMEM185B-12:1 | SEQ0173 | 0,00681 | 0,549 | 0,819 |
| Inc-EBLN1-1:4 | SEQ0016 | 0,00066 | 0,725 | 0,889 | Inc-TMEM185B-12:7 | SEQ0060 | 0,00232 | 1,694 | 0,146 |
| Inc-EDDM13-5:11 | SEQ0231 | 0,01004 | 0,372 | 0,806 | Inc-TMEM185B-2:3 | SEQ0386 | 0,01727 | 1,212 | 0,215 |
| Inc-EDDM13-5:3 | SEQ0035 | 0,00143 | 1,505 | 0,132 | Inc-TMEM211-2:8 | SEQ0644 | 0,02842 | 1,278 | 0,236 |
| Inc-EDEM3-7:3 | SEQ0272 | 0,01209 | 0,86 | 0,799 | Inc-TMEM242-6:1 | SEQ0463 | 0,02049 | 0,879 | 0,778 |
| Inc-EORF1-1 :5 | SEQ0916 | 0, 0449 | 0,728 | 0,743 | Inc-TMEM248-4:11 | SEQ0845 | 0,03872 | 0,842 | 0,75 |
| Inc-EEF1AKMT1-3:6 | SEQ0202 | 0,00829 | 0,423 | 0,813 | Inc-TMEM70-7:1 | SEQ0217 | 0,00829 | 1,404 | 0,188 |
| Inc-EEF2-3:1 | SEQ0687 | 0,03324 | 1,301 | 0,243 | Inc-TMX4-3:1 | SEQ0464 | 0,02049 | 0,776 | 0,778 |
| Inc-EFR3B-7:2 | SEQ0362 | 0,01727 | 0,821 | 0,785 | Inc-TNFRSF19-2:1 | SEQ0744 | 0,03324 | 1,177 | 0,243 |
| Inc-EGFR-7:1 | SEQ0917 | 0,0449 | 1,181 | 0,257 | Inc-TNFSF4-3:3 | SEQ0337 | 0,01449 | 1,714 | 0,208 |
| Inc-EIF2AK3-31:7 | SEQ0918 | 0,0449 | 0,848 | 0,743 | Inc-TOGARAM2-1:5 | SEQ0645 | 0,02842 | 0,81 | 0,764 |
| Inc-EIF2AK3-4:81 | SEQ0688 | 0,03324 | 1,466 | 0,243 | Inc-TOGARAM2-1:6 | SEQ0646 | 0,02842 | 0,81 | 0,764 |
| Inc-ELF1-5:1 | SEQ0510 | 0,02418 | 0,799 | 0,771 | Inc-TP53TG3-65:1 | SEQ0465 | 0,02049 | 1,227 | 0,222 |
| Inc-ELFN2-1:3 | SEQ0080 | 0,00364 | 0,764 | 0,84 | Inc-TP53TG3D-2:1 | SEQ0846 | 0,03872 | 0,9 | 0,75 |
| Inc-EPB42-1:3 | SEQ0689 | 0,03324 | 0,917 | 0,757 | Inc-TP53TG3F-8:1 | SEQ0647 | 0,02842 | 1,33 | 0,236 |
| Inc-EPHA7-3:1 | SEQ0081 | 0,00364 | 0,763 | 0,84 | Inc-TPPP-1:2 | SEQ0002 | 0,00014 | 0,158 | 0,924 |
| Inc-ERCC6L2-10:2 | SEQ0605 | 0,02842 | 0,927 | 0,764 | Inc-TPPP-1:3 | SEQ0134 | 0,00556 | 1,834 | 0,174 |
| Inc-ERCC6L2-6:1 | SEQ0363 | 0,01727 | 1,784 | 0,215 | Inc-TRAM1-1:1 | SEQ0074 | 0,00291 | 0,59 | 0,847 |
| Inc-ERFE-1:1 | SEQ0919 | 0, 0449 | 0,489 | 0,743 | Inc-TRIB2-14:1 | SEQ0021 | 0,00066 | 0,788 | 0,889 |
| Inc-ERGIC2-2:2 | SEQ0364 | 0,01727 | 0,799 | 0,785 | Inc-TRIM13-2:1 | SEQ0174 | 0,00681 | 0,739 | 0,819 |
| Inc-ERH-1:1 | SEQ0424 | 0,02049 | 0,708 | 0,778 | Inc-TRIM26-2:33 | SEQ0745 | 0,03324 | 0,782 | 0,757 |
| Inc-ERICH1-9:1 | SEQ0920 | 0, 0449 | 0,85 | 0,743 | Inc-TRI M26-2:74 | SEQ0338 | 0,01449 | 1,231 | 0,208 |
| Inc-ERV3-1-9:1 | SEQ0203 | 0,00829 | 1,32 | 0,188 | Inc-TRIM27-10:2 | SEQ0107 | 0,00451 | 1,227 | 0,167 |
| Inc-ESRP1-2:4 | SEQ0315 | 0,01449 | 0, 542 | 0,792 | Inc-TRIM37-2:1 | SEQ0997 | 0,0449 | 1,221 | 0,257 |
| Inc-ETS1-2:2 | SEQ0082 | 0,00364 | 1,436 | 0,16 | Inc-TRIM43B-1:2 | SEQ0387 | 0,01727 | 0,8 | 0,785 |
| Inc-EXOC2-21:6 | SEQ0204 | 0,00829 | 1,528 | 0,188 | Inc-TRIM49D1-4:1 | SEQ0175 | 0,00681 | 1,36 | 0,181 |
| Inc-EZH2-3:1 | SEQ0273 | 0,01209 | 0,79 | 0,799 | Inc-TRIM77-7:1 | SEQ0847 | 0,03872 | 0,741 | 0,75 |
| Inc-F11-8:1 | SEQ0690 | 0,03324 | 0,876 | 0,757 | Inc-TRIML2-11:1 | SEQ0557 | 0,02418 | 1,296 | 0,229 |
| lnc-F13A1-2:7 | SEQ0691 | 0,03324 | 1,485 | 0,243 | Inc-TRMT11-4:1 | SEQ0218 | 0,00829 | 0,773 | 0,813 |
| Inc-FAM133B-2:1 | SEQ0036 | 0,00143 | 0,767 | 0,868 | Inc-TRPM1-3:1 | SEQ0388 | 0,01727 | 0,83 | 0,785 |
| Inc-FAM171B-1:6 | SEQ0205 | 0,00829 | 0,851 | 0,813 | Inc-TSC2202-1:4 | SEQ0466 | 0,02049 | 0,633 | 0,778 |
| Inc-FAM19A3-6:3 | SEQ0274 | 0,01209 | 0,75 | 0,799 | Inc-TSHB-2:3 | SEQ0295 | 0,01209 | 0,564 | 0,799 |
| Inc-FAM217A-1:2 | SEQ0032 | 0,00111 | 0,854 | 0,875 | Inc-TSHB-2:4 | SEQ0467 | 0,02049 | 1,766 | 0,222 |
| Inc-FAM231B-2:1 | SEQ0511 | 0,02418 | 1,252 | 0,229 | Inc-TSHB-2:5 | SEQ0468 | 0,02049 | 1,766 | 0,222 |
| Inc-FAM231B-2:2 | SEQ0512 | 0,02418 | 1,252 | 0,229 | Inc-TSHB-2:6 | SEQ0296 | 0,01209 | 0,564 | 0,799 |
| Inc-FAM236D-2:1 | SEQ0692 | 0,03324 | 0,601 | 0,757 | Inc-TSR3-1:2 | SEQ0339 | 0,01449 | 0,784 | 0,792 |
| Inc-FAM46C-3:1 | SEQ0794 | 0,03872 | 0,755 | 0,75 | Inc-TSTD2-4:3 | SEQ0848 | 0,03872 | 1,229 | 0,25 |
| Inc-FAM49B-8:1 | SEQ0010 | 0,0005 | 1,758 | 0,104 | Inc-TTC26-9:1 | SEQ0558 | 0,02418 | 0,686 | 0,771 |
| Inc-FAM71F2-5:1 | SEQ0425 | 0,02049 | 0,642 | 0,778 | Inc-TTC38-7:1 | SEQ0849 | 0,03872 | 1,169 | 0,25 |
| Inc-FAM72B-6:3 | SEQ0606 | 0,02842 | 1,327 | 0,236 | Inc-TTF2-4:1 | SEQ0998 | 0,0449 | 0,848 | 0,743 |
| Inc-FAM84A-5:1 | SEQ0607 | 0,02842 | 0,697 | 0,764 | Inc-TUBA1C-1:12 | SEQ0999 | 0,0449 | 0,731 | 0,743 |
| Inc-FAM84B-17:4 | SEQ0608 | 0,02842 | 0,741 | 0,764 | Inc-TUBE1-6:1 | SEQ0850 | 0,03872 | 1,141 | 0,25 |
| Inc-FAM84B-4:3 | SEQ0083 | 0,00364 | 0,803 | 0,84 | Inc-TUBGCP3-11:1 | SEQ0469 | 0,02049 | 0,885 | 0,778 |
| Inc-FAP-3:1 | SEQ0206 | 0,00829 | 0,749 | 0,813 | Inc-TUSC5-3:1 | SEQ1000 | 0,0449 | 1,345 | 0,257 |
| Inc-FARSB-6:1 | SEQ0232 | 0,01004 | 1,292 | 0,194 | Inc-TWSG1-2:1 | SEQ0047 | 0,00183 | 0,673 | 0,861 |
| Inc-FAT1-7:2 | SEQ0017 | 0,00066 | 1,383 | 0,111 | Inc-UBE2QL1-4:1 | SEQ0648 | 0,02842 | 0,864 | 0,764 |
| Inc-FAT4-6:1 | SEQ0609 | 0,02842 | 0,751 | 0,764 | Inc-UBE3C-10:1 | SEQ0075 | 0,00291 | 1,627 | 0,153 |
| Inc-FBRSL1-3:3 | SEQ0610 | 0,02842 | 1,645 | 0,236 | Inc-UBLCP1-2:6 | SEQ0048 | 0,00183 | 0,755 | 0,861 |
| Inc-FCGR3B-4:11 | SEQ0513 | 0,02418 | 0,848 | 0,771 | Inc-UCK1-2:1 | SEQ0851 | 0,03872 | 0,774 | 0,75 |
| Inc-FCGR3B-4:12 | SEQ0514 | 0,02418 | 0,848 | 0,771 | Inc-UGCG-1:1 | SEQ0219 | 0,00829 | 0,802 | 0,813 |
| Inc-FER1L6-2:1 | SEQ0515 | 0,02418 | 0,779 | 0,771 | Inc-UGT1A8-3:1 | SEQ0649 | 0,02842 | 1,428 | 0,236 |
| Inc-FGD4-8:1 | SEQ0233 | 0,01004 | 0,688 | 0,806 | Inc-UGT2B28-1:2 | SEQ1001 | 0, 0449 | 0,818 | 0,743 |
| Inc-FGD4-9:1 | SEQ0084 | 0,00364 | 0,711 | 0,84 | Inc-UGT2B28-2:1 | SEQ0559 | 0,02418 | 0,764 | 0,771 |
| Inc-FILIP1L-3:1 | SEQ0058 | 0,00232 | 0,867 | 0,854 | Inc-UGT3A2-3:1 | SEQ0389 | 0,01727 | 0,855 | 0,785 |
| Inc-FNBP1L-1:11 | SEQ0006 | 0,00027 | 0,743 | 0,91 | Inc-UNC93B1-1:5 | SEQ1002 | 0,0449 | 0,806 | 0,743 |
| Inc-FOXC1-6:2 | SEQ0516 | 0,02418 | 0,736 | 0,771 | Inc-UNCX-3:26 | SEQ0470 | 0,02049 | 0,493 | 0,778 |
| Inc-FOXD4L5-35:1 | SEQ0152 | 0,00681 | 1,878 | 0,181 | Inc-UPK3B-7:1 | SEQ0297 | 0,01209 | 0,607 | 0,799 |
| Inc-FOXO1-2:8 | SEQ0611 | 0,02842 | 1,402 | 0,236 | Inc-USP16-15:1 | SEQ0650 | 0,02842 | 0,811 | 0,764 |
| Inc-FRG2-13:3 | SEQ0275 | 0,01209 | 0,908 | 0,799 | Inc-USP16-9:3 | SEQ0091 | 0,00364 | 0,834 | 0,84 |
| Inc-FSIP1-6:4 | SEQ0795 | 0,03872 | 1,401 | 0,25 | Inc-USP17L7-1:1 | SEQ0746 | 0,03324 | 1,48 | 0,243 |
| Inc-FSIP2-2:1 | SEQ0921 | 0, 0449 | 0,76 | 0,743 | Inc-USP24-2:3 | SEQ0747 | 0,03324 | 0,694 | 0,757 |
| Inc-FTCD-5:1 | SEQ0796 | 0,03872 | 0,722 | 0,75 | Inc-USP31-2:3 | SEQ0022 | 0,00066 | 0,685 | 0,889 |
| Inc-FTMT-2:14 | SEQ0517 | 0,02418 | 0,792 | 0,771 | Inc-USP53-1:1 | SEQ0390 | 0,01727 | 0,824 | 0,785 |
| Inc-GALC-9:8 | SEQ0234 | 0,01004 | 0, 557 | 0,806 | Inc-USP6NL-13:1 | SEQ0748 | 0,03324 | 1,209 | 0,243 |
| Inc-GALNT2-1:1 | SEQ0426 | 0,02049 | 0,729 | 0,778 | Inc-VGLL3-11:1 | SEQ0220 | 0,00829 | 0,81 | 0,813 |
| Inc-GALNTL5-3:1 | SEQ0693 | 0,03324 | 0,759 | 0,757 | Inc-VPS8-2:4 | SEQ0852 | 0,03872 | 0,742 | 0,75 |
| Inc-GCLC-1:13 | SEQ0922 | 0, 0449 | 0, 597 | 0,743 | Inc-VSTM2B-5:12 | SEQ0560 | 0,02418 | 0,771 | 0,771 |
| Inc-GDPD5-6:1 | SEQ0153 | 0,00681 | 0,799 | 0,819 | Inc-VWA5B1-2:1 | SEQ0391 | 0,01727 | 1,479 | 0,215 |
| Inc-GFI1B-2:3 | SEQ0797 | 0,03872 | 0,681 | 0,75 | Inc-WDR4-2:6 | SEQ1003 | 0, 0449 | 0,929 | 0,743 |
| Inc-GGH-3:1 | SEQ0798 | 0,03872 | 0,782 | 0,75 | Inc-WDR63-6:2 | SEQ0471 | 0,02049 | 0,655 | 0,778 |
| Inc-GHR-1:1 | SEQ0003 | 0,0002 | 0,787 | 0,917 | Inc-WDR70-7:6 | SEQ1004 | 0,0449 | 0,929 | 0,743 |
| Inc-GJC1-2:2 | SEQ0799 | 0,03872 | 0,832 | 0,75 | Inc-WDR7-1 1:1 | SEQ0749 | 0,03324 | 1,151 | 0,243 |
| Inc-GJC1-2:3 | SEQ0800 | 0,03872 | 0,832 | 0,75 | Inc-WOYHV1-1:2 | SEQ0561 | 0,02418 | 0,912 | 0,771 |
| Inc-GLIPR1-3:2 | SEQ0923 | 0,0449 | 0,923 | 0,743 | Inc-WISP1-17:2 | SEQ0135 | 0,00556 | 0,715 | 0,826 |
| Inc-GLIPR1L1-2:3 | SEQ0427 | 0,02049 | 1,26 | 0,222 | Inc-WSB1-2:1 | SEQ0256 | 0,01004 | 1,293 | 0,194 |
| Inc-GMOS-6:8 | SEQ0694 | 0,03324 | 0,721 | 0,757 | Inc-XCL2-4:1 | SEQ1005 | 0,0449 | 0,714 | 0,743 |
| Inc-GNG5-8:1 | SEQ0695 | 0,03324 | 0,771 | 0,757 | Inc-XXYLT1-5:1 | SEQ0221 | 0,00829 | 0,681 | 0,813 |
| Inc-GOLGA4-4:7 | SEQ0365 | 0,01727 | 0,855 | 0,785 | Inc-YPEL5-5:1 | SEQ0750 | 0,03324 | 1,357 | 0,243 |
| Inc-GOLGA6L6-9:1 | SEQ0069 | 0,00291 | 0,711 | 0,847 | Inc-ZC3H12D-2:3 | SEQ0392 | 0,01727 | 0,831 | 0,785 |
| Inc-GOLGA8F-2:1 | SEQ0235 | 0,01004 | 0,756 | 0,806 | Inc-ZC3H15-2:1 | SEQ0012 | 0,0005 | 0,765 | 0,896 |
| Inc-GOLGA8O-5:6 | SEQ0428 | 0,02049 | 0,715 | 0,778 | Inc-ZFAT-1:3 | SEQ0562 | 0,02418 | 0,907 | 0,771 |
| Inc-GPAT4-1:3 | SEQ0316 | 0,01449 | 1,56 | 0,208 | Inc-ZFAT-1:8 | SEQ0393 | 0,01727 | 0,841 | 0,785 |
| Inc-GPATCH11-1:1 | SEQ0612 | 0,02842 | 0,703 | 0,764 | Inc-ZFC3H1-16:2 | SEQ0651 | 0,02842 | 0,789 | 0,764 |
| Inc-GPATCH2L-2:1 | SEQ0317 | 0,01449 | 0,85 | 0,792 | Inc-ZFP57-15:1 | SEQ0177 | 0,00701 | 0,402 | 0,826 |
| Inc-GPC2-2:5 | SEQ0518 | 0,02418 | 1,355 | 0,229 | Inc-ZFP90-3:6 | SEQ0563 | 0,02418 | 0,739 | 0,771 |
| Inc-GPR157-6:1 | SEQ0924 | 0,0449 | 0,827 | 0,743 | Inc-ZMYM2-7:1 | SEQ0853 | 0,03872 | 1,335 | 0,25 |
| Inc-GPR161-4:1 | SEQ0070 | 0,00291 | 0,726 | 0,847 | Inc-ZNF107-7:1 | SEQ0652 | 0,02842 | 0,735 | 0,764 |
| Inc-GPR27-18:1 | SEQ0925 | 0, 0449 | 0,774 | 0,743 | Inc-ZNF124-1:2 | SEQ0564 | 0,02418 | 0,827 | 0,771 |
| Inc-GPR33-14:1 | SEQ0236 | 0,01004 | 0,828 | 0,806 | Inc-ZNF189-2:1 | SEQ0751 | 0,03324 | 0,903 | 0,757 |
| Inc-GPR37-1:1 | SEQ0926 | 0, 0449 | 0,793 | 0,743 | Inc-ZNF25-7:1 | SEQ0257 | 0,01004 | 1,37 | 0,194 |
| Inc-GPR39-10:2 | SEQ0154 | 0,00681 | 1,393 | 0,181 | Inc-ZNF25-9:4 | SEQ0298 | 0,01209 | 0,663 | 0,799 |
| Inc-GPRC5A-4:1 | SEQ0613 | 0,02842 | 0,717 | 0,764 | Inc-ZNF273-4:4 | SEQ0092 | 0,00364 | 0,742 | 0,84 |
| lnc-GPRC6A-2:1 | SEQ0927 | 0, 0449 | 0,865 | 0,743 | Inc-ZNF330-2:1 | SEQ0854 | 0,03872 | 0,833 | 0,75 |
| Inc-GPSM1-3:3 | SEQ0928 | 0, 0449 | 0,64 | 0,743 | Inc-ZNF33A-14:1 | SEQ0049 | 0,00183 | 1,499 | 0,139 |
| Inc-GRAMD2B-4:1 | SEQ0801 | 0,03872 | 0,879 | 0,75 | Inc-ZNF33A-8:1 | SEQ1006 | 0,0449 | 1,115 | 0,257 |
| Inc-GRIP1-1:2 | SEQ0429 | 0,02049 | 1,564 | 0,222 | Inc-ZNF33B-3:11 | SEQ1007 | 0,0449 | 0,846 | 0,743 |
| Inc-GRIP1-5:2 | SEQ0519 | 0,02418 | 0,914 | 0,771 | Inc-ZNF33B-6:1 | SEQ0394 | 0,01727 | 2,524 | 0,215 |
| Inc-GRIP1-8:1 | SEQ0802 | 0,03872 | 1,339 | 0,25 | Inc-ZNF33B-6:3 | SEQ0299 | 0,01209 | 0,825 | 0,799 |
| lnc-GRM1-1:19 | SEQ0929 | 0, 0449 | 1,338 | 0,257 | Inc-ZNF385C-4:1 | SEQ0472 | 0,02049 | 1,311 | 0,222 |
| lnc-GRM1-1:31 | SEQ0155 | 0,00681 | 0,864 | 0,819 | Inc-ZNF423-3:3 | SEQ1008 | 0,0449 | 1,258 | 0,257 |
| lnc-GRM1-1:32 | SEQ0156 | 0,00681 | 0,864 | 0,819 | Inc-ZNF430-3:1 | SEQ0855 | 0,03872 | 0,797 | 0,75 |
| Inc-GRM8-2:2 | SEQ0430 | 0,02049 | 0, 902 | 0,778 | Inc-ZNF430-3:4 | SEQ0258 | 0,01004 | 0,788 | 0,806 |
| Inc-GSN-2:4 | SEQ0930 | 0, 0449 | 0,94 | 0,743 | Inc-ZNF442-1:2 | SEQ0061 | 0,00232 | 0,683 | 0,854 |
| Inc-GSN-2:5 | SEQ0803 | 0,03872 | 0, 955 | 0,75 | Inc-ZNF460-2:1 | SEQ0473 | 0,02049 | 0,842 | 0,778 |
| Inc-GTDC1-28:5 | SEQ0157 | 0,00681 | 0,725 | 0,819 | Inc-ZNF506-5:2 | SEQ0108 | 0,00451 | 0,684 | 0,833 |
| Inc-GUCY1A3-1:1 | SEQ0520 | 0,02418 | 0,724 | 0,771 | Inc-ZNF544-2:1 | SEQ0474 | 0,02049 | 0,805 | 0,778 |
| Inc-GUSB-1:1 | SEQ0931 | 0, 0449 | 0,829 | 0,743 | Inc-ZNF573-2:2 | SEQ0856 | 0,03872 | 0,823 | 0,75 |
| Inc-HECA-3:13 | SEQ0237 | 0,01004 | 0,8 | 0,806 | Inc-ZNF624-4:3 | SEQ0395 | 0,01727 | 1,241 | 0,215 |
| Inc-HECA-6:1 | SEQ0007 | 0,00037 | 0,723 | 0,903 | Inc-ZNF654-3:1 | SEQ0857 | 0,03872 | 0,869 | 0,75 |
| Inc-HELT-6:1 | SEQ0932 | 0, 0449 | 0,784 | 0,743 | Inc-ZNF720-5:3 | SEQ0222 | 0,00829 | 1,313 | 0,188 |
| Inc-HHATL-2:1 | SEQ0933 | 0, 0449 | 1,127 | 0,257 | Inc-ZNF724-6:1 | SEQ0176 | 0,00681 | 0,729 | 0,819 |
| Inc-HHLA2-2:1 | SEQ0696 | 0,03324 | 1,238 | 0,243 | Inc-ZNF726-1:3 | SEQ0259 | 0,01004 | 2,115 | 0,194 |
| Inc-HJURP-7:1 | SEQ0431 | 0,02049 | 0,776 | 0,778 | Inc-ZNF827-2:1 | SEQ0653 | 0,02842 | 0,924 | 0,764 |
| Inc-HLCS-5:1 | SEQ0238 | 0,01004 | 0,866 | 0,806 | Inc-ZNF99-3:2 | SEQ0396 | 0,01727 | 0,751 | 0,785 |
| lnc-HMG20A-1:2 | SEQ0432 | 0,02049 | 0,513 | 0,778 | Inc-ZRANB2-2:1 | SEQ0654 | 0,02842 | 0,801 | 0,764 |
| lnc-HMGA1-2:3 | SEQ0804 | 0,03872 | 1,288 | 0,25 | Inc-ZSWIM2-15:1 | SEQ0655 | 0,02842 | 0,696 | 0,764 |
| Inc-HMGN1-2:1 | SEQ0433 | 0,02049 | 1,424 | 0,222 | Inc-ZWINT-2:4 | SEQ0565 | 0,02418 | 0,637 | 0,771 |
| Inc-HOMEZ-4:1 | SEQ0434 | 0,02049 | 0,848 | 0,778 | LRP4-AS1:5 | SEQ0766 | 0,03872 | 1,288 | 0,25 |
| Inc-HOXC4-1:3 | SEQ0805 | 0,03872 | 0,845 | 0,75 | LYRM4-AS1:17 | SEQ0485 | 0,02418 | 0,893 | 0,771 |
| Inc-HS3ST3A1-1:1 | SEQ0239 | 0,01004 | 0,75 | 0,806 | MAPT-AS1:1 | SEQ0767 | 0,03872 | 0,785 | 0,75 |
| Inc-HS6ST1-8:1 | SEQ0697 | 0,03324 | 1,344 | 0,243 | MCPH1-AS1:2 | SEQ0308 | 0,01449 | 1,321 | 0,208 |
| Inc-HSCB-7:1 | SEQ0614 | 0,02842 | 0,742 | 0,764 | MDC1-AS1:5 | SEQ0486 | 0,02418 | 1,248 | 0,229 |
| Inc-HSD17B11-2:1 | SEQ0276 | 0,01209 | 0,792 | 0,799 | MEF2C-AS1:25 | SEQ0582 | 0,02842 | 0,907 | 0,764 |
| Inc-IFRD2-6:1 | SEQ0806 | 0,03872 | 1,31 | 0,25 | MIR155HG:7 | SEQ0876 | 0,0449 | 0,606 | 0,743 |
| Inc-IFT80-8:1 | SEQ0934 | 0,0449 | 0,916 | 0,743 | MIR29B2CHG:33 | SEQ0583 | 0,02842 | 0,624 | 0,764 |
| Inc-IL6-8:4 | SEQ0318 | 0,01449 | 0,77 | 0,792 | MIR29B2CHG:46 | SEQ0181 | 0,00829 | 1,522 | 0,188 |
| Inc-IPO5-7:1 | SEQ0521 | 0,02418 | 0,869 | 0,771 | MIR31HG:9 | SEQ0768 | 0,03872 | 0,835 | 0,75 |
| Inc-IQCF6-2:3 | SEQ0807 | 0,03872 | 2,026 | 0,25 | MIR4290HG:1 | SEQ0405 | 0,02049 | 0,773 | 0,778 |
| Inc-IRF2BP2-11:3 | SEQ0103 | 0,00451 | 1,403 | 0,167 | MIR9-3HG:29 | SEQ0769 | 0,03872 | 0, 557 | 0,75 |
| Inc-IRF6-1:1 | SEQ0808 | 0,03872 | 0,894 | 0,75 | MIR99AHG:104 | SEQ0096 | 0,00451 | 0,871 | 0,833 |
| Inc-IRS1-2:5 | SEQ0615 | 0,02842 | 1,878 | 0,236 | MIR99AHG:37 | SEQ0584 | 0,02842 | 0,879 | 0,764 |
| Inc-IRS1-6:1 | SEQ0037 | 0,00143 | 0,804 | 0,868 | MIR99AHG:46 | SEQ0877 | 0,0449 | 2,516 | 0,257 |
| Inc-IRS1-7:3 | SEQ0522 | 0,02418 | 1,153 | 0,229 | NAV2-AS5:1 | SEQ0051 | 0,00232 | 1,302 | 0,146 |
| Inc-IRX2-10:1 | SEQ0698 | 0,03324 | 0,744 | 0,757 | NAV2-AS5:2 | SEQ0052 | 0,00232 | 1,302 | 0,146 |
| Inc-ITGB8-2:8 | SEQ0616 | 0,02842 | 0,805 | 0,764 | NBAT1:11 | SEQ0347 | 0,01727 | 1,738 | 0,215 |
| Inc-JMJD4-2:1 | SEQ0435 | 0,02049 | 0,736 | 0,778 | NIFK-AS1:25 | SEQ0487 | 0,02418 | 0,846 | 0,771 |
| Inc-JRK-2:1 | SEQ0935 | 0,0449 | 1,162 | 0,257 | NUTM2A-AS1:49 | SEQ0182 | 0,00829 | 0,633 | 0,813 |
| Inc-JRK-2:2 | SEQ0699 | 0,03324 | 0,764 | 0,757 | NUTM2B-AS1:40 | SEQ0348 | 0,01727 | 0,74 | 0,785 |
| Inc-KAT5-2:1 | SEQ0936 | 0, 0449 | 0,769 | 0,743 | NUTM2B-AS1:53 | SEQ0004 | 0,00027 | 0,655 | 0,91 |
| Inc-KBTBD7-1:1 | SEQ0523 | 0,02418 | 0,742 | 0,771 | PACRG-AS3:1 | SEQ0666 | 0,03324 | 1,162 | 0,243 |
| Inc-KBTBD8-4:3 | SEQ0366 | 0,01727 | 1,457 | 0,215 | PCBP1-AS1:250 | SEQ0770 | 0,03872 | 1,266 | 0,25 |
| Inc-KCNA1-1:23 | SEQ0122 | 0,00556 | 0,633 | 0,826 | PITRM1-AS1:10 | SEQ0488 | 0,02418 | 1,274 | 0,229 |
| Inc-KCND3-1:1 | SEQ0085 | 0,00364 | 0,808 | 0,84 | PTPRG-AS1:14 | SEQ0183 | 0,00829 | 0,625 | 0,813 |
| Inc-KCNE1B-15:1 | SEQ0319 | 0,01449 | 0,823 | 0,792 | PURPL:13 | SEQ0585 | 0,02842 | 1,386 | 0,236 |
| Inc-KCNS3-9:1 | SEQ0320 | 0,01449 | 1,265 | 0,208 | RAPGEF4-AS1:1 | SEQ0878 | 0,0449 | 0,824 | 0,743 |
| lnc-KCTD13-3:1 | SEQ0700 | 0,03324 | 0,709 | 0,757 | RFX3-AS1:22 | SEQ0586 | 0,02842 | 0,819 | 0,764 |
| lnc-KCTD19-1:1 | SEQ0701 | 0,03324 | 0,761 | 0,757 | RORB-AS1:6 | SEQ0116 | 0,00556 | 0,845 | 0,826 |
| Inc-KDM3A-1:4 | SEQ0104 | 0,00451 | 0,731 | 0,833 | SCHLAP1:9 | SEQ0144 | 0,00681 | 0,8 | 0,819 |
| lnc-KDM8-3:1 | SEQ0436 | 0,02049 | 0,758 | 0,778 | SEC24B-AS1:19 | SEQ0406 | 0,02049 | 1,852 | 0,222 |
| Inc-KIAA0141-3:1 | SEQ0702 | 0,03324 | 1,15 | 0,243 | SLC16A12-AS1:1 | SEQ0771 | 0,03872 | 0,773 | 0,75 |
| Inc-KIF21B-2:1 | SEQ0937 | 0, 0449 | 0,873 | 0,743 | SMILR:3 | SEQ0040 | 0,00183 | 1,306 | 0,139 |
| Inc-KIN-5:1 | SEQ0703 | 0,03324 | 0,835 | 0,757 | SNCA-AS1:3 | SEQ0041 | 0,00183 | 0,777 | 0,861 |
| Inc-KLF11-1:8 | SEQ0704 | 0,03324 | 0,286 | 0,757 | SPATA41:13 | SEQ0879 | 0,0449 | 1,607 | 0,257 |
| Inc-KLF12-4:1 | SEQ0938 | 0, 0449 | 0,806 | 0,743 | SRGAP3-AS2:10 | SEQ0349 | 0,01727 | 0,674 | 0,785 |
| Inc-KLF12-7:1 | SEQ0240 | 0,01004 | 0,88 | 0,806 | SUCLG2-AS1:14 | SEQ0489 | 0,02418 | 0,92 | 0,771 |
| Inc-KLHL24-2:1 | SEQ0321 | 0,01449 | 1,206 | 0,208 | SUCLG2-AS1:9 | SEQ0490 | 0,02418 | 0,92 | 0,771 |
| Inc-KLK2-4:3 | SEQ0524 | 0,02418 | 0,881 | 0,771 | TCL6:24 | SEQ0066 | 0,00291 | 1,448 | 0,153 |
| Inc-KLRG1-8:1 | SEQ0809 | 0,03872 | 1,222 | 0,25 | TM4SF19-AS1:10 | SEQ0184 | 0,00829 | 1,84 | 0,188 |
| Inc-KREMEN2-1:1 | SEQ0705 | 0,03324 | 1,279 | 0,243 | TM4SF19-AS1:15 | SEQ0491 | 0,02418 | 0,853 | 0,771 |
| Inc-KRR1-4:7 | SEQ0706 | 0,03324 | 0,801 | 0,757 | TMEM9B-AS1:11 | SEQ0185 | 0,00829 | 3,049 | 0,188 |
| Inc-KY-4:1 | SEQ0123 | 0,00556 | 0,708 | 0,826 | TPT1-AS1:26 | SEQ0186 | 0,00829 | 0,763 | 0,813 |
| Inc-L3MBTL2-1:1 | SEQ0367 | 0,01727 | 1,259 | 0,215 | TRIM52-AS1:24 | SEQ0492 | 0,02418 | 1,183 | 0,229 |
| Inc-LARP1B-1:15 | SEQ0277 | 0,01209 | 0,786 | 0,799 | TRIM52-AS1:8 | SEQ0587 | 0,02842 | 1,272 | 0,236 |
| Inc-LARP1B-1: 17 | SEQ0278 | 0,01209 | 0,786 | 0,799 | TTN-AS1:79 | SEQ0187 | 0,00829 | 0,829 | 0,813 |
| Inc-LARP1B-1:18 | SEQ0279 | 0,01209 | 1,214 | 0,201 | TTN-AS1:80 | SEQ0188 | 0,00829 | 0,829 | 0,813 |
| Inc-LARP4-6:1 | SEQ0368 | 0,01727 | 0,803 | 0,785 | UCA1:7 | SEQ0078 | 0,00364 | 0,697 | 0,84 |
| Inc-LBH-4:1 | SEQ0707 | 0,03324 | 1,312 | 0,243 | UGDH-AS1:10 | SEQ0407 | 0,02049 | 0, 541 | 0,778 |
| Inc-LBX1-1:1 | SEQ0241 | 0,01004 | 0,788 | 0,806 | WDR86-AS1:15 | SEQ0588 | 0,02842 | 1,212 | 0,236 |
| Inc-LEPROTL1-12:1 | SEQ0011 | 0,0005 | 0,749 | 0,896 | WEE2-AS1:23 | SEQ0408 | 0,02049 | 0,704 | 0,778 |
| Inc-LIMS3-1:10 | SEQ0086 | 0,00364 | 2,27 | 0,16 | WT1-AS:9 | SEQ0117 | 0,00556 | 0, 542 | 0,826 |
| Inc-LINC00675-1:3 | SEQ0810 | 0,03872 | 0,669 | 0,75 | YEATS2-AS1:3 | SEQ0772 | 0,03872 | 0,806 | 0,75 |
| Inc-LINS1-2:1 | SEQ0811 | 0,03872 | 0,81 | 0,75 | ZNF528-AS1:1 | SEQ0773 | 0,03872 | 0,636 | 0,75 |
| Inc-LMBRD1-5:17 | SEQ0812 | 0,03872 | 0,574 | 0,75 | ZNF529-AS1:21 | SEQ0880 | 0, 0449 | 0,819 | 0,743 |

Out of the 1008 serum IncRNAs differentially expressed with a statistical significance (p value <0.05), 33 IncRNAs showed a fold change of >2 or <0.5 and are shown in the Table 2.

**Table 2: Mean +/-SD of the 33 serum IncRNAs that shows a differential expression with both a statistically significance (p<0.05, Wilcoxon test) and a fold change > 2 or <0.5**

| **IncRNA** | **p value** | **Fold change** | **IncRNA** | **p value** | **Fold change** |
|---|---|---|---|---|---|
| Inc-QRFP-5:1 | 0.00066 | 2.170 | MIR99AHG:46 | 0.04490 | 2.516 |
| Inc-C21orf58-1:2 | 0.00143 | 2.231 | Inc-CPM-2:11 | 0.04490 | 0.424 |
| ARRDC3-AS1:7 | 0.00232 | 0.441 | Inc-ERFE-1:1 | 0.04490 | 0.489 |
| Inc-CRYBB1-1:1 | 0.00232 | 0.487 | Inc-LRRC3B-1:3 | 0.04490 | 0.463 |
| Inc-LIMS3-1:10 | 0.00364 | 2.270 | LINC02177:8 | 0.03872 | 0.494 |
| Inc-MARCH4-2:7 | 0.00364 | 2.000 | Inc-KLF11-1:8 | 0.03324 | 0.286 |
| Inc-SUGT1-3:1 | 0.00556 | 2.047 | Inc-SNCA-3:1 | 0.02418 | 0.498 |
| DARS-AS1:47 | 0.00829 | 2.015 | Inc-UNCX-3:26 | 0.02049 | 0.493 |
| TMEM9B-AS1:11 | 0.00829 | 3.049 | BLACAT1:5 | 0.01727 | 0.424 |
| Inc-ZNF726-1:3 | 0.01004 | 2.115 | Inc-CHD1L-5:13 | 0.01449 | 0.370 |
| Inc-SLC39A11-10:11 | 0.01209 | 4.380 | Inc-EDDM13-5:11 | 0.01004 | 0.372 |
| Inc-PSMB1-6:4 | 0.01449 | 2.134 | Inc-EEF1AKMT1-3:6 | 0.00829 | 0.423 |
| DPH6-AS1:3 | 0.01727 | 2.004 | FLVCR1-AS1:13 | 0.00681 | 0.413 |
| Inc-ZNF33B-6:1 | 0.01727 | 2.524 | Inc-NAXD-6:5 | 0.00556 | 0.479 |
| Inc-PAPPA-1:3 | 0.03324 | 2.192 | Inc-ZFP57-15:1 | 0.00701 | 0.402 |
| Inc-TENM4-4:1 | 0.03324 | 2.209 | Inc-TPPP-1:2 | 0.00014 | 0.158 |
| Inc-IQCF6-2:3 | 0.03872 | 2.026 | | | |

Out of the 1008 serum IncRNAs differentially expressed with a statistical significance (p value <0.05), 60 IncRNAs showed an AUC of ≥0.85 and are shown in Table 3.

**Table 3: 60 serum IncRNAs with a p value <0.05 and an individual AUC value AUC ≥0.85**

| **IncRNA** | **AUC** | | **IncRNA** | **AUC** |
|---|---|---|---|---|
| ARRDC3-AS1:7 | 0.854 | | Inc-FILlP1L-3:1 | 0.854 |
| HAND2-AS1:70 | 0.861 | | Inc-FNBP1L-1:11 | 0.910 |
| LINC00882:70 | 0.875 | | Inc-GHR-1:1 | 0.917 |
| LINC00882:71 | 0.875 | | Inc-HECA-6:1 | 0.903 |
| LINC01410:11 | 0.882 | | Inc-IRS1-6:1 | 0.868 |
| LINC02345:11 | 0.111 | | Inc-LEPROTL1-12:1 | 0.896 |
| NAV2-AS5:1 | 0.146 | | Inc-MGST3-1:3 | 0.139 |
| NAV2-AS5:2 | 0.146 | | Inc-NCR3LG1-3:1 | 0.889 |
| NUTM2B-AS1:53 | 0.910 | | Inc-OR4F29-3:11 | 0.861 |
| SMILR:3 | 0.139 | | Inc-PPP2R3C-4:1 | 0.882 |
| SNCA-AS1:3 | 0.861 | | Inc-QRFP-5:1 | 0.111 |
| Inc-ABCA5-7:1 | 0.111 | | Inc-RHNO1-1:1 | 0.118 |
| Inc-ACOT12-9:1 | 0.854 | | Inc-RNF6-2:1 | 0.854 |
| Inc-APLP2-4:1 | 0.854 | | Inc-RPS21-4:2 | 0.882 |
| Inc-C21orf58-1:2 | 0.132 | | Inc-SOX14-2:1 | 0.861 |
| Inc-CEP170-9:2 | 0.861 | | Inc-SPP1-1:1 | 0.861 |
| Inc-CNDP1-7:1 | 0.882 | | Inc-STOML3-6:1 | 0.868 |
| Inc-CRYBB1-1:1 | 0.854 | | Inc-TACSTD2-2:4 | 0.889 |
| Inc-CSNK1A1-6:1 | 0.875 | | Inc-TENM3-3:3 | 0.938 |
| Inc-DAZAP2-3:1 | 0.854 | | Inc-TENM3-3:4 | 0.097 |
| Inc-DKK1-5:3 | 0.910 | | Inc-TENM3-3:5 | 0.097 |
| Inc-DLG5-1:1 | 0.868 | | Inc-TMEM185B-12:7 | 0.146 |
| Inc-DOCK7-7:1 | 0.889 | | Inc-TPPP-1:2 | 0.924 |
| Inc-DUSP10-6:1 | 0.854 | | Inc-TRIB2-14:1 | 0.889 |
| Inc-DUSP26-3:2 | 0.875 | | Inc-TWSG1-2:1 | 0.861 |
| Inc-EBLN1-1:4 | 0.889 | | Inc-UBLCP1-2:6 | 0.861 |
| Inc-EDDM13-5:3 | 0.132 | | Inc-USP31-2:3 | 0.889 |
| Inc-FAM133B-2:1 | 0.868 | | Inc-ZC3H 15-2:1 | 0.896 |
| Inc-FAM217A-1:2 | 0.875 | | Inc-ZNF33A-14:1 | 0.139 |
| Inc-FAM49B-8:1 | 0.104 | | Inc-ZNF442-1:2 | 0.854 |
| Inc-FAT1-7:2 | 0.111 | | | |

The predictive modelling based on the random forest algorithm to discriminate between AD patient and healthy control populations when using the total of the 19867 serum IncRNAs analyzed, enabled to show that the AUC in function of the number of IncRNA reached a plateau with the following 13 IncRNAs. These 13 IncRNAs were used to construct the model. The results show that this IncRNA signature enabled a discrimination between the 2 populations (mild AD patient and healthy control populations) with an AUC value = 0.993, an accuracy = 95.8%, sensitivity = 100% and specificity = 91.7%.

| IncRNA | Rank |
|---|---|
| Inc-DLG5-1:1 | 1 |
| Inc-EBLN1-1:4 | 2 |
| Inc-FAT1-7:2 | 3 |
| Inc-PRR5-5:1 | 4 |
| Inc-RBKS-6:1 | 5 |
| Inc-FOXD4L5-35:1 | 6 |
| Inc-TENM3-3:3 | 7 |
| Inc-FAM133B-2:1 | 8 |
| Inc-ZNF726-1:3 | 9 |
| Inc-AP3M1-1:1 | 10 |
| Inc-DUSP10-6:1 | 11 |
| Inc-TPPP-1:2 | 12 |
| LINC01206:20 | 13 |

Out of the 1008 serum IncRNAs differentially expressed with a statistical significance (p value <0.05), the 90 IncRNAs with a fold change of > 2 (or < 0.5) or an AUC of ≥ 0.85 (or ≤ 0.15) were used for the predictive modelling based on the random forest algorithm. The results show that with the following 7 top ranked candidates enabled a full discrimination between AD and HV groups, with an AUC value of 100% as well as 100% accuracy, 100% sensitivity and 100% specificity.

| IncRNA | Rank |
|---|---|
| LINC02345:11 | 1 |
| Inc-EBLN1-1:4 | 2 |
| Inc-TPPP-1:2 | 3 |
| Inc-TENM3-3:3 | 4 |
| Inc-FAT1-7:2 | 5 |
| Inc-DKK1-5:3 | 6 |
| Inc-TACSTD2-2:4 | 7 |

When applying another filter considering a list of the IncRNAs which have a fold change of ≥ 1.6 and an AUC of ≥ 0.85, the use of random forest algorithm enabled to select the following 12-top ranked serum IncRNAs to construct the model and this enabled to discriminate between the AD and HV populations with still an excellent AUC=0.979, excellent accuracy of 95.8% sensitivity of 91.7% and specificity of 100%.

| IncRNA | Rank |
|---|---|
| Inc-TPPP-1:2 | 1 |
| ARRDC3-AS1:7 | 2 |
| Inc-TENM3-3:5 | 3 |
| Inc-TENM3-3:4 | 4 |
| Inc-QRFP-5:1 | 5 |
| Inc-CRYBB1-1:1 | 6 |
| Inc-MGST3-1:3 | 7 |
| Inc-FAM49B-8:1 | 8 |
| HAND2-AS1:70 | 9 |
| Inc-TMEM185B-12:7 | 10 |
| Inc-CNDP1-7:1 | 11 |
| Inc-C21orf58-1:2 | 12 |

Further to select the best serum IncRNA candidates, modeling using Random Forest algorithm was applied to a specific set of IncRNA candidates having both a statistically significant differential expression in AD patients versus healthy control populations and a good correlation (Pearson R coefficient) with scores of neurocognitive tests including MMSE and/or MOCA out of 7 neuropsychological tests performed (Table 4): The results show that the signature of the following 3 top ranked IncRNAs enabled an excellent discrimination between AD patients and healthy control populations with AUC=0.953, accuracy, sensitivity and specificity of 91.7%, 91.7% and 91.7%.

| IncRNA | Rank |
|---|---|
| Inc-TENM3-3:3 | 1 |
| Inc-MARCH4-2:7 | 2 |
| Inc-LRRC1-5:2 | 3 |

**Table 4: Serum IncRNAs of the present invention that correlate with neurocognitive tests, MoCA, MMSE. R: correlation coefficient (Pearson). HV: healthy control group, AD: mild to moderate AD group**

| **Cognitive test** | **IncRNA** | **R_HV+AD** | **R_HV** | **R_AD** | **p value** | **AUC** |
|---|---|---|---|---|---|---|
| MoCA | LINC00839:18 | -0,80 | 0,29 | -0,78 | 0,00556 | 0,83 |
| MoCA | LINC01087:1 | -0,74 | -0,13 | -0,89 | 0,00681 | 0,82 |
| MMSE | LINC01087:1 | -0,68 | 0,05 | -0,75 | 0,00681 | 0,82 |
| MMSE | Inc-ANAPC11-2:6 | 0,65 | 0,26 | 0,77 | 0,04490 | 0,26 |
| MoCA | Inc-ANAPC11-2:6 | 0,59 | -0,46 | 0,77 | 0,04490 | 0,26 |
| MoCA | Inc-ARHGAP26-4:33 | -0,64 | -0,66 | -0,72 | 0,03872 | 0,75 |
| MoCA | Inc-CCDC197-2:1 | -0,14 | -0,42 | 0,74 | 0,00291 | 0,85 |
| MMSE | Inc-CFAP36-3:2 | -0,62 | 0,05 | -0,75 | 0,03872 | 0,75 |
| MoCA | Inc-DHX38-25:1 | -0,69 | -0,60 | -0,77 | 0,01004 | 0,81 |
| MMSE | Inc-DHX38-25:1 | -0,65 | -0,57 | -0,71 | 0,01004 | 0,81 |
| MoCA | Inc-EZH2-3:1 | -0,69 | 0,18 | -0,78 | 0,01209 | 0,80 |
| MMSE | Inc-GNG5-8:1 | -0,72 | 0,32 | -0,71 | 0,03324 | 0,76 |
| MoCA | Inc-GPRC5A-4:1 | -0,65 | -0,02 | -0,74 | 0,02842 | 0,76 |
| MMSE | Inc-GRAMD2B-4:1 | -0,59 | 0,42 | -0,72 | 0,03872 | 0,75 |
| MMSE | Inc-GRIP1-8:1 | -0,03 | 0,30 | -0,73 | 0,03872 | 0,25 |
| MoCA | Inc-LYN-8:1 | 0,13 | 0,54 | -0,71 | 0,02842 | 0,24 |
| MMSE | Inc-MAP9-6:1 | -0,73 | -0,08 | -0,74 | 0,01209 | 0,80 |
| MMSE | Inc-NRP1-4:1 | -0,11 | 0,27 | -0,72 | 0,04490 | 0,26 |
| MoCA | Inc-RBFOX1-2:1 | 0,02 | 0,13 | -0,75 | 0,01727 | 0,22 |
| MoCA | Inc-RPL37-2:1 | 0,62 | -0,16 | 0,75 | 0,00829 | 0,19 |
| MMSE | Inc-RUBCN-1:1 | 0,11 | 0,33 | 0,72 | 0,03872 | 0,75 |
| MMSE | Inc-SNCA-3:1 | 0,08 | 0,12 | 0,76 | 0,02418 | 0,77 |
| MMSE | Inc-SRSF2-2:5 | 0,10 | 0,04 | 0,75 | 0,03324 | 0,76 |
| MoCA | Inc-SRSF2-2:5 | -0,05 | -0,32 | 0,73 | 0,03324 | 0,76 |
| MMSE | Inc-TMEM185B-12:7 | 0,06 | -0,14 | -0,73 | 0,00232 | 0,15 |
| MoCA | Inc-TRIB2-14:1 | -0,76 | -0,45 | -0,71 | 0,00066 | 0,89 |
| MMSE | Inc-TRIM43B-1:2 | -0,74 | -0,11 | -0,76 | 0,01727 | 0,78 |
| MoCA | Inc-TRIM43B-1:2 | -0,72 | -0,12 | -0,74 | 0,01727 | 0,78 |
| MMSE | Inc-ZNF189-2:1 | -0,70 | -0,35 | -0,74 | 0,03324 | 0,76 |
| MoCA | Inc-ZNF189-2:1 | -0,67 | 0,03 | -0,76 | 0,03324 | 0,76 |
| MMSE | Inc-ZRANB2-2:1 | -0,65 | -0,36 | -0,82 | 0,02842 | 0,76 |
| MoCA | Inc-ZRANB2-2:1 | -0,59 | 0,16 | -0,80 | 0,02842 | 0,76 |

Modeling using Random Forest algorithm was also applied to a specific set of serum IncRNA candidates from Table 5 having both a statistically significant differential expression in AD patients versus healthy control populations and a good correlation (Pearson) with neuroimaging scores (volume of brain structures of relevance for cognition and memory such as the mediotemporal area, left and right hippocampus, left and right amygdala, entorhinal cortex out of more than 120 structures measured): The results show that the signature of the following 7 top ranked IncRNAs enabled an excellent discrimination between AD patients and healthy control population with AUC=0.993, accuracy=95.8%, sensitivity=91.7% and specificity= 100%.

| IncRNA | Rank |
|---|---|
| Inc-TPPP-1:2 | 1 |
| Inc-TENM3-3:3 | 2 |
| Inc-TMEM185B-12:7 | 3 |
| Inc-NAXD-6:5 | 4 |
| Inc-HECA-6:1 | 5 |
| Inc-COMMD6-10:1 | 6 |
| MIR29B2CHG:46 | 7 |

**Table 5: Serum IncRNAs of the present invention that correlate with MRI (volumes of the brain structures). R: correlation coefficient (Pearson). HV: healthy control group, AD: mild to moderate AD group.**

| **MRI** | **IncRNA** | **R** _ **HV+AD** | **R** _ **HV** | **R** _ **AD** | **p value** | **AUC** |
|---|---|---|---|---|---|---|
| Left.Amygdala | CYTOR:18 | 0,71 | 0,30 | 0,69 | 0,00291 | 0,15 |
| Left.Hippocampus | DLGAP2-AS1:18 | -0,71 | -0,51 | -0,76 | 0,03324 | 0,76 |
| Left.Hippocampus | LINC00458:19 | -0,79 | -0,48 | -0,78 | 0,02842 | 0,76 |
| Mediotemporal | LINC00458:19 | -0,72 | -0,48 | -0,53 | 0,02842 | 0,76 |
| Left.Hippocampus | LINC00938:6 | -0,82 | -0,48 | -0,76 | 0,01209 | 0,80 |
| Mediotemporal | LINC00938:6 | -0,76 | -0,63 | -0,46 | 0,01209 | 0,80 |
| Right. Hippocampus | LINC00938:6 | -0,75 | -0,56 | -0,48 | 0,01209 | 0,80 |
| Left.Amygdala | LINC01629:11 | -0,78 | -0,53 | -0,83 | 0,02842 | 0,76 |
| Left.Hippocampus | Inc-AIG1-5:1 | -0,78 | -0,64 | -0,73 | 0,02418 | 0,77 |
| Left.Hippocampus | Inc-AKR1 D1-5:2 | -0,74 | -0,47 | -0,69 | 0,00451 | 0,83 |
| Right.Hippocampus | Inc-AKR1D1-5:2 | -0,71 | -0,27 | -0,73 | 0,00451 | 0,83 |
| Mediotemporal | Inc-AKR1D1-5:2 | -0,71 | -0,26 | -0,72 | 0,00451 | 0,83 |
| Right.Amygdala | Inc-C3orf58-7:1 | -0,73 | -0,22 | -0,69 | 0,02842 | 0,76 |
| Left.Hippocampus | Inc-C5orf30-10:2 | -0,73 | -0,58 | -0,58 | 0,02842 | 0,76 |
| Right.Amygdala | Inc-CASP9-1:1 | 0,73 | 0,26 | 0,81 | 0,03872 | 0,25 |
| Right.Hippocampus | Inc-CHN1-5:11 | -0,72 | -0,44 | -0,67 | 0,02049 | 0,78 |
| Right.Amygdala | Inc-CLVS2-2:5 | -0,74 | -0,39 | -0,75 | 0,01727 | 0,78 |
| Left.Hippocampus | Inc-DAZAP2-3:1 | -0,72 | -0,53 | -0,58 | 0,00232 | 0,85 |
| Mediotemporal | Inc-DTWD2-14:1 | -0,75 | -0,49 | -0,79 | 0,01727 | 0,78 |
| Left.Amygdala | Inc-DTWD2-14:1 | -0,74 | -0,46 | -0,82 | 0,01727 | 0,78 |
| Ih_entorhinal | Inc-DTWD2-14:1 | -0,74 | -0,59 | -0,61 | 0,01727 | 0,78 |
| Right.Hippocampus | Inc-ELFN2-1:3 | -0,77 | -0,26 | -0,76 | 0,00364 | 0,84 |
| Left.Amygdala | Inc-ELFN2-1:3 | -0,74 | -0,27 | -0,81 | 0,00364 | 0,84 |
| Mediotemporal | Inc-ELFN2-1:3 | -0,74 | -0,22 | -0,73 | 0,00364 | 0,84 |
| Right.Amygdala | Inc-ELFN2-1:3 | -0,74 | -0,37 | -0,61 | 0,00364 | 0,84 |
| Left.Hippocampus | Inc-FAM171B-1:6 | -0,72 | -0,28 | -0,62 | 0,00829 | 0,81 |
| Left.Hippocampus | Inc-FGD4-8:1 | -0,74 | -0,49 | -0,61 | 0,01004 | 0,81 |
| Left.Amygdala | Inc-FGD4-9:1 | -0,76 | -0,49 | -0,56 | 0,00364 | 0,84 |
| Right.Hippocampus | Inc-FGD4-9:1 | -0,74 | -0,19 | -0,64 | 0,00364 | 0,84 |
| Mediotemporal | Inc-FGD4-9:1 | -0,72 | -0,28 | -0,54 | 0,00364 | 0,84 |
| Left.Hippocampus | Inc-FGD4-9:1 | -0,71 | -0,33 | -0,52 | 0,00364 | 0,84 |
| Ih_entorhinal | Inc-GPR39-10:2 | 0,77 | 0,68 | 0,49 | 0,00681 | 0,18 |
| Left.Amygdala | Inc-GPR39-10:2 | 0,72 | 0,54 | 0,37 | 0,00681 | 0,18 |
| Left.Hippocampus | Inc-GRM1-1:31 | -0,76 | -0,39 | -0,72 | 0,00681 | 0,82 |
| Right. Hippocampus | Inc-GRM1-1:31 | -0,71 | -0,38 | -0,56 | 0,00681 | 0,82 |
| Left. Hippocampus | Inc-GRM1-1:32 | -0,76 | -0,39 | -0,72 | 0,00681 | 0,82 |
| Right.Hippocampus | Inc-GRM1-1:32 | -0,71 | -0,38 | -0,56 | 0,00681 | 0,82 |
| Right.Hippocampus | Inc-HECA-3:13 | -0,72 | -0,31 | -0,67 | 0,01004 | 0,81 |
| Right.Amygdala | Inc-HJURP-7:1 | -0,77 | -0,46 | -0,77 | 0,02049 | 0,78 |
| Left.Hippocampus | Inc-HJURP-7:1 | -0,73 | -0,33 | -0,73 | 0,02049 | 0,78 |
| Right.Hippocampus | Inc-HJURP-7:1 | -0,73 | -0,02 | -0,93 | 0,02049 | 0,78 |
| Mediotemporal | Inc-HJURP-7:1 | -0,72 | -0,20 | -0,82 | 0,02049 | 0,78 |
| Left.Amygdala | Inc-IRS1-6:1 | -0,75 | -0,35 | -0,72 | 0,00143 | 0,87 |
| Right.Hippocampus | Inc-IRS1-6:1 | -0,73 | -0,36 | -0,56 | 0,00143 | 0,87 |
| Mediotemporal | Inc-IRS1-6:1 | -0,71 | -0,34 | -0,51 | 0,00143 | 0,87 |
| Right.Hippocampus | Inc-KDM3A-1:4 | -0,81 | -0,35 | -0,80 | 0,00451 | 0,83 |
| Mediotemporal | Inc-KDM3A-1:4 | -0,77 | -0,36 | -0,70 | 0,00451 | 0,83 |
| Left.Hippocampus | Inc-KDM3A-1:4 | -0,72 | -0,26 | -0,61 | 0,00451 | 0,83 |
| Left.Hippocampus | Inc-LARP1B-1:15 | -0,75 | -0,56 | -0,75 | 0,01209 | 0,80 |
| Right.Amygdala | Inc-LARP1B-1:15 | -0,75 | -0,63 | -0,71 | 0,01209 | 0,80 |
| Left.Hippocampus | Inc-LARP1B-1:17 | -0,75 | -0,56 | -0,75 | 0,01209 | 0,80 |
| Right.Amygdala | Inc-LARP1B-1:17 | -0,75 | -0,63 | -0,71 | 0,01209 | 0,80 |
| Right.Amygdala | Inc-MAMDC2-1:1 | -0,75 | -0,72 | -0,70 | 0,02418 | 0,77 |
| Left.Hippocampus | Inc-MAP9-6:1 | -0,79 | -0,43 | -0,87 | 0,01209 | 0,80 |
| Right.Hippocampus | Inc-NAALADL2-8:1 | -0,71 | -0,28 | -0,72 | 0,01449 | 0,79 |
| Right.Hippocampus | Inc-NBPF14-1:2 | -0,80 | -0,38 | -0,82 | 0,00364 | 0,84 |
| Mediotemporal | Inc-NBPF14-1:2 | -0,75 | -0,37 | -0,67 | 0,00364 | 0,84 |
| Right.Amygdala | Inc-NBPF14-1:2 | -0,73 | -0,40 | -0,60 | 0,00364 | 0,84 |
| Left.Hippocampus | Inc-NBPF14-1:2 | -0,72 | -0,48 | -0,52 | 0,00364 | 0,84 |
| Right.Hippocampus | Inc-PIGB-1:5 | -0,73 | -0,26 | -0,79 | 0,02418 | 0,77 |
| Right.Amygdala | Inc-PLN-2:1 | -0,77 | -0,66 | -0,72 | 0,03324 | 0,76 |
| Mediotemporal | Inc-PLN-2:1 | -0,76 | -0,66 | -0,67 | 0,03324 | 0,76 |
| Left.Hippocampus | Inc-PLN-2:1 | -0,75 | -0,67 | -0,64 | 0,03324 | 0,76 |
| Right. Hippocampus | Inc-PLN-2:1 | -0,73 | -0,54 | -0,68 | 0,03324 | 0,76 |
| Left.Hippocampus | Inc-PTPN4-1:1 | -0,74 | -0,59 | -0,69 | 0,02842 | 0,76 |
| Right.Amygdala | Inc-SLC38A2-1:11 | -0,76 | -0,73 | -0,57 | 0,01004 | 0,81 |
| Left.Amygdala | Inc-SLC38A2-1:11 | -0,73 | -0,57 | -0,38 | 0,01004 | 0,81 |
| Right.Amygdala | Inc-SMARCA5-4:18 | -0,72 | -0,35 | -0,78 | 0,01004 | 0,81 |
| Mediotemporal | Inc-SMARCA5-4:18 | -0,72 | -0,29 | -0,84 | 0,01004 | 0,81 |
| Left.Amygdala | Inc-SMARCA5-4:18 | -0,71 | -0,30 | -0,92 | 0,01004 | 0,81 |
| Ih_entorhinal | Inc-TMEM242-6:1 | -0,77 | -0,64 | -0,78 | 0,02049 | 0,78 |
| Mediotemporal | Inc-TMEM242-6:1 | -0,72 | -0,62 | -0,63 | 0,02049 | 0,78 |
| Right. Hippocampus | Inc-TPPP-1:2 | -0,73 | -0,03 | -0,42 | 0,00014 | 0,92 |
| Left.Hippocampus | Inc-TRIM43B-1:2 | -0,75 | -0,47 | -0,72 | 0,01727 | 0,78 |
| Ih_entorhinal | Inc-TRMT11-4:1 | -0,80 | -0,86 | -0,30 | 0,00829 | 0,81 |
| Mediotemporal | Inc-TRMT11-4:1 | -0,74 | -0,73 | -0,30 | 0,00829 | 0,81 |
| Left.Amygdala | Inc-TRMT11-4:1 | -0,73 | -0,58 | -0,35 | 0,00829 | 0,81 |
| Left.Hippocampus | Inc-USP53-1:1 | -0,75 | -0,24 | -0,84 | 0,01727 | 0,78 |
| Left.Hippocampus | Inc-ZNF33B-6:3 | -0,78 | -0,40 | -0,81 | 0,01209 | 0,80 |
| Right.Amygdala | Inc-ZNF33B-6:3 | -0,74 | -0,47 | -0,65 | 0,01209 | 0,80 |
| Right.Amygdala | MIR29B2CHG:46 | 0,74 | 0,35 | 0,82 | 0,00829 | 0,19 |
| rh_entorhinal | MIR29B2CHG:46 | 0,71 | 0,45 | 0,80 | 0,00829 | 0,19 |
| Right.Amygdala | PCBP1-AS1 :250 | 0,73 | 0,20 | 0,71 | 0,03872 | 0,25 |
| Mediotemporal | PCBP1-AS1 :250 | 0,71 | 0,19 | 0,70 | 0,03872 | 0,25 |

Modeling using Random Forest algorithm was also applied to a specific set of serum IncRNA candidates from Table 6 having both a statistically significant differential expression in AD patients versus healthy control populations and a good correlation (Pearson) with level of the CSF biomarkers of high relevance to AD pathology: Aβ42, tau or phosphorylated-tau: The results show that the signature of the following 18 top ranked IncRNAs enabled an excellent discrimination between AD patients and healthy control population with AUC=0.972 accuracy=0,917 sensitivity =0.83 and specificity=1.

| IncRNA | Rank | IncRNA | Rank |
|---|---|---|---|
| Inc-TPPP-1:2 | 1 | Inc-DNALI1-5:4 | 10 |
| LINC02345:11 | 2 | RORB-AS1:6 | 11 |
| Inc-ZNF273-4:4 | 3 | Inc-TPPP-1:3 | 12 |
| lnc-TACC2-8:6 | 4 | Inc-BMS1-2:1 | 13 |
| LINC01206:20 | 5 | Inc-ADRB1-4:1 | 14 |
| Inc-C5orf67-3:1 | 6 | Inc-XXYLT1-5:1 | 15 |
| HAND2-AS1:58 | 7 | MIR99AHG:104 | 16 |
| Inc-PRDM9-20:1 | 8 | LINC01748:17 | 17 |
| Inc-CLK1-1:7 | 9 | Inc-AKR1E2-15:1 | 18 |

**Table 6: Serum IncRNAs of the present invention that correlate with CSF biomarkers**

| **CSF BM** | **IncRNA** | **R_AD** | **PValue** | **AUC** | **CSF BM** | **IncRNA** | **R_AD** | **PValue** | **AUC** | **CSF BM** | **IncRNA** | **R_AD** | **PValue** | **AUC** |
|---|---|---|---|---|---|---|---|---|---|---|---|---|---|---|
| Aβ42 | LINC01748:17 | -0,73 | 0,0242 | 0,77 | T-tau | HAND2-AS1:58 | 0,92 | 0,0045 | 0,83 | p-tau | BLACAT1:3 | -0,71 | 0,0145 | 0,21 |
| Aβ42 | Inc-CSorf67-3:1 | 0,72 | 0,0068 | 0,82 | T-tau | HAND2-AS1:59 | 0,9 | 0,0056 | 0,83 | p-tau | HAND2-AS1:58 | 0,71 | 0,0045 | 0,83 |
| Aβ42 | Inc-CYP2E1-1:1 | -0,74 | 0,0332 | 0,24 | T-tau | HAND2-AS1:70 | 0,8 | 0,0018 | 0,86 | p-tau | KAZN-AS1:4 | -0,73 | 0,0205 | 0,78 |
| Aβ42 | Inc-FAP-3:1 | -0,72 | 0,0083 | 0,81 | T-tau | HAND2-AS1:71 | 0,8 | 0,0036 | 0,84 | p-tau | LINC00649:23 | -0,71 | 0,0036 | 0,84 |
| Aβ42 | Inc-FGD4-9:1 | -0,73 | 0,0036 | 0,84 | T-tau | LINC00200:6 | 0,71 | 0,0029 | 0,15 | p-tau | LINC01206:11 | -0,77 | 0,0332 | 0,76 |
| Aβ42 | Inc-GLIPR1L1-2:3 | 0,72 | 0,0205 | 0,22 | T-tau | LINC00649:23 | -0,74 | 0,0036 | 0,84 | p-tau | LINC01206:20 | 0,84 | 0,0205 | 0,22 |
| Aβ42 | Inc-KDM3A-1:4 | -0,77 | 0,0045 | 0,83 | T-tau | LINC01206:11 | -0,74 | 0,0332 | 0,76 | p-tau | LINC01684:16 | 0,71 | 0,0242 | 0,77 |
| Aβ42 | Inc-LRCH1-1:1 | -0,75 | 0,0205 | 0,78 | T-tau | LINC01355:9 | 0,83 | 0,0332 | 0,76 | p-tau | LINC02246:11 | 0,74 | 0,0173 | 0,78 |
| Aβ42 | Inc-NPBWR1-2:2 | 0,81 | 0,0449 | 0,26 | T-tau | Inc-ADRB1-4:1 | 0,8 | 0,0056 | 0,83 | p-tau | LINC02345:11 | -0,75 | 0,0007 | 0,11 |
| Aβ42 | Inc-PLA2G2F-1:2 | -0,74 | 0,0121 | 0,20 | T-tau | Inc-AKR1E2-15:1 | 0,75 | 0,0205 | 0,22 | p-tau | Inc-AQP8-2:7 | -0,82 | 0,0173 | 0,22 |
| Aβ42 | Inc-POU2AF1-1:2 | -0,84 | 0,0205 | 0,78 | T-tau | Inc-APBA1-5:1 | 0,8 | 0,0284 | 0,76 | p-tau | Inc-AUH-2:9 | -0,78 | 0,0332 | 0,24 |
| Aβ42 | Inc-RCSD1-4:1 | -0,72 | 0,0387 | 0,75 | T-tau | Inc-BMS1-2:1 | 0,76 | 0,0332 | 0,24 | p-tau | Inc-CA7-2:2 | -0,79 | 0,0332 | 0,76 |
| Aβ42 | Inc-SERPINI1-14:1 | -0,74 | 0,0449 | 0,74 | T-tau | Inc-CA7-2:2 | -0,73 | 0,0332 | 0,76 | p-tau | Inc-CLK1-1:7 | -0,73 | 0,0083 | 0,81 |
| Aβ42 | Inc-TAF9-10:1 | -0,75 | 0,0284 | 0,76 | T-tau | Inc-CLK1-1:7 | -0,83 | 0,0083 | 0,81 | p-tau | Inc-ELF1-5:1 | 0,74 | 0,0242 | 0,77 |
| Aβ42 | Inc-TNFRSF19-2:1 | 0,71 | 0,0332 | 0,24 | T-tau | Inc-DAPP1-2:11 | 0,8 | 0,0242 | 0,77 | p-tau | Inc-FER1L6-2:1 | 0,83 | 0,0242 | 0,77 |
| Aβ42 | Inc-TPPP-1:2 | -0,86 | 0,0001 | 0,92 | T-tau | Inc-DNALI1-5:4 | -0,76 | 0,0045 | 0,83 | p-tau | Inc-HS3ST3A1-1:1 | 0,77 | 0,01 | 0,81 |
| Aβ42 | Inc-TPPP-1:3 | 0,8 | 0,0056 | 0,17 | T-tau | Inc-ELF1-5:1 | 0,86 | 0,0242 | 0,77 | p-tau | Inc-KCTD19-1:1 | 0,78 | 0,0332 | 0,76 |
| Aβ42 | Inc-ZNF273-4:4 | -0,87 | 0,0036 | 0,84 | T-tau | Inc-LBH-4:1 | -0,79 | 0,0332 | 0,24 | p-tau | Inc-LMBRD1-5:17 | -0,73 | 0,0387 | 0,75 |
| | | | | | T-tau | Inc-MVB12B-6:1 | 0,89 | 0,0242 | 0,77 | p-tau | Inc-MVB12B-6:1 | 0,72 | 0,0242 | 0,77 |
| | | | | | T-tau | Inc-MYO18B-2:3 | -0,72 | 0,0145 | 0,79 | p-tau | Inc-NKX6-1-2:1 | 0,79 | 0,0121 | 0,80 |
| | | | | | T-tau | Inc-OR8G5-7:2 | -0,81 | 0,0284 | 0,76 | p-tau | Inc-OR8G5-7:2 | -0,85 | 0,0284 | 0,76 |
| | | | | | T-tau | Inc-PAX8-6:2 | 0,71 | 0,0242 | 0,23 | p-tau | Inc-PACRGL-5:1 | 0,71 | 0,0332 | 0,76 |
| | | | | | T-tau | Inc-POLE4-3:1 | 0,71 | 0,0145 | 0,79 | p-tau | Inc-PRDM9-20:1 | 0,73 | 0,0173 | 0,78 |
| | | | | | T-tau | Inc-SERTM1-1:1 | -0,72 | 0,0121 | 0,80 | p-tau | Inc-RHOB-1:3 | -0,76 | 0,0205 | 0,78 |
| | | | | | T-tau | Inc-SOX14-2:1 | 0,71 | 0,0018 | 0,86 | p-tau | Inc-RPE65-4:2 | -0,79 | 0,0056 | 0,83 |
| | | | | | T-tau | Inc-SYCP1-4:1 | -0,83 | 0,0387 | 0,25 | p-tau | Inc-SERTM1-1:1 | -0,82 | 0,0121 | 0,80 |
| | | | | | T-tau | Inc-TACC2-8:6 | -0,78 | 0,0068 | 0,82 | p-tau | Inc-SOX14-2:1 | 0,76 | 0,0018 | 0,86 |
| | | | | | T-tau | Inc-TEAD4-1:1 | -0,71 | 0,0449 | 0,26 | p-tau | Inc-SPAG9-2:1 | 0,71 | 0,0173 | 0,78 |
| | | | | | T-tau | Inc-TF-4:1 | 0,82 | 0,0332 | 0,76 | p-tau | Inc-SPAG9-2:2 | 0,71 | 0,0173 | 0,78 |
| | | | | | T-tau | Inc-TTF2-4:1 | -0,8 | 0,0449 | 0,74 | p-tau | Inc-SYCP1-4:1 | -0,76 | 0,0387 | 0,25 |
| | | | | | T-tau | Inc-XXYLT1-5:1 | -0,71 | 0,0083 | 0,81 | p-tau | Inc-TACC2-8:6 | -0,75 | 0,0068 | 0,82 |
| | | | | | T-tau | Inc-ZC3H12 D-2:3 | -0,72 | 0,0173 | 0,78 | p-tau | Inc-TEKT3-3:1 | -0,84 | 0,0387 | 0,75 |
| | | | | | T-tau | Inc-ZNF430-3:4 | -0,79 | 0,01 | 0,81 | p-tau | Inc-TF-4:1 | 0,73 | 0,0332 | 0,76 |
| | | | | | T-tau | MIR99AHG:104 | 0,9 | 0,0045 | 0,83 | p-tau | Inc-TP53TG3D-2:1 | 0,71 | 0,0387 | 0,75 |
| | | | | | T-tau | PITRM1-AS1:10 | 0,91 | 0,0242 | 0,23 | p-tau | MIR99AHG:104 | 0,92 | 0,0045 | 0,83 |
| | | | | | T-tau | RORB-AS1:6 | 0,73 | 0,0056 | 0,83 | p-tau | RORB-AS1:6 | 0,81 | 0,0056 | 0,83 |
| | | | | | T-tau | SEC24B-AS1:19 | -0,93 | 0,0205 | 0,22 | p-tau | SEC24B-AS1:19 | -0,75 | 0,0205 | 0,22 |
| | | | | | T-tau | ZNF528-AS1:1 | -0,74 | 0,0387 | 0,75 | p-tau | TM4SF19-AS1:10 | 0,76 | 0,0083 | 0,19 |

To identify additional brain IncRNAs implicated specifically in brain disorders, in particular in MCI, AD, for therapeutic applications, frozen tissue samples consisting of postmortem brain mediotemporal cortex from AD patients (tissue histo-pathologically showing typical AD lesions: amyloid plaques and tangles) and from non-demented healthy control (HC) subjects (tissue devoid from AD and other lesions) were used in the experiments of deep sequencing and quantification of novel IncRNAs and the 127802 IncRNAs based on LNCipedia, a database for annotated human IncRNA transcript sequences and structures.

A total of 1091 novel IncRNAs were identified in the AD and/or HC brains. These novel 1091 IncRNAs were subsequently added in the list of the sequenced 127802 IncRNAs totalizing 128,894 IncRNAs profiled in all experiments in the brain. The 1091 novel IncRNAs are shown in Table 7.

**Table 7: The 1091 novel IncRNAs identified by the invention and fold change and p-value (differential expression in AD group versus healthy control group).**

| IncRNA | SEQ | p-value | FC | IncRNA | SEQ | p-value | FC |
|---|---|---|---|---|---|---|---|
| TCONS_00062603 | SEQ2031 | 0.00794 | <0.01 | TCONS_00000640 | SEQ1046 | 0.54762 | 1.31 |
| TCONS_00045361 | SEQ1784 | 0.00794 | 0.36 | TCONS_00045213 | SEQ1743 | 0.54762 | 1.31 |
| TCONS_00059198 | SEQ1931 | 0.00794 | 0.32 | TCONS_00008828 | SEQ1150 | 0.54762 | 1.30 |
| TCONS_00055496 | SEQ1898 | 0.00794 | 0.30 | TCONS_00066367 | SEQ2071 | 0.54762 | 1.29 |
| TCONS_00033736 | SEQ1544 | 0.00794 | 5.08 | TCONS_00040892 | SEQ1684 | 0.54762 | 1.26 |
| TCONS_00027765 | SEQ1436 | 0.00794 | 4.55 | TCONS_00021484 | SEQ1343 | 0.54762 | 1.24 |
| TCONS_00017427 | SEQ1285 | 0.01193 | 0.36 | TCONS_00062554 | SEQ1984 | 0.54762 | 1.23 |
| TCONS_00012083 | SEQ1209 | 0.01193 | 0.09 | TCONS_00027499 | SEQ1475 | 0.54762 | 1.23 |
| TCONS_00005009 | SEQ1130 | 0.01193 | 0.08 | TCONS_00050541 | SEQ1830 | 0.54762 | 1.23 |
| TCONS_00036823 | SEQ1627 | 0.01193 | 2.26 | TCONS_00062304 | SEQ1987 | 0.54762 | 1.22 |
| TCONS_00050539 | SEQ1828 | 0.01471 | 22.25 | TCONS_00026662 | SEQ1405 | 0.54762 | 1.21 |
| TCQNS_00005139 | SEQ1106 | 0.01587 | 0.34 | TCONS_00037058 | SEQ1623 | 0.54762 | 1.21 |
| TCQNS_00050178 | SEQ1833 | 0.01587 | 0.11 | TCONS_00062377 | SEQ2034 | 0.54762 | 1.19 |
| TCONS_00050218 | SEQ1850 | 0.01587 | 0.03 | TCONS_00027949 | SEQ1486 | 0.54762 | 1.18 |
| TCONS_00062555 | SEQ1991 | 0.01587 | 19.87 | TCONS_00059376 | SEQ1926 | 0.54762 | 1.15 |
| TCONS_00000634 | SEQ1045 | 0.01587 | 5.10 | TCONS_00000033 | SEQ1016 | 0.54762 | 1.15 |
| TCONS_00066462 | SEQ2061 | 0.02118 | 9.50 | TCONS_00023104 | SEQ1348 | 0.54762 | 1.13 |
| TCONS_00055487 | SEQ1896 | 0.03175 | 0.50 | TCONS_00055452 | SEQ1889 | 0.54762 | 1.12 |
| TCONS_00036834 | SEQ1628 | 0.03175 | 0.45 | TCONS_00014356 | SEQ1229 | 0.54762 | 1.12 |
| TCONS_00017409 | SEQ1280 | 0.03175 | 0.43 | TCONS_00004942 | SEQ1112 | 0.54762 | 1.10 |
| TCONS_00011974 | SEQ1218 | 0.03175 | 0.42 | TCONS_00036055 | SEQ1613 | 0.54762 | 1.08 |
| TCONS_00026584 | SEQ1418 | 0.03175 | 0.40 | TCONS_00021531 | SEQ1316 | 0.54762 | 1.06 |
| TCONS_00059199 | SEQ1932 | 0.03175 | 0.37 | TCONS_00005265 | SEQ1124 | 0.54762 | 1.05 |
| TCONS_00040994 | SEQ1713 | 0.03175 | 0.36 | TCONS_00000557 | SEQ1027 | 0.54762 | 1.02 |
| TCONS_00005325 | SEQ1141 | 0.03175 | 0.34 | TCONS_00027935 | SEQ1466 | 0.54762 | 1.00 |
| TCONS_00050200 | SEQ1841 | 0.03175 | 0.31 | TCONS_00023190 | SEQ1369 | 0.54762 | 0.97 |
| TCONS_00027438 | SEQ1450 | 0.03175 | 0.22 | TCONS_00026752 | SEQ1423 | 0.54762 | 0.95 |
| TCONS_00024916 | SEQ1404 | 0.03175 | 11.52 | TCONS_00050086 | SEQ1810 | 0.54762 | 0.92 |
| TCONS_00023309 | SEQ1355 | 0.03175 | 4.71 | TCONS_00019798 | SEQ1305 | 0.54762 | 0.92 |
| TCONS_00062602 | SEQ2030 | 0.03175 | 3.75 | TCONS_00062550 | SEQ1980 | 0.54762 | 0.91 |
| TCONS_00023240 | SEQ1383 | 0.03175 | 3.68 | TCONS_00000346 | SEQ1079 | 0.54762 | 0.91 |
| TCONS_00023401 | SEQ1386 | 0.03175 | 2.81 | TCONS_00000742 | SEQ1070 | 0.54762 | 0.90 |
| TCONS_00062306 | SEQ1989 | 0.03175 | 2.27 | TCONS_00062316 | SEQ2001 | 0.54762 | 0.89 |
| TCONS_00027556 | SEQ1495 | 0.03445 | 0.32 | TCONS_00021546 | SEQ1323 | 0.54762 | 0.87 |
| TCONS_00000570 | SEQ1032 | 0.03615 | 0.31 | TCONS_00050197 | SEQ1839 | 0.54762 | 0.83 |
| TCONS_00000571 | SEQ1033 | 0.03615 | 0.25 | TCONS_00027601 | SEQ1506 | 0.54762 | 0.80 |
| TCONS_00033653 | SEQ1548 | 0.03615 | 7.72 | TCONS_00008980 | SEQ1184 | 0.54762 | 0.78 |
| TCONS_00040878 | SEQ1680 | 0.03615 | 7.58 | TCONS_00008697 | SEQ1161 | 0.54762 | 0.76 |
| TCONS_00066358 | SEQ2068 | 0.03615 | 2.31 | TCONS_00055628 | SEQ1921 | 0.54762 | 0.76 |
| TCONS_00004915 | SEQ1109 | 0.04322 | 0.15 | TCONS_00045230 | SEQ1747 | 0.54762 | 0.76 |
| TCONS_00050234 | SEQ1855 | 0.04587 | 0.19 | TCONS_00027402 | SEQ1445 | 0.54762 | 0.75 |
| TCONS_00000601 | SEQ1037 | 0.04653 | 0.44 | TCONS_00035975 | SEQ1594 | 0.54762 | 0.75 |
| TCONS_00008841 | SEQ1158 | 0.05556 | 0.44 | TCONS_00014260 | SEQ1248 | 0.54762 | 0.74 |
| TCONS_00050014 | SEQ1803 | 0.05556 | 0.44 | TCONS_00027932 | SEQ1463 | 0.54762 | 0.74 |
| TCONS_00012062 | SEQ1202 | 0.05556 | 0.43 | TCONS_00036814 | SEQ1625 | 0.54762 | 0.73 |
| TCONS_00008942 | SEQ1177 | 0.05556 | 0.43 | TCONS_00011832 | SEQ1192 | 0.54762 | 0.68 |
| TCONS_00012009 | SEQ1185 | 0.05556 | 0.42 | TCONS_00050729 | SEQ1881 | 0.54762 | 0.68 |
| TCONS_00050606 | SEQ1861 | 0.05556 | 0.32 | TCONS_00033750 | SEQ1545 | 0.54762 | 0.67 |
| TCONS_00035031 | SEQ1583 | 0.05556 | 0.32 | TCONS_00066563 | SEQ2107 | 0.54762 | 0.65 |
| TCONS_00055481 | SEQ1894 | 0.05556 | 0.31 | TCONS_00027585 | SEQ1500 | 0.54762 | 0.60 |
| TCONS_00028007 | SEQ1498 | 0.05556 | 0.15 | TCONS_00050731 | SEQ1882 | 0.54762 | 0.60 |
| TCONS_00035091 | SEQ1565 | 0.05556 | 2.67 | TCONS_00000120 | SEQ1029 | 0.54762 | 0.59 |
| TCONS_00066501 | SEQ2066 | 0.05556 | 2.54 | TCONS_00045017 | SEQ1766 | 0.54762 | 0.57 |
| TCONS_00023133 | SEQ1357 | 0.05556 | 2.54 | TCONS_00059243 | SEQ1945 | 0.54762 | 0.57 |
| TCONS_00005141 | SEQ1108 | 0.05556 | 2.21 | TCONS_00023215 | SEQ1375 | 0.54762 | 0.53 |
| TCONS_00055535 | SEQ1909 | 0.05556 | 1.88 | TCONS_00005363 | SEQ1145 | 0.59703 | 0.56 |
| TCONS_00040949 | SEQ1706 | 0.05556 | 1.88 | TCONS_00014200 | SEQ1234 | 0.59816 | 1.89 |
| TCONS_00036895 | SEQ1643 | 0.05556 | 1.57 | TCONS_00037098 | SEQ1633 | 0.59816 | 0.74 |
| TCONS_00028131 | SEQ1522 | 0.05556 | 0.60 | TCONS_00027536 | SEQ1494 | 0.59929 | 1.58 |
| TCONS_00000237 | SEQ1052 | 0.05556 | 0.57 | TCONS_00035088 | SEQ1563 | 0.59929 | 1.48 |
| TCONS_00027683 | SEQ1521 | 0.05556 | 0.56 | TCONS_00008776 | SEQ1180 | 0.59929 | 0.71 |
| TCONS_00012063 | SEQ1203 | 0.05556 | 0.56 | TCONS_00019694 | SEQ1291 | 0.59929 | 0.70 |
| TCONS_00062422 | SEQ2040 | 0.05556 | 0.54 | TCONS_00000321 | SEQ1069 | 0.60040 | 1.81 |
| TCONS_00024801 | SEQ1392 | 0.05701 | 0.15 | TCONS_00017396 | SEQ1278 | 0.60040 | 1.63 |
| TCONS_00068994 | SEQ2110 | 0.05701 | 6.03 | TCONS_00040930 | SEQ1700 | 0.60040 | 1.50 |
| TCONS_00008747 | SEQ1175 | 0.05933 | 4.40 | TCONS_00066384 | SEQ2081 | 0.60040 | 1.49 |
| TCONS 00066312 | SEQ2053 | 0.07201 | 1.09 | TCONS_00036109 | SEQ1601 | 0.60040 | 1.47 |
| TCONS_00050655 | SEQ1875 | 0.07464 | 0.19 | TCONS_00050306 | SEQ1870 | 0.60040 | 1.18 |
| TCONS_00040935 | SEQ1702 | 0.07491 | 6.49 | TCONS_00005183 | SEQ1111 | 0.60040 | 1.16 |
| TCONS_00045332 | SEQ1775 | 0.09269 | 0.31 | TCONS_00000523 | SEQ1019 | 0.60040 | 1.01 |
| TCONS_00062739 | SEQ2052 | 0.09269 | 2.40 | TCONS_00062549 | SEQ1979 | 0.60040 | 0.80 |
| TCONS_00035032 | SEQ1585 | 0.09369 | 0.46 | TCONS_00066479 | SEQ2064 | 0.60040 | 0.77 |
| TCONS_00014419 | SEQ1250 | 0.09369 | 0.40 | TCONS_00062496 | SEQ1966 | 0.60724 | >100 |
| TCONS_00045210 | SEQ1742 | 0.09369 | 0.19 | TCONS_00027590 | SEQ1504 | 0.60724 | 10.54 |
| TCONS_00040809 | SEQ1720 | 0.09369 | 6.83 | TCONS_00000531 | SEQ1020 | 0.60724 | 2.40 |
| TCONS_00021525 | SEQ1313 | 0.09369 | 1.77 | TCONS_00028006 | SEQ1497 | 0.60724 | 1.02 |
| TCONS_00066459 | SEQ2058 | 0.09369 | 1.71 | TCONS_00040662 | SEQ1694 | 0.66231 | 1.25 |
| TCONS_00000337 | SEQ1073 | 0.09369 | 0.59 | TCONS_00040905 | SEQ1688 | 0.66843 | 1.54 |
| TCONS_00028030 | SEQ1505 | 0.09369 | 0.57 | TCONS_00008965 | SEQ1181 | 0.67040 | 2.00 |
| TCONS_00027502 | SEQ1479 | 0.09369 | 0.56 | TCONS_00014150 | SEQ1226 | 0.67040 | 1.90 |
| TCONS_00000744 | SEQ1071 | 0.09524 | 0.50 | TCONS_00062278 | SEQ1970 | 0.67137 | 0.36 |
| TCONS_00059197 | SEQ1930 | 0.09524 | 0.49 | TCONS_00008851 | SEQ1164 | 0.67137 | 0.19 |
| TCONS_00037029 | SEQ1675 | 0.09524 | 0.48 | TCONS_00062704 | SEQ2046 | 0.67137 | 1.16 |
| TCONS_00045125 | SEQ1790 | 0.09524 | 0.47 | TCONS_00055724 | SEQ1897 | 0.67137 | 0.69 |
| TCONS_00066422 | SEQ2093 | 0.09524 | 0.46 | TCONS_00050563 | SEQ1836 | 0.67234 | 2.39 |
| TCONS_00017476 | SEQ1261 | 0.09524 | 0.46 | TCONS_00044945 | SEQ1739 | 0.67234 | 1.36 |
| TCONS_00000765 | SEQ1085 | 0.09524 | 0.46 | TCONS_00005005 | SEQ1127 | 0.67234 | 0.71 |
| TCONS_00012067 | SEQ1204 | 0.09524 | 0.41 | TCONS_00019799 | SEQ1306 | 0.67424 | 1.48 |
| TCONS_00050090 | SEQ1814 | 0.09524 | 0.37 | TCONS_00050235 | SEQ1856 | 0.67424 | 1.06 |
| TCONS_00027933 | SEQ1464 | 0.09524 | 0.37 | TCONS_00008677 | SEQ1154 | 0.67424 | 0.57 |
| TCONS_00019766 | SEQ1300 | 0.09524 | 0.26 | TCONS_00059214 | SEQ1938 | 0.67517 | 2.88 |
| TCONS_00005240 | SEQ1115 | 0.09524 | 0.26 | TCONS_00059212 | SEQ1936 | 0.67517 | 2.41 |
| TCONS_00005376 | SEQ1147 | 0.09524 | 4.67 | TCONS_00017530 | SEQ1282 | 0.67517 | 1.93 |
| TCONS_00028169 | SEQ1538 | 0.09524 | 2.52 | TCONS_00000551 | SEQ1023 | 0.67517 | 1.49 |
| TCONS_00055703 | SEQ1892 | 0.09524 | 2.38 | TCONS_00027471 | SEQ1454 | 0.67517 | 0.86 |
| TCONS_00021393 | SEQ1318 | 0.09524 | 2.12 | TCONS_00027750 | SEQ1429 | 0.67517 | 0.70 |
| TCONS_00028111 | SEQ1513 | 0.09524 | 1.89 | TCONS_00027703 | SEQ1526 | 0.67517 | 0.54 |
| TCONS_00000468 | SEQ1014 | 0.09524 | 1.86 | TCONS_00036836 | SEQ1630 | 0.69048 | 0.47 |
| TCONS_00027920 | SEQ1455 | 0.09524 | 1.82 | TCONS_00062275 | SEQ1968 | 0.69048 | 0.34 |
| TCONS_00036021 | SEQ1604 | 0.09524 | 1.79 | TCONS_00050089 | SEQ1813 | 0.69048 | 0.29 |
| TCONS_00036894 | SEQ1642 | 0.09524 | 1.75 | TCONS_00017348 | SEQ1262 | 0.69048 | 2.42 |
| TCONS_00062311 | SEQ1996 | 0.09524 | 1.71 | TCONS_00044919 | SEQ1733 | 0.69048 | 2.23 |
| TCONS_00027931 | SEQ1462 | 0.09524 | 1.70 | TCONS_00023221 | SEQ1380 | 0.69048 | 1.93 |
| TCONS_00021544 | SEQ1320 | 0.09524 | 1.59 | TCONS_00023323 | SEQ1359 | 0.69048 | 1.91 |
| TCONS_00021556 | SEQ1332 | 0.09524 | 1.56 | TCONS_00027767 | SEQ1438 | 0.69048 | 1.88 |
| TCONS_00045262 | SEQ1758 | 0.09524 | 1.51 | TCONS_00036034 | SEQ1610 | 0.69048 | 1.81 |
| TCONS_00019840 | SEQ1292 | 0.09524 | 0.66 | TCONS_00028167 | SEQ1537 | 0.69048 | 1.76 |
| TCONS_00033666 | SEQ1553 | 0.09524 | 0.61 | TCONS_00012068 | SEQ1205 | 0.69048 | 1.73 |
| TCONS_00026547 | SEQ1410 | 0.09524 | 0.58 | TCONS_00023174 | SEQ1368 | 0.69048 | 1.72 |
| TCONS_00023102 | SEQ1346 | 0.09524 | 0.56 | TCONS_00028054 | SEQ1508 | 0.69048 | 1.60 |
| TCONS_00014407 | SEQ1245 | 0.09524 | 0.52 | TCONS_00062558 | SEQ1994 | 0.69048 | 1.55 |
| TCONS_00062324 | SEQ2004 | 0.09524 | 0.52 | TCONS_00045182 | SEQ1799 | 0.69048 | 1.52 |
| TCONS_00044977 | SEQ1753 | 0.11496 | 2.62 | TCONS_00027944 | SEQ1482 | 0.69048 | 1.44 |
| TCONS_00028123 | SEQ1519 | 0.11496 | 2.31 | TCONS_00040703 | SEQ1707 | 0.69048 | 1.44 |
| TCONS_00055574 | SEQ1917 | 0.11496 | 0.52 | TCONS_00028113 | SEQ1515 | 0.69048 | 1.43 |
| TCONS_00011923 | SEQ1208 | 0.11607 | 0.32 | TCONS_00045346 | SEQ1781 | 0.69048 | 1.41 |
| TCONS_00026753 | SEQ1424 | 0.11607 | 6.14 | TCONS_00000260 | SEQ1054 | 0.69048 | 1.39 |
| TCONS_00040926 | SEQ1698 | 0.11752 | 0.15 | TCONS_00055508 | SEQ1899 | 0.69048 | 1.38 |
| TCONS_00008975 | SEQ1183 | 0.13386 | 0.41 | TCONS_00050070 | SEQ1806 | 0.69048 | 1.33 |
| TCONS_00040879 | SEQ1681 | 0.13756 | 3.00 | TCONS_00035093 | SEQ1570 | 0.69048 | 1.29 |
| TCONS_00026537 | SEQ1408 | 0.13756 | 2.67 | TCONS_00005037 | SEQ1136 | 0.69048 | 1.29 |
| TCONS_00062522 | SEQ1976 | 0.13879 | 0.14 | TCONS_00000650 | SEQ1049 | 0.69048 | 1.29 |
| TCONS_00005286 | SEQ1134 | 0.13879 | 0.13 | TCONS_00027494 | SEQ1468 | 0.69048 | 1.28 |
| TCONS_00033735 | SEQ1543 | 0.13879 | 3.16 | TCONS_00066504 | SEQ2069 | 0.69048 | 1.27 |
| TCONS_00000311 | SEQ1063 | 0.13879 | 0.55 | TCONS_00008871 | SEQ1169 | 0.69048 | 1.26 |
| TCONS_00000139 | SEQ1035 | 0.14124 | 0.47 | TCONS_00027416 | SEQ1449 | 0.69048 | 1.25 |
| TCONS_00037036 | SEQ1616 | 0.14124 | 0.32 | TCONS_00062579 | SEQ2018 | 0.69048 | 1.24 |
| TCONS_00040743 | SEQ1715 | 0.14246 | 0.43 | TCONS_00014136 | SEQ1223 | 0.69048 | 1.23 |
| TCONS_00037019 | SEQ1672 | 0.14246 | 2.20 | TCONS_00021619 | SEQ1341 | 0.69048 | 1.23 |
| TCONS_00037027 | SEQ1674 | 0.14246 | 1.96 | TCONS_00000009 | SEQ1011 | 0.69048 | 1.23 |
| TCONS_00040820 | SEQ1721 | 0.14246 | 0.84 | TCONS_00040658 | SEQ1689 | 0.69048 | 1.22 |
| TCONS_00050289 | SEQ1866 | 0.14246 | 0.61 | TCONS_00059271 | SEQ1948 | 0.69048 | 1.22 |
| TCONS_00045439 | SEQ1795 | 0.14246 | 0.55 | TCONS_00036123 | SEQ1605 | 0.69048 | 1.20 |
| TCONS_00066425 | SEQ2096 | 0.15079 | 0.47 | TCONS_00000751 | SEQ1072 | 0.69048 | 1.20 |
| TCONS_00024810 | SEQ1396 | 0.15079 | 0.46 | TCONS_00027400 | SEQ1444 | 0.69048 | 1.17 |
| TCONS_00027448 | SEQ1451 | 0.15079 | 0.44 | TCONS_00045438 | SEQ1794 | 0.69048 | 1.15 |
| TCONS_00035100 | SEQ1577 | 0.15079 | 0.42 | TCONS_00062551 | SEQ1981 | 0.69048 | 1.14 |
| TCONS_00035102 | SEQ1579 | 0.15079 | 0.40 | TCONS_00012059 | SEQ1200 | 0.69048 | 1.14 |
| TCONS_00059196 | SEQ1929 | 0.15079 | 0.37 | TCONS_00033689 | SEQ1560 | 0.69048 | 1.13 |
| TCONS_00027930 | SEQ1461 | 0.15079 | 0.34 | TCONS_00050017 | SEQ1804 | 0.69048 | 1.12 |
| TCONS_00000734 | SEQ1066 | 0.15079 | 0.22 | TCONS_00036113 | SEQ1603 | 0.69048 | 1.11 |
| TCONS_00024864 | SEQ1388 | 0.15079 | 7.03 | TCONS_00004983 | SEQ1121 | 0.69048 | 1.11 |
| TCONS_00023390 | SEQ1384 | 0.15079 | 4.26 | TCONS_00033710 | SEQ1540 | 0.69048 | 1.10 |
| TCONS_00035092 | SEQ1569 | 0.15079 | 3.18 | TCONS_00040895 | SEQ1686 | 0.69048 | 1.10 |
| TCONS_00059499 | SEQ1963 | 0.15079 | 2.96 | TCONS_00014428 | SEQ1253 | 0.69048 | 1.07 |
| TCONS_00024865 | SEQ1389 | 0.15079 | 2.81 | TCONS_00005077 | SEQ1142 | 0.69048 | 1.06 |
| TCONS_00066369 | SEQ2072 | 0.15079 | 2.64 | TCONS_00059297 | SEQ1957 | 0.69048 | 1.04 |
| TCONS_00035090 | SEQ1564 | 0.15079 | 2.60 | TCONS_00036901 | SEQ1646 | 0.69048 | 1.03 |
| TCONS_00050071 | SEQ1807 | 0.15079 | 2.43 | TCONS_00055681 | SEQ1887 | 0.69048 | 1.00 |
| TCONS_00033669 | SEQ1555 | 0.15079 | 2.31 | TCONS_00050638 | SEQ1871 | 0.69048 | 0.99 |
| TCONS_00004965 | SEQ1114 | 0.15079 | 2.04 | TCONS_00062730 | SEQ2051 | 0.69048 | 0.98 |
| TCONS_00062729 | SEQ2050 | 0.15079 | 2.04 | TCONS_00005008 | SEQ1129 | 0.69048 | 0.97 |
| TCONS_00035094 | SEQ1573 | 0.15079 | 1.97 | TCONS_00023131 | SEQ1356 | 0.69048 | 0.95 |
| TCONS_00045265 | SEQ1761 | 0.15079 | 1.97 | TCONS_00040590 | SEQ1677 | 0.69048 | 0.94 |
| TCONS_00005138 | SEQ1105 | 0.15079 | 1.93 | TCONS_00023085 | SEQ1344 | 0.69048 | 0.93 |
| TCONS_00066313 | SEQ2054 | 0.15079 | 1.90 | TCONS_00062305 | SEQ1988 | 0.69048 | 0.92 |
| TCONS_00027929 | SEQ1460 | 0.15079 | 1.73 | TCONS_00000127 | SEQ1034 | 0.69048 | 0.92 |
| TCONS_00055752 | SEQ1906 | 0.15079 | 1.72 | TCONS_00062347 | SEQ2021 | 0.69048 | 0.89 |
| TCONS_00062338 | SEQ2009 | 0.15079 | 1.68 | TCONS_00036813 | SEQ1624 | 0.69048 | 0.89 |
| TCONS_00005242 | SEQ1118 | 0.15079 | 1.55 | TCONS_00017328 | SEQ1260 | 0.69048 | 0.89 |
| TCONS_00027945 | SEQ1483 | 0.15079 | 1.45 | TCONS_00036984 | SEQ1664 | 0.69048 | 0.89 |
| TCONS_00027766 | SEQ1437 | 0.15079 | 1.43 | TCONS_00008856 | SEQ1166 | 0.69048 | 0.87 |
| TCONS_00026617 | SEQ1422 | 0.15079 | 1.32 | TCONS_00004943 | SEQ1113 | 0.69048 | 0.86 |
| TCONS_00066314 | SEQ2055 | 0.15079 | 1.29 | TCONS_00062572 | SEQ2007 | 0.69048 | 0.86 |
| TCONS_00062497 | SEQ1967 | 0.15079 | 1.22 | TCONS_00037090 | SEQ1632 | 0.69048 | 0.85 |
| TCONS_00019689 | SEQ1290 | 0.15079 | 1.13 | TCONS_00036093 | SEQ1595 | 0.69048 | 0.83 |
| TCONS_00062351 | SEQ2026 | 0.15079 | 0.69 | TCONS_00066409 | SEQ2090 | 0.69048 | 0.83 |
| TCONS_00066357 | SEQ2067 | 0.15079 | 0.65 | TCONS_00019776 | SEQ1302 | 0.69048 | 0.82 |
| TCONS_00062341 | SEQ2012 | 0.15079 | 0.61 | TCONS_00026614 | SEQ1421 | 0.69048 | 0.82 |
| TCONS_00037250 | SEQ1673 | 0.15079 | 0.60 | TCONS_00059284 | SEQ1952 | 0.69048 | 0.82 |
| TCONS_00017351 | SEQ1265 | 0.15079 | 0.60 | TCONS_00035978 | SEQ1598 | 0.69048 | 0.82 |
| TCONS_00019772 | SEQ1301 | 0.15079 | 0.58 | TCONS_00044941 | SEQ1737 | 0.69048 | 0.81 |
| TCONS_00027507 | SEQ1484 | 0.15079 | 0.57 | TCONS_00000345 | SEQ1078 | 0.69048 | 0.81 |
| TCONS_00000828 | SEQ1096 | 0.15079 | 0.55 | TCONS_00033664 | SEQ1552 | 0.69048 | 0.80 |
| TCONS_00036133 | SEQ1607 | 0.15079 | 0.55 | TCONS_00062302 | SEQ1985 | 0.69048 | 0.79 |
| TCONS_00050012 | SEQ1801 | 0.15079 | 0.54 | TCONS_00035096 | SEQ1576 | 0.69048 | 0.79 |
| TCONS_00035046 | SEQ1588 | 0.15079 | 0.54 | TCONS_00000006 | SEQ1010 | 0.69048 | 0.79 |
| TCONS_00044939 | SEQ1736 | 0.15079 | 0.53 | TCONS_00024807 | SEQ1393 | 0.69048 | 0.77 |
| TCONS_00014261 | SEQ1249 | 0.15079 | 0.53 | TCONS_00000735 | SEQ1067 | 0.69048 | 0.73 |
| TCONS_00055513 | SEQ1901 | 0.15794 | 0.03 | TCONS_00050598 | SEQ1857 | 0.69048 | 0.71 |
| TCONS_00019796 | SEQ1303 | 0.16926 | 0.33 | TCONS_00014211 | SEQ1239 | 0.69048 | 0.71 |
| TCONS_00008691 | SEQ1159 | 0.17190 | 0.39 | TCONS_00027711 | SEQ1533 | 0.69048 | 0.69 |
| TCONS_00000350 | SEQ1084 | 0.17190 | 4.60 | TCONS_00059424 | SEQ1947 | 0.69048 | 0.69 |
| TCONS_00000760 | SEQ1083 | 0.17322 | 0.50 | TCONS_00017350 | SEQ1264 | 0.69048 | 0.65 |
| TCONS_00019866 | SEQ1295 | 0.17322 | 0.44 | TCONS_00044948 | SEQ1741 | 0.69048 | 0.64 |
| TCONS_00055525 | SEQ1907 | 0.17322 | 0.40 | TCONS_00050219 | SEQ1851 | 0.69048 | 0.64 |
| TCONS_00033608 | SEQ1541 | 0.17322 | 2.45 | TCONS_00033649 | SEQ1546 | 0.69048 | 0.64 |
| TCONS_00036061 | SEQ1614 | 0.17322 | 2.39 | TCONS_00066412 | SEQ2091 | 0.69048 | 0.63 |
| TCONS_00033668 | SEQ1554 | 0.17322 | 0.51 | TCONS_00019765 | SEQ1299 | 0.69048 | 0.59 |
| TCONS_00021549 | SEQ1327 | 0.17971 | >100 | TCONS_00014453 | SEQ1256 | 0.69048 | 0.59 |
| TCONS_00050263 | SEQ1860 | 0.17971 | >100 | TCONS_00000723 | SEQ1058 | 0.69048 | 0.54 |
| TCONS_00068989 | SEQ2111 | 0.17971 | >100 | TCONS_00027530 | SEQ1491 | 0.72408 | 2.29 |
| TCONS_00037221 | SEQ1667 | 0.20309 | 0.16 | TCONS_00050316 | SEQ1872 | 0.74568 | 0.27 |
| TCONS_00011831 | SEQ1191 | 0.20309 | 4.10 | TCONS_00055421 | SEQ1885 | 0.75033 | 2.82 |
| TCONS_00044961 | SEQ1746 | 0.20309 | 2.12 | TCONS_00005243 | SEQ1120 | 0.75033 | 1.39 |
| TCONS_00066547 | SEQ2099 | 0.20309 | 2.08 | TCONS_00000338 | SEQ1074 | 0.75033 | 0.78 |
| TCONS_00045056 | SEQ1773 | 0.20450 | 0.50 | TCONS_00066451 | SEQ2105 | 0.75257 | 0.48 |
| TCONS_00037177 | SEQ1657 | 0.20450 | 0.46 | TCONS_00027505 | SEQ1480 | 0.75257 | 1.29 |
| TCONS_00066513 | SEQ2077 | 0.20450 | 1.13 | TCONS_00040947 | SEQ1704 | 0.75257 | 1.14 |
| TCONS_00012089 | SEQ1210 | 0.20450 | 0.55 | TCONS_00021392 | SEQ1317 | 0.75257 | 0.69 |
| TCONS_00027715 | SEQ1535 | 0.20590 | 0.24 | TCONS_00005182 | SEQ1110 | 0.75257 | 0.69 |
| TCONS_00000536 | SEQ1021 | 0.20730 | 0.45 | TCONS_00026597 | SEQ1420 | 0.75330 | 1.73 |
| TCONS_00027498 | SEQ1474 | 0.20730 | 1.34 | TCONS_00062376 | SEQ2033 | 0.75330 | 1.43 |
| TCONS_00036797 | SEQ1621 | 0.20730 | 0.75 | TCONS_00024847 | SEQ1403 | 0.75330 | 1.25 |
| TCONS_00062437 | SEQ2044 | 0.20869 | 0.47 | TCONS_00035994 | SEQ1602 | 0.75330 | 1.23 |
| TCONS_00023224 | SEQ1381 | 0.20869 | 2.27 | TCONS_00045240 | SEQ1748 | 0.75330 | 1.15 |
| TCONS_00036792 | SEQ1620 | 0.20869 | 2.03 | TCONS_00050459 | SEQ1808 | 0.75330 | 1.13 |
| TCONS_00045156 | SEQ1796 | 0.20869 | 1.59 | TCONS_00059174 | SEQ1924 | 0.75330 | 1.09 |
| TCONS_00036933 | SEQ1655 | 0.20869 | 0.75 | TCONS_00050703 | SEQ1879 | 0.75330 | 1.04 |
| TCONS_00027513 | SEQ1487 | 0.20869 | 0.51 | TCONS_00050115 | SEQ1817 | 0.75330 | 1.00 |
| TCONS_00050460 | SEQ1809 | 0.22222 | 0.50 | TCONS_00066517 | SEQ2082 | 0.75330 | 0.96 |
| TCONS_00011972 | SEQ1217 | 0.22222 | 0.50 | TCONS_00008889 | SEQ1172 | 0.75330 | 0.95 |
| TCONS_00059439 | SEQ1956 | 0.22222 | 0.49 | TCONS_00024818 | SEQ1399 | 0.75330 | 0.88 |
| TCONS_00037143 | SEQ1649 | 0.22222 | 0.44 | TCONS_00040697 | SEQ1701 | 0.75330 | 0.85 |
| TCONS_00045093 | SEQ1782 | 0.22222 | 0.40 | TCONS_00028140 | SEQ1532 | 0.75330 | 0.82 |
| TCONS_00045133 | SEQ1792 | 0.22222 | 0.33 | TCONS_00024808 | SEQ1394 | 0.75330 | 0.78 |
| TCONS_00035101 | SEQ1578 | 0.22222 | 0.28 | TCONS_00059185 | SEQ1927 | 0.75330 | 0.76 |
| TCONS_00050087 | SEQ1811 | 0.22222 | 0.20 | TCONS_00019685 | SEQ1289 | 0.75330 | 0.70 |
| TCONS_00036899 | SEQ1644 | 0.22222 | 16.33 | TCONS_00024812 | SEQ1397 | 0.75330 | 0.64 |
| TCONS_00036100 | SEQ1600 | 0.22222 | 4.21 | TCONS_00055414 | SEQ1883 | 0.79625 | 0.32 |
| TCONS_00044916 | SEQ1729 | 0.22222 | 4.18 | TCONS_00068985 | SEQ2108 | 0.79625 | 1.50 |
| TCONS_00044921 | SEQ1734 | 0.22222 | 4.01 | TCONS_00045134 | SEQ1793 | 0.79717 | <0.01 |
| TCONS_00035021 | SEQ1566 | 0.22222 | 3.65 | TCONS_00066372 | SEQ2075 | 0.79717 | <0.01 |
| TCONS_00045203 | SEQ1731 | 0.22222 | 3.29 | TCONS_00014398 | SEQ1241 | 0.79717 | 0.41 |
| TCONS_00017370 | SEQ1271 | 0.22222 | 2.90 | TCONS_00019846 | SEQ1293 | 0.79717 | 0.28 |
| TCONS_00037224 | SEQ1668 | 0.22222 | 2.08 | TCONS_00037193 | SEQ1658 | 0.79717 | 0.09 |
| TCONS_00000357 | SEQ1087 | 0.22222 | 1.98 | TCONS_00045108 | SEQ1783 | 0.82306 | 0.56 |
| TCONS_00035024 | SEQ1571 | 0.22222 | 1.90 | TCONS_00026535 | SEQ1406 | 0.82366 | 0.70 |
| TCONS_00000204 | SEQ1044 | 0.22222 | 1.65 | TCONS_00026562 | SEQ1415 | 0.82881 | 0.67 |
| TCONS_00021407 | SEQ1326 | 0.22222 | 1.65 | TCONS_00023171 | SEQ1367 | 0.82936 | 3.24 |
| TCONS_00050176 | SEQ1831 | 0.22222 | 1.62 | TCONS_00059213 | SEQ1937 | 0.82936 | 1.57 |
| TCONS_00062700 | SEQ2045 | 0.22222 | 1.61 | TCONS_00019869 | SEQ1296 | 0.82936 | 1.50 |
| TCONS_00045048 | SEQ1772 | 0.22222 | 1.61 | TCONS_00040700 | SEQ1703 | 0.82936 | 1.29 |
| TCONS_00021555 | SEQ1331 | 0.22222 | 1.60 | TCONS_00008901 | SEQ1173 | 0.83148 | 0.32 |
| TCONS_00000202 | SEQ1042 | 0.22222 | 1.59 | TCONS_00024904 | SEQ1401 | 0.83148 | 2.25 |
| TCONS_00044996 | SEQ1755 | 0.22222 | 1.58 | TCONS_00000121 | SEQ1030 | 0.83148 | 1.17 |
| TCONS_00021554 | SEQ1330 | 0.22222 | 1.54 | TCONS_00062636 | SEQ2035 | 0.83200 | 1.11 |
| TCONS_00000809 | SEQ1090 | 0.22222 | 1.48 | TCONS_00023219 | SEQ1378 | 0.83252 | 0.32 |
| TCONS_00045266 | SEQ1762 | 0.22222 | 1.47 | TCONS_00027819 | SEQ1441 | 0.83252 | 2.35 |
| TCONS_00027936 | SEQ1469 | 0.22222 | 1.44 | TCONS_00044998 | SEQ1763 | 0.83252 | 1.57 |
| TCONS_00059492 | SEQ1962 | 0.22222 | 1.42 | TCONS_00045396 | SEQ1788 | 0.83252 | 1.32 |
| TCONS_00037052 | SEQ1619 | 0.22222 | 1.42 | TCONS_00045394 | SEQ1787 | 0.83252 | 1.23 |
| TCONS_00059234 | SEQ1942 | 0.22222 | 0.76 | TCONS_00062458 | SEQ2048 | 0.83252 | 1.18 |
| TCONS_00023148 | SEQ1363 | 0.22222 | 0.73 | TCONS_00066383 | SEQ2080 | 0.83252 | 0.78 |
| TCONS_00059245 | SEQ1946 | 0.22222 | 0.69 | TCONS_00027705 | SEQ1528 | 0.83252 | 0.71 |
| TCONS_00017373 | SEQ1274 | 0.22222 | 0.68 | TCONS_00059433 | SEQ1953 | 0.83303 | 0.12 |
| TCONS_00050224 | SEQ1854 | 0.22222 | 0.67 | TCONS_00008716 | SEQ1168 | 0.83353 | 0.49 |
| TCONS_00014393 | SEQ1240 | 0.22222 | 0.65 | TCONS_00045024 | SEQ1767 | 0.83353 | 2.93 |
| TCONS_00062299 | SEQ1977 | 0.22222 | 0.64 | TCONS_00040829 | SEQ1723 | 0.83353 | 1.89 |
| TCONS_00005308 | SEQ1139 | 0.22222 | 0.62 | TCONS_00055425 | SEQ1886 | 0.83353 | 1.37 |
| TCONS_00062317 | SEQ2002 | 0.22222 | 0.61 | TCONS_00008905 | SEQ1174 | 0.83353 | 1.35 |
| TCONS_00040718 | SEQ1711 | 0.22222 | 0.61 | TCONS_00005111 | SEQ1144 | 0.83353 | 1.15 |
| TCONS_00045364 | SEQ1785 | 0.22222 | 0.55 | TCONS_00055652 | SEQ1884 | 0.83353 | 1.13 |
| TCONS_00044917 | SEQ1732 | 0.22222 | 0.54 | TCONS_00011967 | SEQ1215 | 0.83353 | 1.12 |
| TCONS_00017354 | SEQ1268 | 0.22222 | 0.53 | TCONS_00017420 | SEQ1283 | 0.83353 | 0.91 |
| TCONS_00066426 | SEQ2097 | 0.22222 | 0.53 | TCONS_00045003 | SEQ1765 | 0.83353 | 0.78 |
| TCONS_00045333 | SEQ1776 | 0.22222 | 0.52 | TCONS_00027533 | SEQ1493 | 0.83404 | 1.18 |
| TCONS_00000341 | SEQ1075 | 0.22222 | 0.52 | TCONS_00062369 | SEQ2032 | 0.83404 | 1.14 |
| TCONS_00050220 | SEQ1852 | 0.22222 | 0.51 | TCONS_00008955 | SEQ1179 | 0.83404 | 1.06 |
| TCONS_00045227 | SEQ1745 | 0.22222 | 0.51 | TCONS_00036780 | SEQ1615 | 0.83404 | 1.03 |
| TCONS_00050127 | SEQ1820 | 0.23932 | 0.07 | TCONS_00066327 | SEQ2060 | 0.83404 | 0.92 |
| TCONS_00000692 | SEQ1055 | 0.24184 | 0.21 | TCONS_00000826 | SEQ1093 | 0.83404 | 0.83 |
| TCONS_00014277 | SEQ1254 | 0.24332 | 0.90 | TCONS_00021579 | SEQ1338 | 0.83404 | 0.80 |
| TCONS_00066518 | SEQ2084 | 0.24771 | 0.49 | TCONS_00012010 | SEQ1186 | 0.83404 | 0.65 |
| TCONS_00027340 | SEQ1435 | 0.24771 | 4.23 | TCONS_00062411 | SEQ2036 | 0.83404 | 0.64 |
| TCONS_00050549 | SEQ1835 | 0.24771 | 1.25 | TCONS_00045214 | SEQ1744 | 0.84127 | 0.33 |
| TCONS_00045466 | SEQ1798 | 0.24771 | 0.91 | TCONS_00008880 | SEQ1171 | 0.84127 | 2.77 |
| TCONS_00027787 | SEQ1439 | 0.24771 | 0.57 | TCONS_00014402 | SEQ1243 | 0.84127 | 1.82 |
| TCONS_00037103 | SEQ1635 | 0.24915 | 0.47 | TCONS_00036891 | SEQ1639 | 0.84127 | 1.66 |
| TCONS_00023136 | SEQ1358 | 0.24915 | 5.81 | TCONS_00033686 | SEQ1557 | 0.84127 | 1.60 |
| TCONS_00036799 | SEQ1622 | 0.24915 | 0.68 | TCONS_00012031 | SEQ1196 | 0.84127 | 1.54 |
| TCONS_00045086 | SEQ1780 | 0.24915 | 0.66 | TCONS_00044995 | SEQ1754 | 0.84127 | 1.47 |
| TCONS_00000556 | SEQ1026 | 0.24915 | 0.59 | TCONS_00023324 | SEQ1360 | 0.84127 | 1.40 |
| TCONS_00024823 | SEQ1400 | 0.24915 | 0.58 | TCONS_00037122 | SEQ1647 | 0.84127 | 1.39 |
| TCONS_00023237 | SEQ1382 | 0.28733 | 3.80 | TCONS_00040917 | SEQ1691 | 0.84127 | 1.35 |
| TCONS_00050217 | SEQ1849 | 0.29035 | 0.39 | TCONS_00050198 | SEQ1840 | 0.84127 | 1.33 |
| TCONS_00062303 | SEQ1986 | 0.29035 | 0.01 | TCONS_00037148 | SEQ1653 | 0.84127 | 1.32 |
| TCONS_00050615 | SEQ1865 | 0.29035 | 1.60 | TCONS_00059205 | SEQ1933 | 0.84127 | 1.31 |
| TCONS_00036878 | SEQ1636 | 0.29035 | 1.46 | TCONS_00037198 | SEQ1659 | 0.84127 | 1.31 |
| TCONS_00062432 | SEQ2043 | 0.29184 | 0.36 | TCONS_00005001 | SEQ1125 | 0.84127 | 1.30 |
| TCONS_00023255 | SEQ1385 | 0.29184 | 1.40 | TCONS_00014417 | SEQ1247 | 0.84127 | 1.29 |
| TCONS_00040948 | SEQ1705 | 0.29184 | 0.62 | TCONS_00050274 | SEQ1863 | 0.84127 | 1.28 |
| TCONS_00050540 | SEQ1829 | 0.29333 | 2.93 | TCONS_00062556 | SEQ1992 | 0.84127 | 1.27 |
| TCONS_00019817 | SEQ1310 | 0.29333 | 1.52 | TCONS_00055750 | SEQ1904 | 0.84127 | 1.25 |
| TCONS_00062492 | SEQ1965 | 0.29333 | 0.71 | TCONS_00059386 | SEQ1940 | 0.84127 | 1.22 |
| TCONS_00027901 | SEQ1453 | 0.29480 | 0.26 | TCONS_00028112 | SEQ1514 | 0.84127 | 1.22 |
| TCONS_00026536 | SEQ1407 | 0.29480 | 2.63 | TCONS_00000041 | SEQ1017 | 0.84127 | 1.21 |
| TCONS_00000424 | SEQ1094 | 0.29480 | 1.61 | TCONS_00045078 | SEQ1777 | 0.84127 | 1.21 |
| TCONS_00008797 | SEQ1182 | 0.29480 | 0.71 | TCONS_00050275 | SEQ1864 | 0.84127 | 1.18 |
| TCONS_00066544 | SEQ2098 | 0.29480 | 0.63 | TCONS_00005140 | SEQ1107 | 0.84127 | 1.18 |
| TCONS_00040973 | SEQ1709 | 0.30952 | 0.50 | TCONS_00040821 | SEQ1722 | 0.84127 | 1.17 |
| TCONS_00035104 | SEQ1582 | 0.30952 | 0.47 | TCONS_00050193 | SEQ1838 | 0.84127 | 1.17 |
| TCONS_00062518 | SEQ1972 | 0.30952 | 0.46 | TCONS_00036032 | SEQ1609 | 0.84127 | 1.17 |
| TCONS_00033687 | SEQ1558 | 0.30952 | 0.41 | TCONS_00036900 | SEQ1645 | 0.84127 | 1.16 |
| TCONS_00040951 | SEQ1708 | 0.30952 | 0.40 | TCONS_00026539 | SEQ1409 | 0.84127 | 1.16 |
| TCONS_00036892 | SEQ1640 | 0.30952 | 0.38 | TCONS_00023164 | SEQ1365 | 0.84127 | 1.16 |
| TCONS_00035976 | SEQ1596 | 0.30952 | 0.35 | TCONS_00036886 | SEQ1637 | 0.84127 | 1.15 |
| TCONS_00066533 | SEQ2088 | 0.30952 | 0.35 | TCONS_00000727 | SEQ1060 | 0.84127 | 1.15 |
| TCONS_00014406 | SEQ1244 | 0.30952 | 0.34 | TCONS_00062577 | SEQ2016 | 0.84127 | 1.14 |
| TCONS_00040803 | SEQ1719 | 0.30952 | 0.29 | TCONS_00014408 | SEQ1246 | 0.84127 | 1.14 |
| TCONS_00068990 | SEQ2112 | 0.30952 | 7.13 | TCONS_00028114 | SEQ1516 | 0.84127 | 1.13 |
| TCONS_00044915 | SEQ1728 | 0.30952 | 3.75 | TCONS_00050609 | SEQ1862 | 0.84127 | 1.13 |
| TCONS_00045202 | SEQ1730 | 0.30952 | 2.94 | TCONS_00014465 | SEQ1258 | 0.84127 | 1.12 |
| TCONS_00035025 | SEQ1572 | 0.30952 | 2.73 | TCONS_00000628 | SEQ1039 | 0.84127 | 1.12 |
| TCONS_00028005 | SEQ1496 | 0.30952 | 2.50 | TCONS_00062574 | SEQ2013 | 0.84127 | 1.12 |
| TCONS_00068991 | SEQ2113 | 0.30952 | 2.49 | TCONS_00014168 | SEQ1228 | 0.84127 | 1.12 |
| TCONS_00059210 | SEQ1935 | 0.30952 | 2.45 | TCONS_00005006 | SEQ1128 | 0.84127 | 1.11 |
| TCONS_00027492 | SEQ1459 | 0.30952 | 1.96 | TCONS_00062301 | SEQ1982 | 0.84127 | 1.11 |
| TCONS_00055571 | SEQ1915 | 0.30952 | 1.87 | TCONS_00050673 | SEQ1878 | 0.84127 | 1.11 |
| TCONS_00045041 | SEQ1769 | 0.30952 | 1.85 | TCONS_00027497 | SEQ1473 | 0.84127 | 1.11 |
| TCONS_00062340 | SEQ2011 | 0.30952 | 1.84 | TCONS_00055751 | SEQ1905 | 0.84127 | 1.10 |
| TCONS_00062276 | SEQ1969 | 0.30952 | 1.80 | TCONS_00012069 | SEQ1206 | 0.84127 | 1.10 |
| TCONS_00062557 | SEQ1993 | 0.30952 | 1.69 | TCONS_00062345 | SEQ2020 | 0.84127 | 1.09 |
| TCONS_00045252 | SEQ1752 | 0.30952 | 1.62 | TCONS_00021571 | SEQ1337 | 0.84127 | 1.09 |
| TCONS_00012112 | SEQ1214 | 0.30952 | 1.61 | TCONS_00000439 | SEQ1099 | 0.84127 | 1.08 |
| TCONS_00035027 | SEQ1575 | 0.30952 | 1.60 | TCONS_00050474 | SEQ1816 | 0.84127 | 1.06 |
| TCONS_00026721 | SEQ1417 | 0.30952 | 1.60 | TCONS_00000180 | SEQ1038 | 0.84127 | 1.06 |
| TCONS_00014391 | SEQ1237 | 0.30952 | 1.58 | TCONS_00023199 | SEQ1373 | 0.84127 | 1.05 |
| TCONS_00023372 | SEQ1374 | 0.30952 | 1.58 | TCONS_00012021 | SEQ1193 | 0.84127 | 1.05 |
| TCONS_00045309 | SEQ1771 | 0.30952 | 1.57 | TCONS_00062575 | SEQ2014 | 0.84127 | 1.04 |
| TCONS_00062327 | SEQ2005 | 0.30952 | 1.56 | TCONS_00017371 | SEQ1272 | 0.84127 | 1.02 |
| TCONS_00027493 | SEQ1467 | 0.30952 | 1.52 | TCONS_00023325 | SEQ1361 | 0.84127 | 1.01 |
| TCONS_00059139 | SEQ1923 | 0.30952 | 1.52 | TCONS_00027938 | SEQ1477 | 0.84127 | 1.01 |
| TCONS_00021559 | SEQ1333 | 0.30952 | 1.49 | TCONS_00017408 | SEQ1279 | 0.84127 | 1.00 |
| TCONS_00027495 | SEQ1471 | 0.30952 | 1.49 | TCONS_00028093 | SEQ1509 | 0.84127 | 1.00 |
| TCONS_00050207 | SEQ1846 | 0.30952 | 1.48 | TCONS_00045472 | SEQ1800 | 0.84127 | 0.99 |
| TCONS_00012060 | SEQ1201 | 0.30952 | 1.45 | TCONS_00000032 | SEQ1015 | 0.84127 | 0.99 |
| TCONS_00014141 | SEQ1224 | 0.30952 | 1.44 | TCONS_00005340 | SEQ1143 | 0.84127 | 0.98 |
| TCONS_00033703 | SEQ1561 | 0.30952 | 1.41 | TCONS_00008833 | SEQ1151 | 0.84127 | 0.95 |
| TCONS_00035095 | SEQ1574 | 0.30952 | 1.41 | TCONS_00062359 | SEQ2028 | 0.84127 | 0.95 |
| TCONS_00021427 | SEQ1335 | 0.30952 | 1.40 | TCONS_00050129 | SEQ1821 | 0.84127 | 0.93 |
| TCONS_00033615 | SEQ1542 | 0.30952 | 1.33 | TCONS_00066371 | SEQ2074 | 0.84127 | 0.92 |
| TCONS_00045264 | SEQ1760 | 0.30952 | 1.33 | TCONS_00021405 | SEQ1325 | 0.84127 | 0.91 |
| TCONS_00050203 | SEQ1842 | 0.30952 | 1.33 | TCONS_00062307 | SEQ1990 | 0.84127 | 0.90 |
| TCONS_00055690 | SEQ1890 | 0.30952 | 1.31 | TCONS_00000733 | SEQ1065 | 0.84127 | 0.89 |
| TCONS_00000437 | SEQ1097 | 0.30952 | 1.27 | TCONS_00035023 | SEQ1568 | 0.84127 | 0.89 |
| TCONS_00005137 | SEQ1104 | 0.30952 | 1.15 | TCONS_00050262 | SEQ1859 | 0.84127 | 0.89 |
| TCONS_00027501 | SEQ1478 | 0.30952 | 1.15 | TCONS_00059274 | SEQ1950 | 0.84127 | 0.88 |
| TCONS_00021372 | SEQ1311 | 0.30952 | 1.10 | TCONS_00033688 | SEQ1559 | 0.84127 | 0.88 |
| TCONS_00045398 | SEQ1789 | 0.30952 | 0.84 | TCONS_00062421 | SEQ2039 | 0.84127 | 0.86 |
| TCONS_00066424 | SEQ2095 | 0.30952 | 0.83 | TCONS_00036132 | SEQ1606 | 0.84127 | 0.85 |
| TCONS_00066441 | SEQ2102 | 0.30952 | 0.79 | TCONS_00036919 | SEQ1650 | 0.84127 | 0.84 |
| TCONS_00027951 | SEQ1488 | 0.30952 | 0.74 | TCONS_00000347 | SEQ1080 | 0.84127 | 0.83 |
| TCONS_00044932 | SEQ1735 | 0.30952 | 0.73 | TCONS_00005011 | SEQ1132 | 0.84127 | 0.81 |
| TCONS₋0001 9873 | SEQ1297 | 0.30952 | 0.73 | TCONS_00035105 | SEQ1584 | 0.84127 | 0.80 |
| TCONS_00000438 | SEQ1098 | 0.30952 | 0.72 | TCONS_00024809 | SEQ1395 | 0.84127 | 0.80 |
| TCONS_00000416 | SEQ1091 | 0.30952 | 0.71 | TCONS_00062350 | SEQ2025 | 0.84127 | 0.79 |
| TCONS_00040733 | SEQ1712 | 0.30952 | 0.71 | TCONS_00021416 | SEQ1328 | 0.84127 | 0.78 |
| TCONS_00008952 | SEQ1178 | 0.30952 | 0.70 | TCONS_00035973 | SEQ1593 | 0.84127 | 0.77 |
| TCONS_00012038 | SEQ1198 | 0.30952 | 0.70 | TCONS_00066532 | SEQ2087 | 0.84127 | 0.77 |
| TCONS_00059378 | SEQ1928 | 0.30952 | 0.69 | TCONS_00000537 | SEQ1022 | 0.84127 | 0.76 |
| TCONS_00028120 | SEQ1518 | 0.30952 | 0.68 | TCONS_00027954 | SEQ1489 | 0.84127 | 0.72 |
| TCONS_00059394 | SEQ1941 | 0.30952 | 0.67 | TCONS_00045402 | SEQ1791 | 0.84127 | 0.71 |
| TCONS_00059321 | SEQ1959 | 0.30952 | 0.67 | TCONS_00036031 | SEQ1608 | 0.84127 | 0.69 |
| TCONS_00019850 | SEQ1294 | 0.30952 | 0.66 | TCONS_00008840 | SEQ1157 | 0.84127 | 0.66 |
| TCONS_00062349 | SEQ2024 | 0.30952 | 0.65 | TCONS_00005136 | SEQ1103 | 0.84127 | 0.65 |
| TCONS_00017349 | SEQ1263 | 0.30952 | 0.64 | TCONS_00036816 | SEQ1626 | 0.84127 | 0.65 |
| TCONS_00027658 | SEQ1511 | 0.30952 | 0.63 | TCONS_00000440 | SEQ1100 | 0.84127 | 0.60 |
| TCONS_00027934 | SEQ1465 | 0.30952 | 0.60 | TCONS_00040802 | SEQ1718 | 0.84127 | 0.59 |
| TCONS_00035030 | SEQ1580 | 0.30952 | 0.56 | TCONS_00027586 | SEQ1501 | 0.84127 | 0.53 |
| TCONS_00027702 | SEQ1525 | 0.30952 | 0.54 | TCONS_00021419 | SEQ1329 | 0.91098 | 1.42 |
| TCONS_00050192 | SEQ1837 | 0.30952 | 0.54 | TCONS_00027825 | SEQ1446 | 0.91418 | 0.96 |
| TCONS_00062313 | SEQ1998 | 0.30952 | 0.52 | TCONS_00005121 | SEQ1146 | 0.91553 | 0.47 |
| TCONS₋00000002 | SEQ1009 | 0.31430 | 5.14 | TCONS_00027470 | SEQ1452 | 0.91553 | 0.45 |
| TCONS_00055627 | SEQ1920 | 0.32579 | 0.31 | TCONS_00066456 | SEQ2056 | 0.91553 | 2.45 |
| TCONS_00027725 | SEQ1536 | 0.33827 | 2.21 | TCONS_00021397 | SEQ1321 | 0.91553 | 1.52 |
| TCONS_00055542 | SEQ1911 | 0.34427 | 3.03 | TCONS_00040996 | SEQ1714 | 0.91605 | 1.11 |
| TCONS_00055450 | SEQ1888 | 0.34427 | 2.82 | TCONS_00045110 | SEQ1786 | 0.91631 | 4.00 |
| TCONS_00017439 | SEQ1286 | 0.34427 | 2.36 | TCONS_00026688 | SEQ1412 | 0.91631 | 2.52 |
| TCONS_00005307 | SEQ1138 | 0.34427 | 1.77 | TCONS_00000553 | SEQ1025 | 0.91631 | 1.52 |
| TCONS_00023357 | SEQ1370 | 0.34574 | 0.49 | TCONS_00055512 | SEQ1900 | 0.91631 | 0.96 |
| TCONS_00000147 | SEQ1036 | 0.34574 | 0.41 | TCONS_00055543 | SEQ1912 | 0.91631 | 0.96 |
| TCONS_00023193 | SEQ1372 | 0.34574 | 0.41 | TCONS_00033770 | SEQ1550 | 0.91631 | 0.94 |
| TCONS_00012126 | SEQ1220 | 0.34574 | 2.38 | TCONS_00027706 | SEQ1529 | 0.91631 | 0.91 |
| TCONS_00028014 | SEQ1499 | 0.34574 | 2.35 | TCONS_00059333 | SEQ1961 | 0.91656 | 0.41 |
| TCONS_00000427 | SEQ1095 | 0.34574 | 1.88 | TCONS_00028099 | SEQ1510 | 0.91656 | 0.38 |
| TCONS_00008852 | SEQ1165 | 0.34574 | 1.80 | TCONS_00000792 | SEQ1088 | 0.91656 | 2.28 |
| TCONS_00021588 | SEQ1339 | 0.34574 | 1.63 | TCONS_00062664 | SEQ2037 | 0.91656 | 1.67 |
| TCONS_00050505 | SEQ1823 | 0.34574 | 1.33 | TCONS_00000552 | SEQ1024 | 0.91656 | 1.34 |
| TCONS_00021547 | SEQ1324 | 0.34574 | 1.24 | TCONS_00066463 | SEQ2062 | 0.91656 | 1.34 |
| TCONS_00066397 | SEQ2083 | 0.34574 | 0.75 | TCONS_00008683 | SEQ1156 | 0.91656 | 1.32 |
| TCONS_00059405 | SEQ1944 | 0.34574 | 0.66 | TCONS_00000315 | SEQ1068 | 0.91656 | 1.27 |
| TCONS_00005302 | SEQ1137 | 0.34574 | 0.64 | TCONS_00005264 | SEQ1123 | 0.91656 | 1.20 |
| TCONS_00055591 | SEQ1918 | 0.34652 | 0.30 | TCONS_00000420 | SEQ1092 | 0.91656 | 1.10 |
| TCONS_00014196 | SEQ1232 | 0.36605 | 0.14 | TCONS_00050059 | SEQ1805 | 0.91656 | 1.10 |
| TCONS_00024813 | SEQ1398 | 0.39614 | 1.29 | TCONS_00059183 | SEQ1925 | 0.91656 | 1.08 |
| TCONS_00026636 | SEQ1425 | 0.39614 | 1.16 | TCONS_00000461 | SEQ1013 | 0.91656 | 1.01 |
| TCONS_00023192 | SEQ1371 | 0.39761 | 5.31 | TCONS_00059435 | SEQ1954 | 0.91656 | 0.99 |
| TCONS_00037244 | SEQ1670 | 0.39761 | 4.55 | TCONS_00011961 | SEQ1212 | 0.91656 | 0.95 |
| TCONS_00000122 | SEQ1031 | 0.39761 | 1.42 | TCONS_00000559 | SEQ1028 | 0.91656 | 0.92 |
| TCONS_00036983 | SEQ1663 | 0.39761 | 1.01 | TCONS_00066444 | SEQ2104 | 0.91656 | 0.87 |
| TCONS_00027697 | SEQ1524 | 0.39761 | 0.87 | TCONS_00066318 | SEQ2057 | 0.91656 | 0.61 |
| TCONS_00000305 | SEQ1062 | 0.39761 | 0.58 | TCONS_00011903 | SEQ1207 | 1.00000 | 0.50 |
| TCONS_00011954 | SEQ1211 | 0.39908 | 1.81 | TCONS_00045299 | SEQ1768 | 1.00000 | 0.48 |
| TCONS_00008860 | SEQ1167 | 0.39908 | 1.45 | TCONS_00059464 | SEQ1958 | 1.00000 | 0.32 |
| TCONS₋0001 7451 | SEQ1287 | 0.40053 | 3.43 | TCONS_00017426 | SEQ1284 | 1.00000 | 0.27 |
| TCONS_00023333 | SEQ1364 | 0.40053 | 2.91 | TCONS_00050543 | SEQ1834 | 1.00000 | 0.24 |
| TCONS_00014210 | SEQ1238 | 0.40053 | 1.59 | TCONS_00062333 | SEQ2006 | 1.00000 | 0.14 |
| TCONS_00066400 | SEQ2085 | 0.40053 | 1.43 | TCONS_00027922 | SEQ1456 | 1.00000 | 0.07 |
| TCONS_00023086 | SEQ1345 | 0.40053 | 1.20 | TCONS_00069000 | SEQ2114 | 1.00000 | 56.73 |
| TCONS_00050221 | SEQ1853 | 0.40053 | 0.78 | TCONS_00004967 | SEQ1119 | 1.00000 | 11.00 |
| TCONS_00036782 | SEQ1618 | 0.40053 | 0.61 | TCONS_00004966 | SEQ1117 | 1.00000 | 5.60 |
| TCONS_00017363 | SEQ1269 | 0.40053 | 0.57 | TCONS_00000732 | SEQ1064 | 1.00000 | 3.88 |
| TCONS_00066494 | SEQ2065 | 0.40197 | 1.89 | TCONS_00008879 | SEQ1170 | 1.00000 | 2.91 |
| TCONS_00023122 | SEQ1354 | 0.40197 | 1.71 | TCONS_00023170 | SEQ1366 | 1.00000 | 2.68 |
| TCONS_00008663 | SEQ1149 | 0.40197 | 1.40 | TCONS_00012016 | SEQ1188 | 1.00000 | 2.66 |
| TCONS_00027714 | SEQ1534 | 0.40197 | 0.90 | TCONS_00050175 | SEQ1827 | 1.00000 | 1.90 |
| TCONS_00037110 | SEQ1641 | 0.40197 | 0.74 | TCONS_00000793 | SEQ1089 | 1.00000 | 1.79 |
| TCONS_00062521 | SEQ1975 | 0.42063 | 0.46 | TCONS_00050502 | SEQ1822 | 1.00000 | 1.73 |
| TCONS_00014425 | SEQ1252 | 0.42063 | 0.43 | TCONS_00005241 | SEQ1116 | 1.00000 | 1.65 |
| TCONS_00050494 | SEQ1818 | 0.42063 | 0.14 | TCONS₋0000501 0 | SEQ1131 | 1.00000 | 1.59 |
| TCONS_00055453 | SEQ1891 | 0.42063 | 2.33 | TCONS_00000203 | SEQ1043 | 1.00000 | 1.52 |
| TCONS_00017372 | SEQ1273 | 0.42063 | 2.03 | TCONS_00021599 | SEQ1340 | 1.00000 | 1.48 |
| TCONS_00066562 | SEQ2106 | 0.42063 | 2.00 | TCONS_00062343 | SEQ2019 | 1.00000 | 1.39 |
| TCONS_00000343 | SEQ1076 | 0.42063 | 1.98 | TCONS_00040834 | SEQ1725 | 1.00000 | 1.39 |
| TCONS_00027923 | SEQ1458 | 0.42063 | 1.77 | TCONS_00000837 | SEQ1102 | 1.00000 | 1.38 |
| TCONS_00023114 | SEQ1351 | 0.42063 | 1.75 | TCONS_00062520 | SEQ1974 | 1.00000 | 1.38 |
| TCONS_00055786 | SEQ1913 | 0.42063 | 1.68 | TCONS_00055634 | SEQ1922 | 1.00000 | 1.37 |
| TCONS_00012029 | SEQ1195 | 0.42063 | 1.61 | TCONS_00036922 | SEQ1651 | 1.00000 | 1.36 |
| TCONS_00050177 | SEQ1832 | 0.42063 | 1.57 | TCONS_00050295 | SEQ1868 | 1.00000 | 1.36 |
| TCONS_00062728 | SEQ2049 | 0.42063 | 1.53 | TCONS_00021481 | SEQ1342 | 1.00000 | 1.36 |
| TCONS_00062427 | SEQ2042 | 0.42063 | 1.50 | TCONS_00024798 | SEQ1390 | 1.00000 | 1.35 |
| TCONS_00066420 | SEQ2092 | 0.42063 | 1.49 | TCONS_00036838 | SEQ1631 | 1.00000 | 1.33 |
| TCONS_00059385 | SEQ1939 | 0.42063 | 1.47 | TCONS_00050214 | SEQ1848 | 1.00000 | 1.31 |
| TCONS_00008693 | SEQ1160 | 0.42063 | 1.42 | TCONS_00014187 | SEQ1231 | 1.00000 | 1.28 |
| TCONS_00027937 | SEQ1470 | 0.42063 | 1.42 | TCONS_00062454 | SEQ2047 | 1.00000 | 1.28 |
| TCONS_00040601 | SEQ1679 | 0.42063 | 1.37 | TCONS_00017352 | SEQ1266 | 1.00000 | 1.27 |
| TCONS_00050574 | SEQ1845 | 0.42063 | 1.36 | TCONS_00035075 | SEQ1590 | 1.00000 | 1.26 |
| TCONS_00027496 | SEQ1472 | 0.42063 | 1.31 | TCONS_00012032 | SEQ1197 | 1.00000 | 1.24 |
| TCONS_00055468 | SEQ1893 | 0.42063 | 1.25 | TCONS_00036985 | SEQ1665 | 1.00000 | 1.24 |
| TCONS_00008918 | SEQ1176 | 0.42063 | 1.24 | TCONS_00000348 | SEQ1081 | 1.00000 | 1.24 |
| TCONS_00027531 | SEQ1492 | 0.42063 | 1.24 | TCONS_00000300 | SEQ1061 | 1.00000 | 1.23 |
| TCONS_00040919 | SEQ1693 | 0.42063 | 1.24 | TCONS_00004987 | SEQ1122 | 1.00000 | 1.22 |
| TCONS_00000201 | SEQ1041 | 0.42063 | 1.22 | TCONS_00026734 | SEQ1419 | 1.00000 | 1.21 |
| TCONS_00021541 | SEQ1319 | 0.42063 | 1.21 | TCONS_00050526 | SEQ1824 | 1.00000 | 1.20 |
| TCONS_00012109 | SEQ1213 | 0.42063 | 1.16 | TCONS_00011994 | SEQ1222 | 1.00000 | 1.20 |
| TCONS_00027754 | SEQ1432 | 0.42063 | 1.16 | TCONS_00008682 | SEQ1155 | 1.00000 | 1.20 |
| TCONS_00027500 | SEQ1476 | 0.42063 | 1.07 | TCONS_00026557 | SEQ1413 | 1.00000 | 1.18 |
| TCONS_00014326 | SEQ1225 | 0.42063 | 0.95 | TCONS_00019797 | SEQ1304 | 1.00000 | 1.18 |
| TCONS_00045322 | SEQ1774 | 0.42063 | 0.93 | TCONS_00045042 | SEQ1770 | 1.00000 | 1.16 |
| TCONS_00026552 | SEQ1411 | 0.42063 | 0.89 | TCONS_00011830 | SEQ1190 | 1.00000 | 1.16 |
| TCONS_00062559 | SEQ1995 | 0.42063 | 0.87 | TCONS_00037176 | SEQ1656 | 1.00000 | 1.16 |
| TCONS_00033704 | SEQ1562 | 0.42063 | 0.85 | TCONS_00041064 | SEQ1726 | 1.00000 | 1.15 |
| TCONS_00037031 | SEQ1676 | 0.42063 | 0.84 | TCONS_00017376 | SEQ1277 | 1.00000 | 1.15 |
| TCONS_00037216 | SEQ1662 | 0.42063 | 0.83 | TCONS_00037147 | SEQ1652 | 1.00000 | 1.14 |
| TCONS_00019918 | SEQ1308 | 0.42063 | 0.82 | TCONS_00033755 | SEQ1547 | 1.00000 | 1.14 |
| TCONS_00050212 | SEQ1847 | 0.42063 | 0.82 | TCONS_00017375 | SEQ1276 | 1.00000 | 1.13 |
| TCONS_00062420 | SEQ2038 | 0.42063 | 0.81 | TCONS_00005274 | SEQ1133 | 1.00000 | 1.13 |
| TCONS_00062314 | SEQ1999 | 0.42063 | 0.81 | TCONS_00037212 | SEQ1661 | 1.00000 | 1.13 |
| TCONS_00019917 | SEQ1307 | 0.42063 | 0.80 | TCONS_00036781 | SEQ1617 | 1.00000 | 1.13 |
| TCONS_00062315 | SEQ2000 | 0.42063 | 0.79 | TCONS_00036095 | SEQ1599 | 1.00000 | 1.12 |
| TCONS_00062503 | SEQ1971 | 0.42063 | 0.78 | TCONS_00037152 | SEQ1654 | 1.00000 | 1.12 |
| TCONS_00050088 | SEQ1812 | 0.42063 | 0.78 | TCONS_00027948 | SEQ1485 | 1.00000 | 1.11 |
| TCONS_00045244 | SEQ1750 | 0.42063 | 0.77 | TCONS_00066382 | SEQ2079 | 1.00000 | 1.11 |
| TCONS_00023220 | SEQ1379 | 0.42063 | 0.77 | TCONS_00050168 | SEQ1825 | 1.00000 | 1.10 |
| TCONS_00059438 | SEQ1955 | 0.42063 | 0.76 | TCONS_00008698 | SEQ1162 | 1.00000 | 1.10 |
| TCONS_00050204 | SEQ1843 | 0.42063 | 0.74 | TCONS_00037000 | SEQ1669 | 1.00000 | 1.09 |
| TCONS_00041037 | SEQ1717 | 0.42063 | 0.74 | TCONS_00062548 | SEQ1978 | 1.00000 | 1.09 |
| TCONS_00062348 | SEQ2023 | 0.42063 | 0.74 | TCONS_00040916 | SEQ1690 | 1.00000 | 1.08 |
| TCONS_00044971 | SEQ1749 | 0.42063 | 0.73 | TCONS_00062552 | SEQ1983 | 1.00000 | 1.08 |
| TCONS_00021570 | SEQ1336 | 0.42063 | 0.73 | TCONS_00014387 | SEQ1235 | 1.00000 | 1.06 |
| TCONS_00027397 | SEQ1443 | 0.42063 | 0.70 | TCONS_00059500 | SEQ1964 | 1.00000 | 1.06 |
| TCONS₋0001 7374 | SEQ1275 | 0.42063 | 0.70 | TCONS_00011969 | SEQ1216 | 1.00000 | 1.05 |
| TCONS_00024800 | SEQ1391 | 0.42063 | 0.69 | TCONS_00023276 | SEQ1347 | 1.00000 | 1.05 |
| TCONS_00045249 | SEQ1751 | 0.42063 | 0.69 | TCONS_00012043 | SEQ1199 | 1.00000 | 1.05 |
| TCONS_00035103 | SEQ1581 | 0.42063 | 0.68 | TCONS_00014237 | SEQ1242 | 1.00000 | 1.05 |
| TCONS_00027339 | SEQ1434 | 0.42063 | 0.67 | TCONS_00066440 | SEQ2101 | 1.00000 | 1.05 |
| TCONS_00014199 | SEQ1233 | 0.42063 | 0.67 | TCONS_00066373 | SEQ2076 | 1.00000 | 1.04 |
| TCONS_00066535 | SEQ2089 | 0.42063 | 0.66 | TCONS_00023115 | SEQ1352 | 1.00000 | 1.04 |
| TCONS_00026643 | SEQ1427 | 0.42063 | 0.65 | TCONS_00000349 | SEQ1082 | 1.00000 | 1.04 |
| TCONS_00066423 | SEQ2094 | 0.42063 | 0.63 | TCONS_00023218 | SEQ1377 | 1.00000 | 1.04 |
| TCONS_00037245 | SEQ1671 | 0.42063 | 0.63 | TCONS_00055486 | SEQ1895 | 1.00000 | 1.04 |
| TCONS_00027389 | SEQ1440 | 0.42063 | 0.61 | TCONS_00027760 | SEQ1433 | 1.00000 | 1.04 |
| TCONS₋0000001 0 | SEQ1012 | 0.42063 | 0.58 | TCONS_00066370 | SEQ2073 | 1.00000 | 1.04 |
| TCONS_00035977 | SEQ1597 | 0.42063 | 0.55 | TCONS_00040762 | SEQ1716 | 1.00000 | 1.03 |
| TCONS_00062426 | SEQ2041 | 0.42063 | 0.51 | TCONS_00040893 | SEQ1685 | 1.00000 | 1.03 |
| TCONS_00033768 | SEQ1549 | 0.42371 | <0.01 | TCONS_00050604 | SEQ1858 | 1.00000 | 1.03 |
| TCONS_00045267 | SEQ1764 | 0.42371 | <0.01 | TCONS_00014165 | SEQ1227 | 1.00000 | 1.03 |
| TCONS_00055749 | SEQ1903 | 0.42371 | <0.01 | TCONS_00035022 | SEQ1567 | 1.00000 | 1.03 |
| TCONS_00062367 | SEQ2029 | 0.42371 | <0.01 | TCONS_00008675 | SEQ1152 | 1.00000 | 1.02 |
| TCONS_00000657 | SEQ1051 | 0.42371 | >100 | TCONS_00008699 | SEQ1163 | 1.00000 | 1.02 |
| TCONS_00021545 | SEQ1322 | 0.42371 | >100 | TCONS_00040890 | SEQ1683 | 1.00000 | 1.02 |
| TCONS_00027410 | SEQ1448 | 0.42371 | >100 | TCONS_00000224 | SEQ1048 | 1.00000 | 1.02 |
| TCONS_00040694 | SEQ1699 | 0.42371 | >100 | TCONS_00055758 | SEQ1908 | 1.00000 | 1.02 |
| TCONS_00050293 | SEQ1867 | 0.43385 | 0.32 | TCONS_00026652 | SEQ1428 | 1.00000 | 1.02 |
| TCONS_00024843 | SEQ1402 | 0.43858 | 10.00 | TCONS_00026558 | SEQ1414 | 1.00000 | 1.02 |
| TCONS_00000344 | SEQ1077 | 0.44069 | >100 | TCONS_00062589 | SEQ2027 | 1.00000 | 1.01 |
| TCONS_00045261 | SEQ1757 | 0.44069 | >100 | TCONS_00045341 | SEQ1778 | 1.00000 | 1.01 |
| TCONS_00000836 | SEQ1101 | 0.45781 | 1.19 | TCONS_00000655 | SEQ1050 | 1.00000 | 1.01 |
| TCONS_00050345 | SEQ1876 | 0.45781 | 1.17 | TCONS_00027670 | SEQ1520 | 1.00000 | 1.01 |
| TCONS_00027963 | SEQ1490 | 0.46060 | 1.51 | TCONS_00027708 | SEQ1530 | 1.00000 | 1.00 |
| TCONS_00027394 | SEQ1442 | 0.46198 | 1.94 | TCONS_00062581 | SEQ2022 | 1.00000 | 1.00 |
| TCONS_00027745 | SEQ1539 | 0.46198 | 1.10 | TCONS_00037199 | SEQ1660 | 1.00000 | 0.99 |
| TCONS_00035112 | SEQ1587 | 0.46334 | 2.00 | TCONS_00014388 | SEQ1236 | 1.00000 | 0.99 |
| TCONS_00021382 | SEQ1312 | 0.46334 | 1.70 | TCONS_00050301 | SEQ1869 | 1.00000 | 0.99 |
| TCONS_00019813 | SEQ1309 | 0.46334 | 1.62 | TCONS_00026766 | SEQ1426 | 1.00000 | 0.98 |
| TCONS_00023106 | SEQ1349 | 0.46334 | 1.61 | TCONS_00050728 | SEQ1880 | 1.00000 | 0.97 |
| TCONS_00014185 | SEQ1230 | 0.46334 | 1.53 | TCONS_00040670 | SEQ1696 | 1.00000 | 0.97 |
| TCONS_00045175 | SEQ1797 | 0.46334 | 0.79 | TCONS_00050653 | SEQ1874 | 1.00000 | 0.97 |
| TCONS_00021388 | SEQ1314 | 0.46334 | 0.65 | TCONS_00050536 | SEQ1826 | 1.00000 | 0.96 |
| TCONS_00050126 | SEQ1819 | 0.50233 | 0.15 | TCONS_00027752 | SEQ1430 | 1.00000 | 0.96 |
| TCONS_00066442 | SEQ2103 | 0.51790 | 1.19 | TCONS_00040692 | SEQ1697 | 1.00000 | 0.96 |
| TCONS_00040618 | SEQ1682 | 0.51925 | 1.51 | TCONS_00017410 | SEQ1281 | 1.00000 | 0.96 |
| TCONS_00027637 | SEQ1507 | 0.52058 | 0.45 | TCONS_00023300 | SEQ1353 | 1.00000 | 0.96 |
| TCONS_00050646 | SEQ1873 | 0.52322 | 0.15 | TCONS_00062337 | SEQ2008 | 1.00000 | 0.96 |
| TCONS_00023144 | SEQ1362 | 0.52452 | 0.52 | TCONS_00040918 | SEQ1692 | 1.00000 | 0.95 |
| TCONS_00021528 | SEQ1315 | 0.52709 | 0.40 | TCONS_00059276 | SEQ1951 | 1.00000 | 0.95 |
| TCONS_00027689 | SEQ1523 | 0.52836 | 0.50 | TCONS_00066553 | SEQ2100 | 1.00000 | 0.95 |
| TCONS_00059396 | SEQ1943 | 0.52836 | 0.43 | TCONS_00040901 | SEQ1687 | 1.00000 | 0.95 |
| TCONS_00000296 | SEQ1059 | 0.52836 | 0.40 | TCONS_00062578 | SEQ2017 | 1.00000 | 0.95 |
| TCONS_00023262 | SEQ1387 | 0.52836 | 2.32 | TCONS_00035061 | SEQ1589 | 1.00000 | 0.95 |
| TCONS_00012120 | SEQ1219 | 0.52836 | 2.13 | TCONS_00000281 | SEQ1056 | 1.00000 | 0.95 |
| TCONS_00000773 | SEQ1086 | 0.52836 | 1.58 | TCONS_00066505 | SEQ2070 | 1.00000 | 0.94 |
| TCONS_00017460 | SEQ1259 | 0.52836 | 1.11 | TCONS_00014293 | SEQ1257 | 1.00000 | 0.94 |
| TCONS_00066515 | SEQ2078 | 0.52836 | 1.06 | TCONS_00040974 | SEQ1710 | 1.00000 | 0.94 |
| TCONS_00050346 | SEQ1877 | 0.52836 | 0.55 | TCONS_00040857 | SEQ1727 | 1.00000 | 0.94 |
| TCONS_00066404 | SEQ2086 | 0.52962 | 1.57 | TCONS_00040922 | SEQ1695 | 1.00000 | 0.94 |
| TCONS_00059427 | SEQ1949 | 0.52962 | 1.39 | TCONS_00027709 | SEQ1531 | 1.00000 | 0.94 |
| TCONS_00014431 | SEQ1255 | 0.52962 | 1.06 | TCONS_00019822 | SEQ1288 | 1.00000 | 0.94 |
| TCONS₋0001 9761 | SEQ1298 | 0.52962 | 0.98 | TCONS_00036143 | SEQ1611 | 1.00000 | 0.94 |
| TCONS_00044946 | SEQ1740 | 0.52962 | 0.90 | TCONS_00055516 | SEQ1902 | 1.00000 | 0.94 |
| TCONS_00000256 | SEQ1053 | 0.52962 | 0.75 | TCONS_00005003 | SEQ1126 | 1.00000 | 0.93 |
| TCONS_00062576 | SEQ2015 | 0.54762 | 3.63 | TCONS_00035036 | SEQ1586 | 1.00000 | 0.93 |
| TCONS_00011991 | SEQ1221 | 0.54762 | 2.36 | TCONS_00033779 | SEQ1556 | 1.00000 | 0.93 |
| TCONS_00037101 | SEQ1634 | 0.54762 | 2.17 | TCONS_00027753 | SEQ1431 | 1.00000 | 0.89 |
| TCONS_00055570 | SEQ1914 | 0.54762 | 2.00 | TCONS_00044942 | SEQ1738 | 1.00000 | 0.88 |
| TCONS_00055541 | SEQ1910 | 0.54762 | 1.90 | TCONS_00037139 | SEQ1648 | 1.00000 | 0.88 |
| TCONS_00044997 | SEQ1756 | 0.54762 | 1.58 | TCONS_00036041 | SEQ1612 | 1.00000 | 0.86 |
| TCONS_00026700 | SEQ1416 | 0.54762 | 1.56 | TCONS_00036835 | SEQ1629 | 1.00000 | 0.86 |
| TCONS_00036986 | SEQ1666 | 0.54762 | 1.55 | TCONS_00068992 | SEQ2109 | 1.00000 | 0.85 |
| TCONS_00033771 | SEQ1551 | 0.54762 | 1.54 | TCONS_00028116 | SEQ1517 | 1.00000 | 0.85 |
| TCONS_00062339 | SEQ2010 | 0.54762 | 1.54 | TCONS_00059207 | SEQ1934 | 1.00000 | 0.85 |
| TCONS_00008820 | SEQ1148 | 0.54762 | 1.54 | TCONS_00062561 | SEQ2003 | 1.00000 | 0.84 |
| TCONS_00000059 | SEQ1018 | 0.54762 | 1.46 | TCONS_00005060 | SEQ1140 | 1.00000 | 0.84 |
| TCONS_00055612 | SEQ1919 | 0.54762 | 1.46 | TCONS_00066323 | SEQ2059 | 1.00000 | 0.79 |
| TCONS_00000708 | SEQ1057 | 0.54762 | 1.45 | TCONS_00000199 | SEQ1040 | 1.00000 | 0.78 |
| TCONS_00023281 | SEQ1350 | 0.54762 | 1.44 | TCONS_00014424 | SEQ1251 | 1.00000 | 0.77 |
| TCONS_00011829 | SEQ1189 | 0.54762 | 1.43 | TCONS_00050206 | SEQ1844 | 1.00000 | 0.73 |
| TCONS_00023217 | SEQ1376 | 0.54762 | 1.41 | TCONS_00050096 | SEQ1815 | 1.00000 | 0.72 |
| TCONS_00027589 | SEQ1503 | 0.54762 | 1.39 | TCONS_00040833 | SEQ1724 | 1.00000 | 0.71 |
| TCONS_00027943 | SEQ1481 | 0.54762 | 1.36 | TCONS_00059322 | SEQ1960 | 1.00000 | 0.67 |
| TCONS_00027704 | SEQ1527 | 0.54762 | 1.35 | TCONS_00036887 | SEQ1638 | 1.00000 | 0.61 |
| TCONS_00011821 | SEQ1187 | 0.54762 | 1.34 | TCONS_00027663 | SEQ1512 | 1.00000 | 0.61 |
| TCONS_00027587 | SEQ1502 | 0.54762 | 1.33 | TCONS_00055573 | SEQ1916 | 1.00000 | 0.58 |
| TCONS_00008676 | SEQ1153 | 0.54762 | 1.33 | TCONS_00027826 | SEQ1447 | 1.00000 | 0.52 |
| TCONS_00005287 | SEQ1135 | 0.54762 | 1.32 | | | | |

Out of 1091 novel brain IncRNAs (Table 7), 492 IncRNAs showed a fold change >1.1 and 431 IncRNAs showed a fold change <0.9 and represent potential novel therapeutic candidates for silencing or to enhancing such IncRNA in the brain respectively, for treatment of brain disorder, in particular a cognitive disorder such as MCI and Alzheimer.

Out of the 1091 novel brain IncRNAs, some preferred novel IncRNA candidates for use for therapeutic applications with no or limited peripheral side effects are, for example, those novel brain IncRNAs with fold change ≤0.5 or ≥2 and p value <0.05 in brain and with low or no expression in the peripheral tissues and peripheral body fluids, such as blood.

Out of the total number of 10122 novel and IncRNAs from LINCipedia sequenced by the invention in the AD and control brains and having an expression > 5 CPM (median), 1202 IncRNAs, including 42 novel IncRNAs, which all showed a statistically significant differential expression when comparing AD brains to HC brains were identified. These 1202 IncRNAs are listed in Table 8 and represent therapeutic candidates for treatment of cognitive disorders in particular MCI and Alzheimer.

**Table 8: The 1202 IncRNAs with known sequence identified by the present invention having a deficient expression (FC≤0.84) or over-expression (FC>1.22) in human AD brain temporal cortex versus human healthy control brain temporal cortex.**

| **IncRNA** | **SEQ** | **p-value** | **FC** | **AUC** | **IncRNA** | **SEQ** | **p-value** | **FC** | **AUC** |
|---|---|---|---|---|---|---|---|---|---|
| MIR100HG:20 | SEQ2115 | 0.00794 | 0.01 | 1.00 | NIFK-AS1:24 | SEQ3168 | 0.01587 | 2.70 | 0.96 |
| Inc-CBSL-1:6 | SEQ2117 | 0.00794 | 0.04 | 1.00 | Inc-RABEP2-7:2 | SEQ3172 | 0.01587 | 2.74 | 0.96 |
| Inc-SMIM11B-4:3 | SEQ2118 | 0.00794 | 0.04 | 1.00 | Inc-ZBED4-7:1 | SEQ3173 | 0.01587 | 2.74 | 0.96 |
| CD27-AS1:18 | SEQ2123 | 0.00794 | 0.08 | 1.00 | Inc-GSG1L-1:12 | SEQ3175 | 0.01587 | 2.77 | 0.96 |
| Inc-ZRANB2-2:1 | SEQ0654 | 0.00794 | 0.09 | 1.00 | Inc-SBDS-4:11 | SEQ3176 | 0.01587 | 2.78 | 0.96 |
| MIR4500HG:2 | SEQ2125 | 0.00794 | 0.09 | 1.00 | Inc-OR4F21-3:4 | SEQ3178 | 0.01587 | 2.80 | 0.96 |
| LINC02338:2 | SEQ2127 | 0.00794 | 0.09 | 1.00 | LINC00574:9 | SEQ3179 | 0.01587 | 2.80 | 0.96 |
| Inc-GNA13-2:1 | SEQ2128 | 0.00794 | 0.10 | 1.00 | PSG8-AS1:2 | SEQ3188 | 0.01587 | 2.97 | 0.96 |
| Inc-FOXD4L6-1:5 | SEQ2129 | 0.00794 | 0.11 | 1.00 | Inc-TMEM144-3:1 | SEQ3189 | 0.01587 | 2.97 | 0.96 |
| Inc-ZNF644-1:16 | SEQ2130 | 0.00794 | 0.11 | 1.00 | Inc-RNF24-2:5 | SEQ3190 | 0.01587 | 2.97 | 0.96 |
| Inc-SYT16-4:9 | SEQ2131 | 0.00794 | 0.11 | 1.00 | Inc-ZNF236-7:1 | SEQ3191 | 0.01587 | 2.98 | 0.96 |
| THUMPD3-AS1:27 | SEQ2132 | 0.00794 | 0.12 | 1.00 | Inc-ZNF366-1:1 | SEQ3197 | 0.01587 | 3.05 | 0.96 |
| Inc-POM121L2-2:1 | SEQ2133 | 0.00794 | 0.12 | 1.00 | Inc-ZNF366-1:2 | SEQ3198 | 0.01587 | 3.05 | 0.96 |
| LY86-AS1:12 | SEQ2134 | 0.00794 | 0.13 | 1.00 | ITPKB-AS1:3 | SEQ3199 | 0.01587 | 3.06 | 0.96 |
| Inc-ZEB1-10:2 | SEQ2135 | 0.00794 | 0.14 | 1.00 | DUBR:16 | SEQ3200 | 0.01587 | 3.06 | 0.96 |
| Inc-SLC6A12-2:31 | SEQ2137 | 0.00794 | 0.15 | 1.00 | Inc-FAM71E1-2:9 | SEQ3219 | 0.01587 | 3.34 | 0.96 |
| Inc-SETSIP-2:8 | SEQ2141 | 0.00794 | 0.17 | 1.00 | Inc-ZNF131-1:24 | SEQ3221 | 0.01587 | 3.38 | 0.96 |
| Inc-ST3GAL4-10:12 | SEQ2143 | 0.00794 | 0.17 | 1.00 | XIST:8 | SEQ3227 | 0.01587 | 3.56 | 0.96 |
| Inc-DIS3-4:1 | SEQ2144 | 0.00794 | 0.18 | 1.00 | Inc-GOLGA6L6-14:1 | SEQ3228 | 0.01587 | 3.60 | 0.96 |
| Inc-PMM2-6:2 | SEQ2146 | 0.00794 | 0.18 | 1.00 | Inc-CAPN15-4:1 | SEQ3229 | 0.01587 | 3.66 | 0.96 |
| Inc-S100P-4:9 | SEQ2152 | 0.00794 | 0.20 | 1.00 | Inc-PAICS-3:5 | SEQ3230 | 0.01587 | 3.67 | 0.96 |
| Inc-KAAG1-1:1 | SEQ2154 | 0.00794 | 0.20 | 1.00 | XIST:10 | SEQ3233 | 0.01587 | 3.74 | 0.96 |
| Inc-POTEI-2:8 | SEQ2155 | 0.00794 | 0.21 | 1.00 | LINC01608:15 | SEQ3240 | 0.01587 | 4.17 | 0.96 |
| Inc-BANP-1:9 | SEQ2160 | 0.00794 | 0.23 | 1.00 | Inc-EMCN-1:1 | SEQ3241 | 0.01587 | 4.17 | 0.96 |
| Inc-ACSBG1-4:1 | SEQ2161 | 0.00794 | 0.23 | 1.00 | LINC02199:5 | SEQ3244 | 0.01587 | 4.21 | 0.96 |
| TPT1-AS1:42 | SEQ2164 | 0.00794 | 0.24 | 1.00 | Inc-ST18-4:2 | SEQ3245 | 0.01587 | 4.22 | 0.96 |
| RFPL1S:26 | SEQ2166 | 0.00794 | 0.24 | 1.00 | XIST:43 | SEQ3248 | 0.01587 | 4.46 | 0.96 |
| RFPL1S:5 | SEQ2167 | 0.00794 | 0.24 | 1.00 | LINC01608:16 | SEQ3250 | 0.01587 | 4.56 | 0.96 |
| Inc-ZMAT3-3:11 | SEQ2169 | 0.00794 | 0.24 | 1.00 | Inc-ENGASE-1:1 | SEQ3252 | 0.01587 | 4.89 | 0.96 |
| SNHG16:10 | SEQ2173 | 0.00794 | 0.25 | 1.00 | WAC-AS1:21 | SEQ3255 | 0.01587 | 5.31 | 0.96 |
| SNHG16:36 | SEQ2174 | 0.00794 | 0.25 | 1.00 | Inc-SLC38A8-4:5 | SEQ3261 | 0.01587 | 5.93 | 0.96 |
| LINC01122:9 | SEQ2175 | 0.00794 | 0.25 | 1.00 | Inc-SBDS-4:6 | SEQ3263 | 0.01587 | 6.66 | 0.96 |
| Inc-SVOP-1:1 | SEQ2179 | 0.00794 | 0.26 | 1.00 | Inc-EIF2AK3-1:6 | SEQ3267 | 0.01587 | 6.96 | 0.96 |
| Inc-ZNF644-1:29 | SEQ2180 | 0.00794 | 0.26 | 1.00 | Inc-LTBP3-2:2 | SEQ3275 | 0.01587 | 24.44 | 0.96 |
| PWRN1:28 | SEQ2187 | 0.00794 | 0.28 | 1.00 | Inc-LTBP3-2:3 | SEQ3277 | 0.01587 | 25.74 | 0.96 |
| RFPL1S:1 | SEQ2191 | 0.00794 | 0.29 | 1.00 | lnc-B3GNT5-11:1 | SEQ2120 | 0.03175 | 0.06 | 0.92 |
| LINC-PINT:16 | SEQ2192 | 0.00794 | 0.29 | 1.00 | Inc-DRICH1-3:22 | SEQ2122 | 0.03175 | 0.08 | 0.92 |
| lnc-LMF1-3:4 | SEQ2202 | 0.00794 | 0.30 | 1.00 | Inc-LAMTOR5-1:1 | SEQ2124 | 0.03175 | 0.09 | 0.92 |
| PWRN1:9 | SEQ2208 | 0.00794 | 0.31 | 1.00 | SLC26A4-AS1:17 | SEQ2136 | 0.03175 | 0.14 | 0.92 |
| ILF3-AS1:6 | SEQ2209 | 0.00794 | 0.31 | 1.00 | Inc-DGCR6-7:12 | SEQ2138 | 0.03175 | 0.16 | 0.92 |
| Inc-ACO1-10:1 | SEQ2213 | 0.00794 | 0.31 | 1.00 | LINC02389:3 | SEQ2140 | 0.03175 | 0.16 | 0.92 |
| Inc-LMF1-3:19 | SEQ2218 | 0.00794 | 0.32 | 1.00 | SNHG16:1 | SEQ2142 | 0.03175 | 0.17 | 0.92 |
| Inc-ZNF518B-2:2 | SEQ2224 | 0.00794 | 0.32 | 1.00 | Inc-SOWAHB-5:10 | SEQ2145 | 0.03175 | 0.18 | 0.92 |
| Inc-NEK6-2:2 | SEQ2226 | 0.00794 | 0.32 | 1.00 | Inc-GRAP-3:3 | SEQ2147 | 0.03175 | 0.18 | 0.92 |
| lnc-SPON2-1:2 | SEQ2237 | 0.00794 | 0.35 | 1.00 | lnc-GRAP-3:1 | SEQ2148 | 0.03175 | 0.18 | 0.92 |
| Inc-DFNA5-2:9 | SEQ2239 | 0.00794 | 0.35 | 1.00 | Inc-SRRD-1:6 | SEQ2149 | 0.03175 | 0.19 | 0.92 |
| Inc-DUSP1-2:1 | SEQ2242 | 0.00794 | 0.35 | 1.00 | Inc-POTEI-2:6 | SEQ2151 | 0.03175 | 0.20 | 0.92 |
| Inc-GABRA2-1:1 | SEQ2243 | 0.00794 | 0.36 | 1.00 | Inc-SNRPN-1:5 | SEQ2153 | 0.03175 | 0.20 | 0.92 |
| Inc-FNBP1L-2:6 | SEQ2246 | 0.00794 | 0.36 | 1.00 | DANT2:6 | SEQ2157 | 0.03175 | 0.21 | 0.92 |
| LY86-AS1:11 | SEQ2252 | 0.00794 | 0.36 | 1.00 | Inc-FBRSL1-3:6 | SEQ2162 | 0.03175 | 0.23 | 0.92 |
| SNHG8:9 | SEQ2255 | 0.00794 | 0.37 | 1.00 | Inc-GLB1L2-4:1 | SEQ2163 | 0.03175 | 0.24 | 0.92 |
| TTC28-AS1:1 | SEQ2262 | 0.00794 | 0.38 | 1.00 | LINC02009:2 | SEQ2170 | 0.03175 | 0.24 | 0.92 |
| Inc-ZCCHC7-2:24 | SEQ2279 | 0.00794 | 0.40 | 1.00 | Inc-FBXO28-1:12 | SEQ2181 | 0.03175 | 0.26 | 0.92 |
| Inc-IER5-3:1 | SEQ2286 | 0.00794 | 0.40 | 1.00 | Inc-EMP1-6:1 | SEQ2182 | 0.03175 | 0.26 | 0.92 |
| Inc-GABBR1-1:2 | SEQ2290 | 0.00794 | 0.40 | 1.00 | MRPL23-AS1:9 | SEQ2183 | 0.03175 | 0.26 | 0.92 |
| Inc-COMMD1-1:1 | SEQ2294 | 0.00794 | 0.41 | 1.00 | MAGI2-AS3:83 | SEQ2184 | 0.03175 | 0.26 | 0.92 |
| Inc-ZNF45-2:5 | SEQ2301 | 0.00794 | 0.41 | 1.00 | LINC00664:11 | SEQ2185 | 0.03175 | 0.27 | 0.92 |
| Inc-ATP6V1C1-12:1 | SEQ2310 | 0.00794 | 0.42 | 1.00 | Inc-ZNF721-4:1 | SEQ2186 | 0.03175 | 0.27 | 0.92 |
| Inc-KPNA2-6:15 | SEQ2322 | 0.00794 | 0.43 | 1.00 | lnc-NPBWR1-11:6 | SEQ2188 | 0.03175 | 0.28 | 0.92 |
| Inc-C11orf94-6:1 | SEQ2324 | 0.00794 | 0.43 | 1.00 | CADM3-AS1:3 | SEQ2189 | 0.03175 | 0.28 | 0.92 |
| Inc-COL3A1-3:1 | SEQ2332 | 0.00794 | 0.43 | 1.00 | Inc-COX7B2-1:1 | SEQ2190 | 0.03175 | 0.29 | 0.92 |
| Inc-AP3S1-4:15 | SEQ2335 | 0.00794 | 0.43 | 1.00 | LINC01476:4 | SEQ2193 | 0.03175 | 0.29 | 0.92 |
| Inc-PUM3-7:1 | SEQ2337 | 0.00794 | 0.44 | 1.00 | Inc-C14orf166-2:5 | SEQ2194 | 0.03175 | 0.30 | 0.92 |
| Inc-EML1-1:1 | SEQ2344 | 0.00794 | 0.44 | 1.00 | LINC01007:2 | SEQ2196 | 0.03175 | 0.30 | 0.92 |
| Inc-TUBB2A-1:1 | SEQ2358 | 0.00794 | 0.45 | 1.00 | LINC01007:6 | SEQ2197 | 0.03175 | 0.30 | 0.92 |
| OTUD6B-AS1:11 | SEQ2360 | 0.00794 | 0.46 | 1.00 | LINC01007:4 | SEQ2198 | 0.03175 | 0.30 | 0.92 |
| OTUD6B-AS1:9 | SEQ2361 | 0.00794 | 0.46 | 1.00 | Inc-FZD4-1:5 | SEQ2199 | 0.03175 | 0.30 | 0.92 |
| Inc-PRSS22-4:1 | SEQ2362 | 0.00794 | 0.46 | 1.00 | Inc-WNT5A-3:4 | SEQ2200 | 0.03175 | 0.30 | 0.92 |
| Inc-FNDC10-1:4 | SEQ2367 | 0.00794 | 0.46 | 1.00 | Inc-SRGAP2C-5:2 | SEQ2201 | 0.03175 | 0.30 | 0.92 |
| ATP2B1-AS1:5 | SEQ2369 | 0.00794 | 0.46 | 1.00 | MIR4500HG:1 | SEQ2203 | 0.03175 | 0.31 | 0.92 |
| Inc-C7orf77-5:1 | SEQ2370 | 0.00794 | 0.46 | 1.00 | Inc-LRCH1-1:1 | SEQ0437 | 0.03175 | 0.31 | 0.92 |
| Inc-CMTM7-2:3 | SEQ2376 | 0.00794 | 0.46 | 1.00 | Inc-LIPI-11:1 | SEQ2204 | 0.03175 | 0.31 | 0.92 |
| Inc-TEX15-4:1 | SEQ2382 | 0.00794 | 0.46 | 1.00 | LINC00507:2 | SEQ2205 | 0.03175 | 0.31 | 0.92 |
| LINC01128:37 | SEQ2391 | 0.00794 | 0.47 | 1.00 | Inc-KCNA3-4:2 | SEQ2206 | 0.03175 | 0.31 | 0.92 |
| Inc-SNAPC2-2:2 | SEQ2400 | 0.00794 | 0.48 | 1.00 | COPG2IT1:2 | SEQ2207 | 0.03175 | 0.31 | 0.92 |
| Inc-DUSP1-2:2 | SEQ2407 | 0.00794 | 0.48 | 1.00 | HTR5A-AS1:4 | SEQ2210 | 0.03175 | 0.31 | 0.92 |
| Inc-TINCR-3:1 | SEQ2415 | 0.00794 | 0.48 | 1.00 | Inc-KCNA3-4:1 | SEQ2212 | 0.03175 | 0.31 | 0.92 |
| Inc-VSTM5-1:13 | SEQ2419 | 0.00794 | 0.49 | 1.00 | Inc-APH1B-1:1 | SEQ2215 | 0.03175 | 0.31 | 0.92 |
| Inc-MACROD1-5:1 | SEQ2422 | 0.00794 | 0.49 | 1.00 | Inc-NPY5R-4:1 | SEQ0823 | 0.03175 | 0.31 | 0.92 |
| Inc-MASTL-6:1 | SEQ2430 | 0.00794 | 0.49 | 1.00 | Inc-ABCD2-4:1 | SEQ2216 | 0.03175 | 0.32 | 0.92 |
| ZNF529-AS1:14 | SEQ2434 | 0.00794 | 0.49 | 1.00 | Inc-NOL6-6:1 | SEQ2217 | 0.03175 | 0.32 | 0.92 |
| Inc-DPP7-2:2 | SEQ2436 | 0.00794 | 0.49 | 1.00 | Inc-ACOD1-5:1 | SEQ2222 | 0.03175 | 0.32 | 0.92 |
| Inc-EMC3-3:4 | SEQ2441 | 0.00794 | 0.50 | 1.00 | lnc-SIPA1L1-5:1 | SEQ2225 | 0.03175 | 0.32 | 0.92 |
| LINC00667:14 | SEQ2442 | 0.00794 | 0.50 | 1.00 | Inc-CADPS-1:1 | SEQ2227 | 0.03175 | 0.32 | 0.92 |
| Inc-SCPEP1-3:1 | SEQ2448 | 0.00794 | 0.50 | 1.00 | Inc-HDX-2:1 | SEQ2228 | 0.03175 | 0.33 | 0.92 |
| Inc-BBOX1-1:11 | SEQ2450 | 0.00794 | 0.50 | 1.00 | Inc-ZNF816-2:17 | SEQ2229 | 0.03175 | 0.33 | 0.92 |
| Inc-DFNB59-2:22 | SEQ2451 | 0.00794 | 0.50 | 1.00 | Inc-SENP6-3:11 | SEQ2230 | 0.03175 | 0.33 | 0.92 |
| Inc-MTMR2-4:1 | SEQ2456 | 0.00794 | 0.51 | 1.00 | Inc-KCNH5-3:1 | SEQ2232 | 0.03175 | 0.34 | 0.92 |
| Inc-KLHL10-2:1 | SEQ2459 | 0.00794 | 0.51 | 1.00 | Inc-TENM1-4:2 | SEQ2234 | 0.03175 | 0.34 | 0.92 |
| Inc-CCDC7-2:2 | SEQ2460 | 0.00794 | 0.51 | 1.00 | Inc-NYAP2-2:2 | SEQ2236 | 0.03175 | 0.34 | 0.92 |
| RASSF8-AS1:42 | SEQ2467 | 0.00794 | 0.51 | 1.00 | Inc-IL12RB2-1:1 | SEQ2238 | 0.03175 | 0.35 | 0.92 |
| Inc-POTEI-2:7 | SEQ2476 | 0.00794 | 0.52 | 1.00 | Inc-FAM106A-2:10 | SEQ2240 | 0.03175 | 0.35 | 0.92 |
| Inc-SLC10A7-8:1 | SEQ2484 | 0.00794 | 0.53 | 1.00 | Inc-ENC1-5:1 | SEQ2244 | 0.03175 | 0.36 | 0.92 |
| lnc-TRIML1-9:1 | SEQ2490 | 0.00794 | 0.53 | 1.00 | Inc-MYF5-2:3 | SEQ2245 | 0.03175 | 0.36 | 0.92 |
| VLDLR-AS1:21 | SEQ2501 | 0.00794 | 0.53 | 1.00 | Inc-TGS1-3:1 | SEQ2251 | 0.03175 | 0.36 | 0.92 |
| Inc-VAT1-4:1 | SEQ2511 | 0.00794 | 0.54 | 1.00 | Inc-UBE2B-2:1 | SEQ2253 | 0.03175 | 0.37 | 0.92 |
| Inc-NPIPB4-3:2 | SEQ2518 | 0.00794 | 0.54 | 1.00 | Inc-XKR4-3:1 | SEQ2254 | 0.03175 | 0.37 | 0.92 |
| CASC15:17 | SEQ2522 | 0.00794 | 0.54 | 1.00 | Inc-MPPED2-4:1 | SEQ2256 | 0.03175 | 0.37 | 0.92 |
| Inc-PCSK1N-3:3 | SEQ2523 | 0.00794 | 0.54 | 1.00 | LINC01197:31 | SEQ2258 | 0.03175 | 0.37 | 0.92 |
| Inc-MAVS-3:1 | SEQ2534 | 0.00794 | 0.55 | 1.00 | Inc-SMARCA5-4:18 | SEQ0253 | 0.03175 | 0.37 | 0.92 |
| lnc-LRRC57-1:1 | SEQ2537 | 0.00794 | 0.55 | 1.00 | MIR583HG:2 | SEQ2260 | 0.03175 | 0.38 | 0.92 |
| Inc-ESCO2-1:3 | SEQ2554 | 0.00794 | 0.56 | 1.00 | Inc-COL9A2-1:3 | SEQ2261 | 0.03175 | 0.38 | 0.92 |
| Inc-IL31RA-2:1 | SEQ2557 | 0.00794 | 0.56 | 1.00 | Inc-POM121L2-2:3 | SEQ2263 | 0.03175 | 0.38 | 0.92 |
| Inc-ATG12-3:2 | SEQ2558 | 0.00794 | 0.56 | 1.00 | Inc-SYT16-4:15 | SEQ2264 | 0.03175 | 0.38 | 0.92 |
| Inc-FGFBP3-2:3 | SEQ2559 | 0.00794 | 0.56 | 1.00 | Inc-NAA50-3:1 | SEQ2266 | 0.03175 | 0.38 | 0.92 |
| Inc-SPOPL-10:1 | SEQ2567 | 0.00794 | 0.57 | 1.00 | Inc-CTXN1-1:1 | SEQ2267 | 0.03175 | 0.38 | 0.92 |
| Inc-EIF5-2:1 | SEQ2571 | 0.00794 | 0.57 | 1.00 | Inc-DNAH9-4:1 | SEQ2270 | 0.03175 | 0.39 | 0.92 |
| Inc-RAI2-5:1 | SEQ2575 | 0.00794 | 0.57 | 1.00 | Inc-SNRPN-8:19 | SEQ2271 | 0.03175 | 0.39 | 0.92 |
| Inc-MAP1B-2:1 | SEQ2587 | 0.00794 | 0.58 | 1.00 | Inc-PKIA-3:2 | SEQ2273 | 0.03175 | 0.39 | 0.92 |
| Inc-ABTB2-4:2 | SEQ2589 | 0.00794 | 0.58 | 1.00 | Inc-CALN1-2:1 | SEQ2274 | 0.03175 | 0.39 | 0.92 |
| Inc-EIF2B1-1:1 | SEQ2595 | 0.00794 | 0.59 | 1.00 | Inc-CXorf67-1:5 | SEQ2275 | 0.03175 | 0.39 | 0.92 |
| Inc-CHD1L-5:1 | SEQ2596 | 0.00794 | 0.59 | 1.00 | Inc-CES5A-3:14 | SEQ2276 | 0.03175 | 0.39 | 0.92 |
| Inc-ERAL1-3:2 | SEQ2598 | 0.00794 | 0.59 | 1.00 | SLC8A1-AS1:1 | SEQ2277 | 0.03175 | 0.39 | 0.92 |
| Inc-ERAL1-3:4 | SEQ2599 | 0.00794 | 0.59 | 1.00 | Inc-STRAP-5:1 | SEQ2280 | 0.03175 | 0.40 | 0.92 |
| Inc-CCDC85C-2:1 | SEQ2601 | 0.00794 | 0.59 | 1.00 | KRTAP5-AS1:12 | SEQ2281 | 0.03175 | 0.40 | 0.92 |
| Inc-DUXA-4:1 | SEQ2603 | 0.00794 | 0.59 | 1.00 | Inc-FAM110C-1:11 | SEQ2282 | 0.03175 | 0.40 | 0.92 |
| Inc-CHMP2B-1:11 | SEQ0679 | 0.00794 | 0.60 | 1.00 | Inc-SOWAHB-5:3 | SEQ2283 | 0.03175 | 0.40 | 0.92 |
| Inc-SSTR2-1:2 | SEQ2604 | 0.00794 | 0.60 | 1.00 | Inc-NEUROD1-3:1 | SEQ2284 | 0.03175 | 0.40 | 0.92 |
| Inc-PAX1-5:1 | SEQ2611 | 0.00794 | 0.60 | 1.00 | Inc-SV2B-3:1 | SEQ2285 | 0.03175 | 0.40 | 0.92 |
| Inc-ASPHD2-2:2 | SEQ2612 | 0.00794 | 0.60 | 1.00 | Inc-METTL22-5:1 | SEQ2287 | 0.03175 | 0.40 | 0.92 |
| Inc-CDC42SE2-1:10 | SEQ2613 | 0.00794 | 0.60 | 1.00 | Inc-LINGO2-2:3 | SEQ2288 | 0.03175 | 0.40 | 0.92 |
| Inc-TMEM154-2:1 | SEQ2616 | 0.00794 | 0.60 | 1.00 | Inc-SPIDR-1:1 | SEQ2292 | 0.03175 | 0.40 | 0.92 |
| Inc-FEM1B-4:1 | SEQ2624 | 0.00794 | 0.61 | 1.00 | Inc-KNSTRN-2:1 | SEQ2293 | 0.03175 | 0.41 | 0.92 |
| Inc-PYGO1-1:1 | SEQ2626 | 0.00794 | 0.61 | 1.00 | Inc-TACR1-1:5 | SEQ2295 | 0.03175 | 0.41 | 0.92 |
| Inc-F2RL2-6:1 | SEQ2628 | 0.00794 | 0.61 | 1.00 | Inc-MYF5-2:4 | SEQ2296 | 0.03175 | 0.41 | 0.92 |
| LINC00205:7 | SEQ2630 | 0.00794 | 0.61 | 1.00 | Inc-SYT16-1:1 | SEQ2297 | 0.03175 | 0.41 | 0.92 |
| THUMPD3-AS1:78 | SEQ2636 | 0.00794 | 0.62 | 1.00 | Inc-FAAP100-2:1 | SEQ2299 | 0.03175 | 0.41 | 0.92 |
| Inc-SLC35F5-3:11 | SEQ2639 | 0.00794 | 0.62 | 1.00 | Inc-DLG5-5:1 | SEQ2302 | 0.03175 | 0.41 | 0.92 |
| Inc-SH3BGRL2-2:14 | SEQ2644 | 0.00794 | 0.62 | 1.00 | Inc-GTF2F2-14:1 | SEQ2304 | 0.03175 | 0.41 | 0.92 |
| Inc-NEMF-2:2 | SEQ2656 | 0.00794 | 0.63 | 1.00 | Inc-ITFG1-2:1 | SEQ2307 | 0.03175 | 0.41 | 0.92 |
| Inc-CCNG1-1:4 | SEQ2660 | 0.00794 | 0.63 | 1.00 | Inc-SYNE2-4:1 | SEQ2308 | 0.03175 | 0.42 | 0.92 |
| Inc-NEMF-2:1 | SEQ2662 | 0.00794 | 0.64 | 1.00 | Inc-CD59-2:5 | SEQ2309 | 0.03175 | 0.42 | 0.92 |
| Inc-NPR2-2:3 | SEQ2685 | 0.00794 | 0.65 | 1.00 | Inc-TNFRSF13B-2:1 | SEQ2311 | 0.03175 | 0.42 | 0.92 |
| Inc-HDGFL2-6:2 | SEQ2688 | 0.00794 | 0.65 | 1.00 | Inc-CHRM3-1:1 | SEQ2312 | 0.03175 | 0.42 | 0.92 |
| Inc-PLEKHA8-3:8 | SEQ2689 | 0.00794 | 0.65 | 1.00 | Inc-PPP5D1-1:1 | SEQ2313 | 0.03175 | 0.42 | 0.92 |
| Inc-TIMM21-5:8 | SEQ2693 | 0.00794 | 0.65 | 1.00 | Inc-ZNF674-1:12 | SEQ2314 | 0.03175 | 0.42 | 0.92 |
| Inc-EYA3-2:2 | SEQ2694 | 0.00794 | 0.65 | 1.00 | Inc-CBLN2-1:1 | SEQ2318 | 0.03175 | 0.43 | 0.92 |
| Inc-ATP6V1 C2-2:2 | SEQ2695 | 0.00794 | 0.66 | 1.00 | Inc-SCN2A-7:1 | SEQ2321 | 0.03175 | 0.43 | 0.92 |
| Inc-RBPMS2-3:1 | SEQ2697 | 0.00794 | 0.66 | 1.00 | Inc-ZSCAN2-5:11 | SEQ2323 | 0.03175 | 0.43 | 0.92 |
| Inc-ADAM30-1:1 | SEQ2698 | 0.00794 | 0.66 | 1.00 | Inc-PPFIA4-1:1 | SEQ2325 | 0.03175 | 0.43 | 0.92 |
| Inc-GPR39-9:1 | SEQ2704 | 0.00794 | 0.67 | 1.00 | Inc-XRCC5-3:1 | SEQ2326 | 0.03175 | 0.43 | 0.92 |
| Inc-ITGB8-10:1 | SEQ2706 | 0.00794 | 0.67 | 1.00 | Inc-PLEKHB2-4:3 | SEQ2327 | 0.03175 | 0.43 | 0.92 |
| Inc-ANKRD55-6:1 | SEQ2711 | 0.00794 | 0.68 | 1.00 | LINC00665:2 | SEQ2328 | 0.03175 | 0.43 | 0.92 |
| Inc-CCDC186-1:1 | SEQ2713 | 0.00794 | 0.68 | 1.00 | Inc-CTAGE6-1:1 | SEQ2329 | 0.03175 | 0.43 | 0.92 |
| Inc-YIF1A-8:3 | SEQ2715 | 0.00794 | 0.68 | 1.00 | Inc-MEST-2:1 | SEQ2333 | 0.03175 | 0.43 | 0.92 |
| Inc-NUDT3-1:3 | SEQ2725 | 0.00794 | 0.69 | 1.00 | LINC00630:5 | SEQ2334 | 0.03175 | 0.43 | 0.92 |
| Inc-NAXD-4:1 | SEQ2736 | 0.00794 | 0.70 | 1.00 | MEF2C-AS1:49 | SEQ2336 | 0.03175 | 0.44 | 0.92 |
| Inc-AMMECR1L-1:2 | SEQ2743 | 0.00794 | 0.72 | 1.00 | THAP9-AS1:24 | SEQ2338 | 0.03175 | 0.44 | 0.92 |
| Inc-ZNF718-5:1 | SEQ2744 | 0.00794 | 0.73 | 1.00 | Inc-NHS-2:1 | SEQ2339 | 0.03175 | 0.44 | 0.92 |
| Inc-FAM72D-8:1 | SEQ2753 | 0.00794 | 0.75 | 1.00 | SNHG1:49 | SEQ2340 | 0.03175 | 0.44 | 0.92 |
| DLEU2:32 | SEQ2776 | 0.00794 | 1.32 | 1.00 | Inc-POM121L2-2:2 | SEQ2341 | 0.03175 | 0.44 | 0.92 |
| Inc-FAAH2-1:9 | SEQ2785 | 0.00794 | 1.38 | 1.00 | LIMD1-AS1:5 | SEQ2342 | 0.03175 | 0.44 | 0.92 |
| Inc-NEK6-2:4 | SEQ2786 | 0.00794 | 1.38 | 1.00 | Inc-APOL5-4:1 | SEQ2343 | 0.03175 | 0.44 | 0.92 |
| Inc-NEK6-2:1 | SEQ2787 | 0.00794 | 1.38 | 1.00 | Inc-PAM-1:10 | SEQ2345 | 0.03175 | 0.44 | 0.92 |
| Inc-PTPN14-2:6 | SEQ2790 | 0.00794 | 1.40 | 1.00 | Inc-ZFP69B-1:2 | SEQ2346 | 0.03175 | 0.44 | 0.92 |
| Inc-MAFF-6:1 | SEQ2792 | 0.00794 | 1.41 | 1.00 | Inc-ZFP69B-1:1 | SEQ2347 | 0.03175 | 0.44 | 0.92 |
| Inc-ANXA2R-6:1 | SEQ2793 | 0.00794 | 1.41 | 1.00 | Inc-PDK3-1:1 | SEQ2348 | 0.03175 | 0.44 | 0.92 |
| Inc-SPATA21-4:6 | SEQ2798 | 0.00794 | 1.42 | 1.00 | Inc-KBTBD6-2:1 | SEQ2349 | 0.03175 | 0.45 | 0.92 |
| Inc-ECHDC3-8:1 | SEQ2799 | 0.00794 | 1.44 | 1.00 | Inc-VPS33B-6:1 | SEQ2350 | 0.03175 | 0.45 | 0.92 |
| Inc-RBM19-2:1 | SEQ2800 | 0.00794 | 1.45 | 1.00 | Inc-SMURF1-2:1 | SEQ2351 | 0.03175 | 0.45 | 0.92 |
| CAHM:3 | SEQ2810 | 0.00794 | 1.49 | 1.00 | Inc-CLEC18B-7:5 | SEQ2353 | 0.03175 | 0.45 | 0.92 |
| Inc-OSBPL7-3:2 | SEQ2813 | 0.00794 | 1.49 | 1.00 | Inc-UGT3A2-3:1 | SEQ0389 | 0.03175 | 0.45 | 0.92 |
| Inc-ZNF331-1:1 | SEQ2814 | 0.00794 | 1.49 | 1.00 | Inc-PTPMT1-2:1 | SEQ2354 | 0.03175 | 0.45 | 0.92 |
| Inc-ZNF124-2:4 | SEQ2822 | 0.00794 | 1.52 | 1.00 | Inc-KIF1C-1:14 | SEQ2355 | 0.03175 | 0.45 | 0.92 |
| Inc-MID1-1:14 | SEQ2829 | 0.00794 | 1.53 | 1.00 | Inc-WBP4-2:9 | SEQ2356 | 0.03175 | 0.45 | 0.92 |
| Inc-DERL3-6:1 | SEQ2835 | 0.00794 | 1.55 | 1.00 | LINC01963:3 | SEQ2357 | 0.03175 | 0.45 | 0.92 |
| Inc-NPIPA8-3:1 | SEQ2836 | 0.00794 | 1.55 | 1.00 | LINC-PINT:59 | SEQ2363 | 0.03175 | 0.46 | 0.92 |
| Inc-PIGP-6:2 | SEQ2843 | 0.00794 | 1.56 | 1.00 | Inc-NMD3-1:1 | SEQ2364 | 0.03175 | 0.46 | 0.92 |
| Inc-LRRC56-1:4 | SEQ2844 | 0.00794 | 1.57 | 1.00 | Inc-MPPE1-8:2 | SEQ2365 | 0.03175 | 0.46 | 0.92 |
| Inc-C19orf47-1:1 | SEQ2848 | 0.00794 | 1.58 | 1.00 | NIPBL-AS1:8 | SEQ2366 | 0.03175 | 0.46 | 0.92 |
| Inc-OCIAD2-3:5 | SEQ2849 | 0.00794 | 1.58 | 1.00 | Inc-HIST2H3D-1:1 | SEQ2368 | 0.03175 | 0.46 | 0.92 |
| Inc-KCNRG-1:1 | SEQ2856 | 0.00794 | 1.59 | 1.00 | Inc-ERCC4-7:1 | SEQ2371 | 0.03175 | 0.46 | 0.92 |
| Inc-ZNF169-7:4 | SEQ2857 | 0.00794 | 1.59 | 1.00 | Inc-PPP5C-1:2 | SEQ2372 | 0.03175 | 0.46 | 0.92 |
| Inc-NMS-4:1 | SEQ2859 | 0.00794 | 1.59 | 1.00 | Inc-GCGR-1:2 | SEQ2373 | 0.03175 | 0.46 | 0.92 |
| ASAP1-IT2:1 | SEQ2868 | 0.00794 | 1.61 | 1.00 | Inc-TBL1XR1-17:1 | SEQ2374 | 0.03175 | 0.46 | 0.92 |
| Inc-WDR4-2:5 | SEQ2874 | 0.00794 | 1.62 | 1.00 | Inc-AKAP12-2:1 | SEQ2375 | 0.03175 | 0.46 | 0.92 |
| Inc-MAP3K2-4:1 | SEQ2875 | 0.00794 | 1.62 | 1.00 | Inc-KHDC3L-2:3 | SEQ2377 | 0.03175 | 0.46 | 0.92 |
| LINC01000:7 | SEQ2877 | 0.00794 | 1.62 | 1.00 | Inc-RBMS2-4:1 | SEQ2378 | 0.03175 | 0.46 | 0.92 |
| Inc-C19orf47-3:1 | SEQ2881 | 0.00794 | 1.63 | 1.00 | Inc-CACNA2D1-1:1 | SEQ0897 | 0.03175 | 0.46 | 0.92 |
| Inc-ZNF778-1:10 | SEQ2882 | 0.00794 | 1.64 | 1.00 | Inc-IPO5-7:1 | SEQ0521 | 0.03175 | 0.46 | 0.92 |
| LCMT1-AS1:9 | SEQ2883 | 0.00794 | 1.64 | 1.00 | Inc-SOWAHB-5:4 | SEQ2381 | 0.03175 | 0.46 | 0.92 |
| Inc-BIN3-2:1 | SEQ2884 | 0.00794 | 1.64 | 1.00 | MIF-AS1:8 | SEQ2383 | 0.03175 | 0.46 | 0.92 |
| Inc-FABP2-1:1 | SEQ2886 | 0.00794 | 1.65 | 1.00 | Inc-NXNL1-5:1 | SEQ2384 | 0.03175 | 0.47 | 0.92 |
| Inc-WDR70-5:4 | SEQ2893 | 0.00794 | 1.67 | 1.00 | Inc-ARHGAP6-2:1 | SEQ2385 | 0.03175 | 0.47 | 0.92 |
| Inc-ILKAP-6:1 | SEQ2895 | 0.00794 | 1.68 | 1.00 | Inc-CACNB2-1:1 | SEQ2386 | 0.03175 | 0.47 | 0.92 |
| Inc-CYP2E1-13:1 | SEQ2901 | 0.00794 | 1.69 | 1.00 | Inc-FAM120C-1:1 | SEQ2388 | 0.03175 | 0.47 | 0.92 |
| lnc-ZFP36L1-2:13 | SEQ2904 | 0.00794 | 1.70 | 1.00 | Inc-CENPVL2-3:1 | SEQ2389 | 0.03175 | 0.47 | 0.92 |
| Inc-SCAF11-4:1 | SEQ2908 | 0.00794 | 1.71 | 1.00 | LINC00884:15 | SEQ2390 | 0.03175 | 0.47 | 0.92 |
| lnc-NMRAL1-4:1 | SEQ2910 | 0.00794 | 1.71 | 1.00 | Inc-LINC00890-7:1 | SEQ2393 | 0.03175 | 0.47 | 0.92 |
| Inc-MNX1-10:1 | SEQ0322 | 0.00794 | 1.72 | 1.00 | Inc-TMCC2-2:1 | SEQ2395 | 0.03175 | 0.47 | 0.92 |
| CACTIN-AS1:5 | SEQ2914 | 0.00794 | 1.73 | 1.00 | Inc-ZNF333-4:2 | SEQ2397 | 0.03175 | 0.48 | 0.92 |
| Inc-FAHD2B-1:2 | SEQ2915 | 0.00794 | 1.73 | 1.00 | PEG3-AS1:1 | SEQ2398 | 0.03175 | 0.48 | 0.92 |
| Inc-LHFPL1-2:1 | SEQ2916 | 0.00794 | 1.73 | 1.00 | Inc-APTX-3:1 | SEQ2399 | 0.03175 | 0.48 | 0.92 |
| Inc-TAF9-2:3 | SEQ2917 | 0.00794 | 1.74 | 1.00 | Inc-PSEN2-4:1 | SEQ2403 | 0.03175 | 0.48 | 0.92 |
| Inc-PAG1-4:1 | SEQ2928 | 0.00794 | 1.76 | 1.00 | Inc-CMBL-8:2 | SEQ2404 | 0.03175 | 0.48 | 0.92 |
| Inc-ARMCX4-3:1 | SEQ2939 | 0.00794 | 1.79 | 1.00 | LINC00174:21 | SEQ2406 | 0.03175 | 0.48 | 0.92 |
| Inc-ZNF852-2:3 | SEQ2944 | 0.00794 | 1.79 | 1.00 | Inc-C8B-4:1 | SEQ2410 | 0.03175 | 0.48 | 0.92 |
| Inc-STARD10-1:6 | SEQ0840 | 0.00794 | 1.80 | 1.00 | Inc-EVX1-15:1 | SEQ2412 | 0.03175 | 0.48 | 0.92 |
| Inc-SERPIND1-2:1 | SEQ2948 | 0.00794 | 1.80 | 1.00 | Inc-BICD1-1:1 | SEQ2413 | 0.03175 | 0.48 | 0.92 |
| Inc-PMM2-2:4 | SEQ2955 | 0.00794 | 1.82 | 1.00 | Inc-SESN1-5:1 | SEQ2414 | 0.03175 | 0.48 | 0.92 |
| Inc-CRYM-4:1 | SEQ2958 | 0.00794 | 1.84 | 1.00 | Inc-DTNA-5:1 | SEQ2416 | 0.03175 | 0.48 | 0.92 |
| Inc-PRR26-5:1 | SEQ2961 | 0.00794 | 1.85 | 1.00 | MIR100HG:97 | SEQ2417 | 0.03175 | 0.49 | 0.92 |
| Inc-THAP3-2:2 | SEQ2979 | 0.00794 | 1.91 | 1.00 | OIP5-AS1:4 | SEQ2423 | 0.03175 | 0.49 | 0.92 |
| Inc-NOP9-1:2 | SEQ2990 | 0.00794 | 1.94 | 1.00 | Inc-AP3S1-4:3 | SEQ2424 | 0.03175 | 0.49 | 0.92 |
| Inc-PRPF40B-1:2 | SEQ2997 | 0.00794 | 1.95 | 1.00 | Inc-SCAF4-1:2 | SEQ2425 | 0.03175 | 0.49 | 0.92 |
| Inc-SAMD11-14:1 | SEQ3001 | 0.00794 | 1.97 | 1.00 | Inc-SNRPN-8:5 | SEQ2426 | 0.03175 | 0.49 | 0.92 |
| Inc-STX8-4:1 | SEQ3002 | 0.00794 | 1.97 | 1.00 | Inc-SRPRB-1:1 | SEQ2427 | 0.03175 | 0.49 | 0.92 |
| Inc-NRDE2-3:1 | SEQ3003 | 0.00794 | 1.98 | 1.00 | Inc-SLC3A2-6:1 | SEQ2428 | 0.03175 | 0.49 | 0.92 |
| Inc-SLC29A4-5:5 | SEQ3004 | 0.00794 | 1.99 | 1.00 | Inc-TMEM185B-9:1 | SEQ2432 | 0.03175 | 0.49 | 0.92 |
| Inc-SZT2-2:3 | SEQ3006 | 0.00794 | 2.00 | 1.00 | Inc-FGF13-1:1 | SEQ2435 | 0.03175 | 0.49 | 0.92 |
| Inc-SLC39A11-1:34 | SEQ3008 | 0.00794 | 2.00 | 1.00 | Inc-CHMP2B-5:1 | SEQ2437 | 0.03175 | 0.50 | 0.92 |
| Inc-MAFF-2:1 | SEQ3013 | 0.00794 | 2.01 | 1.00 | Inc-LASP1-5:1 | SEQ2438 | 0.03175 | 0.50 | 0.92 |
| Inc-ATP6V0E2-1:1 | SEQ3014 | 0.00794 | 2.01 | 1.00 | Inc-DAPP1-8:3 | SEQ2439 | 0.03175 | 0.50 | 0.92 |
| Inc-PHB2-7:1 | SEQ3018 | 0.00794 | 2.02 | 1.00 | SOX1-OT:5 | SEQ2440 | 0.03175 | 0.50 | 0.92 |
| Inc-AGRP-1:26 | SEQ3020 | 0.00794 | 2.04 | 1.00 | Inc-AADACL2-4:1 | SEQ2443 | 0.03175 | 0.50 | 0.92 |
| Inc-PPARA-3:13 | SEQ3022 | 0.00794 | 2.04 | 1.00 | SLC8A1-AS1:32 | SEQ2444 | 0.03175 | 0.50 | 0.92 |
| Inc-USP6-2:20 | SEQ3027 | 0.00794 | 2.05 | 1.00 | SNHG6:10 | SEQ2446 | 0.03175 | 0.50 | 0.92 |
| Inc-CANX-1:10 | SEQ3029 | 0.00794 | 2.06 | 1.00 | Inc-SLC25A3-7:1 | SEQ2447 | 0.03175 | 0.50 | 0.92 |
| Inc-ZMYND19-2:1 | SEQ3032 | 0.00794 | 2.06 | 1.00 | Inc-GBP2-1:2 | SEQ2449 | 0.03175 | 0.50 | 0.92 |
| Inc-DHRS7B-1:6 | SEQ3034 | 0.00794 | 2.06 | 1.00 | Inc-MRPS14-1:6 | SEQ2452 | 0.03175 | 0.50 | 0.92 |
| Inc-LEF1-3:13 | SEQ3036 | 0.00794 | 2.07 | 1.00 | Inc-FZD4-1:6 | SEQ2454 | 0.03175 | 0.51 | 0.92 |
| SMCR5:1 | SEQ3037 | 0.00794 | 2.07 | 1.00 | Inc-ZNF275-4:1 | SEQ2455 | 0.03175 | 0.51 | 0.92 |
| SRP14-AS1:20 | SEQ3039 | 0.00794 | 2.08 | 1.00 | Inc-CIB1-1:1 | SEQ2457 | 0.03175 | 0.51 | 0.92 |
| Inc-ERMARD-2:6 | SEQ3042 | 0.00794 | 2.09 | 1.00 | Inc-TMEM57-1:2 | SEQ2461 | 0.03175 | 0.51 | 0.92 |
| Inc-C8orf48-7:1 | SEQ3046 | 0.00794 | 2.10 | 1.00 | Inc-FOXM1-1:1 | SEQ2462 | 0.03175 | 0.51 | 0.92 |
| Inc-PPARA-3:7 | SEQ3050 | 0.00794 | 2.12 | 1.00 | Inc-CKAP2L-2:1 | SEQ2463 | 0.03175 | 0.51 | 0.92 |
| Inc-MISP3-5:1 | SEQ3052 | 0.00794 | 2.13 | 1.00 | RNF219-AS1:17 | SEQ2464 | 0.03175 | 0.51 | 0.92 |
| Inc-NAV1-9:6 | SEQ3053 | 0.00794 | 2.13 | 1.00 | Inc-DEFB115-5:2 | SEQ2465 | 0.03175 | 0.51 | 0.92 |
| Inc-HEATR4-5:1 | SEQ3063 | 0.00794 | 2.16 | 1.00 | Inc-POLD3-1:2 | SEQ2469 | 0.03175 | 0.52 | 0.92 |
| Inc-TEF-1:1 | SEQ3075 | 0.00794 | 2.21 | 1.00 | Inc-ZNF587-2:1 | SEQ2470 | 0.03175 | 0.52 | 0.92 |
| Inc-ZNF541-4:1 | SEQ3078 | 0.00794 | 2.21 | 1.00 | Inc-CENPM-4:1 | SEQ2475 | 0.03175 | 0.52 | 0.92 |
| HDAC2-AS2:10 | SEQ3080 | 0.00794 | 2.22 | 1.00 | Inc-MTA3-7:2 | SEQ2477 | 0.03175 | 0.52 | 0.92 |
| Inc-CAND2-2:5 | SEQ3083 | 0.00794 | 2.23 | 1.00 | Inc-GPR180-8:1 | SEQ2478 | 0.03175 | 0.52 | 0.92 |
| Inc-CAND2-2:6 | SEQ3084 | 0.00794 | 2.23 | 1.00 | Inc-DNAL1-1:1 | SEQ2480 | 0.03175 | 0.52 | 0.92 |
| Inc-CD40LG-2:9 | SEQ3085 | 0.00794 | 2.24 | 1.00 | Inc-SUMF1-18:1 | SEQ2482 | 0.03175 | 0.52 | 0.92 |
| Inc-PHYHD1-1:1 | SEQ3086 | 0.00794 | 2.24 | 1.00 | Inc-BAZ1A-2:1 | SEQ2485 | 0.03175 | 0.53 | 0.92 |
| Inc-PDYN-1:9 | SEQ3089 | 0.00794 | 2.26 | 1.00 | Inc-POTEI-2:11 | SEQ2488 | 0.03175 | 0.53 | 0.92 |
| Inc-MB-6:1 | SEQ3094 | 0.00794 | 2.27 | 1.00 | Inc-AACS-5:1 | SEQ2489 | 0.03175 | 0.53 | 0.92 |
| Inc-DNAL4-4:1 | SEQ3095 | 0.00794 | 2.28 | 1.00 | Inc-MOCS1-3:1 | SEQ2491 | 0.03175 | 0.53 | 0.92 |
| Inc-CTXN1-6:1 | SEQ3096 | 0.00794 | 2.28 | 1.00 | Inc-TFRC-7:2 | SEQ2492 | 0.03175 | 0.53 | 0.92 |
| Inc-TUBA1C-1:14 | SEQ3099 | 0.00794 | 2.29 | 1.00 | Inc-PSMC5-4:1 | SEQ2496 | 0.03175 | 0.53 | 0.92 |
| Inc-SHC3-5:3 | SEQ3102 | 0.00794 | 2.30 | 1.00 | Inc-CCDC125-1:6 | SEQ2497 | 0.03175 | 0.53 | 0.92 |
| Inc-SNRNP35-2:1 | SEQ3103 | 0.00794 | 2.30 | 1.00 | Inc-NDUFAB1-1:1 | SEQ2498 | 0.03175 | 0.53 | 0.92 |
| ATXN2-AS:5 | SEQ3104 | 0.00794 | 2.30 | 1.00 | Inc-ZNF41-2:1 | SEQ2499 | 0.03175 | 0.53 | 0.92 |
| Inc-CHRNE-4:1 | SEQ3106 | 0.00794 | 2.32 | 1.00 | Inc-C12orf40-1:1 | SEQ2500 | 0.03175 | 0.53 | 0.92 |
| Inc-MEGF6-2:1 | SEQ3107 | 0.00794 | 2.32 | 1.00 | Inc-EXTL3-2:2 | SEQ2502 | 0.03175 | 0.53 | 0.92 |
| Inc-CCDC146-4:6 | SEQ3108 | 0.00794 | 2.32 | 1.00 | Inc-ZFYVE9-2:2 | SEQ2503 | 0.03175 | 0.53 | 0.92 |
| Inc-UBE2H-2:3 | SEQ3112 | 0.00794 | 2.32 | 1.00 | Inc-PXN-1:1 | SEQ2506 | 0.03175 | 0.53 | 0.92 |
| Inc-SLC25A6-4:1 | SEQ3114 | 0.00794 | 2.32 | 1.00 | Inc-RPIA-1:4 | SEQ2507 | 0.03175 | 0.54 | 0.92 |
| Inc-POFUT2-5:1 | SEQ3120 | 0.00794 | 2.37 | 1.00 | Inc-REL-1:3 | SEQ2508 | 0.03175 | 0.54 | 0.92 |
| Inc-GNG13-5:1 | SEQ3121 | 0.00794 | 2.37 | 1.00 | Inc-SBDS-19:2 | SEQ2509 | 0.03175 | 0.54 | 0.92 |
| Inc-SEC24A-1:1 | SEQ3126 | 0.00794 | 2.41 | 1.00 | Inc-GFOD1-2:1 | SEQ2510 | 0.03175 | 0.54 | 0.92 |
| Inc-USP34-1:10 | SEQ3127 | 0.00794 | 2.42 | 1.00 | Inc-ZNF33B-6:1 | SEQ0394 | 0.03175 | 0.54 | 0.92 |
| Inc-RDH13-4:1 | SEQ3134 | 0.00794 | 2.44 | 1.00 | Inc-TACSTD2-2:4 | SEQ0020 | 0.03175 | 0.54 | 0.92 |
| Inc-PHB2-6:1 | SEQ3135 | 0.00794 | 2.45 | 1.00 | Inc-YTHDF3-6:3 | SEQ2514 | 0.03175 | 0.54 | 0.92 |
| Inc-DPH1-1:2 | SEQ3136 | 0.00794 | 2.45 | 1.00 | Inc-YTHDF3-6:1 | SEQ2515 | 0.03175 | 0.54 | 0.92 |
| Inc-BAZ1A-4:1 | SEQ3138 | 0.00794 | 2.46 | 1.00 | Inc-CENPV-2:1 | SEQ2516 | 0.03175 | 0.54 | 0.92 |
| Inc-PDE6B-1:11 | SEQ3140 | 0.00794 | 2.47 | 1.00 | Inc-DUSP1-6:1 | SEQ2517 | 0.03175 | 0.54 | 0.92 |
| Inc-SBDS-4:9 | SEQ3145 | 0.00794 | 2.50 | 1.00 | Inc-MOV10-2:1 | SEQ2519 | 0.03175 | 0.54 | 0.92 |
| Inc-PAXIP1-3:2 | SEQ3147 | 0.00794 | 2.50 | 1.00 | Inc-SHC3-1:2 | SEQ2520 | 0.03175 | 0.54 | 0.92 |
| Inc-NKX2-2-2:2 | SEQ3150 | 0.00794 | 2.52 | 1.00 | Inc-LEKR1-4:9 | SEQ2521 | 0.03175 | 0.54 | 0.92 |
| Inc-NLN-3:1 | SEQ3152 | 0.00794 | 2.53 | 1.00 | Inc-EIF2B1-6:1 | SEQ2524 | 0.03175 | 0.54 | 0.92 |
| Inc-SMG1-1:9 | SEQ3156 | 0.00794 | 2.57 | 1.00 | Inc-HSD1 1 B2-1:1 | SEQ2525 | 0.03175 | 0.54 | 0.92 |
| Inc-RIPOR1-1:7 | SEQ3158 | 0.00794 | 2.57 | 1.00 | Inc-UGDH-3:3 | SEQ2526 | 0.03175 | 0.54 | 0.92 |
| Inc-SAMD11-10:1 | SEQ3159 | 0.00794 | 2.59 | 1.00 | Inc-CBY3-3:2 | SEQ2527 | 0.03175 | 0.54 | 0.92 |
| Inc-MOCS1-1:6 | SEQ3160 | 0.00794 | 2.59 | 1.00 | Inc-C14orf28-2:1 | SEQ2528 | 0.03175 | 0.54 | 0.92 |
| Inc-ANKRD9-1:1 | SEQ3162 | 0.00794 | 2.62 | 1.00 | Inc-FAM60A-3:1 | SEQ2531 | 0.03175 | 0.54 | 0.92 |
| MIR663AHG:66 | SEQ3165 | 0.00794 | 2.67 | 1.00 | Inc-DHX33-1:1 | SEQ2533 | 0.03175 | 0.55 | 0.92 |
| Inc-MOCS1-6:1 | SEQ3167 | 0.00794 | 2.70 | 1.00 | Inc-ARSG-2:8 | SEQ2535 | 0.03175 | 0.55 | 0.92 |
| Inc-HNRNPU-8:1 | SEQ3170 | 0.00794 | 2.72 | 1.00 | Inc-CA6-8:1 | SEQ2536 | 0.03175 | 0.55 | 0.92 |
| Inc-KIAA0141-1:2 | SEQ3171 | 0.00794 | 2.74 | 1.00 | Inc-DEFB115-5:3 | SEQ2538 | 0.03175 | 0.55 | 0.92 |
| Inc-HMOX1-2:2 | SEQ3174 | 0.00794 | 2.75 | 1.00 | Inc-PNLIPRP1-1:2 | SEQ2540 | 0.03175 | 0.55 | 0.92 |
| Inc-HDGFL2-7:1 | SEQ3180 | 0.00794 | 2.83 | 1.00 | Inc-FAM104A-2:1 | SEQ2542 | 0.03175 | 0.55 | 0.92 |
| Inc-UBASH3A-6:2 | SEQ3182 | 0.00794 | 2.91 | 1.00 | Inc-KCNA3-3:6 | SEQ2546 | 0.03175 | 0.55 | 0.92 |
| SLC2A1-AS1:15 | SEQ3183 | 0.00794 | 2.94 | 1.00 | Inc-PNLIPRP1-1:3 | SEQ2548 | 0.03175 | 0.55 | 0.92 |
| TRPM2-AS:5 | SEQ3184 | 0.00794 | 2.95 | 1.00 | Inc-KIAA1586-4:1 | SEQ2549 | 0.03175 | 0.55 | 0.92 |
| Inc-AGO2-2:3 | SEQ0668 | 0.00794 | 2.95 | 1.00 | Inc-H2AFJ-2:7 | SEQ2551 | 0.03175 | 0.56 | 0.92 |
| Inc-DENND1A-5:1 | SEQ3185 | 0.00794 | 2.96 | 1.00 | Inc-MED14-6:1 | SEQ2552 | 0.03175 | 0.56 | 0.92 |
| Inc-MOCS1-1:5 | SEQ3186 | 0.00794 | 2.96 | 1.00 | Inc-LRRC63-6:2 | SEQ2555 | 0.03175 | 0.56 | 0.92 |
| Inc-IL3RA-1 :24 | SEQ3193 | 0.00794 | 3.02 | 1.00 | Inc-MAPK6-5:1 | SEQ2556 | 0.03175 | 0.56 | 0.92 |
| Inc-GRAP-1:1 | SEQ3195 | 0.00794 | 3.04 | 1.00 | Inc-CHN1-5:11 | SEQ0419 | 0.03175 | 0.56 | 0.92 |
| Inc-ANKRD13A-5:1 | SEQ3196 | 0.00794 | 3.05 | 1.00 | Inc-ARHGAP28-10:4 | SEQ2560 | 0.03175 | 0.56 | 0.92 |
| Inc-SPANXB1-5:4 | SEQ3202 | 0.00794 | 3.10 | 1.00 | Inc-AIDA-1:1 | SEQ2562 | 0.03175 | 0.56 | 0.92 |
| PACRG-AS3:6 | SEQ3204 | 0.00794 | 3.13 | 1.00 | Inc-CLDN19-3:1 | SEQ2564 | 0.03175 | 0.56 | 0.92 |
| Inc-ANKRD13A-5:3 | SEQ3206 | 0.00794 | 3.14 | 1.00 | Inc-TOR1AIP1-2:1 | SEQ2565 | 0.03175 | 0.57 | 0.92 |
| Inc-BCL2L13-4:1 | SEQ3208 | 0.00794 | 3.15 | 1.00 | Inc-DLGAP5-2:1 | SEQ2566 | 0.03175 | 0.57 | 0.92 |
| Inc-TFF3-1:1 | SEQ3209 | 0.00794 | 3.15 | 1.00 | Inc-TMEM55A-6:1 | SEQ2568 | 0.03175 | 0.57 | 0.92 |
| Inc-ZNF585B-5:7 | SEQ3212 | 0.00794 | 3.20 | 1.00 | Inc-ABTB1-1:1 | SEQ2569 | 0.03175 | 0.57 | 0.92 |
| MALAT1:25 | SEQ3213 | 0.00794 | 3.21 | 1.00 | Inc-KMT2C-1:1 | SEQ2570 | 0.03175 | 0.57 | 0.92 |
| Inc-ACSBG2-2:3 | SEQ3214 | 0.00794 | 3.23 | 1.00 | Inc-GNA14-3:1 | SEQ2572 | 0.03175 | 0.57 | 0.92 |
| Inc-HIPK2-1:1 | SEQ3215 | 0.00794 | 3.23 | 1.00 | Inc-ATG2B-9:2 | SEQ2576 | 0.03175 | 0.57 | 0.92 |
| LINC01159:14 | SEQ3216 | 0.00794 | 3.27 | 1.00 | Inc-IL13RA2-3:1 | SEQ2577 | 0.03175 | 0.57 | 0.92 |
| Inc-TMEM53-2:1 | SEQ3218 | 0.00794 | 3.29 | 1.00 | Inc-SHB-4:1 | SEQ2579 | 0.03175 | 0.57 | 0.92 |
| MIR646HG:26 | SEQ3222 | 0.00794 | 3.38 | 1.00 | Inc-SLC25A32-9:1 | SEQ2581 | 0.03175 | 0.58 | 0.92 |
| Inc-MUSK-3:1 | SEQ3224 | 0.00794 | 3.39 | 1.00 | OIP5-AS1:10 | SEQ2582 | 0.03175 | 0.58 | 0.92 |
| Inc-TMEM53-1:1 | SEQ3226 | 0.00794 | 3.47 | 1.00 | OIP5-AS1:34 | SEQ2583 | 0.03175 | 0.58 | 0.92 |
| Inc-NAIP-5:1 | SEQ3231 | 0.00794 | 3.68 | 1.00 | Inc-SPRED2-22:1 | SEQ2584 | 0.03175 | 0.58 | 0.92 |
| Inc-ZNF236-1:7 | SEQ3232 | 0.00794 | 3.73 | 1.00 | NORAD:8 | SEQ2585 | 0.03175 | 0.58 | 0.92 |
| TSIX:1 | SEQ3234 | 0.00794 | 3.78 | 1.00 | Inc-PHIP-3:1 | SEQ2586 | 0.03175 | 0.58 | 0.92 |
| Inc-MPHOSPH10-2:1 | SEQ3236 | 0.00794 | 3.86 | 1.00 | Inc-MAN2A2-2:3 | SEQ2588 | 0.03175 | 0.58 | 0.92 |
| Inc-ENGASE-4:1 | SEQ3237 | 0.00794 | 3.97 | 1.00 | Inc-DCAF8-1:1 | SEQ2590 | 0.03175 | 0.58 | 0.92 |
| HDAC2-AS2:35 | SEQ3239 | 0.00794 | 4.10 | 1.00 | Inc-MBOAT4-2:1 | SEQ2592 | 0.03175 | 0.58 | 0.92 |
| Inc-SPANXB1-5:3 | SEQ3242 | 0.00794 | 4.19 | 1.00 | Inc-FOXD4L5-18:4 | SEQ2593 | 0.03175 | 0.58 | 0.92 |
| Inc-ZNF236-1:6 | SEQ3246 | 0.00794 | 4.26 | 1.00 | Inc-SPINK2-4:1 | SEQ2594 | 0.03175 | 0.59 | 0.92 |
| Inc-THOC1-3:1 | SEQ3247 | 0.00794 | 4.41 | 1.00 | Inc-MLH1-1:1 | SEQ2597 | 0.03175 | 0.59 | 0.92 |
| Inc-TARDBP-5:6 | SEQ3249 | 0.00794 | 4.51 | 1.00 | Inc-AHCY-4:1 | SEQ2600 | 0.03175 | 0.59 | 0.92 |
| Inc-GPR37-1:1 | SEQ0926 | 0.00794 | 4.72 | 1.00 | Inc-MCTS1-2:1 | SEQ2602 | 0.03175 | 0.59 | 0.92 |
| LINC01608:9 | SEQ3251 | 0.00794 | 4.76 | 1.00 | Inc-SCD-2:4 | SEQ2606 | 0.03175 | 0.60 | 0.92 |
| SRP14-AS1:28 | SEQ3253 | 0.00794 | 5.10 | 1.00 | Inc-FAM103A1-2:5 | SEQ2607 | 0.03175 | 0.60 | 0.92 |
| Inc-CXorf51B-1:1 | SEQ3257 | 0.00794 | 5.47 | 1.00 | Inc-SURF2-4:1 | SEQ2609 | 0.03175 | 0.60 | 0.92 |
| Inc-ZNF608-20:2 | SEQ3258 | 0.00794 | 5.65 | 1.00 | Inc-HAO2-2:26 | SEQ2610 | 0.03175 | 0.60 | 0.92 |
| Inc-LEF1-3:4 | SEQ3259 | 0.00794 | 5.77 | 1.00 | SNHG7:15 | SEQ2614 | 0.03175 | 0.60 | 0.92 |
| LINC01515:31 | SEQ3260 | 0.00794 | 5.90 | 1.00 | Inc-HMG20A-6:1 | SEQ2615 | 0.03175 | 0.60 | 0.92 |
| Inc-BCL2L13-5:2 | SEQ3262 | 0.00794 | 6.65 | 1.00 | Inc-PANK3-13:2 | SEQ2618 | 0.03175 | 0.60 | 0.92 |
| Inc-LTBP3-2:6 | SEQ3264 | 0.00794 | 6.67 | 1.00 | Inc-ATCAY-1:1 | SEQ2619 | 0.03175 | 0.60 | 0.92 |
| NR2F1-AS1:49 | SEQ3265 | 0.00794 | 6.78 | 1.00 | EIF3J-AS1:21 | SEQ0261 | 0.03175 | 0.61 | 0.92 |
| LINC01608.12 | SEQ3266 | 0.00794 | 6.91 | 1.00 | Inc-DNAH8-2:6 | SEQ2620 | 0.03175 | 0.61 | 0.92 |
| Inc-NMUR2-4:1 | SEQ3268 | 0.00794 | 6.99 | 1.00 | Inc-ATOX1-4:1 | SEQ2621 | 0.03175 | 0.61 | 0.92 |
| LINC-PINT:27 | SEQ3269 | 0.00794 | 8.08 | 1.00 | Inc-AGAP2-1:4 | SEQ2622 | 0.03175 | 0.61 | 0.92 |
| Inc-DRICH1-3:26 | SEQ3271 | 0.00794 | 8.28 | 1.00 | Inc-HRASLS-5:3 | SEQ2623 | 0.03175 | 0.61 | 0.92 |
| MALAT1:23 | SEQ3272 | 0.00794 | 10.88 | 1.00 | Inc-HMGCS1-3:1 | SEQ2627 | 0.03175 | 0.61 | 0.92 |
| MALAT1:48 | SEQ3273 | 0.00794 | 10.88 | 1.00 | Inc-PANK3-13:1 | SEQ2629 | 0.03175 | 0.61 | 0.92 |
| Inc-BAHCC1-2:1 | SEQ3274 | 0.00794 | 12.21 | 1.00 | Inc-FAM 174A-6:2 | SEQ2631 | 0.03175 | 0.62 | 0.92 |
| Inc-NDUFB4-1:3 | SEQ2116 | 0.01116 | 0.02 | 1.00 | Inc-UCHL3-1:1 | SEQ2632 | 0.03175 | 0.62 | 0.92 |
| Inc-CTH-14:1 | SEQ2121 | 0.01193 | 0.07 | 1.00 | Inc-PALLD-8:1 | SEQ2633 | 0.03175 | 0.62 | 0.92 |
| Inc-ZNF717-4:8 | SEQ2119 | 0.01587 | 0.04 | 0.96 | Inc-ZNF596-9:2 | SEQ2634 | 0.03175 | 0.62 | 0.92 |
| Inc-PSMB9-6:1 | SEQ2126 | 0.01587 | 0.09 | 0.96 | Inc-PTAR1-4:1 | SEQ2638 | 0.03175 | 0.62 | 0.92 |
| Inc-SYT16-4:11 | SEQ2139 | 0.01587 | 0.16 | 0.96 | Inc-GLRX2-1:2 | SEQ2640 | 0.03175 | 0.62 | 0.92 |
| Inc-ZNF705B-3:1 | SEQ2150 | 0.01587 | 0.20 | 0.96 | Inc-PHOSPH02-2:1 | SEQ2641 | 0.03175 | 0.62 | 0.92 |
| Inc-DIRC3-1:4 | SEQ2156 | 0.01587 | 0.21 | 0.96 | Inc-KLHL28-3:5 | SEQ2642 | 0.03175 | 0.62 | 0.92 |
| Inc-NID2-2:1 | SEQ2158 | 0.01587 | 0.22 | 0.96 | CASC2:1 | SEQ2645 | 0.03175 | 0.62 | 0.92 |
| Inc-SYT16-4:10 | SEQ2159 | 0.01587 | 0.23 | 0.96 | Inc-KIF1C-1:2 | SEQ2646 | 0.03175 | 0.62 | 0.92 |
| Inc-MED10-3:1 | SEQ2165 | 0.01587 | 0.24 | 0.96 | Inc-STARD8-3:1 | SEQ2647 | 0.03175 | 0.63 | 0.92 |
| Inc-PRSS27-4:24 | SEQ2168 | 0.01587 | 0.24 | 0.96 | Inc-ASXL1-3:1 | SEQ2650 | 0.03175 | 0.63 | 0.92 |
| Inc-SCARB2-1:4 | SEQ2171 | 0.01587 | 0.25 | 0.96 | Inc-LPCAT3-1:1 | SEQ2652 | 0.03175 | 0.63 | 0.92 |
| Inc-GAPT-10:1 | SEQ2172 | 0.01587 | 0.25 | 0.96 | MCPH1-AS1:2 | SEQ0308 | 0.03175 | 0.63 | 0.92 |
| CERNA1:2 | SEQ2176 | 0.01587 | 0.25 | 0.96 | ZFAS1:23 | SEQ2657 | 0.03175 | 0.63 | 0.92 |
| Inc-HNRNPA2B1-15:16 | SEQ2177 | 0.01587 | 0.25 | 0.96 | Inc-SSB-2:1 | SEQ2661 | 0.03175 | 0.63 | 0.92 |
| THAP9-AS1:27 | SEQ2178 | 0.01587 | 0.25 | 0.96 | Inc-KANSL2-1:2 | SEQ2664 | 0.03175 | 0.64 | 0.92 |
| Inc-LIPI-7:1 | SEQ2195 | 0.01587 | 0.30 | 0.96 | Inc-BICRAL-3:1 | SEQ2666 | 0.03175 | 0.64 | 0.92 |
| Inc-PRDM4-6:5 | SEQ2211 | 0.01587 | 0.31 | 0.96 | Inc-USP53-8:5 | SEQ2668 | 0.03175 | 0.64 | 0.92 |
| Inc-SOCS6-10:1 | SEQ2214 | 0.01587 | 0.31 | 0.96 | LINC01124:11 | SEQ2670 | 0.03175 | 0.64 | 0.92 |
| Inc-UNC80-1:1 | SEQ2219 | 0.01587 | 0.32 | 0.96 | Inc-TARBP1-6:1 | SEQ2671 | 0.03175 | 0.64 | 0.92 |
| Inc-SYT16-4:3 | SEQ2220 | 0.01587 | 0.32 | 0.96 | Inc-MRPS16-2:4 | SEQ2674 | 0.03175 | 0.64 | 0.92 |
| Inc-SYT16-4:6 | SEQ2221 | 0.01587 | 0.32 | 0.96 | Inc-SIDT2-2:2 | SEQ2675 | 0.03175 | 0.64 | 0.92 |
| Inc-SMARCA5-4:7 | SEQ2223 | 0.01587 | 0.32 | 0.96 | Inc-C11orf94-1:1 | SEQ2676 | 0.03175 | 0.64 | 0.92 |
| Inc-USP17L7-1:1 | SEQ0746 | 0.01587 | 0.33 | 0.96 | Inc-GNA11-3:1 | SEQ2681 | 0.03175 | 0.65 | 0.92 |
| LINC00630:38 | SEQ2231 | 0.01587 | 0.33 | 0.96 | Inc-BTBD1-2:1 | SEQ2683 | 0.03175 | 0.65 | 0.92 |
| Inc-GUCY1A3-1:1 | SEQ0520 | 0.01587 | 0.34 | 0.96 | Inc-MZT2A-19:1 | SEQ2684 | 0.03175 | 0.65 | 0.92 |
| Inc-GLIPR1L1-2:3 | SEQ0427 | 0.01587 | 0.34 | 0.96 | Inc-SMC5-6:1 | SEQ2686 | 0.03175 | 0.65 | 0.92 |
| RFPL1S:11 | SEQ2233 | 0.01587 | 0.34 | 0.96 | Inc-NPIPB4-3:5 | SEQ2687 | 0.03175 | 0.65 | 0.92 |
| Inc-PPIE-5:1 | SEQ2235 | 0.01587 | 0.34 | 0.96 | Inc-TOX4-4:2 | SEQ2696 | 0.03175 | 0.66 | 0.92 |
| Inc-ITGBL1-2:1 | SEQ2241 | 0.01587 | 0.35 | 0.96 | Inc-KCNT1-6:1 | SEQ2699 | 0.03175 | 0.66 | 0.92 |
| NR2F1-AS1:6 | SEQ2247 | 0.01587 | 0.36 | 0.96 | Inc-ATPIF1-2:1 | SEQ2700 | 0.03175 | 0.66 | 0.92 |
| RFPL1S:10 | SEQ2248 | 0.01587 | 0.36 | 0.96 | Inc-ITGB1BP2-2:1 | SEQ2701 | 0.03175 | 0.66 | 0.92 |
| CHMP1B-AS1:2 | SEQ2249 | 0.01587 | 0.36 | 0.96 | Inc-TMEM186-1:2 | SEQ2702 | 0.03175 | 0.66 | 0.92 |
| Inc-PPP1R3C-4:1 | SEQ2250 | 0.01587 | 0.36 | 0.96 | Inc-INAFM2-1:1 | SEQ2705 | 0.03175 | 0.67 | 0.92 |
| Inc-SUSD1-1:5 | SEQ0638 | 0.01587 | 0.37 | 0.96 | Inc-RGPD8-5:2 | SEQ2707 | 0.03175 | 0.67 | 0.92 |
| LINC01184:30 | SEQ2257 | 0.01587 | 0.37 | 0.96 | Inc-CDT1-1:1 | SEQ2708 | 0.03175 | 0.67 | 0.92 |
| Inc-TMSB15A-3:1 | SEQ2259 | 0.01587 | 0.37 | 0.96 | Inc-BTBD6-2:1 | SEQ2709 | 0.03175 | 0.67 | 0.92 |
| Inc-COPG2-3:1 | SEQ2265 | 0.01587 | 0.38 | 0.96 | Inc-CRHR2-3:1 | SEQ2710 | 0.03175 | 0.67 | 0.92 |
| Inc-SNRPN-8:36 | SEQ2268 | 0.01587 | 0.38 | 0.96 | PINK1-AS:1 | SEQ2712 | 0.03175 | 0.68 | 0.92 |
| Inc-TYRO3-3:1 | SEQ2269 | 0.01587 | 0.39 | 0.96 | Inc-GUSB-4:1 | SEQ2716 | 0.03175 | 0.68 | 0.92 |
| Inc-ZNF736-1:2 | SEQ2272 | 0.01587 | 0.39 | 0.96 | Inc-GP1BA-1:1 | SEQ2718 | 0.03175 | 0.68 | 0.92 |
| Inc-NT5E-11:3 | SEQ2278 | 0.01587 | 0.39 | 0.96 | Inc-EDEM3-7:3 | SEQ0272 | 0.03175 | 0.68 | 0.92 |
| Inc-MUC20-5:2 | SEQ2289 | 0.01587 | 0.40 | 0.96 | Inc-PRCP-1:1 | SEQ2720 | 0.03175 | 0.68 | 0.92 |
| Inc-FANCM-8:3 | SEQ2291 | 0.01587 | 0.40 | 0.96 | Inc-ALX4-17:1 | SEQ2722 | 0.03175 | 0.68 | 0.92 |
| Inc-FSHR-4:1 | SEQ2298 | 0.01587 | 0.41 | 0.96 | Inc-AP3S1-1:3 | SEQ2723 | 0.03175 | 0.68 | 0.92 |
| Inc-LIPI-4:6 | SEQ2300 | 0.01587 | 0.41 | 0.96 | Inc-FUT9-1:1 | SEQ2724 | 0.03175 | 0.69 | 0.92 |
| Inc-VPREB1-7:3 | SEQ2303 | 0.01587 | 0.41 | 0.96 | Inc-CHD2-13:2 | SEQ2726 | 0.03175 | 0.69 | 0.92 |
| Inc-KHDC3L-2:16 | SEQ2305 | 0.01587 | 0.41 | 0.96 | Inc-BAG3-1:1 | SEQ2727 | 0.03175 | 0.69 | 0.92 |
| Inc-CHST7-2:1 | SEQ2306 | 0.01587 | 0.41 | 0.96 | Inc-TMEM243-1:1 | SEQ2728 | 0.03175 | 0.69 | 0.92 |
| Inc-PARD3B-4:1 | SEQ2315 | 0.01587 | 0.42 | 0.96 | Inc-RPS6KB1-1:7 | SEQ2729 | 0.03175 | 0.69 | 0.92 |
| SNHG3:5 | SEQ2316 | 0.01587 | 0.42 | 0.96 | Inc-ZC3HC1-4:1 | SEQ2731 | 0.03175 | 0.70 | 0.92 |
| Inc-ZNF704-7:6 | SEQ2317 | 0.01587 | 0.42 | 0.96 | Inc-PINK1-2:1 | SEQ2734 | 0.03175 | 0.70 | 0.92 |
| Inc-PRMT6-9:1 | SEQ2319 | 0.01587 | 0.43 | 0.96 | Inc-RTL9-2:1 | SEQ2735 | 0.03175 | 0.70 | 0.92 |
| Inc-GNA13-2:13 | SEQ2320 | 0.01587 | 0.43 | 0.96 | Inc-MAML3-2:1 | SEQ0438 | 0.03175 | 0.71 | 0.92 |
| Inc-FGFBP3-2:2 | SEQ2330 | 0.01587 | 0.43 | 0.96 | Inc-NOB1-1:1 | SEQ2738 | 0.03175 | 0.71 | 0.92 |
| Inc-NOL4-6:1 | SEQ2331 | 0.01587 | 0.43 | 0.96 | Inc-ANAPC11-2:9 | SEQ2739 | 0.03175 | 0.71 | 0.92 |
| IDI2-AS1:5 | SEQ2352 | 0.01587 | 0.45 | 0.96 | Inc-C14orf177-5:1 | SEQ2740 | 0.03175 | 0.72 | 0.92 |
| Inc-ZNF316-3:8 | SEQ2359 | 0.01587 | 0.45 | 0.96 | Inc-ACTRT3-5:1 | SEQ2741 | 0.03175 | 0.72 | 0.92 |
| ADIRF-AS1:2 | SEQ2379 | 0.01587 | 0.46 | 0.96 | Inc-GPBP1L1-1:8 | SEQ2745 | 0.03175 | 0.73 | 0.92 |
| Inc-CA6-8:2 | SEQ2380 | 0.01587 | 0.46 | 0.96 | Inc-ZNF221-2:6 | SEQ2746 | 0.03175 | 0.73 | 0.92 |
| Inc-DCAF4L1-2:1 | SEQ2387 | 0.01587 | 0.47 | 0.96 | Inc-LEAP2-2:1 | SEQ2749 | 0.03175 | 0.74 | 0.92 |
| SNAI3-AS1:6 | SEQ2392 | 0.01587 | 0.47 | 0.96 | Inc-CIB3-1:2 | SEQ2750 | 0.03175 | 0.74 | 0.92 |
| Inc-COX7B-2:1 | SEQ2394 | 0.01587 | 0.47 | 0.96 | Inc-SLTM-1:2 | SEQ0982 | 0.03175 | 0.75 | 0.92 |
| Inc-TAOK3-9:1 | SEQ2396 | 0.01587 | 0.48 | 0.96 | Inc-NR2C2-1:1 | SEQ2751 | 0.03175 | 0.75 | 0.92 |
| LINC01006:1 | SEQ2401 | 0.01587 | 0.48 | 0.96 | Inc-DFFA-12:2 | SEQ2754 | 0.03175 | 0.75 | 0.92 |
| LINC01006:2 | SEQ2402 | 0.01587 | 0.48 | 0.96 | Inc-PLEKHA8-3:5 | SEQ0962 | 0.03175 | 0.75 | 0.92 |
| Inc-AP3S1-1:2 | SEQ2405 | 0.01587 | 0.48 | 0.96 | Inc-PGBD5-1:1 | SEQ2755 | 0.03175 | 0.75 | 0.92 |
| OIP5-AS1:2 | SEQ2408 | 0.01587 | 0.48 | 0.96 | Inc-CDH8-4:1 | SEQ2756 | 0.03175 | 0.76 | 0.92 |
| Inc-FAM 172A-7:8 | SEQ2409 | 0.01587 | 0.48 | 0.96 | Inc-TCTA-2:1 | SEQ2757 | 0.03175 | 0.76 | 0.92 |
| Inc-PMM2-6:4 | SEQ2411 | 0.01587 | 0.48 | 0.96 | Inc-ERW-2-9:1 | SEQ2758 | 0.03175 | 0.76 | 0.92 |
| UGDH-AS1:10 | SEQ0407 | 0.01587 | 0.49 | 0.96 | Inc-DDX49-1:1 | SEQ2759 | 0.03175 | 0.76 | 0.92 |
| Inc-S100P-4:13 | SEQ2418 | 0.01587 | 0.49 | 0.96 | Inc-HSD11B1L-1:1 | SEQ2760 | 0.03175 | 0.77 | 0.92 |
| Inc-ZNF449-3:5 | SEQ2420 | 0.01587 | 0.49 | 0.96 | Inc-MOGAT2-4:1 | SEQ2762 | 0.03175 | 0.78 | 0.92 |
| Inc-ZNF449-3:4 | SEQ2421 | 0.01587 | 0.49 | 0.96 | Inc-C20orf96-1:1 | SEQ2764 | 0.03175 | 0.79 | 0.92 |
| Inc-GRAP2-2:1 | SEQ2429 | 0.01587 | 0.49 | 0.96 | Inc-TRIM59-1:1 | SEQ2766 | 0.03175 | 0.79 | 0.92 |
| Inc-TPTE2-4:1 | SEQ2431 | 0.01587 | 0.49 | 0.96 | Inc-GSDMB-1:1 | SEQ2767 | 0.03175 | 0.84 | 0.92 |
| Inc-H2AFV-3:1 | SEQ2433 | 0.01587 | 0.49 | 0.96 | Inc-RFNG-1:7 | SEQ2768 | 0.03175 | 1.22 | 0.92 |
| Inc-XXYLT1-5:1 | SEQ0221 | 0.01587 | 0.50 | 0.96 | Inc-SCRN1-1:5 | SEQ2769 | 0.03175 | 1.25 | 0.92 |
| Inc-EPC1-5:1 | SEQ2445 | 0.01587 | 0.50 | 0.96 | Inc-RRN3-3:5 | SEQ2770 | 0.03175 | 1.26 | 0.92 |
| Inc-NUB1-4:1 | SEQ0126 | 0.01587 | 0.50 | 0.96 | Inc-ANKS3-2:1 | SEQ2771 | 0.03175 | 1.28 | 0.92 |
| Inc-GPRIN2-2:1 | SEQ2453 | 0.01587 | 0.50 | 0.96 | Inc-ADAT3-5:1 | SEQ2772 | 0.03175 | 1.28 | 0.92 |
| Inc-ACTR10-6:1 | SEQ2458 | 0.01587 | 0.51 | 0.96 | Inc-TMC8-4:1 | SEQ2775 | 0.03175 | 1.32 | 0.92 |
| Inc-KCNE4-7:1 | SEQ2466 | 0.01587 | 0.51 | 0.96 | EP300-AS1:13 | SEQ2778 | 0.03175 | 1.34 | 0.92 |
| Inc-RMDN2-3:24 | SEQ2468 | 0.01587 | 0.52 | 0.96 | Inc-SLC22A18-1:1 | SEQ2779 | 0.03175 | 1.34 | 0.92 |
| OTUD6B-AS1:5 | SEQ2471 | 0.01587 | 0.52 | 0.96 | Inc-PARVG-5:1 | SEQ2781 | 0.03175 | 1.35 | 0.92 |
| Inc-CGREF1-2:1 | SEQ2472 | 0.01587 | 0.52 | 0.96 | Inc-MRPL3-2:6 | SEQ2782 | 0.03175 | 1.36 | 0.92 |
| Inc-FGF23-5:3 | SEQ2473 | 0.01587 | 0.52 | 0.96 | DUBR:25 | SEQ2783 | 0.03175 | 1.38 | 0.92 |
| Inc-FGF23-5:1 | SEQ2474 | 0.01587 | 0.52 | 0.96 | HDHD5-AS1:6 | SEQ2784 | 0.03175 | 1.38 | 0.92 |
| Inc-DLK1-18:121 | SEQ2479 | 0.01587 | 0.52 | 0.96 | Inc-NPIPB12-1:1 | SEQ0822 | 0.03175 | 1.39 | 0.92 |
| Inc-SMARCC2-5:1 | SEQ2481 | 0.01587 | 0.52 | 0.96 | Inc-CMTM3-1:1 | SEQ2789 | 0.03175 | 1.39 | 0.92 |
| Inc-ARHGAP15-2:3 | SEQ2483 | 0.01587 | 0.52 | 0.96 | Inc-SSH3-5:1 | SEQ2791 | 0.03175 | 1.40 | 0.92 |
| EIF3J-AS1:1 | SEQ2486 | 0.01587 | 0.53 | 0.96 | WDFY3-AS2:5 | SEQ2794 | 0.03175 | 1.41 | 0.92 |
| SNHG22:7 | SEQ2487 | 0.01587 | 0.53 | 0.96 | LINC00342:1 | SEQ2795 | 0.03175 | 1.41 | 0.92 |
| Inc-ST7L-5:1 | SEQ2493 | 0.01587 | 0.53 | 0.96 | Inc-TMEM211-1:1 | SEQ2797 | 0.03175 | 1.42 | 0.92 |
| NORAD:9 | SEQ2494 | 0.01587 | 0.53 | 0.96 | Inc-USP35-11:2 | SEQ2802 | 0.03175 | 1.45 | 0.92 |
| Inc-LEKR1-4:10 | SEQ2495 | 0.01587 | 0.53 | 0.96 | Inc-DHX38-4:13 | SEQ2803 | 0.03175 | 1.45 | 0.92 |
| Inc-CT47A1-1:1 | SEQ2504 | 0.01587 | 0.53 | 0.96 | Inc-RPP25-3:1 | SEQ2804 | 0.03175 | 1.46 | 0.92 |
| Inc-SOCS2-1:2 | SEQ2505 | 0.01587 | 0.53 | 0.96 | SEMA6A-AS1:1 | SEQ2805 | 0.03175 | 1.47 | 0.92 |
| Inc-ADGRA3-7:1 | SEQ2512 | 0.01587 | 0.54 | 0.96 | TRAF3IP2-AS1:58 | SEQ2806 | 0.03175 | 1.47 | 0.92 |
| Inc-CHODL-2:2 | SEQ2513 | 0.01587 | 0.54 | 0.96 | Inc-HBEGF-2:8 | SEQ2807 | 0.03175 | 1.47 | 0.92 |
| Inc-SSR3-11:1 | SEQ2529 | 0.01587 | 0.54 | 0.96 | HYI-AS1:3 | SEQ2809 | 0.03175 | 1.48 | 0.92 |
| NORAD:3 | SEQ2530 | 0.01587 | 0.54 | 0.96 | Inc-WDFY4-2:1 | SEQ2812 | 0.03175 | 1.49 | 0.92 |
| NORAD:4 | SEQ2532 | 0.01587 | 0.55 | 0.96 | Inc-KLHDC4-2:2 | SEQ2815 | 0.03175 | 1.49 | 0.92 |
| Inc-CIB3-6:1 | SEQ2539 | 0.01587 | 0.55 | 0.96 | Inc-CTNS-1:1 | SEQ2816 | 0.03175 | 1.50 | 0.92 |
| Inc-LRRK2-1:5 | SEQ2541 | 0.01587 | 0.55 | 0.96 | Inc-TRPM1-3:1 | SEQ0388 | 0.03175 | 1.51 | 0.92 |
| LINC01537:4 | SEQ2543 | 0.01587 | 0.55 | 0.96 | Inc-AUNIP-1:12 | SEQ2821 | 0.03175 | 1.52 | 0.92 |
| Inc-ANAPC11-2:12 | SEQ2544 | 0.01587 | 0.55 | 0.96 | Inc-MYOM1-4:5 | SEQ2823 | 0.03175 | 1.52 | 0.92 |
| Inc-COL20A1-3:2 | SEQ2545 | 0.01587 | 0.55 | 0.96 | Inc-SPATA21-6:3 | SEQ2824 | 0.03175 | 1.52 | 0.92 |
| Inc-JAG1-6:1 | SEQ2547 | 0.01587 | 0.55 | 0.96 | Inc-AGBL3-2:1 | SEQ2825 | 0.03175 | 1.52 | 0.92 |
| NORAD:1 | SEQ2550 | 0.01587 | 0.55 | 0.96 | Inc-NPIPB12-2:1 | SEQ2827 | 0.03175 | 1.53 | 0.92 |
| Inc-WDR77-1:2 | SEQ2553 | 0.01587 | 0.56 | 0.96 | Inc-C11orf95-1:2 | SEQ2828 | 0.03175 | 1.53 | 0.92 |
| Inc-TBCB-1:2 | SEQ2561 | 0.01587 | 0.56 | 0.96 | Inc-TPT1-2:1 | SEQ2830 | 0.03175 | 1.54 | 0.92 |
| Inc-ARMCX1-2:1 | SEQ2563 | 0.01587 | 0.56 | 0.96 | Inc-SLC25A16-1:2 | SEQ2831 | 0.03175 | 1.54 | 0.92 |
| Inc-SRD5A1-2:1 | SEQ2573 | 0.01587 | 0.57 | 0.96 | Inc-USP6-2:22 | SEQ2832 | 0.03175 | 1.54 | 0.92 |
| Inc-ZNF613-7:1 | SEQ2574 | 0.01587 | 0.57 | 0.96 | Inc-MED9-1:2 | SEQ2833 | 0.03175 | 1.54 | 0.92 |
| Inc-MPPE1-8:3 | SEQ2578 | 0.01587 | 0.57 | 0.96 | Inc-NOP14-3:4 | SEQ2834 | 0.03175 | 1.54 | 0.92 |
| Inc-RSPH10B2-1:3 | SEQ2580 | 0.01587 | 0.57 | 0.96 | Inc-ADAP1-1:1 | SEQ2837 | 0.03175 | 1.55 | 0.92 |
| GABPB1-IT1:5 | SEQ2591 | 0.01587 | 0.58 | 0.96 | Inc-ZFP3-3:1 | SEQ2838 | 0.03175 | 1.55 | 0.92 |
| Inc-ARHGEF2-3:4 | SEQ2605 | 0.01587 | 0.60 | 0.96 | KIAA1614-AS1:1 | SEQ2839 | 0.03175 | 1.56 | 0.92 |
| Inc-WASHC4-4:1 | SEQ2608 | 0.01587 | 0.60 | 0.96 | Inc-PLD3-3:1 | SEQ2840 | 0.03175 | 1.56 | 0.92 |
| Inc-ZNF639-4:1 | SEQ2617 | 0.01587 | 0.60 | 0.96 | Inc-TARBP2-2:1 | SEQ2845 | 0.03175 | 1.57 | 0.92 |
| Inc-MFSD4A-1:1 | SEQ2625 | 0.01587 | 0.61 | 0.96 | LINC01410:8 | SEQ2846 | 0.03175 | 1.57 | 0.92 |
| OIP5-AS1:1 | SEQ2635 | 0.01587 | 0.62 | 0.96 | Inc-CYP4F22-5:1 | SEQ2850 | 0.03175 | 1.58 | 0.92 |
| Inc-ZNF613-6:1 | SEQ2637 | 0.01587 | 0.62 | 0.96 | PPP3CB-AS1:22 | SEQ2852 | 0.03175 | 1.58 | 0.92 |
| Inc-SNX20-5:3 | SEQ2643 | 0.01587 | 0.62 | 0.96 | Inc-PPARA-3:8 | SEQ2854 | 0.03175 | 1.58 | 0.92 |
| Inc-KDM3A-1:3 | SEQ2648 | 0.01587 | 0.63 | 0.96 | Inc-MTG1-2:1 | SEQ2858 | 0.03175 | 1.59 | 0.92 |
| Inc-NOL6-6:2 | SEQ2649 | 0.01587 | 0.63 | 0.96 | DLEU2:26 | SEQ0862 | 0.03175 | 1.60 | 0.92 |
| Inc-FGL2-4:1 | SEQ2651 | 0.01587 | 0.63 | 0.96 | Inc-ABCE1-5:1 | SEQ2862 | 0.03175 | 1.60 | 0.92 |
| Inc-EIF1AD-1:1 | SEQ2653 | 0.01587 | 0.63 | 0.96 | IRAIN:2 | SEQ2863 | 0.03175 | 1.60 | 0.92 |
| Inc-TEX37-1:2 | SEQ2654 | 0.01587 | 0.63 | 0.96 | IRAIN:3 | SEQ2864 | 0.03175 | 1.60 | 0.92 |
| Inc-TEX37-1:1 | SEQ2655 | 0.01587 | 0.63 | 0.96 | Inc-ZNF778-1:4 | SEQ2865 | 0.03175 | 1.60 | 0.92 |
| Inc-POM121-2:5 | SEQ2658 | 0.01587 | 0.63 | 0.96 | MAGI2-AS3:85 | SEQ2867 | 0.03175 | 1.61 | 0.92 |
| Inc-SLC16A11-7:7 | SEQ2659 | 0.01587 | 0.63 | 0.96 | TPT1-AS1:54 | SEQ2870 | 0.03175 | 1.61 | 0.92 |
| Inc-PIGU-3:1 | SEQ2663 | 0.01587 | 0.64 | 0.96 | LINC02482:2 | SEQ2871 | 0.03175 | 1.61 | 0.92 |
| Inc-AGAP4-2:1 | SEQ2665 | 0.01587 | 0.64 | 0.96 | Inc-PPP6R2-2:2 | SEQ2876 | 0.03175 | 1.62 | 0.92 |
| Inc-URGCP-MRPS24-2:1 | SEQ2667 | 0.01587 | 0.64 | 0.96 | Inc-CLEC18B-5:1 | SEQ2878 | 0.03175 | 1.63 | 0.92 |
| Inc-EFEMP1-3:12 | SEQ2669 | 0.01587 | 0.64 | 0.96 | Inc-MYOZ1-1:2 | SEQ2879 | 0.03175 | 1.63 | 0.92 |
| Inc-SHISA5-1:2 | SEQ2672 | 0.01587 | 0.64 | 0.96 | LINC00599:13 | SEQ2880 | 0.03175 | 1.63 | 0.92 |
| Inc-GLOD4-3:1 | SEQ2673 | 0.01587 | 0.64 | 0.96 | LIMD1-AS1:2 | SEQ2885 | 0.03175 | 1.64 | 0.92 |
| Inc-STX10-3:1 | SEQ2677 | 0.01587 | 0.64 | 0.96 | Inc-TELO2-3:2 | SEQ2887 | 0.03175 | 1.65 | 0.92 |
| Inc-MAOA-5:1 | SEQ2678 | 0.01587 | 0.64 | 0.96 | Inc-DLK1-18:77 | SEQ2888 | 0.03175 | 1.65 | 0.92 |
| Inc-MB-5:1 | SEQ2679 | 0.01587 | 0.64 | 0.96 | Inc-MACC1-3:6 | SEQ2890 | 0.03175 | 1.66 | 0.92 |
| Inc-IL31RA-1:1 | SEQ2680 | 0.01587 | 0.64 | 0.96 | SVIL-AS1:39 | SEQ2891 | 0.03175 | 1.66 | 0.92 |
| Inc-FNTB-2:1 | SEQ2682 | 0.01587 | 0.65 | 0.96 | Inc-CELF4-10:1 | SEQ2892 | 0.03175 | 1.67 | 0.92 |
| Inc-FANCL-4:1 | SEQ2690 | 0.01587 | 0.65 | 0.96 | Inc-MED15-1:2 | SEQ0620 | 0.03175 | 1.68 | 0.92 |
| Inc-CES5A-1:2 | SEQ2691 | 0.01587 | 0.65 | 0.96 | lnc-LIPG-11:1 | SEQ2894 | 0.03175 | 1.68 | 0.92 |
| Inc-NOP53-2:1 | SEQ2692 | 0.01587 | 0.65 | 0.96 | Inc-TOB2-3:1 | SEQ2902 | 0.03175 | 1.69 | 0.92 |
| Inc-CPM-3:1 | SEQ0228 | 0.01587 | 0.66 | 0.96 | lnc-PLA2G1B-2:3 | SEQ2905 | 0.03175 | 1.70 | 0.92 |
| Inc-PTBP3-7:1 | SEQ2703 | 0.01587 | 0.67 | 0.96 | Inc-SLC37A2-2:1 | SEQ2906 | 0.03175 | 1.71 | 0.92 |
| Inc-FAM228B-2:1 | SEQ2714 | 0.01587 | 0.68 | 0.96 | Inc-RNF208-3:1 | SEQ2911 | 0.03175 | 1.72 | 0.92 |
| Inc-ABCC6-11:1 | SEQ2717 | 0.01587 | 0.68 | 0.96 | Inc-ANKRD36B-3:9 | SEQ2913 | 0.03175 | 1.72 | 0.92 |
| Inc-VLDLR-4:1 | SEQ2719 | 0.01587 | 0.68 | 0.96 | Inc-CD40LG-2:12 | SEQ2919 | 0.03175 | 1.74 | 0.92 |
| Inc-CYTIP-2:1 | SEQ0200 | 0.01587 | 0.68 | 0.96 | lnc-EVI5L-3:1 | SEQ2920 | 0.03175 | 1.74 | 0.92 |
| Inc-EPM2AIP1-9:2 | SEQ2721 | 0.01587 | 0.68 | 0.96 | Inc-ZNF587-1:1 | SEQ2921 | 0.03175 | 1.74 | 0.92 |
| Inc-LYN-8:1 | SEQ0619 | 0.01587 | 0.69 | 0.96 | Inc-SERPINE3-2:1 | SEQ2922 | 0.03175 | 1.74 | 0.92 |
| Inc-PSMC3IP-5:1 | SEQ2730 | 0.01587 | 0.70 | 0.96 | Inc-SHC3-5:1 | SEQ2924 | 0.03175 | 1.75 | 0.92 |
| Inc-P4HTM-2:1 | SEQ2732 | 0.01587 | 0.70 | 0.96 | Inc-RABEP2-6:1 | SEQ2929 | 0.03175 | 1.77 | 0.92 |
| Inc-GDPD4-1:3 | SEQ2733 | 0.01587 | 0.70 | 0.96 | Inc-NUDT4P1-1:12 | SEQ2932 | 0.03175 | 1.78 | 0.92 |
| Inc-KIFC2-1:8 | SEQ2737 | 0.01587 | 0.70 | 0.96 | Inc-CRYM-3:1 | SEQ2933 | 0.03175 | 1.78 | 0.92 |
| Inc-GAMT-6:2 | SEQ2742 | 0.01587 | 0.72 | 0.96 | GEMIN7-AS1:2 | SEQ2934 | 0.03175 | 1.78 | 0.92 |
| Inc-PRPF4B-5:1 | SEQ2747 | 0.01587 | 0.73 | 0.96 | lnc-FGFR1OP-11:1 | SEQ2935 | 0.03175 | 1.78 | 0.92 |
| Inc-CXorf65-2:1 | SEQ2748 | 0.01587 | 0.74 | 0.96 | Inc-NTAN1-5:1 | SEQ2936 | 0.03175 | 1.78 | 0.92 |
| Inc-AGO1-1:1 | SEQ2752 | 0.01587 | 0.75 | 0.96 | Inc-GOLGA8F-7:1 | SEQ2938 | 0.03175 | 1.78 | 0.92 |
| Inc-PMM1-1:1 | SEQ2761 | 0.01587 | 0.77 | 0.96 | Inc-LTC4S-1:1 | SEQ2941 | 0.03175 | 1.79 | 0.92 |
| Inc-GPAT4-2:2 | SEQ2763 | 0.01587 | 0.78 | 0.96 | Inc-RIPOR1-1:6 | SEQ2943 | 0.03175 | 1.79 | 0.92 |
| BACE1-AS:5 | SEQ2765 | 0.01587 | 0.79 | 0.96 | Inc-TIGD5-1:1 | SEQ2945 | 0.03175 | 1.80 | 0.92 |
| Inc-MFSD14B-18:1 | SEQ2773 | 0.01587 | 1.30 | 0.96 | Inc-NAIP-4:1 | SEQ2946 | 0.03175 | 1.80 | 0.92 |
| Inc-RARRES2-2:2 | SEQ2774 | 0.01587 | 1.31 | 0.96 | Inc-OR4F29-8:25 | SEQ2949 | 0.03175 | 1.81 | 0.92 |
| Inc-DNAH10-2:1 | SEQ2777 | 0.01587 | 1.33 | 0.96 | Inc-BDH1-5:8 | SEQ2950 | 0.03175 | 1.81 | 0.92 |
| Inc-OR4F15-4:5 | SEQ2780 | 0.01587 | 1.35 | 0.96 | Inc-SKI-5:1 | SEQ2951 | 0.03175 | 1.81 | 0.92 |
| Inc-IL17RA-33:6 | SEQ2788 | 0.01587 | 1.39 | 0.96 | MIR9-3HG:11 | SEQ2957 | 0.03175 | 1.82 | 0.92 |
| Inc-SEPT7-4:1 | SEQ2796 | 0.01587 | 1.42 | 0.96 | Inc-C1orf198-6:3 | SEQ2959 | 0.03175 | 1.84 | 0.92 |
| Inc-CCL27-2:1 | SEQ2801 | 0.01587 | 1.45 | 0.96 | CCDC183-AS1:3 | SEQ2960 | 0.03175 | 1.85 | 0.92 |
| Inc-ZNF417-1:2 | SEQ2808 | 0.01587 | 1.48 | 0.96 | Inc-OAZ3-2:12 | SEQ2965 | 0.03175 | 1.86 | 0.92 |
| Inc-PPP6R2-5:1 | SEQ2811 | 0.01587 | 1.49 | 0.96 | LHX4-AS1:3 | SEQ2966 | 0.03175 | 1.86 | 0.92 |
| Inc-PNOC-2:1 | SEQ2817 | 0.01587 | 1.51 | 0.96 | Inc-EPHA6-1:2 | SEQ2967 | 0.03175 | 1.87 | 0.92 |
| Inc-SPATA21-4:8 | SEQ2818 | 0.01587 | 1.51 | 0.96 | UGDH-AS1:8 | SEQ2968 | 0.03175 | 1.87 | 0.92 |
| Inc-EFCAB12-2:17 | SEQ2819 | 0.01587 | 1.51 | 0.96 | Inc-GRINA-2:1 | SEQ2969 | 0.03175 | 1.87 | 0.92 |
| Inc-TCP11-2:6 | SEQ2820 | 0.01587 | 1.51 | 0.96 | Inc-SAMD11-12:3 | SEQ2970 | 0.03175 | 1.87 | 0.92 |
| Inc-PDGFB-2:1 | SEQ2826 | 0.01587 | 1.52 | 0.96 | Inc-PLA2G6-1:2 | SEQ2971 | 0.03175 | 1.88 | 0.92 |
| Inc-YRDC-3:1 | SEQ2841 | 0.01587 | 1.56 | 0.96 | SVIL-AS1:17 | SEQ2972 | 0.03175 | 1.88 | 0.92 |
| Inc-RIOX2-3:4 | SEQ2842 | 0.01587 | 1.56 | 0.96 | SVIL-AS1:35 | SEQ2973 | 0.03175 | 1.88 | 0.92 |
| Inc-KMT2C-3:1 | SEQ2847 | 0.01587 | 1.58 | 0.96 | LINC01372:8 | SEQ2975 | 0.03175 | 1.89 | 0.92 |
| Inc-SULT1A4-4:1 | SEQ2851 | 0.01587 | 1.58 | 0.96 | Inc-IGIP-5:1 | SEQ2977 | 0.03175 | 1.90 | 0.92 |
| Inc-TSPAN33-1:1 | SEQ2853 | 0.01587 | 1.58 | 0.96 | Inc-ELK4-1:4 | SEQ2978 | 0.03175 | 1.90 | 0.92 |
| Inc-HMGCS2-1:2 | SEQ2855 | 0.01587 | 1.59 | 0.96 | MAGI2-AS3:51 | SEQ2983 | 0.03175 | 1.92 | 0.92 |
| Inc-NTN3-1:2 | SEQ2860 | 0.01587 | 1.59 | 0.96 | Inc-ZNF467-4:1 | SEQ2985 | 0.03175 | 1.92 | 0.92 |
| Inc-LAT-2:1 | SEQ2861 | 0.01587 | 1.60 | 0.96 | Inc-PABPN1L-1:1 | SEQ2986 | 0.03175 | 1.93 | 0.92 |
| Inc-HIST3H2BB-1:8 | SEQ2866 | 0.01587 | 1.60 | 0.96 | Inc-IGSF9B-2:1 | SEQ2987 | 0.03175 | 1.93 | 0.92 |
| Inc-CPLX1-2:8 | SEQ2869 | 0.01587 | 1.61 | 0.96 | Inc-MC5R-11:1 | SEQ2989 | 0.03175 | 1.93 | 0.92 |
| Inc-CXorf40B-3:1 | SEQ2872 | 0.01587 | 1.62 | 0.96 | Inc-CLRN2-1:2 | SEQ2991 | 0.03175 | 1.94 | 0.92 |
| Inc-FXYD2-2:1 | SEQ2873 | 0.01587 | 1.62 | 0.96 | Inc-COPZ2-1:8 | SEQ2992 | 0.03175 | 1.94 | 0.92 |
| Inc-FAAP20-2:1 | SEQ2889 | 0.01587 | 1.66 | 0.96 | Inc-DHRS7B-1:7 | SEQ2993 | 0.03175 | 1.94 | 0.92 |
| Inc-ANKRD34B-4:1 | SEQ2896 | 0.01587 | 1.69 | 0.96 | Inc-FAM184B-4:1 | SEQ2994 | 0.03175 | 1.94 | 0.92 |
| Inc-CA7-4:3 | SEQ2897 | 0.01587 | 1.69 | 0.96 | Inc-ISOC2-3:1 | SEQ2996 | 0.03175 | 1.95 | 0.92 |
| NTM-AS1:3 | SEQ2898 | 0.01587 | 1.69 | 0.96 | Inc-CSPG4-1:12 | SEQ2998 | 0.03175 | 1.95 | 0.92 |
| NTM-AS1:4 | SEQ2899 | 0.01587 | 1.69 | 0.96 | Inc-PPM1H-6:1 | SEQ3011 | 0.03175 | 2.01 | 0.92 |
| Inc-TCP10L-2:1 | SEQ2900 | 0.01587 | 1.69 | 0.96 | Inc-TTYH3-2:1 | SEQ3012 | 0.03175 | 2.01 | 0.92 |
| Inc-NAA38-4:1 | SEQ2903 | 0.01587 | 1.70 | 0.96 | Inc-TMC5-3:1 | SEQ3015 | 0.03175 | 2.01 | 0.92 |
| Inc-FAAP20-3:1 | SEQ2907 | 0.01587 | 1.71 | 0.96 | Inc-TMEM211-7:1 | SEQ3023 | 0.03175 | 2.04 | 0.92 |
| Inc-PGAM2-1:1 | SEQ2909 | 0.01587 | 1.71 | 0.96 | Inc-NBPF1-1:6 | SEQ3026 | 0.03175 | 2.05 | 0.92 |
| Inc-FBXO11-1:10 | SEQ2912 | 0.01587 | 1.72 | 0.96 | Inc-ATG4B-2:1 | SEQ3030 | 0.03175 | 2.06 | 0.92 |
| Inc-NEUROD1-2:3 | SEQ2918 | 0.01587 | 1.74 | 0.96 | Inc-PRKN-17:1 | SEQ3031 | 0.03175 | 2.06 | 0.92 |
| Inc-KLHL18-6:1 | SEQ2923 | 0.01587 | 1.75 | 0.96 | Inc-ATAD5-2:1 | SEQ3035 | 0.03175 | 2.07 | 0.92 |
| CCDC183-AS1:5 | SEQ2925 | 0.01587 | 1.76 | 0.96 | CNTFR-AS1:14 | SEQ3038 | 0.03175 | 2.08 | 0.92 |
| Inc-CNTN2-4:1 | SEQ2926 | 0.01587 | 1.76 | 0.96 | Inc-TRUB2-8:1 | SEQ3040 | 0.03175 | 2.08 | 0.92 |
| Inc-TAX1BP3-1:2 | SEQ2927 | 0.01587 | 1.76 | 0.96 | ASB16-AS1:9 | SEQ3041 | 0.03175 | 2.08 | 0.92 |
| Inc-UNC5B-1:1 | SEQ2930 | 0.01587 | 1.77 | 0.96 | Inc-RNF24-2:8 | SEQ3043 | 0.03175 | 2.10 | 0.92 |
| LMCD1-AS1:11 | SEQ2931 | 0.01587 | 1.77 | 0.96 | Inc-DNAJB6-10:3 | SEQ3044 | 0.03175 | 2.10 | 0.92 |
| Inc-ATAD5-5:1 | SEQ2937 | 0.01587 | 1.78 | 0.96 | Inc-DNAJB6-10:2 | SEQ3045 | 0.03175 | 2.10 | 0.92 |
| Inc-CASP9-5:1 | SEQ2940 | 0.01587 | 1.79 | 0.96 | Inc-DNAAF5-1:6 | SEQ3048 | 0.03175 | 2.11 | 0.92 |
| Inc-PEX6-1:1 | SEQ2942 | 0.01587 | 1.79 | 0.96 | Inc-SCIMP-1:1 | SEQ3051 | 0.03175 | 2.13 | 0.92 |
| Inc-SMAD7-2:3 | SEQ2947 | 0.01587 | 1.80 | 0.96 | Inc-PFDN4-11:4 | SEQ3054 | 0.03175 | 2.13 | 0.92 |
| Inc-RHNO1-1:1 | SEQ0026 | 0.01587 | 1.81 | 0.96 | PPP3CB-AS1:5 | SEQ3057 | 0.03175 | 2.14 | 0.92 |
| Inc-GTF2H2C-3:1 | SEQ2952 | 0.01587 | 1.82 | 0.96 | Inc-MSLNL-3:1 | SEQ3059 | 0.03175 | 2.15 | 0.92 |
| Inc-PPEF1-7:2 | SEQ2953 | 0.01587 | 1.82 | 0.96 | Inc-HAS1-2:1 | SEQ3061 | 0.03175 | 2.15 | 0.92 |
| Inc-SDCCAG8-5:1 | SEQ2954 | 0.01587 | 1.82 | 0.96 | Inc-CPLX1-11:1 | SEQ3064 | 0.03175 | 2.16 | 0.92 |
| Inc-BDH1-5:11 | SEQ2956 | 0.01587 | 1.82 | 0.96 | Inc-PPDPF-2:1 | SEQ3067 | 0.03175 | 2.18 | 0.92 |
| Inc-CRYM-3:2 | SEQ2962 | 0.01587 | 1.85 | 0.96 | Inc-GLRX5-2:2 | SEQ3070 | 0.03175 | 2.20 | 0.92 |
| Inc-CRABP2-3:1 | SEQ2963 | 0.01587 | 1.86 | 0.96 | TNRC6C-AS1:1 | SEQ3072 | 0.03175 | 2.20 | 0.92 |
| Inc-PRKN-10:1 | SEQ2964 | 0.01587 | 1.86 | 0.96 | ITPK1-AS1:2 | SEQ3073 | 0.03175 | 2.20 | 0.92 |
| Inc-PACRG-2:1 | SEQ2974 | 0.01587 | 1.88 | 0.96 | ITPK1-AS1:1 | SEQ3074 | 0.03175 | 2.20 | 0.92 |
| PCBP1-AS1:201 | SEQ2976 | 0.01587 | 1.90 | 0.96 | Inc-SLC7A14-3:12 | SEQ3079 | 0.03175 | 2.22 | 0.92 |
| Inc-NPTX1-4:1 | SEQ2980 | 0.01587 | 1.91 | 0.96 | MALAT1:26 | SEQ3081 | 0.03175 | 2.23 | 0.92 |
| Inc-CHN2-2:4 | SEQ2981 | 0.01587 | 1.92 | 0.96 | Inc-GPC2-2:6 | SEQ3088 | 0.03175 | 2.25 | 0.92 |
| Inc-ZNF705D-6:1 | SEQ2982 | 0.01587 | 1.92 | 0.96 | Inc-GSG1L-1:9 | SEQ3090 | 0.03175 | 2.26 | 0.92 |
| Inc-PCIF1-1:1 | SEQ2984 | 0.01587 | 1.92 | 0.96 | Inc-COX19-2:1 | SEQ3091 | 0.03175 | 2.26 | 0.92 |
| Inc-OSGIN1-1:9 | SEQ2988 | 0.01587 | 1.93 | 0.96 | Inc-LGR6-8:1 | SEQ3092 | 0.03175 | 2.27 | 0.92 |
| Inc-PEX10-6:1 | SEQ2995 | 0.01587 | 1.95 | 0.96 | Inc-LRIG2-5:1 | SEQ3093 | 0.03175 | 2.27 | 0.92 |
| Inc-OTOA-3:4 | SEQ2999 | 0.01587 | 1.96 | 0.96 | Inc-IL3RA-1 :22 | SEQ3101 | 0.03175 | 2.29 | 0.92 |
| ADNP-AS1:7 | SEQ3000 | 0.01587 | 1.96 | 0.96 | Inc-POM121L2-2:5 | SEQ3110 | 0.03175 | 2.32 | 0.92 |
| Inc-LRRK1-3:4 | SEQ0242 | 0.01587 | 1.97 | 0.96 | Inc-NMRK2-5:9 | SEQ3111 | 0.03175 | 2.32 | 0.92 |
| Inc-TMEM211-2:8 | SEQ0644 | 0.01587 | 1.99 | 0.96 | Inc-COA6-1:2 | SEQ3113 | 0.03175 | 2.32 | 0.92 |
| Inc-LRRC56-1:7 | SEQ3005 | 0.01587 | 2.00 | 0.96 | Inc-ATAD2B-2:1 | SEQ3116 | 0.03175 | 2.34 | 0.92 |
| Inc-OR4F29-8:23 | SEQ3007 | 0.01587 | 2.00 | 0.96 | Inc-SSH3-5:2 | SEQ3117 | 0.03175 | 2.35 | 0.92 |
| Inc-PILRB-1:5 | SEQ3009 | 0.01587 | 2.00 | 0.96 | ZMIZ1-AS1:22 | SEQ3122 | 0.03175 | 2.38 | 0.92 |
| Inc-CEP83-7:1 | SEQ3010 | 0.01587 | 2.01 | 0.96 | Inc-GLRX5-7:2 | SEQ3123 | 0.03175 | 2.38 | 0.92 |
| Inc-ALG12-5:3 | SEQ3016 | 0.01587 | 2.01 | 0.96 | Inc-FAM184B-6:1 | SEQ3128 | 0.03175 | 2.42 | 0.92 |
| Inc-GTPBP1-1:2 | SEQ3017 | 0.01587 | 2.02 | 0.96 | Inc-PRR26-2:1 | SEQ3129 | 0.03175 | 2.42 | 0.92 |
| Inc-PPIF-1:3 | SEQ3019 | 0.01587 | 2.02 | 0.96 | MALAT1:14 | SEQ3130 | 0.03175 | 2.43 | 0.92 |
| Inc-TOP1MT-5:1 | SEQ3021 | 0.01587 | 2.04 | 0.96 | Inc-SPANXB1-7:1 | SEQ3131 | 0.03175 | 2.44 | 0.92 |
| Inc-RPL12-1:3 | SEQ3024 | 0.01587 | 2.04 | 0.96 | Inc-CEL-5:2 | SEQ3132 | 0.03175 | 2.44 | 0.92 |
| Inc-NKPD1-1:1 | SEQ3025 | 0.01587 | 2.04 | 0.96 | Inc-CANT1-3:1 | SEQ3133 | 0.03175 | 2.44 | 0.92 |
| Inc-NEURL4-1:4 | SEQ3028 | 0.01587 | 2.05 | 0.96 | OLMALINC:4 | SEQ3137 | 0.03175 | 2.45 | 0.92 |
| DLG5-AS1:5 | SEQ3033 | 0.01587 | 2.06 | 0.96 | Inc-CRLF3-3:1 | SEQ3139 | 0.03175 | 2.46 | 0.92 |
| Inc-GRINA-3:1 | SEQ3047 | 0.01587 | 2.10 | 0.96 | L!NC01759:10 | SEQ3141 | 0.03175 | 2.48 | 0.92 |
| Inc-PLPP2-2:1 | SEQ3049 | 0.01587 | 2.11 | 0.96 | Inc-RGS9-16:1 | SEQ3149 | 0.03175 | 2.52 | 0.92 |
| Inc-TMEM163-2:1 | SEQ3055 | 0.01587 | 2.14 | 0.96 | Inc-KCNQ2-2:2 | SEQ3151 | 0.03175 | 2.52 | 0.92 |
| Inc-LGALS7B-1:4 | SEQ3056 | 0.01587 | 2.14 | 0.96 | Inc-FAM104A-8:2 | SEQ3153 | 0.03175 | 2.56 | 0.92 |
| Inc-CNP-1:1 | SEQ3058 | 0.01587 | 2.14 | 0.96 | Inc-EMX1-1:1 | SEQ3154 | 0.03175 | 2.56 | 0.92 |
| Inc-HGH1-1:1 | SEQ3060 | 0.01587 | 2.15 | 0.96 | Inc-SPANXB1-7:5 | SEQ3157 | 0.03175 | 2.57 | 0.92 |
| Inc-ERV3-1-10:8 | SEQ3062 | 0.01587 | 2.16 | 0.96 | Inc-MINDY1-2:1 | SEQ3163 | 0.03175 | 2.66 | 0.92 |
| ZMIZ1-AS1:31 | SEQ3065 | 0.01587 | 2.17 | 0.96 | Inc-PLEKHA2-4:1 | SEQ3164 | 0.03175 | 2.67 | 0.92 |
| Inc-SPANXB1-7:7 | SEQ3066 | 0.01587 | 2.17 | 0.96 | Inc-CDCA2-4:1 | SEQ3166 | 0.03175 | 2.68 | 0.92 |
| Inc-ERLEC1-6:1 | SEQ3068 | 0.01587 | 2.18 | 0.96 | SNHG12:10 | SEQ3169 | 0.03175 | 2.71 | 0.92 |
| Inc-NAV1-8:1 | SEQ3069 | 0.01587 | 2.20 | 0.96 | Inc-TXNDC15-1:1 | SEQ3177 | 0.03175 | 2.79 | 0.92 |
| Inc-SELENON-2:2 | SEQ3071 | 0.01587 | 2.20 | 0.96 | Inc-LRRC56-1:11 | SEQ3181 | 0.03175 | 2.87 | 0.92 |
| Inc-MYO15B-3:1 | SEQ3076 | 0.01587 | 2.21 | 0.96 | Inc-DRICH1-3:35 | SEQ3187 | 0.03175 | 2.97 | 0.92 |
| Inc-ARFRP1-1:1 | SEQ3077 | 0.01587 | 2.21 | 0.96 | Inc-SNX20-8:1 | SEQ3192 | 0.03175 | 2.98 | 0.92 |
| INc-RFPL3S-4:1 | SEQ3082 | 0.01587 | 2.23 | 0.96 | Inc-SLC7A7-1:6 | SEQ3194 | 0.03175 | 3.04 | 0.92 |
| Inc-NPC1-1:1 | SEQ3087 | 0.01587 | 2.24 | 0.96 | Inc-ST18-4:4 | SEQ3201 | 0.03175 | 309 | 0.92 |
| LINC01608:19 | SEQ3097 | 0.01587 | 2.29 | 0.96 | Inc-CANT1-2:5 | SEQ3203 | 0.03175 | 3.11 | 0.92 |
| Inc-DRICH1-3:51 | SEQ3098 | 0.01587 | 2.29 | 0.96 | Inc-TRUB2-8:2 | SEQ3205 | 0.03175 | 3.13 | 0.92 |
| Inc-KIF13B-2:1 | SEQ3100 | 0.01587 | 2.29 | 0.96 | Inc-SLC29A4-5:9 | SEQ3207 | 0.03175 | 3.15 | 0.92 |
| Inc-TCF19-1:54 | SEQ3105 | 0.01587 | 2.31 | 0.96 | Inc-DCP1B-6:1 | SEQ3210 | 0.03175 | 3.17 | 0.92 |
| Inc-CCL25-1:5 | SEQ3109 | 0.01587 | 2.32 | 0.96 | Inc-SFTPA2-6:1 | SEQ3211 | 0.03175 | 3.18 | 0.92 |
| Inc-GOLGA8O-3:6 | SEQ3115 | 0.01587 | 2.32 | 0.96 | Inc-FAM187A-2:1 | SEQ3217 | 0.03175 | 3.28 | 0.92 |
| Inc-GRINA-1:1 | SEQ3118 | 0.01587 | 2.36 | 0.96 | DANT2:3 | SEQ3220 | 0.03175 | 3.36 | 0.92 |
| Inc-ANKRD20A3-19:1 | SEQ3119 | 0.01587 | 2.36 | 0.96 | Inc-BLM-6:5 | SEQ3223 | 0.03175 | 3.39 | 0.92 |
| Inc-CLRN2-1:1 | SEQ3124 | 0.01587 | 2.40 | 0.96 | Inc-OR4F3-5:9 | SEQ3225 | 0.03175 | 3.42 | 0.92 |
| NORAD:2 | SEQ3125 | 0.01587 | 2.41 | 0.96 | LINC00639:9 | SEQ3235 | 0.03175 | 3.85 | 0.92 |
| TNRC6C-AS1:5 | SEQ3142 | 0.01587 | 2.48 | 0.96 | Inc-SSTR2-5:1 | SEQ3238 | 0.03175 | 4.08 | 0.92 |
| TNRC6C-AS1:2 | SEQ3143 | 0.01587 | 2.48 | 0.96 | LINC01608:17 | SEQ3243 | 0.03175 | 4.20 | 0.92 |
| Inc-NBPF3-3:6 | SEQ3144 | 0.01587 | 2.49 | 0.96 | Inc-CD46-7:4 | SEQ3254 | 0.03175 | 5.19 | 0.92 |
| GSN-AS1:1 | SEQ3146 | 0.01587 | 2.50 | 0.96 | LINC01715:4 | SEQ3256 | 0.03175 | 5.35 | 0.92 |
| MIR646HG:31 | SEQ3148 | 0.01587 | 2.51 | 0.96 | TDRG1:6 | SEQ3270 | 0.03175 | 8.23 | 0.92 |
| Inc-IL3RA-1:18 | SEQ3155 | 0.01587 | 2.57 | 0.96 | MALAT1:11 | SEQ3276 | 0.03175 | 25.39 | 0.92 |
| Inc-SULT1A3-5:1 | SEQ3161 | 0.01587 | 2.59 | 0.96 | LINC00092:11 | SEQ3278 | 0.03175 | 41.83 | 0.92 |

386 IncRNAs comprising 28 novel IncRNAs showed (i) high expression in postmortem brain (CPM>25), (ii) significant differential expression in AD brain versus HC brain, and (iii) no or low expression (<5 CPM) in plasma and blood (Paxgene) of living AD patients or healthy controls. These 386 IncRNAs are listed and their profile included in Tables 7 and 8.

To identify for diagnostic and therapeutic applications, circulating IncRNAs in the peripheral body fluids, blood and plasma lncRNA signatures involved specifically in the pathogenesis of brain disorders, including but not limited to cognitive disorders such as mild cognitive impairment (MCI), Alzheimer (AD), frontotemporal (FTD) dementia and/or dementia with Lewy bodies (DLB), a total of 128,893 IncRNAs comprising the 1091 novel IncRNAs and the 127802 IncRNAs based on LNCipedia were screened and profiled in the brain from AD cases and from non-demented cases as well as in plasma and/or whole blood samples from different groups of subjects, including a group of patients with MCI, a group of patients with mild AD, a group of patients with moderate-to-severe AD, a group of patients with DLB and a group of patients with FTD as well as a control reference group of cognitively intact healthy controls having normal neurocognitive scores and with no brain imaging abnormalities, have been screened.

Subsequently, out of 128 894 IncRNAs in all samples, 53747 IncRNAs in brain samples, 35618 IncRNAs in plasma samples, 71132 IncRNAs in whole blood (Paxgene RNA tubes) samples have been detected with the level of at least one count in a sample among the samples included in the experiments. Further applying an additional threshold of expression level > 5CPM in 50% of samples from at least one of the subject groups studied, the following numbers of IncRNAs were retained for Statistical analysis: 10122 brain IncRNAs, 3774 plasma IncRNAs and 9367 whole blood IncRNAs.

For each lncRNA, the differential expression level was determined by comparing the expression level between two groups (for example AD group and healthy control group), using a two-tailed Welch t test and/or Wilcoxon Mann-Whitney test. Significant differential expression was identified as p<0.05. 410 IncRNAs were differentially expressed in AD plasma versus HC plasma, with statistically significance (p<0.05, Wilcoxon test) and a fold change FC<0.80 or >1.20. Table 9 shows the 410 plasma IncRNAs.

**Table 9: The 410 plasma IncRNAs that are differentially expressed in AD plasma versus HC plasma.**

| **lncRNA** | **SEQ** | **p value** | **FC** | **AUC** | **lncRNA** | **SEQ** | **p value** | **FC** | **AUC** |
|---|---|---|---|---|---|---|---|---|---|
| lnc-KLHL36-2:2 | SEQ3279 | 0.0022 | 2.46 | 1.00 | lnc-PYDC2-5:1 | SEQ3459 | 0.0022 | 10.28 | 1.00 |
| LINC01000:5 | SEQ3280 | 0.0022 | 0.20 | 1.00 | lnc-PPIAL4D-5:7 | SEQ3460 | 0.0411 | 0.38 | 0.86 |
| lnc-ABCC5-3:1 | SEQ3281 | 0.0022 | 2.44 | 1.00 | lnc-REL-6:3 | SEQ3461 | 0.0043 | 13.49 | 0.97 |
| lnc-ABCC5-3:4 | SEQ3282 | 0.0022 | 3.49 | 1.00 | lnc-ZNF460-2:1 | SEQ0473 | 0.0450 | 6.14 | 0.86 |
| lnc-ANAPC11-2:8 | SEQ3283 | 0.0022 | 2.51 | 1.00 | lnc-TOP1-9:1 | SEQ3462 | 0.0260 | 4.56 | 0.89 |
| lnc-CLLU1-2:6 | SEQ3284 | 0.0022 | 3.65 | 1.00 | TCONS_00050494 | SEQ1818 | 0.0087 | 3.27 | 0.94 |
| lnc-CREB3L1-1:6 | SEQ3285 | 0.0022 | 0.30 | 1.00 | lnc-TAF9-10:1 | SEQ0639 | 0.0411 | 3.12 | 0.86 |
| lnc-FGL2-3:1 | SEQ3286 | 0.0022 | 3.99 | 1.00 | lnc-UROC1-1:1 | SEQ3463 | 0.0115 | 12.52 | 0.94 |
| lnc-HERC3-1:7 | SEQ3287 | 0.0022 | 2.87 | 1.00 | lnc-PMM2-6:4 | SEQ2411 | 0.0260 | 3.55 | 0.89 |
| lnc-IL9R-1:5 | SEQ3288 | 0.0022 | 0.00 | 1.00 | lnc-SPATA32-2:4 | SEQ3464 | 0.0411 | 4.81 | 0.86 |
| lnc-LCOR-4:3 | SEQ3289 | 0.0022 | 4.31 | 1.00 | lnc-ZNF843-2:4 | SEQ3465 | 0.0303 | 347 | 0.89 |
| lnc-LRRC10-1:1 | SEQ3290 | 0.0022 | 3.26 | 1.00 | lnc-RIPOR2-7:1 | SEQ3466 | 0.0411 | 3.56 | 0.86 |
| lnc-MAGEE2-2:1 | SEQ3291 | 0.0050 | 5.09 | 1.00 | lnc-NUTM1-6:4 | SEQ3467 | 0.0043 | 6.40 | 0.97 |
| lnc-NEK6-2:1 | SEQ2787 | 0.0022 | 5.82 | 1.00 | lnc-PFDN6-2:3 | SEQ3468 | 0.0411 | 3.59 | 0.86 |
| lnc-NEK6-2:4 | SEQ2786 | 0.0022 | 4.65 | 1.00 | lnc-SSH3-5:1 | SEQ2791 | 0.0152 | 2.07 | 0.92 |
| CCDC183-AS1:5 | SEQ2925 | 0.0260 | 4.07 | 0.89 | lnc-USP47-2:1 | SEQ3469 | 0.0022 | 3.87 | 1.00 |
| CRTC3-AS1:3 | SEQ3292 | 0.0022 | 3.33 | 1.00 | lnc-STX10-2:1 | SEQ3470 | 0.0022 | 6.21 | 1.00 |
| FGD5-AS1:14 | SEQ3293 | 0.0152 | 2.71 | 0.92 | lnc-SMN2-4:2 | SEQ3471 | 0.0022 | 19.3 | 1.00 |
| LINC02288:8 | SEQ3294 | 0.0087 | 5.60 | 0.94 | lnc-TCF7-1:3 | SEQ0990 | 0.0022 | 3.05 | 1.00 |
| lnc-ABTB2-3:1 | SEQ3295 | 0.0129 | 4.23 | 0.94 | lnc-SBK1-1:1 | SEQ3472 | 0.0050 | 6.05 | 1.00 |
| lnc-ARAF-4:1 | SEQ3296 | 0.0260 | 2.34 | 0.89 | lnc-RBM12B-10:1 | SEQ3473 | 0.0087 | 0.42 | 0.94 |
| lnc-ATP6V1C2-2:2 | SEQ2695 | 0.0260 | 4.33 | 0.89 | lnc-RNF39-9:1 | SEQ3474 | 0.0087 | 1.54 | 0.94 |
| lnc-ATXN2-1:1 | SEQ0099 | 0.0087 | 1.62 | 0.94 | lnc-ZC3H12B-1:5 | SEQ3475 | 0.0043 | 0.51 | 0.97 |
| lnc-BORA-25:1 | SEQ3297 | 0.0260 | 3.39 | 0.89 | lnc-RMDN2-3:24 | SEQ2468 | 0.0022 | 2.35 | 1.00 |
| lnc-DEFB115-5:1 | SEQ3298 | 0.0081 | 174.03 | 0.97 | lnc-ZNF552-3:1 | SEQ3476 | 0.0022 | 2.28 | 1.00 |
| lnc-DTX1-1:1 | SEQ3299 | 0.0450 | 3.40 | 0.86 | lnc-SPRYD3-1:17 | SEQ3477 | 0.0043 | 2.84 | 0.97 |
| lnc-EXOSC6-1:2 | SEQ3300 | 0.0411 | 3.95 | 0.86 | lnc-ROM1-7:1 | SEQ3478 | 0.0022 | 2.57 | 1.00 |
| lnc-FAM174A-6:2 | SEQ2631 | 0.0411 | 2.87 | 0.86 | lnc-OSBPL7-1:1 | SEQ3479 | 0.0050 | 4.81 | 1.00 |
| lnc-GCN1-3:1 | SEQ3301 | 0.0450 | 3.14 | 0.86 | lnc-ZNF559-ZNF177-1:1 | SEQ3480 | 0.0043 | 11.85 | 1.00 |
| lnc-GIMAP5-1:1 | SEQ3302 | 0.0152 | 6.09 | 0.92 | lnc-SLC45A3-3:1 | SEQ3481 | 0.0022 | 3.96 | 1.00 |
| lnc-IL18BP-1:1 | SEQ3303 | 0.0411 | 2.29 | 0.86 | lnc-PMM2-2:1 | SEQ3482 | 0.0028 | Inf | 1.00 |
| lnc-MIS12-3:1 | SEQ3304 | 0.0260 | 1.66 | 0.89 | SNHG12:13 | SEQ3483 | 0.0022 | 26.17 | 1.00 |
| lnc-NLRC4-3:3 | SEQ3305 | 0.0087 | 3.16 | 0.94 | TSPOAP1-AS1:39 | SEQ3484 | 0.0022 | 169 | 1.00 |
| lnc-NPIPB13-3:1 | SEQ3306 | 0.0152 | 3.15 | 0.92 | TCONS_00019798 | SEQ1305 | 0.0022 | 4.07 | 1.00 |
| CA3-AS1:13 | SEQ3307 | 0.0087 | 0.25 | 0.94 | lnc-PAPLN-1:1 | SEQ3485 | 0.0411 | 2.41 | 0.86 |
| CSTF3-AS1:10 | SEQ3308 | 0.0152 | 2.30 | 0.92 | lnc-SYCP1-7:1 | SEQ3486 | 0.0260 | 1.50 | 0.89 |
| EP300-AS1:13 | SEQ2778 | 0.0043 | 0.45 | 0.97 | lnc-RABL2B-3:1 | SEQ3487 | 0.0411 | 6.26 | 0.86 |
| FAM239A:16 | SEQ3309 | 0.0260 | 2.85 | 0.89 | lnc-SIAH1-1:1 | SEQ3488 | 0.0152 | 1.80 | 0.92 |
| FGD5-AS1:29 | SEQ3310 | 0.0260 | 3.67 | 0.89 | lnc-RAB17-2:1 | SEQ3489 | 0.0087 | 2.41 | 0.94 |
| FGD5-AS1:8 | SEQ3311 | 0.0411 | 0.10 | 0.86 | lnc-ZCCHC7-2:12 | SEQ3490 | 0.0260 | 7.75 | 0.89 |
| GAS5:31 | SEQ3312 | 0.0411 | 4.65 | 0.86 | lnc-NRGN-1:6 | SEQ3491 | 0.0411 | 0.41 | 0.86 |
| H1FX-AS1:15 | SEQ3313 | 0.0260 | 3.93 | 0.89 | lnc-TEKT1-2:1 | SEQ3492 | 0.0087 | 2.51 | 0.94 |
| HCG11:10 | SEQ3314 | 0.0043 | 30.61 | 0.97 | TCONS_00066544 | SEQ2098 | 0.0303 | 6.13 | 0.89 |
| HCG11:2 | SEQ3315 | 0.0043 | 30.61 | 0.97 | lnc-REL-1:3 | SEQ2508 | 0.0260 | 2.57 | 0.89 |
| HCG11:9 | SEQ3316 | 0.0043 | 30.61 | 0.97 | lnc-SATB1-8:1 | SEQ3493 | 0.0043 | 4.01 | 0.97 |
| IL10RB-AS1:3 | SEQ3317 | 0.0022 | 4.E+11 | 1.00 | lnc-RNMT-8:1 | SEQ3494 | 0.0284 | 0.00 | 0.83 |
| IQCH-AS1:17 | SEQ3318 | 0.0129 | 6.46 | 0.94 | lnc-YPEL5-5:1 | SEQ0750 | 0.0152 | 2.94 | 0.92 |
| JHDM1D-AS1:2 | SEQ3319 | 0.0260 | 0.07 | 0.89 | THRIL:1 | SEQ3495 | 0.0115 | 30.93 | 0.94 |
| LINC00869:69 | SEQ3320 | 0.0411 | 0.23 | 0.86 | TCONS_00050563 | SEQ1836 | 0.0411 | 2.84 | 0.86 |
| LINC00909:10 | SEQ3321 | 0.0411 | 4.91 | 0.86 | lnc-SYNJ2-1:1 | SEQ3496 | 0.0152 | 2.94 | 0.92 |
| LINC00926:6 | SEQ3322 | 0.0260 | 27.17 | 0.89 | lnc-STAT1-2:4 | SEQ3497 | 0.0087 | 2.75 | 0.94 |
| LINC01000:15 | SEQ3323 | 0.0411 | 2.95 | 0.86 | TCONS_00023171 | SEQ1367 | 0.0284 | Inf | 0.83 |
| LINC01419:1 | SEQ3324 | 0.0411 | 7.61 | 0.86 | PSMA3-AS1:27 | SEQ3498 | 0.0152 | 0.41 | 0.92 |
| LINC01473:6 | SEQ3325 | 0.0078 | 0.07 | 0.97 | TCONS_00045364 | SEQ1785 | 0.0152 | 3.23 | 0.92 |
| LINC01952:8 | SEQ3326 | 0.0411 | 1.90 | 0.86 | LRRC75A-AS1:3 | SEQ3499 | 0.0260 | 4.37 | 0.89 |
| LINC02062:2 | SEQ3327 | 0.0152 | 179.88 | 0.92 | lnc-TEAD2-2:4 | SEQ3500 | 0.0260 | 3.19 | 0.89 |
| LINC02397:10 | SEQ3328 | 0.0152 | 8.19 | 0.92 | lnc-YY1AP1-2:1 | SEQ3501 | 0.0303 | 3.93 | 0.89 |
| LIX1-AS1:2 | SEQ3329 | 0.0152 | 13.41 | 0.92 | lnc-STPG1-3:4 | SEQ3502 | 0.0087 | 4.72 | 0.94 |
| lnc-ACOT9-2:1 | SEQ3330 | 0.0087 | 2.32 | 0.94 | lnc-SH3D19-2:1 | SEQ3503 | 0.0260 | 2.86 | 0.89 |
| lnc-ADAM30-1:1 | SEQ2698 | 0.0295 | 6.92 | 0.89 | lnc-TRDMT1-5:3 | SEQ3504 | 0.0087 | 2.45 | 0.94 |
| lnc-AGO1-1:1 | SEQ2752 | 0.0129 | 5.66 | 0.94 | NNT-AS1:11 | SEQ3505 | 0.0411 | 0.18 | 0.86 |
| lnc-AGO3-3:1 | SEQ3331 | 0.0411 | 3.66 | 0.86 | lnc-OST4-6:2 | SEQ0954 | 0.0260 | 6.67 | 0.89 |
| lnc-AHSA2-5:6 | SEQ3332 | 0.0438 | 6.26 | 0.86 | lnc-SMG1-3:1 | SEQ3506 | 0.0200 | 3.60 | 0.92 |
| lnc-AIM2-3:3 | SEQ3333 | 0.0411 | 2.53 | 0.86 | lnc-ZNF613-2:1 | SEQ3507 | 0.0438 | 2.87 | 0.86 |
| lnc-AKAP12-2:1 | SEQ2375 | 0.0260 | 2.25 | 0.89 | lnc-ZNF891-1:1 | SEQ3508 | 0.0260 | 5.52 | 0.89 |
| lnc-AMPH-10:8 | SEQ3334 | 0.0115 | 7.51 | 0.94 | lnc-PTPA-3:4 | SEQ3509 | 0.0152 | 6.31 | 0.92 |
| lnc-ANAPC11-2:2 | SEQ3335 | 0.0411 | 3.91 | 0.86 | lnc-RAB37-1:4 | SEQ3510 | 0.0152 | 2.19 | 0.92 |
| lnc-ANGPTL1-2:1 | SEQ3336 | 0.0411 | 2.22 | 0.86 | lnc-TMEM212-1:4 | SEQ3511 | 0.0152 | 0.09 | 0.92 |
| lnc-ANKRD13A-4:1 | SEQ3337 | 0.0048 | 8.02 | 1.00 | lnc-RPL39L-3:1 | SEQ3512 | 0.0303 | 4.08 | 0.89 |
| lnc-ANKRD20A2-22:3 | SEQ3338 | 0.0411 | 3.36 | 0.86 | lnc-SLC25A47-5:1 | SEQ3513 | 0.0043 | 2.75 | 0.97 |
| lnc-ANO8-1:2 | SEQ3339 | 0.0250 | 22.08 | 0.89 | lnc-PLEKHA3-20:1 | SEQ3514 | 0.0260 | 2.39 | 0.89 |
| lnc-APBA2-5:4 | SEQ3340 | 0.0129 | 6.87 | 0.94 | lnc-TMEM30B-9:2 | SEQ3515 | 0.0411 | 4.59 | 0.86 |
| lnc-APTX-3:1 | SEQ2399 | 0.0411 | 1.92 | 0.86 | THAP9-AS1:28 | SEQ3516 | 0.0043 | 5.97 | 0.97 |
| lnc-ARF6-3:1 | SEQ3341 | 0.0087 | 2.93 | 0.94 | lnc-PAX8-6:1 | SEQ3517 | 0.0200 | 7.45 | 0.92 |
| lnc-ARL14EP-4:1 | SEQ3342 | 0.0043 | 10.23 | 1.00 | lnc-TAF1B-2:1 | SEQ3518 | 0.0167 | 162 | 0.90 |
| lnc-BCL2L13-3:1 | SEQ3343 | 0.0260 | 2.56 | 0.89 | lnc-ZNF121-1:1 | SEQ3519 | 0.0043 | 3.91 | 0.97 |
| lnc-BLACE-2:15 | SEQ3344 | 0.0087 | 0.27 | 0.94 | TCONS_00011972 | SEQ1217 | 0.0087 | 2.22 | 0.94 |
| lnc-BLACE-2:5 | SEQ3345 | 0.0152 | 0.03 | 0.92 | lnc-TIGD5-3:1 | SEQ3520 | 0.0087 | 2.50 | 0.94 |
| lnc-C10orf10-2:22 | SEQ3346 | 0.0260 | 1.67 | 0.89 | lnc-ROM1-7:5 | SEQ3521 | 0.0411 | 2.36 | 0.86 |
| lnc-C10orf10-2:23 | SEQ3347 | 0.0260 | 1.67 | 0.89 | lnc-RNF123-1:3 | SEQ3522 | 0.0260 | 4.31 | 0.89 |
| lnc-C11orf21-3:1 | SEQ3348 | 0.0411 | 2.71 | 0.86 | UGDH-AS1:10 | SEQ0407 | 0.0411 | 6.05 | 0.86 |
| lnc-C12orf73-1:1 | SEQ3349 | 0.0411 | 2.02 | 0.86 | lnc-RAB3C-1:1 | SEQ3523 | 0.0411 | 2.75 | 0.86 |
| lnc-C1orf112-4:2 | SEQ3350 | 0.0200 | 4.47 | 0.92 | lnc-ZNF506-2:3 | SEQ3524 | 0.0152 | 5.13 | 0.92 |
| lnc-C1QBP-1:1 | SEQ3351 | 0.0438 | 3.96 | 0.86 | WAC-AS1:12 | SEQ3525 | 0.0043 | 3.99 | 0.97 |
| lnc-C20orf96-1:1 | SEQ2764 | 0.0152 | 2.76 | 0.92 | SNHG16:2 | SEQ3526 | 0.0411 | 7.48 | 0.86 |
| lnc-C6orf106-2:2 | SEQ3352 | 0.0043 | 1.79 | 0.97 | TCONS_00037052 | SEQ1619 | 0.0438 | 6.17 | 0.86 |
| lnc-C9orf152-5:1 | SEQ3353 | 0.0303 | 4.54 | 0.89 | lnc-TMTC3-1:1 | SEQ3527 | 0.0411 | 0.44 | 0.86 |
| lnc-C9orf57-1:1 | SEQ3354 | 0.0152 | 6.22 | 0.92 | lnc-TSTD3-1:3 | SEQ3528 | 0.0043 | 2.63 | 0.97 |
| lnc-CBARP-2:1 | SEQ3355 | 0.0411 | 2.34 | 0.86 | ZMYM4-AS1:1 | SEQ3529 | 0.0087 | 2.47 | 0.94 |
| lnc-CCT4-1:8 | SEQ3356 | 0.0411 | 2.60 | 0.86 | lnc-PPP4C-2:7 | SEQ3530 | 0.0087 | 2.23 | 0.94 |
| lnc-CDC42BPB-4:1 | SEQ3357 | 0.0411 | 3.25 | 0.86 | lnc-TM2D2-1:1 | SEQ3531 | 0.0260 | 2.74 | 0.89 |
| lnc-CDH20-3:1 | SEQ3358 | 0.0087 | 3.08 | 0.94 | lnc-PCED1B-5:3 | SEQ3532 | 0.0260 | 0.11 | 0.89 |
| lnc-CDIPT-1:11 | SEQ3359 | 0.0152 | 2.30 | 0.92 | lnc-ZGRF1-8:1 | SEQ3533 | 0.0260 | 2.15 | 0.89 |
| lnc-CDK12-2:5 | SEQ3360 | 0.0411 | 19.25 | 0.86 | lnc-SAG-3:1 | SEQ3534 | 0.0260 | 6.89 | 0.89 |
| lnc-CDK2AP1-1:9 | SEQ3361 | 0.0411 | 2.20 | 0.86 | lnc-ZCCHC7-2:1 | SEQ3535 | 0.0411 | 2.28 | 0.86 |
| lnc-CEACAM21-5:6 | SEQ3362 | 0.0087 | 5.09 | 0.94 | lnc-TMX2-1:1 | SEQ3536 | 0.0078 | 9.44 | 0.97 |
| lnc-CECR2-1:4 | SEQ3363 | 0.0152 | 74.37 | 0.92 | lnc-SULT1A4-1:30 | SEQ3537 | 0.0411 | 3.45 | 0.86 |
| lnc-CELA3A-5:11 | SEQ3364 | 0.0303 | 2.E+09 | 0.89 | lnc-TET3-2:2 | SEQ3538 | 0.0124 | 5.24 | 0.94 |
| lnc-CEMP1-5:1 | SEQ3365 | 0.0200 | 4.68 | 0.92 | NDUFA6-AS1:42 | SEQ3539 | 0.0260 | 2.90 | 0.89 |
| lnc-CISD2-1:3 | SEQ3366 | 0.0152 | 2.57 | 0.92 | lnc-UBAC1-2:2 | SEQ3540 | 0.0303 | 14.74 | 0.89 |
| lnc-CKS1B-3:1 | SEQ3367 | 0.0087 | 3.27 | 0.94 | lnc-SNX14-5:1 | SEQ3541 | 0.0152 | 2.14 | 0.92 |
| lnc-CLDN11-7:1 | SEQ3368 | 0.0260 | 0.32 | 0.89 | lnc-PPA2-1:1 | SEQ3542 | 0.0087 | 3.28 | 0.94 |
| lnc-CLEC2D-8:7 | SEQ3369 | 0.0152 | 4.52 | 0.92 | lnc-S1PR1-6:1 | SEQ3543 | 0.0260 | 2.34 | 0.89 |
| lnc-CNTRL-6:8 | SEQ3370 | 0.0250 | 5.E+05 | 0.89 | lnc-TARS-6:1 | SEQ3544 | 0.0260 | 2.32 | 0.89 |
| lnc-CPM-1:1 | SEQ3371 | 0.0048 | 21.75 | 1.00 | lnc-USP35-11:2 | SEQ2802 | 0.0411 | 1.87 | 0.86 |
| lnc-CTSV-3:1 | SEQ3372 | 0.0411 | 2.91 | 0.86 | PSMA3-AS1:8 | SEQ3545 | 0.0260 | 0.20 | 0.89 |
| lnc-CXCL2-3:1 | SEQ3373 | 0.0411 | 0.53 | 0.86 | TCONS_00027402 | SEQ1445 | | 0.07 | 0.89 |
| lnc-DALRD3-1:4 | SEQ3374 | 0.0260 | 3.91 | 0.89 | OR2A1-AS1:19 | SEQ3546 | 0.0129 | 24.12 | 0.94 |
| Inc-DAO-3:1 | SEQ0685 | 0.0043 | 1.70 | 0.97 | lnc-POLR3E-3:4 | SEQ3547 | 0.0152 | 1.71 | 0.92 |
| lnc-DCAF1-1:1 | SEQ3375 | 0.0087 | 2.54 | 0.94 | lnc-RMDN2-3:2 | SEQ3548 | 0.0411 | 3.24 | 0.86 |
| lnc-DEF8-1:1 | SEQ3376 | 0.0303 | 5.60 | 0.89 | lnc-SLC25A29-1:2 | SEQ3549 | 0.0081 | 8.76 | 0.97 |
| lnc-DNAL4-4:1 | SEQ3095 | 0.0411 | 4.63 | 0.86 | ZBTB11-AS1:9 | SEQ3550 | 0.0411 | 54.24 | 0.86 |
| lnc-DPH5-5:1 | SEQ3377 | 0.0048 | 17.28 | 1.00 | PEG13:2 | SEQ3551 | 0.0411 | 3.20 | 0.86 |
| lnc-DUSP26-14:1 | SEQ3378 | 0.0043 | 1.99 | 0.97 | lnc-PHF3-12:1 | SEQ3552 | 0.0152 | 2.20 | 0.92 |
| lnc-EBF3-6:2 | SEQ3379 | 0.0152 | 0.33 | 0.92 | lnc-ZNF16-2:11 | SEQ3553 | 0.0152 | 3.42 | 0.92 |
| lnc-EFCAB8-1:3 | SEQ3380 | 0.0411 | 2.73 | 0.86 | RAD51-AS1:7 | SEQ3554 | 0.0341 | 264.44 | 0.88 |
| lnc-EIF1AD-1:1 | SEQ2653 | 0.0152 | 2.12 | 0.92 | lnc-SLC17A5-1:7 | SEQ3555 | 0.0260 | 0.18 | 0.89 |
| lnc-ENOPH1-3:3 | SEQ3381 | 0.0087 | 2.42 | 0.94 | lnc-TMEM155-3:1 | SEQ3556 | 0.0043 | 3.02 | 0.97 |
| lnc-EPGN-4:1 | SEQ3382 | 0.0411 | 0.05 | 0.86 | SH3BP5-AS1:2 | SEQ3557 | 0.0260 | 2.54 | 0.89 |
| lnc-ERCC6L2-14:1 | SEQ3383 | 0.0152 | 2.20 | 0.92 | lnc-SPRED2-22:1 | SEQ2584 | 0.0260 | 2.44 | 0.89 |
| lnc-ESRP2-2:9 | SEQ3384 | 0.0260 | 2.54 | 0.89 | lnc-NSUN5-2:1 | SEQ3558 | 0.0411 | 2.72 | 0.86 |
| lnc-EXOC3L4-3:1 | SEQ3385 | 0.0043 | 13.96 | 0.97 | lnc-TAGLN-2:1 | SEQ3559 | 0.0043 | 2.88 | 0.97 |
| lnc-EYS-1:1 | SEQ3386 | 0.0411 | 3.12 | 0.86 | lnc-OR1A2-1:1 | SEQ3560 | 0.0411 | 2.72 | 0.86 |
| lnc-FAAP100-2:1 | SEQ2299 | 0.0260 | 1.40 | 0.89 | lnc-SYNGR1-5:1 | SEQ3561 | 0.0260 | 0.52 | 0.89 |
| lnc-FAM103A1-2:5 | SEQ2607 | 0.0087 | 2.37 | 0.94 | POT1-AS1:2 | SEQ3562 | 0.0260 | 5.77 | 0.89 |
| Inc-FAM156A-2:1 | SEQ3387 | 0.0129 | 3.46 | 0.94 | TCONS_00011974 | SEQ1218 | 0.0411 | 2.10 | 0.86 |
| Inc-FAM177B-1:1 | SEQ3388 | 0.0411 | 1.86 | 0.86 | Inc-TMEM234-1:4 | SEQ3563 | 0.0260 | 3.40 | 0.89 |
| lnc-FAM231C-7:6 | SEQ3389 | 0.0260 | 2.23 | 0.89 | Inc-RRN3-3:5 | SEQ2770 | 0.0043 | 3.61 | 0.97 |
| Inc-FAM72B-7:2 | SEQ3390 | 0.0152 | 4.07 | 0.92 | Inc-STAU2-3:4 | SEQ3564 | 0.0072 | 33.24 | 0.97 |
| Inc-FAM72D-4:1 | SEQ3391 | 0.0260 | 2.98 | 0.89 | Inc-PLA2G15-2:2 | SEQ3565 | 0.0411 | 2.29 | 0.86 |
| Inc-FANCL-4:1 | SEQ2690 | 0.0152 | 2.43 | 0.92 | Inc-PXN-1:1 | SEQ2506 | 0.0411 | 3.05 | 0.86 |
| Inc-FBN1-3:2 | SEQ3392 | 0.0043 | 7.16 | 0.97 | lnc-UBAC1-2:1 | SEQ3566 | 0.0411 | 4.42 | 0.86 |
| Inc-FER-6:1 | SEQ3393 | 0.0260 | 3.17 | 0.89 | TUG1:21 | SEQ3567 | 0.0411 | 0.36 | 0.86 |
| Inc-FKBP6-4:1 | SEQ3394 | 0.0152 | 2.65 | 0.92 | MIR4453HG:2 | SEQ3568 | 0.0411 | 2.78 | 0.86 |
| Inc-FNDC1-9:9 | SEQ3395 | 0.0411 | 2.55 | 0.86 | Inc-SUGT1-3:1 | SEQ0131 | 0.0341 | 56.87 | 0.88 |
| Inc-GAPT-6:2 | SEQ3396 | 0.0152 | 5.48 | 0.92 | Inc-OSBPL7-3:1 | SEQ3569 | 0.0260 | 0.44 | 0.89 |
| Inc-GAS2-1:15 | SEQ3397 | 0.0411 | 2.21 | 0.86 | I n c-S LA2-1:1 | SEQ3570 | 0.0411 | 2.70 | 0.86 |
| Inc-GDAP2-3:2 | SEQ3398 | 0.0411 | 1.82 | 0.86 | Inc-TRIM28-13:1 | SEQ3571 | 0.0152 | 3.75 | 0.92 |
| lnc-GNA11-3:1 | SEQ2681 | 0.0411 | 2.23 | 0.86 | Inc-ZBTB20-1:4 | SEQ3572 | 0.0411 | 3.11 | 0.86 |
| lnc-GNA11-3:2 | SEQ3399 | 0.0438 | 5.64 | 0.86 | PXN-AS1:15 | SEQ3573 | 0.0411 | 4.12 | 0.86 |
| Inc-GOLGA8F-8:1 | SEQ3400 | | 3.85 | 0.89 | PSMA3-AS1:6 | SEQ3574 | 0.0087 | 14.53 | 0.94 |
| Inc-GPR160-2:1 | SEQ3401 | 0.0411 | 1.49 | 0.86 | Inc-SP140-3:4 | SEQ3575 | 0.0124 | 11.42 | 0.94 |
| lnc-GPR37L1-7:4 | SEQ3402 | 0.0260 | 3.80 | 0.89 | VIM-AS1:7 | SEQ3576 | 0.0411 | 5.99 | 0.86 |
| Inc-GPR83-5:1 | SEQ3403 | 0.0043 | 1.86 | 0.97 | Inc-TUFM-3:3 | SEQ3577 | 0.0260 | 2.21 | 0.89 |
| Inc-GRB7-1:1 | SEQ3404 | 0.0043 | 48.62 | 0.97 | Inc-SLC45A4-8:1 | SEQ3578 | 0.0152 | 3.34 | 0.92 |
| Inc-GRINA-2:1 | SEQ2969 | 0.0411 | 2.10 | 0.86 | lnc-RLIM-4:1 | SEQ3579 | 0.0260 | 2.41 | 0.89 |
| Inc-GRK4-2:7 | SEQ3405 | 0.0411 | 1.97 | 0.86 | Inc-RACK1-1:2 | SEQ3580 | 0.0260 | 4.55 | 0.89 |
| Inc-HAAO-7:6 | SEQ3406 | 0.0411 | 0.25 | 0.86 | Inc-TRIM28-13:2 | SEQ3581 | 0.0260 | 10.16 | 0.89 |
| lnc-HAO2-2:1 | SEQ3407 | 0.0078 | 27.85 | 0.97 | Inc-SPOCK2-1:2 | SEQ3582 | 0.0416 | 2.43 | 0.86 |
| Inc-HARS-1:1 | SEQ3408 | 0.0411 | 0.50 | 0.86 | Inc-USP44-3:1 | SEQ3583 | 0.0411 | 3.20 | 0.86 |
| Inc-HBG2-1:1 | SEQ3409 | 0.0043 | 3.45 | 0.97 | ZNF341-AS1:10 | SEQ3584 | 0.0087 | 1.96 | 0.94 |
| Inc-HEATR4-6:1 | SEQ3410 | 0.0341 | 12.48 | 0.88 | Inc-RBM11-5:4 | SEQ3585 | 0.0278 | 6.38 | 0.89 |
| Inc-HELB-2:1 | SEQ3411 | 0.0260 | 3.55 | 0.89 | TCONS_00066563 | SEQ2107 | 0.0260 | 0.19 | 0.89 |
| Inc-HLA-DOB-1:1 | SEQ3412 | 0.0450 | 4.00 | 0.86 | Inc-ROM1-7:3 | SEQ3586 | 0.0152 | 2.37 | 0.92 |
| Inc-HMBOX1-1:1 | SEQ3413 | 0.0087 | 33.72 | 0.94 | Inc-WNT1-5:1 | SEQ3587 | 0.0411 | 2.54 | 0.86 |
| lnc-HMGA1-2:11 | SEQ3414 | 0.0411 | 5.75 | 0.86 | Inc-WDR45B-1:2 | SEQ3588 | 0.0194 | 9.06 | 0.92 |
| lnc-HMGA1-2:4 | SEQ3415 | 0.0260 | 2.70 | 0.89 | Inc-ROM1-7:6 | SEQ3589 | 0.0411 | 2.31 | 0.86 |
| Inc-HMGN5-3:1 | SEQ3416 | 0.0260 | 2.42 | 0.89 | ST7-AS1:1 | SEQ3590 | 0.0411 | 1.76 | 0.86 |
| Inc-HMHB1-6:2 | SEQ3417 | 0.0087 | 4.03 | 0.94 | Inc-TBP-3:1 | SEQ3591 | 0.0411 | 2.93 | 0.86 |
| lnc-HTR1B-1:4 | SEQ3418 | 0.0411 | 3.85 | 0.86 | lnc-RASGRP1-6:1 | SEQ3592 | 0.0200 | 6.95 | 0.92 |
| lnc-IER5-3:1 | SEQ2286 | 0.0284 | Inf | 0.83 | lnc-ZNF726-5:2 | SEQ3593 | 0.0260 | 2.69 | 0.89 |
| lnc-IFFO1-3:1 | SEQ3419 | 0.0152 | 1.35 | 0.92 | TUG1:9 | SEQ3594 | 0.0411 | 2.02 | 0.86 |
| lnc-IFNG-3:1 | SEQ3420 | 0.0450 | 5.01 | 0.86 | lnc-SLC9A3-1:2 | SEQ3595 | 0.0200 | 9.54 | 0.92 |
| lnc-IGIP-2:3 | SEQ3421 | 0.0087 | 3.95 | 0.94 | lnc-RBL2-4:3 | SEQ3596 | 0.0081 | 9.83 | 0.97 |
| lnc-IGSF9B-2:1 | SEQ2987 | 0.0152 | 3.85 | 0.92 | lnc-VSNL1-5:1 | SEQ3597 | 0.0260 | 2.45 | 0.89 |
| lnc-IL18BP-1:2 | SEQ3422 | 0.0411 | 2.29 | 0.86 | TCONS_00059492 | SEQ1962 | 0.0438 | 3.97 | 0.86 |
| lnc-IL18BP-1:3 | SEQ3423 | 0.0411 | 2.29 | 0.86 | lnc-RIPOR2-3:1 | SEQ3598 | 0.0411 | 2.37 | 0.86 |
| lnc-IL20RA-3:1 | SEQ3424 | 0.0260 | 0.27 | 0.89 | lnc-TCTA-2:1 | SEQ2757 | 0.0260 | 1.35 | 0.89 |
| lnc-ITGB4-2:3 | SEQ3425 | 0.0260 | 2.59 | 0.89 | lnc-PSMC3IP-1:1 | SEQ3599 | 0.0260 | 3.02 | 0.89 |
| lnc-JAM2-11:1 | SEQ3426 | 0.0152 | 1.77 | 0.92 | lnc-PROC-1:11 | SEQ3600 | 0.0152 | 4.36 | 0.92 |
| lnc-JMJD6-1:5 | SEQ3427 | 0.0411 | 4.30 | 0.86 | TP53TG1:3 | SEQ3601 | 0.0043 | 21.53 | 0.97 |
| lnc-JMJD7-PLA2G4B-2:8 | SEQ3428 | 0.0411 | 3.59 | 0.86 | MALAT1:11 | SEQ3276 | 0.0260 | 2.03 | 0.89 |
| lnc-KALRN-5:10 | SEQ3429 | 0.0260 | 3.54 | 0.89 | lnc-PCGF2-4:1 | SEQ3602 | 0.0260 | 6.82 | 0.89 |
| lnc-KIAA1644-1:1 | SEQ3430 | 0.0411 | 3.72 | 0.86 | lnc-SQSTM1-2:2 | SEQ3603 | 0.0411 | 1.77 | 0.86 |
| lnc-KLHDC9-5:4 | SEQ3431 | 0.0260 | 3.67 | 0.89 | TUG1:44 | SEQ3604 | 0.0411 | 6.33 | 0.86 |
| lnc-KLHL25-6:1 | SEQ3432 | 0.0152 | 2.93 | 0.92 | SH3BP5-AS1:1 | SEQ3605 | 0.0260 | 2.54 | 0.89 |
| lnc-KRT80-3:1 | SEQ3433 | 0.0438 | 3.65 | 0.86 | TRG-AS1:14 | SEQ3606 | 0.0152 | 20.18 | 0.92 |
| lnc-LAMA5-1:4 | SEQ3434 | 0.0022 | 10.44 | 1.00 | lnc-RSC1A1-1:1 | SEQ3607 | 0.0411 | 2.65 | 0.86 |
| lnc-LAMA5-4:1 | SEQ3435 | 0.0260 | 2.18 | 0.89 | lnc-NUP153-3:5 | SEQ3608 | 0.0450 | 3.13 | 0.86 |
| lnc-LHPP-6:6 | SEQ3436 | 0.0411 | 3.85 | 0.86 | lnc-TAOK1-4:1 | SEQ3609 | 0.0043 | 3.26 | 0.97 |
| lnc-LIN7A-3:1 | SEQ3437 | 0.0167 | 12.31 | 0.90 | lnc-ZBTB2-6:1 | SEQ3610 | 0.0411 | 3.43 | 0.86 |
| lnc-LPIN3-1:1 | SEQ3438 | 0.0124 | 5.66 | 0.94 | lnc-PILRB-1:3 | SEQ3611 | 0.0411 | 39.86 | 0.86 |
| lnc-LRCH1-6:1 | SEQ3439 | 0.0152 | 1.86 | 0.92 | lnc-RBMS2-4:1 | SEQ2378 | 0.0087 | 4.62 | 0.94 |
| lnc-LRIG2-10:1 | SEQ3440 | 0.0411 | 1.81 | 0.86 | lnc-SUGCT-4:1 | SEQ3612 | 0.0152 | 2.79 | 0.92 |
| lnc-LRR1-1:2 | SEQ0813 | 0.0411 | 0.29 | 0.86 | lnc-RNF24-3:1 | SEQ3613 | 0.0411 | 1.57 | 0.86 |
| lnc-LRR1-1:3 | SEQ0814 | 0.0411 | 0.81 | 0.86 | lnc-NUS1-5:1 | SEQ3614 | 0.0411 | 2.03 | 0.86 |
| lnc-LRRC10-1:2 | SEQ3441 | 0.0087 | 3.06 | 0.94 | ZNF341-AS1:9 | SEQ3615 | 0.0152 | 1.81 | 0.92 |
| lnc-LRRC37A3-5:8 | SEQ3442 | 0.0050 | 1.E+21 | 1.00 | lnc-STAR-1:2 | SEQ3616 | 0.0043 | 2.51 | 0.97 |
| lnc-MANBA-2:9 | SEQ3443 | 0.0260 | 2.39 | 0.89 | TRHDE-AS1:14 | SEQ3617 | 0.0087 | 0.23 | 0.94 |
| lnc-MARCH7-3:1 | SEQ3444 | 0.0260 | 3.61 | 0.89 | lnc-SLC3A2-6:1 | SEQ2428 | 0.0411 | 1.89 | 0.86 |
| lnc-MASTL-6:1 | SEQ2430 | 0.0278 | 5.38 | 0.89 | lnc-WRB-7:2 | SEQ3618 | 0.0411 | 2.32 | 0.86 |
| lnc-MAX-16:1 | SEQ3445 | 0.0260 | 1.68 | 0.89 | lnc-PSMB9-6:5 | SEQ3619 | 0.0260 | 3.91 | 0.89 |
| lnc-MCTP2-7:4 | SEQ3446 | 0.0411 | 3.33 | 0.86 | lnc-TIPIN-1:1 | SEQ3620 | 0.0303 | 5.03 | 0.89 |
| lnc-MEP1B-2:1 | SEQ3447 | 0.0260 | 2.36 | 0.89 | lnc-SPATA32-4:1 | SEQ3621 | 0.0087 | 3.81 | 0.94 |
| lnc-MFSD13A-2:1 | SEQ3448 | 0.0152 | 2.34 | 0.92 | lnc-VSIG4-2:1 | SEQ3622 | 0.0087 | 3.33 | 0.94 |
| lnc-MIEN1-1:1 | SEQ3449 | 0.0411 | 2.60 | 0.86 | lnc-TRAK1-1:1 | SEQ3623 | 0.0379 | 41.45 | 0.86 |
| lnc-MLLT1-2:1 | SEQ3450 | 0.0152 | 2.16 | 0.92 | lnc-TMEM178A-1:7 | SEQ3624 | 0.0295 | 21.47 | 0.89 |
| lnc-MLX-3:1 | SEQ3451 | 0.0087 | 3.16 | 0.94 | PITPNA-AS1:3 | SEQ3625 | 0.0260 | 3.07 | 0.89 |
| lnc-MNX1-11:1 | SEQ3452 | 0.0124 | 5.74 | 0.94 | lnc-RNF135-1:27 | SEQ3626 | 0.0129 | 117.53 | 0.94 |
| lnc-MROH6-1:1 | SEQ3453 | 0.0129 | 17.08 | 0.94 | lnc-OTULIN-1:1 | SEQ3627 | 0.0260 | 3.04 | 0.89 |
| lnc-MYOM1-6:3 | SEQ3454 | 0.0022 | 5.94 | 1.00 | PSMA3-AS1:40 | SEQ3628 | 0.0152 | 7.16 | 0.92 |
| lnc-NAMPT-1:2 | SEQ3455 | 0.0260 | 2.03 | 0.89 | lnc-TSSK6-1:1 | SEQ3629 | 0.0152 | 4.37 | 0.92 |
| lnc-NINJ1-2:1 | SEQ3456 | 0.0043 | 2.75 | 0.97 | lnc-PLPP2-5:3 | SEQ3630 | 0.0411 | 1.71 | 0.86 |
| lnc-NR2F6-5:1 | SEQ3457 | 0.0043 | 2.51 | 0.97 | lnc-STON2-5:1 | SEQ3631 | 0.0087 | 1.92 | 0.94 |
| PCBP1-AS1:217 | SEQ3458 | 0.0087 | 1.54 | 0.94 | ZNF528-AS1:16 | SEQ3632 | 0.0194 | 4.E+08 | 0.92 |

2847 lncRNAs were differentially expressed in whole blood (Paxgene RNA tube) of MCI group or mild AD group or moderate-to-severe AD group or both of these AD groups or FTD group or DLB group as compared to healthy control group, and having statistical significance (p value <0.05, Wilcoxon test) and a fold change FC≤0.80 or ≥1.20. These lncRNAs showed a differential expression with a statistical significance for at least one disease group when compared to healthy control group (p value<0.05, Wilcoxon). The 2847 lncRNAs are shown in Table 10.

**Table 10: 2847 whole blood (Paxgene RNA tube) IncRNAs differentially expressed in MCI group or mild AD group or moderate-to-severe AD group or both of these AD groups or FTD group or DLB group as compared to healthy control group.**

| **Group** | **IncRNA** | **SEQ** | **p-value** | **FC** | **Group** | **lncRNA** | **SEQ** | **p-value** | **FC** |
|---|---|---|---|---|---|---|---|---|---|
| DLB | A1BG-AS1:13 | SEQ5267 | 1. E-04 | 1.8 | DLB | lnc-MRPS5-2:2 | SEQ4304 | 1.E-03 | 1.8 |
| MCI | A1BG-AS1:13 | SEQ5267 | 5. E-04 | 1.6 | miAD | lnc-MRPS5-2:2 | SEQ4304 | 7.E-03 | 1.4 |
| DLB | A2M-AS1:3 | SEQ5584 | 2.E-04 | 2.5 | All AD | lnc-MRPS5-2:2 | SEQ4304 | 1.E-02 | 1.4 |
| DLB | AATBC:4 | SEQ4566 | 2.E-03 | 1.4 | MCI | lnc-MRPS5-2:3 | SEQ4165 | 1. E-04 | 2.1 |
| DLB | AATBC:5 | SEQ4567 | 2.E-03 | 1.4 | miAD | lnc-MRPS5-2:3 | SEQ4165 | 1.E-02 | 1.4 |
| DLB | ACBD3-AS1:14 | SEQ5759 | 8.E-05 | 2.2 | All AD | lnc-MRPS5-2:3 | SEQ4165 | 2.E-02 | 1.3 |
| msAD | ACTN1-AS1:6 | SEQ3638 | 3.E-02 | 1.2 | MCI | lnc-MRPS5-4:3 | SEQ5499 | 2.E-04 | 2.2 |
| All AD | ACTN1-AS1:6 | SEQ3638 | 4.E-02 | 1.2 | DLB | lnc-MTERF1-5:1 | SEQ4758 | 1.E-03 | 1.5 |
| All AD | ADAMTSL4-AS1:1 | SEQ3640 | 2.E-02 | 0.8 | All AD | lnc-MTFR1L-1:4 | SEQ5202 | 3.E-02 | 1.3 |
| msAD | ADAMTSL4-AS1:1 | SEQ3640 | 2.E-02 | 0.8 | FTD | lnc-MTHFD1-3:1 | SEQ4184 | 8.E-07 | 0.5 |
| FTD | ADNP-AS1:12 | SEQ0340 | 3.E-04 | 0.6 | All AD | lnc-MTHFD1-3:1 | SEQ4184 | 8.E-03 | 0.7 |
| MCI | AG AP2-AS 1:1 | SEQ3643 | 4.E-05 | 2.0 | miAD | lnc-MTHFD1-3:1 | SEQ4184 | 1.E-02 | 0.6 |
| All AD | AG AP2-AS 1:1 | SEQ3643 | 3.E-02 | 1.3 | FTD | lnc-MTPN-1:1 | SEQ4462 | 2.E-05 | 0.3 |
| All AD | AIRN:2 | SEQ3645 | 2.E-02 | 0.8 | miAD | lnc-MTPN-1:1 | SEQ4462 | 2.E-04 | 0.4 |
| FTD | ANKRD10-IT1:2 | SEQ5990 | 1.E-06 | 0.4 | All AD | lnc-MTPN-1:1 | SEQ4462 | 3.E-04 | 0.5 |
| FTD | ANKRD44-IT1:1 | SEQ4807 | 1.E-03 | 0.7 | msAD | lnc-MTPN-1:1 | SEQ4462 | 2.E-03 | 0.5 |
| DLB | APOBEC3B-AS1:2 | SEQ5079 | 2.E-05 | 0.4 | miAD | lnc-MTRNR2L3-1:2 | SEQ4241 | 1.E-02 | 1.3 |
| MCI | APOBEC3B-A51:2 | SEQ5079 | 1. E-04 | 0.5 | All AD | lnc-MTRNR2L3-1:2 | SEQ4241 | 1.E-02 | 1.2 |
| FTD | APOBEC3B-AS1:2 | SEQ5079 | 6.E-04 | 0.5 | DLB | lnc-MTUS2-9:1 | SEQ4829 | 9.E-04 | 1.3 |
| miAD | APTR:15 | SEQ3651 | 4.E-03 | 0.7 | DLB | lnc-MUC17-4:1 | SEQ5244 | 5.E-04 | 1.4 |
| All AD | APTR:15 | SEQ3651 | 5.E-03 | 0.7 | DLB | lnc-MUC20-16:1 | SEQ4224 | 6.E-05 | 1.5 |
| msAD | APTR:15 | SEQ3651 | 3.E-02 | 0.8 | MCI | lnc-MUC20-16:1 | SEQ4224 | 6.E-05 | 1.5 |
| All AD | APTR:16 | SEQ3636 | 2.E-02 | 0.8 | miAD | lnc-MUC20-16:1 | SEQ4224 | 7.E-04 | 1.3 |
| msAD | APTR:16 | SEQ3636 | 5.E-02 | 0.8 | All AD | lnc-MUC20-16:1 | SEQ4224 | 1.E-03 | 1.3 |
| miAD | APTR:17 | SEQ0260 | S.E-03 | 0.7 | msAD | lnc-MUC20-16:1 | SEQ4224 | 1.E-02 | 1.3 |
| All AD | APTR:17 | SEQ0260 | 9.E-03 | 0.7 | MCI | lnc-MUC20-16:2 | SEQ4127 | 4.E-07 | 2.1 |
| DLB | ARAP1-AS1:1 | SEQ4621 | 1.E-03 | 1.4 | DLB | lnc-MUC20-16:2 | SEQ4127 | 6.E-06 | 2.0 |
| MCI | ASH1L-AS1:1 | SEQ4883 | 9.E-04 | 2.3 | FTD | lnc-MUC20-16:2 | SEQ4127 | 1.E-05 | 1.9 |
| DLB | ATP1A1-AS1:5 | SEQ5498 | 2.E-04 | 2.0 | miAD | lnc-MUC20-16:2 | SEQ4127 | 2.E-03 | 1.4 |
| DLB | ATP2A1-AS1:12 | SEQ5180 | 5. E-04 | 1.4 | All AD | lnc-MUC20-16:2 | SEQ4127 | 2.E-03 | 1.3 |
| DLB | ATP6V0E2-AS1:1 | SEQ4643 | 1.E-03 | 1.6 | msAD | lnc-MUC20-16:2 | SEQ4127 | 1.E-02 | 1.3 |
| MCI | ATP6V0E2-AS1:14 | SEQ3659 | 3.E-06 | 2.3 | DLB | lnc-MUC5B-1:1 | SEQ5724 | 9.E-05 | 1.4 |
| FTD | ATP6V0E2-AS1:14 | SEQ3659 | 6.E-04 | 1.6 | DLB | lnc-MUSTN1-1:1 | SEQ5656 | 1. E-04 | 1.4 |
| miAD | ATP6V0E2-AS1:14 | SEQ3659 | 7.E-04 | 1.6 | FTD | lnc-MVB12B-7:1 | SEQ5716 | 1. E-04 | 0.5 |
| All AD | ATP6V0E2-AS1:14 | SEQ3659 | 2.E-02 | 1.3 | DLB | lnc-MVP-3:6 | SEQ5362 | 7.E-06 | 2.1 |
| DLB | ATP6V0E2-AS1:18 | SEQ3663 | 4.E-04 | 1.9 | MCI | lnc-MVP-3:6 | SEQ5362 | 1.E-05 | 2.3 |
| MCI | ATP6V0E2-AS1:18 | SEQ3663 | 2.E-03 | 1.6 | FTD | lnc-MVP-3:6 | SEQ5362 | 4.E-04 | 1.8 |
| msAD | ATP6V0E2-AS1:18 | SEQ.3663 | 2.E-02 | 1.2 | msAD | lnc-MYBBP1A-2:1 | SEQ4100 | 1.E-02 | 1.4 |
| All AD | ATP6V0E2-AS1:18 | SEQ3663 | 3.E-02 | 1.2 | All AD | lnc-MYBBP1A-2:1 | SEQ4100 | 2.E-02 | 1.4 |
| MCI | ATP6V0E2-AS1:19 | SEQ4794 | 1.E-03 | 2.4 | FTD | lnc-MYF5-2:3 | SEQ2245 | 4.E-08 | 0.4 |
| DLB | ATP6V0E2-AS1:22 | SEQ5142 | 6.E-04 | 1.8 | DLB | lnc-MYF5-2:3 | SEQ2245 | 9.E-05 | 0.5 |
| MCI | ATP6V0E2-AS1:27 | SEQ5188 | 3.E-04 | 1.5 | MCI | lnc-MYF5-2:3 | SEQ2245 | 1. E-04 | 0.5 |
| DLB | ATP6V0E2-AS1:27 | SEQ5188 | 5. E-04 | 1.5 | miAD | lnc-MYF5-2:3 | SEQ2245 | 1.E-03 | 0.6 |
| FTD | BACH1-IT1:1 | SEQ5153 | 5. E-04 | 0.6 | All AD | lnc-MYF5-2:3 | SEQ2245 | 6.E-03 | 0.6 |
| DLB | BISPR:20 | SEQ5519 | 2.E-04 | 1.4 | FTD | lnc-MYF5-2:4 | SEQ2296 | 4.E-09 | 0.4 |
| miAD | BOLA3-AS1:14 | SEQ3673 | 9.E-05 | 2.3 | MCI | lnc-MYF5-2:4 | SEQ2296 | 3.E-05 | 0.4 |
| All AD | BOLA3-AS1:14 | SEQ3673 | 2.E-04 | 2.0 | DLB | lnc-MYF5-2:4 | SEQ2296 | 4.E-05 | 0.5 |
| FTD | BOLA3-AS1:14 | SEQ3673 | 3.E-04 | 2.8 | miAD | lnc-MYF5-2:4 | SEQ2296 | 2.E-04 | 0.5 |
| msAD | BOLA3-AS1:14 | SEQ3673 | 4.E-03 | 1.7 | All AD | lnc-MYF5-2:4 | SEQ2296 | 2.E-03 | 0.6 |
| FTD | BOLA3-AS1:25 | SEQ3677 | 1.E-05 | 0.6 | msAD | lnc-MYF5-2:4 | SEQ2296 | 4.E-02 | 0.6 |
| All AD | BOLA3-AS1:25 | SEQ3677 | 3.E-02 | 0.8 | FTD | lnc-MYF5-2:5 | SEQ4262 | 4.E-05 | 0.6 |
| DLB | C6orf47-AS1:1 | SEQ4838 | 9.E-04 | 1.4 | miAD | lnc-MYF5-2:5 | SEQ4262 | 9.E-03 | 0.7 |
| DLB | CACTIN-AS1:2 | SEQ3680 | 4.E-05 | 1.7 | All AD | lnc-MYF5-2:5 | SEQ4262 | 2.E-02 | 0.7 |
| msAD | CACTIN-AS1:2 | SEQ3680 | 1.E-03 | 1.3 | All AD | lnc-MYH9-1:1 | SEQ4021 | 1.E-02 | 0.7 |
| All AD | CACTIN-AS1:2 | SEQ3680 | 1.E-03 | 1.3 | msAD | lnc-MYH9-1:1 | SEQ4021 | 2.E-02 | 0.7 |
| MCI | CACTIN-AS1:2 | SEQ3680 | 1.E-03 | 1.3 | DLB | lnc-MYLPF-3:2 | SEQ5847 | 4.E-05 | 1.5 |
| miAD | CACTIN-AS1:2 | SEQ3680 | 6.E-03 | 1.3 | MCI | lnc-MYO16-7:1 | SEQ4673 | 1.E-03 | 0.8 |
| DLB | CACTIN-AS1:5 | SEQ2914 | 3.E-04 | 1.7 | DLB | lnc-MYO1D-1:2 | SEQ5020 | 7.E-04 | 1.4 |
| MCI | CACTIN-AS1:5 | SEQ2914 | 7.E-04 | 1.7 | FTD | lnc-MY06-1:1 | SEQ4205 | 8.E-06 | 0.5 |
| All AD | CACTIN-AS1:6 | SEQ3686 | 5.E-02 | 1.2 | miAD | lnc-MY06-1:1 | SEQ4205 | 1.E-02 | 0.7 |
| DLB | CAPN10-AS1:2 | SEQ5209 | 5.E-04 | 1.8 | All AD | lnc-MYO6-1:1 | SEQ4205 | 1.E-02 | 0.7 |
| DLB | CARD8-AS1:5 | SEQ4426 | 2.E-03 | 1.5 | FTD | lnc-MYOM1-6:2 | SEQ5872 | 3.E-05 | 0.6 |
| DLB | CASC2:1 | SEQ2645 | 5.E-05 | 1.5 | DLB | lnc-N4BP2-3:1 | SEQ3688 | 5.E-05 | 2.4 |
| All AD | CATIP-AS1:6 | SEQ3690 | 5.E-02 | 0.8 | All AD | lnc-N4BP2-3:1 | SEQ3688 | 3.E-02 | 1.3 |
| DLB | CCDC144NL-AS1:22 | SEQ4979 | 5.E-05 | 1.9 | msAD | lnc-N4BP2-3:1 | SEQ3688 | 5.E-02 | 1.3 |
| MCI | CCDC144NL-AS1:22 | SEQ4979 | 8.E-04 | 1.8 | DLB | lnc-N4BP2L1-4:1 | SEQ5458 | 3.E-04 | 2.0 |
| DLB | CCDC144NL-AS1:50 | SEQ3693 | 2.E-05 | 1.9 | All AD | lnc-NAA35-13:2 | SEQ5233 | 4.E-02 | 0.8 |
| MCI | CCDC144NL-AS1:50 | SEQ3693 | 2.E-05 | 2.1 | FTD | lnc-NAA38-5:1 | SEQ4193 | 5. E-04 | 0.7 |
| msAD | CCDC144NL-AS1:50 | SEQ3693 | 2.E-02 | 1.2 | miAD | lnc-NAA38-5:1 | SEQ4193 | 1.E-02 | 0.8 |
| All AD | CCDC144NL-AS1:50 | SEQ3693 | 4.E-02 | 1.2 | All AD | lnc-NAA38-5:1 | SEQ4193 | 4.E-02 | 0.8 |
| DLB | CCDC144NL-AS1:51 | SEQ3697 | 2.E-05 | 1.9 | FTD | lnc-NABP1-1:1 | SEQ5416 | 3.E-04 | 0.7 |
| MCI | CCDC144NL-AS1:51 | SEQ3697 | 4.E-05 | 2.0 | FTD | lnc-NABP1-1:2 | SEQ4356 | 1.E-05 | 0.6 |
| FTD | CCDC144NL-AS1:51 | SEQ3697 | 6.E-04 | 1.4 | miAD | lnc-NABP1-1:2 | SEQ4356 | 4.E-03 | 0.7 |
| All AD | CCDC144NL-AS1:51 | SEQ3697 | 1.E-02 | 1.2 | All AD | lnc-NABP1-1:2 | SEQ4356 | 1.E-02 | 0.7 |
| msAD | CCDC144NL-AS1:51 | SEQ3697 | 3.E-02 | 1.2 | MCI | lnc-NABP1-5:1 | SEQ4160 | 1.E-03 | 1.6 |
| DLB | CCDC183-AS1:4 | SEQ3701 | 2.E-05 | 1.5 | All AD | lnc-NABP1-5:1 | SEQ4160 | 7.E-03 | 1.3 |
| MCI | CCDC183-AS1:4 | SEQ3701 | 6.E-04 | 1.3 | msAD | lnc-NABP1-5:1 | SEQ4160 | 1.E-02 | 1.3 |
| All AD | CCDC183-AS1:4 | SEQ3701 | 2.E-02 | 1.1 | FTD | lnc-NAMPT-1:2 | SEQ3455 | 8.E-05 | 0.6 |
| msAD | CCDC183-AS1:4 | SEQ3701 | 2.E-02 | 1.1 | DLB | lnc-NAT1-7:2 | SEQ4687 | 1.E-03 | 1.4 |
| MCI | CCDC183-AS1:5 | SEQ2925 | 7.E-04 | 1.6 | FTD | lnc-NAT9-1:1 | SEQ4159 | 3.E-07 | 1.7 |
| All AD | CCDC18-AS1:45 | SEQ3706 | 5.E-02 | 1.3 | MCI | lnc-NAT9-1:1 | SEQ4159 | 1.E-06 | 2.1 |
| miAD | CD27-AS1:1 | SEQ3707 | 1.E-02 | 1.4 | DLB | lnc-NAT9-1:1 | SEQ4159 | 3.E-06 | 2.0 |
| All AD | CD27-AS1:1 | SEQ3707 | 2.E-02 | 1.3 | miAD | lnc-NAT9-1:1 | SEQ4159 | 1.E-02 | 1.2 |
| FTD | CEBPB-AS1:2 | SEQ4719 | 2.E-04 | 1.8 | All AD | lnc-NAT9-1:1 | SEQ4159 | 2.E-02 | 1.2 |
| MCI | CEBPB-AS1:2 | SEQ4719 | 5. E-04 | 1.9 | DLB | lnc-NAT9-1:4 | SEQ5013 | 7.E-04 | 1.4 |
| DLB | CEBPB-AS1:2 | SEQ4719 | 1.E-03 | 1.7 | MCI | lnc-NAV1-9:1 | SEQ5343 | 5.E-05 | 2.1 |
| miAD | CFAP58-AS1:4 | SEQ0109 | 1.E-02 | 0.7 | DLB | lnc-NAV1-9:1 | SEQ5343 | 4.E-04 | 2.2 |
| All AD | CFAP58-AS1:4 | SEQ0109 | 3.E-02 | 0.7 | DLB | lnc-NAXD-4:1 | SEQ2736 | 2.E-03 | 1.3 |
| FTD | CFLAR-AS1:22 | SEQ3712 | 2.E-05 | 0.6 | FTD | lnc-NBN-2:1 | SEQ3838 | 1.E-05 | 0.6 |
| All AD | CFLAR-AS1:22 | SEQ3712 | 2.E-02 | 0.7 | All AD | lnc-NBN-2:1 | SEQ3838 | 1.E-02 | 0.7 |
| FTD | CHMP1B-AS1:2 | SEQ2249 | 1.E-07 | 0.4 | miAD | lnc-NBN-2:1 | SEQ3838 | 1.E-02 | 0.7 |
| MCI | CHMP1B-AS1:2 | SEQ2249 | 3.E-04 | 0.4 | msAD | lnc-NBN-2:1 | SEQ3838 | 3.E-02 | 0.8 |
| DLB | CHMP1B-AS1:2 | SEQ2249 | 2.E-03 | 0.5 | DLB | lnc-NBPF1-1:12 | SEQ4728 | 2.E-04 | 3.1 |
| All AD | CHMP1B-AS1:2 | SEQ2249 | 2.E-02 | 0.6 | MCI | lnc-NBPF1-1:12 | SEQ4728 | 1.E-03 | 2.5 |
| miAD | CKMT2-AS1:32 | SEQ3713 | 3.E-03 | 1.7 | DLB | lnc-NBPF1-1:6 | SEQ3026 | 8.E-05 | 2.4 |
| All AD | CKMT2-AS1:32 | SEQ3713 | 9.E-03 | 1.5 | FTD | lnc-NBPF1-1:6 | SEQ3026 | 5. E-04 | 2.0 |
| MCI | CPEB2-AS1:4 | SEQ4788 | 1.E-03 | 1.8 | MCI | lnc-NBPF1-1:6 | SEQ3026 | 1.E-03 | 2.0 |
| DLB | CTBP1-AS:2 | SEQ4927 | 8.E-04 | 1.4 | FTD | lnc-NBPF15-1:2 | SEQ4591 | 1.E-03 | 1.6 |
| DLB | CTBP1-AS:3 | SEQ5245 | 2.E-05 | 1.6 | MCI | lnc-NBPF15-1:2 | SEQ4591 | 2.E-03 | 1.6 |
| MCI | CTBP1-AS:3 | SEQ5245 | 5. E-04 | 1.4 | DLB | lnc-NBPF15-1:2 | SEQ4591 | 2.E-03 | 1.8 |
| FTD | CYTOR:49 | SEQ3721 | 4.E-04 | 0.5 | MCI | lnc-NBPF3-3:6 | SEQ3144 | 1.E-03 | 1.7 |
| miAD | CYTOR:49 | SEQ3721 | 7.E-03 | 0.6 | MCI | lnc-NBPF3-3:7 | SEQ4651 | 2.E-04 | 1.8 |
| All AD | CYTOR:49 | SEQ3721 | 7.E-03 | 0.7 | DLB | lnc-NBPF3-3:7 | SEQ4651 | 1.E-03 | 1.8 |
| msAD | CYTOR:49 | SEQ3721 | 3.E-02 | 0.7 | miAD | lnc-NBPF3-8:1 | SEQ3929 | 4.E-03 | 0.6 |
| DLB | CYTOR:50 | SEQ5178 | 5. E-04 | 1.4 | All AD | lnc-NBPF3-8:1 | SEQ3929 | 5.E-03 | 0.7 |
| FTD | DARS-AS1:43 | SEQ372S | 7.E-04 | 0.7 | msAD | lnc-NBPF3-8:1 | SEQ3929 | 3.E-02 | 0.7 |
| All AD | DARS-AS1:43 | SEQ3725 | 1.E-02 | 0.7 | FTD | lnc-NCKAP1-5:2 | SEQ4381 | 4.E-04 | 0.6 |
| msAD | DARS-AS1:43 | SEQ372S | 3.E-02 | 0.7 | miAD | lnc-NCKAP1-5:2 | SEQ4381 | 3.E-03 | 0.7 |
| MCI | DDIT4-AS1:2 | SEQ5738 | 9.E-05 | 1.7 | All AD | lnc-NCKAP1-5:2 | SEQ4381 | 9.E-03 | 0.7 |
| DLB | DGUOK-AS1:7 | SEQ5186 | 5. E-04 | 1.5 | FTD | lnc-NCOA4-6:1 | SEQ5061 | 7.E-04 | 0.6 |
| FTD | DIAPH2-AS1:8 | SEQ4994 | 8.E-04 | 0.6 | FTD | lnc-NDFIP2-13:1 | SEQ4472 | 1. E-04 | 0.5 |
| FTD | DLEU1:2 | SEQ3732 | 3.E-07 | 0.5 | MCI | lnc-NDFIP2-13:1 | SEQ4472 | 2.E-03 | 0.6 |
| miAD | DLEU1:2 | SEQ3732 | 3.E-04 | 0.6 | All AD | lnc-NDFIP2-13:1 | SEQ4472 | 3.E-02 | 0.7 |
| MCI | DLEU1:2 | SEQ3732 | 2.E-03 | 0.6 | DLB | lnc-NDFIP2-26:2 | SEQ3696 | 4.E-04 | 1.4 |
| All AD | DLEU1:2 | SEQ3732 | 2.E-03 | 0.6 | All AD | lnc-NDFIP2-26:2 | SEQ3696 | 4.E-02 | 1.1 |
| msAD | DLEU1:2 | SEQ3732 | 3.E-02 | 0.7 | msAD | lnc-NDFIP2-26:2 | SEQ3696 | 5.E-02 | 1.1 |
| FTD | DLEU1:3 | SEQ3735 | 8.E-05 | 0.6 | DLB | lnc-NDRG1-2:1 | SEQ4828 | 2.E-04 | 1.4 |
| All AD | DLEU1:3 | SEQ3735 | 4.E-02 | 0.7 | MCI | lnc-NDRG1-2:1 | SEQ4828 | 9.E-04 | 1.3 |
| FTD | DLEU1:54 | SEQ3736 | 2.E-05 | 0.6 | DLB | lnc-NDRG1-5:1 | SEQ4864 | 2.E-05 | 1.8 |
| All AD | DLEU1:54 | SEQ3736 | 5.E-02 | 0.7 | MCI | lnc-NDRG1-5:1 | SEQ4864 | 9.E-04 | 1.6 |
| All AD | DLEU2:26 | SEQ0862 | 2.E-02 | 0.7 | DLB | lnc-NDRG1-7:2 | SEQ5432 | 3.E-04 | 1.4 |
| All AD | DLEU2:32 | SEQ2776 | 7.E-03 | 0.7 | MCI | lnc-NDRG1-7:2 | SEQ5432 | 3.E-04 | 1.4 |
| miAD | DLEU2:32 | SEQ2776 | 1.E-02 | 0.7 | DLB | lnc-NDRG1-7:4 | SEQ4677 | 2.E-04 | 1.3 |
| msAD | DLEU2:32 | SEQ2776 | 2.E-02 | 0.8 | MCI | lnc-NDRG1-7:4 | SEQ4677 | 1.E-03 | 1.3 |
| FTD | DLEU2:45 | SEQ0863 | 9.E-05 | 0.7 | All AD | lnc-NDUFA4-2:1 | SEQ5260 | 5.E-02 | 1.2 |
| All AD | DLEU2:45 | SEQ0863 | 2.E-03 | 0.7 | msAD | lnc-NDUFA6-2:2 | SEQ3899 | 3.E-02 | 1.2 |
| miAD | DLEU2:45 | SEQ0863 | 2.E-03 | 0.7 | DLB | lnc-NDUFAF8-1:3 | SEQ5031 | 7.E-04 | 1.5 |
| msAD | DLEU2:45 | SEQ0863 | 1.E-02 | 0.8 | DLB | lnc-NDUFAF8-1:4 | SEQ4629 | 1.E-03 | 1.4 |
| MCI | DLEU2:5 | SEQ5943 | 9.E-06 | 0.1 | FTD | lnc-NDUFB3-3:3 | SEQ3750 | 2.E-04 | 0.5 |
| FTD | DLGS-AS1:10 | SEQ5411 | 3.E-04 | 0.6 | miAD | lnc-NDUFB3-3:3 | SEQ3750 | 6.E-04 | 0.6 |
| DLB | DLGAP4-AS1:11 | SEQ4702 | 1.E-03 | 1.5 | All AD | lnc-NDUFB3-3:3 | SEQ3750 | 3.E-03 | 0.6 |
| MCI | DNAJC9-AS1:9 | SEQ4869 | 9.E-04 | 1.6 | msAD | lnc-NDUFB3-3:3 | SEQ3750 | 4.E-02 | 0.7 |
| DLB | DPP9-AS1:1 | SEQ4563 | 2.E-03 | 1.4 | DLB | lnc-NECAB3-1:1 | SEQ5198 | 3.E-04 | 1.8 |
| FTD | DPYD-AS1:3 | SEQ3747 | 5.E-05 | 0.7 | MCI | lnc-NECAB3-1:1 | SEQ5198 | 5. E-04 | 1.7 |
| All AD | DPYD-AS1:3 | SEQ3747 | 3.E-02 | 0.7 | DLB | lnc-NEIL1-1:2 | SEQ5266 | 7.E-06 | 1.7 |
| msAD | DPYD-AS1:3 | SEQ3747 | 4.E-02 | 0.8 | FTD | lnc-NEIL1-1:2 | SEQ5266 | 1. E-04 | 1.4 |
| FTD | DPYD-As2:1 | SEQ4360 | 8.E-05 | 0.6 | All AD | lnc-NEIL1-1:2 | SEQ5266 | 4.E-02 | 1.2 |
| miAD | DPYD-As2:1 | SEQ4360 | 4.E-03 | 0.7 | DLB | lnc-NEK5-1:3 | SEQ5482 | 2.E-04 | 1.5 |
| FTD | EAF1-AS1:15 | SEQ5886 | 3.E-05 | 0.7 | FTD | lnc-NEK7-4:1 | SEQ4217 | 4.E-06 | 0.5 |
| FTD | EIF1B-AS1:18 | SEQ5465 | 3.E-04 | 0.6 | miAD | lnc-NEK7-4:1 | SEQ4217 | 1.E-02 | 0.7 |
| FTD | EIF3J-AS1:21 | SEQ0261 | 9.E-04 | 0.6 | All AD | lnc-NEK7-4:1 | SEQ4217 | 2.E-02 | 0.7 |
| DLB | E LOA-AS 1:4 | SEQ5251 | 5. E-04 | 1.5 | FTD | lnc-NEMF-1:4 | SEQ3674 | 3.E-11 | 4.2 |
| FTD | ENTPD1-AS1:12 | SEQ3757 | 9.E-04 | 0.6 | MCI | lnc-NEMF-1:4 | SEQ3674 | 3.E-05 | 3.0 |
| miAD | ENTPD1-AS1:12 | SEQ3757 | 3.E-03 | 0.6 | msAD | lnc-NEMF-1:4 | SEQ3674 | 5.E-02 | 1.3 |
| All AD | ENTPD1-AS1:12 | SEQ3757 | 8.E-03 | 0.7 | FTD | lnc-NEMF-2:2 | SEQ2656 | 6.E-08 | 5.6 |
| All AD | ENTPD1-AS1:23 | SEQ3758 | 3.E-02 | 0.8 | MCI | lnc-NEMF-2:2 | SEQ2656 | 6.E-05 | 4.1 |
| FTD | ENTPD1-AS1:34 | SEQ5552 | 2.E-04 | 0.7 | DLB | lnc-NEMF-2:2 | SEQ2656 | 2.E-03 | 3.0 |
| All AD | ENTPD1-AS1:5 | SEQ3760 | 5.E-02 | 0.8 | All AD | lnc-NEMF-2:2 | SEQ2656 | 2.E-02 | 2.3 |
| FTD | FAM 13A-AS1:1 | SEQ3762 | 1. E-04 | 0.6 | DLB | lnc-NEMF-4:1 | SEQ4738 | 8.E-05 | 1.4 |
| All AD | FAM13A-AS1:1 | SEQ3762 | 8.E-03 | 0.7 | MCI | lnc-NEMF-4:1 | SEQ4738 | 1.E-03 | 1.3 |
| miAD | FAM 13A-AS1:1 | SEQ3762 | 1.E-02 | 0.7 | DLB | lnc-NEMP2-4:1 | SEQ5668 | 1. E-04 | 1.7 |
| msAD | FAM13A-AS1:1 | SEQ3762 | 2.E-02 | 0.8 | FTD | lnc-NETO2-3:1 | SEQ5273 | 8.E-05 | 0.6 |
| DLB | FAM 13A-AS1:5 | SEQ5865 | 4.E-05 | 3.2 | All AD | lnc-NETO2-3:1 | SEQ5273 | 4.E-02 | 0.8 |
| MCI | FAM 157C:3 | SEQ5767 | 4.E-07 | 0.1 | DLB | lnc-NEURL4-1:3 | SEQ4617 | 1.E-03 | 1.4 |
| FTD | FAM 157C:3 | SEQ5767 | 7.E-05 | 0.1 | DLB | lnc-NEURL4-1:4 | SEQ3028 | 1. E-04 | 1.6 |
| DLB | FAM 182A:4 | SEQ4636 | 1.E-03 | 1.5 | MCI | lnc-NEURL4-1:4 | SEQ3028 | 2.E-04 | 1.6 |
| miAD | FAM198B-AS1:7 | SEQ3768 | 1.E-02 | 0.7 | MCI | lnc-NFAM1-4:1 | SEQ5147 | 1. E-04 | 2.0 |
| All AD | FAM198B-AS1:7 | SEQ3768 | 2.E-02 | 0.8 | DLB | lnc-NFAM1-4:1 | SEQ5147 | 6.E-04 | 2.0 |
| DLB | FAM215B:1 | SEQ4971 | 8.E-04 | 1.6 | All AD | lnc-NFAM1-4:1 | SEQ5147 | 3.E-02 | 1.2 |
| MCI | FAM239A:9 | SEQ3770 | 2.E-04 | 2.1 | DLB | lnc-NGFR-1:1 | SEQ3671 | 1.E-03 | 1.3 |
| DLB | FAM239A:9 | SEQ3770 | 2.E-03 | 1.8 | All AD | lnc-NGFR-1:1 | SEQ3671 | 2.E-02 | 1.2 |
| All AD | FAM239A:9 | SEQ3770 | 3.E-02 | 1.3 | msAD | lnc-NGFR-1:1 | SEQ3671 | 5.E-02 | 1.1 |
| msAD | FAM30A:2 | SEQ3772 | 1.E-02 | 1.6 | All AD | lnc-NHS-2:1 | SEQ2339 | 2.E-02 | 0.8 |
| All AD | FAM30A:2 | SEQ3772 | 2.E-02 | 1.5 | FTD | lnc-NID1-4:4 | SEQ4463 | 3.E-10 | 0.3 |
| DLB | FAM 53 B-AS1:1 | SEQ5494 | 2.E-04 | 1.8 | MCI | lnc-NID1-4:4 | SEQ4463 | 4.E-07 | 0.4 |
| miAD | FAM99A:4 | SEQ3775 | 7.E-03 | 0.6 | miAD | lnc-NID1-4:4 | SEQ4463 | 4.E-05 | 0.5 |
| All AD | FAM99A:4 | SEQ3775 | 2.E-02 | 0.7 | All AD | lnc-NID1-4:4 | SEQ4463 | 1. E-04 | 0.5 |
| miAD | FAM99B:7 | SEQ3777 | 1.E-02 | 0.7 | DLB | lnc-NID1-4:4 | SEQ4463 | 2.E-03 | 0.4 |
| All AD | FAM99B:7 | SEQ3777 | 3.E-02 | 0.7 | msAD | lnc-NID1-4:4 | SEQ4463 | 2.E-03 | 0.6 |
| FTD | FGD5-AS1:11 | SEQ3778 | 3.E-07 | 3.1 | FTD | lnc-NIF3L1-3:1 | SEQ5283 | 4.E-04 | 0.6 |
| MCI | FGD5-AS1:11 | SEQ3778 | 9.E-06 | 3.3 | All AD | lnc-NIF3L1-3:1 | SEQ5283 | 3.E-02 | 0.8 |
| DLB | FGD5-AS1:11 | SEQ3778 | 1. E-04 | 2.5 | miAD | lnc-NIN-1:1 | SEQ4149 | 1.E-02 | 0.6 |
| msAD | FGD5-AS1:11 | SEQ3778 | 1.E-02 | 1.4 | All AD | lnc-NIN-1:1 | SEQ4149 | 1.E-02 | 0.7 |
| All AD | FGD5-AS1:11 | SEQ3778 | 2.E-02 | 1.4 | FTD | lnc-NINJ2-3:1 | SEQ6000 | 3.E-07 | 0.4 |
| DLB | FGD5-AS1:28 | SEQ3782 | 5. E-04 | 2.4 | FTD | lnc-NIPBL-3:1 | SEQ5604 | 2.E-04 | 0.6 |
| All AD | FGDS-AS1:28 | SEQ3782 | 8.E-03 | 1.6 | msAD | lnc-NKAP-4:2 | SEQ4178 | 1.E-02 | 2.0 |
| msAD | FGDS-AS1:28 | SEQ3782 | 2.E-02 | 1.7 | All AD | lnc-NKX2-4-6:3 | SEQ4164 | 3.E-03 | 1.3 |
| FTD | FGDS-AS1:30 | SEQ3785 | 1.E-07 | 3.1 | msAD | lnc-NKX2-4-6:3 | SEQ4164 | 4.E-03 | 1.3 |
| MCI | FGDS-AS1:30 | SEQ3785 | 2.E-05 | 2.9 | miAD | lnc-NKX2-4-6:3 | SEQ4164 | 1.E-02 | 1.3 |
| DLB | FGDS-AS1:30 | sEQ378S | 3.E-04 | 2.3 | DLB | lnc-NKX6-3-1:1 | SEQ5030 | 9.E-05 | 1.7 |
| All AD | FGDS-AS1:30 | sEQ378S | 8.E-03 | 1.4 | MCI | lnc-NKX6-3-1:1 | SEQ5030 | 7.E-04 | 1.5 |
| msAD | FGDS-AS1:30 | sEQ378S | 8.E-03 | 1.4 | All AD | lnc-NKX6-3-1:1 | SEQ5030 | 3.E-02 | 1.1 |
| MCI | FGD5-AS1:6 | SEQ4466 | 3.E-06 | 0.2 | FTD | lnc-NLN-2:1 | SEQ5286 | 4.E-06 | 0.5 |
| FTD | FGD5-AS1:6 | SEQ4466 | 9.E-04 | 0.2 | All AD | lnc-NLN-2:1 | SEQ5286 | 2.E-02 | 0.7 |
| DLB | FGD5-AS1:6 | SEQ4466 | 2.E-03 | 0.3 | FTD | lnc-NLRC4-3:3 | SEQ3305 | 3.E-04 | 0.7 |
| miAD | FGD5-AS1:7 | SEQ3784 | 2.E-03 | 0.4 | All AD | lnc-NLRC4-3:3 | SEQ3305 | 9.E-03 | 0.8 |
| All AD | FGD5-AS1:7 | SEQ3784 | 4.E-03 | 0.4 | miAD | lnc-NLRC4-3:3 | SEQ3305 | 1.E-02 | 0.7 |
| msAD | FGDS-AS1:7 | SEQ3784 | 4.E-02 | 0.5 | msAD | lnc-NLRC4-3:3 | sEQ330S | 3.E-02 | 0.8 |
| DLB | FGD5-AS1:9 | SEQ4423 | 2.E-03 | 1.5 | MCI | lnc-NLRC4-4:1 | SEQ5821 | 2.E-05 | 1.9 |
| DLB | FLNB-AS1:1 | SEQ3796 | 2.E-04 | 1.9 | DLB | lnc-NLRC4-4:1 | SEQS821 | 5.E-05 | 1.9 |
| MCI | FLNB-AS1:1 | SEQ3796 | 1.E-03 | 1.7 | DLB | lnc-NLRP12-1:12 | SEQ5265 | 5. E-04 | 1.6 |
| All AD | FLNB-AS1:1 | SEQ3796 | 3.E-03 | 1.5 | FTD | lnc-NMD3-1:1 | SEQ2364 | 4.E-04 | 0.6 |
| msAD | FLNB-AS1:1 | SEQ3796 | 6.E-03 | 1.5 | DLB | lnc-NME3-1:2 | SEQ5256 | 5. E-04 | 1.5 |
| miAD | FLNB-AS1:1 | SEQ3796 | 8.E-03 | 1.5 | DLB | lnc-NMT1-1:2 | SEQ3980 | 2.E-04 | 1.6 |
| DLB | FLNB-AS1:3 | SEQ3800 | 2.E-05 | 2.4 | MCI | lnc-NMT1-1:2 | SEQ3980 | 1.E-03 | 1.4 |
| MCI | FLNB-AS1:3 | SEQ3800 | 1. E-04 | 2.0 | All AD | lnc-NMT1-1:2 | SEQ3980 | 2.E-02 | 1.2 |
| All AD | FLNB-AS1:3 | SEQ3800 | 4.E-04 | 1.5 | msAD | lnc-NMT1-1:2 | SEQ3980 | 2.E-02 | 1.2 |
| msAD | FLNB-AS1:3 | SEQ3800 | 1.E-03 | 1.5 | FTD | lnc-NNT-3:S | sEQ490S | 9.E-04 | 0.7 |
| miAD | FLNB-AS1:3 | SEQ3800 | 1.E-03 | 1.6 | DLB | lnc-NOB1-1:1 | SEQ2738 | 1. E-04 | 1.5 |
| FTD | FOXP1-IT1:1 | SEQ3805 | 1.E-05 | 0.6 | All AD | lnc-NOB1-1:1 | SEQ2738 | 3.E-02 | 1.1 |
| miAD | FOXP1-IT1:1 | SEQ3805 | 1.E-02 | 0.7 | FTD | lnc-NOD1-1:1 | SEQ5295 | 2.E-05 | 0.6 |
| All AD | FOXP1-IT1:1 | sEQ380S | 3.E-02 | 0.7 | All AD | lnc-NOD1-1:1 | SEQ5295 | 4.E-02 | 0.7 |
| FTD | FRY-AS1:6 | SEQ5829 | 5.E-05 | 0.6 | DLB | lnc-NOL6-7:1 | SEQSS16 | 2.E-04 | 1.4 |
| FTD | FTX: 19 | SEQ3811 | 3.E-09 | 0.1 | DLB | lnc-NOP14-3:4 | SEQ2834 | 4.E-04 | 1.6 |
| MCI | FTX: 19 | SEQ3811 | 2.E-07 | 0.1 | MCI | lnc-NOP16-2:6 | SEQ4973 | 5. E-04 | 1.7 |
| DLB | FTX: 19 | SEQ3811 | 7.E-06 | 0.1 | DLB | lnc-NOP16-2:6 | SEQ4973 | 8.E-04 | 1.7 |
| All AD | FTX: 19 | SEQ3811 | 4.E-03 | 0.1 | DLB | lnc-NOP9-1:2 | SEQ2990 | 3.E-04 | 1.4 |
| miAD | FTX:19 | SEQ3811 | 5.E-03 | 0.1 | DLB | lnc-NOS2-2:1 | SEQ4307 | 1.E-03 | 1.7 |
| msAD | FTX: 19 | SEQ3811 | 1.E-02 | 0.2 | msAD | lnc-NOS2-2:1 | SEQ4307 | 7.E-03 | 1.4 |
| FTD | FTX:24 | SEQ5089 | 6.E-04 | 0.7 | All AD | lnc-NOS2-2:1 | SEQ4307 | 3.E-02 | 1.3 |
| FTD | FTX:28 | SEQ4395 | 1. E-04 | 0.5 | DLB | lnc-NPB-1:5 | SEQ4837 | 8.E-04 | 1.4 |
| MCI | FTX:28 | sEQ439S | 2.E-03 | 0.5 | MCI | lnc-NPB-1:5 | SEQ4837 | 9.E-04 | 1.4 |
| All AD | FTX:37 | SEQ3814 | 3.E-02 | 0.7 | FTD | lnc-NPEPL1-3:1 | SEQ5470 | 3.E-04 | 0.7 |
| DLB | FTX:4 | SEQ4793 | 1.E-03 | 2.1 | MCI | lnc-NPIPA7-4:1 | SEQ4441 | 2.E-03 | 1.6 |
| DLB | FTX:44 | SEQ4642 | 1.E-03 | 1.6 | FTD | lnc-NPIPB12-2:2 | SEQ5357 | 4.E-04 | 0.6 |
| FTD | FTX:50 | SEQ3819 | 3.E-08 | 0.3 | FTD | lnc-NPIPB12-4:1 | SEQ5858 | 4.E-05 | 0.7 |
| MCI | FTX:50 | SEQ3819 | 8.E-05 | 0.4 | DLB | lnc-NPIPB3-1:1 | SEQ4519 | 6.E-05 | 1.7 |
| All AD | FTX:50 | SEQ3819 | 3.E-02 | 0.6 | MCI | lnc-NPIPB3-1:1 | SEQ4519 | 2.E-03 | 1.5 |
| DLB | FTX:52 | SEQ5282 | 3.E-05 | 2.2 | DLB | lnc-NPIPB3-2:1 | SEQ4765 | 1.E-03 | 1.5 |
| MCI | FTX:52 | SEQ5282 | 5. E-04 | 1.9 | All AD | lnc-NPL-2:1 | SEQ5306 | 2.E-02 | 0.7 |
| FTD | FTX:54 | SEQ4798 | 1.E-03 | 0.4 | FTD | lnc-NPL-2:2 | SEQ3740 | 3.E-04 | 0.6 |
| DLB | GABPB1-AS1:24 | SEQ3823 | 7.E-06 | 3.8 | All AD | lnc-NPL-2:2 | SEQ3740 | 1.E-02 | 0.7 |
| FTD | GABPB1-AS1:24 | SEQ3823 | 1.E-03 | 1.9 | msAD | lnc-NPL-2:2 | SEQ3740 | 4.E-02 | 0.8 |
| MCI | GABPB1-AS1:24 | SEQ3823 | 1.E-03 | 2.4 | FTD | lnc-NPM2-3:1 | sEQS780 | 7.E-05 | 0.6 |
| miAD | GABPB1-AS1:24 | SEQ3823 | 3.E-03 | 1.6 | DLB | lnc-NPTX1-2:12 | SEQ4521 | 2.E-03 | 1.6 |
| All AD | GABPB1-AS1:24 | SEQ3823 | 8.E-03 | 1.4 | DLB | lnc-NPTX1-2:2 | SEQ5131 | 6.E-04 | 1.6 |
| FTD | GABPB1-IT1:2 | SEQ3828 | 2.E-04 | 2.4 | msAD | lnc-NPTX1-2:5 | SEQ4251 | 9.E-03 | 0.6 |
| All AD | GABPB1-IT1:2 | SEQ3828 | 4.E-02 | 1.3 | All AD | lnc-NPTX 1-2:5 | SEQ4251 | 2.E-02 | 0.6 |
| miAD | GABPB1-IT1:5 | sEQ2S91 | 7.E-03 | 1.6 | msAD | lnc-NPTX1-2:6 | SEQ3742 | 4.E-02 | 0.8 |
| All AD | GABPB1-IT1:5 | SEQ2591 | 1.E-02 | 1.4 | DLB | lnc-NPTX1-2:9 | sEQS130 | 6.E-04 | 1.6 |
| msAD | GABPB1-IT1:5 | sEQ2S91 | 5.E-02 | 1.3 | msAD | lnc-NR1D1-1:6 | SEQ4228 | 1.E-02 | 0.7 |
| DLB | GAS6-AS1:3 | sEQS908 | 2.E-05 | 2.3 | All AD | lnc-NR1D1-1:6 | SEQ4228 | 4.E-02 | 0.8 |
| msAD | GFOD1-AS1:3 | SEQ3957 | 3.E-02 | 1.5 | FTD | lnc-NR1I2-3:1 | SEQ5229 | 5.E-04 | 0.6 |
| DLB | GHRLOS:9 | SEQ5051 | 7.E-04 | 2.0 | DLB | lnc-NR2C2-1:1 | SEQ2751 | 8.E-04 | 1.3 |
| miAD | GK-AS1:1 | SEQ3749 | 7.E-03 | 0.6 | DLB | lnc-NR2F6-5:1 | sEQ34S7 | 6.E-04 | 1.4 |
| All AD | GK-AS1:1 | SEQ3749 | 9.E-03 | 0.7 | MCI | lnc-NR2F6-5:1 | sEQ34S7 | 2.E-03 | 1.4 |
| msAD | GK-AS1:1 | SEQ3749 | 4.E-02 | 0.7 | FTD | lnc-NRBF2-2:1 | SEQ4087 | 1.E-08 | 0.4 |
| DLB | GLYCTK-AS1:7 | SEQ5124 | 6.E-04 | 1.6 | MCI | lnc-NRBF2-2:1 | SEQ4087 | 5.E-04 | 0.6 |
| DLB | G LYCTK-AS1:8 | SEQ5015 | 7.E-04 | 1.4 | miAD | lnc-NRBF2-2:1 | SEQ4087 | 2.E-03 | 0.5 |
| miAD | GSEC:2 | SEQ3835 | 4.E-03 | 0.6 | All AD | lnc-NRBF2-2:1 | SEQ4087 | 2.E-03 | 0.6 |
| All AD | GSEC:2 | SEQ3835 | 3.E-02 | 0.7 | msAD | lnc-NRBF2-2:1 | SEQ4087 | 1.E-02 | 0.6 |
| MCI | H1FX-AS1:15 | SEQ3313 | 2.E-05 | 1.6 | FTD | lnc-NRF1-2:1 | sEQS313 | 4.E-05 | 0.5 |
| DLB | H1FX-AS1:15 | SEQ3313 | 5.E-05 | 1.6 | All AD | lnc-NRF1-2:1 | sEQS313 | 4.E-02 | 0.7 |
| FTD | H1FX-AS1:15 | SEQ3313 | 2.E-04 | 1.5 | FTD | lnc-NT5C2-2:1 | SEQ3787 | 1. E-04 | 0.6 |
| All AD | H1FX-AS1:15 | SEQ3313 | 3.E-03 | 1.3 | miAD | lnc-NT5C2-2:1 | SEQ3787 | 6.E-03 | 0.7 |
| msAD | H1FX-AS1:15 | SEQ3313 | S.E-03 | 1.3 | All AD | lnc-NT5C2-2:1 | SEQ3787 | 8.E-03 | 0.7 |
| miAD | H1FX-AS1:15 | SEQ3313 | 8.E-03 | 1.3 | msAD | lnc-NT5C2-2:1 | SEQ3787 | 4.E-02 | 0.8 |
| DLB | H1FX-AS1:16 | SEQ3841 | 2.E-04 | 1.8 | DLB | lnc-NT5M-1:3 | SEQ5274 | 5. E-04 | 1.7 |
| miAD | H1FX-AS1:16 | SEQ3841 | 1.E-03 | 1.5 | FTD | lnc-NTNG1-4:1 | SEQ4301 | 1.E-05 | 0.6 |
| All AD | H1FX-AS1:16 | SEQ3841 | 1.E-03 | 1.4 | miAD | lnc-NTNG1-4:1 | SEQ4301 | 7.E-03 | 0.7 |
| msAD | H1FX-AS1:16 | SEQ3841 | 8.E-03 | 1.4 | All AD | lnc-NTNG1-4:1 | SEQ4301 | 1.E-02 | 0.8 |
| DLB | HCG26:5 | SEQ5696 | 1. E-04 | 1.6 | DLB | lnc-NTNG2-2:1 | SEQ5661 | 1. E-04 | 1.5 |
| DLB | HCP5:1 | sEQS322 | 4.E-04 | 1.5 | All AD | lnc-NUAK1-5:1 | sEQS317 | 4.E-02 | 0.9 |
| DLB | HCP5:3 | SEQ5476 | 2.E-04 | 1.3 | FTD | lnc-NUCB2-1:1 | SEQ4907 | 9.E-04 | 0.7 |
| FTD | HCP5:4 | SEQ5344 | 3.E-04 | 2.4 | All AD | lnc-NUDT3-1:3 | SEQ2725 | 3.E-02 | 1.2 |
| MCI | HCP5:4 | SEQ5344 | 4.E-04 | 2.2 | FTD | lnc-NUDT4P1-1:11 | SEQ5954 | 7.E-06 | 0.5 |
| DLB | HCP5:7 | sEQS321 | 4.E-04 | 1.5 | DLB | lnc-NUDT4P1-1:13 | SEQ4757 | 1.E-03 | 1.5 |
| FTD | HCP5:9 | SEQ5345 | 3.E-04 | 2.4 | DLB | lnc-NUDT4P1-1:2 | SEQ4564 | 2.E-03 | 1.4 |
| MCI | HCP5:9 | SEQ5345 | 4.E-04 | 2.2 | FTD | lnc-NUDT4P1-1:5 | SEQ5899 | 2.E-05 | 0.6 |
| FTD | HOTAIRM1:14 | SEQ3852 | 2.E-04 | 0.7 | FTD | lnc-NUDT4P1-9:1 | SEQ4171 | 1.E-07 | 0.5 |
| msAD | HOTAIRM1:14 | SEQ3852 | 9.E-03 | 0.7 | MCI | lnc-NUDT4P1-9:1 | SEQ4171 | 4.E-05 | 0.6 |
| All AD | HOTA!RM1:14 | SEQ3852 | 1.E-02 | 0.6 | All AD | lnc-NUDT4P1-9:1 | SEQ4171 | 3.E-03 | 0.7 |
| DLB | IL21R-AS1:1 | SEQ4596 | 2.E-03 | 1.7 | miAD | lnc-NUDT4P1-9:1 | SEQ4171 | 4.E-03 | 0.6 |
| DLB | IL21R-AS1:2 | SEQ5206 | 5. E-04 | 1.7 | msAD | lnc-NUDT4P1-9:1 | SEQ4171 | 1.E-02 | 0.7 |
| DLB | IL21R-AS1:3 | sEQ4S9S | 2.E-03 | 1.7 | All AD | lnc-NUMB-1:11 | SEQ5325 | 5.E-02 | 0.8 |
| MCI | IRAIN:2 | SEQ2863 | 1.E-03 | 1.6 | All AD | lnc-NUMB-1:6 | SEQS326 | 4.E-02 | 0.8 |
| MCI | IRAIN:3 | SEQ2864 | 1.E-03 | 1.6 | All AD | lnc-NUMB-2:1 | sEQS327 | 2.E-02 | 0.8 |
| DLB | ITFG2-AS1:3 | SEQ5620 | 2.E-04 | 1.6 | DLB | lnc-NUP188-1:1 | SEQ4556 | 2.E-03 | 1.3 |
| DLB | ITPKB-IT1:1 | SEQ4587 | 2.E-03 | 1.6 | All AD | lnc-NUS1-5:1 | SEQ3614 | 2.E-02 | 1.2 |
| FTD | JMJD1C-AS1:6 | SEQ5643 | 1. E-04 | 0.6 | DLB | lnc-NXNL1-7:2 | SEQ4402 | 2.E-03 | 1.3 |
| FTD | JPX:13 | SEQ4473 | 3.E-07 | 0.5 | FTD | lnc-NXPH2-1:1 | SEQ4903 | 9.E-04 | 0.6 |
| MCI | JPX:13 | SEQ4473 | 2.E-03 | 0.7 | All AD | lnc-NXPH2-1:1 | SEQ4903 | 2.E-02 | 0.8 |
| FTD | JPX:20 | SEQ5292 | 4.E-04 | 0.6 | FTD | lnc-NXPH2-3:10 | SEQ4666 | 7.E-05 | 0.3 |
| FTD | JPX:22 | SEQ5885 | 3.E-05 | 0.5 | MCI | lnc-NXPH2-3:10 | SEQ4666 | 9.E-05 | 0.3 |
| FTD | KCNJ2-AS1:3 | SEQ3865 | 5. E-04 | 0.6 | DLB | lnc-NXPH2-3:10 | SEQ4666 | 1.E-03 | 0.3 |
| miAD | KCNJ2-AS1:3 | SEQ3865 | 2.E-03 | 0.6 | miAD | lnc-NXPH2-3:5 | SEQ3634 | 1.E-02 | 0.6 |
| All AD | KCNJ2-AS1:3 | sEQ386S | 7.E-03 | 0.6 | All AD | lnc-NXPH2-3:5 | SEQ3634 | 1.E-02 | 0.7 |
| DLB | KCNQ1OT1:1 | SEQ4434 | 2.E-03 | 1.5 | msAD | lnc-NXPH2-3:5 | SEQ3634 | 5.E-02 | 0.8 |
| FTD | KCNQ10T1:2 | SEQ5774 | 7.E-05 | 0.5 | MCI | lnc-OBSCN-AS1-2:1 | SEQ5039 | 7.E-04 | 1.6 |
| DLB | KCNQ10T1:3 | SEQ4433 | 2.E-03 | 1.5 | DLB | lnc-OCM-3:4 | SEQ5076 | 2.E-08 | 3.3 |
| FTD | KCNQ10T1:5 | SEQ5762 | 8.E-05 | 0.5 | MCI | lnc-OCM-3:4 | SEQ5076 | 4.E-08 | 3.1 |
| FTD | KCNQ10T1:8 | SEQ0139 | 2.E-04 | 0.6 | msAD | lnc-OCM-3:4 | SEQ5076 | 6.E-05 | 1.7 |
| DLB | KDM4A-AS1:5 | SEQ4616 | 1.E-03 | 1.4 | All AD | lnc-OCM-3:4 | SEQ5076 | 7.E-05 | 1.7 |
| DLB | KIF9-AS1:9 | SEQ4492 | 2.E-03 | 1.4 | miAD | lnc-OCM-3:4 | SEQ5076 | 7.E-04 | 1.7 |
| DLB | LAMTORS-AS1:1 | SEQ5672 | 1. E-04 | 1.7 | FTD | lnc-ODF1-9:1 | SEQ0824 | 2.E-06 | 0.6 |
| DLB | LCMT1-AS2:4 | SEQ4657 | 1.E-03 | 1.9 | DLB | lnc-OGDH-2:3 | SEQ4682 | 1.E-03 | 1.4 |
| MCI | LEF1-AS1:1 | SEQ3877 | 1.E-03 | 1.9 | DLB | lnc-OIP5-1:1 | SEQ5863 | 4.E-05 | 1.9 |
| DLB | LEF1-AS1:1 | SEQ3877 | 2.E-03 | 1.9 | FTD | lnc-OIT3-1:1 | sEQS230 | 5. E-04 | 0.6 |
| miAD | LEF1-AS1:1 | SEQ3877 | 3.E-03 | 1.6 | msAD | lnc-OLIG1-3:2 | SEQ4101 | 1.E-02 | 1.6 |
| All AD | LEF1-AS1:1 | SEQ3877 | 4.E-03 | 1.5 | DLB | lnc-OPLAH-1:1 | SEQ5811 | 5.E-05 | 1.4 |
| msAD | LEF1-AS1:1 | SEQ3877 | 2.E-02 | 1.4 | miAD | lnc-OR10V1-1:1 | SEQ4329 | 6.E-03 | 0.8 |
| MCI | LEF1-AS1:2 | SEQ3882 | 3.E-04 | 2.3 | All AD | lnc-OR10V1-1:1 | SEQ4329 | 3.E-02 | 0.8 |
| DLB | LEF1-AS1:2 | SEQ3882 | 4.E-04 | 2.3 | DLB | lnc-OR1A2-1:1 | SEQ3560 | 1.E-03 | 1.4 |
| miAD | LEF1-AS1:2 | SEQ3882 | 2.E-03 | 1.7 | msAD | lnc-OR1J1-2:1 | SEQ3840 | 3.E-02 | 1.3 |
| All AD | LEF1-AS1:2 | SEQ3882 | 3.E-03 | 1.6 | DLB | lnc-OR4F15-4:5 | SEQ2780 | 4.E-07 | 1.7 |
| msAD | LEF1-AS1:2 | SEQ3882 | 2.E-02 | 1.5 | MCI | lnc-OR4F15-4:5 | SEQ2780 | 3.E-05 | 1.3 |
| All AD | LEF1-AS1:24 | SEQ3886 | 4.E-02 | 1.3 | FTD | lnc-OR4F15-4:5 | SEQ2780 | 8.E-04 | 1.3 |
| DLB | LIMD1-AS1:2 | SEQ2885 | 2.E-03 | 1.5 | msAD | lnc-OR4F15-4:5 | SEQ2780 | 1.E-02 | 1.2 |
| msAD | LINC00205:10 | SEQ3689 | 5.E-02 | 1.3 | All AD | lnc-OR4F15-4:5 | SEQ2780 | 4.E-02 | 1.1 |
| MCI | LINC00205:26 | SEQ4533 | 6.E-04 | 1.9 | All AD | lnc-OR4F16-15:5 | SEQ5342 | 5.E-02 | 0.7 |
| DLB | LINC00205:26 | SEQ4533 | 2.E-03 | 1.9 | FTD | lnc-OR4F17-5:1 | SEQ5002 | 8.E-04 | 0.7 |
| MCI | LINC00211:13 | SEQ4965 | 8.E-04 | 1.6 | miAD | lnc-OR4F17-8:1 | SEQ4085 | 3.E-03 | 0.6 |
| FTD | LINC00211:15 | SEQ3890 | 3.E-04 | 0.4 | All AD | lnc-OR4F17-8:1 | SEQ4085 | 3.E-03 | 0.6 |
| All AD | LINC00211:15 | SEQ3890 | 6.E-03 | 0.5 | msAD | lnc-OR4F17-8:1 | SEQ4085 | 1.E-02 | 0.6 |
| msAD | LINC00211:15 | SEQ3890 | 7.E-03 | 0.4 | All AD | lnc-OR4F29-1:10 | sEQS346 | 4.E-02 | 0.6 |
| MCI | LINC00235:1 | SEQ3731 | 5. E-04 | 2.1 | DLB | lnc-OR4F29-11:1 | SEQ4438 | 2.E-03 | 1.5 |
| DLB | LINC00235:! | SEQ3731 | 9.E-04 | 2.3 | DLB | lnc-OR4F29-11:5 | SEQ5967 | 4.E-06 | 1.9 |
| All AD | LINC00235:! | SEQ3731 | 2.E-02 | 1.4 | DLB | lnc-OR4F29-11:6 | SEQ5611 | 2.E-04 | 1.4 |
| msAD | LINC00235:! | SEQ3731 | 4.E-02 | 1.4 | miAD | lnc-OR4F29-3:11 | SEQ0044 | 2.E-03 | 0.5 |
| All AD | LINC00402:4 | SEQ3893 | 2.E-02 | 1.3 | All AD | lnc-OR4F29-3:11 | SEQ0044 | 4.E-03 | 0.6 |
| FTD | LINC00472:23 | SEQ4670 | 5. E-04 | 0.7 | msAD | lnc-OR4F29-3:11 | SEQ0044 | 4.E-02 | 0.7 |
| MCI | LINC00472:23 | SEQ4670 | 1.E-03 | 0.8 | miAD | lnc-OR4F29-3:7 | SEQ4602 | 1.E-03 | 0.6 |
| FTD | LINC00472:38 | SEQ5930 | 1.E-05 | 0.6 | All AD | lnc-OR4F29-3:7 | SEQ4602 | 7.E-03 | 0.7 |
| FTD | LINC00472:41 | SEQ3848 | 3.E-11 | 0.1 | All AD | lnc-OR4F29-8:3 | SEQ3836 | 2.E-02 | 0.7 |
| MCI | LINC00472:41 | SEQ3848 | 2.E-08 | 0.2 | msAD | lnc-OR4F29-8:3 | SEQ3836 | 3.E-02 | 0.7 |
| miAD | LINC00472:41 | SEQ3848 | 3.E-05 | 0.3 | DLB | lnc-OR4F29-8:30 | SEQ5241 | 5. E-04 | 1.4 |
| DLB | LINC00472:41 | SEQ3848 | 9.E-05 | 0.2 | FTD | lnc-OR4F29-8:48 | SEQ5882 | 3.E-05 | 0.4 |
| All AD | LINC00472:41 | SEQ3848 | 7.E-04 | 0.3 | All AD | lnc-OR4F3-5:4 | SEQ5350 | 3.E-02 | 0.6 |
| msAD | LINC00472:41 | SEQ3848 | 3.E-02 | 0.3 | msAD | lnc-OR4F4-4:3 | SEQ3996 | 2.E-02 | 1.7 |
| All AD | LINC00476:1 | SEQ3719 | 2.E-02 | 1.3 | miAD | lnc-OR4FS-2:1 | SEQ4371 | 4.E-03 | 0.6 |
| msAD | LINC00476:1 | SEQ3719 | 4.E-02 | 1.3 | All AD | lnc-OR4FS-2:1 | SEQ4371 | 8.E-03 | 0.6 |
| FTD | LINC00533:8 | SEQ3864 | 5. E-04 | 0.5 | DLB | lnc-OR4F6-1:1 | SEQ4266 | 6.E-05 | 2.0 |
| miAD | LINC00533:8 | SEQ3864 | 6.E-03 | 0.5 | msAD | lnc-OR4F6-1:1 | SEQ4266 | 9.E-03 | 1.4 |
| All AD | LINC00533:8 | SEQ3864 | 7.E-03 | 0.6 | All AD | lnc-OR4F6-1:1 | SEQ4266 | 2.E-02 | 1.3 |
| msAD | LINC00533:8 | SEQ3864 | 3.E-02 | 0.6 | msAD | lnc-OR52K2-1:1 | SEQ4300 | 7.E-03 | 0.7 |
| DLB | LINC00649:37 | SEQ5639 | 2.E-04 | 2.1 | All AD | lnc-OR52K2-1:1 | SEQ4300 | 1.E-02 | 0.8 |
| MCI | LINC00659:10 | SEQ4726 | 1.E-03 | 2.2 | DLB | lnc-OR6C65-3:1 | SEQ5441 | 3.E-04 | 1.5 |
| FTD | LINC00667:21 | SEQ5085 | 6.E-04 | 0.6 | DLB | lnc-OR7C2-1:2 | sEQS740 | 9.E-05 | 1.9 |
| FTD | LINC00667:3 | sEQS304 | 4.E-04 | 1.9 | MCI | lnc-OSBPL7-3:1 | sEQ3S69 | 5.E-05 | 1.6 |
| DLB | LINC00677:6 | SEQ4555 | 2.E-03 | 1.3 | FTD | lnc-OSBPL7-3:1 | SEQ3569 | 1. E-04 | 1.6 |
| DLB | LINC00685:6 | SEQ4963 | 3.E-04 | 1.8 | DLB | lnc-OSBPL7-3:1 | SEQ3569 | 2.E-03 | 1.5 |
| MCI | LINC00685:6 | SEQ4963 | 8.E-04 | 1.6 | All AD | lnc-OSBPL7-3:2 | SEQ2813 | 4.E-02 | 0.8 |
| DLB | LINC00847:2 | SEQ4857 | 9.E-04 | 1.5 | FTD | lnc-OSBPL7-3:3 | SEQ0534 | 2.E-05 | 0.7 |
| miAD | LINC00847:3 | SEQ3912 | 5.E-03 | 1.7 | miAD | lnc-OSBPL7-3:3 | SEQ0534 | 1.E-02 | 0.8 |
| All AD | LINC00847:3 | SEQ3912 | 3.E-02 | 1.4 | All AD | lnc-OSBPL7-3:3 | SEQ0534 | 2.E-02 | 0.8 |
| FTD | LINC00861:6 | sEQ391S | 3.E-08 | 5.9 | FTD | lnc-OSBPL7-3:4 | SEQ0535 | 4.E-05 | 0.7 |
| DLB | LINC00861:6 | SEQ3915 | 6.E-08 | 6.6 | FTD | lnc-OSBPL7-3:6 | SEQ0536 | 4.E-05 | 0.7 |
| MCI | LINC00861:6 | sEQ391S | 3.E-06 | 5.1 | All AD | lnc-OSBPL7-3:6 | SEQ0536 | 3.E-02 | 0.8 |
| All AD | LINC00861:6 | sEQ391S | 1. E-04 | 1.8 | msAD | lnc-OSMR-2:2 | SEQ4340 | 5.E-03 | 0.6 |
| miAD | LINC00861:6 | sEQ391S | 3.E-04 | 1.8 | FTD | lnc-OTOA-1:2 | SEQ3943 | 1. E-04 | 0.5 |
| msAD | LINC00861:6 | sEQ391S | 1.E-03 | 1.8 | All AD | lnc-OTOA-1:2 | SEQ3943 | 6.E-03 | 0.6 |
| MCI | LINC00869:68 | SEQ3730 | 3.E-04 | 2.1 | miAD | lnc-OTOA-1:2 | SEQ3943 | 7.E-03 | 0.6 |
| DLB | LINC00869:68 | SEQ3730 | 8.E-04 | 1.9 | msAD | lnc-OTOA-1:2 | SEQ3943 | 3.E-02 | 0.7 |
| miAD | LINC00869:68 | SEQ3730 | 1.E-03 | 1.7 | DLB | lnc-OTULIN-4:1 | SEQ5694 | 1. E-04 | 1.5 |
| All AD | LINC00869:68 | SEQ3730 | 4.E-03 | 1.5 | DLB | lnc-P4HTM-4:1 | sEQS334 | 4.E-04 | 1.7 |
| msAD | LINC00869:68 | SEQ3730 | 4.E-02 | 1.4 | MCI | lnc-PABPC1L2B-3:1 | SEQ4448 | 3.E-04 | 1.7 |
| miAD | LINC00869:73 | SEQ3922 | 3.E-03 | 0.5 | DLB | lnc-PABPC1L2B-3:1 | SEQ4448 | 2.E-03 | 1.6 |
| All AD | LINC00869:73 | SEQ3922 | 3.E-03 | 0.6 | DLB | lnc-PABPN1L-1:1 | SEQ2986 | 2.E-03 | 1.6 |
| msAD | LINC00869:73 | SEQ3922 | 2.E-02 | 0.6 | MCI | lnc-PACSIN1-1:1 | SEQ5452 | 1. E-04 | 1.6 |
| All AD | LINC00877:15 | SEQ3743 | 3.E-02 | 0.8 | DLB | lnc-PACSIN1-1:1 | SEQ5452 | 3.E-04 | 1.8 |
| msAD | LINC00877:15 | SEQ3743 | 4.E-02 | 0.8 | DLB | lnc-PADI2-1:1 | SEQ5936 | 1.E-05 | 1.8 |
| FTD | LINC00894:50 | SEQ3925 | 6.E-05 | 0.6 | DLB | lnc-PAF1-3:1 | SEQ5113 | 6.E-04 | 1.4 |
| All AD | LINC00894:50 | sEQ392S | 4.E-02 | 0.7 | MCI | lnc-PAGR1-2:8 | SEQ5430 | 1. E-04 | 1.4 |
| msAD | LINC00910:32 | SEQ3926 | 2.E-02 | 1.3 | DLB | lnc-PAGR1-2:8 | SEQ5430 | 3.E-04 | 1.4 |
| All AD | LINC00910:32 | SEQ3926 | 4.E-02 | 1.2 | FTD | lnc-PAIP2B-2:1 | SEQ5874 | 3.E-05 | 0.6 |
| msAD | LINC00926:2 | SEQ3927 | 2.E-02 | 1.6 | FTD | lnc-PALLD-8:1 | SEQ2633 | 2.E-05 | 0.6 |
| All AD | LINC00926:2 | SEQ3927 | 4.E-02 | 1.5 | DLB | lnc-PANK3-13:1 | SEQ2629 | 5. E-04 | 1.5 |
| miAD | LINC00937:21 | SEQ3930 | 1.E-02 | 0.6 | FTD | lnc-PANK3-13:2 | SEQ2618 | 7.E-05 | 0.6 |
| All AD | LINC00937:21 | SEQ3930 | 2.E-02 | 0.7 | DLB | lnc-PAN01-1:1 | SEQ5485 | 2.E-04 | 1.5 |
| msAD | LINC00938:7 | SEQ3931 | 2.E-02 | 1.4 | MCI | lnc-PAPDS-2:1 | SEQ5851 | 4.E-05 | 1.8 |
| All AD | LINC00938:7 | SEQ3931 | 3.E-02 | 1.2 | DLB | lnc-PAPDS-2:1 | SEQ5851 | 4.E-05 | 1.9 |
| DLB | LINC00957:7 | SEQ5252 | 4.E-05 | 1.7 | FTD | lnc-PAPLN-2:1 | SEQ5163 | 5.E-04 | 0.8 |
| MCI | LINC00957:7 | SEQ5252 | 5. E-04 | 1.5 | FTD | lnc-PAPPA2-2:1 | SEQ4328 | 2.E-05 | 0.6 |
| MCI | LINC00963:12 | SEQ5349 | 2.E-04 | 0.5 | miAD | lnc-PAPPA2-2:1 | SEQ4328 | 6.E-03 | 0.7 |
| FTD | LINC00963:12 | SEQ5349 | 4.E-04 | 0.5 | All AD | lnc-PAPPA2-2:1 | SEQ4328 | 1.E-02 | 0.8 |
| DLB | LINC00963:7 | SEQ4528 | 2.E-03 | 1.7 | FTD | lnc-PAPPA2-6:1 | SEQ4247 | 5. E-04 | 0.7 |
| MCI | LINC00989:1 | SEQ3938 | 6.E-04 | 1.6 | miAD | lnc-PAPPA2-6:1 | SEQ4247 | 9.E-03 | 0.7 |
| msAD | LINC00989:1 | SEQ3938 | 4.E-03 | 1.4 | All AD | lnc-PAPPA2-6:1 | SEQ4247 | 1.E-02 | 0.8 |
| All AD | LINC00989:1 | SEQ3938 | 5.E-03 | 1.3 | FTD | lnc-PAPPA2-7:1 | SEQ0165 | 2.E-04 | 0.6 |
| DLB | LINC01000:7 | SEQ2877 | 2.E-04 | 1.5 | All AD | lnc-PAPPA2-7:1 | SEQ0165 | 2.E-03 | 0.7 |
| MCI | LINC01002:1 | SEQ5287 | 3.E-04 | 0.6 | miAD | lnc-PAPPA2-7:1 | SEQ0165 | 2.E-03 | 0.7 |
| FTD | LINC01002:1 | SEQ5287 | 4.E-04 | 0.5 | msAD | lnc-PAPPA2-7:1 | SEQ0165 | 8.E-03 | 0.7 |
| miAD | LINC01002:27 | sEQ394S | 2.E-03 | 0.5 | miAD | lnc-PAPSS2-10:1 | SEQ4072 | 1.E-02 | 0.7 |
| All AD | LINC01002:27 | SEQ3945 | 1.E-02 | 0.6 | All AD | lnc-PAPSS2-10:1 | SEQ4072 | 3.E-02 | 0.8 |
| miAD | LINC01002:9 | SEQ3759 | S.E-03 | 0.5 | All AD | lnc-PATL2-9:1 | SEQ3944 | 1.E-02 | 0.7 |
| All AD | LINC01002:9 | sEQ37S9 | 7.E-03 | 0.6 | msAD | lnc-PATL2-9:1 | SEQ3944 | 3.E-02 | 0.7 |
| msAD | LINC01002:9 | sEQ37S9 | 4.E-02 | 0.7 | FTD | lnc-PAWR-14:1 | SEQ4343 | 3.E-05 | 0.5 |
| DLB | LINC01089:27 | SEQ5862 | 2.E-05 | 1.9 | miAD | lnc-PAWR-14:1 | SEQ4343 | 5.E-03 | 0.6 |
| MCI | LINC01089:27 | SEQ5862 | 4.E-05 | 1.9 | All AD | lnc-PAWR-14:1 | SEQ4343 | 7.E-03 | 0.6 |
| DLB | LINC01089:3 | SEQ5705 | 5.E-05 | 1.9 | All AD | lnc-PAXX-2:1 | SEQ3639 | 4.E-02 | 0.8 |
| MCI | LINC01089:3 | SEQ5705 | 1. E-04 | 1.8 | msAD | lnc-PAXX-2:1 | SEQ3639 | 5.E-02 | 0.9 |
| DLB | LINC01128:4 | SEQ3953 | 1. E-04 | 1.9 | miAD | lnc-PCBP1-2:1 | SEQ3999 | 2.E-03 | 0.6 |
| MCI | LINC01128:4 | SEQ3953 | 3.E-04 | 1.7 | All AD | lnc-PCBP1-2:1 | SEQ3999 | 3.E-03 | 0.6 |
| All AD | LINC01128:4 | SEQ3953 | 6.E-03 | 1.3 | msAD | lnc-PCBP1-2:1 | SEQ3999 | 2.E-02 | 0.6 |
| miAD | LINC01128:4 | SEQ3953 | 6.E-03 | 1.3 | FTD | lnc-PCDHGA1-1:3 | SEQ5923 | 1.E-05 | 0.6 |
| msAD | LINC01128:4 | SEQ3953 | 2.E-02 | 1.3 | FTD | lnc-PCDHGA1-1:4 | SEQ5922 | 1.E-05 | 0.6 |
| All AD | LINC01136:8 | SEQ3958 | 2.E-03 | 0.5 | FTD | lnc-PCDHGA1-3:2 | SEQ4819 | 4.E-07 | 0.4 |
| miAD | LINC01136:8 | SEQ3958 | 2.E-03 | 0.5 | MCI | lnc-PCDHGA1-3:2 | SEQ4819 | 4.E-04 | 0.5 |
| msAD | LINC01136:8 | SEQ3958 | 8.E-03 | 0.5 | miAD | lnc-PCDHGA1-3:2 | SEQ4819 | 1.E-03 | 0.5 |
| DLB | LINC01138:20 | SEQ3672 | 6.E-04 | 1.7 | DLB | lnc-PCGF2-3:1 | SEQ5700 | 1. E-04 | 1.6 |
| msAD | LINC01138:20 | SEQ3672 | 5.E-02 | 1.2 | DLB | lnc-PCGF2-4:1 | SEQ3602 | 4.E-05 | 1.5 |
| msAD | LINC01138:23 | SEQ3889 | 3.E-02 | 0.8 | FTD | lnc-PCNX4-3:1 | SEQ4285 | 1.E-08 | 0.5 |
| All AD | LINC01138:23 | SEQ3889 | 3.E-02 | 0.8 | MCI | lnc-PCNX4-3:1 | SEQ4285 | 8.E-05 | 0.5 |
| FTD | LINC01154:11 | SEQ5747 | 9.E-05 | 0.7 | miAD | lnc-PCNX4-3:1 | SEQ4285 | 7.E-03 | 0.7 |
| MCI | LINC01215:3 | SEQ3963 | 2.E-04 | 2.5 | All AD | lnc-PCNX4-3:1 | SEQ4285 | 1.E-02 | 0.7 |
| msAD | LINC01215:3 | SEQ3963 | 1.E-02 | 1.5 | FTD | lnc-PCP4L1-3:2 | SEQ4902 | 9.E-04 | 0.6 |
| All AD | LINC01215:3 | SEQ3963 | 4.E-02 | 1.3 | DLB | lnc-PCSK7-1:4 | SEQ5619 | 2.E-04 | 1.6 |
| DLB | LINC01224:32 | SEQ5454 | 3.E-04 | 1.8 | FTD | lnc-PCYOX1-4:1 | SEQ5998 | 2.E-07 | 0.3 |
| DLB | LINC01224:33 | SEQ4653 | 2.E-04 | 2.1 | MCI | lnc-PCYOX1-4:1 | sEQS998 | 4.E-07 | 0.4 |
| MCI | LINC01224:33 | SEQ4653 | 1.E-03 | 1.8 | FTD | lnc-PDCD4-6:1 | SEQ5384 | 2.E-05 | 0.6 |
| DLB | LINC01224:56 | SEQ5278 | 5. E-04 | 1.8 | All AD | lnc-PDCD4-6:1 | SEQ5384 | 5.E-02 | 0.8 |
| DLB | LINC01311:5 | SEQ3967 | 6.E-04 | 1.5 | FTD | lnc-PDCL2-4:1 | SEQ5475 | 2.E-07 | 0.6 |
| MCI | LINC01311:5 | SEQ3967 | 1.E-03 | 1.5 | MCI | lnc-PDCL2-4:1 | SEQ5475 | 2.E-04 | 0.7 |
| All AD | LINC01311:5 | SEQ3967 | 4.E-02 | 1.2 | msAD | lnc-PDE6D-3:1 | SEQ3793 | 4.E-02 | 1.3 |
| FTD | LINC01355:1 | SEQ5509 | 2.E-04 | 0.6 | MCI | lnc-PDGFA-6:2 | SEQ5057 | 4.E-08 | 6.3 |
| miAD | LINC01359:11 | SEQ3826 | 1.E-03 | 0.6 | FTD | lnc-PDGFA-6:2 | SEQ5057 | 7.E-06 | 3.3 |
| All AD | LINC01359:11 | SEQ3826 | 4.E-03 | 0.6 | DLB | lnc-PDGFA-6:2 | SEQ5057 | 7.E-04 | 3.1 |
| msAD | LINC01359:11 | SEQ3826 | 4.E-02 | 0.7 | All AD | lnc-PDGFA-6:S | SEQ5389 | 4.E-02 | 0.6 |
| FTD | LI N C01410:15 | sEQS978 | 5.E-07 | 0.2 | miAD | lnc-PDGFA-6:6 | SEQ4378 | 3.E-03 | 0.7 |
| MCI | LINC01410:15 | SEQ5978 | 3.E-06 | 0.2 | All AD | lnc-PDGFA-6:6 | SEQ4378 | 1.E-02 | 0.7 |
| FTD | LINC01410:17 | SEQ3799 | 9.E-05 | 0.5 | DLB | lnc-PDGFB-2:1 | SEQ2826 | 5. E-04 | 1.6 |
| All AD | LINC01410:17 | SEQ3799 | 1.E-02 | 0.6 | FTD | lnc-PDK3-1:1 | SEQ2348 | 7.E-04 | 0.7 |
| msAD | LINC01410:17 | SEQ3799 | 4.E-02 | 0.7 | All AD | lnc-PDK3-1:1 | SEQ2348 | 3.E-02 | 0.8 |
| DLB | LINC01465:! | SEQ4432 | 1.E-04 | 1.8 | FTD | lnc-PDSSB-2:1 | SEQ5392 | 5. E-06 | 0.6 |
| FTD | LINC01465:! | SEQ4432 | 6.E-04 | 1.6 | All AD | lnc-PDSSB-2:1 | SEQ5392 | 3.E-02 | 0.8 |
| MCI | LINC01465:! | SEQ4432 | 2.E-03 | 1.5 | FTD | lnc-PDSS1-4:1 | SEQ5363 | 1.E-08 | 0.5 |
| FTD | LINC01481:13 | SEQ5902 | 1.E-08 | 0.1 | MCI | lnc-PDSS1-4:1 | SEQ5363 | 2.E-06 | 0.5 |
| MCI | LINC01481:13 | SEQ5902 | 2.E-05 | 0.1 | miAD | lnc-PDSS1-4:1 | SEQ5363 | 4.E-04 | 0.7 |
| FTD | LINC01481:8 | SEQ0478 | 4.E-06 | 0.5 | All AD | lnc-PDSS1-4:1 | SEQ5363 | 4.E-03 | 0.7 |
| All AD | LINC01550:1 | SEQ3981 | 4.E-02 | 1.3 | FTD | lnc-PDSS1-6:1 | SEQ4152 | 2.E-07 | 0.5 |
| MCI | LINC01550:11 | SEQ5742 | 9.E-05 | 3.5 | miAD | lnc-PDSS1-6:1 | SEQ4152 | 1.E-02 | 0.7 |
| FTD | LINC01578:13 | sEQ398S | 6.E-09 | 0.4 | FTD | lnc-PDSS1-7:1 | SEQ3851 | 2.E-04 | 0.6 |
| MCI | LINC01578:13 | SEQ3985 | 4.E-05 | 0.4 | miAD | lnc-PDSS1-7:1 | SEQ3851 | 7.E-03 | 0.7 |
| miAD | LINC01578:13 | sEQ398S | 8.E-04 | 0.6 | All AD | lnc-PDSS1-7:1 | SEQ3851 | 8.E-03 | 0.7 |
| DLB | LINC01578:13 | sEQ398S | 1.E-03 | 0.6 | msAD | lnc-PDSS1-7:1 | SEQ3851 | 3.E-02 | 0.7 |
| All AD | LINC01578:13 | sEQ398S | 8.E-03 | 0.6 | FTD | lnc-PDSS1-9:1 | SEQ3667 | 4.E-05 | 0.7 |
| FTD | LINC01578:2 | SEQ3987 | 2.E-04 | 0.6 | All AD | lnc-PDSS1-9:1 | SEQ3667 | 2.E-02 | 0.8 |
| All AD | LINC01578:2 | SEQ3987 | 3.E-02 | 0.7 | msAD | lnc-PDSS1-9:1 | SEQ3667 | 5.E-02 | 0.8 |
| DLB | LINC01623:9 | SEQ5725 | 9.E-05 | 1.4 | FTD | lnc-PDYN-1:13 | SEQ4327 | 6.E-04 | 0.6 |
| DLB | LINC01678:2 | SEQ5580 | 2.E-04 | 1.9 | miAD | lnc-PDYN-1:13 | SEQ4327 | 6.E-03 | 0.6 |
| DLB | LINC01715:4 | sEQ32S6 | 1.E-05 | 0.4 | All AD | lnc-PDYN-1:13 | SEQ4327 | 2.E-02 | 0.7 |
| MCI | LINC01715:4 | SEQ3256 | 5. E-04 | 0.3 | miAD | lnc-PDYN-1:16 | SEQ4111 | 1.E-02 | 0.7 |
| DLB | LINC01762:6 | SEQ5671 | 1. E-04 | 1.7 | All AD | lnc-PDYN-1:16 | SEQ4111 | 3.E-02 | 0.7 |
| DLB | LINC01762:7 | SEQ5670 | 1. E-04 | 1.7 | All AD | lnc-PDYN-1:9 | SEQ3089 | 5.E-02 | 0.7 |
| FTD | LINC01814:4 | SEQ3869 | 9.E-04 | 0.7 | DLB | lnc-PDZD2-1:1 | SEQ3861 | 8.E-05 | 1.5 |
| miAD | LINC01814:4 | SEQ3869 | 2.E-03 | 0.6 | All AD | lnc-PDZD2-1:1 | SEQ3861 | 2.E-02 | 1.2 |
| All AD | LINC01814:4 | SEQ3869 | 4.E-03 | 0.7 | msAD | lnc-PDZD2-1:1 | SEQ3861 | 3.E-02 | 1.2 |
| msAD | LINC01814:4 | SEQ3869 | 3.E-02 | 0.8 | DLB | lnc-PEA15-1:2 | SEQ4931 | 8.E-04 | 1.4 |
| MCI | LINC01891:2 | SEQ4455 | 2.E-03 | 1.8 | DLB | lnc-PEBP4-1:3 | SEQ4055 | 2.E-04 | 1.4 |
| All AD | LINC02035:2 | SEQ3993 | 6.E-03 | 0.8 | msAD | lnc-PEBP4-1:3 | SEQ4055 | 2.E-02 | 1.2 |
| miAD | LINC02035:2 | SEQ3993 | 1.E-02 | 0.8 | MCI | lnc-PELP1-2:1 | SEQ5134 | 6.E-04 | 1.7 |
| msAD | LINC02035:2 | SEQ3993 | 2.E-02 | 0.8 | DLB | lnc-PER2-5:1 | SEQ5695 | 1. E-04 | 1.6 |
| msAD | LINC02035:4 | SEQ3780 | 4.E-02 | 1.3 | DLB | lnc-PEX11G-3:1 | SEQ5315 | 9.E-06 | 1.6 |
| FTD | LINC02062:11 | sEQS912 | 2.E-05 | 0.5 | FTD | lnc-PEX11G-3:1 | SEQ5315 | 3.E-04 | 1.4 |
| FTD | LINC02062:12 | SEQ3998 | 8.E-05 | 0.6 | MCI | lnc-PEX11G-3:1 | SEQ5315 | 4.E-04 | 1.5 |
| miAD | LINC02062:12 | SEQ3998 | 9.E-03 | 0.7 | DLB | lnc-PEX11G-4:3 | SEQ5281 | 2.E-05 | 2.0 |
| All AD | LINC02062:12 | SEQ3998 | 2.E-02 | 0.7 | MCI | lnc-PEX11G-4:3 | SEQ5281 | 5. E-04 | 1.9 |
| miAD | LINC02068:5 | SEQ4145 | 1.E-02 | 0.6 | DLB | lnc-PEX6-1:1 | SEQ2942 | 6.E-05 | 1.6 |
| All AD | LINC02100:2 | SEQ4000 | 3.E-02 | 0.7 | FTD | lnc-PEX6-3:1 | SEQ5896 | 2.E-06 | 1.7 |
| FTD | LINC02100:8 | SEQ5150 | 5.E-04 | 0.6 | DLB | lnc-PEX6-3:1 | SEQ5896 | 4.E-06 | 1.8 |
| MCI | LINC02288:2 | SEQ5435 | 3.E-04 | 1.5 | MCI | lnc-PEX6-3:1 | SEQ5896 | 2.E-05 | 1.8 |
| MCI | LINC02288:4 | SEQ4002 | 2.E-05 | 2.1 | All AD | lnc-PEX7-1:4 | SEQ5402 | 3.E-02 | 0.8 |
| FTD | LINC02288:4 | SEQ4002 | 2.E-04 | 1.7 | FTD | lnc-PEX7-1:7 | SEQ5606 | 2.E-04 | 0.7 |
| DLB | LINC02288:4 | SEQ4002 | 5.E-04 | 1.8 | All AD | lnc-PEX7-4:1 | SEQ3855 | 1.E-02 | 0.8 |
| All AD | LINC02288:4 | SEQ4002 | 2.E-02 | 1.2 | msAD | lnc-PEX7-4:1 | SEQ3855 | 3.E-02 | 0.8 |
| FTD | LINC02328:9 | SEQ4007 | 3.E-05 | 0.5 | FTD | lnc-PFDN4-11:2 | SEQ5086 | 6.E-04 | 0.6 |
| All AD | LINC02328:9 | SEQ4007 | 1.E-02 | 0.7 | All AD | lnc-PFDN4-11:2 | SEQ5086 | 2.E-02 | 0.7 |
| msAD | LINC02328:9 | SEQ4007 | 1.E-02 | 0.7 | MCI | lnc-PFDN6-2:3 | SEQ3468 | 1.E-07 | 2.5 |
| MCI | LINC02361:3 | SEQ4009 | 2.E-04 | 1.7 | FTD | lnc-PFDN6-2:3 | SEQ3468 | 2.E-07 | 2.0 |
| All AD | LINC02361:3 | SEQ4009 | 3.E-02 | 1.2 | DLB | lnc-PFDN6-2:3 | SEQ3468 | 4.E-06 | 2.4 |
| miAD | LINC02362:13 | SEQ4012 | 1.E-02 | 0.6 | miAD | lnc-PFDN6-2:3 | SEQ3468 | 1.E-03 | 1.3 |
| All AD | LINC02362:13 | SEQ4012 | 2.E-02 | 0.7 | All AD | lnc-PFDN6-2:3 | SEQ3468 | 5.E-03 | 1.2 |
| miAD | LINC02362:14 | SEQ4014 | 1.E-02 | 0.6 | All AD | lnc-PFKFB3-3:5 | SEQ5408 | 5.E-02 | 0.7 |
| All AD | LINC02362:14 | SEQ4014 | 2.E-02 | 0.7 | DLB | lnc-PGAM2-1:1 | SEQ2909 | 6.E-04 | 1.5 |
| FTD | LINC02362:17 | SEQ4825 | 6.E-06 | 0.4 | FTD | lnc-PGAP1-2:1 | SEQ4147 | 1.E-09 | 0.4 |
| MCI | LINC02362:17 | SEQ4825 | 3.E-04 | 0.5 | MCI | lnc-PGAP1-2:1 | SEQ4147 | 4.E-05 | 0.5 |
| DLB | LINC02362:17 | SEQ4825 | 9.E-04 | 0.5 | miAD | lnc-PGAP1-2:1 | SEQ4147 | 1.E-02 | 0.6 |
| miAD | LINC02363:2 | SEQ4017 | 1.E-02 | 0.7 | All AD | lnc-PGAP1-2:1 | SEQ4147 | 2.E-02 | 0.7 |
| All AD | LINC02363:2 | SEQ4017 | 3.E-02 | 0.8 | DLB | lnc-PGBD5-1:1 | SEQ2755 | 4.E-04 | 1.5 |
| miAD | LINC02363:6 | SEQ4019 | 7.E-03 | 0.6 | FTD | lnc-PGGT1B-1:1 | SEQ5999 | 3.E-07 | 0.2 |
| All AD | LINC02363:6 | SEQ4019 | 3.E-02 | 0.7 | FTD | lnc-PGM1-1:1 | SEQ5646 | 1. E-04 | 0.6 |
| All AD | LINC02384:5 | SEQ4020 | 3.E-02 | 0.6 | DLB | lnc-PHB2-2:1 | SEQ4561 | 2.E-03 | 1.4 |
| All AD | LINC02397:10 | SEQ3328 | 3.E-02 | 1.5 | All AD | lnc-PHB2-4:3 | SEQ5414 | 3.E-02 | 1.3 |
| msAD | LINC02397:10 | SEQ3328 | 4.E-02 | 1.5 | FTD | lnc-PHF20L1-6:1 | SEQ4316 | 1.E-08 | 0.4 |
| msAD | LINC02397:15 | SEQ3901 | 3.E-02 | 1.5 | MCI | lnc-PHF20L1-6:1 | SEQ4316 | 6.E-05 | 0.5 |
| All AD | LINC02397:15 | SEQ3901 | 4.E-02 | 1.4 | miAD | lnc-PHF20L1-6:1 | SEQ4316 | 6.E-03 | 0.6 |
| FTD | LINC02422:5 | SEQ4022 | 6.E-05 | 0.7 | All AD | lnc-PHF20L1-6:1 | SEQ4316 | 1.E-02 | 0.6 |
| All AD | LINC02422:5 | SEQ4022 | 5.E-02 | 0.8 | FTD | lnc-PHLDA1-1:2 | SEQ5221 | 5. E-04 | 0.4 |
| miAD | LINC02471:3 | SEQ3866 | 2.E-03 | 0.6 | DLB | lnc-PHYHD1-1:1 | SEQ3086 | 8.E-05 | 1.7 |
| All AD | LINC02471:3 | SEQ3866 | 4.E-03 | 0.6 | All AD | lnc-PIGBOS1-2:1 | SEQ5417 | 3.E-02 | 0.8 |
| msAD | LINC02471:3 | SEQ3866 | 3.E-02 | 0.7 | All AD | lnc-PIGBOS1-3:1 | SEQ5418 | 4.E-02 | 0.8 |
| MCI | LINC02482:12 | SEQ3880 | 6.E-05 | 2.0 | FTD | lnc-PIGBOS1-4:1 | SEQ5062 | 7.E-04 | 0.6 |
| All AD | LINC02482:12 | SEQ3880 | 1.E-02 | 1.3 | FTD | lnc-PIGBOS1-5:1 | SEQ5546 | 2.E-04 | 0.6 |
| msAD | LINC02482:12 | SEQ3880 | 3.E-02 | 1.4 | msAD | lnc-PIGN-7:3 | SEQ4342 | 5.E-03 | 0.6 |
| FTD | LINC-PINT:10 | SEQ4029 | 4.E-04 | 0.7 | All AD | lnc-PIGN-7:3 | SEQ4342 | 8.E-03 | 0.6 |
| miAD | LINC-PINT:10 | SEQ4029 | 1.E-02 | 0.8 | FTD | lnc-PIK3AP1-2:1 | SEQ5136 | 5. E-04 | 1.6 |
| All AD | LINC-PINT:10 | SEQ4029 | 3.E-02 | 0.8 | MCI | lnc-PIK3AP1-2:1 | SEQ5136 | 6.E-04 | 1.7 |
| DLB | LINC-PINT:11 | SEQ5167 | 3.E-06 | 5.E-02 | All AD | lnc-PIK3AP1-2:1 | SEQ5136 | 4.E-02 | 1.2 |
| FTD | LINC-PINT:11 | SEQ5167 | 2.E-04 | 0.1 | miAD | lnc-PIK3IP1-2:1 | SEQ4299 | 7.E-03 | 0.7 |
| MCI | LINC-PINT:11 | SEQ5167 | 5. E-04 | 4.E-02 | All AD | lnc-PIK3IP1-2:1 | SEQ4299 | 1.E-02 | 0.7 |
| FTD | LINC-PINT:16 | SEQ2192 | 7.E-06 | 0.6 | MCI | lnc-PILRB-1:5 | SEQ3009 | 2.E-04 | 1.8 |
| FTD | LINC-PINT:25 | SEQ5971 | 4.E-08 | 0.2 | DLB | lnc-PILRB-1:5 | SEQ3009 | 3.E-04 | 1.7 |
| MCI | LINC-PINT:25 | SEQ5971 | 3.E-06 | 0.3 | FTD | lnc-PINK1-2:1 | SEQ2734 | 1.E-10 | 0.3 |
| All AD | LINC-PINT:33 | SEQ4036 | 1.E-02 | 0.7 | MCI | lnc-PINK1-2:1 | SEQ2734 | 3.E-05 | 0.3 |
| msAD | LINC-PINT:33 | SEQ4036 | 1.E-02 | 0.7 | DLB | lnc-PINK1-2:1 | SEQ2734 | 1.E-03 | 0.4 |
| MCI | LINC-PINT:36 | SEQ5836 | 4.E-05 | 0.1 | DLB | lnc-PKHD1-2:3 | SEQ4646 | 1.E-03 | 1.6 |
| FTD | LINC-PINT:39 | SEQ4999 | 8.E-04 | 0.7 | DLB | lnc-PKHD1-6:1 | SEQ4967 | 8.E-04 | 1.6 |
| DLB | LINC-PINT:4 | SEQ5555 | 2.E-04 | 0.2 | msAD | lnc-PKN2-5:1 | SEQ4089 | 1.E-02 | 0.7 |
| miAD | LINC-PINT:46 | SEQ4040 | 2.E-03 | 1.4 | FTD | lnc-PLA2G15-1:2 | SEQ3907 | 1. E-04 | 2.4 |
| All AD | LINC-PINT:46 | SEQ4040 | 7.E-03 | 1.3 | MCI | lnc-PLA2G15-1:2 | SEQ3907 | 2.E-04 | 2.3 |
| FTD | LINC-PINT:70 | SEQ4033 | 3.E-07 | 0.2 | DLB | lnc-PLA2G15-1:2 | SEQ3907 | 2.E-03 | 2.1 |
| MCI | LINC-PINT:70 | SEQ4033 | 4.E-06 | 0.1 | msAD | lnc-PLA2G15-1:2 | SEQ3907 | 3.E-02 | 1.4 |
| msAD | LINC-PINT:70 | SEQ4033 | 2.E-02 | 0.3 | All AD | lnc-PLA2G15-1:2 | SEQ3907 | 5.E-02 | 1.3 |
| All AD | LINC-PINT:70 | SEQ4033 | 2.E-02 | 0.3 | DLB | lnc-PLA2G15-2:2 | SEQ3565 | 2.E-03 | 1.5 |
| FTD | LINC-PINT:71 | sEQ366S | 7.E-06 | 0.6 | DLB | lnc-PLA2G6-1:2 | SEQ2971 | 2.E-04 | 1.6 |
| All AD | LINC-PINT:71 | sEQ366S | 3.E-02 | 0.7 | MCI | lnc-PLA2G6-1:2 | SEQ2971 | 2.E-03 | 1.5 |
| msAD | LINC-PINT:71 | SEQ3665 | 5.E-02 | 0.8 | All AD | lnc-PLAGL2-6:1 | SEQ5429 | 5.E-02 | 0.8 |
| FTD | LINC-PINT:77 | SEQ4909 | 9.E-04 | 0.7 | DLB | lnc-PLCB1-1:7 | SEQ5490 | 7.E-07 | 1.8 |
| DLB | LIPE-AS1:16 | SEQ3727 | 6.E-05 | 2.0 | MCI | lnc-PLCB1-1:7 | sEQS490 | 2.E-04 | 1.6 |
| MCI | LIPE-AS1:16 | SEQ3727 | 9.E-04 | 1.7 | DLB | lnc-PLCD3-4:4 | SEQ4841 | 9.E-04 | 1.4 |
| All AD | LIPE-AS1:16 | SEQ3727 | 3.E-02 | 1.2 | FTD | lnc-PLCD3-4:7 | SEQ5433 | 6.E-05 | 0.5 |
| msAD | LIPE-AS1:16 | SEQ3727 | 4.E-02 | 1.2 | All AD | lnc-PLCD3-4:7 | SEQ5433 | 4.E-02 | 0.6 |
| All AD | LMNB1-DT:3 | SEQ4051 | S.E-03 | 1.3 | FTD | lnc-PLCG1-2:1 | SEQ5645 | 1. E-04 | 0.6 |
| miAD | LMNB1-DT:3 | SEQ4051 | 1.E-02 | 1.3 | FTD | lnc-PLEKHB2-4:3 | SEQ2327 | 1.E-05 | 0.6 |
| msAD | LMNB1-DT:3 | SEQ4051 | 1.E-02 | 1.3 | MCI | lnc-PLEKHB2-4:3 | SEQ2327 | 4.E-05 | 0.6 |
| DLB | lnc-A1BG-1:8 | SEQ4706 | 1.E-03 | 1.5 | FTD | lnc-PLEKHD1-4:1 | SEQ5353 | 4.E-04 | 0.6 |
| FTD | lnc-ABCAS-7:1 | SEQ0014 | 1.E-05 | 0.6 | All AD | lnc-PLEKHD1-4:1 | SEQ5353 | 4.E-02 | 0.7 |
| All AD | lnc-ABCAS-7:1 | SEQ0014 | 5.E-02 | 0.8 | DLB | lnc-PLEKHH2-4:1 | SEQ4483 | 2.E-03 | 1.3 |
| DLB | lnc-ABCCS-3:1 | SEQ3281 | 3.E-04 | 1.5 | DLB | lnc-PLEKHH2-4:2 | SEQ4482 | 2.E-03 | 1.3 |
| FTD | lnc-ABCCS-3:4 | SEQ3282 | 5.E-05 | 1.8 | DLB | lnc-PLPP2-5:5 | SEQ5632 | 2.E-04 | 1.8 |
| MCI | lnc-ABCCS-3:4 | SEQ3282 | 5.E-05 | 1.9 | FTD | lnc-PLPPRS-4:1 | SEQ5684 | 1. E-04 | 0.5 |
| DLB | lnc-ABCC5-3:4 | SEQ3282 | 2.E-03 | 1.7 | FTD | lnc-PLSCR2-2:1 | SEQ0964 | 2.E-05 | 0.5 |
| All AD | lnc-ABCCS-3:4 | SEQ3282 | 2.E-02 | 1.3 | All AD | lnc-PLSCR2-2:1 | SEQ0964 | 7.E-03 | 0.6 |
| DLB | lnc-ABCC6-11:1 | SEQ2717 | 2.E-03 | 1.4 | miAD | lnc-PLSCR2-2:1 | SEQ0964 | 8.E-03 | 0.6 |
| MCI | lnc-ABCF2-2:1 | SEQ5473 | 3.E-06 | 1.6 | msAD | lnc-PLSCR2-2:1 | SEQ0964 | 2.E-02 | 0.6 |
| DLB | lnc-ABCF2-2:1 | SEQ5473 | 2.E-05 | 1.6 | FTD | lnc-PLXND1-1:1 | SEQ5223 | 5. E-04 | 0.5 |
| FTD | lnc-ABCF2-2:1 | SEQ5473 | 3.E-04 | 1.4 | FTD | lnc-PLXND1-3:1 | SEQ5715 | 1. E-04 | 0.5 |
| miAD | lnc-ABHD12B-2:1 | SEQ4060 | 4.E-03 | 0.7 | FTD | lnc-PLXND1-3:2 | SEQ3703 | 3.E-10 | 0.3 |
| All AD | lnc-ABHD12B-2:1 | SEQ4060 | 8.E-03 | 0.7 | MCI | lnc-PLXND1-3:2 | SEQ3703 | 4.E-05 | 0.5 |
| msAD | lnc-ABTB1-2:1 | SEQ3881 | 3.E-02 | 1.4 | All AD | lnc-PLXND1-3:2 | SEQ3703 | 2.E-02 | 0.5 |
| All AD | lnc-ABTBl-2:1 | SEQ3881 | 5.E-02 | 1.3 | msAD | lnc-PLXND1-3:2 | SEQ3703 | 5.E-02 | 0.6 |
| MCI | lnc-ABTB2-4:1 | SEQ4062 | 6.E-06 | 1.6 | DLB | lnc-PMM2-2:1 | SEQ3482 | 6.E-04 | 1.5 |
| DLB | lnc-ABTB2-4:1 | SEQ4062 | 2.E-05 | 1.6 | DLB | lnc-PMM2-6:1 | SEQ4792 | 1.E-03 | 2.0 |
| FTD | lnc-ABTB2-4:1 | SEQ4062 | 1.E-04 | 1.4 | FTD | lnc-PMM2-6:3 | SEQ5443 | 2.E-09 | 0.2 |
| All AD | lnc-ABTB2-4:1 | SEQ4062 | 8.E-03 | 1.2 | DLB | lnc-PMM2-6:3 | SEQ5443 | 1. E-06 | 0.2 |
| msAD | lnc-ABTB2-4:1 | SEQ4062 | 1.E-02 | 1.2 | MCI | lnc-PMM2-6:3 | SEQ5443 | 3.E-06 | 0.2 |
| DLB | lnc-ACBD6-2:1 | SEQ5444 | 3.E-04 | 1.6 | All AD | lnc-PMM2-6:3 | SEQ5443 | 4.E-02 | 0.4 |
| msAD | lnc-ACO1-1:1 | SEQ0590 | 9.E-03 | 0.7 | FTD | lnc-PNMA2-1:1 | SEQ5162 | 5. E-04 | 0.7 |
| All AD | lnc-ACO1-1:1 | SEQ0590 | 1.E-02 | 0.7 | FTD | lnc-PNO1-1:1 | SEQ4348 | 9.E-10 | 0.4 |
| FTD | lnc-ACTA2-1:1 | SEQ5550 | 2.E-04 | 0.6 | MCI | lnc-PNO1-1:1 | SEQ4348 | 5.E-05 | 0.5 |
| DLB | lnc-ACTR2-11:1 | SEQ3921 | 6.E-06 | 1.6 | All AD | lnc-PNO1-1:1 | SEQ4348 | 1.E-03 | 0.6 |
| msAD | lnc-ACTR2-11:1 | SEQ3921 | 3.E-02 | 1.1 | miAD | lnc-PNO1-1:1 | SEQ4348 | 3.E-03 | 0.6 |
| All AD | lnc-ACTR2-11:1 | SEQ3921 | 3.E-02 | 1.1 | msAD | lnc-PNO1-1:1 | SEQ4348 | 5.E-03 | 0.7 |
| All AD | lnc-ACTR3-2:1 | SEQ4067 | 5.E-02 | 0.8 | miAD | lnc-PNOC-2:1 | SEQ2817 | 9.E-03 | 1.3 |
| miAD | lnc-ACTR6-4:1 | SEQ4069 | 8.E-03 | 0.7 | All AD | lnc-PNOC-2:1 | SEQ2817 | 1.E-02 | 1.2 |
| All AD | lnc-ACTR6-4:1 | SEQ4069 | 1.E-02 | 0.7 | FTD | lnc-POCS-4:2 | SEQ5161 | 5. E-04 | 0.7 |
| FTD | lnc-ACTRT3-2:5 | SEQ3699 | 6.E-04 | 1.7 | All AD | lnc-POLD3-2:1 | SEQ5447 | 5.E-02 | 0.9 |
| All AD | lnc-ACTRT3-2:S | SEQ3699 | 2.E-02 | 1.3 | DLB | lnc-POLR2J2-2:1 | SEQ5533 | 2.E-04 | 1.8 |
| msAD | lnc-ACTRT3-2:S | SEQ3699 | 5.E-02 | 1.2 | MCI | lnc-POLR2J2-2:1 | SEQ5533 | 2.E-04 | 1.7 |
| All AD | lnc-ACTRT3-5:1 | SEQ2741 | 5.E-02 | 1.2 | DLB | lnc-POLR3E-3:4 | SEQ3547 | 1.E-03 | 1.5 |
| All AD | lnc-ACY1-2:1 | SEQ4071 | 2.E-02 | 1.2 | DLB | lnc-POLR3GL-10:1 | SEQ4515 | 2.E-03 | 1.5 |
| FTD | lnc-ADAD1-3:1 | SEQ0774 | 9. E-10 | 0.4 | msAD | lnc-POLR3GL-6:4 | SEQ3974 | 2.E-02 | 1.5 |
| MCI | lnc-ADAD1-3:1 | SEQ0774 | 3.E-06 | 0.4 | DLB | lnc-POLR3K-1:7 | SEQ4703 | 1.E-03 | 1.5 |
| miAD | lnc-ADAD1-3:1 | SEQ0774 | 2.E-04 | 0.5 | DLB | lnc-POLR3K-1:9 | SEQ4481 | 2.E-03 | 1.3 |
| All AD | lnc-ADAD1-3:1 | SEQ0774 | 3.E-04 | 0.6 | All AD | lnc-POTEH-1:1 | SEQ4048 | 1.E-02 | 1.2 |
| msAD | lnc-ADAD1-3:1 | SEQ0774 | 3.E-03 | 0.6 | msAD | lnc-POTEH-1:1 | SEQ4048 | 2.E-02 | 1.2 |
| FTD | lnc-ADAM22-2:10 | SEQ4076 | 6.E-05 | 0.5 | FTD | lnc-POU5F2-5:1 | SEQ5608 | 2.E-04 | 0.7 |
| miAD | lnc-ADAM22-2:10 | SEQ4076 | 1.E-03 | 0.6 | DLB | lnc-PPARA-3:11 | SEQ5621 | 2.E-04 | 1.6 |
| All AD | lnc-ADAM22-2:10 | SEQ4076 | 2.E-03 | 0.6 | MCI | lnc-PPARA-3:8 | sEQ28S4 | 2.E-03 | 1.5 |
| msAD | lnc-ADAM22-2:10 | SEQ4076 | 1.E-02 | 0.6 | DLB | lnc-PPARA-4:1 | SEQ4739 | 1.E-03 | 1.4 |
| FTD | lnc-ADAM22-2:13 | SEQ4079 | 3.E-04 | 0.6 | miAD | lnc-PPEF1-7:4 | SEQ4078 | 1.E-02 | 0.8 |
| miAD | lnc-ADAM22-2:13 | SEQ4079 | 2.E-03 | 0.5 | All AD | lnc-PPEF1-7:4 | SEQ4078 | 3.E-02 | 0.8 |
| All AD | lnc-ADAM22-2:13 | SEQ4079 | 2.E-03 | 0.6 | DLB | lnc-PPIAL4D-5:15 | SEQ5493 | 2.E-04 | 1.6 |
| msAD | lnc-ADAM22-2:13 | SEQ4079 | 1.E-02 | 0.6 | MCI | lnc-PPIAL4D-5:4 | SEQ3660 | 7.E-04 | 2.6 |
| miAD | lnc-ADAM30-1:1 | SEQ2698 | 1.E-02 | 0.7 | DLB | lnc-PPIAL4D-5:4 | SEQ3660 | 1.E-03 | 2.1 |
| All AD | lnc-ADAM30-1:1 | SEQ2698 | 2.E-02 | 0.7 | All AD | lnc-PPIAL4D-5:4 | SEQ3660 | 4.E-02 | 1.5 |
| DLB | lnc-ADAMTS12-2:6 | SEQ4081 | 1.E-03 | 1.8 | msAD | lnc-PPIAL4D-5:4 | SEQ3660 | 5.E-02 | 1.5 |
| MCI | lnc-ADAMTS12-2:6 | SEQ4081 | 1.E-03 | 1.7 | miAD | lnc-PPIAL4F-3:2 | SEQ0828 | 5.E-03 | 0.6 |
| All AD | lnc-ADAMTS12-2:6 | SEQ4081 | 3.E-02 | 1.2 | All AD | lnc-PPIAL4F-3:2 | SEQ0828 | 5.E-03 | 0.5 |
| miAD | lnc-ADAMTSL4-4:1 | SEQ3903 | 6.E-03 | 0.6 | msAD | lnc-PPIAL4F-3:2 | SEQ0828 | 2.E-02 | 0.5 |
| All AD | lnc-ADAMTSL4-4:1 | SEQ3903 | 6.E-03 | 0.7 | miAD | lnc-PPIAL4F-3:3 | SEQ3932 | 5.E-03 | 0.6 |
| msAD | lnc-ADAMTSL4-4:1 | SEQ3903 | 3.E-02 | 0.7 | All AD | lnc-PPIAL4F-3:3 | SEQ3932 | 6.E-03 | 0.5 |
| DLB | lnc-ADAMTSL4-7:2 | SEQ4676 | 1.E-03 | 1.3 | msAD | lnc-PPIAL4F-3:3 | SEQ3932 | 3.E-02 | 0.5 |
| miAD | lnc-ADAP2-1:1 | SEQ4086 | 1.E-02 | 0.7 | miAD | lnc-PPIAL4F-6:13 | SEQ4192 | 1.E-02 | 0.8 |
| All AD | lnc-ADAP2-1:1 | SEQ4086 | 3.E-02 | 0.8 | All AD | lnc-PPIAL4F-6:13 | SEQ4192 | 3.E-02 | 0.8 |
| msAD | lnc-ADGRL4-6:1 | SEQ3810 | 4.E-02 | 0.6 | msAD | lnc-PPIAL4G-11:1 | SEQ3641 | 5.E-02 | 0.9 |
| FTD | lnc-ADGRV1-6:1 | SEQ4088 | 1. E-04 | 0.6 | FTD | lnc-PPIAL4G-3:5 | SEQ3650 | 3.E-04 | 0.6 |
| All AD | lnc-ADGRV1-6:1 | SEQ4088 | 4.E-02 | 0.7 | All AD | lnc-PPIAL4G-3:5 | sEQ36S0 | 2.E-02 | 0.7 |
| FTD | lnc-ADGRV1-8:1 | SEQ4091 | 2.E-05 | 0.5 | msAD | lnc-PPIAL4G-3:5 | SEQ3650 | 5.E-02 | 0.8 |
| MCI | lnc-ADGRV1-8:1 | SEQ4091 | 1.E-03 | 0.6 | DLB | lnc-PPIL2-1:4 | SEQ4551 | 2.E-03 | 1.3 |
| All AD | lnc-ADGRV1-8:1 | SEQ4091 | 2.E-02 | 0.7 | DLB | lnc-PPIL4-9:1 | SEQ4781 | 1.E-03 | 1.7 |
| FTD | lnc-ADHS-6:1 | SEQ5409 | 3.E-04 | 0.6 | MCI | lnc-PPP1R10-1:3 | SEQ4826 | 2.E-06 | 0.6 |
| DLB | lnc-ADIPOQ-3:2 | SEQ4093 | 1.E-03 | 1.8 | FTD | lnc-PPP1R10-1:3 | SEQ4826 | 2.E-06 | 0.6 |
| All AD | lnc-ADIPOQ-3:2 | SEQ4093 | 5.E-02 | 1.2 | DLB | lnc-PPP1R10-1:3 | SEQ4826 | 9.E-04 | 0.6 |
| DLB | lnc-ADIPOQ-3:3 | sEQS336 | 4.E-04 | 1.7 | All AD | lnc-PPP1R10-1:3 | SEQ4826 | 2.E-02 | 0.8 |
| miAD | lnc-ADO-2:1 | SEQ4096 | 1.E-02 | 0.7 | DLB | lnc-PPP1R1A-2:1 | SEQ5879 | 3.E-05 | 1.6 |
| All AD | lnc-ADO-2:1 | SEQ4096 | 3.E-02 | 0.8 | DLB | lnc-PPP1R27-2:1 | SEQ4761 | 4.E-05 | 1.6 |
| FTD | lnc-ADRB1-4:1 | SEQ0118 | 1.E-03 | 0.7 | FTD | lnc-PPP1R27-2:1 | SEQ4761 | 3.E-04 | 1.4 |
| miAD | lnc-ADRB2-1:1 | SEQ4099 | 9.E-03 | 0.7 | MCI | lnc-PPP1R27-2:1 | SEQ4761 | 1.E-03 | 1.5 |
| All AD | lnc-ADRB2-1:1 | SEQ4099 | 2.E-02 | 0.8 | All AD | lnc-PPP1R27-2:1 | SEQ4761 | 5.E-02 | 1.1 |
| All AD | lnc-ADSS-4:1 | SEQ3813 | 3.E-02 | 0.8 | DLB | lnc-PPP1R3B-11:4 | SEQ5277 | 5. E-04 | 1.8 |
| msAD | lnc-ADSS-4:1 | SEQ3813 | 4.E-02 | 0.8 | DLB | lnc-PPP4C-2:7 | sEQ3S30 | 2.E-04 | 1.4 |
| DLB | lnc-AEBP1-1:1 | SEQ4975 | 2.E-04 | 1.6 | MCI | lnc-PPP4C-2:7 | sEQ3S30 | 3.E-04 | 1.5 |
| MCI | lnc-AEBP1-1:1 | sEQ497S | 8.E-04 | 1.7 | DLB | lnc-PPP4R1-18:3 | sEQS377 | 3.E-04 | 1.5 |
| DLB | lnc-AES-6:3 | SEQ4772 | 9.E-06 | 1.7 | MCI | lnc-PPP4R1-18:3 | sEQS377 | 3.E-04 | 1.5 |
| MCI | lnc-AES-6:3 | SEQ4772 | 1.E-03 | 1.5 | DLB | lnc-PPPSD1-1:1 | SEQ2313 | 1.E-03 | 1.3 |
| DLB | lnc-AES-8:1 | SEQ4930 | 8.E-04 | 1.4 | DLB | lnc-PPP6R2-5:1 | SEQ2811 | 1.E-05 | 1.7 |
| FTD | lnc-AFG1L-5:1 | SEQ0263 | 1.E-05 | 0.5 | FTD | lnc-PPP6R2-5:1 | SEQ2811 | 1.E-03 | 1.4 |
| FTD | lnc-AFGlL-7:1 | SEQ6011 | 3. E-10 | 0.1 | MCI | lnc-PPP6R2-5:1 | SEQ2811 | 2.E-03 | 1.4 |
| miAD | lnc-AFG1L-8:1 | SEQ4107 | 9.E-03 | 0.8 | All AD | lnc-PQLC1-9:1 | SEQ5467 | 3.E-02 | 1.2 |
| All AD | lnc-AFG1L-8:1 | SEQ4107 | 2.E-02 | 0.8 | DLB | lnc-PQLC2-1:1 | sEQS794 | 6.E-05 | 1.7 |
| DLB | lnc-AGAP2-2:1 | SEQ4693 | 5. E-04 | 1.4 | DLB | lnc-PRAME-7:1 | SEQ4053 | 5.E-05 | 2.4 |
| MCI | lnc-AGAP2-2:1 | SEQ4693 | 1.E-03 | 1.4 | All AD | lnc-PRAME-7:1 | SEQ4053 | 2.E-02 | 1.3 |
| DLB | lnc-AGAPS-6:1 | SEQ5626 | 2.E-04 | 1.7 | msAD | lnc-PRAME-7:1 | SEQ4053 | 2.E-02 | 1.4 |
| FTD | lnc-AGAP5-6:4 | SEQ4803 | 1.E-03 | 0.5 | miAD | lnc-PRCD-2:2 | SEQ4106 | 1.E-02 | 0.6 |
| FTD | lnc-AGBL1-7:1 | SEQ5809 | 6.E-05 | 0.7 | All AD | lnc-PRCD-2:2 | SEQ4106 | 3.E-02 | 0.7 |
| DLB | lnc-AGBL2-1:1 | sEQS126 | 6.E-04 | 1.6 | FTD | lnc-PRDM14-4:1 | SEQ6009 | 2.E-09 | 0.3 |
| FTD | lnc-AGBL3-1:1 | SEQ4046 | 1.E-06 | 0.5 | All AD | lnc-PRDM7-1:2 | SEQ5472 | 5.E-02 | 0.8 |
| MCI | lnc-AGBL3-1:1 | SEQ4046 | 2.E-03 | 0.7 | FTD | lnc-PRDX6-3:1 | SEQ4997 | 8.E-04 | 0.6 |
| All AD | lnc-AGBL3-1:1 | SEQ4046 | 4.E-03 | 0.7 | All AD | lnc-PRDX6-3:1 | SEQ4997 | 4.E-02 | 0.8 |
| miAD | lnc-AGBL3-1:1 | SEQ4046 | 4.E-03 | 0.7 | DLB | lnc-PRELID1-1:1 | SEQ5613 | 2.E-04 | 1.4 |
| msAD | lnc-AGBL3-1:1 | SEQ4046 | 2.E-02 | 0.7 | DLB | lnc-PRICKLE4-1:1 | SEQ5195 | 5. E-04 | 1.6 |
| All AD | lnc-AGO1-1:1 | SEQ2752 | 5.E-02 | 0.8 | MCI | lnc-PRKCQ-3:1 | SEQ4886 | 3.E-04 | 6.5 |
| DLB | lnc-AGO2-2:2 | SEQ0119 | 2.E-04 | 1.6 | DLB | lnc-PRKCQ-3:1 | SEQ4886 | 9.E-04 | 5.0 |
| DLB | lnc-AGRP-1:13 | SEQ4599 | 2.E-03 | 1.8 | FTD | lnc-PRKD3-2:1 | sEQS911 | 2.E-05 | 0.5 |
| MCI | lnc-AGRP-1:8 | SEQ5330 | 9.E-05 | 1.6 | FTD | lnc-PRKN-4:1 | SEQ3854 | 4.E-05 | 0.6 |
| DLB | lnc-AGRP-1:8 | SEQ5330 | 4.E-04 | 1.6 | All AD | lnc-PRKN-4:1 | SEQ3854 | 1.E-02 | 0.8 |
| DLB | Inc-AHNAK2-7:1 | SEQ5698 | 1. E-04 | 1.6 | msAD | Inc-PRKN-4:1 | sEQ38S4 | 3.E-02 | 0.8 |
| FTD | Inc-AHSA2-2:1 | SEQ5800 | 6.E-05 | 0.6 | FTD | Inc-PRLHR-2:1 | sEQ363S | 3.E-07 | 0.6 |
| FTD | Inc-AHSA2-5:6 | SEQ3332 | 2.E-09 | 3.E-02 | miAD | Inc-PRLHR-2:1 | SEQ.3635 | 1.E-02 | 0.7 |
| MCI | Inc-AHSA2-5:6 | SEQ3332 | 9.E-06 | 2.E-02 | All AD | Inc-PRLHR-2:1 | SEQ3635 | 1.E-02 | 0.7 |
| DLB | Inc-AHSA2-5:6 | SEQ3332 | 2.E-04 | 3.E-02 | msAD | Inc-PRLHR-2:1 | SEQ3635 | 5.E-02 | 0.8 |
| FTD | Inc-AIDA-2:1 | SEQ4124 | 3.E-04 | 0.7 | FTD | Inc-PRPF4B-1:1 | SEQ5654 | 5.E-07 | 0.5 |
| miAD | Inc-AIDA-2:1 | SEQ4124 | 8.E-03 | 0.8 | MCI | Inc-PRPF4B-1:1 | SEQ5654 | 1. E-04 | 0.6 |
| All AD | Inc-AIDA-2:1 | SEQ4124 | 2.E-02 | 0.8 | DLB | Inc-PRR14-1:7 | SEQ5531 | 2.E-04 | 1.6 |
| All AD | Inc-AIM2-3:2 | SEQ4125 | 5.E-02 | 0.8 | DLB | Inc-PRRG2-1:1 | SEQ4499 | 8.E-04 | 1.5 |
| FTD | Inc-AIM2-3:3 | SEQ3333 | 7.E-05 | 0.6 | MCI | Inc-PRRG2-1:1 | SEQ4499 | 2.E-03 | 1.4 |
| All AD | Inc-AIM2-3:3 | SEQ3333 | 4.E-03 | 0.7 | DLB | Inc-PRSS2-5:1 | SEQ5208 | 3.E-04 | 1.9 |
| miAD | Inc-AIM2-3:3 | SEQ3333 | 5.E-03 | 0.7 | MCI | Inc-PRSS2-5:1 | SEQ5208 | 5. E-04 | 1.8 |
| msAD | Inc-AIM2-3:3 | SEQ3333 | 2.E-02 | 0.7 | All AD | Inc-PRSS2-5:1 | SEQ5208 | 3.E-02 | 1.2 |
| All AD | Inc-AIM2-3:4 | SEQ4128 | 4.E-02 | 0.7 | DLB | Inc-PRSS27-2:20 | SEQ5557 | 2.E-04 | 1.3 |
| FTD | Inc-AIM2-3:5 | SEQ4130 | 5.E-05 | 0.6 | DLB | Inc-PRSS27-4:12 | SEQ5703 | 1. E-04 | 1.7 |
| All AD | Inc-AIM2-3:5 | SEQ4130 | 2.E-03 | 0.7 | DLB | Inc-PRSS27-4:14 | SEQ5216 | 5.E-04 | 2.0 |
| miAD | Inc-AIM2-3:5 | SEQ4130 | 2.E-03 | 0.7 | FTD | Inc-PRSS27-4:24 | SEQ2168 | 8.E-09 | 0.1 |
| msAD | Inc-AIM2-3:5 | SEQ4130 | 9.E-03 | 0.7 | MCI | Inc-PRSS27-4:24 | SEQ2168 | 3.E-08 | 0.1 |
| All AD | Inc-AK4-2:1 | SEQ3741 | 2.E-02 | 0.7 | DLB | Inc-PRSS27-4:24 | SEQ2168 | 4.E-04 | 0.1 |
| msAD | Inc-AK4-2:1 | SEQ3741 | 4.E-02 | 0.8 | DLB | Inc-PRSS27-4:6 | SEQ4532 | 2.E-03 | 1.9 |
| FTD | Inc-AK7-6:1 | SEQ4134 | 2.E-06 | 0.5 | DLB | Inc-PSMA7-1:1 | SEQ5951 | 7.E-06 | 1.9 |
| MCI | Inc-AK7-6:1 | SEQ4134 | 9.E-04 | 0.6 | DLB | Inc-PSMB1-1:1 | SEQ4416 | 2.E-03 | 1.4 |
| miAD | Inc-AK7-6:1 | SEQ4134 | 4.E-03 | 0.7 | DLB | Inc-PSMB7-2:1 | SEQ5622 | 2.E-04 | 1.6 |
| All AD | Inc-AK7-6:1 | SEQ4134 | 2.E-02 | 0.7 | DLB | Inc-PSMB9-6:3 | SEQ5503 | 2.E-04 | 2.9 |
| FTD | Inc-AKAP11-1:1 | SEQ5677 | 1. E-04 | 0.4 | DLB | Inc-PSMC3-1:1 | SEQ4929 | 2.E-04 | 1.5 |
| All AD | Inc-AKIRIN1-1:10 | SEQ4135 | 3.E-02 | 0.8 | MCI | Inc-PSMC3-1:1 | SEQ4929 | 8.E-04 | 1.4 |
| All AD | Inc-AKNAD1-3:1 | SEQ4136 | 2.E-02 | 0.8 | DLB | Inc-PSMC3IP-1:1 | sEQ3S99 | 3.E-04 | 1.3 |
| FTD | Inc-AKR1D1-5:2 | SEQ0098 | 3.E-04 | 0.6 | MCI | Inc-PSMC3IP-3:1 | sEQ37S4 | 8.E-05 | 1.7 |
| DLB | Inc-AKR7A2-2:1 | SEQ0145 | 1.E-03 | 1.4 | FTD | Inc-PSMC3IP-3:1 | sEQ37S4 | 9.E-04 | 1.5 |
| FTD | Inc-AKTIP-3:1 | SEQ4139 | 1.E-05 | 0.6 | DLB | Inc-PSMC3IP-3:1 | sEQ37S4 | 1.E-03 | 1.6 |
| All AD | Inc-AKTIP-3:1 | SEQ4139 | 3.E-02 | 0.8 | All AD | Inc-PSMC3IP-3:1 | sEQ37S4 | 2.E-02 | 1.2 |
| FTD | Inc-ALB-7:2 | SEQ4141 | 1.E-07 | 0.4 | msAD | Inc-PSMC3IP-3:1 | sEQ37S4 | 4.E-02 | 1.2 |
| MCI | Inc-ALB-7:2 | SEQ4141 | 2.E-04 | 0.5 | DLB | Inc-PSMC3IP-3:11 | SEQ4960 | 2.E-04 | 1.6 |
| miAD | Inc-ALB-7:2 | SEQ4141 | 7.E-03 | 0.6 | MCI | Inc-PSMC3IP-3:11 | SEQ4960 | 8.E-04 | 1.6 |
| All AD | Inc-ALB-7:2 | SEQ4141 | 2.E-02 | 0.7 | DLB | Inc-PSMC3IP-3:13 | SEQ3726 | 5. E-04 | 1.7 |
| MCI | Inc-ALDH3B2-1:1 | SEQ5648 | 1. E-04 | 2.4 | MCI | Inc-PSMC3IP-3:13 | SEQ3726 | 1.E-03 | 1.5 |
| FTD | Inc-ALDH3B2-1:1 | SEQ5648 | 1. E-04 | 1.8 | msAD | Inc-PSMC3IP-3:13 | SEQ3726 | 4.E-02 | 1.2 |
| DLB | Inc-ALG12-5:3 | SEQ3016 | 2.E-03 | 1.4 | All AD | Inc-PSMC3IP-3:13 | SEQ3726 | 5.E-02 | 1.2 |
| All AD | Inc-ALPK1-2:3 | SEQ4144 | 4.E-02 | 0.7 | FTD | Inc-PSMC3IP-3:6 | SEQ3982 | 2.E-04 | 1.6 |
| FTD | Inc-ALPK1-2:4 | SEQ4148 | 1.E-08 | 0.4 | MCI | Inc-PSMC3IP-3:6 | SEQ3982 | 2.E-04 | 1.6 |
| MCI | Inc-ALPK1-2:4 | SEQ4148 | 3.E-05 | 0.4 | DLB | Inc-PSMC3IP-3:6 | SEQ3982 | 1.E-03 | 1.6 |
| miAD | Inc-ALPK1-2:4 | SEQ4148 | 3.E-04 | 0.5 | All AD | Inc-PSMC3IP-3:6 | SEQ3982 | 6.E-03 | 1.3 |
| DLB | Inc-ALPK1-2:4 | SEQ4148 | 4.E-04 | 0.5 | miAD | Inc-PSMC3IP-3:6 | SEQ3982 | 8.E-03 | 1.3 |
| All AD | Inc-ALPK1-2:4 | SEQ4148 | 5.E-04 | 0.5 | msAD | Inc-PSMC3IP-3:6 | SEQ3982 | 2.E-02 | 1.2 |
| msAD | Inc-ALPK1-2:4 | SEQ4148 | 5.E-03 | 0.6 | FTD | Inc-PSMC6-1:1 | SEQ5982 | 2.E-06 | 0.4 |
| FTD | Inc-AMDHD1-1:2 | SEQ4073 | 3.E-05 | 0.6 | FTD | Inc-PSMD6-1:1 | SEQ5926 | 1.E-05 | 0.6 |
| MCI | Inc-AMDHD1-1:2 | SEQ4073 | 2.E-04 | 0.6 | DLB | Inc-PSMD7-2:1 | SEQ4720 | 1. E-03 | 1.8 |
| miAD | Inc-AMDHD1-1:2 | SEQ4073 | 1.E-02 | 0.7 | DLB | Inc-PSMG4-1:1 | SEQ3724 | 7.E-04 | 1.5 |
| All AD | Inc-AMDHD1-1:2 | SEQ4073 | 3.E-02 | 0.8 | All AD | Inc-PSMG4-1:1 | SEQ3724 | 2.E-02 | 1.2 |
| FTD | Inc-AMY1B-1:10 | SEQ5686 | 1. E-04 | 0.6 | msAD | Inc-PSMG4-1:1 | SEQ3724 | 4.E-02 | 1.2 |
| FTD | Inc-AMZ2-5:5 | SEQ4154 | 1. E-04 | 0.4 | FTD | Inc-PTBP2-11:1 | SEQ3669 | 7.E-06 | 0.6 |
| MCI | Inc-AMZ2-5:5 | SEQ4154 | 2.E-04 | 0.5 | All AD | Inc-PTBP2-11:1 | SEQ3669 | 3.E-02 | 0.8 |
| miAD | Inc-AMZ2-5:5 | SEQ4154 | 7.E-03 | 0.6 | msAD | Inc-PTBP2-11:1 | SEQ3669 | 5.E-02 | 0.8 |
| All AD | Inc-AMZ2-5:5 | SEQ4154 | 2.E-02 | 0.7 | DLB | Inc-PTBP3-7:1 | SEQ2703 | 5. E-04 | 1.3 |
| MCI | Inc-AMZ2-8:1 | SEQ4396 | 2.E-03 | 0.5 | FTD | Inc-PTP4A2-3:4 | SEQ5007 | 8.E-04 | 2.9 |
| All AD | Inc-ANAPC11-2:11 | SEQ3746 | 2.E-02 | 1.2 | FTD | Inc-PTP4A2-3:6 | SEQ5107 | 8.E-05 | 0.6 |
| msAD | Inc-ANAPC11-2:11 | SEQ3746 | 4.E-02 | 1.2 | MCI | Inc-PTP4A2-3:6 | SEQ5107 | 6.E-04 | 0.7 |
| MCI | Inc-ANAPC11-2:2 | SEQ3335 | 1.E-05 | 2.0 | DLB | Inc-PTPMT1-2:1 | SEQ2354 | 5.E-05 | 1.5 |
| FTD | Inc-ANAPC11-2:2 | SEQ3335 | 1.E-05 | 1.8 | DLB | Inc-PTPN23-1:2 | SEQ4934 | 8.E-04 | 1.4 |
| DLB | Inc-ANAPC11-2:2 | SEQ3335 | 3.E-05 | 2.1 | FTD | Inc-PUM3-2:1 | SEQ5291 | 4.E-04 | 0.6 |
| All AD | Inc-ANAPC11-2:2 | SEQ3335 | 2.E-03 | 1.3 | FTD | Inc-PUM3-7:1 | SEQ2337 | 8.E-04 | 0.6 |
| miAD | Inc-ANAPC11-2:2 | SEQ3335 | 3.E-03 | 1.3 | FTD | Inc-PUS7-2:1 | SEQ3909 | 4.E-08 | 0.5 |
| msAD | Inc-ANAPC11-2:2 | SEQ3335 | 9.E-03 | 1.2 | miAD | Inc-PUS7-2:1 | SEQ3909 | 1. E-03 | 0.6 |
| DLB | Inc-ANAPC11-2:8 | SEQ3283 | 1.E-05 | 1.5 | All AD | Inc-PUS7-2:1 | SEQ3909 | 3.E-03 | 0.7 |
| MCI | Inc-ANAPC11-2:8 | SEQ3283 | 4.E-05 | 1.4 | msAD | Inc-PUS7-2:1 | SEQ3909 | 3.E-02 | 0.7 |
| DLB | Inc-ANGPTL1-2:1 | SEQ3336 | 5.E-05 | 1.8 | FTD | Inc-PUS7-3:1 | SEQ6014 | 3.E-11 | 0.2 |
| FTD | Inc-ANKFY1-5:1 | SEQ5997 | 4.E-07 | 0.6 | DLB | Inc-PXDC1-12:3 | SEQ4641 | 1.E-03 | 1.5 |
| MCI | Inc-ANKH-1:8 | SEQ5053 | 3.E-04 | 2.3 | All AD | Inc-PXDC1-12:3 | SEQ4641 | 5.E-02 | 1.2 |
| FTD | Inc-ANKH-1:8 | SEQ5053 | 6.E-04 | 1.9 | DLB | Inc-PYCARD-1:2 | SEQ4964 | 8.E-04 | 1.6 |
| DLB | Inc-ANKH-1:8 | SEQ5053 | 7.E-04 | 2.6 | DLB | Inc-QPRT-3:1 | SEQ5329 | 4.E-04 | 1.6 |
| DLB | Inc-ANKRD20A2-22:3 | SEQ3338 | 1.E-05 | 1.7 | MCI | Inc-RAB1A-6:1 | SEQ5185 | 2.E-04 | 1.5 |
| FTD | Inc-ANKRD20A2-22:3 | SEQ3338 | 6.E-04 | 1.4 | DLB | Inc-RAB1A-6:1 | SEQ5185 | 5. E-04 | 1.5 |
| All AD | Inc-ANKRD20A2-22:3 | SEQ3338 | 5.E-02 | 1.1 | DLB | Inc-RAB23-20:13 | SEQ4851 | 9.E-04 | 1.5 |
| miAD | lnc-ANKRD20A4-5:4 | SEQ4167 | 7.E-03 | 0.7 | FTD | Inc-RAB2B-2:2 | SEQ5644 | 1. E-04 | 0.6 |
| All AD | lnc-ANKRD20A4-5:4 | SEQ4167 | l.E-02 | 0.7 | DLB | Inc-RAB37-1:5 | SEQ5380 | 3.E-04 | 1.6 |
| miAD | Inc-ANKRD27-9:1 | SEQ4169 | 6.E-03 | 0.7 | DLB | Inc-RAB3A-5:1 | SEQ5567 | 2.E-04 | 1.6 |
| All AD | Inc-ANKRD27-9:1 | SEQ4169 | 4.E-02 | 0.8 | MCI | Inc-RABEP2-4:1 | SEQ5391 | 3.E-04 | 1.8 |
| msAD | Inc-ANKRD33B-2:1 | SEQ3825 | 4.E-02 | 1.4 | DLB | Inc-RABEP2-7:1 | SEQ5341 | 2.E-05 | 2.1 |
| FTD | Inc-ANKRD34B-2:12 | SEQ4174 | 8.E-05 | 0.6 | MCI | Inc-RABEP2-7:1 | SEQ5341 | 4.E-04 | 2.0 |
| MCI | Inc-ANKRD34B-2:12 | SEQ4174 | 9.E-04 | 0.6 | DLB | Inc-RABEP2-7:2 | SEQ3172 | 7.E-04 | 1.7 |
| miAD | Inc-ANKRD34B-2:12 | SEQ4174 | 5.E-03 | 0.7 | MCI | Inc-RACK1-1:2 | SEQ3580 | 9.E-06 | 1.6 |
| All AD | Inc-ANKRD34B-2:12 | SEQ4174 | 2.E-02 | 0.7 | DLB | Inc-RACK1-1:2 | SEQ3580 | 2.E-05 | 1.6 |
| FTD | Inc-ANKRD34B-2:13 | SEQ4176 | 8.E-05 | 0.6 | DLB | Inc-RADIL-1:1 | SEQ5262 | 9.E-05 | 1.6 |
| MCI | Inc-ANKRD34B-2:13 | SEQ4176 | 1.E-03 | 0.6 | MCI | Inc-RADIL-1:1 | SEQ5262 | 5. E-04 | 1.5 |
| miAD | Inc-ANKRD34B-2:13 | SEQ4176 | 5.E-03 | 0.7 | All AD | Inc-RAG1-4:1 | SEQ5504 | 4.E-02 | 0.8 |
| All AD | Inc-ANKRD34B-2:13 | SEQ4176 | 2.E-02 | 0.7 | DLB | Inc-RAI1-2:2 | SEQ5573 | 2.E-04 | 1.6 |
| FTD | Inc-ANKRD34B-2:7 | SEQ4179 | 1.E-04 | 0.5 | All AD | Inc-RAMP3-1:1 | SEQ5506 | 2.E-02 | 0.7 |
| miAD | Inc-ANKRD34B-2:7 | SEQ4179 | 7.E-04 | 0.6 | DLB | Inc-RANBP9-1:3 | SEQ4092 | 2.E-04 | 1.4 |
| All AD | Inc-ANKRD34B-2:7 | SEQ4179 | 1.E-02 | 0.7 | All AD | Inc-RANBP9-1:3 | SEQ4092 | 6.E-03 | 1.2 |
| FTD | Inc-ANKRD34B-2:8 | SEQ4181 | 3.E-04 | 0.4 | miAD | Inc-RANBP9-1:3 | SEQ4092 | 1. E-02 | 1.2 |
| MCI | Inc-ANKRD34B-2:8 | SEQ4181 | 1.E-03 | 0.5 | msAD | Inc-RANBP9-1:3 | SEQ4092 | 1. E-02 | 1.2 |
| All AD | Inc-ANKRD34B-2:8 | SEQ4181 | 4.E-02 | 0.6 | FTD | Inc-RAP2C-4:1 | SEQ4377 | 1. E-06 | 0.5 |
| miAD | Inc-ANKRD34B-4:1 | SEQ2896 | 4.E-03 | 1.2 | MCI | Inc-RAP2C-4:1 | SEQ4377 | 2.E-04 | 0.5 |
| All AD | Inc-ANKRD34B-4:1 | SEQ2896 | 5.E-03 | 1.2 | miAD | Inc-RAP2C-4:1 | SEQ4377 | 3.E-03 | 0.6 |
| msAD | Inc-ANKRD34B-4:1 | SEQ2896 | 2.E-02 | 1.2 | All AD | Inc-RAP2C-4:1 | SEQ4377 | 8.E-03 | 0.7 |
| All AD | Inc-ANKRD36-1:4 | SEQ4183 | 4.E-03 | 1.5 | DLB | Inc-RAPSN-2:1 | SEQ4610 | 1. E-03 | 1.3 |
| msAD | Inc-ANKRD36-1:4 | SEQ4183 | 8.E-03 | 1.5 | FTD | Inc-RARB-4:1 | SEQ5210 | 2.E-04 | 1.6 |
| miAD | Inc-ANKRD36-1:4 | SEQ4183 | 1.E-02 | 1.5 | MCI | Inc-RARB-4:1 | SEQ5210 | 3.E-04 | 2.0 |
| DLB | Inc-ANKRD39-1:1 | SEQ4491 | 2.E-03 | 1.4 | DLB | Inc-RARB-4:1 | SEQ5210 | 5. E-04 | 1.8 |
| FTD | Inc-ANKS1B-1:1 | SEQ3860 | 1.E-07 | 0.5 | MCI | Inc-RARRES2-2:2 | SEQ2774 | 4.E-06 | 1.6 |
| MCI | Inc-ANKS1B-1:1 | SEQ3860 | 6.E-05 | 0.6 | DLB | Inc-RARRES2-2:2 | SEQ2774 | 9.E-06 | 1.6 |
| All AD | Inc-ANKS1B-1:1 | SEQ3860 | 1.E-02 | 0.7 | FTD | Inc-RARRES2-2:2 | SEQ2774 | 6.E-05 | 1.4 |
| msAD | Inc-ANKS1B-1:1 | SEQ3860 | 3.E-02 | 0.8 | All AD | Inc-RARRES2-2:2 | SEQ2774 | 3.E-03 | 1.2 |
| DLB | Inc-ANKS3-2:1 | SEQ2771 | 2.E-03 | 1.5 | miAD | Inc-RARRES2-2:2 | SEQ2774 | 5.E-03 | 1.2 |
| All AD | Inc-ANLN-6:1 | SEQ4186 | 4.E-02 | 0.8 | msAD | Inc-RARRES2-2:2 | SEQ2774 | 8.E-03 | 1.2 |
| DLB | Inc-ANP32E-1:1 | SEQ4400 | 2.E-03 | 1.2 | DLB | Inc-RARRES2-5:1 | SEQ5383 | 2.E-04 | 1.7 |
| FTD | Inc-ANTXR2-1:8 | SEQ5547 | 2.E-04 | 0.6 | MCI | Inc-RARRES2-5:1 | SEQ5383 | 3.E-04 | 1.6 |
| FTD | Inc-ANXA3-7:1 | SEQ5781 | 7.E-05 | 0.6 | All AD | Inc-RARS2-4:1 | SEQ3642 | 3.E-02 | 0.8 |
| FTD | Inc-ANXA3-8:1 | SEQ0670 | 1. E-06 | 0.6 | msAD | Inc-RARS2-4:1 | SEQ3642 | 5.E-02 | 0.9 |
| MCI | Inc-ANXA3-8:1 | SEQ0670 | 1. E-04 | 0.6 | FTD | Inc-RASA2-1:1 | SEQ5294 | 4.E-04 | 0.7 |
| All AD | Inc-ANXA3-8:1 | SEQ0670 | 4.E-02 | 0.8 | miAD | Inc-RASSF3-1:1 | SEQ4155 | 1. E-02 | 0.7 |
| FTD | Inc-AP1AR-1:1 | SEQ4189 | 4.E-08 | 0.5 | All AD | Inc-RASSF3-1:1 | SEQ4155 | 2.E-02 | 0.8 |
| miAD | Inc-AP1AR-1:1 | SEQ4189 | 4.E-04 | 0.5 | DLB | Inc-RBAK-RBAKDN-2:13 | SEQ5129 | 6.E-04 | 1.6 |
| All AD | Inc-AP1AR-1:1 | SEQ4189 | 8.E-04 | 0.6 | MCI | Inc-RBBP8NL-2:1 | SEQ5128 | 6.E-04 | 1.6 |
| MCI | Inc-AP1AR-1:1 | SEQ4189 | 2.E-03 | 0.6 | FTD | Inc-RBL2-1:10 | SEQ4900 | 9.E-04 | 0.6 |
| msAD | Inc-AP1AR-1:1 | SEQ4189 | 9.E-03 | 0.6 | FTD | Inc-RBL2-1:11 | SEQ5587 | 2.E-04 | 0.5 |
| FTD | Inc-AP3S1-4:3 | SEQ2424 | 1.E-08 | 0.3 | MCI | Inc-RBL2-1:3 | SEQ4730 | 4.E-04 | 3.5 |
| MCI | Inc-AP3S1-4:3 | SEQ2424 | 1. E-04 | 0.4 | DLB | Inc-RBL2-1:3 | SEQ4730 | 1. E-03 | 2.7 |
| MCI | Inc-APAF1-3:1 | SEQ5059 | 2.E-05 | 0.4 | FTD | Inc-RBM12B-4:2 | SEQ5071 | 7.E-04 | 0.7 |
| FTD | Inc-APAF1-3:1 | SEQ5059 | 7.E-04 | 0.6 | MCI | Inc-RBM23-3:1 | SEQ4562 | 3.E-05 | 1.6 |
| miAD | Inc-APBB2-3:1 | SEQ4195 | 8.E-03 | 0.6 | DLB | Inc-RBM23-3:1 | SEQ4562 | 2.E-03 | 1.4 |
| All AD | Inc-APBB2-3:1 | SEQ4195 | 1.E-02 | 0.7 | DLB | Inc-RBM28-3:2 | SEQ5532 | 2.E-04 | 1.7 |
| DLB | Inc-APCDD1L-3:2 | SEQ5727 | 9.E-05 | 1.4 | FTD | Inc-RC3H1-2:1 | SEQ4355 | 6.E-08 | 0.5 |
| FTD | Inc-APLNR-3:1 | SEQ5227 | 5. E-04 | 0.6 | miAD | Inc-RC3H1-2:1 | SEQ4355 | 4.E-03 | 0.7 |
| All AD | Inc-APOBEC3A-2:1 | SEQ3666 | 2.E-02 | 0.8 | All AD | Inc-RC3H1-2:1 | SEQ4355 | 1. E-02 | 0.7 |
| msAD | Inc-APOBEC3A-2:1 | SEQ3666 | 5.E-02 | 0.8 | All AD | Inc-RC3H2-1:1 | SEQ5518 | 4.E-02 | 0.8 |
| DLB | Inc-APOBEC3H-3:1 | SEQ5259 | 5. E-04 | 1.5 | FTD | Inc-RCC1L-3:1 | SEQ3975 | 7.E-07 | 0.5 |
| DLB | Inc-APOC3-2:7 | SEQ4685 | 1.E-03 | 1.4 | MCI | Inc-RCC1L-3:1 | SEQ3975 | 2.E-05 | 0.6 |
| DLB | Inc-APOL2-5:1 | SEQ5264 | 5. E-04 | 1.6 | All AD | Inc-RCC1L-3:1 | SEQ3975 | 2.E-02 | 0.7 |
| msAD | Inc-APOL4-1:5 | SEQ4202 | 8.E-03 | 0.8 | msAD | Inc-RCC1L-3:1 | SEQ3975 | 2.E-02 | 0.7 |
| All AD | Inc-APOL4-1:5 | SEQ4202 | 1.E-02 | 0.8 | All AD | Inc-RCSD1-4:1 | SEQ0831 | 5.E-02 | 0.8 |
| MCI | Inc-APOLS-4:1 | SEQ2343 | 2.E-05 | 0.6 | FTD | Inc-RDH10-3:1 | SEQ5869 | 4.E-05 | 0.6 |
| FTD | Inc-APOLS-4:1 | SEQ2343 | 4.E-05 | 0.6 | DLB | Inc-RDH13-1:2 | SEQ0250 | 1. E-03 | 1.5 |
| All AD | Inc-APOLS-4:1 | SEQ2343 | 2.E-03 | 0.7 | DLB | Inc-REC8-2:1 | SEQ4479 | 9.E-06 | 1.5 |
| msAD | Inc-APOL5-4:1 | SEQ2343 | 3.E-03 | 0.6 | MCI | Inc-REC8-2:1 | SEQ4479 | 2.E-03 | 1.3 |
| miAD | Inc-APOLS-4:1 | SEQ2343 | 6.E-03 | 0.7 | FTD | Inc-REL-1:3 | SEQ2508 | 2.E-05 | 0.6 |
| All AD | Inc-AQP8-2:1 | SEQ3756 | 3.E-02 | 1.4 | FTD | Inc-REL-6:3 | SEQ3461 | 1. E-04 | 0.7 |
| msAD | Inc-AQP8-2:1 | SEQ3756 | 4.E-02 | 1.5 | All AD | Inc-REL-6:3 | SEQ3461 | 5.E-02 | 0.8 |
| DLB | Inc-AQP8-2:5 | SEQ4835 | 9.E-04 | 1.4 | msAD | Inc-REXOS-1:2 | SEQ4665 | 1. E-03 | 2.0 |
| DLB | Inc-ARAF-1:1 | SEQ5459 | 2.E-04 | 1.9 | All AD | Inc-REXOS-1:2 | SEQ4665 | 5.E-03 | 1.7 |
| MCI | Inc-ARAF-1:1 | SEQ5459 | 3.E-04 | 2.0 | MCI | Inc-RFNG-1:7 | SEQ2768 | 3.E-05 | 1.5 |
| DLB | Inc-ARF5-7:1 | SEQ4756 | 1.E-05 | 1.6 | DLB | Inc-RFNG-1:7 | SEQ2768 | 4.E-05 | 1.4 |
| MCI | Inc-ARFS-7:1 | SEQ4756 | 1.E-03 | 1.4 | miAD | Inc-RFNG-1:7 | SEQ2768 | 6.E-03 | 1.2 |
| MCI | Inc-ARF6-1:1 | SEQ4542 | 3.E-05 | 2.E-04 | All AD | Inc-RFNG-1:7 | SEQ2768 | 2.E-02 | 1.1 |
| DLB | Inc-ARF6-1:1 | SEQ4542 | 2.E-03 | 2.E-04 | msAD | Inc-RFT1-3:1 | SEQ4061 | 2.E-02 | 1.5 |
| All AD | Inc-ARHGAP10-5:2 | SEQ3781 | 2.E-02 | 1.3 | All AD | Inc-RFT1-3:1 | SEQ4061 | 3.E-02 | 1.3 |
| msAD | Inc-ARHGAP10-5:2 | SEQ3781 | 4.E-02 | 1.3 | FTD | Inc-RGPD2-2:1 | SEQ5354 | 4.E-04 | 0.6 |
| MCI | Inc-ARHGEF1-5:1 | SEQ4210 | 1.E-05 | 1.6 | FTD | Inc-RGS6-3:1 | SEQ5974 | 3.E-06 | 0.6 |
| FTD | Inc-ARHGEF1-5:1 | SEQ4210 | 4.E-05 | 1.5 | FTD | Inc-RGS9-15:4 | SEQ4117 | 1. E-03 | 0.7 |
| DLB | Inc-ARHGEF1-5:1 | SEQ4210 | 2.E-04 | 1.5 | All AD | Inc-RGS9-15:4 | SEQ4117 | 1. E-02 | 0.8 |
| All AD | Inc-ARHGEF1-5:1 | SEQ4210 | 4.E-02 | 1.1 | miAD | Inc-RGS9-15:4 | SEQ4117 | 1. E-02 | 0.8 |
| FTD | Inc-ARHGEF39-1:1 | SEQ3834 | 6.E-05 | 1.5 | FTD | Inc-RICTOR-2:1 | SEQ5065 | 7.E-04 | 0.7 |
| All AD | Inc-ARHGEF39-1:1 | SEQ3834 | 3.E-02 | 1.2 | MCI | Inc-RIMBP3C-3:1 | SEQ5754 | 8.E-05 | 1.7 |
| msAD | Inc-ARHGEF39-1:1 | SEQ3834 | 3.E-02 | 1.2 | DLB | Inc-RIMBP3C-5:1 | SEQ4637 | 3.E-04 | 1.7 |
| FTD | Inc-ARHGEF39-1:3 | SEQ3718 | 2.E-04 | 1.5 | MCI | Inc-RIMBP3C-5:1 | SEQ4637 | 1. E-03 | 1.5 |
| All AD | Inc-ARHGEF39-1:3 | SEQ3718 | 4.E-02 | 1.2 | MCI | Inc-RIMKLB-6:5 | SEQ4663 | 1. E-03 | 3.4 |
| msAD | Inc-ARHGEF39-1:3 | SEQ3718 | 4.E-02 | 1.2 | msAD | Inc-RIPOR1-1:5 | SEQ3647 | 5.E-02 | 1.2 |
| MCI | Inc-ARID2-8:1 | SEQ3792 | 1.E-03 | 1.7 | DLB | Inc-RIPOR1-1:6 | SEQ2943 | 2.E-03 | 1.4 |
| DLB | Inc-ARID2-8:1 | SEQ3792 | 2.E-03 | 1.7 | DLB | Inc-RIPOR1-1:7 | SEQ3158 | 5. E-04 | 1.5 |
| All AD | Inc-ARID2-8:1 | SEQ3792 | 1.E-02 | 1.3 | DLB | Inc-RIPOR1-1:8 | SEQ4771 | 1. E-03 | 1.5 |
| miAD | Inc-ARID2-8:1 | SEQ3792 | 1.E-02 | 1.3 | DLB | Inc-RIPOR2-7:1 | SEQ3466 | 2.E-03 | 1.5 |
| msAD | Inc-ARID2-8:1 | SEQ3792 | 4.E-02 | 1.3 | DLB | Inc-RIPOR2-7:2 | SEQ4506 | 2.E-03 | 1.5 |
| MCI | Inc-ARID3B-4:1 | SEQ4216 | 3.E-04 | 1.6 | DLB | Inc-RIPOR3-1:1 | SEQ4925 | 8.E-04 | 1.4 |
| All AD | Inc-ARID3B-4:1 | SEQ4216 | 4.E-02 | 1.1 | DLB | Inc-RLBP1-3:1 | SEQ4750 | 1. E-03 | 1.4 |
| DLB | Inc-ARIDSA-2:1 | SEQ4523 | 2.E-04 | 1.7 | DLB | Inc-RLIM-4:1 | SEQ3579 | 1.E-03 | 1.5 |
| MCI | Inc-ARID5A-2:1 | SEQ4523 | 2.E-03 | 1.6 | All AD | Inc-RMDN2-3:24 | SEQ2468 | 3.E-02 | 0.8 |
| MCI | Inc-ARL14-2:3 | SEQ4782 | 1.E-03 | 1.7 | All AD | Inc-RM12-3:1 | SEQ5534 | 4.E-02 | 0.7 |
| DLB | Inc-ARL16-1:1 | SEQ5616 | 2.E-04 | 1.4 | MCI | Inc-RNASET2-1:4 | SEQ4516 | 2.E-04 | 1.5 |
| DLB | Inc-ARL16-1:2 | SEQ4559 | 1. E-04 | 1.5 | DLB | Inc-RNASET2-1:4 | SEQ4516 | 2.E-03 | 1.5 |
| MCI | Inc-ARL16-1:2 | SEQ4559 | 2.E-03 | 1.4 | FTD | Inc-RNASET2-1:5 | SEQ5545 | 2.E-04 | 0.6 |
| FTD | Inc-ARL5A-1:1 | SEQ5745 | 9.E-05 | 0.6 | FTD | Inc-RNASET2-1:9 | SEQ4282 | 4.E-08 | 0.3 |
| DLB | Inc-ARMC6-1:1 | SEQ4624 | 1.E-03 | 1.4 | MCI | Inc-RNASET2-1:9 | SEQ4282 | 1. E-03 | 0.5 |
| DLB | Inc-ARMC6-1:2 | SEQ4854 | 9.E-04 | 1.5 | miAD | Inc-RNASET2-1:9 | SEQ4282 | 7.E-03 | 0.5 |
| DLB | Inc-ARMC7-1:1 | SEQ5527 | 2.E-04 | 1.5 | All AD | Inc-RNASET2-1:9 | SEQ4282 | 1. E-02 | 0.6 |
| DLB | Inc-ARRDC2-3:1 | SEQ5618 | 2.E-04 | 1.6 | FTD | Inc-RNF111-3:1 | SEQ5468 | 3.E-04 | 0.6 |
| All AD | Inc-ARSG-2:8 | SEQ2535 | 3.E-02 | 0.6 | DLB | Inc-RNF123-1:1 | SEQ4013 | 2.E-04 | 1.6 |
| MCI | Inc-ARVCF-4:11 | SEQ5818 | 3.E-05 | 1.6 | MCI | Inc-RNF123-1:1 | SEQ4013 | 8.E-04 | 1.5 |
| DLB | Inc-ARVCF-4:11 | SEQ5818 | 5.E-05 | 1.6 | msAD | Inc-RNF123-1:1 | SEQ4013 | 2.E-02 | 1.2 |
| MCI | Inc-ARVCF-4:13 | SEQ4194 | 1.E-05 | 2.0 | All AD | Inc-RNF123-1:1 | SEQ4013 | 3.E-02 | 1.2 |
| FTD | Inc-ARVCF-4:13 | SEQ4194 | 2.E-04 | 1.6 | miAD | Inc-RNF125-2:1 | SEQ4605 | 1. E-03 | 1.6 |
| All AD | Inc-ARVCF-4:13 | SEQ4194 | 4.E-03 | 1.2 | DLB | Inc-RNF125-2:2 | SEQ5760 | 8.E-05 | 2.3 |
| miAD | Inc-ARVCF-4:13 | SEQ4194 | 8.E-03 | 1.2 | FTD | Inc-RNF13-4:1 | SEQ5837 | 4.E-09 | 0.2 |
| msAD | Inc-ARVCF-4:13 | SEQ4194 | 1.E-02 | 1.2 | MCI | Inc-RNF13-4:1 | SEQ5837 | 6.E-08 | 0.3 |
| FTD | Inc-ASCC2-1:1 | SEQ3888 | 1. E-04 | 0.6 | DLB | Inc-RNF13-4:1 | SEQ5837 | 4.E-05 | 0.3 |
| All AD | Inc-ASCC2-1:1 | SEQ3888 | 8.E-03 | 0.7 | miAD | Inc-RNF149-3:1 | SEQ3914 | 1. E-03 | 0.7 |
| miAD | Inc-ASCC2-1:1 | SEQ3888 | 8.E-03 | 0.6 | All AD | Inc-RNF149-3:1 | SEQ3914 | 3.E-03 | 0.7 |
| msAD | Inc-ASCC2-1:1 | SEQ3888 | 3.E-02 | 0.8 | msAD | Inc-RNF149-3:1 | SEQ3914 | 3.E-02 | 0.8 |
| FTD | Inc-ASF1A-6:1 | SEQ4225 | 4.E-04 | 0.7 | FTD | Inc-RNF219-3:1 | SEQ5989 | 2.E-06 | 0.5 |
| MCI | Inc-ASF1A-6:1 | SEQ4225 | 5. E-04 | 0.7 | FTD | Inc-RNF219-6:1 | SEQ5653 | 4.E-07 | 0.5 |
| All AD | Inc-ASF1A-6:1 | SEQ4225 | 7.E-03 | 0.7 | MCI | Inc-RNF219-6:1 | SEQ5653 | 1. E-04 | 0.5 |
| msAD | Inc-ASF1A-6:1 | SEQ4225 | 8.E-03 | 0.7 | DLB | Inc-RNF24-2:5 | SEQ3190 | 5. E-04 | 1.7 |
| All AD | Inc-ASPHD2-2:2 | SEQ2612 | 4.E-03 | 1.1 | DLB | Inc-RNF24-2:9 | SEQ5790 | 6.E-05 | 1.5 |
| msAD | Inc-ASPHD2-2:2 | SEQ2612 | 5.E-03 | 1.2 | DLB | Inc-RNF40-1:1 | SEQ3839 | 8.E-05 | 1.6 |
| miAD | Inc-ASPHD2-2:2 | SEQ2612 | 1.E-02 | 1.1 | MCI | Inc-RNF40-1:1 | SEQ3839 | 6.E-04 | 1.5 |
| FTD | Inc-ASPRV1-2:1 | SEQ4229 | 4.E-05 | 0.6 | msAD | Inc-RNF40-1:1 | SEQ3839 | 3.E-02 | 1.2 |
| All AD | Inc-ASPRV1-2:1 | SEQ4229 | 3.E-02 | 0.8 | DLB | Inc-RNFT2-1:7 | SEQ4939 | 8.E-04 | 1.5 |
| DLB | Inc-ASXL1-3:1 | SEQ2650 | 3.E-04 | 1.5 | MCI | Inc-RNMT-7:1 | SEQ4875 | 8.E-04 | 1.8 |
| MCI | Inc-ASXL1-4:3 | SEQ4231 | 2.E-04 | 1.8 | DLB | Inc-RNMT-7:1 | SEQ4875 | 9.E-04 | 1.9 |
| DLB | Inc-ASXL1-4:3 | SEQ4231 | 7.E-04 | 1.8 | FTD | Inc-ROCK1-1:1 | SEQ5980 | 3.E-06 | 0.6 |
| All AD | Inc-ASXL1-4:3 | SEQ4231 | 4.E-02 | 1.2 | DLB | Inc-ROGDI-1:1 | SEQ5950 | 7.E-06 | 1.4 |
| DLB | Inc-ATAD2-1:1 | SEQ4447 | 2.E-03 | 1.6 | DLB | Inc-ROM1-4:1 | SEQ5258 | 5. E-04 | 1.5 |
| FTD | lnc-ATAD5-9:9 | SEQ3904 | 1.E-06 | 0.5 | DLB | Inc-ROM1-7:1 | SEQ3478 | 3.E-04 | 1.5 |
| miAD | lnc-ATAD5-9:9 | SEQ3904 | 4.E-03 | 0.7 | DLB | Inc-ROM1-7:3 | SEQ3586 | 2.E-04 | 1.6 |
| All AD | Inc-ATAD5-9:9 | SEQ3904 | 6.E-03 | 0.7 | DLB | Inc-ROM1-7:6 | SEQ3589 | 2.E-03 | 1.5 |
| msAD | lnc-ATAD5-9:9 | SEQ3904 | 3.E-02 | 0.7 | FTD | Inc-ROPN1L-2:2 | SEQ5554 | 3.E-08 | 0.5 |
| MCI | Inc-ATG12-3:2 | SEQ2558 | 2.E-03 | 1.8 | MCI | Inc-ROPN1L-2:2 | SEQ5554 | 2.E-05 | 0.6 |
| All AD | Inc-ATG3-1:1 | SEQ4191 | 5.E-03 | 0.7 | All AD | Inc-ROPN1L-2:2 | SEQ5554 | 4.E-02 | 0.7 |
| miAD | Inc-ATG3-1:1 | SEQ4191 | 1.E-02 | 0.7 | FTD | Inc-RORA-2:1 | SEQ3798 | 8.E-06 | 0.4 |
| msAD | Inc-ATG3-1:1 | SEQ4191 | 1.E-02 | 0.7 | All AD | Inc-RORA-2:1 | SEQ3798 | 2.E-02 | 0.7 |
| FTD | Inc-ATL1-4:1 | SEQ5001 | 8.E-04 | 0.7 | msAD | Inc-RORA-2:1 | SEQ3798 | 4.E-02 | 0.7 |
| FTD | Inc-ATOH7-3:3 | SEQ3867 | 3.E-07 | 0.5 | FTD | Inc-RPE-4:2 | SEQ5964 | 5. E-06 | 0.6 |
| MCI | Inc-ATOH7-3:3 | SEQ3867 | 6.E-04 | 0.6 | DLB | Inc-RPIA-4:2 | SEQ4656 | 1. E-03 | 1.9 |
| miAD | Inc-ATOH7-3:3 | SEQ3867 | 3.E-03 | 0.7 | DLB | Inc-RPIA-5:1 | SEQ4982 | 8.E-04 | 2.1 |
| All AD | Inc-ATOH7-3:3 | SEQ3867 | 5.E-03 | 0.7 | All AD | Inc-RPIA-5:1 | SEQ4982 | 3.E-02 | 1.3 |
| msAD | Inc-ATOH7-3:3 | SEQ3867 | 3.E-02 | 0.7 | DLB | Inc-RPL12-1:3 | SEQ3024 | 1. E-03 | 1.5 |
| DLB | Inc-ATOX1-4:1 | SEQ2621 | 1.E-03 | 1.4 | DLB | Inc-RPL13-5:1 | SEQ5446 | 3.E-04 | 1.6 |
| FTD | Inc-ATP11B-6:1 | SEQ4668 | 7.E-07 | 0.5 | DLB | Inc-RPL36AL-1:1 | SEQ4550 | 2.E-03 | 1.3 |
| MCI | Inc-ATP11B-6:1 | SEQ4668 | 1.E-03 | 0.6 | DLB | Inc-RPP38-5:1 | SEQ5046 | 7.E-04 | 1.8 |
| miAD | Inc-ATP1A1-1:2 | SEQ4077 | 1.E-02 | 0.8 | FTD | Inc-RPP38-6:2 | SEQ5782 | 7.E-05 | 0.6 |
| All AD | Inc-ATP1A1-1:2 | SEQ4077 | 2.E-02 | 0.8 | miAD | Inc-RPS12-1:1 | SEQ4218 | 1. E-02 | 0.7 |
| DLB | Inc-ATP6AP2-3:1 | SEQ5197 | 5. E-04 | 1.6 | All AD | Inc-RPS12-1:1 | SEQ4218 | 2.E-02 | 0.7 |
| FTD | Inc-ATP6V1C1-9:4 | SEQ3802 | 7.E-07 | 0.6 | MCI | Inc-RPS25-5:1 | SEQ4968 | 8.E-04 | 1.6 |
| All AD | Inc-ATP6V1C1-9:4 | SEQ3802 | 2.E-02 | 0.7 | MCI | Inc-RRN3-4:1 | SEQ4461 | 1. E-06 | 2.1 |
| msAD | Inc-ATP6V1C1-9:4 | SEQ3802 | 4.E-02 | 0.8 | DLB | Inc-RRN3-4:1 | SEQ4461 | 3.E-06 | 2.1 |
| MCI | Inc-ATP6V1E2-5:2 | SEQ4243 | 8.E-05 | 1.7 | FTD | Inc-RRN3-4:1 | SEQ4461 | 3.E-05 | 1.8 |
| DLB | Inc-ATP6V1E2-5:2 | SEQ4243 | 3.E-04 | 1.5 | miAD | Inc-RRN3-4:1 | SEQ4461 | 2.E-03 | 1.3 |
| All AD | Inc-ATP6V1E2-5:2 | SEQ4243 | 3.E-02 | 1.2 | All AD | Inc-RRN3-4:1 | SEQ4461 | 7.E-03 | 1.3 |
| DLB | Inc-ATP6V1G1-2:1 | SEQ5199 | 5. E-04 | 1.7 | FTD | Inc-RSBN1-1:1 | SEQ5104 | 1.E-05 | 0.5 |
| FTD | Inc-ATP6V1G3-2:1 | SEQ4045 | 3.E-07 | 0.5 | MCI | Inc-RSBN1-1:1 | SEQ5104 | 6.E-04 | 0.6 |
| MCI | Inc-ATP6V1G3-2:1 | SEQ4045 | 4.E-05 | 0.6 | DLB | Inc-RSPH10B2-1:3 | SEQ2580 | 1. E-03 | 1.5 |
| All AD | Inc-ATP6V1G3-2:1 | SEQ4045 | 5.E-03 | 0.7 | DLB | Inc-RSPHl-7:2 | SEQ4513 | 2.E-03 | 1.5 |
| miAD | Inc-ATP6V1G3-2:1 | SEQ4045 | 6.E-03 | 0.6 | DLB | Inc-RTL8B-1:2 | SEQ5693 | 1. E-04 | 1.5 |
| msAD | Inc-ATP6V1G3-2:1 | SEQ4045 | 2.E-02 | 0.7 | FTD | Inc-RTN4-1:1 | SEQ5361 | 4.E-04 | 0.7 |
| MCI | Inc-ATPAF2-2:1 | SEQ4451 | 2.E-03 | 1.6 | All AD | Inc-RTP2-1:1 | SEQ3786 | 1. E-02 | 0.7 |
| DLB | Inc-ATPAF2-2:1 | SEQ4451 | 2.E-03 | 1.7 | msAD | Inc-RTP2-1:1 | SEQ3786 | 4.E-02 | 0.7 |
| DLB | Inc-ATXN7L3-1:3 | SEQ4480 | 2.E-03 | 1.3 | miAD | Inc-RTP2-1:2 | SEQ3661 | 1. E-02 | 0.7 |
| DLB | Inc-AUNIP-1:1 | SEQ5492 | 2.E-04 | 1.6 | All AD | Inc-RTP2-1:2 | SEQ3661 | 1. E-02 | 0.7 |
| DLB | Inc-AUNIP-1:12 | SEQ2821 | 2.E-05 | 1.6 | msAD | Inc-RTP2-1:2 | SEQ3661 | 5.E-02 | 0.7 |
| msAD | Inc-AUNIP-1:12 | SEQ2821 | 7.E-03 | 1.2 | All AD | Inc-RTP4-5:2 | SEQ5563 | 4.E-02 | 0.8 |
| All AD | Inc-AUNIP-1:12 | SEQ2821 | 1.E-02 | 1.2 | FTD | Inc-RXFP2-2:2 | SEQ5941 | 1.E-05 | 0.6 |
| MCI | Inc-AUNIP-2:1 | SEQ4658 | 1.E-03 | 2.0 | FTD | Inc-RXFP2-3:1 | SEQ5289 | 4.E-04 | 0.6 |
| msAD | Inc-B2M-1:1 | SEQ3816 | 4.E-02 | 0.8 | MCI | Inc-SAG-3:1 | SEQ3534 | 2.E-03 | 1.8 |
| msAD | Inc-B2 M-1:2 | SEQ3986 | 2.E-02 | 0.7 | FTD | Inc-SAMD11-12:4 | SEQ4200 | 8.E-07 | 0.2 |
| All AD | Inc-B2M-1:2 | SEQ3986 | 3.E-02 | 0.8 | MCI | Inc-SAMD11-12:4 | SEQ4200 | 1.E-06 | 0.2 |
| All AD | Inc-B4GALNT3-2:11 | SEQ4249 | 5.E-02 | 0.8 | DLB | Inc-SAMD11-12:4 | SEQ4200 | 4.E-05 | 0.3 |
| MCI | Inc-B4GALNT3-4:2 | SEQ4583 | 2.E-03 | 1.5 | All AD | Inc-SAMD11-12:4 | SEQ4200 | 4.E-03 | 0.5 |
| MCI | Inc-BANP-10:1 | SEQ4430 | 2.E-03 | 1.6 | msAD | Inc-SAMD11-12:4 | SEQ4200 | 9.E-03 | 0.6 |
| DLB | Inc-BANP-10:1 | SEQ4430 | 2.E-03 | 1.5 | miAD | Inc-SAMD11-12:4 | SEQ4200 | 1. E-02 | 0.5 |
| DLB | Inc-BASP1-1:1 | SEQ5255 | 5. E-04 | 1.5 | All AD | Inc-SAMD11-13:2 | SEQ3700 | 4.E-02 | 1.2 |
| MCI | Inc-BAZ1A-2:1 | SEQ2485 | 5.E-05 | 1.7 | msAD | Inc-SAMD11-13:2 | SEQ3700 | 5.E-02 | 1.3 |
| FTD | Inc-BAZ1A-2:1 | SEQ2485 | 1. E-04 | 1.7 | FTD | Inc-SAMD11-14:1 | SEQ3001 | 1. E-04 | 0.7 |
| miAD | Inc-BAZ1A-2:1 | SEQ2485 | 7.E-03 | 1.3 | MCI | Inc-SAMD11-14:1 | SEQ3001 | 1. E-03 | 0.7 |
| All AD | Inc-BAZ1A-2:1 | SEQ2485 | 1.E-02 | 1.2 | FTD | Inc-SAMD12-4:1 | SEQ5404 | 3.E-04 | 0.6 |
| FTD | Inc-BAZ2B-1:7 | SEQ3737 | 7.E-05 | 0.6 | All AD | Inc-SAMD12-4:1 | SEQ5404 | 5.E-02 | 0.7 |
| miAD | Inc-BAZ2B-1:7 | SEQ3737 | 4.E-03 | 0.6 | DLB | Inc-SAMD4A-3:1 | SEQ4442 | 7.E-04 | 1.8 |
| All AD | Inc-BAZ2B-1:7 | SEQ3737 | 7.E-03 | 0.7 | MCI | Inc-SAMD4A-3:1 | SEQ4442 | 2.E-03 | 1.6 |
| msAD | Inc-BAZ2B-1:7 | SEQ3737 | 4.E-02 | 0.7 | FTD | Inc-SAMD5-1:10 | SEQ0090 | 3.E-06 | 0.5 |
| FTD | Inc-BAZ2B-2:1 | SEQ3649 | 3.E-05 | 0.6 | miAD | Inc-SAMD5-1:10 | SEQ0090 | 4.E-03 | 0.6 |
| miAD | Inc-BAZ2B-2:1 | SEQ3649 | 1.E-02 | 0.7 | msAD | Inc-SAMD9L-1:1 | SEQ4296 | 7.E-03 | 0.6 |
| All AD | Inc-BAZ2B-2:1 | SEQ3649 | 1.E-02 | 0.7 | All AD | Inc-SAMD9L-1:1 | SEQ4296 | 1. E-02 | 0.7 |
| msAD | Inc-BAZ2B-2:1 | SEQ3649 | 5.E-02 | 0.8 | FTD | Inc-SAR1B-1:1 | SEQ5839 | 1.E-05 | 0.5 |
| FTD | Inc-BCL2L11-3:1 | SEQ5605 | 2.E-04 | 0.7 | MCI | Inc-SAR1B-1:1 | SEQ5839 | 4.E-05 | 0.5 |
| FTD | Inc-BCL2L11-3:3 | SEQ5234 | 5. E-04 | 0.7 | FTD | Inc-SBDS-3:1 | SEQ5159 | 5. E-04 | 0.7 |
| All AD | Inc-BCL2L11-6:1 | SEQ4257 | 4.E-02 | 0.8 | DLB | Inc-SBDS-4:3 | SEQ5480 | 2.E-04 | 1.4 |
| All AD | Inc-BDH1-5:10 | SEQ4258 | S.E-03 | 1.8 | DLB | Inc-SBDS-4:9 | SEQ3145 | 6.E-05 | 1.7 |
| msAD | Inc-BDH1-5:10 | SEQ4258 | 6.E-03 | 1.9 | FTD | Inc-SBF2-1:5 | SEQ4028 | 4.E-05 | 0.6 |
| FTD | Inc-BDH1-5:12 | SEQ4105 | 8.E-08 | 0.4 | All AD | Inc-SBF2-1:5 | SEQ4028 | 5.E-03 | 0.6 |
| miAD | Inc-BDH1-5:12 | SEQ4105 | 1.E-02 | 0.6 | miAD | Inc-SBF2-1:5 | SEQ4028 | 6.E-03 | 0.6 |
| All AD | Inc-BDH1-5:12 | SEQ4105 | 2.E-02 | 0.6 | msAD | Inc-SBF2-1:5 | SEQ4028 | 2.E-02 | 0.7 |
| MCI | Inc-BICD1-5:1 | SEQ4439 | 5. E-04 | 1.8 | DLB | Inc-SBK1-1:1 | SEQ3472 | 2.E-03 | 1.8 |
| DLB | Inc-BICD1-5:1 | SEQ4439 | 2.E-03 | 1.5 | DLB | Inc-SBN01-1:1 | SEQ4866 | 9.E-04 | 1.6 |
| DLB | Inc-BICRAL-3:1 | SEQ2666 | 4.E-04 | 1.5 | DLB | Inc-SBN02-1:3 | SEQ5440 | 3.E-04 | 1.5 |
| DLB | Inc-BIN3-1:1 | SEQ5614 | 2.E-04 | 1.4 | FTD | Inc-SCAF8-5:3 | SEQ5959 | 7.E-06 | 0.6 |
| DLB | Inc-BIN3-2:1 | SEQ2884 | 9.E-04 | 1.4 | DLB | Inc-SCARB1-4:1 | SEQ5040 | 7.E-04 | 1.6 |
| FTD | Inc-BIRC6-1:4 | SEQ0892 | 5. E-04 | 0.7 | MCI | Inc-SCEL-6:1 | SEQ3655 | 6.E-06 | 2.0 |
| FTD | Inc-BOLA2B-1:5 | SEQ5096 | 6.E-04 | 1.6 | DLB | Inc-SCEL-6:1 | SEQ3655 | 6.E-06 | 2.1 |
| DLB | Inc-BOLA2B-1:7 | SEQ4698 | 7.E-04 | 1.4 | FTD | Inc-SCEL-6:1 | SEQ3655 | 2.E-05 | 1.8 |
| MCI | Inc-BOLA2B-1:7 | SEQ4698 | 1.E-03 | 1.5 | All AD | Inc-SCEL-6:1 | SEQ3655 | 3.E-02 | 1.2 |
| DLB | Inc-BOLL-1:1 | SEQ3744 | 3.E-05 | 1.9 | msAD | Inc-SCEL-6:1 | SEQ3655 | 5.E-02 | 1.2 |
| MCI | Inc-BOLL-1:1 | SEQ3744 | 4.E-04 | 1.6 | DLB | Inc-SCG3-5:1 | SEQ4780 | 1. E-03 | 1.6 |
| All AD | Inc-BOLL-1:1 | SEQ3744 | 3.E-02 | 1.2 | DLB | Inc-SCGB2B2-11:1 | SEQ5561 | 5.E-05 | 1.6 |
| msAD | Inc-BOLL-1:1 | SEQ3744 | 4.E-02 | 1.2 | MCI | Inc-SCGB2B2-11:1 | SEQ5561 | 2.E-04 | 1.5 |
| FTD | Inc-BOLL-6:1 | SEQ5586 | 2.E-04 | 0.4 | msAD | Inc-SCGB3A1-3:5 | SEQ3842 | 3.E-02 | 1.3 |
| MCI | Inc-BORCS5-1:2 | SEQ3820 | 1.E-03 | 1.5 | All AD | Inc-SCGB3A1-3:5 | SEQ3842 | 4.E-02 | 1.3 |
| DLB | Inc-BORCS5-1:2 | SEQ3820 | 1.E-03 | 1.5 | All AD | Inc-SC01-1:1 | SEQ5582 | 5.E-02 | 1.2 |
| msAD | Inc-BORCS5-1:2 | SEQ3820 | 4.E-02 | 1.2 | FTD | Inc-SCRN3-5:1 | SEQ3830 | 2.E-04 | 0.7 |
| DLB | Inc-BPNT1-2:5 | SEQ4510 | 2.E-03 | 1.5 | msAD | Inc-SCRN3-5:1 | SEQ3830 | 3.E-02 | 0.8 |
| MCI | Inc-BRAT1-2:1 | SEQ3709 | 1. E-04 | 2.0 | All AD | Inc-SCRN3-5:1 | SEQ3830 | 4.E-02 | 0.8 |
| DLB | Inc-BRAT1-2:1 | SEQ3709 | 1.E-03 | 1.6 | FTD | Inc-SCRN3-7:1 | SEQ5236 | 5. E-04 | 0.7 |
| All AD | Inc-BRAT1-2:1 | SEQ3709 | 3.E-02 | 1.3 | MCI | Inc-SCTR-2:1 | SEQ5676 | 3.E-05 | 2.4 |
| msAD | Inc-BRAT1-2:1 | SEQ3709 | 5.E-02 | 1.3 | FTD | Inc-SCTR-2:1 | SEQ5676 | 9.E-05 | 1.8 |
| msAD | Inc-BRD1-7:1 | SEQ3908 | 3.E-02 | 1.6 | DLB | Inc-SCTR-2:1 | SEQ5676 | 1.E-04 | 2.0 |
| DLB | Inc-BRF1-12:1 | SEQ4271 | 2.E-04 | 2.6 | All AD | Inc-SDCBP-2:8 | SEQ3720 | 4.E-02 | 0.8 |
| msAD | Inc-BRF1-12:1 | SEQ4271 | 8.E-04 | 1.9 | msAD | Inc-SDCBP-2:8 | SEQ3720 | 4.E-02 | 0.8 |
| All AD | Inc-BRF1-12:1 | SEQ4271 | 1.E-03 | 1.8 | FTD | Inc-SDE2-1:15 | SEQ5367 | 5.E-05 | 0.4 |
| miAD | Inc-BRF1-12:1 | SEQ4271 | 7.E-03 | 1.7 | MCI | Inc-SDE2-1:15 | SEQ5367 | 3.E-04 | 0.5 |
| DLB | Inc-BRF2-2:1 | SEQ5012 | 7.E-04 | 1.3 | MCI | Inc-SDE2-1:5 | SEQ5211 | 5.E-04 | 1.8 |
| DLB | Inc-BR13-2:1 | SEQ4573 | 1. E-04 | 1.6 | FTD | Inc-SEC22C-2:12 | SEQ5469 | 3.E-04 | 0.6 |
| MCI | Inc-BR13-2:1 | SEQ4573 | 2.E-03 | 1.5 | All AD | Inc-SEC22C-2:12 | SEQ5469 | 5.E-02 | 0.8 |
| FTD | Inc-BST1-2:1 | SEQ4736 | 7.E-04 | 0.7 | DLB | Inc-SEC24A-2:2 | SEQ4707 | 1.E-03 | 1.5 |
| MCI | Inc-BST1-2:1 | SEQ4736 | 1.E-03 | 0.6 | miAD | Inc-SELENOP-5:1 | SEQ5077 | 6.E-04 | 1.3 |
| FTD | Inc-BTBD1-2:1 | SEQ2683 | 4.E-04 | 0.7 | All AD | Inc-SELENOP-5:1 | SEQ5077 | 5.E-03 | 1.2 |
| DLB | Inc-BTBD6-2:1 | SEQ2709 | 2.E-05 | 1.7 | FTD | Inc-SEMA4B-6:2 | SEQ5588 | 2.E-04 | 0.5 |
| FTD | Inc-BTD-2:1 | SEQ5307 | 8.E-09 | 1.5 | All AD | Inc-SEMA4D-5:3 | SEQ5589 | 4.E-02 | 0.8 |
| MCI | Inc-BTD-2:1 | SEQ5307 | 4.E-04 | 1.3 | FTD | Inc-SENP6-12:1 | SEQ4188 | 5.E-06 | 0.5 |
| All AD | Inc-BTN2A2-1:1 | SEQ4230 | 9.E-03 | 0.7 | miAD | Inc-SENP6-12:1 | SEQ4188 | 1. E-02 | 0.7 |
| msAD | Inc-BTN2A2-1:1 | SEQ4230 | 1.E-02 | 0.7 | All AD | Inc-SENP6-12:1 | SEQ4188 | 2.E-02 | 0.7 |
| FTD | Inc-BTNL8-7:1 | SEQ4119 | 1.E-06 | 1.7 | DLB | Inc-SENP8-2:1 | SEQ3941 | 3.E-05 | 1.5 |
| MCI | Inc-BTNL8-7:1 | SEQ4119 | 1.E-05 | 2.0 | MCI | Inc-SENP8-2:1 | SEQ3941 | 1. E-03 | 1.4 |
| DLB | Inc-BTNL8-7:1 | SEQ4119 | 1. E-04 | 1.8 | All AD | Inc-SENP8-2:1 | SEQ3941 | 8.E-03 | 1.2 |
| miAD | Inc-BTNL8-7:1 | SEQ4119 | 1.E-02 | 1.2 | miAD | Inc-SENP8-2:1 | SEQ3941 | 1. E-02 | 1.2 |
| All AD | Inc-BTNL8-7:1 | SEQ4119 | 2.E-02 | 1.2 | msAD | Inc-SENP8-2:1 | SEQ3941 | 3.E-02 | 1.2 |
| DLB | Inc-BYSL-2:1 | SEQ3918 | 6.E-05 | 1.7 | DLB | Inc-SEPT14-4:1 | SEQ5254 | 5. E-04 | 1.5 |
| MCI | Inc-BYSL-2:1 | SEQ3918 | 4.E-04 | 1.6 | DLB | Inc-SEPT3-4:1 | SEQ4635 | 1. E-03 | 1.5 |
| FTD | Inc-BYSL-2:1 | SEQ3918 | 6.E-04 | 1.3 | MCI | Inc-SERF1B-1:4 | SEQ5583 | 9.E-05 | 2.4 |
| All AD | Inc-BYSL-2:1 | SEQ3918 | 2.E-02 | 1.2 | DLB | Inc-SERF1B-1:4 | SEQ5583 | 2.E-04 | 2.2 |
| msAD | Inc-BYSL-2:1 | SEQ3918 | 3.E-02 | 1.2 | All AD | Inc-SERF1B-1:4 | SEQ5583 | 5.E-02 | 1.2 |
| DLB | Inc-BYSL-3:2 | SEQ4613 | 1.E-03 | 1.3 | MCI | Inc-SERHL2-1:11 | SEQ3755 | 7.E-04 | 1.7 |
| miAD | Inc-BZW1-1:1 | SEQ4153 | 1.E-02 | 0.7 | DLB | Inc-SERHL2-1:11 | SEQ3755 | 2.E-03 | 1.7 |
| All AD | Inc-BZW1-1:1 | SEQ4153 | 4.E-02 | 0.8 | All AD | Inc-SERHL2-1:11 | SEQ3755 | 3.E-02 | 1.2 |
| DLB | Inc-C11orf54-1:1 | SEQ5712 | 1. E-04 | 2.1 | msAD | Inc-SERHL2-1:11 | SEQ3755 | 4.E-02 | 1.3 |
| FTD | Inc-C11orf63-5:4 | SEQ4816 | 1.E-03 | 1.4 | MCI | Inc-SERHL2-1:6 | SEQ4318 | 2.E-05 | 1.8 |
| FTD | Inc-C12orf40-7:1 | SEQ4278 | 3.E-06 | 0.5 | DLB | Inc-SERHL2-1:6 | SEQ4318 | 9.E-04 | 1.6 |
| All AD | Inc-C12orf40-7:1 | SEQ4278 | 2.E-04 | 0.6 | FTD | Inc-SERHL2-1:6 | SEQ4318 | 1. E-03 | 1.5 |
| miAD | Inc-C12orf40-7:1 | SEQ4278 | 3.E-04 | 0.5 | All AD | Inc-SERHL2-1:6 | SEQ4318 | 3.E-03 | 1.3 |
| msAD | Inc-C12orf40-7:1 | SEQ4278 | 2.E-03 | 0.6 | msAD | Inc-SERHL2-1:6 | SEQ4318 | 6.E-03 | 1.3 |
| DLB | Inc-C12orf73-1:1 | SEQ3349 | 2.E-05 | 1.5 | miAD | Inc-SERHL2-1:6 | SEQ4318 | 6.E-03 | 1.3 |
| miAD | Inc-C13orf42-1:1 | SEQ4110 | 1.E-02 | 0.7 | FTD | Inc-SERHL2-1:7 | SEQ4881 | 4.E-04 | 2.4 |
| All AD | Inc-C13orf42-1:1 | SEQ4110 | 3.E-02 | 0.7 | MCI | Inc-SERHL2-1:7 | SEQ4881 | 8.E-04 | 2.5 |
| FTD | Inc-C14orf28-2:1 | SEQ2528 | 6.E-05 | 0.5 | DLB | Inc-SERHL2-1:7 | SEQ4881 | 9.E-04 | 2.2 |
| All AD | Inc-C14orf28-2:1 | SEQ2528 | 2.E-02 | 0.7 | MCI | Inc-SERHL2-1:8 | SEQ0546 | 7.E-04 | 1.6 |
| msAD | Inc-C14orf28-2:1 | SEQ2528 | 5.E-02 | 0.7 | All AD | Inc-SERHL2-1:8 | SEQ0546 | 6.E-03 | 1.3 |
| msAD | Inc-C14orf79-5:1 | SEQ3897 | 3.E-02 | 1.2 | msAD | Inc-SERHL2-1:8 | SEQ0546 | 9.E-03 | 1.3 |
| All AD | Inc-C14orf79-5:1 | SEQ3897 | 4.E-02 | 1.2 | MCI | Inc-SERHL2-4:2 | SEQ4393 | 2.E-03 | 0.5 |
| All AD | Inc-C15orf39-1:1 | SEQ4281 | 2.E-02 | 0.8 | MCI | Inc-SERHL2-4:4 | SEQ4394 | 2.E-03 | 0.5 |
| DLB | Inc-C16orf45-4:1 | SEQ5312 | 4.E-04 | 1.4 | miAD | Inc-SERHL2-8:1 | SEQ4540 | 2.E-03 | 1.3 |
| DLB | Inc-C17orf97-5:2 | SEQ5523 | 2.E-04 | 1.4 | All AD | Inc-SERHL2-8:1 | SEQ4540 | 9.E-03 | 1.2 |
| DLB | Inc-C19orf44-5:1 | SEQ5788 | 6.E-05 | 1.5 | FTD | Inc-SERINC1-3:1 | SEQ5720 | 1. E-04 | 0.6 |
| DLB | Inc-C19orf44-8:3 | SEQ4697 | 1.E-03 | 1.5 | MCI | Inc-SERP1-4:1 | SEQ5692 | 2.E-05 | 1.4 |
| All AD | Inc-C19orf57-6:6 | SEQ4283 | 4.E-02 | 0.8 | DLB | Inc-SERP1-4:1 | SEQ5692 | 1. E-04 | 1.4 |
| All AD | Inc-C19orf57-6:7 | SEQ4284 | 3.E-02 | 0.8 | All AD | Inc-SERPINA1-1:1 | SEQ3844 | 2.E-02 | 0.7 |
| FTD | Inc-C1orf186-6:1 | SEQ6003 | 6.E-08 | 0.5 | msAD | Inc-SERPINA1-1:1 | SEQ3844 | 3.E-02 | 0.8 |
| MCI | Inc-C1orf35-3:1 | SEQ4052 | 8.E-05 | 1.7 | FTD | Inc-SERPINA11-1:1 | SEQ3698 | 1. E-04 | 2.0 |
| DLB | Inc-Clorf35-3:1 | SEQ4052 | 7.E-04 | 1.6 | MCI | Inc-SERPINA11-1:1 | SEQ3698 | 3.E-04 | 2.3 |
| msAD | Inc-C1orf35-3:1 | SEQ4052 | 2.E-02 | 1.2 | msAD | Inc-SERPINA11-1:1 | SEQ3698 | 5.E-02 | 1.2 |
| All AD | Inc-Clorf35-3:1 | SEQ4052 | 3.E-02 | 1.2 | DLB | Inc-SERPINB1-1:10 | SEQ5697 | 1. E-04 | 1.6 |
| MCI | Inc-C1orf35-3:2 | SEQ4274 | 4.E-05 | 1.7 | DLB | Inc-SERPINF1-1:1 | SEQ4683 | 1. E-03 | 1.4 |
| DLB | Inc-C1orf35-3:2 | SEQ4274 | 5. E-04 | 1.6 | DLB | Inc-SERTAD2-1:3 | SEQ5660 | 1. E-04 | 1.5 |
| msAD | Inc-Clorf35-3:2 | SEQ4274 | 8.E-03 | 1.3 | msAD | Inc-SERTAD2-3:4 | SEQ3883 | 3.E-02 | 1.5 |
| All AD | Inc-C1orf35-3:2 | SEQ4274 | 2.E-02 | 1.2 | FTD | Inc-SETD7-3:3 | SEQ5235 | 5. E-04 | 0.7 |
| DLB | Inc-C1QBP-1:1 | SEQ3351 | 8.E-04 | 1.5 | DLB | Inc-SFMBT2-3:1 | SEQ5024 | 7.E-04 | 1.5 |
| FTD | Inc-C1QTNF1-9:1 | SEQ3652 | 3.E-04 | 0.6 | DLB | Inc-SFPQ-2:1 | SEQ0169 | 1. E-03 | 1.5 |
| All AD | Inc-C1QTNF1-9:1 | SEQ3652 | 2.E-02 | 0.7 | FTD | Inc-SFPQ-2:2 | SEQ5549 | 2.E-04 | 0.6 |
| msAD | Inc-C1QTNF1-9:1 | SEQ3652 | 5.E-02 | 0.8 | DLB | Inc-SFPQ-2:4 | SEQ4721 | 1. E-03 | 1.8 |
| DLB | Inc-C1QTNF8-4:1 | SEQ5257 | 5. E-04 | 1.5 | miAD | Inc-SFRP4-3:1 | SEQ4187 | 1. E-02 | 0.7 |
| FTD | Inc-C1R-1:1 | SEQ4288 | 2.E-06 | 0.5 | All AD | Inc-SFRP4-3:1 | SEQ4187 | 1. E-02 | 0.7 |
| All AD | Inc-C1R-1:1 | SEQ4288 | 2.E-02 | 0.7 | FTD | Inc-SFRP4-3:3 | SEQ4215 | 4.E-04 | 0.6 |
| FTD | Inc-C20orf196-4:1 | SEQ4290 | 4.E-04 | 0.6 | All AD | Inc-SFRP4-3:3 | SEQ4215 | 4.E-03 | 0.7 |
| All AD | Inc-C20orf196-4:1 | SEQ4290 | 5.E-02 | 0.7 | msAD | Inc-SFRP4-3:3 | SEQ4215 | 9.E-03 | 0.7 |
| msAD | Inc-C20orf85-3:1 | SEQ3946 | 3.E-02 | 0.8 | miAD | Inc-SFRP4-3:3 | SEQ4215 | 1. E-02 | 0.6 |
| All AD | Inc-C20orf85-3:1 | SEQ3946 | 3.E-02 | 0.8 | DLB | Inc-SGCA-4:2 | SEQ5566 | 2.E-04 | 1.6 |
| DLB | Inc-C20orf96-1:1 | SEQ2764 | 3.E-05 | 1.5 | DLB | Inc-SGSM3-6:1 | SEQ4748 | 1.E-03 | 1.4 |
| DLB | Inc-C21orf58-1:2 | SEQ0033 | 2.E-04 | 1.7 | FTD | Inc-SGTB-5:1 | SEQ4353 | 1. E-04 | 0.6 |
| DLB | Inc-C21orf58-1:5 | SEQ5207 | 5. E-04 | 1.7 | miAD | Inc-SGTB-5:1 | SEQ4353 | 4.E-03 | 0.6 |
| All AD | Inc-C21orf58-1:8 | SEQ4293 | 4.E-02 | 1.4 | All AD | Inc-SGTB-5:1 | SEQ4353 | 2.E-02 | 0.7 |
| All AD | Inc-C5orf56-3:1 | SEQ4294 | 3.E-02 | 0.8 | MCI | Inc-SH2D7-3:1 | SEQ5864 | 7.E-06 | 2.1 |
| miAD | Inc-C6orf132-5:1 | SEQ4197 | 7.E-04 | 1.4 | DLB | Inc-SH2D7-3:1 | SEQ5864 | 4.E-05 | 2.1 |
| All AD | Inc-C6orf132-5:1 | SEQ4197 | 1. E-03 | 1.4 | FTD | Inc-SH3BGRL-1:1 | SEQ5743 | 9.E-05 | 0.6 |
| msAD | Inc-C6orf132-5:1 | SEQ4197 | 1.E-02 | 1.3 | DLB | Inc-SH3GL1-1:1 | SEQ5375 | 3.E-04 | 1.5 |
| FTD | Inc-C6orf201-6:2 | SEQ5871 | 3.E-05 | 0.5 | FTD | Inc-SH3KBP1-1:1 | SEQ5090 | 6.E-04 | 0.7 |
| FTD | Inc-C6orf222-4:2 | SEQ4297 | 4.E-04 | 0.7 | miAD | Inc-SHANK2-1:7 | SEQ4349 | 5.E-03 | 0.7 |
| miAD | Inc-C6orf222-4:2 | SEQ4297 | 4.E-03 | 0.6 | All AD | Inc-SHANK2-1:7 | SEQ4349 | 9.E-03 | 0.8 |
| All AD | Inc-C6orf222-4:2 | SEQ4297 | 1.E-02 | 0.7 | FTD | Inc-SHANK2-5:1 | SEQ4366 | 8.E-07 | 0.5 |
| FTD | Inc-C6orf62-1:1 | SEQ3990 | 4.E-07 | 0.5 | All AD | Inc-SHANK2-5:1 | SEQ4366 | 9.E-04 | 0.7 |
| MCI | Inc-C6orf62-1:1 | SEQ3990 | 8.E-04 | 0.6 | miAD | Inc-SHANK2-5:1 | SEQ4366 | 1. E-03 | 0.7 |
| All AD | Inc-C6orf62-1:1 | SEQ3990 | 8.E-03 | 0.7 | msAD | Inc-SHANK2-5:1 | SEQ4366 | 4.E-03 | 0.7 |
| miAD | Inc-C6orf62-1:1 | SEQ3990 | 1.E-02 | 0.6 | DLB | Inc-SHISA5-2:1 | SEQ4926 | 8.E-04 | 1.4 |
| msAD | Inc-C6orf62-1:1 | SEQ3990 | 2.E-02 | 0.7 | FTD | Inc-SHOC2-1:2 | SEQ4604 | 1.E-05 | 0.6 |
| All AD | Inc-C9orf3-6:1 | SEQ4302 | 3.E-02 | 0.8 | MCI | Inc-SHOC2-1:2 | SEQ4604 | 1. E-03 | 0.7 |
| All AD | Inc-CA4-2:1 | SEQ4303 | 2.E-02 | 0.8 | miAD | Inc-SHOC2-1:2 | SEQ4604 | 1.E-03 | 0.7 |
| DLB | Inc-CA6-8:1 | SEQ2536 | 2.E-04 | 1.4 | All AD | Inc-SHOC2-1:2 | SEQ4604 | 9.E-03 | 0.7 |
| miAD | Inc-CA6-8:1 | SEQ2536 | 2.E-04 | 1.3 | FTD | Inc-SHOC2-3:1 | SEQ5833 | 5.E-05 | 0.6 |
| All AD | Inc-CA6-8:1 | SEQ2536 | 2.E-04 | 1.2 | FTD | Inc-SHOC2-3:2 | SEQ5985 | 2.E-06 | 0.6 |
| msAD | Inc-CA6-8:1 | SEQ2536 | 2.E-03 | 1.2 | FTD | Inc-SHOX2-8:1 | SEQ5290 | 4.E-04 | 0.6 |
| DLB | Inc-CA6-8:2 | SEQ2380 | 4.E-06 | 1.6 | DLB | Inc-SIKE1-2:2 | SEQ5110 | 6.E-04 | 1.4 |
| FTD | Inc-CA6-8:2 | SEQ2380 | 4.E-05 | 1.3 | DLB | Inc-SIPA1-2:1 | SEQ5438 | 3.E-04 | 1.5 |
| MCI | Inc-CA6-8:2 | SEQ2380 | 4.E-05 | 1.5 | FTD | Inc-SIPA1L1-2:13 | SEQ5681 | 1. E-04 | 0.5 |
| msAD | Inc-CA6-8:2 | SEQ2380 | 2.E-04 | 1.2 | FTD | Inc-SIPA1L1-2:8 | SEQ5856 | 4.E-05 | 0.5 |
| All AD | Inc-CA6-8:2 | SEQ2380 | 5. E-04 | 1.2 | DLB | Inc-SIPA1L3-4:2 | SEQ4764 | 2.E-04 | 1.5 |
| miAD | Inc-CA6-8:2 | SEQ2380 | 6.E-03 | 1.2 | MCI | Inc-SIPA1L3-4:2 | SEQ4764 | 1. E-03 | 1.5 |
| FTD | Inc-CACNB2-1:1 | SEQ2386 | 5. E-04 | 0.6 | miAD | Inc-SIRPG-5:3 | SEQ4357 | 4.E-03 | 0.7 |
| FTD | Inc-CACNG1-1:2 | SEQ5592 | 2.E-04 | 0.6 | All AD | Inc-SIRPG-5:3 | SEQ4357 | 3.E-02 | 0.8 |
| FTD | Inc-CALCB-4:1 | SEQ5939 | 1.E-05 | 0.6 | DLB | Inc-SIVA1-1:1 | SEQ5249 | 5. E-04 | 1.5 |
| DLB | Inc-CALHM1-1:3 | SEQ3992 | 1.E-03 | 1.5 | FTD | Inc-SKIL-4:1 | SEQ3966 | 8.E-06 | 0.4 |
| All AD | Inc-CALHM1-1:3 | SEQ3992 | 2.E-02 | 1.2 | MCI | Inc-SKIL-4:1 | SEQ3966 | 6.E-04 | 0.4 |
| msAD | Inc-CALHM1-1:3 | SEQ3992 | 2.E-02 | 1.2 | All AD | Inc-SKIL-4:1 | SEQ3966 | 7.E-03 | 0.6 |
| DLB | Inc-CAMK2D-1:1 | SEQ4309 | 2.E-03 | 2.0 | miAD | Inc-SKIL-4:1 | SEQ3966 | 9.E-03 | 0.6 |
| All AD | Inc-CAMK2D-1:1 | SEQ4309 | 4.E-02 | 1.3 | msAD | Inc-SKIL-4:1 | SEQ3966 | 2.E-02 | 0.6 |
| FTD | Inc-CAND2-2:2 | SEQ5507 | 2.E-04 | 0.3 | DLB | Inc-SKIV2L2-1:2 | SEQ4745 | 1. E-03 | 1.4 |
| FTD | Inc-CAND2-2:4 | SEQ5098 | 2.E-04 | 0.2 | DLB | Inc-SLA2-1:1 | SEQ3570 | 2.E-03 | 1.4 |
| MCI | Inc-CAND2-2:4 | SEQ5098 | 6.E-04 | 0.2 | FTD | Inc-SLAMF7-2:13 | SEQ5719 | 1. E-04 | 0.6 |
| MCI | Inc-CARD14-1:1 | SEQ4701 | 1.E-03 | 1.5 | DLB | Inc-SLC10A1-1:1 | SEQ5022 | 7.E-04 | 1.4 |
| MCI | Inc-CASC1-1:1 | SEQ4548 | 2.E-03 | 0.6 | DLB | Inc-SLC11A2-8:1 | SEQ4443 | 1. E-04 | 1.7 |
| FTD | Inc-CASC1-2:1 | SEQ4146 | 3.E-04 | 0.5 | MCI | Inc-SLC11A2-8:1 | SEQ4443 | 2.E-03 | 1.6 |
| miAD | Inc-CASC1-2:1 | SEQ4146 | 1.E-02 | 0.6 | FTD | Inc-SLC12A6-1:1 | SEQ4113 | 1.E-06 | 0.6 |
| All AD | Inc-CASC1-2:1 | SEQ4146 | 2.E-02 | 0.7 | miAD | Inc-SLC12A6-1:1 | SEQ4113 | 1. E-02 | 0.7 |
| FTD | Inc-CASC1-3:1 | SEQ4315 | 1.E-05 | 0.6 | All AD | Inc-SLC12A6-1:1 | SEQ4113 | 4.E-02 | 0.7 |
| miAD | Inc-CASC1-3:1 | SEQ4315 | 4.E-03 | 0.7 | FTD | Inc-SLC12A8-3:1 | SEQ5629 | 6.E-06 | 0.5 |
| All AD | Inc-CASC1-3:1 | SEQ4315 | 1.E-02 | 0.8 | All AD | Inc-SLC12A8-3:1 | SEQ5629 | 3.E-02 | 0.7 |
| FTD | Inc-CASP10-1:3 | SEQ4317 | 3.E-05 | 0.6 | DLB | Inc-SLC15A3-2:1 | SEQ5708 | 9.E-05 | 1.9 |
| All AD | Inc-CASP10-1:3 | SEQ4317 | 3.E-02 | 0.8 | MCI | Inc-SLC15A3-2:1 | SEQ5708 | 1. E-04 | 1.8 |
| DLB | Inc-CASTOR2-1:1 | SEQ4415 | 2.E-03 | 1.4 | DLB | Inc-SLC16A11-7:11 | SEQ4711 | 1. E-03 | 1.5 |
| DLB | Inc-CATSPERB-1:1 | SEQ4695 | 1.E-03 | 1.4 | FTD | Inc-SLC16A3-7:1 | SEQ5963 | 5. E-06 | 0.5 |
| FTD | Inc-CAV2-2:1 | SEQ5984 | 2.E-06 | 0.5 | All AD | Inc-SLC16A3-8:1 | SEQ3859 | 8.E-03 | 0.7 |
| FTD | Inc-CAVIN2-1:1 | SEQ4219 | 1.E-07 | 0.5 | miAD | Inc-SLC16A3-8:1 | SEQ3859 | 9.E-03 | 0.7 |
| miAD | Inc-CAVIN2-1:1 | SEQ4219 | 2.E-03 | 0.6 | msAD | Inc-SLC16A3-8:1 | SEQ3859 | 3.E-02 | 0.7 |
| All AD | Inc-CAVIN2-1:1 | SEQ4219 | 2.E-03 | 0.7 | FTD | Inc-SLC1A3-1:1 | SEQ0547 | 2.E-07 | 0.5 |
| msAD | Inc-CAVIN2-1:1 | SEQ4219 | 1.E-02 | 0.7 | MCI | Inc-SLC1A3-1:1 | SEQ0547 | 5. E-04 | 0.6 |
| DLB | Inc-CBARP-6:1 | SEQ5560 | 2.E-04 | 1.5 | All AD | Inc-SLC1A3-1:1 | SEQ0547 | 2.E-02 | 0.7 |
| FTD | Inc-CBR3-2:2 | SEQ5972 | 2.E-06 | 0.5 | miAD | Inc-SLC22A1-2:1 | SEQ4277 | 8.E-03 | 0.7 |
| MCI | Inc-CBR3-2:2 | SEQ5972 | 3.E-06 | 0.5 | All AD | Inc-SLC22A1-2:1 | SEQ4277 | 1. E-02 | 0.8 |
| DLB | Inc-CBS-2:1 | SEQ5386 | 3.E-04 | 1.7 | miAD | Inc-SLC22A1-3:2 | SEQ4114 | 1. E-02 | 0.7 |
| miAD | Inc-CBWD5-1:1 | SEQ3662 | 5.E-03 | 0.6 | All AD | Inc-SLC22A1-3:2 | SEQ4114 | 3.E-02 | 0.8 |
| All AD | Inc-CBWD5-1:1 | SEQ3662 | 8.E-03 | 0.7 | All AD | Inc-SLC22A2-1:1 | SEQ5638 | 5.E-02 | 0.8 |
| msAD | Inc-CBWD5-1:1 | SEQ3662 | 5.E-02 | 0.7 | miAD | Inc-SLC22A2-3:1 | SEQ4286 | 7.E-03 | 0.7 |
| All AD | Inc-CBX6-2:1 | SEQ3911 | 8.E-03 | 0.7 | All AD | Inc-SLC22A2-3:1 | SEQ4286 | 1. E-02 | 0.7 |
| miAD | Inc-CBX6-2:1 | SEQ3911 | 9.E-03 | 0.7 | DLB | Inc-SLC22A31-2:1 | SEQ4490 | 1. E-03 | 1.4 |
| msAD | Inc-CBX6-2:1 | SEQ3911 | 3.E-02 | 0.7 | MCI | Inc-SLC22A31-2:1 | SEQ4490 | 2.E-03 | 1.4 |
| DLB | Inc-CCDC146-1:12 | SEQ4553 | 2.E-03 | 1.3 | DLB | Inc-SLC22A31-4:1 | SEQ5559 | 2.E-04 | 1.4 |
| FTD | Inc-CCDC146-2:1 | SEQ5407 | 3.E-04 | 0.6 | FTD | Inc-SLC25A3-7:1 | SEQ2447 | 2.E-07 | 0.5 |
| FTD | Inc-CCDC150-3:1 | SEQ4325 | 1. E-06 | 0.6 | MCI | Inc-SLC25A3-7:1 | SEQ2447 | 2.E-05 | 0.6 |
| All AD | Inc-CCDC150-3:1 | SEQ4325 | 4.E-02 | 0.8 | All AD | Inc-SLC25A3-7:1 | SEQ2447 | 4.E-03 | 0.7 |
| FTD | Inc-CCDC150-4:1 | SEQ5948 | 8.E-06 | 0.6 | miAD | Inc-SLC25A3-7:1 | SEQ2447 | 5.E-03 | 0.7 |
| DLB | Inc-CCDC150-7:1 | SEQ5217 | 3.E-04 | 2.2 | msAD | Inc-SLC25A3-7:1 | SEQ2447 | 1. E-02 | 0.7 |
| MCI | Inc-CCDC150-7:1 | SEQ5217 | 5. E-04 | 2.0 | DLB | Inc-SLC25A39-2:1 | SEQ5393 | 4.E-05 | 0.2 |
| FTD | Inc-CCDC17-1:1 | SEQ5415 | 3.E-04 | 0.7 | MCI | Inc-SLC25A39-2:1 | SEQ5393 | 3.E-04 | 0.2 |
| DLB | Inc-CCDC173-6:1 | SEQ4524 | 2.E-03 | 1.6 | FTD | Inc-SLC25A39-2:1 | SEQ5393 | 3.E-04 | 0.2 |
| FTD | Inc-CCDC175-2:1 | SEQ4894 | 9.E-04 | 0.6 | DLB | Inc-SLC25A51-1:2 | SEQ4740 | 1. E-03 | 1.4 |
| DLB | Inc-CCDC22-1:1 | SEQ4414 | 2.E-03 | 1.4 | DLB | Inc-SLC25A6-4:1 | SEQ3114 | 2.E-04 | 1.6 |
| DLB | Inc-CCDC54-6:4 | SEQ5385 | 3.E-04 | 1.7 | MCI | Inc-SLC25A6-4:1 | SEQ3114 | 8.E-04 | 1.5 |
| FTD | Inc-CCDC68-1:1 | SEQ5424 | 7.E-07 | 0.4 | msAD | Inc-SLC25A6-4:1 | SEQ3114 | 3.E-02 | 1.2 |
| MCI | Inc-CCDC68-1:1 | SEQ5424 | 3.E-04 | 0.5 | MCI | Inc-SLC27A5-2:1 | SEQ4504 | 6.E-04 | 1.5 |
| DLB | Inc-CCDC69-1:1 | SEQ5374 | 3.E-04 | 1.5 | DLB | Inc-SLC27 A5-2:1 | SEQ4504 | 2.E-03 | 1.4 |
| FTD | Inc-CCDC7-1:6 | SEQ3656 | 1.E-05 | 2.1 | DLB | Inc-SLC27A5-3:2 | SEQ4552 | 4.E-05 | 1.4 |
| MCI | Inc-CCDC7-1:6 | SEQ3656 | 8.E-05 | 2.1 | MCI | Inc-SLC27A5-3:2 | SEQ4552 | 2.E-03 | 1.3 |
| DLB | Inc-CCDC7-1:6 | SEQ3656 | 2.E-04 | 1.8 | FTD | Inc-SLC2A13-2:3 | SEQ4541 | 6.E-08 | 0.4 |
| All AD | Inc-CCDC7-1:6 | SEQ3656 | 2.E-02 | 1.3 | MCI | Inc-SLC2A13-2:3 | SEQ4541 | 6.E-05 | 0.4 |
| msAD | Inc-CCDC7-1:6 | SEQ3656 | 5.E-02 | 1.3 | All AD | Inc-SLC2A13-2:3 | SEQ4541 | 4.E-04 | 0.5 |
| FTD | Inc-CCDC71L-1:13 | SEQ4331 | 8.E-05 | 0.5 | msAD | Inc-SLC2A13-2:3 | SEQ4541 | 9.E-04 | 0.5 |
| All AD | Inc-CCDC71L-1:13 | SEQ4331 | 3.E-02 | 0.7 | miAD | Inc-SLC2A13-2:3 | SEQ4541 | 2.E-03 | 0.5 |
| FTD | Inc-CCDC71L-1:14 | SEQ4332 | 8.E-05 | 0.5 | DLB | Inc-SLC2A9-3:1 | SEQ5173 | 5. E-04 | 1.3 |
| All AD | Inc-CCDC71L-1:14 | SEQ4332 | 2.E-02 | 0.7 | DLB | Inc-SLC30A5-6:1 | SEQ4050 | 3.E-04 | 1.5 |
| DLB | Inc-CCDC74A-1:3 | SEQ3824 | 2.E-05 | 1.9 | MCI | Inc-SLC30A5-6:1 | SEQ4050 | 4.E-04 | 1.4 |
| MCI | Inc-CCDC74A-1:3 | SEQ3824 | 5. E-04 | 1.6 | miAD | Inc-SLC30A5-6:1 | SEQ4050 | 2.E-03 | 1.2 |
| msAD | Inc-CCDC74A-1:3 | SEQ3824 | 4.E-02 | 1.2 | All AD | Inc-SLC30A5-6:1 | SEQ4050 | 2.E-03 | 1.2 |
| MCI | Inc-CCDC78-4:1 | SEQ5880 | 3.E-05 | 1.7 | msAD | Inc-SLC30A5-6:1 | SEQ4050 | 2.E-02 | 1.2 |
| FTD | Inc-CCDC83-1:1 | SEQ4001 | 8.E-07 | 0.5 | FTD | Inc-SLC35E3-7:1 | SEQ5993 | 1. E-06 | 0.6 |
| miAD | Inc-CCDC83-1:1 | SEQ4001 | 2.E-03 | 0.6 | DLB | Inc-SLC35E3-8:1 | SEQ5769 | 2.E-07 | 0.3 |
| All AD | Inc-CCDC83-1:1 | SEQ4001 | 3.E-03 | 0.6 | MCI | Inc-SLC35E3-8:1 | SEQ5769 | 2.E-06 | 0.2 |
| msAD | Inc-CCDC83-1:1 | SEQ4001 | 2.E-02 | 0.7 | FTD | Inc-SLC35E3-8:1 | SEQ5769 | 7.E-05 | 0.2 |
| FTD | Inc-CCDC85C-2:1 | SEQ2601 | 3.E-04 | 0.7 | FTD | Inc-SLC35E3-8:2 | SEQ4267 | 8.E-07 | 0.4 |
| FTD | Inc-CCNB1IP1-1:2 | SEQ0903 | 1.E-07 | 1.8 | All AD | Inc-SLC35E3-8:2 | SEQ4267 | 3.E-03 | 0.5 |
| MCI | Inc-CCNB1IP1-1:2 | SEQ0903 | 4.E-05 | 1.7 | msAD | Inc-SLC35E3-8:2 | SEQ4267 | 7.E-03 | 0.6 |
| DLB | Inc-CCNB1IP1-1:2 | SEQ0903 | 2.E-04 | 1.5 | miAD | Inc-SLC35E3-8:2 | SEQ4267 | 8.E-03 | 0.5 |
| FTD | Inc-CCNB1IP1-1:3 | SEQ4026 | 4.E-08 | 0.5 | DLB | Inc-SLC35F5-3:11 | SEQ2639 | 1. E-04 | 1.3 |
| DLB | Inc-CCNB1IP1-1:3 | SEQ4026 | 4.E-05 | 0.6 | MCI | Inc-SLC35F5-3:11 | SEQ2639 | 5.E-04 | 1.2 |
| MCI | Inc-CCNB1IP1-1:3 | SEQ4026 | 2.E-04 | 0.6 | MCI | Inc-SLC35G3-1:3 | SEQ3900 | 1. E-04 | 2.9 |
| All AD | Inc-CCNB1IP1-1:3 | SEQ4026 | 6.E-03 | 0.7 | DLB | Inc-SLC35G3-1:3 | SEQ3900 | 6.E-04 | 2.5 |
| miAD | Inc-CCNB1IP1-1:3 | SEQ4026 | 1.E-02 | 0.7 | All AD | Inc-SLC35G3-1:3 | SEQ3900 | 1. E-02 | 1.5 |
| msAD | Inc-CCNB1IP1-1:3 | SEQ4026 | 2.E-02 | 0.7 | msAD | Inc-SLC35G3-1:3 | SEQ3900 | 3.E-02 | 1.5 |
| FTD | Inc-CCNB1IP1-1:4 | SEQ5261 | 1.E-07 | 1.9 | DLB | Inc-SLC37A2-2:1 | SEQ2906 | 2.E-04 | 1.5 |
| MCI | Inc-CCNB1IP1-1:4 | SEQ5261 | 3.E-05 | 1.7 | DLB | Inc-SLC38A3-1:1 | SEQ4724 | 6.E-05 | 2.3 |
| DLB | Inc-CCNB11P1-1:4 | SEQ5261 | 5.E-04 | 1.5 | MCI | Inc-SLC38A3-1:1 | SEQ4724 | 1.E-03 | 1.9 |
| FTD | Inc-CCNB1IP1-1:5 | SEQ4027 | 2.E-08 | 0.5 | FTD | Inc-SLC39A10-10:1 | SEQ3806 | 6.E-08 | 1.7 |
| DLB | Inc-CCNB1IP1-1:5 | SEQ4027 | 4.E-05 | 0.6 | MCI | Inc-SLC39A10-10:1 | SEQ3806 | 4.E-06 | 1.8 |
| MCI | Inc-CCNB1IP1-1:5 | SEQ4027 | 2.E-04 | 0.6 | DLB | Inc-SLC39A10-10:1 | SEQ3806 | 5.E-05 | 1.6 |
| All AD | Inc-CCNB1IP1-1:5 | SEQ4027 | 7.E-03 | 0.7 | miAD | Inc-SLC39A10-10:1 | SEQ3806 | 2.E-03 | 1.3 |
| miAD | Inc-CCNB1IP1-1:5 | SEQ4027 | 1.E-02 | 0.7 | All AD | Inc-SLC39A10-10:1 | SEQ3806 | 4.E-03 | 1.2 |
| msAD | Inc-CCNB1IP1-1:5 | SEQ4027 | 2.E-02 | 0.7 | msAD | Inc-SLC39A10-10:1 | SEQ3806 | 4.E-02 | 1.2 |
| FTD | Inc-CCNYL1-2:1 | SEQ5810 | 1.E-06 | 0.3 | FTD | Inc-SLC39A10-3:1 | SEQ3934 | 1. E-08 | 0.5 |
| MCI | Inc-CCNYL1-2:1 | SEQ5810 | 5.E-05 | 0.3 | MCI | Inc-SLC39A10-3:1 | SEQ3934 | 4.E-04 | 0.7 |
| FTD | Inc-CCNYL1-2:2 | SEQ4344 | 2.E-05 | 0.6 | All AD | Inc-SLC39A10-3:1 | SEQ3934 | 1. E-02 | 0.7 |
| All AD | Inc-CCNYL1-2:2 | SEQ4344 | 3.E-02 | 0.7 | msAD | Inc-SLC39A10-3:1 | SEQ3934 | 3.E-02 | 0.7 |
| DLB | Inc-CCT4-1:8 | SEQ3356 | 2.E-03 | 1.9 | miAD | Inc-SLC45A1-1:7 | SEQ4103 | 1. E-02 | 0.6 |
| miAD | Inc-CCT8-6:4 | SEQ4292 | 7.E-03 | 0.6 | All AD | Inc-SLC45A1-1:7 | SEQ4103 | 3.E-02 | 0.7 |
| All AD | Inc-CCT8-6:4 | SEQ4292 | 2.E-02 | 0.6 | All AD | Inc-SLC48A1-1:8 | SEQ3648 | 2.E-02 | 0.7 |
| miAD | Inc-CD300C-2:1 | SEQ4256 | 9.E-03 | 0.5 | msAD | Inc-SLC48A1-1:8 | SEQ3648 | 5.E-02 | 0.7 |
| All AD | Inc-CD300C-2:1 | SEQ4256 | 2.E-02 | 0.5 | DLB | Inc-SLC4A11-1:1 | SEQ5515 | 2.E-04 | 1.4 |
| DLB | Inc-CD3D-3:1 | SEQ4140 | 6.E-04 | 1.8 | DLB | Inc-SLC6A18-2:1 | SEQ4175 | 8.E-05 | 2.5 |
| MCI | Inc-CD3D-3:1 | SEQ4140 | 7.E-04 | 1.6 | All AD | Inc-SLC6A18-2:1 | SEQ4175 | 8.E-03 | 1.4 |
| miAD | Inc-CD3D-3:1 | SEQ4140 | 1.E-02 | 1.2 | msAD | Inc-SLC6A18-2:1 | SEQ4175 | 1. E-02 | 1.5 |
| FTD | Inc-CD47-8:1 | SEQ5884 | 3.E-05 | 0.5 | DLB | Inc-SLC6A9-6:1 | SEQ5663 | 1.E-04 | 1.7 |
| FTD | Inc-CD53-1:1 | SEQ5092 | 6.E-04 | 0.7 | All AD | Inc-SLC6A9-6:1 | SEQ5663 | 4.E-02 | 1.2 |
| DLB | Inc-CDC42-2:1 | SEQ4880 | 9.E-04 | 2.2 | DLB | Inc-SLC9A3-1:3 | SEQ5148 | 6.E-04 | 2.1 |
| DLB | Inc-CDC42BPB-4:3 | SEQ4486 | 2.E-03 | 1.4 | All AD | Inc-SLC9A3-1:3 | SEQ5148 | 4.E-02 | 1.3 |
| DLB | Inc-CDC42SE2-1:10 | SEQ2613 | 1. E-04 | 1.6 | DLB | Inc-SLC9A6-1:2 | SEQ4755 | 2.E-04 | 1.5 |
| MCI | Inc-CDC42SE2-1:10 | SEQ2613 | 7.E-04 | 1.5 | MCI | Inc-SLC9A6-1:2 | SEQ4755 | 1.E-03 | 1.4 |
| DLB | Inc-CDC42SE2-1:3 | SEQ4652 | 1. E-04 | 2.1 | All AD | Inc-SLC9A8-3:4 | SEQ5665 | 2.E-02 | 0.6 |
| MCI | Inc-CDC42SE2-1:3 | SEQ4652 | 1.E-03 | 1.8 | miAD | Inc-SLC9A8-3:5 | SEQ4190 | 1. E-02 | 0.7 |
| FTD | Inc-CDC73-5:1 | SEQ5969 | 4.E-06 | 0.6 | FTD | Inc-SLCO4C1-5:1 | SEQ3971 | 8.E-08 | 0.4 |
| DLB | Inc-CDC73-6:2 | SEQ4609 | 1.E-03 | 1.3 | miAD | Inc-SLCO4C1-5:1 | SEQ3971 | 5. E-04 | 0.5 |
| miAD | Inc-CDCA2-4:1 | SEQ3166 | 7.E-03 | 0.7 | MCI | Inc-SLCO4C1-5:1 | SEQ3971 | 9.E-04 | 0.5 |
| All AD | Inc-CDCA2-4:1 | SEQ3166 | 2.E-02 | 0.7 | All AD | Inc-SLCO4C1-5:1 | SEQ3971 | 2.E-03 | 0.6 |
| FTD | Inc-CDH26-9:1 | SEQ5746 | 9.E-05 | 0.7 | msAD | Inc-SLCO4C1-5:1 | SEQ3971 | 2.E-02 | 0.6 |
| All AD | Inc-CDHR3-7:1 | SEQ4352 | 5.E-02 | 0.7 | FTD | Inc-SLCO6A1-3:1 | SEQ5678 | 1. E-04 | 0.4 |
| DLB | Inc-CDIPT-1:11 | SEQ3359 | 2.E-03 | 1.4 | FTD | Inc-SLF1-4:2 | SEQ4985 | 8.E-06 | 0.5 |
| msAD | Inc-CDIPT-1:9 | SEQ3716 | 4.E-02 | 1.2 | miAD | Inc-SLF1-4:2 | SEQ4985 | 8.E-04 | 0.5 |
| FTD | Inc-CDIPT-2:1 | SEQ4354 | 8.E-08 | 1.5 | All AD | Inc-SLF1-4:2 | SEQ4985 | 5.E-03 | 0.6 |
| DLB | Inc-CDIPT-2:1 | SEQ4354 | 2.E-07 | 1.9 | FTD | Inc-SLF1-7:1 | SEQ3722 | 1. E-03 | 0.6 |
| MCI | Inc-CDIPT-2:1 | SEQ4354 | 6.E-07 | 1.7 | All AD | Inc-SLF1-7:1 | SEQ3722 | 1.E-02 | 0.8 |
| All AD | Inc-CDIPT-2:1 | SEQ4354 | 1. E-04 | 1.3 | miAD | Inc-SLF1-7:1 | SEQ3722 | 1. E-02 | 0.7 |
| msAD | Inc-CDIPT-2:1 | SEQ4354 | 2.E-04 | 1.3 | msAD | Inc-SLF1-7:1 | SEQ3722 | 4.E-02 | 0.8 |
| miAD | Inc-CDIPT-2:1 | SEQ4354 | 8.E-04 | 1.3 | DLB | Inc-SLFN12-1:1 | SEQ5272 | 5. E-04 | 1.7 |
| DLB | Inc-CDIPT-3:1 | SEQ3833 | 3.E-05 | 1.6 | DLB | Inc-SLFN14-2:1 | SEQ4717 | 2.E-04 | 1.9 |
| MCI | Inc-CDIPT-3:1 | SEQ3833 | 8.E-05 | 1.5 | MCI | Inc-SLFN14-2:1 | SEQ4717 | 1. E-03 | 1.7 |
| All AD | Inc-CDIPT-3:1 | SEQ3833 | 1.E-02 | 1.2 | FTD | Inc-SLTM-1:2 | SEQ0982 | 2.E-05 | 0.5 |
| msAD | Inc-CDIPT-3:1 | SEQ3833 | 3.E-02 | 1.2 | MCI | Inc-SLTM-1:2 | SEQ0982 | 3.E-04 | 0.6 |
| FTD | Inc-CDK19-4:1 | SEQ4090 | 7.E-05 | 0.6 | All AD | Inc-SLTM-1:2 | SEQ0982 | 5.E-02 | 0.8 |
| miAD | Inc-CDK19-4:1 | SEQ4090 | 2.E-03 | 0.6 | MCI | Inc-SMARCC2-3:2 | SEQ4314 | 6.E-06 | 1.7 |
| All AD | Inc-CDK19-4:1 | SEQ4090 | 3.E-03 | 0.7 | DLB | Inc-SMARCC2-3:2 | SEQ4314 | 3.E-05 | 1.6 |
| msAD | Inc-CDK19-4:1 | SEQ4090 | 1.E-02 | 0.8 | FTD | Inc-SMARCC2-3:2 | SEQ4314 | 1. E-04 | 1.5 |
| FTD | Inc-CDK2AP1-1:10 | SEQ4667 | 1.E-05 | 0.4 | All AD | Inc-SMARCC2-3:2 | SEQ4314 | 3.E-03 | 1.2 |
| MCI | Inc-CDK2AP1-1:10 | SEQ4667 | 1.E-03 | 0.5 | msAD | Inc-SMARCC2-3:2 | SEQ4314 | 6.E-03 | 1.3 |
| DLB | Inc-CDK2AP1-1:9 | SEQ3361 | 1.E-03 | 1.4 | miAD | Inc-SMARCC2-3:2 | SEQ4314 | 7.E-03 | 1.2 |
| FTD | Inc-CDNF-2:3 | SEQ4361 | 2.E-06 | 0.4 | DLB | Inc-SMARCC2-9:1 | SEQ4833 | 9.E-04 | 1.4 |
| MCI | Inc-CDNF-2:3 | SEQ4361 | 5. E-04 | 0.6 | MCI | Inc-SMC5-6:1 | SEQ2686 | 4.E-04 | 2.0 |
| All AD | Inc-CDNF-2:3 | SEQ4361 | 4.E-02 | 0.7 | FTD | Inc-SMCO1-2:2 | SEQ5398 | 3.E-04 | 0.6 |
| DLB | Inc-CDPF1-2:1 | SEQ4059 | 5. E-04 | 1.5 | All AD | Inc-SMCO1-2:2 | SEQ5398 | 2.E-02 | 0.7 |
| msAD | Inc-CDPF1-2:1 | SEQ4059 | 2.E-02 | 1.3 | MCI | Inc-SMCO4-3:1 | SEQ4543 | 2.E-03 | 0.6 |
| All AD | Inc-CDPF1-2:1 | SEQ4059 | 4.E-02 | 1.2 | DLB | Inc-SMCR8-1:1 | SEQ4498 | 8.E-05 | 1.5 |
| msAD | Inc-CDR2-1:4 | SEQ3808 | 4.E-02 | 1.3 | MCI | Inc-SMCR8-1:1 | SEQ4498 | 2.E-03 | 1.4 |
| DLB | Inc-CDR2-4:2 | SEQ5484 | 2.E-04 | 1.5 | FTD | Inc-SMG1-3:1 | SEQ3506 | 3.E-04 | 0.7 |
| DLB | Inc-CDS2-2:1 | SEQ4741 | 1. E-04 | 1.5 | MCI | Inc-SMIM11B-11:1 | SEQ3891 | 6.E-07 | 2.8 |
| MCI | Inc-CDS2-2:1 | SEQ4741 | 1.E-03 | 1.4 | DLB | Inc-SMIM11B-11:1 | SEQ3891 | 1. E-06 | 2.5 |
| FTD | Inc-CEACAM20-2:5 | SEQ5360 | 4.E-04 | 0.7 | FTD | Inc-SMIM11B-11:1 | SEQ3891 | 2.E-05 | 2.2 |
| MCI | Inc-CEBPB-13:10 | SEQ4547 | 2.E-03 | 0.6 | All AD | Inc-SMIM11B-11:1 | SEQ3891 | 9.E-03 | 1.4 |
| DLB | Inc-CEL-5:2 | SEQ3132 | 1.E-03 | 1.7 | miAD | Inc-SMIM11B-11:1 | SEQ3891 | 1. E-02 | 1.4 |
| MCI | Inc-CEMP1-1:1 | SEQ5146 | 3.E-04 | 2.1 | msAD | Inc-SMIM11B-11:1 | SEQ3891 | 3.E-02 | 1.3 |
| DLB | Inc-CEMP1-1:1 | SEQ5146 | 6.E-04 | 1.9 | miAD | Inc-SMIM13-5:1 | SEQ4279 | 8.E-03 | 0.8 |
| MCI | Inc-CEMP1-1:2 | SEQ5799 | 6.E-05 | 2.0 | All AD | Inc-SMIM13-5:1 | SEQ4279 | 2.E-02 | 0.8 |
| DLB | Inc-CEMP1-1:6 | SEQ4620 | 1.E-03 | 1.4 | msAD | Inc-SMTNL2-4:1 | SEQ3997 | 2.E-02 | 2.6 |
| DLB | Inc-CEMP1-1:7 | SEQ4876 | 9.E-04 | 2.0 | All AD | Inc-SMTNL2-4:1 | SEQ3997 | 3.E-02 | 2.2 |
| DLB | Inc-CEMP1-10:1 | SEQ4839 | 9.E-04 | 1.4 | DLB | Inc-SNAP47-2:1 | SEQ5143 | 2.E-04 | 2.1 |
| DLB | Inc-CEMP1-5:1 | SEQ3365 | 4.E-05 | 1.6 | MCI | Inc-SNAP47-2:1 | SEQ5143 | 6.E-04 | 1.8 |
| MCI | Inc-CEMP1-5:1 | SEQ3365 | 6.E-04 | 1.6 | FTD | Inc-SNAPC1-4:1 | SEQ5682 | 3.E-07 | 0.5 |
| All AD | Inc-CENPB-3:1 | SEQ4367 | 3.E-02 | 0.7 | All AD | Inc-SNAPC1-4:1 | SEQ5682 | 5.E-02 | 0.7 |
| All AD | Inc-CENPB-3:2 | SEQ4368 | 3.E-02 | 0.7 | DLB | Inc-SNAPC2-2:3 | SEQ4933 | 5. E-04 | 1.5 |
| DLB | Inc-CENPBD1-2:8 | SEQ4696 | 3.E-04 | 1.5 | MCI | Inc-SNAPC2-2:3 | SEQ4933 | 8.E-04 | 1.4 |
| MCI | Inc-CENPBD1-2:8 | SEQ4696 | 1.E-03 | 1.4 | DLB | Inc-SNAPCS-1:2 | SEQ3920 | 5. E-04 | 1.6 |
| FTD | Inc-CENPT-1:5 | SEQ4369 | 3.E-07 | 2.3 | msAD | Inc-SNAPCS-1:2 | SEQ3920 | 3.E-02 | 1.3 |
| MCI | Inc-CENPT-1:5 | SEQ4369 | 4.E-06 | 2.5 | All AD | Inc-SNAPC5-1:2 | SEQ3920 | 4.E-02 | 1.2 |
| DLB | Inc-CENPT-1:5 | SEQ4369 | 1.E-05 | 2.0 | MCI | Inc-SNF8-1:3 | SEQ5169 | 5. E-04 | 0.8 |
| All AD | Inc-CENPT-1:5 | SEQ4369 | 9.E-04 | 1.3 | msAD | Inc-SNPH-5:1 | SEQ4233 | 1. E-02 | 2.4 |
| msAD | Inc-CENPT-1:5 | SEQ4369 | 2.E-03 | 1.3 | All AD | Inc-SNPH-5:1 | SEQ4233 | 3.E-02 | 1.7 |
| miAD | Inc-CENPT-1:5 | SEQ4369 | 2.E-03 | 1.3 | FTD | Inc-SNPH-6:5 | SEQ4544 | 3.E-06 | 0.6 |
| MCI | Inc-CENPT-1:6 | SEQ5048 | 2.E-04 | 1.8 | MCI | Inc-SNPH-6:5 | SEQ4544 | 2.E-03 | 0.6 |
| DLB | Inc-CENPT-1:6 | SEQ5048 | 7.E-04 | 1.8 | DLB | Inc-SNRPC-2:6 | SEQ3685 | 7.E-06 | 2.0 |
| All AD | Inc-CENPU-1:1 | SEQ4373 | 3.E-02 | 0.7 | MCI | Inc-SNRPC-2:6 | SEQ3685 | 2.E-05 | 1.8 |
| DLB | Inc-CENPVL2-3:1 | SEQ2389 | 2.E-03 | 1.6 | FTD | Inc-SNRPC-2:6 | SEQ3685 | 2.E-04 | 1.6 |
| DLB | Inc-CEP19-1:2 | SEQ5673 | 1. E-04 | 1.7 | All AD | Inc-SNRPC-2:6 | SEQ3685 | 1. E-02 | 1.2 |
| All AD | Inc-CEP350-3:4 | SEQ4375 | 4.E-02 | 0.8 | msAD | Inc-SNRPC-2:6 | SEQ3685 | 5.E-02 | 1.2 |
| FTD | Inc-CEP83-7:1 | SEQ3010 | 5. E-04 | 0.6 | FTD | Inc-SNRPN-8:18 | SEQ5060 | 7.E-04 | 0.6 |
| FTD | Inc-CERKL-7:1 | SEQ5970 | 4.E-06 | 0.6 | MCI | Inc-SNRPN-8:44 | SEQ4445 | 2.E-03 | 1.6 |
| DLB | Inc-CETP-2:2 | SEQ5324 | 4.E-04 | 1.6 | DLB | Inc-SNRPN-8:44 | SEQ4445 | 2.E-03 | 1.8 |
| FTD | Inc-CFAP36-3:10 | SEQ5826 | 5.E-05 | 0.4 | MCI | Inc-SNRPN-8:7 | SEQ5640 | 2.E-04 | 2.8 |
| FTD | Inc-CFAP36-3:2 | SEQ0786 | 7.E-04 | 0.6 | FTD | Inc-SNRPN-8:85 | SEQ5091 | 6.E-04 | 0.7 |
| DLB | Inc-CFAP36-3:3 | SEQ4977 | 8.E-04 | 1.8 | MCI | Inc-SNRPN-8:92 | SEQ5103 | 6.E-04 | 0.6 |
| DLB | Inc-CFAP36-3:4 | SEQ5451 | 3.E-04 | 1.7 | DLB | Inc-SNTG2-4:3 | SEQ4961 | 8.E-04 | 1.6 |
| miAD | Inc-CFLAR-1:4 | SEQ4116 | 1.E-02 | 0.7 | FTD | Inc-SNX11-2:1 | SEQ5679 | 1. E-04 | 0.5 |
| All AD | Inc-CFLAR-1:4 | SEQ4116 | 2.E-02 | 0.8 | FTD | Inc-SNX11-2:2 | SEQ5403 | 3.E-04 | 0.5 |
| DLB | Inc-CGA-2:2 | SEQ4437 | 2.E-03 | 1.5 | FTD | Inc-SNX2-1:2 | SEQ4824 | 3.E-06 | 0.4 |
| FTD | Inc-CGREF1-2:1 | SEQ2472 | 4.E-08 | 0.6 | MCI | Inc-SNX2-1:2 | SEQ4824 | 9.E-04 | 0.5 |
| MCI | Inc-CGREF1-2:1 | SEQ2472 | 5.E-05 | 0.7 | All AD | Inc-SNX33-6:1 | SEQ5691 | 3.E-02 | 1.2 |
| DLB | Inc-CGREF1-2:1 | SEQ2472 | 4.E-04 | 0.8 | FTD | Inc-SNX7-11:1 | SEQ4115 | 1.E-05 | 0.5 |
| DLB | Inc-CHADL-1:7 | SEQ5196 | 5. E-04 | 1.6 | All AD | Inc-SNX7-11:1 | SEQ4115 | 3.E-03 | 0.7 |
| FTD | Inc-CHIC2-2:1 | SEQ5765 | 8.E-05 | 0.7 | miAD | Inc-SNX7-11:1 | SEQ4115 | 3.E-03 | 0.6 |
| FTD | Inc-CHN2-5:1 | SEQ3761 | 6.E-06 | 0.6 | msAD | Inc-SNX7-11:1 | SEQ4115 | 1. E-02 | 0.7 |
| miAD | Inc-CHN2-5:1 | SEQ3761 | 1.E-03 | 0.6 | DLB | Inc-SNX8-1:1 | SEQ4465 | 7.E-06 | 2.1 |
| All AD | Inc-CHN2-5:1 | SEQ3761 | 3.E-03 | 0.7 | MCI | Inc-SNX8-1:1 | SEQ4465 | 7.E-04 | 1.6 |
| msAD | Inc-CHN2-5:1 | SEQ3761 | 4.E-02 | 0.7 | msAD | Inc-SNX8-1:1 | SEQ4465 | 2.E-03 | 1.3 |
| DLB | Inc-CHRAC1-6:1 | SEQ0420 | 4.E-04 | 1.7 | All AD | Inc-SNX8-1:1 | SEQ4465 | 1. E-02 | 1.2 |
| MCI | Inc-CHRAC1-6:1 | SEQ0420 | 1.E-03 | 1.6 | MCI | Inc-SOCS3-3:1 | SEQ5667 | 1. E-04 | 1.7 |
| MCI | Inc-CHRDL2-3:1 | SEQ4264 | 1.E-05 | 3.0 | FTD | Inc-SOCS6-10:1 | SEQ2214 | 2.E-04 | 0.5 |
| FTD | Inc-CHRDL2-3:1 | SEQ4264 | 2.E-05 | 2.4 | All AD | Inc-SOCS6-10:1 | SEQ2214 | 2.E-03 | 0.7 |
| miAD | Inc-CHRDL2-3:1 | SEQ4264 | 9.E-03 | 1.4 | msAD | Inc-SOCS6-10:1 | SEQ2214 | 2.E-03 | 0.7 |
| All AD | Inc-CHRDL2-3:1 | SEQ4264 | 2.E-02 | 1.3 | miAD | Inc-SOCS6-10:1 | SEQ2214 | 1.E-02 | 0.7 |
| miAD | Inc-CHRNA9-2:1 | SEQ4275 | 8.E-03 | 0.6 | FTD | Inc-SOWAHB-6:1 | SEQ5688 | 1. E-04 | 0.6 |
| All AD | Inc-CHRNA9-2:1 | SEQ4275 | 2.E-02 | 0.7 | DLB | Inc-SOX15-1:6 | SEQ5525 | 2.E-04 | 1.5 |
| msAD | Inc-CHRNE-3:3 | SEQ4054 | 2.E-02 | 1.5 | DLB | Inc-SP110-8:1 | SEQ5578 | 2.E-04 | 1.7 |
| All AD | Inc-CHRNE-3:3 | SEQ4054 | 2.E-02 | 1.4 | FTD | Inc-SP110-8:4 | SEQ3913 | 8.E-08 | 0.5 |
| All AD | Inc-CHST3-1:1 | SEQ3822 | 1.E-02 | 1.3 | MCI | Inc-SP110-8:4 | SEQ3913 | 5.E-05 | 0.6 |
| miAD | Inc-CHST3-1:1 | SEQ3822 | 1.E-02 | 1.3 | All AD | Inc-SP110-8:4 | SEQ3913 | 2.E-02 | 0.7 |
| msAD | Inc-CHST3-1:1 | SEQ3822 | 4.E-02 | 1.2 | msAD | Inc-SP110-8:4 | SEQ3913 | 3.E-02 | 0.7 |
| DLB | Inc-CIB3-1:1 | SEQ5176 | 5. E-04 | 1.4 | All AD | Inc-SP110-8:5 | SEQ3738 | 2.E-02 | 0.7 |
| DLB | Inc-CIB3-1:2 | SEQ2750 | 5. E-04 | 1.3 | msAD | Inc-SP110-8:5 | SEQ3738 | 4.E-02 | 0.7 |
| DLB | Inc-CIB3-1:3 | SEQ5179 | 5. E-04 | 1.4 | FTD | Inc-SP110-9:1 | SEQ5228 | 5. E-04 | 0.6 |
| FTD | Inc-CISD2-1:2 | SEQ4031 | 1. E-04 | 0.6 | All AD | Inc-SP140-3:4 | SEQ3575 | 4.E-02 | 0.8 |
| All AD | Inc-CISD2-1:2 | SEQ4031 | 7.E-03 | 0.7 | DLB | Inc-SP140-3:7 | SEQ5250 | 5.E-04 | 1.5 |
| miAD | Inc-CISD2-1:2 | SEQ4031 | 1.E-02 | 0.7 | miAD | Inc-SP9-13:4 | SEQ4245 | 9.E-03 | 0.6 |
| msAD | Inc-CISD2-1:2 | SEQ4031 | 2.E-02 | 0.7 | miAD | Inc-SP9-8:1 | SEQ4209 | 1. E-02 | 0.7 |
| FTD | Inc-CISD2-1:3 | SEQ3366 | 4.E-04 | 1.4 | FTD | Inc-SPACAS-1:1 | SEQ5830 | 5.E-05 | 0.6 |
| MCI | Inc-CISD2-1:3 | SEQ3366 | 5. E-04 | 1.3 | miAD | Inc-SPAG1-7:1 | SEQ4333 | 1. E-04 | 1.3 |
| DLB | Inc-CISD2-11:1 | SEQ3654 | 5.E-05 | 1.6 | All AD | Inc-SPAG1-7:1 | SEQ4333 | 4.E-04 | 1.3 |
| MCI | Inc-CISD2-11:1 | SEQ3654 | 2.E-03 | 1.5 | msAD | Inc-SPAG1-7:1 | SEQ4333 | 6.E-03 | 1.2 |
| msAD | Inc-CISD2-11:1 | SEQ3654 | 5.E-02 | 1.2 | DLB | Inc-SPATA21-3:1 | SEQ3769 | 6.E-05 | 1.5 |
| DLB | Inc-CKB-1:1 | SEQ4952 | 8.E-04 | 1.5 | msAD | Inc-SPATA21-3:1 | SEQ3769 | 4.E-02 | 1.1 |
| DLB | Inc-CKS1B-2:2 | SEQ5479 | 2.E-04 | 1.4 | DLB | Inc-SPATA21-4:9 | SEQ5177 | 5. E-04 | 1.4 |
| MCI | Inc-CKS1B-3:1 | SEQ3367 | 3.E-06 | 1.7 | DLB | Inc-SPATA21-6:3 | SEQ2824 | 2.E-04 | 1.6 |
| DLB | Inc-CKS1B-3:1 | SEQ3367 | 1. E-04 | 1.6 | All AD | Inc-SPATA31A1-3:1 | SEQ5702 | 4.E-02 | 0.7 |
| All AD | Inc-CKS1B-3:1 | SEQ3367 | 4.E-02 | 1.1 | MCI | Inc-SPATA32-1:1 | SEQ4779 | 1. E-03 | 1.6 |
| MCI | Inc-CKS1B-3:3 | SEQ5798 | 6.E-05 | 1.9 | All AD | Inc-SPATA32-2:16 | SEQ4238 | 7.E-03 | 0.8 |
| FTD | Inc-CLASP2-1:2 | SEQ4047 | 7.E-07 | 1.5 | msAD | Inc-SPATA32-2:16 | SEQ4238 | 1. E-02 | 0.8 |
| DLB | Inc-CLASP2-1:2 | SEQ4047 | 1.E-06 | 1.7 | DLB | Inc-SPATA32-2:4 | SEQ3464 | 2.E-03 | 1.8 |
| MCI | Inc-CLASP2-1:2 | SEQ4047 | 5.E-05 | 1.6 | All AD | Inc-SPATA32-2:7 | SEQ5704 | 4.E-02 | 0.8 |
| All AD | Inc-CLASP2-1:2 | SEQ4047 | 1.E-02 | 1.2 | FTD | Inc-SPERT-4:1 | SEQ5777 | 7.E-05 | 0.6 |
| msAD | Inc-CLASP2-1:2 | SEQ4047 | 2.E-02 | 1.2 | miAD | Inc-SPOCK2-1:2 | SEQ3582 | 1. E-03 | 1.4 |
| FTD | Inc-CLDN10-7:1 | SEQ5913 | 2.E-05 | 0.5 | All AD | Inc-SPOCK2-1:2 | SEQ3582 | 2.E-03 | 1.3 |
| DLB | Inc-CLDN4-2:1 | SEQ5371 | 3.E-04 | 1.4 | msAD | Inc-SPOCK2-1:2 | SEQ3582 | 1. E-02 | 1.3 |
| DLB | Inc-CLEC16A-1:1 | SEQ4387 | 5.E-05 | 1.8 | miAD | Inc-SPON2-1:2 | SEQ2237 | 1. E-02 | 0.7 |
| MCI | Inc-CLEC16A-1:1 | SEQ4387 | 2.E-03 | 1.5 | All AD | Inc-SPON2-1:2 | SEQ2237 | 2.E-02 | 0.7 |
| All AD | Inc-CLEC16A-1:1 | SEQ4387 | 2.E-02 | 1.3 | MCI | Inc-SPOPL-10:1 | SEQ2567 | 8.E-05 | 0.4 |
| DLB | Inc-CLEC18B-5:1 | SEQ2878 | 2.E-03 | 1.5 | DLB | Inc-SPOPL-10:1 | SEQ2567 | 8.E-04 | 0.4 |
| miAD | Inc-CLEC2D-8:4 | SEQ4386 | 2.E-03 | 0.3 | FTD | Inc-SPOPL-2:3 | SEQ4138 | 8.E-04 | 0.6 |
| DLB | Inc-CLEC2D-8:7 | SEQ3369 | 3.E-04 | 1.6 | miAD | Inc-SPOPL-2:3 | SEQ4138 | 1. E-02 | 0.8 |
| MCI | Inc-CLEC2D-8:7 | SEQ3369 | 1.E-03 | 1.5 | All AD | Inc-SPOPL-2:3 | SEQ4138 | 2.E-02 | 0.8 |
| All AD | Inc-CLEC2D-8:7 | SEQ3369 | 4.E-03 | 1.3 | FTD | Inc-SPOPL-2:4 | SEQ5101 | 4.E-08 | 0.3 |
| msAD | Inc-CLEC2D-8:7 | SEQ3369 | 5.E-03 | 1.3 | miAD | Inc-SPOPL-2:4 | SEQ5101 | 3.E-04 | 0.4 |
| FTD | Inc-CLEC3A-2:2 | SEQ4802 | 1.E-03 | 0.5 | MCI | Inc-SPOPL-2:4 | SEQ5101 | 6.E-04 | 0.5 |
| FTD | Inc-CLIC6-5:1 | SEQ5544 | 2.E-04 | 0.6 | All AD | Inc-SPOPL-2:4 | SEQ5101 | 9.E-04 | 0.5 |
| All AD | Inc-CLK3-1:1 | SEQ4389 | 4.E-02 | 1.1 | FTD | Inc-SPPL2A-2:1 | SEQ4363 | 2.E-09 | 0.5 |
| DLB | Inc-CLLU1-2:1 | SEQ4390 | 2.E-03 | 1.2 | MCI | Inc-SPPL2A-2:1 | SEQ4363 | 3.E-06 | 0.5 |
| All AD | Inc-CLLU1-2:1 | SEQ4390 | 4.E-02 | 1.1 | All AD | Inc-SPPL2A-2:1 | SEQ4363 | 1. E-03 | 0.7 |
| msAD | Inc-CLLU1-2:2 | SEQ4016 | 2.E-02 | 1.4 | miAD | Inc-SPPL2A-2:1 | SEQ4363 | 2.E-03 | 0.7 |
| All AD | Inc-CLLU1-2:2 | SEQ4016 | 5.E-02 | 1.2 | msAD | Inc-SPPL2A-2:1 | SEQ4363 | 4.E-03 | 0.7 |
| All AD | Inc-CLSPN-2:1 | SEQ4392 | 4.E-02 | 0.8 | DLB | Inc-SPPL3-2:1 | SEQ5246 | 5. E-04 | 1.4 |
| DLB | Inc-CLUAP1-1:2 | SEQ4705 | 1.E-03 | 1.5 | DLB | Inc-SPPL3-3:1 | SEQ5726 | 9.E-05 | 1.4 |
| DLB | Inc-CLUAP1-8:2 | SEQ5247 | 6.E-05 | 1.5 | DLB | Inc-SPPL3-4:1 | SEQ4776 | 3.E-04 | 1.7 |
| MCI | Inc-CLUAP1-8;2 | SEQ5247 | 5. E-04 | 1.4 | MCI | Inc-SPPL3-4:1 | SEQ4776 | 1. E-03 | 1.6 |
| DLB | Inc-CLUAP1-9:1 | SEQ5428 | 3.E-04 | 1.4 | FTD | Inc-SPRTN-2:1 | SEQ4204 | 6.E-08 | 0.5 |
| FTD | Inc-CLUH-2:1 | SEQ4815 | 1.E-03 | 0.8 | miAD | Inc-SPRTN-2:1 | SEQ4204 | 1.E-03 | 0.6 |
| DLB | Inc-CMTM3-1:1 | SEQ2789 | 4.E-04 | 1.3 | MCI | Inc-SPRTN-2:1 | SEQ4204 | 1. E-03 | 0.6 |
| DLB | Inc-CMTM4-5:1 | SEQ5706 | 1. E-04 | 1.8 | All AD | Inc-SPRTN-2:1 | SEQ4204 | 2.E-03 | 0.6 |
| DLB | Inc-CMTM7-2:1 | SEQ3789 | 9.E-04 | 1.3 | msAD | Inc-SPRTN-2:1 | SEQ4204 | 1. E-02 | 0.6 |
| msAD | Inc-CMTM7-2:1 | SEQ3789 | 4.E-02 | 1.2 | DLB | Inc-SPRYD3-1:12 | SEQ3876 | 2.E-05 | 2.2 |
| FTD | Inc-CMTR1-10:1 | SEQ0788 | 4.E-09 | 0.4 | MCI | Inc-SPRYD3-1:12 | SEQ3876 | 3.E-05 | 2.0 |
| MCI | Inc-CMTR1-10:1 | SEQ0788 | 7.E-04 | 0.6 | FTD | Inc-SPRYD3-1:12 | SEQ3876 | 2.E-04 | 1.6 |
| miAD | Inc-CMTR1-10:1 | SEQ0788 | 1.E-02 | 0.5 | All AD | Inc-SPRYD3-1:12 | SEQ3876 | 9.E-03 | 1.3 |
| All AD | Inc-CMTR1-10:1 | SEQ0788 | 2.E-02 | 0.6 | miAD | Inc-SPRYD3-1:12 | SEQ3876 | 1. E-02 | 1.3 |
| DLB | Inc-CNN1-6:1 | SEQ5036 | 9.E-05 | 1.7 | msAD | Inc-SPRYD3-1:12 | SEQ3876 | 3.E-02 | 1.3 |
| MCI | Inc-CNN1-6:1 | SEQ5036 | 7.E-04 | 1.5 | DLB | Inc-SPRYD3-1:8 | SEQ4196 | 7.E-04 | 1.5 |
| DLB | Inc-CNP-1:1 | SEQ3058 | 6.E-04 | 1.5 | miAD | Inc-SPRYD3-1:8 | SEQ4196 | 1. E-02 | 1.3 |
| FTD | Inc-CNTNAP3B-10:1 | SEQ4987 | 8.E-04 | 0.4 | All AD | Inc-SPRYD3-1:8 | SEQ4196 | 3.E-02 | 1.2 |
| msAD | Inc-CNTRL-6:6 | SEQ3795 | 4.E-02 | 1.6 | DLB | Inc-SPTBN4-1:5 | SEQ3774 | 3.E-04 | 1.6 |
| DLB | Inc-COA6-4:1 | SEQ4752 | 3.E-05 | 1.5 | All AD | Inc-SPTBN4-1:5 | SEQ3774 | 3.E-02 | 1.2 |
| MCI | Inc-COA6-4:1 | SEQ4752 | 1.E-03 | 1.4 | msAD | Inc-SPTBN4-1:5 | SEQ3774 | 4.E-02 | 1.2 |
| DLB | Inc-COG7-1:1 | SEQ4630 | 1.E-03 | 1.5 | MCI | Inc-SQSTM1-2:3 | SEQ4572 | 4.E-04 | 1.7 |
| DLB | Inc-COG7-3:3 | SEQ5741 | 9.E-05 | 2.1 | DLB | Inc-SQSTM1-2:3 | SEQ4572 | 2.E-03 | 1.5 |
| DLB | Inc-COG8-2:1 | SEQ4580 | 8.E-05 | 1.7 | DLB | Inc-SQSTM1-4:1 | SEQ5526 | 2.E-04 | 1.5 |
| MCI | Inc-COG8-2:1 | SEQ4580 | 2.E-03 | 1.5 | FTD | Inc-SRD5A3-4:1 | SEQ5600 | 2.E-04 | 0.6 |
| All AD | Inc-COIL-7:1 | SEQ4411 | 4.E-02 | 0.8 | miAD | Inc-SRGN-4:1 | SEQ4157 | 1. E-02 | 0.8 |
| All AD | Inc-COPG1-2:3 | SEQ4412 | 3.E-02 | 0.7 | All AD | Inc-SRGN-4:1 | SEQ4157 | 3.E-02 | 0.8 |
| DLB | Inc-COPG1-3:3 | SEQ5135 | 6.E-04 | 1.7 | MCI | Inc-SRP54-1:2 | SEQ4762 | 1. E-03 | 1.5 |
| FTD | Inc-COPSS-2:1 | SEQ5834 | 5.E-05 | 0.7 | FTD | Inc-SRPK2-1:16 | SEQ5859 | 3.E-08 | 0.1 |
| DLB | Inc-COPS7A-4:3 | SEQ5655 | 1. E-04 | 1.4 | MCI | Inc-SRPK2-1:16 | SEQ5859 | 3.E-06 | 0.1 |
| msAD | Inc-COQ4-1:3 | SEQ3653 | 5.E-02 | 0.9 | DLB | Inc-SRPK2-1:16 | SEQ5859 | 4.E-05 | 0.1 |
| DLB | Inc-COQ9-1:1 | SEQ4440 | 2.E-03 | 1.6 | FTD | Inc-SRPK2-1:17 | SEQ4662 | 2.E-07 | 7.3 |
| MCI | Inc-COX19-2:1 | SEQ3091 | 8.E-05 | 2.1 | MCI | Inc-SRPK2-1:17 | SEQ4662 | 7.E-06 | 4.6 |
| DLB | Inc-COX19-2:1 | SEQ3091 | 5. E-04 | 1.8 | DLB | Inc-SRPK2-1:17 | SEQ4662 | 1. E-03 | 2.7 |
| DLB | Inc-COX411-6:1 | SEQ4446 | 2.E-03 | 1.6 | FTD | Inc-SRPK2-1:18 | SEQ5722 | 1.E-05 | 0.6 |
| miAD | Inc-COX6A2-5:1 | SEQ4280 | 8.E-03 | 0.8 | All AD | Inc-SRPK2-1:18 | SEQ5722 | 3.E-02 | 0.8 |
| All AD | Inc-COX6A2-5:1 | SEQ4280 | 2.E-02 | 0.8 | DLB | Inc-SRPK2-1:5 | SEQ4647 | 1.E-03 | 1.7 |
| MCI | Inc-COX7A2L-4:9 | SEQ4095 | 3.E-04 | 1.4 | FTD | Inc-SRPK2-1:7 | SEQ6004 | 5.E-08 | 0.2 |
| DLB | Inc-COX7A2L-4:9 | SEQ4095 | 8.E-04 | 1.5 | FTD | Inc-SRPK2-5:4 | SEQ5352 | 4.E-04 | 0.6 |
| All AD | Inc-COX7A2L-4:9 | SEQ4095 | 3.E-03 | 1.3 | FTD | Inc-SRPK2-6:1 | SEQ6012 | 2.E-10 | 0.3 |
| miAD | Inc-COX7A2L-4:9 | SEQ4095 | 3.E-03 | 1.3 | FTD | Inc-SRPK2-7:1 | SEQ5931 | 1.E-05 | 0.7 |
| msAD | Inc-COX7A2L-4:9 | SEQ4095 | 1.E-02 | 1.3 | FTD | Inc-SRPK2-8:1 | SEQ4380 | 7.E-06 | 0.4 |
| FTD | Inc-COX7B-4:2 | SEQ4421 | 1. E-04 | 0.6 | miAD | Inc-SRPK2-8:1 | SEQ4380 | 3.E-03 | 0.5 |
| All AD | Inc-COX7B-4:2 | SEQ4421 | 3.E-02 | 0.8 | DLB | Inc-SRRM2-1:2 | SEQ4244 | 5. E-04 | 2.1 |
| MCI | Inc-CPLX1-2:15 | SEQ5214 | 5. E-04 | 2.0 | FTD | Inc-SRRM2-1:2 | SEQ4244 | 7.E-04 | 2.1 |
| DLB | Inc-CPLX1-2:17 | SEQ5918 | 2.E-05 | 1.9 | All AD | Inc-SRRM2-1:2 | SEQ4244 | 9.E-03 | 1.5 |
| MCI | Inc-CPLX1-2:6 | SEQ5464 | 8.E-05 | 4.5 | msAD | Inc-SRRM2-1:2 | SEQ4244 | 1. E-02 | 1.5 |
| FTD | Inc-CPLX1-2:6 | SEQ5464 | 1. E-04 | 3.2 | MCI | Inc-SRSF12-2:1 | SEQ3670 | 1. E-06 | 1.8 |
| DLB | Inc-CPLX1-2:6 | SEQ5464 | 3.E-04 | 3.5 | DLB | Inc-SRSF12-2:1 | SEQ3670 | 3.E-06 | 1.8 |
| DLB | Inc-CPLX1-2:8 | SEQ2869 | 4.E-04 | 1.6 | FTD | Inc-SRSF12-2:1 | SEQ3670 | 6.E-04 | 1.4 |
| FTD | Inc-CPM-1:1 | SEQ3371 | 4.E-05 | 0.6 | miAD | Inc-SRSF12-2:1 | SEQ3670 | 4.E-03 | 1.1 |
| FTD | Inc-CPM-3:1 | SEQ0228 | 1.E-07 | 0.5 | All AD | Inc-SRSF12-2:1 | SEQ3670 | 8.E-03 | 1.1 |
| MCI | Inc-CPM-3:1 | SEQ0228 | 3.E-04 | 0.5 | msAD | Inc-SRSF12-2:1 | SEQ3670 | 5.E-02 | 1.1 |
| All AD | Inc-CPM-3:1 | SEQ0228 | 3.E-02 | 0.8 | DLB | Inc-SRSF2-1:1 | SEQ4861 | 9.E-04 | 1.5 |
| DLB | Inc-CPM-6:1 | SEQ5338 | 4.E-04 | 1.8 | MCI | Inc-SRSF2-10:1 | SEQ4626 | 2.E-04 | 1.5 |
| DLB | Inc-CPPED1-1:1 | SEQ3862 | 2.E-04 | 1.7 | DLB | Inc-SRSF2-10:1 | SEQ4626 | 1. E-03 | 1.4 |
| All AD | Inc-CPPED1-1:1 | SEQ3862 | 1.E-02 | 1.2 | DLB | Inc-SRSF2-9:1 | SEQ5120 | 6.E-04 | 1.5 |
| msAD | Inc-CPPED1-1:1 | SEQ3862 | 3.E-02 | 1.3 | MCI | Inc-SRSF2-9:2 | SEQ4935 | 2.E-04 | 1.6 |
| miAD | Inc-CPSF2-5:1 | SEQ4384 | 3.E-05 | 1.7 | DLB | Inc-SRSF2-9:2 | SEQ4935 | 8.E-04 | 1.4 |
| FTD | Inc-CPSF2-5:1 | SEQ4384 | 8.E-05 | 1.8 | DLB | Inc-SRY-11:1 | SEQ4418 | 2.E-03 | 1.4 |
| All AD | Inc-CPSF2-5:1 | SEQ4384 | 1. E-04 | 1.6 | DLB | Inc-SSBP4-2:1 | SEQ4638 | 1.E-03 | 1.5 |
| MCI | Inc-CPSF2-5:1 | SEQ4384 | 2.E-04 | 1.8 | All AD | Inc-SSH3-4:1 | SEQ5734 | 3.E-02 | 0.8 |
| DLB | Inc-CPSF2-5:1 | SEQ4384 | 2.E-04 | 1.7 | DLB | Inc-SSH3-5:1 | SEQ2791 | 3.E-04 | 1.4 |
| msAD | Inc-CPSF2-5:1 | SEQ4384 | 3.E-03 | 1.4 | MCI | Inc-SSH3-5:1 | SEQ2791 | 7.E-04 | 1.3 |
| MCI | Inc-CRACR2A-5:2 | SEQ5302 | 2.E-04 | 1.6 | MCI | Inc-SSR2-1:4 | SEQ4922 | 8.E-04 | 0.7 |
| DLB | Inc-CRACR2A-5:2 | SEQ5302 | 3.E-04 | 1.6 | FTD | Inc-SSTR4-9:1 | SEQ4890 | 9.E-04 | 0.5 |
| FTD | Inc-CRACR2A-5:2 | SEQ5302 | 4.E-04 | 1.4 | DLB | Inc-ST3GAL2-1:1 | SEQ5310 | 4.E-04 | 1.4 |
| FTD | Inc-CRCP-1:1 | SEQ4805 | 1.E-03 | 0.6 | FTD | Inc-STARD8-3:1 | SEQ2647 | 2.E-04 | 0.7 |
| msAD | Inc-CREG1-3:1 | SEQ3949 | 3.E-02 | 1.2 | All AD | Inc-STARD8-3:1 | SEQ2647 | 1. E-02 | 0.8 |
| All AD | Inc-CREG1-3:1 | SEQ3949 | 3.E-02 | 1.2 | msAD | Inc-STARD8-3:1 | SEQ2647 | 3.E-02 | 0.8 |
| FTD | Inc-CRIPT-5:1 | SEQ5900 | 2.E-05 | 0.6 | FTD | Inc-STARD9-1:6 | SEQ5009 | 5. E-04 | 0.4 |
| DLB | Inc-CRLF1-1:2 | SEQ4674 | 1.E-03 | 1.3 | MCI | Inc-STARD9-1:6 | SEQ5009 | 7.E-04 | 0.4 |
| DLB | Inc-CRLF1-1:4 | SEQ4435 | 2.E-03 | 1.5 | FTD | Inc-STAT1-1:1 | SEQ5513 | 8.E-05 | 0.5 |
| FTD | Inc-CRYBA4-23:40 | SEQ4888 | 9.E-04 | 0.5 | MCI | Inc-STAT1-1:1 | SEQ5513 | 2.E-04 | 0.5 |
| FTD | Inc-CRYBB1-1:3 | SEQ4444 | 1.E-03 | 1.5 | All AD | Inc-STAT1-1:1 | SEQ5513 | 3.E-02 | 0.7 |
| All AD | Inc-CRYBB1-1:3 | SEQ4444 | 3.E-02 | 1.2 | DLB | Inc-STAT3-1:2 | SEQ4918 | 2.E-04 | 3.9 |
| DLB | Inc-CRYM-4:1 | SEQ2958 | 3.E-04 | 1.6 | FTD | Inc-STAT3-1:2 | SEQ4918 | 9.E-04 | 2.6 |
| FTD | Inc-CSNK1A1L-1:1 | SEQ3695 | 5. E-04 | 0.5 | FTD | Inc-STAT3-1:3 | SEQ3739 | 2.E-08 | 0.6 |
| miAD | Inc-CSNK1A1L-1:1 | SEQ3695 | 1.E-03 | 0.6 | All AD | Inc-STAT3-1:3 | SEQ3739 | 2.E-02 | 0.7 |
| All AD | Inc-CSNK1A1L-1:1 | SEQ3695 | 4.E-03 | 0.6 | msAD | Inc-STAT3-1:3 | SEQ3739 | 4.E-02 | 0.8 |
| msAD | Inc-CSNK1A1L-1:1 | SEQ3695 | 5.E-02 | 0.7 | FTD | Inc-STAU2-3:4 | SEQ3564 | 2.E-04 | 0.6 |
| FTD | Inc-CSNK1E-1:4 | SEQ4474 | 5. E-04 | 0.7 | miAD | Inc-STAU2-3:4 | SEQ3564 | 1. E-02 | 0.7 |
| MCI | Inc-CSNK1E-1:4 | SEQ4474 | 2.E-03 | 0.7 | All AD | Inc-STAU2-3:4 | SEQ3564 | 2.E-02 | 0.8 |
| DLB | Inc-CSNK1G2-4:2 | SEQ5529 | 5.E-05 | 1.7 | FTD | Inc-STEAP4-1:1 | SEQ3694 | 7.E-04 | 0.5 |
| MCI | Inc-CSNK1G2-4:2 | SEQ5529 | 2.E-04 | 1.6 | miAD | Inc-STEAP4-1:1 | SEQ3694 | 7.E-03 | 0.6 |
| FTD | Inc-CSPP1-1:5 | SEQ4452 | 4.E-05 | 1.8 | All AD | Inc-STEAP4-1:1 | SEQ3694 | 9.E-03 | 0.7 |
| MCI | Inc-CSPP1-1:5 | SEQ4452 | 4.E-05 | 1.8 | msAD | Inc-STEAP4-1:1 | SEQ3694 | 5.E-02 | 0.7 |
| DLB | Inc-CSPP1-1:5 | SEQ4452 | 1. E-04 | 1.8 | DLB | Inc-STK38-1:1 | SEQ5520 | 2.E-04 | 1.4 |
| All AD | Inc-CSPP1-1:5 | SEQ4452 | 4.E-02 | 1.2 | miAD | Inc-STK39-3:9 | SEQ3878 | 6.E-03 | 1.4 |
| FTD | Inc-CSRP2-1:1 | SEQ3936 | 5. E-04 | 0.7 | All AD | Inc-STK39-3:9 | SEQ3878 | 7.E-03 | 1.3 |
| miAD | Inc-CSRP2-1:1 | SEQ3936 | 2.E-03 | 0.7 | msAD | Inc-STK39-3:9 | SEQ3878 | 3.E-02 | 1.3 |
| All AD | Inc-CSRP2-1:1 | SEQ3936 | 3.E-03 | 0.7 | FTD | Inc-STK4-1:1 | SEQ5420 | 3.E-04 | 0.7 |
| msAD | Inc-CSRP2-1:1 | SEQ3936 | 3.E-02 | 0.8 | DLB | Inc-STOM-1:1 | SEQ4855 | 9.E-04 | 1.5 |
| DLB | Inc-CT47A1-1:1 | SEQ2504 | 4.E-06 | 1.6 | FTD | Inc-sTON2-5:1 | SEQ3631 | 4.E-05 | 0.6 |
| FTD | Inc-CTAGE5-8:4 | SEQ4898 | 9.E-04 | 0.6 | MCI | Inc-sTON2-5:1 | SEQ3631 | 3.E-04 | 0.6 |
| DLB | Inc-CTC1-2:8 | SEQ4631 | 7.E-04 | 1.5 | All AD | Inc-sTON2-5:1 | SEQ3631 | 4.E-02 | 0.8 |
| MCI | Inc-CTC1-2:8 | SEQ4631 | 1.E-03 | 1.5 | FTD | Inc-STRN-1:1 | SEQ4399 | 1. E-06 | 0.6 |
| FTD | Inc-CTNNBIP1-1:1 | SEQ5155 | 5. E-04 | 0.6 | MCI | Inc-STRN-1:1 | SEQ4399 | 2.E-03 | 0.7 |
| DLB | Inc-CTNS-1:1 | SEQ2816 | 4.E-05 | 1.6 | DLB | Inc-STUB1-2:1 | SEQ5253 | 5. E-04 | 1.5 |
| msAD | Inc-CTNS-1:1 | SEQ2816 | 4.E-03 | 1.2 | DLB | Inc-STX10-2:1 | SEQ3470 | 9.E-06 | 1.6 |
| All AD | Inc-CTNS-1:1 | SEQ2816 | 9.E-03 | 1.1 | MCI | Inc-STX10-2:1 | SEQ3470 | 9.E-06 | 1.7 |
| All AD | Inc-CTSZ-2:11 | SEQ4037 | 1.E-02 | 1.4 | FTD | Inc-STX10-2:1 | SEQ3470 | 2.E-05 | 1.5 |
| msAD | Inc-CTSZ-2:11 | SEQ4037 | 2.E-02 | 1.4 | DLB | Inc-STX10-2:2 | SEQ4686 | 6.E-04 | 1.5 |
| FTD | Inc-CTSZ-7:1 | SEQ5690 | 3.E-07 | 0.5 | MCI | Inc-STX10-2:2 | SEQ4686 | 1.E-03 | 1.4 |
| MCI | Inc-CTSZ-7:1 | SEQ5690 | 1. E-04 | 0.6 | FTD | Inc-STX3-4:2 | SEQ4242 | 2.E-05 | 2.0 |
| DLB | Inc-CTU2-1:1 | SEQ5116 | 6.E-04 | 1.4 | DLB | Inc-STX3-4:2 | SEQ4242 | 4.E-05 | 2.2 |
| DLB | Inc-CTU2-3:2 | SEQ5540 | 2.E-04 | 2.0 | MCI | Inc-STX3-4:2 | SEQ4242 | 6.E-05 | 2.4 |
| MCI | Inc-CTU2-3:4 | SEQ5448 | 3.E-04 | 1.7 | All AD | Inc-STX3-4:2 | SEQ4242 | 2.E-03 | 1.5 |
| DLB | Inc-CTXN1-2:1 | SEQ5946 | 9.E-06 | 1.7 | msAD | Inc-STX3-4:2 | SEQ4242 | 3.E-03 | 1.6 |
| DLB | Inc-CUL2-3:5 | SEQ4848 | 9.E-04 | 1.5 | miAD | Inc-STX3-4:2 | SEQ4242 | 1.E-02 | 1.4 |
| FTD | Inc-CWC15-1:1 | SEQ4464 | 2.E-07 | 0.5 | All AD | Inc-STX3-4:7 | SEQ4065 | 8.E-03 | 0.7 |
| MCI | Inc-CWC15-1:1 | SEQ4464 | 4.E-05 | 0.5 | msAD | Inc-STX3-4:7 | SEQ4065 | 2.E-02 | 0.7 |
| All AD | Inc-CWC15-1:1 | SEQ4464 | 4.E-02 | 0.8 | FTD | Inc-STX3-4:8 | SEQ4268 | 8.E-04 | 0.6 |
| FTD | Inc-CWC15-1:2 | SEQ4374 | 2.E-06 | 3.4 | All AD | Inc-STX3-4:8 | SEQ4268 | 3.E-03 | 0.6 |
| DLB | Inc-CWC15-1:2 | SEQ4374 | 2.E-04 | 2.4 | msAD | Inc-STX3-4:8 | SEQ4268 | 7.E-03 | 0.6 |
| All AD | Inc-CWC15-1:2 | SEQ4374 | 7.E-04 | 1.6 | miAD | Inc-STX3-4:8 | SEQ4268 | 8.E-03 | 0.7 |
| miAD | Inc-CWC15-1:2 | SEQ4374 | 1.E-03 | 1.7 | FTD | Inc-STX BP4-1:2 | SEQ5962 | 6.E-06 | 0.4 |
| msAD | Inc-CWC15-1:2 | SEQ4374 | 3.E-03 | 1.6 | DLB | Inc-SUGCT-4:1 | SEQ3612 | 2.E-03 | 1.4 |
| DLB | Inc-CWC15-5:1 | SEQ4118 | 4.E-05 | 1.5 | FTD | Inc-sUGCT-5:1 | SEQ4035 | 6.E-06 | 0.5 |
| MCI | Inc-CWC15-5:1 | SEQ4118 | 2.E-03 | 1.3 | MCI | Inc-sUGCT-5:1 | SEQ4035 | 3.E-04 | 0.7 |
| miAD | Inc-CWC15-5:1 | SEQ4118 | 1.E-02 | 1.2 | All AD | Inc-sUGCT-5:1 | SEQ4035 | 9.E-03 | 0.7 |
| All AD | Inc-CWC15-5:1 | SEQ4118 | 2.E-02 | 1.2 | msAD | Inc-sUGCT-5:1 | SEQ4035 | 2.E-02 | 0.7 |
| FTD | Inc-CWF19L2-1:2 | SEQ5599 | 2.E-04 | 0.6 | DLB | Inc-SULT1A4-1:9 | SEQ4582 | 2.E-03 | 1.5 |
| miAD | Inc-CXorf21-2:1 | SEQ4180 | 1.E-02 | 0.6 | MCI | Inc-SUMF1-5:1 | SEQ5751 | 1. E-06 | 2.2 |
| All AD | Inc-CXorf21-2:1 | SEQ4180 | 2.E-02 | 0.7 | FTD | Inc-SUMF1-5:1 | SEQ5751 | 2.E-06 | 1.8 |
| DLB | Inc-CXorf65-2:1 | SEQ2748 | 3.E-05 | 1.5 | DLB | Inc-SUMF1-5:1 | SEQ5751 | 6.E-06 | 2.4 |
| FTD | Inc-CYB5D2-2:2 | SEQ5297 | 4.E-04 | 0.7 | All AD | Inc-SUMF1-5:1 | SEQ5751 | 4.E-02 | 1.2 |
| msAD | Inc-CYB5R2-3:10 | SEQ4322 | 6.E-03 | 1.4 | FTD | Inc-SUMO4-2:1 | SEQ5857 | 4.E-05 | 0.6 |
| All AD | Inc-CYB5R2-3:10 | SEQ4322 | 4.E-02 | 1.2 | FTD | Inc-SUPT16H-3:1 | SEQ4923 | 5. E-04 | 0.8 |
| DLB | Inc-CYGB-1:1 | SEQ5753 | 8.E-05 | 1.6 | MCI | Inc-SUPT16H-3:1 | SEQ4923 | 8.E-04 | 0.8 |
| msAD | Inc-CYGB-2:2 | SEQ3872 | 3.E-02 | 0.9 | FTD | Inc-SUPT16H-3:2 | SEQ4030 | 3.E-09 | 0.4 |
| DLB | Inc-CYGB-4:1 | SEQ5318 | 4.E-04 | 1.5 | MCI | Inc-SUPT16H-3:2 | SEQ4030 | 8.E-05 | 0.5 |
| miAD | Inc-CYP1B1-1:3 | SEQ4468 | 2.E-03 | 0.7 | All AD | Inc-SUPT16H-3:2 | SEQ4030 | 6.E-03 | 0.7 |
| All AD | Inc-CYP1B1-1:3 | SEQ4468 | 5.E-03 | 0.7 | miAD | Inc-SUPT16H-3:2 | SEQ4030 | 1. E-02 | 0.7 |
| DLB | Inc-CYP4F22-2:2 | SEQ5815 | 5.E-05 | 1.6 | msAD | Inc-SUPT16H-3:2 | SEQ4030 | 2.E-02 | 0.7 |
| DLB | Inc-CYP4F22-6:1 | SEQ5038 | 7.E-04 | 1.6 | DLB | Inc-SUPT6H-1:2 | SEQ5860 | 4.E-05 | 1.5 |
| All AD | Inc-CYP4F8-1:1 | SEQ4470 | 4.E-02 | 1.2 | DLB | Inc-SUSD1-1:4 | SEQ5144 | 6.E-04 | 1.9 |
| All AD | Inc-CYP4F8-1:2 | SEQ4471 | 4.E-02 | 1.2 | FTD | Inc-SUSD1-1:5 | SEQ0638 | 6.E-04 | 0.6 |
| msAD | Inc-CYR61-3:5 | SEQ3687 | 5.E-02 | 1.2 | miAD | Inc-SUSD1-1:5 | SEQ0638 | 1. E-02 | 0.7 |
| FTD | Inc-CYTIP-2:1 | SEQ0200 | 9.E-04 | 0.7 | All AD | Inc-SUSD1-1:5 | SEQ0638 | 4.E-02 | 0.8 |
| DLB | Inc-DALRD3-1:11 | SEQ4791 | 1.E-03 | 1.9 | DLB | Inc-SWSAP1-4:1 | SEQ4126 | 4.E-05 | 1.5 |
| DLB | Inc-DALRD3-1:4 | SEQ3374 | 2.E-04 | 1.5 | All AD | Inc-SWSAP1-4:1 | SEQ4126 | 3.E-03 | 1.2 |
| DLB | Inc-DALRD3-6:1 | SEQ4475 | 1. E-04 | 1.5 | miAD | Inc-SWSAP1-4:1 | SEQ4126 | 4.E-03 | 1.2 |
| All AD | Inc-DALRD3-6:1 | SEQ4475 | 2.E-02 | 1.2 | msAD | Inc-SWSAP1-4:1 | SEQ4126 | 1. E-02 | 1.2 |
| FTD | Inc-DAPP1-8:3 | SEQ2439 | 5. E-04 | 0.6 | FTD | Inc-SYCE3-1:2 | SEQ3976 | 4.E-04 | 0.7 |
| FTD | Inc-DARS2-3:2 | SEQ4913 | 9.E-04 | 0.8 | msAD | Inc-SYCE3-1:2 | SEQ3976 | 2.E-02 | 0.7 |
| MCI | Inc-DAZAP2-3:1 | SEQ0056 | 2.E-03 | 1.6 | All AD | Inc-SYCE3-1:2 | SEQ3976 | 2.E-02 | 0.7 |
| DLB | Inc-DBT-3:1 | SEQ5340 | 4.E-05 | 1.9 | DLB | Inc-SYNE3-1:1 | SEQ5369 | 3.E-04 | 1.4 |
| MCI | Inc-DBT-3:1 | SEQ5340 | 4.E-04 | 1.8 | DLB | Inc-SYNE3-1:2 | SEQ5368 | 3.E-04 | 1.4 |
| All AD | Inc-DBX2-4:3 | SEQ4477 | 5.E-02 | 1.1 | DLB | Inc-SYNE3-1:3 | SEQ5309 | 4.E-04 | 1.4 |
| DLB | Inc-DCAF1-1:1 | SEQ3375 | 1. E-04 | 1.3 | All AD | Inc-SYNE3-S:1 | SEQ5757 | 3.E-02 | 1.4 |
| FTD | Inc-DCAF4L1-2:1 | SEQ2387 | 1. E-04 | 0.6 | MCI | Inc-SYT13-8:1 | SEQ3708 | 2.E-06 | 1.7 |
| All AD | Inc-DCAF4L1-2:1 | SEQ2387 | 1.E-02 | 0.7 | DLB | Inc-SYT13-8:1 | SEQ3708 | 1.E-05 | 1.6 |
| msAD | Inc-DCAF4L1-2:1 | SEQ2387 | 3.E-02 | 0.7 | All AD | Inc-SYT13-8:1 | SEQ3708 | 4.E-02 | 1.2 |
| MCI | Inc-DCLRE1B-3:1 | SEQ5928 | 2.E-06 | 0.4 | msAD | Inc-SYT13-8:1 | SEQ3708 | 5.E-02 | 1.2 |
| FTD | Inc-DCLRE1B-3:1 | SEQ5928 | 1.E-05 | 0.4 | DLB | Inc-SYT2-4:2 | SEQ5097 | 6.E-04 | 2.E-03 |
| FTD | Inc-DCUN1D1-10:1 | SEQ4291 | 1.E-09 | 0.3 | FTD | Inc-TAAR9-3:4 | SEQ5364 | 3.E-05 | 0.3 |
| MCI | Inc-DCUN1D1-10:1 | SEQ4291 | 2.E-05 | 0.4 | MCI | Inc-TAAR9-3:4 | SEQ5364 | 3.E-04 | 0.3 |
| DLB | Inc-DCUN1D1-10:1 | SEQ4291 | 9.E-05 | 0.4 | FTD | Inc-TAAR9-3:5 | SEQ4732 | 2.E-10 | 0.4 |
| miAD | Inc-DCUN1D1-10:1 | SEQ4291 | 7.E-03 | 0.4 | MCI | Inc-TAAR9-3:5 | SEQ4732 | 6.E-06 | 0.4 |
| All AD | Inc-DCUN1D1-10:1 | SEQ4291 | 1.E-02 | 0.5 | miAD | Inc-TAAR9-3:5 | SEQ4732 | 1. E-03 | 0.6 |
| DLB | Inc-DDB1-1:2 | SEQ5421 | 8.E-05 | 1.7 | All AD | Inc-TAAR9-3:5 | SEQ4732 | 6.E-03 | 0.6 |
| MCI | Inc-DDB1-1:2 | SEQ5421 | 2.E-04 | 1.6 | DLB | Inc-TAF12-5:1 | SEQ3964 | 2.E-04 | 1.4 |
| FTD | Inc-DDB1-1:2 | SEQ5421 | 3.E-04 | 1.5 | msAD | Inc-TAF12-5:1 | SEQ3964 | 2.E-02 | 1.2 |
| MCI | Inc-DDIAS-6:1 | SEQ3745 | 5. E-04 | 1.4 | DLB | Inc-TAF6-1:2 | SEQ5219 | 5. E-04 | 2.7 |
| DLB | Inc-DDIAS-6:1 | SEQ3745 | 7.E-04 | 1.6 | DLB | Inc-TAF9-1:8 | SEQ4558 | 2.E-03 | 1.3 |
| miAD | Inc-DDIAS-6:1 | SEQ3745 | 8.E-03 | 1.3 | miAD | Inc-TAF9-2:2 | SEQ4102 | 1. E-02 | 0.5 |
| All AD | Inc-DDIAS-6:1 | SEQ3745 | 1.E-02 | 1.2 | DLB | Inc-TAF9-6:6 | SEQ4969 | 8.E-04 | 1.6 |
| msAD | Inc-DDIAS-6:1 | SEQ3745 | 4.E-02 | 1.2 | DLB | Inc-TAGLN-2:1 | SEQ3559 | 4.E-04 | 1.4 |
| MCI | Inc-DDX39A-3:1 | SEQ4785 | 1. E-03 | 1.7 | FTD | Inc-TANC1-1:3 | SEQ5238 | 1. E-08 | 0.3 |
| FTD | Inc-DDX58-3:1 | SEQ4044 | 1.E-03 | 0.7 | miAD | Inc-TANC1-1:3 | SEQ5238 | 5.E-04 | 0.5 |
| All AD | Inc-DDX58-3:1 | SEQ4044 | 1.E-02 | 0.7 | FTD | Inc-TAOK1-4:1 | SEQ3609 | 7.E-04 | 0.7 |
| msAD | Inc-DDX58-3:1 | SEQ4044 | 2.E-02 | 0.7 | MCI | Inc-TAOK1-4:2 | SEQ5761 | 1.E-05 | 0.2 |
| DLB | Inc-DDX6-1:1 | SEQ5115 | 6.E-04 | 1.4 | FTD | Inc-TAOK1-4:2 | SEQ5761 | 8.E-05 | 0.2 |
| DLB | Inc-DEC1-7:1 | SEQ3804 | 2.E-04 | 1.6 | DLB | Inc-TAOK3-9:1 | SEQ2396 | 6.E-04 | 1.5 |
| MCI | Inc-DEC1-7:1 | SEQ3804 | 9.E-04 | 1.6 | miAD | Inc-TAOK3-9:1 | SEQ2396 | 2.E-03 | 1.3 |
| msAD | Inc-DEC1-7:1 | SEQ3804 | 4.E-02 | 1.2 | All AD | Inc-TAOK3-9:1 | SEQ2396 | 3.E-03 | 1.2 |
| DLB | Inc-DEFB115-3:1 | SEQ5595 | 9.E-06 | 2.4 | msAD | Inc-TAOK3-9:1 | SEQ2396 | 2.E-02 | 1.2 |
| FTD | Inc-DEFB115-3:1 | SEQ5595 | 2.E-04 | 1.8 | MCI | Inc-TARBP1-6:1 | SEQ2671 | 5.E-05 | 1.7 |
| DLB | Inc-DEFB115-5:2 | SEQ2465 | 8.E-05 | 2.0 | DLB | Inc-TARBP1-6:1 | SEQ2671 | 1. E-04 | 1.7 |
| FTD | Inc-DEFB115-5:2 | SEQ2465 | 6.E-04 | 1.7 | FTD | Inc-TARBP1-6:1 | SEQ2671 | 2.E-04 | 1.6 |
| All AD | Inc-DEFB115-5:2 | SEQ2465 | 4.E-02 | 1.3 | All AD | Inc-TARBP1-6:1 | SEQ2671 | 4.E-04 | 1.5 |
| All AD | Inc-DEFB131B-5:1 | SEQ3683 | 4.E-02 | 1.2 | msAD | Inc-TARBP1-6:1 | SEQ2671 | 1. E-03 | 1.4 |
| msAD | Inc-DEFB131B-5:1 | SEQ3683 | 5.E-02 | 1.2 | miAD | Inc-TARBP1-6:1 | SEQ2671 | 1. E-03 | 1.5 |
| DLB | Inc-DEGS2-2:2 | SEQ4003 | 9.E-06 | 1.6 | MCI | Inc-TARS-6:1 | SEQ3544 | 1. E-03 | 1.7 |
| FTD | Inc-DEGS2-2:2 | SEQ4003 | 9.E-04 | 1.4 | FTD | Inc-TAS2R30-1:2 | SEQ5288 | 4.E-04 | 0.6 |
| MCI | Inc-DEGS2-2:2 | SEQ4003 | 9.E-04 | 1.4 | FTD | Inc-TBC1D19-8:1 | SEQ5084 | 6.E-04 | 0.6 |
| All AD | Inc-DEGS2-2:2 | SEQ4003 | 8.E-03 | 1.2 | FTD | Inc-TBC1D19-8:2 | SEQ5960 | 6.E-06 | 4.E-02 |
| miAD | Inc-DEGS2-2:2 | SEQ4003 | 1.E-02 | 1.3 | FTD | Inc-TBC1D31-3:1 | SEQ5594 | 2.E-04 | 0.6 |
| msAD | Inc-DEGS2-2:2 | SEQ4003 | 2.E-02 | 1.2 | DLB | Inc-TBX19-2:1 | SEQ5795 | 6.E-05 | 1.7 |
| DLB | Inc-DENND1A-5:1 | SEQ3185 | 8.E-05 | 1.6 | DLB | Inc-TBX2-7:1 | SEQ5201 | 5.E-04 | 1.8 |
| MCI | Inc-DENND3-7:1 | SEQ4306 | 2.E-04 | 1.7 | MCI | Inc-TBX2-7:1 | SEQ5201 | 5. E-04 | 1.7 |
| DLB | Inc-DENND3-7:1 | SEQ4306 | 3.E-04 | 1.6 | DLB | Inc-TBXA2R-2:1 | SEQ4459 | 2.E-03 | 2.5 |
| msAD | Inc-DENND3-7:1 | SEQ4306 | 7.E-03 | 1.3 | FTD | Inc-TC2N-1:1 | SEQ4893 | 9.E-04 | 0.6 |
| All AD | Inc-DENND3-7:1 | SEQ4306 | 2.E-02 | 1.2 | FTD | Inc-TCEANC2-1:1 | SEQ5000 | 8.E-04 | 0.7 |
| DLB | Inc-DENND3-9:1 | SEQ4263 | 3.E-04 | 1.7 | All AD | Inc-TCF19-1:104 | SEQ5768 | 4.E-02 | 0.7 |
| MCI | Inc-DENND3-9:1 | SEQ4263 | 8.E-04 | 1.8 | FTD | Inc-TCF19-1:107 | SEQ5466 | 3.E-04 | 0.6 |
| msAD | Inc-DENND3-9:1 | SEQ4263 | 9.E-03 | 1.3 | All AD | Inc-TCF19-1:107 | SEQ5466 | 3.E-02 | 0.7 |
| All AD | Inc-DENND3-9:1 | SEQ4263 | 2.E-02 | 1.2 | DLB | Inc-TCF7-1:3 | SEQ0990 | 2.E-04 | 1.4 |
| DLB | Inc-DFNB59-2:28 | SEQ5937 | 1.E-05 | 2.4 | MCI | Inc-TCFLS-6:1 | SEQ5564 | 1. E-04 | 1.5 |
| DLB | Inc-DHRS7B-7:2 | SEQ5187 | 5. E-04 | 1.5 | FTD | Inc-TCFLS-6:1 | SEQ5564 | 2.E-04 | 1.3 |
| DLB | Inc-DHRSX-1:6 | SEQ5311 | 4.E-04 | 1.4 | DLB | Inc-TCFLS-6:1 | SEQ5564 | 2.E-04 | 1.5 |
| FTD | Inc-DHX33-1:1 | SEQ2533 | 6.E-04 | 0.7 | All AD | Inc-TCP1-4:1 | SEQ5772 | 1. E-02 | 0.8 |
| msAD | Inc-DHX38-4:13 | SEQ2803 | 5.E-02 | 1.2 | DLB | Inc-TCTA-1:1 | SEQ4966 | 8.E-04 | 1.6 |
| DLB | Inc-DLGAPS-2:1 | SEQ2566 | 9.E-04 | 1.3 | FTD | Inc-TCTA-2:1 | SEQ2757 | 1.E-05 | 0.7 |
| DLB | Inc-DMTN-5:1 | SEQ5269 | 2.E-05 | 1.8 | MCI | Inc-TCTA-2:1 | SEQ2757 | 7.E-04 | 0.8 |
| MCI | Inc-DMTN-5:1 | SEQ5269 | 5. E-04 | 1.6 | All AD | Inc-TCTN3-2:1 | SEQ5775 | 3.E-02 | 0.8 |
| All AD | Inc-DMXL1-7:1 | SEQ4018 | 1.E-02 | 1.2 | FTD | Inc-TDP1-6:2 | SEQ5680 | 1. E-04 | 0.5 |
| msAD | Inc-DMXL1-7:1 | SEQ4018 | 2.E-02 | 1.2 | miAD | Inc-TEAD2-2:4 | SEQ3500 | 8.E-03 | 1.3 |
| MCI | Inc-DNAH10OS-6:1 | SEQ5010 | 7.E-04 | 0.5 | All AD | Inc-TEAD2-2:4 | SEQ3500 | 9.E-03 | 1.3 |
| DLB | Inc-DNAH10OS-8:2 | SEQ5044 | 7.E-04 | 1.7 | msAD | Inc-TEAD2-2:4 | SEQ3500 | 4.E-02 | 1.2 |
| DLB | Inc-DNAJAl-1:1 | SEQ5797 | 6.E-05 | 1.9 | MCI | Inc-TEC-2:1 | SEQ5141 | 5.E-05 | 1.8 |
| msAD | Inc-DNAJC15-5:1 | SEQ3843 | 3.E-02 | 0.6 | FTD | Inc-TEC-2:1 | SEQ5141 | 2.E-04 | 1.6 |
| msAD | Inc-DNAJC15-6:1 | SEQ3924 | 3.E-02 | 0.6 | DLB | Inc-TEC-2:1 | SEQ5141 | 6.E-04 | 1.8 |
| All AD | Inc-DNAJC15-6:1 | SEQ3924 | 3.E-02 | 0.6 | DLB | Inc-TEF-1:1 | SEQ3075 | 5.E-05 | 1.6 |
| DLB | Inc-DNAJC16-1:2 | SEQ4625 | 1.E-03 | 1.4 | MCI | Inc-TEF-1:1 | SEQ3075 | 7.E-04 | 1.5 |
| FTD | Inc-DNAJC19-8:1 | SEQ5867 | 4.E-05 | 0.6 | DLB | Inc-TEKT2-1:2 | SEQ4568 | 2.E-03 | 1.4 |
| DLB | Inc-DNAlCS-1:3 | SEQ5372 | 3.E-04 | 1.4 | DLB | Inc-TELO2-3:2 | SEQ2887 | 4.E-07 | 2.1 |
| FTD | Inc-DNAlCSB-1:3 | SEQ5405 | 3.E-04 | 0.6 | MCI | Inc-TELO2-3:2 | SEQ2887 | 1.E-05 | 2.0 |
| DLB | Inc-DNAJC7-1:1 | SEQ4518 | 9.E-06 | 1.6 | DLB | Inc-TELO2-3:3 | SEQ5003 | 4.E-06 | 1.7 |
| All AD | Inc-DNAJC7-1:1 | SEQ4518 | 5.E-02 | 1.1 | MCI | Inc-TELO2-3:3 | SEQ5003 | 2.E-05 | 1.6 |
| DLB | Inc-DNAL1-2:1 | SEQ4509 | 2.E-03 | 1.5 | FTD | Inc-TELO2-3:3 | SEQ5003 | 8.E-04 | 1.3 |
| MCI | Inc-DNAL4-4:1 | SEQ3095 | 4.E-05 | 1.7 | FTD | Inc-TENM1-6:1 | SEQ3849 | 1.E-05 | 0.5 |
| DLB | Inc-DNAL4-4:1 | SEQ3095 | 1. E-04 | 1.8 | miAD | Inc-TENM1-6:1 | SEQ3849 | 1. E-03 | 0.6 |
| DLB | Inc-DNASE1L2-2:11 | SEQ5947 | 9.E-06 | 1.7 | All AD | Inc-TENM1-6:1 | SEQ3849 | 3.E-03 | 0.6 |
| DLB | Inc-DNASE1L2-2:12 | SEQ4422 | 2.E-04 | 1.7 | msAD | Inc-TENM1-6:1 | SEQ3849 | 3.E-02 | 0.6 |
| MCI | Inc-DNASE1L2-2:12 | SEQ4422 | 2.E-03 | 1.4 | All AD | Inc-TEX37-1:1 | SEQ2655 | 4.E-02 | 1.2 |
| FTD | Inc-DOCK11-1:1 | SEQ5358 | 4.E-04 | 0.7 | All AD | Inc-TEX37-1:2 | SEQ2654 | 4.E-02 | 1.2 |
| FTD | Inc-DOCK7-7:1 | SEQ0015 | 3.E-05 | 0.6 | DLB | Inc-TFDP2-5:1 | SEQ4709 | 1. E-03 | 1.5 |
| DLB | Inc-DOLPP1-3:1 | SEQ4598 | 1. E-04 | 1.6 | DLB | Inc-TFEC-7:1 | SEQ4248 | 8.E-05 | 1.4 |
| MCI | Inc-DOLPP1-3:1 | SEQ4598 | 2.E-03 | 1.8 | MCI | Inc-TFEC-7:1 | SEQ4248 | 1.E-03 | 1.3 |
| FTD | Inc-DPP4-10:1 | SEQ5512 | 2.E-04 | 0.6 | All AD | Inc-TFEC-7:1 | SEQ4248 | 4.E-03 | 1.2 |
| DLB | Inc-DPP7-1:1 | SEQ4708 | 1.E-03 | 1.5 | msAD | Inc-TFEC-7:1 | SEQ4248 | 8.E-03 | 1.2 |
| DLB | Inc-DR1-2:1 | SEQ4684 | 1.E-03 | 1.4 | miAD | Inc-TFEC-7:1 | SEQ4248 | 9.E-03 | 1.2 |
| FTD | Inc-DR1-2:2 | SEQ5300 | 4.E-04 | 0.8 | DLB | Inc-TFPT-1:1 | SEQ4694 | 1. E-03 | 1.4 |
| miAD | Inc-DRD2-1:7 | SEQ3704 | 1.E-02 | 0.6 | FTD | Inc-TGFBR2-1:1 | SEQ5419 | 3.E-04 | 0.7 |
| All AD | Inc-DRD2-1:7 | SEQ3704 | 1.E-02 | 0.6 | FTD | Inc-TGM5-1:3 | SEQ5787 | 8.E-08 | 0.5 |
| msAD | Inc-DRD2-1:7 | SEQ3704 | 5.E-02 | 0.7 | MCI | Inc-TGM5-1:3 | SEQ5787 | 6.E-05 | 0.6 |
| DLB | Inc-DRD5-23:2 | SEQ5314 | 4.E-04 | 1.4 | All AD | Inc-TGM5-1:3 | SEQ5787 | 5.E-02 | 0.7 |
| msAD | Inc-DRD5-29:1 | SEQ3847 | 3.E-02 | 1.6 | FTD | Inc-TGM5-1:5 | SEQ6001 | 3.E-07 | 4.1 |
| DLB | Inc-DRICH1-3:10 | SEQ4972 | 1. E-04 | 1.7 | DLB | Inc-TGM6-4:1 | SEQ3919 | 1.E-03 | 1.5 |
| MCI | Inc-DRICH1-3:10 | SEQ4972 | 8.E-04 | 1.7 | All AD | Inc-TG M6-4:1 | SEQ3919 | 2.E-02 | 1.2 |
| FTD | Inc-DRICH1-3:9 | SEQ4454 | 6.E-04 | 1.6 | msAD | Inc-TG M6-4:1 | SEQ3919 | 3.E-02 | 1.2 |
| DLB | Inc-DRICH1-3:9 | SEQ4454 | 7.E-04 | 1.8 | All AD | Inc-TGS1-1:2 | SEQ5789 | 4.E-02 | 0.6 |
| MCI | Inc-DRICH1-3:9 | SEQ4454 | 2.E-03 | 1.7 | msAD | Inc-THBS1-7:1 | SEQ3779 | 4.E-02 | 1.3 |
| DLB | Inc-DSCR3-2:2 | SEQ4408 | 1. E-04 | 1.5 | DLB | Inc-THTPA-2:27 | SEQ5732 | 9.E-05 | 1.5 |
| MCI | Inc-DSCR3-2:2 | SEQ4408 | 2.E-03 | 1.4 | DLB | Inc-THTPA-2:28 | SEQ5733 | 9.E-05 | 1.5 |
| FTD | Inc-DSCR8-4:1 | SEQ4538 | 2.E-04 | 0.5 | DLB | Inc-THTPA-2:33 | SEQ5731 | 9.E-05 | 1.5 |
| All AD | Inc-DSCR8-4:1 | SEQ4538 | 3.E-02 | 0.6 | FTD | Inc-THUMPD2-2:1 | SEQ3837 | 3.E-04 | 0.5 |
| FTD | Inc-DTNBP1-14:1 | SEQ4539 | 2.E-05 | 0.6 | miAD | Inc-THUMPD2-2:1 | SEQ3837 | 9.E-03 | 0.6 |
| All AD | Inc-DTNBP1-14:1 | SEQ4539 | 3.E-02 | 0.7 | All AD | Inc-THUMPD2-2:1 | SEQ3837 | 9.E-03 | 0.6 |
| msAD | Inc-DTNBP1-5:22 | SEQ3658 | 5.E-02 | 1.5 | msAD | Inc-THUMPD2-2:1 | SEQ3837 | 3.E-02 | 0.8 |
| DLB | Inc-DTYMK-1:2 | SEQ5043 | 4.E-05 | 1.7 | FTD | Inc-THUMPD3-6:1 | SEQ4222 | 1. E-06 | 0.6 |
| MCI | Inc-DTYMK-1:2 | SEQ5043 | 7.E-04 | 1.7 | miAD | Inc-THUMPD3-6:1 | SEQ4222 | 1. E-02 | 0.7 |
| DLB | Inc-DUSP28-1:1 | SEQ4846 | 9.E-04 | 1.5 | DLB | Inc-TICAM 1-1:2 | SEQ5514 | 4.E-06 | 1.4 |
| FTD | Inc-DUXA-4:1 | SEQ2603 | 5.E-07 | 0.5 | MCI | Inc-TICAM 1-1:2 | SEQ5514 | 2.E-04 | 1.3 |
| All AD | Inc-DUXA-4:1 | SEQ2603 | 4.E-02 | 0.7 | All AD | Inc-TICAM 1-1:2 | SEQ5514 | 3.E-02 | 1.1 |
| DLB | Inc-DYNLL2-3:1 | SEQ3803 | 2.E-05 | 1.4 | DLB | Inc-TIGD1-1:16 | SEQ5522 | 2.E-04 | 1.4 |
| All AD | Inc-DYNLL2-3:1 | SEQ3803 | 4.E-02 | 1.1 | FTD | Inc-TIGD4-3:1 | SEQ5397 | 3.E-04 | 0.6 |
| msAD | Inc-DYNLL2-3:1 | SEQ3803 | 4.E-02 | 1.1 | DLB | Inc-TIGD5-1:1 | SEQ2945 | 6.E-05 | 1.7 |
| MCI | Inc-DYNLL2-4:1 | SEQ4868 | 9.E-04 | 1.6 | All AD | Inc-TIGD5-1:1 | SEQ2945 | 3.E-02 | 1.2 |
| DLB | Inc-DYNLT1-1:1 | SEQ4431 | 2.E-03 | 1.5 | msAD | Inc-TIGD5-1:1 | SEQ2945 | 4.E-02 | 1.2 |
| FTD | Inc-DYSF-2:2 | SEQ5779 | 7.E-05 | 0.6 | All AD | Inc-TIMM9-4:1 | SEQ4177 | 3.E-03 | 1.8 |
| FTD | Inc-DYSF-4:1 | SEQ3751 | 7.E-05 | 0.6 | miAD | Inc-TIMM9-4:1 | SEQ4177 | 4.E-03 | 2.0 |
| miAD | Inc-DYSF-4:1 | SEQ3751 | 2.E-03 | 0.7 | msAD | Inc-TIMM9-4:1 | SEQ4177 | 1. E-02 | 1.6 |
| All AD | Inc-DYSF-4:1 | SEQ3751 | 5.E-03 | 0.7 | All AD | Inc-TLCD1-1:2 | SEQ5796 | 4.E-02 | 1.1 |
| msAD | Inc-DYSF-4:1 | SEQ3751 | 4.E-02 | 0.7 | DLB | Inc-TLDC2-4:1 | SEQ0555 | 9.E-05 | 1.7 |
| MCI | Inc-EBLN1-1:1 | SEQ3983 | 1. E-04 | 1.7 | MCI | Inc-TLR10-2:1 | SEQ4427 | 2.E-03 | 1.5 |
| FTD | Inc-EBLN1-1:1 | SEQ3983 | 7.E-04 | 1.7 | FTD | Inc-TLR1-1:1 | SEQ5944 | 3.E-08 | 0.4 |
| DLB | Inc-EBLN1-1:1 | SEQ3983 | 9.E-04 | 1.8 | MCI | Inc-TLR1-1:1 | SEQ5944 | 4.E-07 | 0.4 |
| msAD | Inc-EBLN1-1:1 | SEQ3983 | 2.E-02 | 1.3 | DLB | Inc-TLR1-1:1 | SEQ5944 | 9.E-06 | 0.4 |
| All AD | Inc-EBLN1-1:2 | SEQ4545 | 4.E-02 | 1.3 | FTD | Inc-TLR1-1:4 | SEQ3887 | 7.E-07 | 0.5 |
| All AD | Inc-EBPL-1:1 | SEQ4546 | 3.E-02 | 0.8 | All AD | Inc-TLR1-1:4 | SEQ3887 | 2.E-02 | 0.7 |
| miAD | Inc-ECE1-1:1 | SEQ4182 | 1.E-02 | 0.6 | msAD | Inc-TLR1-1:4 | SEQ3887 | 3.E-02 | 0.7 |
| All AD | Inc-ECE1-1:1 | SEQ4182 | 1.E-02 | 0.7 | MCI | Inc-TMA16-1:1 | SEQ4896 | 8.E-04 | 0.6 |
| DLB | Inc-ECSCR-2:5 | SEQ5431 | 8.E-05 | 1.6 | FTD | Inc-TMA16-1:1 | SEQ4896 | 9.E-04 | 0.6 |
| MCI | Inc-ECSCR-2:5 | SEQ5431 | 3.E-04 | 1.4 | FTD | Inc-TMCS-1:1 | SEQ4737 | 8.E-06 | 0.7 |
| MCI | Inc-EDC3-2:10 | SEQ5714 | 1. E-04 | 3.1 | MCI | Inc-TMC5-1:1 | SEQ4737 | 1. E-04 | 0.7 |
| MCI | Inc-EDC3-2:16 | SEQ4760 | 1.E-03 | 1.5 | DLB | Inc-TMC5-1:1 | SEQ4737 | 1. E-03 | 0.8 |
| All AD | Inc-EDEM3-7:3 | SEQ0272 | 3.E-02 | 0.7 | All AD | Inc-TMC5-1:1 | SEQ4737 | 3.E-02 | 0.9 |
| msAD | Inc-EDEM3-7:3 | SEQ0272 | 5.E-02 | 0.7 | DLB | Inc-TMEM105-1:1 | SEQ4951 | 8.E-04 | 1.5 |
| DLB | Inc-EFCAB12-2:16 | SEQ5894 | 2.E-05 | 1.7 | DLB | Inc-TMEM109-2:1 | SEQ5133 | 2.E-04 | 1.9 |
| DLB | Inc-EFCAB12-2:4 | SEQ4612 | 1.E-03 | 1.3 | MCI | Inc-TMEM109-2:1 | SEQ5133 | 6.E-04 | 1.6 |
| DLB | Inc-EFCAB12-2:8 | SEQ4436 | 2.E-03 | 1.5 | DLB | Inc-TMEM120B-4:2 | SEQ4586 | 2.E-03 | 1.6 |
| DLB | Inc-EFCAB8-1:3 | SEQ3380 | 5. E-04 | 1.4 | DLB | Inc-TMEM120B-4:4 | SEQ4853 | 9.E-04 | 1.5 |
| DLB | Inc-EFCAB9-5:2 | SEQ4496 | 2.E-03 | 1.4 | DLB | Inc-TMEM121-2:15 | SEQ4057 | 2.E-03 | 2.3 |
| FTD | Inc-EFHB-6:1 | SEQ4989 | 8.E-04 | 0.5 | All AD | Inc-TMEM121-2:15 | SEQ4057 | 1. E-02 | 1.6 |
| FTD | Inc-EFR3A-6:1 | SEQ5956 | 7.E-06 | 0.6 | msAD | Inc-TMEM121-2:15 | SEQ4057 | 2.E-02 | 1.6 |
| MCI | Inc-EGFL7-3:1 | SEQ4645 | 1.E-03 | 1.6 | DLB | Inc-TMEM121-2:16 | SEQ4214 | 8.E-04 | 1.8 |
| miAD | Inc-EGR2-5:1 | SEQ4460 | 2.E-03 | 0.6 | All AD | Inc-TMEM121-2:16 | SEQ4214 | 3.E-03 | 1.4 |
| All AD | Inc-EGR2-5:1 | SEQ4460 | 6.E-03 | 0.6 | miAD | Inc-TMEM121-2:16 | SEQ4214 | 3.E-03 | 1.5 |
| DLB | Inc-EIF1AD-1:1 | SEQ2653 | 5. E-04 | 1.4 | msAD | Inc-TMEM121-2:16 | SEQ4214 | 1. E-02 | 1.3 |
| All AD | Inc-EIF2AK3-26:2 | SEQ4334 | 2.E-03 | 1.3 | DLB | Inc-TMEM121-2:17 | SEQ4379 | 5. E-04 | 3.6 |
| msAD | Inc-EIF2AK3-26:2 | SEQ4334 | 4.E-03 | 1.1 | All AD | Inc-TMEM121-2:17 | SEQ4379 | 6.E-04 | 2.3 |
| miAD | Inc-EIF2AK3-26:2 | SEQ4334 | 6.E-03 | 1.5 | msAD | Inc-TMEM121-2:17 | SEQ4379 | 1. E-03 | 2.5 |
| DLB | Inc-EIF2AK3-4:58 | SEQ4358 | 1.E-03 | 3.1 | miAD | Inc-TMEM121-2:17 | SEQ4379 | 3.E-03 | 2.1 |
| miAD | Inc-EIF2AK3-4:58 | SEQ4358 | 4.E-03 | 1.9 | msAD | Inc-TMEM121-2:19 | SEQ3733 | 4.E-02 | 1.5 |
| All AD | Inc-EIF2AK3-4:58 | SEQ4358 | 2.E-02 | 1.7 | miAD | Inc-TMEM121-2:20 | SEQ4041 | 3.E-03 | 1.7 |
| DLB | Inc-EIF2AK3-4:59 | SEQ4232 | 2.E-04 | 2.8 | All AD | Inc-TMEM121-2:20 | SEQ4041 | 4.E-03 | 1.7 |
| FTD | Inc-EIF2AK3-4:59 | SEQ4232 | 6.E-04 | 2.8 | msAD | Inc-TMEM121-2:20 | SEQ4041 | 2.E-02 | 1.6 |
| msAD | Inc-EIF2AK3-4:59 | SEQ4232 | 1.E-02 | 1.7 | All AD | Inc-TMEM121-2:21 | SEQ5806 | 4.E-02 | 1.4 |
| All AD | Inc-EIF2AK3-4:59 | SEQ4232 | 1.E-02 | 1.6 | miAD | Inc-TMEM132D-5:1 | SEQ4212 | 1. E-02 | 0.7 |
| DLB | Inc-EIF2AK3-4:65 | SEQ3873 | 4.E-05 | 2.4 | All AD | Inc-TMEM132D-5:1 | SEQ4212 | 2.E-02 | 0.8 |
| MCI | Inc-EIF2AK3-4:65 | SEQ3873 | 1.E-03 | 1.8 | All AD | Inc-TMEM140-1:2 | SEQ5808 | 3.E-02 | 0.7 |
| All AD | Inc-EIF2AK3-4:65 | SEQ3873 | 1.E-02 | 1.1 | FTD | Inc-TMEM155-3:1 | SEQ3556 | 7.E-06 | 0.5 |
| msAD | Inc-EIF2AK3-4:65 | SEQ3873 | 3.E-02 | 1.1 | MCI | Inc-TMEM155-3:1 | SEQ3556 | 2.E-05 | 0.5 |
| miAD | Inc-EIF2AK3-4:75 | SEQ4250 | 9.E-03 | 1.4 | DLB | Inc-TMEM160-1:1 | SEQ5181 | 5. E-04 | 1.4 |
| All AD | Inc-EIF2AK3-4:75 | SEQ4250 | 1.E-02 | 1.3 | MCI | Inc-TMEM167B-1:2 | SEQ4004 | 7.E-07 | 2.4 |
| All AD | Inc-EIF2AK3-4:77 | SEQ4571 | 4.E-02 | 1.2 | FTD | Inc-TMEM167B-1:2 | SEQ4004 | 2.E-06 | 2.0 |
| MCI | Inc-EIF2B1-1:1 | SEQ2595 | 2.E-05 | 1.5 | DLB | Inc-TMEM167B-1:2 | SEQ4004 | 1. E-04 | 2.1 |
| FTD | Inc-EIF2B1-1:1 | SEQ2595 | 3.E-04 | 1.3 | miAD | Inc-TMEM167B-1:2 | SEQ4004 | 4.E-03 | 1.3 |
| All AD | Inc-EIF2B1-1:1 | SEQ2595 | 3.E-02 | 1.2 | All AD | Inc-TMEM167B-1:2 | SEQ4004 | 4.E-03 | 1.3 |
| DLB | Inc-EIF2D-3:1 | SEQ4850 | 9.E-04 | 1.5 | msAD | Inc-TMEM167B-1:2 | SEQ4004 | 2.E-02 | 1.2 |
| FTD | Inc-EIF2S3-1:1 | SEQ5351 | 4.E-04 | 0.6 | MCI | Inc-TMEM167B-1:3 | SEQ5635 | 1. E-06 | 2.2 |
| DLB | Inc-EIF3 B-2:3 | SEQ3940 | 1.E-05 | 1.8 | FTD | Inc-TMEM167B-1:3 | SEQ5635 | 2.E-05 | 1.7 |
| MCI | Inc-EIF3 B-2:3 | SEQ3940 | 2.E-05 | 1.5 | DLB | Inc-TMEM167B-1:3 | SEQ5635 | 2.E-04 | 1.9 |
| miAD | Inc-EIF3 B-2:3 | SEQ3940 | 4.E-04 | 1.3 | All AD | Inc-TMEM167B-1:3 | SEQ5635 | 2.E-02 | 1.2 |
| FTD | Inc-EIF3 B-2:3 | SEQ3940 | 8.E-04 | 1.3 | MCI | Inc-TMEM167B-1:4 | SEQ5541 | 3.E-06 | 2.1 |
| All AD | Inc-EIF3 B-2:3 | SEQ3940 | 2.E-03 | 1.2 | FTD | Inc-TMEM167B-1:4 | SEQ5541 | 2.E-04 | 1.7 |
| msAD | Inc-EIF3 B-2:3 | SEQ3940 | 3.E-02 | 1.2 | DLB | Inc-TMEM167B-1:4 | SEQ5541 | 2.E-04 | 2.0 |
| miAD | Inc-EIF4E3-2:1 | SEQ4132 | 1.E-02 | 0.6 | All AD | Inc-TMEM167B-1:4 | SEQ5541 | 4.E-02 | 1.2 |
| All AD | Inc-EIF4E3-2:1 | SEQ4132 | 1.E-02 | 0.7 | FTD | Inc-TMEM167B-1:5 | SEQ3821 | 2.E-06 | 1.9 |
| All AD | Inc-EIF4E3-2:2 | SEQ4579 | 3.E-02 | 0.7 | MCI | Inc-TMEM167B-1:5 | SEQ3821 | 6.E-06 | 2.4 |
| DLB | Inc-EIF4ENIF1-3:1 | SEQ3788 | 3.E-04 | 1.6 | DLB | Inc-TMEM167B-1:5 | SEQ3821 | 2.E-04 | 2.2 |
| MCI | Inc-EIF4ENIF1-3:1 | SEQ3788 | 2.E-03 | 1.4 | All AD | Inc-TMEM167B-1:5 | SEQ3821 | 1. E-02 | 1.2 |
| msAD | Inc-EIF4ENIF1-3:1 | SEQ3788 | 4.E-02 | 1.2 | miAD | Inc-TMEM167B-1:5 | SEQ3821 | 1. E-02 | 1.3 |
| All AD | Inc-EIF4G3-4:1 | SEQ4581 | 3.E-02 | 0.8 | msAD | Inc-TMEM167B-1:5 | SEQ3821 | 4.E-02 | 1.2 |
| MCI | Inc-EIF5-2:1 | SEQ2571 | 1.E-05 | 1.7 | DLB | Inc-TMEM173-3:3 | SEQ5194 | 5. E-04 | 1.6 |
| DLB | Inc-EIF5-2:1 | SEQ2571 | 5.E-05 | 1.9 | FTD | Inc-TMEM178A-5:1 | SEQ4104 | 1. E-04 | 0.5 |
| FTD | Inc-EIF5-2:1 | SEQ2571 | 1. E-04 | 1.4 | miAD | Inc-TMEM178A-5:1 | SEQ4104 | 1. E-02 | 0.6 |
| All AD | Inc-EIFS-2:1 | SEQ2571 | 2.E-02 | 1.1 | All AD | Inc-TMEM178A-5:1 | SEQ4104 | 3.E-02 | 0.7 |
| msAD | Inc-EIFS-2:1 | SEQ2571 | 3.E-02 | 1.1 | FTD | Inc-TMEM178A-6:5 | SEQ3797 | 3.E-05 | 0.5 |
| FTD | Inc-ELF2-5:2 | SEQ4109 | 1. E-04 | 0.6 | miAD | Inc-TMEM178A-6:5 | SEQ3797 | 5.E-03 | 0.5 |
| miAD | Inc-ELF2-5:2 | SEQ4109 | 1.E-02 | 0.7 | All AD | Inc-TMEM178A-6:5 | SEQ3797 | 7.E-03 | 0.6 |
| All AD | Inc-ELF2-5:2 | SEQ4109 | 3.E-02 | 0.7 | msAD | Inc-TMEM178A-6:5 | SEQ3797 | 4.E-02 | 0.6 |
| DLB | Inc-ELFN1-3:2 | SEQ4585 | 3.E-05 | 1.7 | msAD | Inc-TMEM183A-1:1 | SEQ3991 | 2.E-02 | 1.2 |
| All AD | Inc-ELFN1-3:2 | SEQ4585 | 4.E-02 | 1.2 | All AD | Inc-TMEM185B-1:6 | SEQ5817 | 5.E-02 | 0.8 |
| DLB | Inc-ELFN1-6:2 | SEQ4594 | 2.E-04 | 1.9 | msAD | Inc-TMEM185B-12:2 | SEQ4025 | 2.E-02 | 0.5 |
| MCI | Inc-ELFN1-6:2 | SEQ4594 | 2.E-03 | 1.7 | All AD | Inc-TMEM185B-12:2 | SEQ4025 | 2.E-02 | 0.6 |
| MCI | Inc-ELL3-1:2 | SEQ4383 | 2.E-06 | 1.7 | All AD | Inc-TMEM185B-12:3 | SEQ4226 | 8.E-03 | 0.6 |
| FTD | Inc-ELL3-1:2 | SEQ4383 | 8.E-06 | 1.6 | msAD | Inc-TMEM185B-12:3 | SEQ4226 | 1. E-02 | 0.5 |
| DLB | Inc-ELL3-1:2 | SEQ4383 | 9.E-05 | 1.7 | msAD | Inc-TMEM186-1:2 | SEQ2702 | 2.E-02 | 1.3 |
| miAD | Inc-ELL3-1:2 | SEQ4383 | 4.E-04 | 1.3 | DLB | Inc-TMEM202-2:2 | SEQ5373 | 3.E-04 | 1.5 |
| All AD | Inc-ELL3-1:2 | SEQ4383 | 4.E-04 | 1.3 | FTD | 3 zTMEM206- | SEQ3714 | 4.E-05 | 0.6 |
| msAD | Inc-ELL3-1:2 | SEQ4383 | 3.E-03 | 1.3 | miAD | Inc-TMEM206-3:2 | SEQ3714 | 1. E-02 | 0.7 |
| FTD | Inc-EMC3-2:1 | SEQ4593 | 2.E-04 | 0.7 | All AD | Inc-TMEM206-3:2 | SEQ3714 | 1. E-02 | 0.7 |
| All AD | Inc-EMC3-2:1 | SEQ4593 | 3.E-02 | 0.8 | msAD | Inc-TMEM206-3:2 | SEQ3714 | 4.E-02 | 0.8 |
| FTD | Inc-EMCN-4:1 | SEQ4137 | 1. E-04 | 0.6 | All AD | Inc-TMEM212-5:1 | SEQ5819 | 4.E-02 | 0.8 |
| miAD | Inc-EMCN-4:1 | SEQ4137 | 1.E-02 | 0.7 | DLB | Inc-TMEM231-2:1 | SEQ5792 | 6.E-05 | 1.6 |
| All AD | Inc-EMCN-4:1 | SEQ4137 | 3.E-02 | 0.8 | DLB | Inc-TMEM234-4:1 | SEQ5877 | 3.E-05 | 1.6 |
| All AD | Inc-EME2-1:1 | SEQ3682 | 2.E-02 | 1.2 | DLB | Inc-TMEM240-1:1 | SEQ4946 | 8.E-04 | 1.5 |
| msAD | Inc-EME2-1:1 | SEQ3682 | 5.E-02 | 1.2 | All AD | Inc-TMEM255B-5:13 | SEQ5820 | 3.E-02 | 1.5 |
| MCI | Inc-EMG1-1:1 | SEQ4928 | 8.E-04 | 1.4 | FTD | Inc-TMEM30B-9:1 | SEQ4108 | 6.E-11 | 0.3 |
| DLB | Inc-EMG1-1:2 | SEQ5636 | 2.E-04 | 1.9 | MCI | Inc-TMEM30B-9:1 | SEQ4108 | 7.E-06 | 0.4 |
| All AD | Inc-EML6-6:1 | SEQ3684 | 3.E-02 | 1.2 | DLB | Inc-TMEM30B-9:1 | SEQ4108 | 2.E-03 | 0.4 |
| msAD | Inc-EML6-6:1 | SEQ3684 | 5.E-02 | 1.2 | miAD | Inc-TMEM30B-9:1 | SEQ4108 | 1. E-02 | 0.6 |
| FTD | Inc-EMX2-3:4 | SEQ5359 | 4.E-04 | 0.7 | FTD | Inc-TMEM43-1:4 | SEQ5285 | 4.E-04 | 0.5 |
| DLB | Inc-ENC1-5:1 | SEQ2244 | 2.E-03 | 1.7 | DLB | Inc-TMEM50B-4:2 | SEQ4870 | 9.E-04 | 1.7 |
| All AD | Inc-ENOPH1-3:3 | SEQ3381 | 3.E-03 | 1.2 | All AD | Inc-TMEM63A-2:1 | SEQ5822 | 3.E-02 | 1.2 |
| miAD | Inc-ENOPH1-3:3 | SEQ3381 | 3.E-03 | 1.3 | DLB | Inc-TMEM88B-6:1 | SEQ5055 | 2.E-05 | 3.8 |
| msAD | Inc-ENOPH1-3:3 | SEQ3381 | 1.E-02 | 1.2 | MCI | Inc-TMEM88B-6:1 | SEQ5055 | 7.E-04 | 2.7 |
| DLB | Inc-EPHA1-1:4 | SEQ5276 | 5. E-04 | 1.7 | FTD | Inc-TMTC3-13:11 | SEQ3765 | 2.E-05 | 0.6 |
| DLB | Inc-EPHA6-7:1 | SEQ4574 | 1.E-05 | 1.7 | miAD | Inc-TMTC3-13:11 | SEQ3765 | 9.E-03 | 0.7 |
| MCI | Inc-EPHA6-7:1 | SEQ4574 | 2.E-03 | 1.5 | All AD | Inc-TMTC3-13:11 | SEQ3765 | 1. E-02 | 0.7 |
| FTD | Inc-EPHB3-5:1 | SEQ4809 | 1.E-03 | 0.7 | msAD | Inc-TMTC3-13:11 | SEQ3765 | 4.E-02 | 0.7 |
| DLB | Inc-EPM2AIP1-5:2 | SEQ4648 | 1.E-03 | 1.7 | DLB | Inc-TMUB2-1:11 | SEQ5664 | 1.E-04 | 1.5 |
| FTD | Inc-EPN2-3:2 | SEQ4005 | 2.E-06 | 2.9 | DLB | Inc-TMUB2-2:1 | SEQ5814 | 5.E-05 | 1.5 |
| DLB | Inc-EPN2-3:2 | SEQ4005 | 2.E-04 | 2.5 | All AD | Inc-TNFAIP6-5:1 | SEQ5824 | 5.E-02 | 0.7 |
| MCI | Inc-EPN2-3:2 | SEQ4005 | 5.E-04 | 2.2 | DLB | Inc-TNFAIP8L1-2:1 | SEQ4950 | 8.E-04 | 1.5 |
| msAD | Inc-EPN2-3:2 | SEQ4005 | 2.E-02 | 1.2 | All AD | Inc-TNFAIP8L1-2:1 | SEQ4950 | 4.E-02 | 1.2 |
| All AD | Inc-EPN2-3:2 | SEQ4005 | 2.E-02 | 1.2 | FTD | Inc-TNFRSF11A-5:1 | SEQ5825 | 8.E-08 | 0.5 |
| FTD | Inc-EPN2-3:3 | SEQ5074 | 7.E-04 | 1.8 | All AD | Inc-TNFRSF11A-5:1 | SEQ5825 | 2.E-02 | 0.7 |
| FTD | Inc-ERAP2-4:3 | SEQ5601 | 2.E-04 | 0.6 | DLB | Inc-TNFRSF13C-1:1 | SEQ4142 | 3.E-04 | 2.2 |
| DLB | Inc-ERCC6L2-14:1 | SEQ3383 | 2.E-04 | 1.5 | All AD | Inc-TNFRSF13C-1:1 | SEQ4142 | 3.E-03 | 1.5 |
| MCI | Inc-ERCC6L2-14:1 | SEQ3383 | 8.E-04 | 1.4 | msAD | Inc-TNFRSF13C-1:1 | SEQ4142 | 4.E-03 | 1.6 |
| miAD | Inc-ERCC6L2-14:1 | SEQ3383 | 8.E-03 | 1.2 | miAD | Inc-TNFRSF13C-1:1 | SEQ4142 | 1.E-02 | 1.4 |
| All AD | Inc-ERCC6L2-14:1 | SEQ3383 | 9.E-03 | 1.2 | miAD | Inc-TNFRSF14-2:7 | SEQ4234 | 1.E-02 | 0.5 |
| msAD | Inc-ERCC6L2-14:1 | SEQ3383 | 4.E-02 | 1.2 | All AD | Inc-TNFRSF14-2:7 | SEQ4234 | 1.E-02 | 0.6 |
| FTD | Inc-ERRFI1-3:1 | SEQ5607 | 2.E-04 | 0.7 | MCI | Inc-TNFRSF14-4:2 | SEQ5213 | 4.E-07 | 1.9 |
| DLB | Inc-ERV3-1-1:4 | SEQ4405 | 2.E-03 | 1.3 | FTD | Inc-TNFRSF14-4:2 | SEQ5213 | 5.E-06 | 1.7 |
| FTD | Inc-ERV3-1-2:1 | SEQ3853 | 1.E-04 | 0.6 | miAD | Inc-TNFRSF14-4:2 | SEQ5213 | 4.E-04 | 1.5 |
| miAD | Inc-ERV3-1-2:1 | SEQ3853 | 4.E-03 | 0.7 | DLB | Inc-TNFRSF14-4:2 | SEQ5213 | 5.E-04 | 1.9 |
| All AD | Inc-ERV3-1-2:1 | SEQ3853 | 6.E-03 | 0.7 | All AD | Inc-TNFRSF14-4:2 | SEQ5213 | 3.E-03 | 1.4 |
| msAD | Inc-ERV3-1-2:1 | SEQ3853 | 3.E-02 | 0.8 | msAD | Inc-TNFRSF1A-3:1 | SEQ3977 | 2.E-02 | 0.7 |
| miAD | Inc-ES M 1-3:3 | SEQ4203 | 1.E-02 | 0.6 | All AD | Inc-TNFRSF1A-3:1 | SEQ3977 | 3.E-02 | 0.7 |
| DLB | Inc-ESRP2-4:1 | SEQ4417 | 2.E-03 | 1.4 | DLB | Inc-TNFRSF8-2:1 | SEQ4710 | 1.E-03 | 1.5 |
| DLB | Inc-ESRRB-1:1 | SEQ4575 | 9.E-04 | 1.4 | FTD | Inc-TNFSF4-3:2 | SEQ5063 | 7.E-04 | 0.6 |
| MCI | Inc-ESRRB-1:1 | SEQ4575 | 2.E-03 | 1.5 | All AD | Inc-TNFSF4-3:2 | SEQ5063 | 4.E-02 | 0.7 |
| FTD | Inc-ESRRB-3:1 | SEQ4912 | 9.E-04 | 0.7 | DLB | Inc-TNIP2-1:1 | SEQ4845 | 9.E-04 | 1.4 |
| MCI | Inc-EVL-6:10 | SEQ4872 | 1.E-04 | 1.7 | All AD | Inc-TNRC6A-3:2 | SEQ5831 | 3.E-02 | 1.2 |
| DLB | Inc-EVL-6:10 | SEQ4872 | 9.E-04 | 1.7 | MCI | Inc-TNRC6C-3:1 | SEQ5627 | 2.E-05 | 1.9 |
| FTD | Inc-EVPL-1:1 | SEQ4064 | 1.E-04 | 0.5 | DLB | Inc-TNRC6C-3:1 | SEQ5627 | 2.E-04 | 1.7 |
| All AD | Inc-EVPL-1:1 | SEQ4064 | 5.E-03 | 0.6 | FTD | Inc-TOB1-4:1 | SEQ5647 | 1.E-04 | 0.7 |
| miAD | Inc-EVPL-1:1 | SEQ4064 | 7.E-03 | 0.6 | MCI | Inc-TOB2-2:1 | SEQ3968 | 2.E-04 | 1.4 |
| msAD | Inc-EVPL-1:1 | SEQ4064 | 2.E-02 | 0.7 | DLB | Inc-TOB2-2:1 | SEQ3968 | 2.E-03 | 1.4 |
| DLB | Inc-EVX1-15:1 | SEQ2412 | 4.E-05 | 1.3 | msAD | Inc-TOB2-2:1 | SEQ3968 | 2.E-02 | 1.2 |
| DLB | Inc-EVX2-8:1 | SEQ4082 | 2.E-04 | 1.7 | All AD | Inc-TOMM20-2:22 | SEQ5835 | 4.E-02 | 0.7 |
| miAD | Inc-EVX2-8:1 | SEQ4082 | 1.E-02 | 1.3 | DLB | Inc-TOMM22-2:2 | SEQ4766 | 9.E-04 | 1.4 |
| All AD | Inc-EVX2-8:1 | SEQ4082 | 3.E-02 | 1.2 | MCI | Inc-TOMM22-2:2 | SEQ4766 | 1.E-03 | 1.5 |
| All AD | Inc-EXOSC1-1:2 | SEQ4611 | 5.E-02 | 0.8 | DLB | Inc-TOR1AIP1-2:1 | SEQ2565 | 1.E-03 | 1.7 |
| FTD | Inc-EXOSC6-1:2 | SEQ3300 | 5.E-05 | 0.6 | FTD | Inc-TOX4-4:2 | SEQ2696 | 4.E-05 | 0.6 |
| DLB | Inc-EXOSC6-1:6 | SEQ5218 | 4.E-04 | 2.4 | DLB | Inc-TOX4-4:2 | SEQ2696 | 6.E-04 | 0.7 |
| MCI | Inc-EXOSC6-1:6 | SEQ5218 | 5.E-04 | 2.4 | miAD | Inc-TOX4-4:2 | SEQ2696 | 5.E-03 | 0.7 |
| All AD | Inc-EXOSC8-4:1 | SEQ4615 | 5.E-02 | 0.7 | All AD | Inc-TOX4-4:2 | SEQ2696 | 3.E-02 | 0.8 |
| FTD | Inc-EXTL2-2:1 | SEQ5225 | 5.E-04 | 0.6 | All AD | Inc-TP53BP2-8:2 | SEQ5838 | 3.E-02 | 0.7 |
| FTD | Inc-F2RL2-1:2 | SEQ3827 | 3.E-06 | 0.5 | MCI | Inc-TP53TG5-3:1 | SEQ4914 | 6.E-06 | 1.9 |
| miAD | Inc-F2RL2-1:2 | SEQ3827 | 9.E-03 | 0.7 | DLB | Inc-TP53TG5-3:1 | SEQ4914 | 4.E-05 | 1.8 |
| All AD | Inc-F2RL2-1:2 | SEQ3827 | 9.E-03 | 0.7 | FTD | Inc-TP53TG5-3:1 | SEQ4914 | 9.E-04 | 1.4 |
| msAD | Inc-F2RL2-1:2 | SEQ3827 | 4.E-02 | 0.8 | DLB | Inc-TPBGL-2:1 | SEQ4424 | 2.E-03 | 1.5 |
| DLB | Inc-FAAP100-2:1 | SEQ2299 | 5.E-04 | 1.3 | MCI | Inc-TPBGL-2:1 | SEQ4424 | 2.E-03 | 1.5 |
| MCI | Inc-FAAP20-2:1 | SEQ2889 | 4.E-04 | 1.7 | DLB | Inc-TPCN1-1:1 | SEQ4584 | 4.E-04 | 1.6 |
| DLB | Inc-FAAP20-2:1 | SEQ2889 | 5.E-04 | 1.7 | MCI | Inc-TPCN1-1:1 | SEQ4584 | 2.E-03 | 1.5 |
| DLB | Inc-FAHD2B-1:1 | SEQ5339 | 4.E-04 | 1.8 | FTD | Inc-TRAF5-8:1 | SEQ5763 | 8.E-05 | 0.6 |
| DLB | Inc-FAHD2B-1:2 | SEQ2915 | 2.E-04 | 1.9 | All AD | Inc-TRAF6-3:1 | SEQ5842 | 4.E-02 | 0.8 |
| All AD | Inc-FAM106A-2:10 | SEQ2240 | 7.E-03 | 0.5 | DLB | Inc-TRAPPC2L-2:4 | SEQ4777 | 1. E-03 | 1.6 |
| miAD | Inc-FAM106A-2:10 | SEQ2240 | 8.E-03 | 0.5 | MCI | Inc-TRDMT1-5:1 | SEQ4984 | 9.E-05 | 3.6 |
| msAD | Inc-FAM106A-2:10 | SEQ2240 | 3.E-02 | 0.6 | FTD | Inc-TRDMT1-5:1 | SEQ4984 | 2.E-04 | 2.5 |
| All AD | Inc-FAM106A-2:7 | SEQ4623 | 3.E-02 | 0.6 | DLB | Inc-TRDMT1-5:1 | SEQ4984 | 8.E-04 | 3.1 |
| DLB | Inc-FAM111B-1:3 | SEQ4406 | 2.E-03 | 1.3 | All AD | Inc-TRDMT1-5:1 | SEQ4984 | 4.E-02 | 1.4 |
| FTD | Inc-FAM114A1-1:1 | SEQ4326 | 6.E-08 | 0.5 | FTD | Inc-TREML2-4:1 | SEQ4995 | 8.E-04 | 0.6 |
| MCI | Inc-FAM114A1-1:1 | SEQ4326 | 7.E-04 | 0.6 | DLB | Inc-TREX1-10:5 | SEQ5200 | 5.E-04 | 1.7 |
| miAD | Inc-FAM114A1-1:1 | SEQ4326 | 6.E-03 | 0.6 | miAD | Inc-TREX1-5:1 | SEQ3723 | 1.E-02 | 0.8 |
| All AD | Inc-FAM114A1-1:1 | SEQ4326 | 1.E-02 | 0.7 | All AD | Inc-TREX1-5:1 | SEQ3723 | 1.E-02 | 0.8 |
| All AD | Inc-FAM126B-1:2 | SEQ3668 | 2.E-02 | 0.8 | msAD | Inc-TREX1-5:1 | SEQ3723 | 4.E-02 | 0.8 |
| msAD | Inc-FAM126B-1:2 | SEQ3668 | 5.E-02 | 0.8 | FTD | Inc-TRIL-3:1 | SEQ4347 | 2.E-04 | 0.5 |
| FTD | Inc-FAM131B-2:1 | SEQ3679 | 8.E-05 | 0.5 | miAD | Inc-TRIL-3:1 | SEQ4347 | 5.E-03 | 0.6 |
| miAD | Inc-FAM131B-2:1 | SEQ3679 | 1.E-02 | 0.6 | All AD | Inc-TRIL-3:1 | SEQ4347 | 3.E-02 | 0.7 |
| All AD | Inc-FAM131B-2:1 | SEQ3679 | 1.E-02 | 0.6 | miAD | Inc-TRIL-3:2 | SEQ4206 | 1.E-02 | 0.7 |
| msAD | Inc-FAM131B-2:1 | SEQ3679 | 5.E-02 | 0.6 | All AD | Inc-TRIL-3:2 | SEQ4206 | 2.E-02 | 0.7 |
| FTD | Inc-FAM133B-1:1 | SEQ5226 | 5.E-04 | 0.6 | FTD | Inc-TRIM23-1:1 | SEQ5102 | 1.E-08 | 0.5 |
| FTD | Inc-FAM13C-5:1 | SEQ4372 | 7.E-05 | 0.6 | MCI | Inc-TRIM23-1:1 | SEQ5102 | 6.E-04 | 0.5 |
| miAD | Inc-FAM13C-5:1 | SEQ4372 | 4.E-03 | 0.7 | All AD | Inc-TRIM25-1:1 | SEQ5849 | 1.E-02 | 0.7 |
| All AD | Inc-FAM13C-5:1 | SEQ4372 | 9.E-03 | 0.7 | DLB | Inc-TRIM26-2:27 | SEQ4644 | 1.E-03 | 1.6 |
| DLB | Inc-FAM156A-1:2 | SEQ5434 | 3.E-04 | 1.4 | MCI | Inc-TRIM26-2:53 | SEQ5649 | 1.E-04 | 0.1 |
| DLB | Inc-FAM156A-3:5 | SEQ5881 | 3.E-05 | 1.7 | DLB | Inc-TRIM28-13:2 | SEQ3581 | 2.E-03 | 1.4 |
| FTD | Inc-FAM162A-2:12 | SEQ4634 | 7.E-05 | 0.7 | DLB | Inc-TRIM39-2:1 | SEQ5205 | 5.E-04 | 1.7 |
| All AD | Inc-FAM162A-2:12 | SEQ4634 | 4.E-02 | 0.8 | FTD | Inc-TRIM55-1:14 | SEQ4916 | 9.E-04 | 1.8 |
| FTD | Inc-FAM162A-2:6 | SEQ6002 | 8.E-08 | 0.3 | FTD | Inc-TRIM59-1:1 | SEQ2766 | 8.E-07 | 0.6 |
| FTD | Inc-FAM167A-5:2 | SEQ5898 | 2.E-05 | 0.5 | miAD | Inc-TRIM59-1:1 | SEQ2766 | 1.E-02 | 0.7 |
| DLB | Inc-FAM167B-2:1 | SEQ4453 | 2.E-03 | 1.7 | All AD | Inc-TRIM59-1:1 | SEQ2766 | 2.E-02 | 0.8 |
| DLB | Inc-FAM168A-1:1 | SEQ4858 | 9.E-04 | 1.5 | FTD | Inc-TRIM61-6:1 | SEQ5220 | 2.E-04 | 2.3 |
| FTD | Inc-FAM174A-6:1 | SEQ4639 | 5.E-05 | 0.5 | MCI | Inc-TRIM61-6:1 | SEQ5220 | 5.E-04 | 2.9 |
| All AD | Inc-FAM174A-6:1 | SEQ4639 | 3.E-02 | 0.7 | DLB | Inc-TRIM62-2:1 | SEQ5263 | 5.E-04 | 1.6 |
| All AD | Inc-FAM174A-6:6 | SEQ4640 | 5.E-02 | 0.6 | FTD | Inc-TRIM69-2:8 | SEQ4814 | 1.E-03 | 0.7 |
| FTD | Inc-FAM177B-1:1 | SEQ3388 | 1.E-04 | 0.6 | MCI | Inc-TRIM74-1:6 | SEQ4254 | 7.E-06 | 3.3 |
| miAD | Inc-FAM177B-1:1 | SEQ3388 | 8.E-03 | 0.7 | DLB | Inc-TRIM74-1:6 | SEQ4254 | 2.E-03 | 2.0 |
| All AD | Inc-FAM177B-1:1 | SEQ3388 | 1.E-02 | 0.8 | All AD | Inc-TRIM74-1:6 | SEQ4254 | 6.E-03 | 1.6 |
| DLB | Inc-FAM184B-1:2 | SEQ5791 | 6.E-05 | 1.5 | msAD | Inc-TRIM74-1:6 | SEQ4254 | 9.E-03 | 1.6 |
| DLB | Inc-FAM187B-3:1 | SEQ5109 | 6.E-04 | 1.2 | FTD | Inc-TRMT10B-4:1 | SEQ5609 | 3.E-07 | 0.5 |
| FTD | Inc-FAM192A-3:1 | SEQ4170 | 4.E-05 | 0.6 | MCI | Inc-TRMT10B-4:1 | SEQ5609 | 2.E-04 | 0.6 |
| All AD | Inc-FAM192A-3:1 | SEQ4170 | 3.E-03 | 0.6 | All AD | Inc-TRMT10B-4:1 | SEQ5609 | 2.E-02 | 0.8 |
| miAD | Inc-FAM192A-3:1 | SEQ4170 | 4.E-03 | 0.6 | FTD | Inc-TRMT10C-3:1 | SEQ4398 | 1.E-07 | 0.5 |
| msAD | Inc-FAM192A-3:1 | SEQ4170 | 1.E-02 | 0.6 | MCI | Inc-TRMT10C-3:1 | SEQ4398 | 2.E-03 | 0.7 |
| msAD | Inc-FAM192A-4:1 | SEQ4298 | 7.E-03 | 0.7 | All AD | Inc-TRMT10C-3:1 | SEQ4398 | 2.E-02 | 0.7 |
| All AD | Inc-FAM192A-4:1 | SEQ4298 | 2.E-02 | 0.7 | FTD | Inc-TRMT10C-4:1 | SEQ4992 | 8.E-04 | 0.6 |
| msAD | Inc-FAM192A-4:3 | SEQ4603 | 1.E-03 | 0.7 | MCI | Inc-TRMT1-1:1 | SEQ4158 | 7.E-04 | 1.3 |
| All AD | Inc-FAM192A-4:3 | SEQ4603 | 5.E-03 | 0.7 | miAD | Inc-TRMT1-1:1 | SEQ4158 | 1.E-02 | 1.1 |
| msAD | Inc-FAM192A-4:5 | SEQ4150 | 1.E-02 | 0.7 | All AD | Inc-TRMT1-1:1 | SEQ4158 | 2.E-02 | 1.1 |
| All AD | Inc-FAM192A-4:5 | SEQ4150 | 2.E-02 | 0.7 | All AD | Inc-TRMT61B-5:3 | SEQ5853 | 3.E-02 | 0.8 |
| DLB | Inc-FAM193B-2:1 | SEQ5387 | 3.E-04 | 1.7 | All AD | Inc-TRPC5-3:1 | SEQ5854 | 3.E-02 | 1.2 |
| FTD | Inc-FAM200A-1:1 | SEQ5687 | 1.E-04 | 0.6 | FTD | Inc-TRPC5OS-5:1 | SEQ5597 | 2.E-04 | 0.4 |
| FTD | Inc-FAM200B-3:1 | SEQ3664 | 2.E-04 | 0.7 | DLB | Inc-TRPM5-1:1 | SEQ4867 | 9.E-04 | 1.6 |
| miAD | Inc-FAM200B-3:1 | SEQ3664 | 8.E-03 | 0.7 | DLB | Inc-TRPT1-1:2 | SEQ5701 | 1.E-04 | 1.7 |
| All AD | Inc-FAM200B-3:1 | SEQ3664 | 1.E-02 | 0.7 | DLB | Inc-TRRAP-1:1 | SEQ5570 | 2.E-04 | 1.6 |
| msAD | Inc-FAM200B-3:1 | SEQ3664 | 5.E-02 | 0.7 | DLB | Inc-TRRAP-1:4 | SEQ5571 | 2.E-04 | 1.6 |
| DLB | Inc-FAM207A-7:1 | SEQ5711 | 1.E-04 | 1.9 | MCI | Inc-TRRAP-1:5 | SEQ4747 | 1.E-03 | 1.4 |
| MCI | Inc-FAM208B-5:3 | SEQ3973 | 2.E-05 | 1.6 | DLB | Inc-TRRAP-1:6 | SEQ5628 | 2.E-04 | 1.7 |
| FTD | Inc-FAM208B-5:3 | SEQ3973 | 2.E-05 | 1.5 | FTD | Inc-TSPOAP1-1:1 | SEQ5299 | 4.E-04 | 0.7 |
| DLB | Inc-FAM208B-5:3 | SEQ3973 | 8.E-05 | 1.7 | DLB | Inc-TSPOAP1-1:2 | SEQ5674 | 2.E-05 | 1.8 |
| All AD | Inc-FAM208B-5:3 | SEQ3973 | 2.E-02 | 1.2 | MCI | Inc-TSPOAP1-1:2 | SEQ5674 | 1.E-04 | 1.7 |
| msAD | Inc-FAM208B-5:3 | SEQ3973 | 2.E-02 | 1.2 | DLB | Inc-TSPOAP1-1:4 | SEQ4597 | 2.E-03 | 1.7 |
| FTD | Inc-FAM217A-1:7 | SEQ4988 | 8.E-04 | 0.5 | FTD | Inc-TSPYL5-4:1 | SEQ4804 | 1.E-03 | 0.6 |
| DLB | Inc-FAM231C-7:7 | SEQ5203 | 3.E-04 | 1.7 | All AD | Inc-TSPYL5-4:1 | SEQ4804 | 3.E-02 | 0.7 |
| MCI | Inc-FAM231C-7:7 | SEQ5203 | 5.E-04 | 1.7 | DLB | Inc-TSR3-1:2 | SEQ0339 | 1.E-04 | 1.6 |
| All AD | Inc-FAM3B-3:2 | SEQ4659 | 4.E-02 | 0.8 | MCI | Inc-TSR3-1:2 | SEQ0339 | 3.E-04 | 1.5 |
| All AD | Inc-FAM49B-1:1 | SEQ4660 | 4.E-02 | 0.7 | MCI | Inc-TSSK2-1:1 | SEQ5709 | 4.E-05 | 2.0 |
| FTD | Inc-FAM71D-1:2 | SEQ4895 | 9.E-04 | 0.6 | DLB | Inc-TSSK2-1:1 | SEQ5709 | 1.E-04 | 1.8 |
| DLB | Inc-FAM71E1-2:10 | SEQ5752 | 8.E-05 | 1.5 | DLB | Inc-TSS K3-2:2 | SEQ4428 | 2.E-03 | 1.5 |
| All AD | Inc-FAM71E1-2:9 | SEQ3219 | 1.E-02 | 1.3 | DLB | Inc-TSSK4-3:2 | SEQ5308 | 4.E-04 | 1.4 |
| msAD | Inc-FAM71E1-2:9 | SEQ3219 | 2.E-02 | 1.3 | DLB | Inc-TSSK6-1:2 | SEQ5823 | 2.E-05 | 1.6 |
| miAD | Inc-FAM72B-17:1 | SEQ3884 | 4.E-03 | 0.6 | MCI | Inc-TSSK6-1:2 | SEQ5823 | 5.E-05 | 1.9 |
| All AD | Inc-FAM72B-17:1 | SEQ3884 | 5.E-03 | 0.5 | DLB | Inc-TSTA3-2:1 | SEQ4409 | 2.E-03 | 1.4 |
| msAD | Inc-FAM72B-17:1 | SEQ3884 | 3.E-02 | 0.5 | FTD | Inc-TTC27-10:1 | SEQ5976 | 3.E-06 | 0.6 |
| DLB | Inc-FAM72D-8:1 | SEQ2753 | 3.E-04 | 1.4 | DLB | Inc-TTC4-1:1 | SEQ3776 | 4.E-05 | 1.7 |
| miAD | Inc-FAM84B-20:1 | SEQ3995 | 3.E-03 | 1.4 | msAD | Inc-TTC4-1:1 | SEQ3776 | 4.E-02 | 1.2 |
| All AD | Inc-FAM84B-20:1 | SEQ3995 | 4.E-03 | 1.4 | DLB | Inc-TTC9-3:1 | SEQ5917 | 2.E-05 | 1.7 |
| msAD | Inc-FAM84B-20:1 | SEQ3995 | 2.E-02 | 1.4 | FTD | Inc-TTC9-4:1 | SEQ5224 | 5.E-04 | 0.5 |
| MCI | Inc-FAM84B-21:1 | SEQ4956 | 3.E-04 | 1.5 | DLB | Inc-TUBA1C-1:15 | SEQ4456 | 2.E-03 | 1.8 |
| DLB | Inc-FAM84B-21:1 | SEQ4956 | 8.E-04 | 1.5 | DLB | Inc-TUBA1C-1:8 | SEQ5558 | 2.E-04 | 1.4 |
| DLB | Inc-FAM84B-4:3 | SEQ0083 | 9.E-04 | 1.8 | FTD | Inc-TUBA1C-3:2 | SEQ3773 | 9.E-05 | 0.6 |
| DLB | Inc-FAM84B-6:5 | SEQ4529 | 2.E-03 | 1.7 | miAD | Inc-TUBA1C-3:2 | SEQ3773 | 2.E-03 | 0.6 |
| DLB | Inc-FAM8A1-2:5 | SEQ4622 | 2.E-04 | 1.5 | All AD | Inc-TUBA1C-3:2 | SEQ3773 | 5.E-03 | 0.7 |
| MCI | Inc-FAM8A1-2:5 | SEQ4622 | 1.E-03 | 1.4 | msAD | Inc-TUBA1C-3:2 | SEQ3773 | 4.E-02 | 0.7 |
| DLB | Inc-FAM92A-2:1 | SEQ4503 | 2.E-03 | 1.4 | FTD | Inc-TUBA3C-16:1 | SEQ4801 | 1.E-03 | 0.5 |
| DLB | Inc-FAM92B-4:1 | SEQ5535 | 2.E-04 | 1.7 | DLB | Inc-TUBA3D-1:3 | SEQ4712 | 1.E-03 | 1.5 |
| MCI | Inc-FANCA-3:1 | SEQ4011 | 4.E-05 | 1.5 | All AD | Inc-TUBB2A-7:2 | SEQ3767 | 2.E-02 | 0.8 |
| DLB | Inc-FANCA-3:1 | SEQ4011 | 9.E-05 | 1.6 | msAD | Inc-TUBB2A-7:2 | SEQ3767 | 4.E-02 | 0.8 |
| All AD | Inc-FANCA-3:1 | SEQ4011 | 2.E-02 | 1.2 | All AD | Inc-TUBD1-1:1 | SEQ3961 | 7.E-03 | 0.7 |
| msAD | Inc-FANCA-3:1 | SEQ4011 | 2.E-02 | 1.2 | miAD | Inc-TUBD1-1:1 | SEQ3961 | 8.E-03 | 0.7 |
| DLB | Inc-FANCL-4:1 | SEQ2690 | 7.E-04 | 1.5 | msAD | Inc-TUBD1-1:1 | SEQ3961 | 2.E-02 | 0.8 |
| All AD | Inc-FANCL-4:1 | SEQ2690 | 2.E-02 | 1.2 | MCI | Inc-TUBGCP6-5:4 | SEQ5689 | 9.E-06 | 1.7 |
| msAD | Inc-FANCL-4:1 | SEQ2690 | 3.E-02 | 1.2 | DLB | Inc-TUBGCP6-5:4 | SEQ5689 | 9.E-05 | 1.7 |
| All AD | Inc-FAP-3:1 | SEQ0206 | 2.E-02 | 0.7 | FTD | Inc-TUBGCP6-5:4 | SEQ5689 | 1.E-04 | 1.5 |
| msAD | Inc-FAP-3:1 | SEQ0206 | 4.E-02 | 0.7 | MCI | Inc-TUBGCP6-5:6 | SEQ4865 | 7.E-04 | 1.7 |
| All AD | Inc-FAS-1:1 | SEQ4671 | 3.E-02 | 0.8 | DLB | Inc-TUBGCP6-5:6 | SEQ4865 | 9.E-04 | 1.6 |
| FTD | Inc-FAS-2:2 | SEQ5399 | 3.E-04 | 0.7 | MCI | Inc-TULP2-4:1 | SEQ5890 | 1.E-05 | 1.6 |
| DLB | Inc-FBXL22-1:1 | SEQ3898 | 9.E-04 | 1.4 | DLB | Inc-TULP2-4:1 | SEQ5890 | 2.E-05 | 1.6 |
| MCI | Inc-FBXL22-1:1 | SEQ3898 | 2.E-03 | 1.4 | DLB | Inc-TUSC5-5:2 | SEQ3875 | 1.E-05 | 1.7 |
| All AD | Inc-FBXL22-1:1 | SEQ3898 | 2.E-02 | 1.2 | MCI | Inc-TUSC5-5:2 | SEQ3875 | 1.E-03 | 1.4 |
| msAD | Inc-FBXL22-1:1 | SEQ3898 | 3.E-02 | 1.2 | msAD | Inc-TUSC5-5:2 | SEQ3875 | 3.E-02 | 1.2 |
| DLB | Inc-FBXO11-1:8 | SEQ5237 | 9.E-06 | 1.9 | All AD | Inc-TUSC5-5:2 | SEQ3875 | 4.E-02 | 1.2 |
| MCI | Inc-FBXO11-1:8 | SEQ5237 | 9.E-05 | 1.6 | DLB | Inc-TVP23A-6:1 | SEQ5530 | 2.E-04 | 1.6 |
| FTD | Inc-FBXO11-1:8 | SEQ5237 | 5.E-04 | 1.6 | FTD | Inc-TWSG1-1:1 | SEQ5776 | 7.E-05 | 0.5 |
| miAD | Inc-FBXO11-4:1 | SEQ3717 | 7.E-03 | 1.4 | All AD | Inc-TWSG1-1:1 | SEQ5776 | 3.E-02 | 0.7 |
| All AD | Inc-FBXO11-4:1 | SEQ3717 | 9.E-03 | 1.3 | FTD | Inc-TWSG1-2:1 | SEQ0047 | 6.E-10 | 0.4 |
| msAD | Inc-FBXO11-4:1 | SEQ3717 | 4.E-02 | 1.2 | MCI | Inc-TWSG1-2:1 | SEQ0047 | 1.E-05 | 0.4 |
| All AD | Inc-FBXO28-1:10 | SEQ4679 | 5.E-02 | 0.8 | miAD | Inc-TWSG1-2:1 | SEQ0047 | 2.E-03 | 0.6 |
| FTD | Inc-FBXO28-1:13 | SEQ5785 | 4.E-06 | 0.3 | All AD | Inc-TWSG1-2:1 | SEQ0047 | 3.E-02 | 0.6 |
| MCI | Inc-FBXO28-1:13 | SEQ5785 | 6.E-05 | 0.4 | FTD | Inc-TXLNB-3:4 | SEQ5914 | 2.E-05 | 0.5 |
| FTD | Inc-FBXO30-2:1 | SEQ5683 | 1.E-04 | 0.5 | FTD | Inc-TXLNB-3:6 | SEQ5988 | 2.E-06 | 0.4 |
| FTD | Inc-FBXO45-4:1 | SEQ4199 | 3.E-07 | 2.1 | DLB | Inc-TXNRD2-2:1 | SEQ5328 | 1.E-04 | 1.7 |
| MCI | Inc-FBXO45-4:1 | SEQ4199 | 1.E-05 | 2.6 | MCI | Inc-TXNRD2-2:1 | SEQ5328 | 4.E-04 | 1.6 |
| DLB | Inc-FBXO45-4:1 | SEQ4199 | 1.E-04 | 2.1 | DLB | Inc-TYW 1-7:8 | SEQ5952 | 7.E-06 | 2.0 |
| miAD | Inc-FBXO45-4:1 | SEQ4199 | 2.E-03 | 1.3 | MCI | Inc-U2AF2-1:4 | SEQ5174 | 3.E-04 | 1.3 |
| All AD | Inc-FBXO45-4:1 | SEQ4199 | 2.E-03 | 1.3 | DLB | Inc-U2AF2-1:4 | SEQ5174 | 5.E-04 | 1.4 |
| msAD | Inc-FBXO45-4:1 | SEQ4199 | 1.E-02 | 1.3 | MCI | Inc-UACA-5:1 | SEQ4784 | 2.E-04 | 1.9 |
| DLB | Inc-FBXO47-1:1 | SEQ4419 | 2.E-03 | 1.4 | DLB | Inc-UACA-5:1 | SEQ4784 | 1.E-03 | 1.7 |
| FTD | Inc-FCAR-1:1 | SEQ3850 | 9.E-05 | 0.6 | FTD | Inc-UAP1-4:1 | SEQ4669 | 5.E-06 | 0.6 |
| All AD | Inc-FCAR-1:1 | SEQ3850 | 1.E-02 | 0.7 | MCI | Inc-UAP1-4:1 | SEQ4669 | 1.E-03 | 0.6 |
| miAD | Inc-FCAR-1:1 | SEQ3850 | 1.E-02 | 0.7 | All AD | Inc-UAP1-4:1 | SEQ4669 | 2.E-02 | 0.8 |
| msAD | Inc-FCAR-1:1 | SEQ3850 | 3.E-02 | 0.7 | DLB | Inc-UBAC1-2:2 | SEQ3540 | 1.E-03 | 2.3 |
| All AD | Inc-FCGR2B-5:1 | SEQ4688 | 3.E-02 | 0.7 | DLB | Inc-UBALD1-1:1 | SEQ5739 | 9.E-05 | 1.9 |
| All AD | Inc-FCGR3A-4:1 | SEQ4689 | 3.E-02 | 0.8 | DLB | Inc-UBAP2L-1:1 | SEQ4401 | 2.E-03 | 1.3 |
| FTD | Inc-FEM1B-4:1 | SEQ2624 | 6.E-10 | 0.3 | DLB | Inc-UBE2D2-2:1 | SEQ4832 | 5.E-05 | 1.6 |
| MCI | Inc-FEM1B-4:1 | SEQ2624 | 9.E-06 | 0.4 | MCI | Inc-UBE2D2-2:1 | SEQ4832 | 9.E-04 | 1.4 |
| All AD | Inc-FEM1B-4:1 | SEQ2624 | 1.E-02 | 0.6 | msAD | Inc-UBL5-1:4 | SEQ3948 | 3.E-02 | 1.2 |
| msAD | Inc-FEM1B-4:1 | SEQ2624 | 2.E-02 | 0.6 | All AD | Inc-UBR2-4:2 | SEQ5873 | 4.E-02 | 0.8 |
| FTD | Inc-FFAR2-1:1 | SEQ4692 | 3.E-05 | 0.6 | DLB | Inc-UCK1-3:2 | SEQ5850 | 4.E-05 | 1.6 |
| All AD | Inc-FFAR2-1:1 | SEQ4692 | 3.E-02 | 0.8 | DLB | Inc-UCP3-2:3 | SEQ3728 | 4.E-05 | 2.2 |
| DLB | Inc-FGF23-5:1 | SEQ2474 | 2.E-04 | 1.5 | MCI | Inc-UCP3-2:3 | SEQ3728 | 9.E-05 | 2.4 |
| DLB | Inc-FGF23-5:3 | SEQ2473 | 2.E-04 | 1.6 | FTD | Inc-UCP3-2:3 | SEQ3728 | 1.E-04 | 1.9 |
| DLB | Inc-FGF9-11:1 | SEQ4915 | 5.E-05 | 1.8 | All AD | Inc-UCP3-2:3 | SEQ3728 | 1.E-02 | 1.3 |
| MCI | Inc-FGF9-11:1 | SEQ4915 | 2.E-04 | 1.7 | msAD | Inc-UCP3-2:3 | SEQ3728 | 4.E-02 | 1.2 |
| FTD | Inc-FGF9-11:1 | SEQ4915 | 9.E-04 | 1.5 | All AD | Inc-UGDH-3:3 | SEQ2526 | 2.E-02 | 0.8 |
| All AD | Inc-FGFBP2-1:2 | SEQ3763 | 2.E-02 | 0.7 | FTD | Inc-UGP2-3:2 | SEQ5158 | 5.E-04 | 0.7 |
| msAD | Inc-FGFBP2-1:2 | SEQ3763 | 4.E-02 | 0.7 | All AD | Inc-UGT3A2-3:1 | SEQ0389 | 5.E-02 | 0.8 |
| MCI | Inc-FGL2-3:1 | SEQ3286 | 5.E-05 | 0.4 | DLB | Inc-ULK4-1:2 | SEQ5565 | 2.E-04 | 1.5 |
| FTD | Inc-FGL2-3:1 | SEQ3286 | 2.E-04 | 0.5 | All AD | Inc-UPK3B-5:5 | SEQ5875 | 4.E-02 | 1.3 |
| DLB | Inc-FGL2-3:1 | SEQ3286 | 9.E-04 | 0.6 | All AD | Inc-UQCC3-1:1 | SEQ3979 | 1.E-02 | 1.2 |
| miAD | Inc-FGL2-3:1 | SEQ3286 | 1.E-02 | 0.7 | msAD | Inc-UQCC3-1:1 | SEQ3979 | 2.E-02 | 1.2 |
| All AD | Inc-FGL2-3:1 | SEQ3286 | 1.E-02 | 0.6 | FTD | Inc-UQCC3-2:1 | SEQ4084 | 2.E-09 | 3.3 |
| msAD | Inc-FGL2-3:1 | SEQ3286 | 4.E-02 | 0.6 | MCI | Inc-UQCC3-2:1 | SEQ4084 | 4.E-05 | 2.3 |
| FTD | Inc-FIGNL1-4:1 | SEQ5548 | 2.E-04 | 0.6 | DLB | Inc-UQCC3-2:1 | SEQ4084 | 2.E-04 | 2.0 |
| FTD | Inc-FILIP1-2:2 | SEQ6005 | 4.E-08 | 0.4 | All AD | Inc-UQCC3-2:1 | SEQ4084 | 6.E-03 | 1.5 |
| DLB | Inc-FKBP6-3:1 | SEQ4501 | 2.E-03 | 1.4 | msAD | Inc-UQCC3-2:1 | SEQ4084 | 1.E-02 | 1.5 |
| DLB | Inc-FLII-1:4 | SEQ4273 | 2.E-07 | 1.8 | miAD | Inc-UQCC3-2:1 | SEQ4084 | 1.E-02 | 1.4 |
| FTD | Inc-FLII-1:4 | SEQ4273 | 3.E-07 | 1.5 | FTD | Inc-UQCRHL-1:4 | SEQ4295 | 1.E-04 | 0.5 |
| MCI | Inc-FLII-1:4 | SEQ4273 | 1.E-06 | 1.7 | miAD | Inc-UQCRHL-1:4 | SEQ4295 | 7.E-03 | 0.6 |
| miAD | Inc-FLII-1:4 | SEQ4273 | 2.E-04 | 1.3 | All AD | Inc-UQCRHL-1:4 | SEQ4295 | 9.E-03 | 0.7 |
| All AD | Inc-FLII-1:4 | SEQ4273 | 6.E-04 | 1.2 | DLB | Inc-URGCP-2:11 | SEQ5906 | 2.E-05 | 1.8 |
| msAD | Inc-FLII-1:4 | SEQ4273 | 8.E-03 | 1.2 | MCI | Inc-URGCP-2:8 | SEQ5108 | 6.E-04 | 0.8 |
| FTD | Inc-FLNB-3:1 | SEQ4220 | 3.E-05 | 0.6 | FTD | Inc-URGCP-MRPS24-2:1 | SEQ2667 | 1.E-04 | 0.7 |
| miAD | Inc-FLNB-3:1 | SEQ4220 | 1.E-02 | 0.7 | DLB | Inc-USE1-4:1 | SEQ5041 | 7.E-04 | 1.6 |
| All AD | Inc-FLNB-3:1 | SEQ4220 | 1.E-02 | 0.8 | DLB | Inc-USP10-3:1 | SEQ5320 | 4.E-04 | 1.5 |
| DLB | Inc-FLOT1-1:1 | SEQ4403 | 2.E-05 | 1.5 | msAD | Inc-USP12-9:4 | SEQ4097 | 1.E-02 | 1.3 |
| MCI | Inc-FLOT1-1:1 | SEQ4403 | 2.E-03 | 1.3 | FTD | Inc-USP14-2:3 | SEQ4269 | 6.E-05 | 0.5 |
| DLB | Inc-FLOT1-1:2 | SEQ5889 | 2.E-05 | 1.5 | MCI | Inc-USP14-2:3 | SEQ4269 | 1.E-04 | 0.5 |
| DLB | Inc-FMR1NB-3:1 | SEQ4949 | 8.E-04 | 1.5 | miAD | Inc-USP14-2:3 | SEQ4269 | 8.E-03 | 0.7 |
| DLB | Inc-FNBP1-4:1 | SEQ5121 | 6.E-04 | 1.5 | All AD | Inc-USP14-2:3 | SEQ4269 | 2.E-02 | 0.7 |
| msAD | Inc-FNBP1L-1:12 | SEQ4163 | 2.E-03 | 1.4 | DLB | Inc-USP20-1:2 | SEQ5319 | 4.E-04 | 1.5 |
| All AD | Inc-FNBP1L-1:12 | SEQ4163 | 2.E-03 | 1.4 | FTD | Inc-USP25-1:1 | SEQ4207 | 6.E-05 | 0.6 |
| miAD | Inc-FNBP1L-1:12 | SEQ4163 | 1.E-02 | 1.3 | All AD | Inc-USP25-1:1 | SEQ4207 | 4.E-03 | 0.7 |
| miAD | Inc-FNDC1-9:10 | SEQ3845 | 5.E-03 | 1.4 | msAD | Inc-USP25-1:1 | SEQ4207 | 6.E-03 | 0.7 |
| All AD | Inc-FNDC1-9:10 | SEQ3845 | 6.E-03 | 1.4 | miAD | Inc-USP25-1:1 | SEQ4207 | 1.E-02 | 0.7 |
| msAD | Inc-FNDC1-9:10 | SEQ3845 | 3.E-02 | 1.4 | msAD | Inc-USP3-2:1 | SEQ4010 | 2.E-02 | 0.8 |
| All AD | Inc-FNDC1-9:15 | SEQ4713 | 4.E-02 | 0.8 | All AD | Inc-USP3-2:1 | SEQ4010 | 4.E-02 | 0.8 |
| MCI | Inc-FNDC1-9:22 | SEQ5027 | 2.E-04 | 1.5 | MCI | Inc-USP40-2:1 | SEQ5449 | 4.E-05 | 1.9 |
| DLB | Inc-FNDC1-9:22 | SEQ5027 | 7.E-04 | 1.5 | DLB | Inc-USP40-2:1 | SEQ5449 | 3.E-04 | 1.7 |
| miAD | Inc-FNDC1-9:3 | SEQ3846 | 5.E-03 | 1.4 | DLB | Inc-USP47-2:1 | SEQ3469 | 1.E-05 | 1.6 |
| All AD | Inc-FNDC1-9:3 | SEQ3846 | 6.E-03 | 1.4 | msAD | Inc-USP47-2:1 | SEQ3469 | 1. E-02 | 1.2 |
| msAD | Inc-FNDC1-9:3 | SEQ3846 | 3.E-02 | 1.4 | All AD | Inc-USP47-2:1 | SEQ3469 | 3.E-02 | 1.2 |
| FTD | Inc-FNTA-1:4 | SEQ4476 | 8.E-05 | 0.6 | MCI | Inc-USP53-8:1 | SEQ5737 | 9.E-05 | 1.7 |
| MCI | Inc-FNTA-1:4 | SEQ4476 | 2.E-03 | 0.7 | FTD | Inc-USP53-8:20 | SEQ5805 | 6.E-05 | 0.6 |
| All AD | Inc-FNTA-1:4 | SEQ4476 | 5.E-02 | 0.8 | FTD | Inc-USP53-8:32 | SEQ5801 | 6.E-05 | 0.6 |
| MCI | Inc-FOXD4L5-16:1 | SEQ5462 | 1.E-05 | 2.9 | FTD | Inc-USP53-8:5 | SEQ2668 | 4.E-04 | 0.7 |
| FTD | Inc-FOXD4L5-16:1 | SEQ5462 | 2.E-04 | 2.2 | DLB | Inc-USP6-2:20 | SEQ3027 | 2.E-03 | 1.5 |
| DLB | Inc-FOXD4L5-16:1 | SEQ5462 | 3.E-04 | 2.1 | DLB | Inc-USP6-2:22 | SEQ2832 | 2.E-04 | 1.6 |
| All AD | Inc-FOXD4L5-2:2 | SEQ4718 | 3.E-02 | 0.7 | msAD | Inc-USP9Y-17:1 | SEQ3994 | 2.E-02 | 1.3 |
| All AD | Inc-FOXD4L5-26:1 | SEQ4305 | 1.E-03 | 0.5 | FTD | Inc-UTP23-10:3 | SEQ5718 | 1. E-04 | 0.6 |
| miAD | Inc-FOXD4L5-26:1 | SEQ4305 | 1.E-03 | 0.5 | FTD | Inc-UTP23-11:1 | SEQ4260 | 2.E-04 | 0.6 |
| msAD | Inc-FOXD4L5-26:1 | SEQ4305 | 7.E-03 | 0.5 | miAD | Inc-UTP23-11:1 | SEQ4260 | 9.E-03 | 0.7 |
| miAD | Inc-FOXD4L5-28:1 | SEQ4208 | 1.E-02 | 0.7 | All AD | Inc-UTP23-11:1 | SEQ4260 | 1. E-02 | 0.7 |
| All AD | Inc-FOXD4L5-28:1 | SEQ4208 | 3.E-02 | 0.8 | FTD | Inc-UTP23-11:2 | SEQ3766 | 5.E-05 | 0.6 |
| DLB | Inc-FOXL2NB-4:1 | SEQ4512 | 2.E-03 | 1.5 | All AD | Inc-UTP23-11:2 | SEQ3766 | 2.E-02 | 0.7 |
| MCI | Inc-FPGS-1:3 | SEQ5049 | 6.E-04 | 1.8 | msAD | Inc-UTP23-11:2 | SEQ3766 | 4.E-02 | 0.8 |
| DLB | Inc-FPGS-1:3 | SEQ5049 | 7.E-04 | 1.9 | All AD | Inc-UXS1-3:1 | SEQ4239 | 3.E-03 | 1.2 |
| MCI | Inc-FPGS-2:10 | SEQ4725 | 1.E-03 | 1.9 | msAD | Inc-UXS1-3:1 | SEQ4239 | 6.E-03 | 1.2 |
| All AD | Inc-FRAT1-2:1 | SEQ4723 | 2.E-02 | 0.8 | miAD | Inc-UXS1-3:1 | SEQ4239 | 1. E-02 | 1.2 |
| MCI | Inc-FRMD5-1:1 | SEQ5916 | 6.E-07 | 1.6 | FTD | Inc-UXS1-4:9 | SEQ4323 | 4.E-07 | 2.4 |
| DLB | Inc-FRMD5-1:1 | SEQ5916 | 2.E-05 | 1.5 | MCI | Inc-UXS1-4:9 | SEQ4323 | 7.E-06 | 2.3 |
| DLB | Inc-FSCN1-2:1 | SEQ4958 | 5. E-04 | 1.6 | DLB | Inc-UXS1-4:9 | SEQ4323 | 1. E-04 | 2.1 |
| MCI | Inc-FSCN1-2:1 | SEQ4958 | 8.E-04 | 1.6 | All AD | Inc-UXS1-4:9 | SEQ4323 | 2.E-03 | 1.4 |
| DLB | Inc-FSCN2-1:6 | SEQ5192 | 3.E-06 | 1.9 | miAD | Inc-UXS1-4:9 | SEQ4323 | 3.E-03 | 1.4 |
| FTD | Inc-FSCN2-1:6 | SEQ5192 | 3.E-04 | 1.4 | msAD | Inc-UXS1-4:9 | SEQ4323 | 6.E-03 | 1.4 |
| MCI | Inc-FSCN2-1:6 | SEQ5192 | 5. E-04 | 1.5 | DLB | Inc-VAV2-1:1 | SEQ5517 | 2.E-04 | 1.4 |
| DLB | Inc-FSCN2-3:1 | SEQ4608 | 1.E-03 | 1.3 | All AD | Inc-VGLL4-8:1 | SEQ3978 | 1. E-02 | 0.8 |
| FTD | Inc-FUBP1-2:5 | SEQ4729 | 4.E-05 | 0.5 | msAD | Inc-VGLL4-8:1 | SEQ3978 | 2.E-02 | 0.8 |
| All AD | Inc-FUBP1-2:5 | SEQ4729 | 4.E-02 | 0.7 | DLB | Inc-VHLL-1:1 | SEQ4962 | 8.E-04 | 1.6 |
| All AD | Inc-FZD4-1:6 | SEQ2454 | 3.E-02 | 0.7 | DLB | Inc-VIPR1-1:1 | SEQ4485 | 2.E-03 | 1.4 |
| FTD | Inc-FZD5-1:4 | SEQ5296 | 4.E-04 | 0.7 | miAD | Inc-VMP1-8:1 | SEQ3916 | 6.E-03 | 0.7 |
| DLB | Inc-GADD45B-1:2 | SEQ5496 | 2.E-04 | 1.8 | All AD | Inc-VMP1-8:1 | SEQ3916 | 6.E-03 | 0.7 |
| DLB | Inc-GADD45GIP1-1:1 | SEQ4094 | 5.E-05 | 2.0 | msAD | Inc-VMP1-8:1 | SEQ3916 | 3.E-02 | 0.8 |
| MCI | Inc-GADD45GIP1-1:1 | SEQ4094 | 6.E-05 | 2.0 | DLB | Inc-VN1R2-1:1 | SEQ5042 | 7.E-04 | 1.7 |
| FTD | Inc-GADD45GIP1-1:1 | SEQ4094 | 1.E-04 | 1.6 | All AD | Inc-VPREB1-7:15 | SEQ4198 | 3.E-03 | 1.4 |
| msAD | Inc-GADD45GIP1-1:1 | SEQ4094 | 1.E-02 | 1.2 | msAD | Inc-VPREB1-7:15 | SEQ4198 | 4.E-03 | 1.5 |
| All AD | Inc-GADD45GIP1-1:1 | SEQ4094 | 2.E-02 | 1.2 | miAD | Inc-VPREB1-7:15 | SEQ4198 | 1. E-02 | 1.4 |
| All AD | Inc-GALNT15-6:1 | SEQ3879 | 3.E-02 | 1.3 | FTD | Inc-VPS13B-1:2 | SEQ5887 | 1.E-05 | 0.6 |
| msAD | Inc-GALNT15-6:1 | SEQ3879 | 3.E-02 | 1.3 | All AD | Inc-VPS13B-1:2 | SEQ5887 | 3.E-02 | 0.8 |
| DLB | Inc-GALNTL5-2:10 | SEQ4980 | 8.E-04 | 1.9 | All AD | Inc-VRK1-10:3 | SEQ4112 | 9.E-03 | 0.6 |
| DLB | Inc-GAMT-1:2 | SEQ5378 | 3.E-04 | 1.6 | msAD | Inc-VRK1-10:3 | SEQ4112 | 1.E-02 | 0.7 |
| DLB | Inc-GAMT-4:1 | SEQ4704 | 1.E-03 | 1.5 | DLB | Inc-VRK3-2:4 | SEQ4847 | 9.E-04 | 1.5 |
| DLB | Inc-GAN-1:1 | SEQ4410 | 2.E-03 | 1.4 | FTD | Inc-VSIG10-1:1 | SEQ3871 | 2.E-05 | 0.7 |
| FTD | Inc-GAPDH-2:1 | SEQ5305 | 5.E-07 | 9.E-09 | All AD | Inc-VSIG10-1:1 | SEQ3871 | 3.E-02 | 0.8 |
| MCI | Inc-GAPDH-2:1 | SEQ5305 | 2.E-05 | 5.E-09 | msAD | Inc-VSIG10-1:1 | SEQ3871 | 3.E-02 | 0.8 |
| DLB | Inc-GAPDH-2:1 | SEQ5305 | 4.E-04 | 6.E-09 | miAD | Inc-VSNL1-5:1 | SEQ3597 | 2.E-03 | 1.4 |
| DLB | Inc-GAPDH-3:1 | SEQ.5118 | 6.E-05 | 1.6 | All AD | Inc-VSNL1-5:1 | SEQ3597 | 5.E-03 | 1.3 |
| MCI | Inc-GAPDH-3:1 | SEQ5118 | 6.E-04 | 1.5 | msAD | Inc-VSNL1-5:1 | SEQ3597 | 4.E-02 | 1.2 |
| DLB | Inc-GAPDH-3:2 | SEQ4569 | 2.E-03 | 1.4 | DLB | Inc-VSTM2B-9:15 | SEQ5569 | 2.E-04 | 1.6 |
| DLB | Inc-GAPDH-6:1 | SEQ4487 | 2.E-03 | 1.4 | FTD | Inc-VSTM5-1:10 | SEQ4904 | 9.E-04 | 0.7 |
| FTD | Inc-GAPVD1-3:2 | SEQ5786 | 3.E-06 | 0.4 | FTD | Inc-VSTM5-1:13 | SEQ2419 | 8.E-04 | 2.2 |
| MCI | Inc-GAPVD1-3:2 | SEQ5786 | 6.E-05 | 0.5 | DLB | Inc-VSX1-4:1 | SEQ5675 | 1.E-04 | 1.9 |
| DLB | Inc-GAST-2:1 | SEQ4494 | 2.E-03 | 1.4 | FTD | Inc-VTI1B-1:1 | SEQ5590 | 2.E-04 | 0.6 |
| MCI | Inc-GATAS-9:1 | SEQ4488 | 9.E-04 | 1.5 | FTD | Inc-WBP4-2:9 | SEQ2356 | 4.E-04 | 0.7 |
| DLB | Inc-GATA5-9:1 | SEQ4488 | 2.E-03 | 1.4 | All AD | Inc-WBP4-2:9 | SEQ2356 | 3.E-02 | 0.8 |
| DLB | Inc-GATS-3:2 | SEQ5487 | 2.E-04 | 1.6 | FTD | Inc-WDR3-1:1 | SEQ5068 | 7.E-04 | 0.7 |
| FTD | Inc-GBP2-1:2 | SEQ2449 | 3.E-06 | 0.6 | DLB | Inc-WDR4-2:5 | SEQ2874 | 2.E-04 | 1.5 |
| MCI | Inc-GBP2-1:2 | SEQ2449 | 2.E-05 | 0.6 | DLB | Inc-W DR45B-1:2 | SEQ3588 | 3.E-05 | 1.8 |
| All AD | Inc-GBP2-1:2 | SEQ2449 | 3.E-02 | 0.8 | DLB | Inc-WDR77-1:2 | SEQ2553 | 5.E-04 | 1.6 |
| msAD | Inc-GBP2-1:2 | SEQ2449 | 3.E-02 | 0.8 | All AD | Inc-WIPF1-1:1 | SEQ5892 | 4.E-02 | 0.8 |
| FTD | Inc-GBP6-1:1 | SEQ5365 | 3.E-06 | 0.4 | All AD | Inc-WNT2-4:1 | SEQ5893 | 5.E-02 | 0.8 |
| MCI | Inc-GBP6-1:1 | SEQ5365 | 3.E-04 | 0.4 | DLB | Inc-WNT4-2:1 | SEQ4565 | 2.E-03 | 1.4 |
| MCI | Inc-GCDH-3:4 | SEQ5537 | 2.E-04 | 1.8 | FTD | Inc-WRNIP1-13:1 | SEQ5955 | 7.E-06 | 0.5 |
| FTD | Inc-GCGR-1:2 | SEQ2373 | 4.E-05 | 1.5 | FTD | Inc-WWP2-2:1 | SEQ4237 | 1.E-08 | 0.5 |
| DLB | Inc-GCGR-1:2 | SEQ2373 | 9.E-05 | 1.7 | MCI | Inc-WWP2-2:1 | SEQ4237 | 8.E-05 | 0.6 |
| miAD | Inc-GCGR-1:2 | SEQ2373 | 5.E-04 | 1.4 | All AD | Inc-WWP2-2:1 | SEQ4237 | 3.E-03 | 0.7 |
| All AD | Inc-GCGR-1:2 | SEQ2373 | 9.E-04 | 1.3 | miAD | Inc-WWP2-2:1 | SEQ4237 | 4.E-03 | 0.6 |
| MCI | Inc-GCGR-1:2 | SEQ2373 | 9.E-04 | 1.4 | msAD | Inc-WWP2-2:1 | SEQ4237 | 1.E-02 | 0.7 |
| msAD | Inc-GCGR-1:2 | SEQ2373 | 9.E-03 | 1.3 | DLB | Inc-XRCC1-2:1 | SEQ5478 | 2.E-04 | 1.4 |
| All AD | Inc-GCHl-2:4 | SEQ4749 | 4.E-02 | 1.2 | FTD | Inc-XRCC2-13:2 | SEQ5151 | 5.E-04 | 0.6 |
| DLB | Inc-GCN1-3:1 | SEQ3301 | 3.E-04 | 1.5 | DLB | Inc-XRCC3-1:1 | SEQ4763 | 1.E-03 | 1.5 |
| MCI | Inc-GCNT1-7:1 | SEQ4751 | 7.E-06 | 3.0 | DLB | Inc-XRCC3-1:2 | SEQ5437 | 3.E-04 | 1.5 |
| DLB | Inc-GCNT1-7:1 | SEQ4751 | 3.E-04 | 2.5 | FTD | Inc-XXYLT1-5:1 | SEQ0221 | 1.E-06 | 0.5 |
| FTD | Inc-GCNT1-7:1 | SEQ4751 | 6.E-04 | 2.1 | miAD | Inc-XXYLT1-5:1 | SEQ0221 | 5.E-04 | 0.6 |
| All AD | Inc-GCNT1-7:1 | SEQ4751 | 3.E-02 | 1.4 | All AD | Inc-XXYLT1-5:1 | SEQ0221 | 2.E-03 | 0.7 |
| MCI | Inc-GDE1-1:5 | SEQ4590 | 2.E-03 | 1.6 | msAD | Inc-XXYLT1-5:1 | SEQ0221 | 4.E-02 | 0.7 |
| DLB | Inc-GDI1-1:1 | SEQ4743 | 1.E-03 | 1.4 | DLB | Inc-YIF1A-1:1 | SEQ5935 | 1.E-05 | 1.7 |
| MCI | Inc-GEMIN4-6:1 | SEQ4080 | 6.E-04 | 1.4 | DLB | Inc-YIF1B-1:1 | SEQ5461 | 3.E-04 | 2.1 |
| miAD | Inc-GEMIN4-6:1 | SEQ4080 | 1.E-02 | 1.2 | DLB | Inc-YPEL5-5:1 | SEQ0750 | 1.E-04 | 1.7 |
| All AD | Inc-GEMIN4-6:1 | SEQ4080 | 1.E-02 | 1.2 | MCI | Inc-YPELS-5:1 | SEQ0750 | 6.E-04 | 1.5 |
| DLB | Inc-GGCT-1:31 | SEQ5630 | 2.E-04 | 1.7 | All AD | Inc-YPELS-5:1 | SEQ0750 | 4.E-02 | 1.2 |
| DLB | Inc-GGCT-1:4 | SEQ5331 | 4.E-04 | 1.7 | FTD | Inc-YTHDF3-4:1 | SEQ6008 | 8.E-09 | 0.4 |
| FTD | Inc-GGCT-1:64 | SEQ4991 | 8.E-04 | 0.6 | FTD | Inc-YWHAZ-2:1 | SEQ5410 | 3.E-04 | 0.6 |
| FTD | Inc-GGCT-1:81 | SEQ5804 | 6.E-05 | 0.6 | All AD | Inc-YWHAZ-2:1 | SEQ5410 | 4.E-02 | 0.8 |
| FTD | Inc-GGCT-1:82 | SEQ5803 | 6.E-05 | 0.6 | DLB | Inc-YY1-1:2 | SEQ4981 | 8.E-04 | 2.0 |
| DLB | Inc-GHRL-4:2 | SEQ4753 | 2.E-04 | 1.6 | msAD | Inc-YY1AP1-2:1 | SEQ3501 | 8.E-03 | 0.7 |
| MCI | Inc-GHRL-4:2 | SEQ4753 | 1.E-03 | 1.4 | All AD | Inc-YY1AP1-2:1 | SEQ3501 | 2.E-02 | 0.8 |
| miAD | Inc-GHSR-1:1 | SEQ4221 | 1.E-02 | 0.7 | DLB | Inc-YY1AP1-3:1 | SEQ5617 | 2.E-04 | 1.5 |
| All AD | Inc-GHSR-1:1 | SEQ4221 | 2.E-02 | 0.8 | FTD | Inc-ZADH2-3:1 | SEQ5232 | 5.E-04 | 0.7 |
| MCI | Inc-GIMAP5-1:1 | SEQ3302 | 1.E-04 | 1.5 | msAD | Inc-ZBED4-2:1 | SEQ3863 | 3.E-02 | 1.4 |
| DLB | Inc-GIMAP5-1:1 | SEQ3302 | 6.E-04 | 1.4 | msAD | Inc-ZBTB2-7:1 | SEQ3675 | 5.E-02 | 1.4 |
| FTD | Inc-GIMAP7-1:1 | SEQ3984 | 2.E-10 | 0.3 | DLB | Inc-ZBTB46-2:2 | SEQ5242 | 5.E-04 | 1.4 |
| MCI | Inc-GIMAP7-1:1 | SEQ3984 | 6.E-05 | 0.3 | DLB | Inc-ZBTB46-2:3 | SEQ5111 | 6.E-04 | 1.4 |
| All AD | Inc-GIMAP7-1:1 | SEQ3984 | 9.E-03 | 0.6 | FTD | Inc-ZBTB7C-10:1 | SEQ5298 | 4.E-04 | 0.7 |
| miAD | Inc-GIMAP7-1:1 | SEQ3984 | 1.E-02 | 0.6 | MCI | Inc-ZC2HC1C-3:1 | SEQ3856 | 5.E-04 | 1.3 |
| msAD | Inc-GIMAP7-1:1 | SEQ3984 | 2.E-02 | 0.6 | DLB | Inc-ZC2HC1C-3:1 | SEQ3856 | 8.E-04 | 1.3 |
| All AD | Inc-GK5-7:1 | SEQ4767 | 4.E-02 | 0.8 | All AD | Inc-ZC2HC1C-3:1 | SEQ3856 | 2.E-02 | 1.1 |
| DLB | Inc-GLB1-2:1 | SEQ4570 | 1.E-03 | 1.6 | msAD | Inc-ZC2HC1C-3:1 | SEQ3856 | 3.E-02 | 1.1 |
| MCI | Inc-GLB1-2:1 | SEQ4570 | 2.E-03 | 1.5 | DLB | Inc-ZC3H11A-1:3 | SEQ5140 | 6.E-04 | 1.8 |
| DLB | Inc-GLDC-6:1 | SEQ3965 | 2.E-03 | 1.5 | MCI | Inc-ZC3H8-7:2 | SEQ3951 | 4.E-05 | 1.7 |
| msAD | Inc-GLDC-6:1 | SEQ3965 | 2.E-02 | 1.2 | DLB | Inc-ZC3H8-7:2 | SEQ3951 | 8.E-05 | 1.8 |
| All AD | Inc-GLDC-6:1 | SEQ3965 | 3.E-02 | 1.2 | FTD | Inc-ZC3H8-7:2 | SEQ3951 | 2.E-04 | 1.6 |
| DLB | Inc-GLG1-1:1 | SEQ5442 | 3.E-04 | 1.6 | All AD | Inc-ZC3H8-7:2 | SEQ3951 | 8.E-03 | 1.3 |
| DLB | Inc-GLOD4-2:2 | SEQ4773 | 5.E-04 | 1.7 | miAD | Inc-ZC3H8-7:2 | SEQ3951 | 1.E-02 | 1.3 |
| All AD | Inc-GLOD4-2:2 | SEQ4773 | 4.E-02 | 1.2 | msAD | Inc-ZC3H8-7:2 | SEQ3951 | 3.E-02 | 1.3 |
| DLB | Inc-GLOD4-3:1 | SEQ2673 | 8.E-05 | 1.6 | DLB | Inc-ZC3HC1-4:1 | SEQ2731 | 1.E-03 | 1.4 |
| FTD | Inc-GLRX5-7:1 | SEQ5602 | 2.E-04 | 0.6 | FTD | Inc-ZCCHC13-4:1 | SEQ4168 | 3.E-11 | 5.E-03 |
| FTD | Inc-GMNN-5:1 | SEQ5598 | 2.E-04 | 0.6 | MCI | Inc-ZCCHC13-4:1 | SEQ4168 | 9.E-06 | 6.E-03 |
| FTD | Inc-GMNN-5:2 | SEQ5087 | 6.E-04 | 0.6 | DLB | Inc-ZCCHC13-4:1 | SEQ4168 | 7.E-04 | 9.E-03 |
| FTD | Inc-GMPR2-1:1 | SEQ5666 | 3.E-07 | 1.7 | All AD | Inc-ZCCHC13-4:1 | SEQ4168 | 3.E-03 | 1. E-02 |
| MCI | Inc-GMPR2-1:1 | SEQ5666 | 2.E-06 | 1.8 | miAD | Inc-ZCCHC13-4:1 | SEQ4168 | 4.E-03 | 1. E-02 |
| DLB | Inc-GMPR2-1:1 | SEQ5666 | 1.E-04 | 1.6 | msAD | Inc-ZCCHC13-4:1 | SEQ4168 | 1.E-02 | 1. E-02 |
| DLB | Inc-GNA11-3:1 | SEQ2681 | 2.E-04 | 1.6 | DLB | Inc-ZCCHC24-8:1 | SEQ5905 | 2.E-05 | 1.7 |
| miAD | Inc-GNA11-3:1 | SEQ2681 | 3.E-03 | 1.3 | DLB | Inc-ZCCHC7-2:12 | SEQ3490 | 1.E-03 | 2.0 |
| All AD | Inc-GNA11-3:1 | SEQ2681 | 5.E-03 | 1.2 | DLB | Inc-ZCCHC7-2:22 | SEQ3657 | 4.E-05 | 4.6 |
| msAD | Inc-GNA11-3:1 | SEQ2681 | 3.E-02 | 1.2 | MCI | Inc-ZCCHC7-2:22 | SEQ3657 | 1.E-04 | 4.0 |
| DLB | Inc-GNA11-3:2 | SEQ3399 | 2.E-03 | 1.4 | FTD | Inc-ZCCHC7-2:22 | SEQ3657 | 7.E-04 | 2.8 |
| FTD | Inc-GNA14-3:1 | SEQ2572 | 1.E-06 | 0.6 | All AD | Inc-ZCCHC7-2:22 | SEQ3657 | 2.E-02 | 1.5 |
| All AD | Inc-GNA14-3:1 | SEQ2572 | 5.E-02 | 0.7 | msAD | Inc-ZCCHC7-2:22 | SEQ3657 | 5.E-02 | 1.4 |
| DLB | Inc-GNAT2-1:1 | SEQ4948 | 8.E-04 | 1.5 | MCI | Inc-ZCCHC7-2:24 | SEQ2279 | 3.E-04 | 0.5 |
| FTD | Inc-GNB4-1:1 | SEQ4783 | 3.E-05 | 0.5 | FTD | Inc-ZCCHC7-2:24 | SEQ2279 | 4.E-04 | 0.5 |
| MCI | Inc-GNB4-1:1 | SEQ4783 | 3.E-04 | 0.5 | DLB | Inc-ZFP57-11:1 | SEQ4678 | 8.E-04 | 1.4 |
| All AD | Inc-GNB4-1:1 | SEQ4783 | 2.E-02 | 0.7 | MCI | Inc-ZFP57-11:1 | SEQ4678 | 1.E-03 | 1.3 |
| DLB | Inc-GOLGA8J-4:1 | SEQ3857 | 3.E-05 | 1.4 | DLB | Inc-ZFYVE21-2:1 | SEQ5215 | 3.E-04 | 1.9 |
| All AD | Inc-GOLGA8J-4:1 | SEQ3857 | 2.E-02 | 1.1 | MCI | Inc-ZFYVE21-2:1 | SEQ5215 | 5.E-04 | 2.0 |
| msAD | Inc-GOLGA8J-4:1 | SEQ3857 | 3.E-02 | 1.1 | All AD | Inc-ZGLP1-4:1 | SEQ3935 | 9.E-03 | 0.7 |
| FTD | Inc-GOLGA8O-1:1 | SEQ4908 | 9.E-04 | 0.7 | miAD | Inc-ZGLP1-4:1 | SEQ3935 | 1.E-02 | 0.7 |
| DLB | Inc-GPAT4-2:2 | SEQ2763 | 1.E-04 | 1.5 | msAD | Inc-ZGLP1-4:1 | SEQ3935 | 3.E-02 | 0.8 |
| FTD | Inc-GPBP1L1-1:8 | SEQ2745 | 1.E-08 | 0.4 | FTD | Inc-ZMAT3-4:1 | SEQ5154 | 5.E-04 | 0.6 |
| MCI | Inc-GPBP1L1-1:8 | SEQ2745 | 2.E-05 | 0.5 | DLB | Inc-ZMYM4-3:2 | SEQ4023 | 2.E-04 | 1.8 |
| All AD | Inc-GPBP1L1-1:8 | SEQ2745 | 3.E-02 | 0.7 | MCI | Inc-ZMYM4-3:2 | SEQ4023 | 1.E-03 | 1.5 |
| All AD | Inc-GPD2-1:3 | SEQ4038 | 1.E-02 | 1.4 | All AD | Inc-ZMYM4-3:2 | SEQ4023 | 1.E-02 | 1.3 |
| msAD | Inc-GPD2-1:3 | SEQ4038 | 2.E-02 | 1.5 | msAD | Inc-ZMYM4-3:2 | SEQ4023 | 2.E-02 | 1.3 |
| MCI | Inc-GPR137C-1:1 | SEQ4790 | 3.E-06 | 0.5 | DLB | Inc-ZMYND19-1:6 | SEQ4936 | 8.E-04 | 1.4 |
| FTD | Inc-GPR137C-1:1 | SEQ4790 | 1.E-05 | 0.5 | DLB | Inc-ZNF100-7:1 | SEQ5333 | 4.E-04 | 1.7 |
| All AD | Inc-GPR137C-1:1 | SEQ4790 | 4.E-02 | 0.8 | MCI | Inc-ZNF131-1:16 | SEQ4589 | 2.E-03 | 1.6 |
| miAD | Inc-GPR141-2:1 | SEQ4049 | 2.E-03 | 0.6 | DLB | Inc-ZNF131-1:24 | SEQ3221 | 4.E-04 | 1.7 |
| All AD | Inc-GPR141-2:1 | SEQ4049 | 3.E-03 | 0.7 | DLB | Inc-ZNF131-2:5 | SEQ5730 | 9.E-05 | 1.5 |
| msAD | Inc-GPR141-2:1 | SEQ4049 | 2.E-02 | 0.7 | DLB | Inc-ZNF132-1:2 | SEQ4976 | 8.E-04 | 1.7 |
| FTD | Inc-GPR141-3:1 | SEQ4382 | 8.E-08 | 0.4 | DLB | Inc-ZNF174-1:13 | SEQ5117 | 6.E-04 | 1.4 |
| MCI | Inc-GPR141-3:1 | SEQ4382 | 4.E-04 | 0.5 | DLB | Inc-ZNF212-2:2 | SEQ5271 | 5.E-04 | 1.7 |
| All AD | Inc-GPR141-3:1 | SEQ4382 | 1.E-03 | 0.5 | DLB | Inc-ZNF217-5:1 | SEQ5907 | 2.E-05 | 1.8 |
| miAD | Inc-GPR141-3:1 | SEQ4382 | 3.E-03 | 0.5 | MCI | Inc-ZNF2-2:1 | SEQ5052 | 7.E-04 | 2.2 |
| msAD | Inc-GPR141-3:1 | SEQ4382 | 3.E-03 | 0.6 | MCI | Inc-ZNF236-6:3 | SEQ5045 | 7.E-04 | 1.8 |
| FTD | Inc-GPR160-1:1 | SEQ5901 | 2.E-05 | 0.6 | DLB | Inc-ZNF236-9:4 | SEQ5268 | 5.E-04 | 1.6 |
| FTD | Inc-GPR161-4:1 | SEQ0070 | 7.E-05 | 0.5 | DLB | Inc-ZNF266-2:1 | SEQ4502 | 3.E-04 | 1.6 |
| All AD | Inc-GPR161-4:1 | SEQ0070 | 5.E-02 | 0.7 | MCI | Inc-ZNF266-2:1 | SEQ4502 | 2.E-03 | 1.4 |
| All AD | Inc-GPR183-5:6 | SEQ4068 | 1.E-02 | 0.6 | All AD | Inc-ZNF273-4:3 | SEQ5909 | 4.E-02 | 0.8 |
| msAD | Inc-GPR183-5:6 | SEQ4068 | 1.E-02 | 0.6 | FTD | Inc-ZNF284-1:1 | SEQ4778 | 9.E-04 | 1.6 |
| All AD | Inc-GPR183-5:8 | SEQ4796 | 5.E-02 | 0.7 | DLB | Inc-ZNF284-1:1 | SEQ4778 | 1.E-03 | 1.6 |
| FTD | Inc-GPR27-5:1 | SEQ3959 | 2.E-04 | 0.6 | DLB | Inc-ZNF316-2:1 | SEQ4627 | 1.E-03 | 1.4 |
| miAD | Inc-GPR27-5:1 | SEQ3959 | 2.E-03 | 0.5 | FTD | Inc-ZNF330-4:1 | SEQ5770 | 7.E-05 | 0.4 |
| All AD | Inc-GPR27-5:1 | SEQ3959 | 4.E-03 | 0.6 | DLB | Inc-ZNF335-1:1 | SEQ4628 | 3.E-04 | 1.5 |
| msAD | Inc-GPR27-5:1 | SEQ3959 | 2.E-02 | 0.7 | MCI | Inc-ZNF335-1:1 | SEQ4628 | 1.E-03 | 1.4 |
| FTD | Inc-GPR33-14:1 | SEQ0236 | 8.E-07 | 0.5 | DLB | Inc-ZNF33B-4:4 | SEQ4578 | 2.E-03 | 1.5 |
| MCI | Inc-GPR33-14:1 | SEQ0236 | 2.E-05 | 0.5 | FTD | Inc-ZNF362-1:11 | SEQ4346 | 3.E-06 | 0.5 |
| DLB | Inc-GPR37L1-7:4 | SEQ3402 | 2.E-04 | 1.5 | DLB | Inc-ZNF362-1:11 | SEQ4346 | 3.E-04 | 0.7 |
| FTD | Inc-GPR39-9:1 | SEQ2704 | 5.E-06 | 0.5 | miAD | Inc-ZNF362-1:11 | SEQ4346 | 5.E-03 | 0.6 |
| FTD | Inc-GPR83-1:1 | SEQ5543 | 2.E-04 | 0.6 | All AD | Inc-ZNF362-1:11 | SEQ4346 | 1.E-02 | 0.6 |
| DLB | Inc-GPRC5D-3:1 | SEQ4527 | 5.E-04 | 1.9 | FTD | Inc-ZNF362-1:6 | SEQ3910 | 2.E-05 | 0.6 |
| MCI | Inc-GPRC5D-3:1 | SEQ4527 | 2.E-03 | 1.6 | DLB | Inc-ZNF362-1:6 | SEQ3910 | 8.E-04 | 0.8 |
| MCI | Inc-GPRIN2-2:1 | SEQ2453 | 2.E-03 | 0.7 | All AD | Inc-ZNF362-1:6 | SEQ3910 | 8.E-03 | 0.7 |
| DLB | Inc-GPSM1-2:1 | SEQ4554 | 2.E-05 | 1.5 | miAD | Inc-ZNF362-1:6 | SEQ3910 | 8.E-03 | 0.7 |
| MCI | Inc-GPSM1-2:1 | SEQ4554 | 2.E-03 | 1.3 | msAD | Inc-ZNF362-1:6 | SEQ3910 | 3.E-02 | 0.7 |
| DLB | Inc-GPT2-1:5 | SEQ3923 | 2.E-04 | 1.5 | FTD | Inc-ZNF362-1:7 | SEQ4917 | 9.E-04 | 2.5 |
| MCI | Inc-GPT2-1:5 | SEQ3923 | 2.E-04 | 1.5 | DLB | Inc-ZNF362-3:1 | SEQ5119 | 6.E-04 | 1.5 |
| msAD | Inc-GPT2-1:5 | SEQ3923 | 3.E-02 | 1.2 | DLB | Inc-ZNF385C-4:3 | SEQ5816 | 5.E-05 | 1.6 |
| All AD | Inc-GPT2-1:5 | SEQ3923 | 3.E-02 | 1.2 | DLB | Inc-ZNF397-4:1 | SEQ4775 | 1.E-03 | 1.6 |
| DLB | Inc-GPX3-1:1 | SEQ5729 | 9.E-05 | 1.5 | DLB | Inc-ZNF408-5:2 | SEQ5846 | 4.E-05 | 1.5 |
| MCI | Inc-GPX3-1:1 | SEQ5729 | 9.E-05 | 1.6 | All AD | Inc-ZNF408-5:2 | SEQ5846 | 4.E-02 | 1.2 |
| FTD | Inc-GRAP-1:2 | SEQ5870 | 4.E-05 | 2.8 | DLB | Inc-ZNF420-1:1 | SEQ5699 | 1. E-04 | 1.6 |
| DLB | Inc-GRAP2-2:1 | SEQ2429 | 5. E-04 | 1.6 | DLB | Inc-ZNF438-4:2 | SEQ5275 | 2.E-04 | 1.7 |
| DLB | Inc-GRAP-4:1 | SEQ4649 | 1.E-03 | 1.8 | MCI | Inc-ZNF438-4:2 | SEQ5275 | 5. E-04 | 1.7 |
| FTD | Inc-GRAP-7:2 | SEQ3752 | 2.E-06 | 2.1 | DLB | Inc-ZNF439-1:1 | SEQ4525 | 2.E-03 | 1.6 |
| DLB | Inc-GRAP-7:2 | SEQ3752 | 1.E-03 | 1.7 | DLB | Inc-ZNF460-1:1 | SEQ5610 | 2.E-04 | 1.3 |
| All AD | Inc-GRAP-7:2 | SEQ3752 | 4.E-02 | 1.2 | FTD | Inc-ZNF484-1:1 | SEQ5355 | 4.E-04 | 0.6 |
| msAD | Inc-GRAP-7:2 | SEQ3752 | 4.E-02 | 1.2 | FTD | Inc-ZNF516-14:1 | SEQ4259 | 9.E-10 | 0.3 |
| DLB | Inc-GRAPL-1:1 | SEQ3729 | 2.E-03 | 1.8 | MCI | Inc-ZNF516-14:1 | SEQ4259 | 9.E-06 | 0.4 |
| msAD | Inc-GRAPL-1:1 | SEQ3729 | 4.E-02 | 1.3 | miAD | Inc-ZNF516-14:1 | SEQ4259 | 1. E-04 | 0.5 |
| miAD | Inc-GREM1-2:3 | SEQ4143 | 1.E-02 | 0.4 | DLB | Inc-ZNF516-14:1 | SEQ4259 | 5. E-04 | 0.5 |
| DLB | Inc-GRHPR-1:1 | SEQ4500 | 2.E-03 | 1.4 | All AD | Inc-ZNF516-14:1 | SEQ4259 | 5. E-04 | 0.6 |
| MCI | Inc-GRIK2-7:1 | SEQ5652 | 1. E-04 | 0.5 | msAD | Inc-ZNF516-14:1 | SEQ4259 | 9.E-03 | 0.6 |
| DLB | Inc-GRIN3B-3:1 | SEQ5784 | 3.E-06 | 2.0 | FTD | Inc-ZNF518A-1:14 | SEQ5919 | 1.E-05 | 0.6 |
| MCI | Inc-GRIN3B-3:1 | SEQ5784 | 7.E-06 | 1.8 | All AD | Inc-ZNF518A-1:14 | SEQ5919 | 5.E-02 | 0.8 |
| FTD | Inc-GRIN3B-3:1 | SEQ5784 | 7.E-05 | 1.5 | All AD | Inc-ZNF518A-1:5 | SEQ5920 | 4.E-02 | 0.8 |
| DLB | Inc-GRINA-1:1 | SEQ3118 | 2.E-04 | 1.6 | MCI | Inc-ZNF518B-2:4 | SEQ5239 | 5.E-04 | 0.5 |
| MCI | Inc-GRINA-1:1 | SEQ3118 | 5. E-04 | 1.7 | All AD | Inc-ZNF518B-2:4 | SEQ5239 | 4.E-02 | 0.7 |
| MCI | Inc-GRINA-2:1 | SEQ2969 | 4.E-06 | 1.7 | DLB | Inc-ZNF627-3:6 | SEQ5025 | 7.E-04 | 1.5 |
| DLB | Inc-GRINA-2:1 | SEQ2969 | 1. E-04 | 1.6 | DLB | Inc-ZNF629-1:1 | SEQ4680 | 3.E-05 | 1.4 |
| FTD | Inc-GRINA-2:1 | SEQ2969 | 9.E-04 | 1.4 | MCI | Inc-ZNF629-1:1 | SEQ4680 | 1. E-03 | 1.3 |
| All AD | Inc-GRINA-2:1 | SEQ2969 | 4.E-02 | 1.2 | FTD | Inc-ZNF639-4:1 | SEQ2617 | 8.E-06 | 0.5 |
| DLB | Inc-GRINA-3:1 | SEQ3047 | 6.E-05 | 1.5 | All AD | Inc-ZNF639-4:1 | SEQ2617 | 2.E-02 | 0.7 |
| MCI | Inc-GRINA-3:1 | SEQ3047 | 5. E-04 | 1.4 | DLB | Inc-ZNF646-5:2 | SEQ4789 | 1. E-03 | 1.9 |
| DLB | Inc-GRK3-7:4 | SEQ5037 | 7.E-04 | 1.6 | miAD | Inc-ZNF654-3:1 | SEQ0857 | 6.E-03 | 0.7 |
| DLB | Inc-GRK4-2:7 | SEQ3405 | 9.E-05 | 1.4 | All AD | Inc-ZNF654-3:1 | SEQ0857 | 3.E-02 | 0.8 |
| MCI | Inc-GRK4-2:7 | SEQ3405 | 2.E-03 | 1.3 | msAD | Inc-ZNF683-3:1 | SEQ4253 | 9.E-03 | 1.2 |
| FTD | Inc-GRM1-1:2 | SEQ4887 | 9.E-04 | 0.4 | FTD | Inc-ZNF697-2:1 | SEQ5593 | 2.E-04 | 0.6 |
| DLB | Inc-GRMl-2:1 | SEQ4716 | 1.E-03 | 1.6 | All AD | Inc-ZNF697-2:1 | SEQ5593 | 4.E-02 | 0.8 |
| FTD | Inc-GRM8-2:1 | SEQ4812 | 1.E-03 | 0.7 | FTD | Inc-ZNF704-8:1 | SEQ5717 | 1.E-04 | 0.6 |
| FTD | Inc-GRM8-2:2 | SEQ0430 | 1. E-04 | 0.6 | DLB | Inc-ZNF706-1:1 | SEQ4714 | 1.E-03 | 1.6 |
| FTD | \|nc-GRSF1-1:1 | SEQ4817 | 4.E-04 | 0.6 | MCI | Inc-ZNF708-13:1 | SEQ5080 | 2.E-04 | 0.4 |
| All AD | Inc-GRSF1-1:1 | SEQ4817 | 4.E-02 | 0.8 | FTD | Inc-ZNF708-13:1 | SEQ5080 | 6.E-04 | 0.5 |
| MCI | Inc-GSDMD-2:1 | SEQ4522 | 9.E-04 | 1.7 | MCI | Inc-ZNF708-3:1 | SEQ5082 | 2.E-04 | 0.6 |
| DLB | Inc-GSDMD-2:1 | SEQ4522 | 2.E-03 | 1.6 | FTD | Inc-ZNF708-3:1 | SEQ5082 | 6.E-04 | 0.5 |
| All AD | Inc-GSTO2-3:1 | SEQ4818 | 4.E-02 | 0.8 | MCI | Inc-ZNF726-1:3 | SEQ0259 | 1.E-03 | 1.6 |
| FTD | Inc-GSX2-7:1 | SEQ4820 | 1.E-08 | 0.4 | FTD | Inc-ZNF730-11:1 | SEQ4899 | 9.E-04 | 0.6 |
| MCI | Inc-GSX2-7:1 | SEQ4820 | 9.E-05 | 0.6 | All AD | Inc-ZNF740-2:1 | SEQ5924 | 3.E-02 | 0.8 |
| miAD | Inc-GSX2-7:1 | SEQ4820 | 1.E-03 | 0.6 | DLB | Inc-ZNF75D-2:2 | SEQ5659 | 1.E-04 | 1.5 |
| All AD | Inc-GSX2-7:1 | SEQ4820 | 6.E-03 | 0.6 | DLB | Inc-ZNF778-1:4 | SEQ2865 | 8.E-04 | 1.5 |
| All AD | Inc-GTDC1-1:2 | SEQ4821 | 4.E-02 | 0.7 | MCI | Inc-ZNF778-5:1 | SEQ5125 | 6.E-04 | 1.6 |
| FTD | Inc-GTDC1-12:1 | SEQ4822 | 1. E-04 | 0.6 | DLB | Inc-ZNF778-5:1 | SEQ5125 | 6.E-04 | 1.6 |
| All AD | Inc-GTDC1-12:1 | SEQ4822 | 3.E-02 | 0.7 | DLB | Inc-ZNF780B-1:1 | SEQ4526 | 2.E-03 | 1.6 |
| FTD | Inc-GTDC1-17:1 | SEQ5961 | 6.E-06 | 0.3 | FTD | Inc-ZNF792-4:1 | SEQ5866 | 4.E-05 | 0.5 |
| FTD | Inc-GTDC1-21:1 | SEQ5081 | 6.E-04 | 0.5 | DLB | Inc-ZNF8-2:1 | SEQ5379 | 3.E-04 | 1.6 |
| FTD | Inc-GTDC1-22:1 | SEQ5394 | 3.E-04 | 0.5 | DLB | Inc-ZNF835-2:1 | SEQ3790 | 4.E-04 | 1.5 |
| FTD | Inc-GTDC1-28:1 | SEQ5925 | 1.E-05 | 0.6 | msAD | Inc-ZNF835-2:1 | SEQ3790 | 4.E-02 | 1.2 |
| MCI | Inc-GTDC1-3:1 | SEQ3956 | 3.E-06 | 3.2 | All AD | Inc-ZNF835-2:1 | SEQ3790 | 4.E-02 | 1.2 |
| DLB | Inc-GTDC1-3:1 | SEQ3956 | 9.E-06 | 3.1 | DLB | Inc-ZNF836-2:4 | SEQ4699 | 3.E-05 | 1.7 |
| FTD | Inc-GTDC1-3:1 | SEQ3956 | 3.E-05 | 2.5 | MCI | Inc-ZNF836-2:4 | SEQ4699 | 1.E-03 | 1.5 |
| All AD | Inc-GTDC1-3:1 | SEQ3956 | 2.E-02 | 1.3 | DLB | Inc-ZNF837-1:2 | SEQ3783 | 2.E-05 | 2.6 |
| msAD | Inc-GTDC1-3:1 | SEQ3956 | 3.E-02 | 1.3 | MCI | Inc-ZNF837-1:2 | SEQ3783 | 3.E-05 | 2.6 |
| FTD | Inc-GTDC1-3:2 | SEQ5968 | 4.E-06 | 0.5 | FTD | Inc-ZNF837-1:2 | SEQ3783 | 8.E-05 | 2.1 |
| FTD | Inc-GTDC1-5:1 | SEQ5395 | 3.E-04 | 0.5 | miAD | Inc-ZNF837-1:2 | SEQ3783 | 3.E-04 | 1.7 |
| DLB | Inc-GTF2IRD2B-3:1 | SEQ5755 | 8.E-05 | 1.7 | All AD | Inc-ZNF837-1:2 | SEQ3783 | 2.E-03 | 1.6 |
| miAD | Inc-GTPBP1-1:1 | SEQ4120 | 1.E-02 | 1.2 | msAD | Inc-ZNF837-1:2 | SEQ3783 | 4.E-02 | 1.4 |
| All AD | Inc-GTPBP1-1:1 | SEQ4120 | 2.E-02 | 1.2 | MCI | Inc-ZNF843-2:1 | SEQ5495 | 3.E-06 | 1.9 |
| DLB | Inc-GTPBP1-1:2 | SEQ3017 | 2.E-04 | 1.4 | DLB | Inc-ZNF843-2:1 | SEQ5495 | 2.E-04 | 1.8 |
| MCI | Inc-GTPBP1-1:2 | SEQ3017 | 2.E-04 | 1.4 | All AD | Inc-ZNF843-2:1 | SEQ5495 | 3.E-02 | 1.2 |
| FTD | Inc-H2AFJ-4:2 | SEQ5975 | 3.E-06 | 0.6 | MCI | Inc-ZNF843-2:4 | SEQ3465 | 2.E-05 | 2.0 |
| FTD | Inc-H2AFV-3:1 | SEQ2433 | 2.E-04 | 0.7 | DLB | Inc-ZNF843-2:4 | SEQ3465 | 1.E-04 | 1.6 |
| All AD | Inc-H3F3A-7:1 | SEQ4831 | 4.E-02 | 0.8 | FTD | Inc-ZNF843-2:4 | SEQ3465 | 5.E-04 | 1.4 |
| DLB | Inc-H6PD-2:1 | SEQ5426 | 3.E-04 | 1.4 | msAD | Inc-ZNF843-2:4 | SEQ3465 | 1.E-02 | 1.2 |
| DLB | Inc-HAGHL-1:1 | SEQ4505 | 2.E-05 | 1.8 | All AD | Inc-ZNF843-2:4 | SEQ3465 | 1.E-02 | 1.2 |
| MCI | Inc-HAGHL-1:1 | SEQ4505 | 2.E-03 | 1.5 | DLB | Inc-ZNF843-2:6 | SEQ4879 | 9.E-04 | 2.1 |
| DLB | Inc-HAND1-3:1 | SEQ5575 | 2.E-04 | 1.7 | MCI | Inc-ZNF843-2:7 | SEQ5631 | 7.E-07 | 2.1 |
| DLB | Inc-HAUS1-1:1 | SEQ4863 | 9.E-04 | 1.6 | DLB | Inc-ZNF843-2:7 | SEQ5631 | 2.E-04 | 1.8 |
| DLB | Inc-HBG2-1:1 | SEQ3409 | 1.E-03 | 1.4 | msAD | Inc-ZNF852-2:3 | SEQ2944 | 2.E-02 | 1.3 |
| DLB | Inc-HCN3-1:1 | SEQ5316 | 4.E-04 | 1.5 | MCI | Inc-ZNHIT2-1:1 | SEQ3818 | 2.E-06 | 1.9 |
| MCI | Inc-HDDC3-3:2 | SEQ4690 | 1.E-03 | 1.4 | DLB | Inc-ZNHIT2-1:1 | SEQ3818 | 3.E-05 | 1.8 |
| DLB | Inc-HDDC3-3:2 | SEQ4690 | 1.E-03 | 1.4 | FTD | Inc-ZNHIT2-1:1 | SEQ3818 | 5.E-04 | 1.4 |
| DLB | Inc-HDGFL2-7:1 | SEQ3180 | 2.E-04 | 1.8 | miAD | Inc-ZNHIT2-1:1 | SEQ3818 | 8.E-03 | 1.2 |
| MCI | Inc-HDGFL2-7:1 | SEQ3180 | 5.E-04 | 1.7 | All AD | Inc-ZNHIT2-1:1 | SEQ3818 | 9.E-03 | 1.2 |
| FTD | Inc-HELZ-4:1 | SEQ5783 | 7.E-05 | 0.7 | msAD | Inc-ZNHIT2-1:1 | SEQ3818 | 4.E-02 | 1.2 |
| FTD | Inc-HEPH-1:1 | SEQ4549 | 2.E-05 | 0.6 | msAD | Inc-ZNRD1-4:2 | SEQ4122 | 1.E-02 | 0.5 |
| MCI | Inc-HEPH-1:1 | SEQ4549 | 2.E-03 | 0.6 | DLB | Inc-ZNRF2-1:1 | SEQ4700 | 1.E-03 | 1.5 |
| DLB | Inc-HIBADH-5:1 | SEQ3962 | 3.E-05 | 1.4 | FTD | Inc-ZNRF2-4:1 | SEQ5551 | 2.E-04 | 0.7 |
| msAD | Inc-HIBADH-5:1 | SEQ3962 | 2.E-02 | 1.1 | FTD | Inc-ZRANB1-4:1 | SEQ5067 | 7.E-04 | 0.7 |
| All AD | Inc-HIBADH-5:1 | SEQ3962 | 4.E-02 | 1.1 | All AD | Inc-ZRANB1-4:1 | SEQ5067 | 4.E-02 | 0.8 |
| DLB | Inc-HIP1-1:2 | SEQ4849 | 9.E-04 | 1.5 | MCI | Inc-ZSCAN10-1:3 | SEQ5651 | 1.E-04 | 0.5 |
| DLB | Inc-HIST1H2BJ-10:5 | SEQ5707 | 1.E-04 | 1.8 | FTD | Inc-ZSCAN10-1:5 | SEQ5093 | 6.E-04 | 0.7 |
| All AD | Inc-HIST1H3A-4:1 | SEQ4172 | 1.E-02 | 0.8 | DLB | Inc-ZSCAN10-1:7 | SEQ5323 | 4.E-04 | 1.5 |
| msAD | Inc-HIST1H3A-4:1 | SEQ4172 | 1.E-02 | 0.8 | DLB | Inc-ZSCAN10-3:32 | SEQ5713 | 1.E-04 | 2.4 |
| All AD | Inc-HIST1H3D-1:1 | SEQ4844 | 3.E-02 | 0.7 | DLB | Inc-ZSCAN10-7:1 | SEQ5370 | 2.E-04 | 1.4 |
| DLB | Inc-HIST1H3F-1:1 | SEQ5878 | 3.E-05 | 1.6 | MCI | Inc-ZSCAN10-7:1 | SEQ5370 | 3.E-04 | 1.4 |
| FTD | Inc-HIST1H4D-3:1 | SEQ4032 | 2.E-05 | 1.5 | DLB | Inc-ZSCANl-3:3 | SEQ5445 | 3.E-04 | 1.6 |
| DLB | Inc-HIST1H4D-3:1 | SEQ4032 | 2.E-05 | 1.6 | DLB | Inc-ZSCAN21-1:1 | SEQ5240 | 5.E-04 | 1.3 |
| MCI | Inc-HIST1H4D-3:1 | SEQ4032 | 3.E-05 | 1.5 | DLB | Inc-ZSCAN21-2:1 | SEQ5477 | 2.E-04 | 1.4 |
| miAD | Inc-HIST1H4D-3:1 | SEQ4032 | 2.E-03 | 1.2 | DLB | Inc-ZSCAN2-5:1 | SEQ5486 | 2.E-04 | 1.5 |
| All AD | Inc-HIST1H4D-3:1 | SEQ4032 | 3.E-03 | 1.2 | DLB | Inc-ZSCAN2-5:10 | SEQ4859 | 9.E-04 | 1.5 |
| msAD | Inc-HIST1H4D-3:1 | SEQ4032 | 2.E-02 | 1.2 | DLB | Inc-ZSWIM8-2:3 | SEQ4715 | 1.E-03 | 1.6 |
| FTD | Inc-HIST2H2AA3-1:1 | SEQ5069 | 7.E-04 | 0.7 | MCI | LRRC75A-AS1:63 | SEQ5137 | 6.E-04 | 1.8 |
| FTD | Inc-HIST2H2BF-5:1 | SEQ5366 | 3.E-06 | 0.4 | FTD | LUCAT1:1 | SEQ4330 | 3.E-05 | 0.6 |
| MCI | Inc-HIST2H2BF-5:1 | SEQ5366 | 3.E-04 | 0.5 | miAD | LUCAT1:1 | SEQ4330 | 6.E-03 | 0.6 |
| FTD | Inc-HIST2H3A-1:2 | SEQ4852 | 8.E-04 | 0.6 | All AD | LUCAT1:1 | SEQ4330 | 3.E-02 | 0.7 |
| All AD | Inc-HIST2H3A-1:2 | SEQ4852 | 2.E-02 | 0.7 | FTD | LUCAT1:14 | SEQ4151 | 3.E-04 | 0.6 |
| All AD | Inc-HIST2H3D-1:1 | SEQ2368 | 1.E-03 | 0.5 | miAD | LUCAT1:14 | SEQ4151 | 1.E-02 | 0.7 |
| msAD | Inc-HIST2H3D-1:1 | SEQ2368 | 3.E-03 | 0.5 | All AD | LUCAT1:14 | SEQ4151 | 2.E-02 | 0.7 |
| miAD | Inc-HIST2H3D-1:1 | SEQ2368 | 3.E-03 | 0.6 | miAD | LUCAT1:18 | SEQ4351 | 4.E-03 | 0.4 |
| miAD | Inc-HIST2H3PS2-10:1 | SEQ4664 | 1.E-03 | 0.4 | All AD | LUCAT1:18 | SEQ4351 | 1.E-02 | 0.5 |
| All AD | Inc-HIST2H3PS2-10:1 | SEQ4664 | 8.E-03 | 0.4 | All AD | LUCAT1:2 | SEQ5932 | 4.E-02 | 0.8 |
| DLB | Inc-HIST3H2BB-1:4 | SEQ5845 | 4.E-05 | 1.5 | All AD | LUCAT1:20 | SEQ5933 | 3.E-02 | 0.7 |
| FTD | Inc-HIST4H4-1:1 | SEQ5921 | 1.E-05 | 0.2 | All AD | LUCAT1:21 | SEQ5934 | 2.E-02 | 0.7 |
| FTD | Inc-HIST4H4-1:2 | SEQ5750 | 2.E-05 | 0.2 | FTD | LUCAT1:24 | SEQ5510 | 2.E-04 | 0.6 |
| MCI | Inc-HIST4H4-1:2 | SEQ5750 | 8.E-05 | 0.3 | All AD | LUCAT1:24 | SEQ5510 | 2.E-02 | 0.7 |
| All AD | Inc-HIST4H4-4:1 | SEQ4860 | 5.E-02 | 0.8 | FTD | LUCAT1:3 | SEQ5511 | 2.E-04 | 0.6 |
| DLB | Inc-HIST4H4-5:3 | SEQ5897 | 2.E-05 | 2.0 | All AD | LUCAT1:3 | SEQ5511 | 2.E-02 | 0.7 |
| DLB | Inc-HLA-B-2:13 | SEQ4606 | 2.E-04 | 0.2 | FTD | MALAT1:16 | SEQ4201 | 5.E-04 | 0.4 |
| MCI | Inc-HLA-B-2:13 | SEQ4606 | 1.E-03 | 0.2 | msAD | MALAT1:16 | SEQ4201 | 1.E-02 | 0.5 |
| MCI | Inc-HLA-DMA-1:1 | SEQ5942 | 2.E-07 | 1.7 | All AD | MALAT1:16 | SEQ4201 | 2.E-02 | 0.5 |
| DLB | Inc-HLA-DMA-1:1 | SEQ5942 | 4.E-06 | 1.7 | MCI | MALAT1:24 | SEQ4397 | 2.E-04 | 0.5 |
| FTD | Inc-HLA-DMA-1:1 | SEQ5942 | 1.E-05 | 1.4 | DLB | MALAT1:24 | SEQ4397 | 2.E-03 | 0.6 |
| FTD | Inc-HLA-DQA1-7:1 | SEQ5335 | 6.E-05 | 1.7 | miAD | MALAT1:32 | SEQ3633 | 7.E-03 | 0.6 |
| DLB | Inc-HLA-DQA1-7:1 | SEQ5335 | 4.E-04 | 1.7 | All AD | MALAT1:32 | SEQ3633 | 1.E-02 | 0.6 |
| DLB | Inc-HLA-DRB1-5:1 | SEQ4166 | 8.E-04 | 2.7 | msAD | MALAT1:32 | SEQ3633 | 5.E-02 | 0.7 |
| All AD | Inc-HLA-DRB1-5:1 | SEQ4166 | 4.E-03 | 1.6 | FTD | MALAT1:33 | SEQ5927 | 3.E-11 | 4.E-15 |
| msAD | Inc-HLA-DRB1-5:1 | SEQ4166 | 7.E-03 | 1.6 | DLB | MALAT1:33 | SEQ5927 | 1.E-05 | 3.E-15 |
| miAD | Inc-HLA-DRB1-5:1 | SEQ4166 | 1.E-02 | 1.5 | MCI | MALAT1:33 | SEQ5927 | 1.E-05 | 3.E-15 |
| DLB | Inc-HLA-DRB1-5:2 | SEQ5347 | 4.E-04 | 2.4 | MCI | MALAT1:38 | SEQ5650 | 1.E-06 | 0.3 |
| DLB | Inc-HMGA1-2:4 | SEQ3415 | 5.E-04 | 1.3 | FTD | MALAT1:38 | SEQ5650 | 2.E-06 | 0.3 |
| FTD | Inc-HMGB1-4:1 | SEQ5160 | 5.E-04 | 0.7 | DLB | MALAT1:38 | SEQ5650 | 1.E-04 | 0.4 |
| FTD | Inc-HMGB2-5:1 | SEQ3870 | 3.E-05 | 0.6 | DLB | MALAT1:4 | SEQ4834 | 9.E-04 | 1.4 |
| miAD | Inc-HMGB2-5:1 | SEQ3870 | 6.E-03 | 0.8 | MCI | MALAT1:40 | SEQ5556 | 2.E-04 | 0.4 |
| All AD | Inc-HMGB2-5:1 | SEQ3870 | 7.E-03 | 0.8 | DLB | MALAT1:43 | SEQ5018 | 7.E-04 | 1.4 |
| msAD | Inc-HMGB2-5:1 | SEQ3870 | 3.E-02 | 0.8 | DLB | MALAT1:5 | SEQ5019 | 7.E-04 | 1.4 |
| FTD | Inc-HMGB2-8:1 | SEQ5802 | 6.E-05 | 0.6 | DLB | MALAT1:6 | SEQ5016 | 7.E-04 | 1.4 |
| FTD | Inc-HMGN5-3:1 | SEQ3416 | 1.E-09 | 0.4 | DLB | MAP3K14-AS1:27 | SEQ5184 | 5.E-04 | 1.5 |
| MCI | Inc-HMGN5-3:1 | SEQ3416 | 2.E-05 | 0.5 | MCI | MAP3K14-AS1:28 | SEQ4862 | 9.E-04 | 1.5 |
| All AD | Inc-HMGN5-3:1 | SEQ3416 | 1.E-03 | 0.6 | DLB | MAP3K14-AS1:28 | SEQ4862 | 9.E-04 | 1.6 |
| miAD | Inc-HMGN5-3:1 | SEQ3416 | 3.E-03 | 0.6 | FTD | MAP3K20-AS1:6 | SEQ5508 | 2.E-04 | 0.5 |
| msAD | Inc-HMGN5-3:1 | SEQ3416 | 4.E-03 | 0.6 | All AD | MATN1-AS1:2 | SEQ5940 | 3.E-02 | 1.2 |
| FTD | Inc-HNRNPA2B1-14:2 | SEQ5828 | 5.E-05 | 0.5 | DLB | MBNL1-AS1:10 | SEQ5456 | 3.E-04 | 1.9 |
| FTD | Inc-HNRNPA2B1-15:1 | SEQ4878 | 6.E-10 | 0.3 | DLB | MBNL1-AS1:5 | SEQ4754 | 1.E-03 | 1.4 |
| MCI | Inc-HNRNPA2B1-15:1 | SEQ4878 | 3.E-06 | 0.3 | miAD | MBNL1-AS1:8 | SEQ4335 | 6.E-03 | 1.5 |
| DLB | Inc-H NRN PA2Bl-15: 1 | SEQ4878 | 5.E-04 | 0.4 | All AD | MBNL1-AS1:8 | SEQ4335 | 1.E-02 | 1.3 |
| miAD | Inc-HNRNPA2B1-15:1 | SEQ4878 | 6.E-04 | 0.4 | DLB | MCM3AP-AS1:8 | SEQ4507 | 2.E-03 | 1.5 |
| All AD | Inc-HNRNPA2B1-15:1 | SEQ4878 | 3.E-03 | 0.5 | FTD | MIF-AS1:7 | SEQ3858 | 8.E-06 | 1.7 |
| MCI | Inc-HNRNPA2B1-15:3 | SEQ4795 | 1.E-03 | 2.8 | MCI | MIF-AS1:7 | SEQ3858 | 9.E-06 | 1.8 |
| FTD | Inc-HNRNPA2B1-15:4 | SEQ4601 | 1.E-06 | 0.5 | DLB | MIF-AS1:7 | SEQ3858 | 1.E-05 | 1.9 |
| MCI | Inc-HNRNPA2B1-15:4 | SEQ4601 | 2.E-05 | 0.5 | miAD | MIF-AS1:7 | SEQ3858 | 7.E-03 | 1.3 |
| miAD | Inc-HNRNPA281-15:4 | SEQ4601 | 1.E-03 | 0.6 | All AD | MIF-AS1:7 | SEQ3858 | 8.E-03 | 1.3 |
| All AD | Inc-HNRNPA2B1-15:4 | SEQ4601 | 6.E-03 | 0.6 | msAD | MIF-AS1:7 | SEQ3858 | 3.E-02 | 1.3 |
| All AD | Inc-HNRNPA2B1-15:5 | SEQ4882 | 4.E-02 | 0.7 | FTD | MIR133A1HG:2 | SEQ5642 | 1.E-04 | 0.6 |
| MCI | Inc-HNRNPA3-2:2 | SEQ5073 | 2.E-04 | 1.8 | FTD | MIR133A1HG:3 | SEQ5855 | 4.E-05 | 0.5 |
| DLB | Inc-HNRNPA3-2:2 | SEQ5073 | 5.E-04 | 1.7 | MCI | MIR17HG:13 | SEQ5996 | 7.E-07 | 3.1 |
| FTD | Inc-HNRNPA3-2:2 | SEQ5073 | 7.E-04 | 1.5 | FTD | MIR17HG:21 | SEQ4799 | 1.E-03 | 0.4 |
| FTD | Inc-HNRNPLL-4:1 | SEQ4287 | 2.E-06 | 0.5 | FTD | MIR181A2HG:1 | SEQ3905 | 9.E-04 | 0.6 |
| msAD | Inc-HNRNPLL-4:1 | SEQ4287 | 7.E-03 | 0.7 | miAD | MIR181A2HG:1 | SEQ3905 | 5.E-03 | 0.6 |
| All AD | Inc-HNRNPLL-4:1 | SEQ4287 | 8.E-03 | 0.7 | All AD | MIR181A2HG:1 | SEQ3905 | 6.E-03 | 0.7 |
| FTD | Inc-HNRNPU-1:3 | SEQ4467 | 4.E-04 | 0.4 | msAD | MIR181A2HG:1 | SEQ3905 | 3.E-02 | 0.8 |
| MCI | Inc-HNRNPU-1:3 | SEQ4467 | 2.E-03 | 0.4 | FTD | MIR222HG:26 | SEQ5396 | 3.E-04 | 0.5 |
| FTD | Inc-HNRNPU-1:6 | SEQ4889 | 7.E-05 | 0.4 | DLB | MIR29B2CHG:14 | SEQ5637 | 2.E-04 | 1.9 |
| All AD | Inc-HNRNPU-1:6 | SEQ4889 | 5.E-02 | 0.6 | DLB | MIR29B2CHG:21 | SEQ4530 | 2.E-03 | 1.8 |
| DLB | Inc-HOMEZ-1:6 | SEQ5032 | 7.E-04 | 1.5 | DLB | MIR29B2CHG:22 | SEQ4655 | 1.E-03 | 1.9 |
| All AD | Inc-HPCA-1:1 | SEQ4891 | 4.E-02 | 0.8 | MCI | MIR29B2CHG:28 | SEQ5457 | 3.E-04 | 1.9 |
| FTD | Inc-HPGD-5:1 | SEQ5827 | 5.E-05 | 0.5 | FTD | MIR29B2CHG:33 | SEQ0583 | 2.E-05 | 0.4 |
| DLB | Inc-HSD11B2-1:1 | SEQ2525 | 1.E-03 | 1.3 | All AD | MIR29B2CHG:33 | SEQ0583 | 2.E-02 | 0.7 |
| DLB | Inc-HSD17B6-3:1 | SEQ3950 | 7.E-07 | 2.0 | All AD | MIR29B2CHG:34 | SEQ5945 | 2.E-02 | 0.7 |
| MCI | Inc-HSD17B6-3:1 | SEQ3950 | 3.E-06 | 1.8 | MCI | MIR29B2CHG:48 | SEQ5099 | 7.E-06 | 0.3 |
| FTD | Inc-HSD17B6-3:1 | SEQ3950 | 6.E-06 | 1.7 | FTD | MIR29B2CHG:48 | SEQ5099 | 1.E-05 | 0.3 |
| All AD | Inc-HSD17B6-3:1 | SEQ3950 | 2.E-02 | 1.2 | DLB | MIR29B2CHG:48 | SEQ5099 | 6.E-04 | 0.3 |
| msAD | Inc-HSD17B6-3:1 | SEQ3950 | 3.E-02 | 1.2 | DLB | MIR29B2CHG:8 | SEQ4457 | 5.E-05 | 2.0 |
| All AD | Inc-HSDL1-2:1 | SEQ3753 | 3.E-02 | 1.1 | MCI | MIR29B2CHG:8 | SEQ4457 | 2.E-03 | 1.9 |
| msAD | Inc-HSDL1-2:1 | SEQ3753 | 4.E-02 | 1.2 | All AD | MIR3936HG:4 | SEQ3771 | 2.E-02 | 0.7 |
| FTD | Inc-HSP90AA1-13:1 | SEQ5591 | 2.E-04 | 0.6 | msAD | MIR3936HG:4 | SEQ3771 | 4.E-02 | 0.7 |
| All AD | Inc-HSP90AA1-15:2 | SEQ4897 | 4.E-02 | 0.8 | DLB | MIR4435-2HG:43 | SEQ4650 | 1.E-03 | 1.8 |
| DLB | Inc-HSP90AA1-2:1 | SEQ5612 | 2.E-04 | 1.4 | FTD | MIR4435-2HG:6 | SEQ5005 | 8.E-04 | 1.7 |
| DLB | Inc-HSPB1-4:4 | SEQ5813 | 5.E-05 | 1.4 | DLB | MIR4453HG:2 | SEQ3568 | 1.E-03 | 1.4 |
| DLB | Inc-HUS1B-2:1 | SEQ4588 | 2.E-03 | 1.6 | DLB | MIR600HG:4 | SEQ3892 | 6.E-04 | 1.9 |
| DLB | Inc-HYAL4-4:6 | SEQ4618 | 2.E-04 | 1.5 | msAD | MIR600HG:4 | SEQ3892 | 3.E-02 | 1.4 |
| MCI | Inc-HYAL4-4:6 | SEQ4618 | 1.E-03 | 1.4 | All AD | MIR600HG:4 | SEQ3892 | 4.E-02 | 1.3 |
| DLB | Inc-ICA1L-7:1 | SEQ4901 | 5.E-04 | 2.1 | DLB | MIR600HG:5 | SEQ3676 | 1.E-03 | 2.0 |
| MCI | Inc-ICA1L-7:1 | SEQ4901 | 5.E-04 | 2.1 | msAD | MIR600HG:5 | SEQ3676 | 5.E-02 | 1.4 |
| All AD | Inc-ICA1L-7:1 | SEQ4901 | 5.E-02 | 1.3 | FTD | MIR646HG:26 | SEQ3222 | 9.E-04 | 0.6 |
| MCI | Inc-ICAMS-1:1 | SEQ4240 | 1.E-05 | 1.7 | FTD | MIR646HG:29 | SEQ3928 | 1.E-05 | 0.5 |
| DLB | Inc-ICAMS-1:1 | SEQ4240 | 1.E-05 | 1.9 | miAD | MIR646HG:29 | SEQ3928 | 1.E-03 | 0.6 |
| FTD | Inc-ICAMS-1:1 | SEQ4240 | 2.E-04 | 1.4 | All AD | MIR646HG:29 | SEQ3928 | 3.E-03 | 0.6 |
| All AD | Inc-ICAMS-1:1 | SEQ4240 | 4.E-03 | 1.3 | msAD | MIR646HG:29 | SEQ3928 | 3.E-02 | 0.7 |
| miAD | Inc-ICAMS-1:1 | SEQ4240 | 8.E-03 | 1.3 | miAD | MIR646HG:35 | SEQ4313 | 7.E-03 | 0.7 |
| msAD | Inc-ICAMS-1:1 | SEQ4240 | 1.E-02 | 1.3 | All AD | MIR646HG:35 | SEQ4313 | 2.E-02 | 0.8 |
| DLB | Inc-ICOSLG-6:12 | SEQ4450 | 8.E-04 | 1.6 | FTD | MME-AS1:2 | SEQ4056 | 2.E-06 | 0.4 |
| MCI | Inc-ICOSLG-6:12 | SEQ4450 | 2.E-03 | 1.6 | miAD | MME-AS1:2 | SEQ4056 | 3.E-03 | 0.6 |
| miAD | Inc-IDE-3:1 | SEQ4211 | 1.E-02 | 0.7 | All AD | MME-AS1:2 | SEQ4056 | 3.E-03 | 0.6 |
| MCI | Inc-IER3-7:16 | SEQ5183 | 5.E-04 | 1.5 | msAD | MME-AS1:2 | SEQ4056 | 2.E-02 | 0.6 |
| MCI | Inc-IER3-7:17 | SEQ4560 | 1.E-04 | 1.6 | MCI | MMP25-AS1:17 | SEQ4787 | 1.E-03 | 1.8 |
| DLB | Inc-IER3-7:17 | SEQ4560 | 2.E-03 | 1.4 | All AD | MMP25-AS1:24 | SEQ5949 | 3.E-02 | 0.8 |
| DLB | Inc-IERS-4:1 | SEQ5876 | 3.E-05 | 1.5 | MCI | MRPL23-AS1:10 | SEQ3942 | 9.E-05 | 1.7 |
| DLB | Inc-IFT140-2:1 | SEQ5623 | 2.E-04 | 1.6 | DLB | MRPL23-AS1:10 | SEQ3942 | 1.E-04 | 1.7 |
| FTD | Inc-IGIP-2:3 | SEQ3421 | 8.E-06 | 0.3 | FTD | MRPL23-AS1:10 | SEQ3942 | 7.E-04 | 1.5 |
| MCI | Inc-IGIP-2:3 | SEQ3421 | 2.E-04 | 0.3 | msAD | MRPL23-AS1:10 | SEQ3942 | 3.E-02 | 1.2 |
| DLB | Inc-IGIP-2:3 | SEQ3421 | 2.E-03 | 0.4 | All AD | MRPL23-AS1:10 | SEQ3942 | 3.E-02 | 1.2 |
| miAD | Inc-IGIP-5:1 | SEQ2977 | 1.E-02 | 0.7 | MCI | MRPS30-DT:11 | SEQ4024 | 2.E-04 | 1.7 |
| All AD | Inc-IGIP-5:1 | SEQ2977 | 2.E-02 | 0.7 | DLB | MRPS30-DT:11 | SEQ4024 | 9.E-04 | 1.6 |
| MCI | Inc-IGLONS-6:10 | SEQ4600 | 8.E-05 | 2.1 | All AD | MRPS30-DT:11 | SEQ4024 | 1.E-02 | 1.3 |
| msAD | Inc-IGLONS-6:10 | SEQ4600 | 1.E-03 | 1.7 | msAD | MRPS30-DT:11 | SEQ4024 | 2.E-02 | 1.3 |
| DLB | Inc-IGLONS-6:10 | SEQ4600 | 2.E-03 | 1.9 | FTD | MRVI1-AS1:16 | SEQ5778 | 7.E-05 | 0.6 |
| All AD | Inc-IGLONS-6:10 | SEQ4600 | 4.E-03 | 1.5 | FTD | MYLK-AS1:13 | SEQ5471 | 3.E-04 | 0.7 |
| FTD | Inc-IGLONS-6:3 | SEQ4129 | 1.E-08 | 4.7 | DLB | MYLK-AS1:16 | SEQ3954 | 1.E-03 | 1.5 |
| MCI | Inc-IGLONS-6:3 | SEQ4129 | 3.E-08 | 7.5 | MCI | MYLK-AS1:16 | SEQ3954 | 2.E-03 | 1.5 |
| DLB | Inc-IGLONS-6:3 | SEQ4129 | 3.E-05 | 3.7 | msAD | MYLK-AS1:16 | SEQ3954 | 3.E-02 | 1.3 |
| All AD | Inc-IGLONS-6:3 | SEQ4129 | 1.E-02 | 1.6 | miAD | MZF1-AS1:11 | SEQ4877 | 6.E-04 | 1.9 |
| msAD | Inc-IGLONS-6:3 | SEQ4129 | 1.E-02 | 1.7 | DLB | MZF1-AS1:11 | SEQ4877 | 9.E-04 | 2.1 |
| DLB | Inc-IGLONS-6:S | SEQ4265 | 2.E-03 | 1.7 | All AD | MZF1-AS1:11 | SEQ4877 | 3.E-03 | 1.6 |
| msAD | Inc-IGLONS-6:S | SEQ4265 | 9.E-03 | 1.4 | FTD | N4BP2L2-IT2:1 | SEQ5958 | 7.E-06 | 0.6 |
| FTD | Inc-IGSF10-8:1 | SEQ5929 | 1.E-05 | 0.5 | DLB | NAPA-AS1:10 | SEQ5425 | 3.E-04 | 1.4 |
| DLB | Inc-IKBIP-1:1 | SEQ4407 | 2.E-03 | 1.4 | All AD | N CAM 1-AS 1:1 | SEQ5953 | 4.E-02 | 0.7 |
| DLB | Inc-IL18BP-1:1 | SEQ3303 | 1.E-03 | 1.5 | miAD | NCF4-AS1:4 | SEQ4261 | 9.E-03 | 0.7 |
| DLB | Inc-IL18BP-1:2 | SEQ3422 | 8.E-04 | 1.5 | All AD | NCF4-AS1:4 | SEQ4261 | 3.E-02 | 0.8 |
| MCI | Inc-IL18BP-1:2 | SEQ3422 | 1.E-03 | 1.4 | MCI | NEAT1:11 | SEQ4370 | 1.E-08 | 6.E+04 |
| DLB | Inc-IL18BP-1:3 | SEQ3423 | 2.E-03 | 1.5 | FTD | NEAT1:11 | SEQ4370 | 8.E-07 | 16.4 |
| FTD | Inc-IL18R1-1:1 | SEQ4043 | 5.E-04 | 0.6 | DLB | NEAT1:11 | SEQ4370 | 2.E-04 | 5.6 |
| All AD | Inc-IL18R1-1:1 | SEQ4043 | 1.E-02 | 0.7 | miAD | NEAT1:11 | SEQ4370 | 4.E-03 | 2.4 |
| msAD | Inc-IL18R1-1:1 | SEQ4043 | 2.E-02 | 0.7 | All AD | NEAT1:11 | SEQ4370 | 2.E-02 | 1.9 |
| DLB | Inc-IL1RN-5:1 | SEQ4497 | 2.E-03 | 1.4 | MCI | NEAT1:12 | SEQ4350 | 2.E-06 | 3.E+02 |
| DLB | Inc-IL23A-2:5 | SEQ4425 | 2.E-03 | 1.5 | FTD | NEAT1:12 | SEQ4350 | 1.E-05 | 8.4 |
| msAD | Inc-IL23A-2:6 | SEQ4341 | 5.E-03 | 1.2 | miAD | NEAT1:12 | SEQ4350 | 5.E-03 | 2.4 |
| All AD | Inc-IL23A-2:6 | SEQ4341 | 1.E-02 | 1.2 | All AD | NEAT1:12 | SEQ4350 | 1.E-02 | 2.2 |
| DLB | Inc-IL2RA-5:1 | SEQ4484 | 2.E-03 | 1.4 | FTD | NEAT1:18 | SEQ3809 | 5.E-05 | 0.2 |
| DLB | Inc-IL31RA-1:1 | SEQ2680 | 3.E-04 | 1.6 | miAD | NEAT1:18 | SEQ3809 | 1.E-03 | 0.1 |
| MCI | Inc-IL31RA-1:1 | SEQ2680 | 2.E-03 | 1.5 | DLB | NEAT1:18 | SEQ3809 | 1.E-03 | 0.3 |
| miAD | Inc-IL31RA-1:1 | SEQ2680 | 3.E-03 | 1.4 | All AD | NEAT1:18 | SEQ3809 | 3.E-03 | 0.2 |
| All AD | Inc-IL31RA-1:1 | SEQ2680 | 4.E-03 | 1.3 | msAD | NEAT1:18 | SEQ3809 | 4.E-02 | 0.3 |
| msAD | Inc-IL31RA-1:1 | SEQ2680 | 3.E-02 | 1.3 | FTD | NEAT1:20 | SEQ4469 | 2.E-06 | 0.5 |
| MCI | Inc-IL31RA-6:2 | SEQ4535 | 2.E-03 | 2.2 | DLB | NEAT1:20 | SEQ4469 | 5.E-04 | 0.6 |
| All AD | Inc-IL31RA-6:2 | SEQ4535 | 4.E-02 | 1.4 | MCI | NEAT1:20 | SEQ4469 | 2.E-03 | 0.5 |
| DLB | Inc-IL9R-1:3 | SEQ5657 | 1.E-04 | 1.5 | All AD | NEAT1:20 | SEQ4469 | 3.E-02 | 0.7 |
| DLB | Inc-INO80E-2:2 | SEQ5191 | 5.E-04 | 1.5 | All AD | NEAT1:21 | SEQ5957 | 3.E-02 | 0.8 |
| FTD | Inc-INSL4-4:1 | SEQ5105 | 2.E-05 | 0.6 | miAD | NEAT1:22 | SEQ4986 | 2.E-06 | 0.5 |
| MCI | Inc-INSL4-4:1 | SEQ5105 | 6.E-04 | 0.6 | All AD | NEAT1:22 | SEQ4986 | 2.E-05 | 0.6 |
| FTD | Inc-INSL4-5:1 | SEQ4133 | 4.E-07 | 0.5 | msAD | NEAT1:22 | SEQ4986 | 8.E-04 | 0.6 |
| miAD | Inc-INSL4-5:1 | SEQ4133 | 1.E-02 | 0.7 | MCI | NEAT1:7 | SEQ4362 | 4.E-08 | 8.E+03 |
| All AD | Inc-INSL4-5:1 | SEQ4133 | 3.E-02 | 0.7 | FTD | NEAT1:7 | SEQ4362 | 8.E-06 | 4.8 |
| FTD | Inc-INSL4-6:1 | SEQ5423 | 1.E-07 | 0.4 | miAD | NEAT1:7 | SEQ4362 | 4.E-03 | 2.1 |
| MCI | Inc-INSL4-6:1 | SEQ5423 | 3.E-04 | 0.5 | All AD | NEAT1:7 | SEQ4362 | 1.E-02 | 2.2 |
| FTD | Inc-INSL4-8:1 | SEQ5406 | 3.E-04 | 0.6 | FTD | NEAT1:8 | SEQ4734 | 4.E-07 | 0.4 |
| FTD | Inc-INSL4-9:1 | SEQ4937 | 8.E-07 | 0.5 | MCI | NEAT1:8 | SEQ4734 | 1.E-03 | 0.5 |
| MCI | Inc-INSL4-9:1 | SEQ4937 | 1.E-04 | 0.5 | All AD | NEAT1:8 | SEQ4734 | 2.E-02 | 0.5 |
| All AD | Inc-INSL4-9:1 | SEQ4937 | 3.E-02 | 0.7 | MCI | NIPBL-AS1:1 | SEQ5100 | 6.E-04 | 0.4 |
| FTD | Inc-INSL6-3:1 | SEQ4906 | 9.E-04 | 0.7 | DLB | NIPBL-AS1:2 | SEQ4983 | 8.E-04 | 2.2 |
| FTD | Inc-INTS14-1:2 | SEQ5083 | 6.E-04 | 0.6 | FTD | NIPBL-AS1:6 | SEQ5744 | 9.E-05 | 0.6 |
| FTD | Inc-INTS9-2:1 | SEQ4940 | 8.E-05 | 0.6 | FTD | NORAD:10 | SEQ4733 | 7.E-07 | 2.1 |
| All AD | Inc-INTS9-2:1 | SEQ4940 | 3.E-02 | 0.8 | DLB | NORAD:10 | SEQ4733 | 1.E-05 | 2.0 |
| All AD | Inc-IPCEF1-2:1 | SEQ4941 | 3.E-02 | 1.2 | MCI | NORAD:10 | SEQ4733 | 9.E-05 | 2.1 |
| DLB | Inc-IQCF6-2:2 | SEQ4856 | 9.E-04 | 1.5 | All AD | NORAD:10 | SEQ4733 | 4.E-04 | 1.4 |
| All AD | Inc-IQCG-16:1 | SEQ4943 | 3.E-02 | 0.7 | miAD | NORAD:10 | SEQ4733 | 1.E-03 | 1.4 |
| All AD | Inc-IRF1-2:1 | SEQ4944 | 3.E-02 | 0.8 | msAD | NORAD:10 | SEQ4733 | 1.E-03 | 1.4 |
| All AD | Inc-IRF1-2:6 | SEQ4945 | 3.E-02 | 0.8 | FTD | NORAD:2 | SEQ3125 | 1.E-08 | 4.2 |
| DLB | Inc-IRF1-6:1 | SEQ5756 | 8.E-05 | 1.8 | MCI | NORAD:2 | SEQ3125 | 4.E-06 | 3.6 |
| DLB | Inc-IRF7-1:1 | SEQ5624 | 1.E-05 | 1.8 | DLB | NORAD:2 | SEQ3125 | 2.E-05 | 2.8 |
| MCI | Inc-IRF7-1:1 | SEQ5624 | 2.E-04 | 1.6 | All AD | NORAD:2 | SEQ3125 | 2.E-03 | 1.5 |
| All AD | Inc-IRS2-1:6 | SEQ3812 | 1.E-02 | 0.6 | miAD | NORAD:2 | SEQ3125 | 3.E-03 | 1.5 |
| miAD | Inc-IRS2-1:6 | SEQ3812 | 2.E-02 | 0.5 | msAD | NORAD:2 | SEQ3125 | 1.E-02 | 1.5 |
| msAD | Inc-IRS2-1:6 | SEQ3812 | 4.E-02 | 0.6 | All AD | NORAD:5 | SEQ3646 | 3.E-02 | 1.2 |
| msAD | Inc-IRS2-9:1 | SEQ3896 | 3.E-02 | 0.8 | msAD | NORAD:5 | SEQ3646 | 5.E-02 | 1.2 |
| All AD | Inc-IRS2-9:1 | SEQ3896 | 4.E-02 | 0.8 | FTD | NORAD:8 | SEQ2585 | 8.E-07 | 0.3 |
| MCI | Inc-ITGB1BP2-2:1 | SEQ2701 | 1.E-03 | 0.7 | MCI | NORAD:8 | SEQ2585 | 3.E-06 | 0.3 |
| DLB | Inc-ITGB1BP2-2:3 | SEQ5562 | 2.E-04 | 1.5 | All AD | NORAD:8 | SEQ2585 | 3.E-02 | 0.5 |
| DLB | Inc-ITPA-1:1 | SEQ5904 | 2.E-05 | 1.4 | FTD | NORAD:9 | SEQ2494 | 4.E-04 | 0.4 |
| MCI | Inc-ITPA-2:3 | SEQ4577 | 2.E-03 | 1.5 | MCI | NORAD:9 | SEQ2494 | 6.E-04 | 0.5 |
| DLB | Inc-ITPA-2:3 | SEQ4577 | 2.E-03 | 1.5 | All AD | NORAD:9 | SEQ2494 | 5.E-02 | 0.6 |
| FTD | Inc-JAK1-2:2 | SEQ4953 | 4.E-04 | 0.7 | DLB | NRSN2-AS1:11 | SEQ5450 | 3.E-04 | 1.7 |
| All AD | Inc-JAK1-2:2 | SEQ4953 | 2.E-02 | 0.8 | FTD | NUP50-AS1:20 | SEQ5088 | 6.E-04 | 0.6 |
| FTD | Inc-JCAD-10:1 | SEQ4954 | 1.E-05 | 0.6 | FTD | NUP50-AS1:22 | SEQ4654 | 4.E-04 | 1.7 |
| All AD | Inc-JCAD-10:1 | SEQ4954 | 3.E-02 | 0.8 | DLB | NUP50-AS1:22 | SEQ4654 | 7.E-04 | 1.9 |
| DLB | Inc-JMJD7-PLA2G4B-2:8 | SEQ3428 | 8.E-04 | 1.6 | MCI | NUP50-AS1:22 | SEQ4654 | 1.E-03 | 1.9 |
| DLB | Inc-JSRP1-1:2 | SEQ5710 | 5.E-05 | 1.8 | FTD | NUP50-AS1:27 | SEQ5356 | 4.E-04 | 0.6 |
| MCI | Inc-JSRP1-1:2 | SEQ5710 | 1.E-04 | 1.8 | MCI | NUTM2A-AS1:2 | SEQ5749 | 8.E-05 | 0.3 |
| DLB | Inc-JSRP1-2:1 | SEQ4495 | 2.E-03 | 1.4 | FTD | NUTM2A-AS1:28 | SEQ5222 | 5.E-04 | 0.5 |
| MCI | Inc-JUNB-1:1 | SEQ5422 | 1.E-05 | 1.8 | MCI | NUTM2A-AS1:30 | SEQ5903 | 2.E-05 | 0.3 |
| DLB | Inc-JUNB-1:1 | SEQ5422 | 1.E-04 | 1.6 | FTD | NUTM2A-AS1:34 | SEQ4337 | 3.E-07 | 0.5 |
| FTD | Inc-JUNB-1:1 | SEQ5422 | 3.E-04 | 1.5 | MCI | NUTM2A-AS1:34 | SEQ4337 | 2.E-04 | 0.6 |
| MCI | Inc-KANTR-2:1 | SEQ4836 | 9.E-04 | 1.4 | miAD | NUTM2A-AS1:34 | SEQ4337 | 5.E-03 | 0.7 |
| DLB | Inc-KBTBD4-4:1 | SEQ4614 | 1.E-03 | 1.3 | All AD | NUTM2A-AS1:34 | SEQ4337 | 2.E-02 | 0.7 |
| MCI | Inc-KCNC2-1:1 | SEQ4321 | 9.E-05 | 1.7 | FTD | NUTM2B-AS1:20 | SEQ5596 | 2.E-04 | 0.4 |
| DLB | Inc-KCNC2-1:1 | SEQ4321 | 2.E-04 | 1.7 | FTD | NUTM2B-AS1:42 | SEQ4813 | 1. E-03 | 0.7 |
| All AD | Inc-KCNC2-1:1 | SEQ4321 | 1.E-03 | 1.3 | FTD | NUTM2B-AS1:53 | SEQ0004 | 8.E-04 | 0.5 |
| miAD | Inc-KCNC2-1:1 | SEQ4321 | 1.E-03 | 1.3 | FTD | OIPS-AS1:15 | SEQ4823 | 3.E-06 | 0.2 |
| msAD | Inc-KCNC2-1:1 | SEQ4321 | 6.E-03 | 1.3 | MCI | OIP5-AS1:15 | SEQ4823 | 6.E-06 | 0.2 |
| DLB | Inc-KCNC3-1:4 | SEQ4083 | 2.E-05 | 2.0 | DLB | OIP5-AS1:15 | SEQ4823 | 9.E-04 | 0.2 |
| msAD | Inc-KCNC3-1:4 | SEQ4083 | 1.E-02 | 1.3 | DLB | OIP5-AS1:18 | SEQ5034 | 7.E-04 | 1.5 |
| All AD | Inc-KCNC3-1:4 | SEQ4083 | 5.E-02 | 1.2 | FTD | OIPS-AS1:25 | SEQ5168 | 4.E-07 | 0.4 |
| FTD | Inc-KCNE1B-10:2 | SEQ5585 | 2.E-04 | 0.2 | MCI | OIP5-AS1:25 | SEQ5168 | 5.E-04 | 0.5 |
| msAD | Inc-KCNE1B-10:3 | SEQ4324 | 6.E-03 | 1.6 | MCI | OIP5-AS1:3 | SEQ4919 | 8.E-04 | 0.5 |
| All AD | Inc-KCNE1B-10:3 | SEQ4324 | 4.E-02 | 1.4 | FTD | OIP5-AS1:36 | SEQ5807 | 6.E-05 | 0.7 |
| miAD | Inc-KCNE1B-2:1 | SEQ4161 | 1.E-02 | 1.3 | miAD | OR2A1-AS1:20 | SEQ4272 | 8.E-03 | 1.5 |
| miAD | Inc-KCNE1B-5:1 | SEQ4162 | 1.E-02 | 1.3 | All AD | OR2A1-AS1:20 | SEQ4272 | 1.E-02 | 1.4 |
| FTD | Inc-KCNE1B-7:1 | SEQ4885 | 2.E-04 | 2.6 | DLB | PAN 3-AS 1:1 | SEQ4842 | 6.E-04 | 1.5 |
| MCI | Inc-KCNE1B-7:1 | SEQ4885 | 6.E-04 | 2.9 | MCI | PAN3-AS 1:1 | SEQ4842 | 9.E-04 | 1.4 |
| DLB | Inc-KCNE1B-7:1 | SEQ4885 | 9.E-04 | 2.9 | DLB | PAN3-AS1:2 | SEQ4843 | 6.E-04 | 1.5 |
| DLB | Inc-KCNE1B-7:2 | SEQ5056 | 7.E-04 | 2.8 | MCI | PAN3-AS1:2 | SEQ4843 | 9.E-04 | 1.4 |
| MCI | Inc-KCNE1B-7:2 | SEQ5056 | 7.E-04 | 2.9 | All AD | PCBP1-AS1:205 | SEQ3681 | 2.E-02 | 0.7 |
| MCI | Inc-KCNE1B-7:5 | SEQ3702 | 3.E-05 | 3.2 | msAD | PCBP1-AS1:205 | SEQ3681 | 5.E-02 | 0.8 |
| miAD | Inc-KCNE1B-7:5 | SEQ3702 | 7.E-05 | 1.7 | All AD | PCBP1-AS1:213 | SEQ5966 | 3.E-02 | 0.7 |
| DLB | Inc-KCNE1B-7:5 | SEQ3702 | 1.E-04 | 2.5 | DLB | PCBP1-AS1:217 | SEQ3458 | 5.E-04 | 1.2 |
| All AD | Inc-KCNE1B-7:5 | SEQ3702 | 1.E-03 | 1.5 | FTD | PCBP1-AS1:302 | SEQ4731 | 3.E-11 | 0.2 |
| msAD | Inc-KCNE1B-7:5 | SEQ3702 | 5.E-02 | 1.3 | MCI | PCBP1-AS1:302 | SEQ4731 | 3.E-08 | 0.3 |
| FTD | Inc-KCNE1B-7:9 | SEQ4884 | 3.E-07 | 11.9 | miAD | PCBP1-AS1:302 | SEQ4731 | 2.E-06 | 0.2 |
| MCI | Inc-KCNE1B-7:9 | SEQ4884 | 9.E-04 | 2.7 | DLB | PCBP1-AS1:302 | SEQ4731 | 1.E-05 | 0.3 |
| MCI | Inc-KCNE4-7:1 | SEQ2466 | 1.E-03 | 0.6 | All AD | PCBP1-AS1:302 | SEQ4731 | 2.E-05 | 0.3 |
| FTD | Inc-KCNES-3:1 | SEQ5723 | 3.E-06 | 0.5 | msAD | PCBP1-AS1:302 | SEQ4731 | 1.E-03 | 0.3 |
| MCI | Inc-KCNE5-3:1 | SEQ5723 | 9.E-05 | 0.6 | All AD | PCBP1-AS1:304 | SEQ3972 | 2.E-02 | 0.7 |
| FTD | Inc-KCNRG-1:1 | SEQ2856 | 6.E-08 | 0.5 | msAD | PCBP1-AS1:304 | SEQ3972 | 2.E-02 | 0.7 |
| MCI | Inc-KCNRG-1:1 | SEQ2856 | 8.E-04 | 0.6 | DLB | PCED1B-AS1:21 | SEQ5279 | 5.E-04 | 1.8 |
| miAD | Inc-KCNRG-1:1 | SEQ2856 | 6.E-03 | 0.7 | MCI | PCED1B-AS1:21 | SEQ5279 | 5. E-04 | 1.9 |
| All AD | Inc-KCNRG-1:1 | SEQ2856 | 7.E-03 | 0.7 | MCI | PCED1B-AS1:24 | SEQ4320 | 4.E-05 | 3.5 |
| msAD | Inc-KCNRG-1:1 | SEQ2856 | 3.E-02 | 0.7 | DLB | PCED1B-AS1:24 | SEQ4320 | 6.E-05 | 3.0 |
| FTD | Inc-KCNRG-2:2 | SEQ4270 | 1.E-08 | 0.5 | miAD | PCED1B-AS1:24 | SEQ4320 | 6.E-03 | 1.6 |
| MCI | Inc-KCNRG-2:2 | SEQ4270 | 1.E-05 | 0.5 | All AD | PCED1B-AS1:24 | SEQ4320 | 2.E-02 | 1.4 |
| miAD | Inc-KCNRG-2:2 | SEQ4270 | 2.E-04 | 0.6 | MCI | PCED1B-AS1:4 | SEQ5455 | 2.E-04 | 1.9 |
| All AD | Inc-KCNRG-2:2 | SEQ4270 | 5.E-04 | 0.7 | DLB | PCED1B-AS1:4 | SEQ5455 | 3.E-04 | 1.9 |
| msAD | Inc-KCNRG-2:2 | SEQ4270 | 8.E-03 | 0.7 | MCI | PCED1B-AS1:7 | SEQ5348 | 4.E-04 | 3.1 |
| FTD | Inc-KCNRG-5:2 | SEQ4075 | 1.E-08 | 0.5 | All AD | PCED1B-AS1:7 | SEQ5348 | 3.E-02 | 1.4 |
| MCI | Inc-KCNRG-5:2 | SEQ4075 | 9.E-05 | 0.6 | MCI | PCF11-AS1:12 | SEQ5841 | 4.E-05 | 0.5 |
| miAD | Inc-KCNRG-5:2 | SEQ4075 | 1.E-03 | 0.6 | FTD | PDCD4-AS1:5 | SEQ5721 | 1.E-04 | 0.6 |
| All AD | Inc-KCNRG-5:2 | SEQ4075 | 2.E-03 | 0.7 | DLB | PDCD4-AS1:6 | SEQ5401 | 1.E-05 | 1.9 |
| msAD | Inc-KCNRG-5:2 | SEQ4075 | 1.E-02 | 0.7 | MCI | PDCD4-AS1:6 | SEQ5401 | 6.E-05 | 1.7 |
| DLB | Inc-KCNT1-1:7 | SEQ5577 | 2.E-04 | 1.7 | FTD | PDCD4-AS1:6 | SEQ5401 | 3.E-04 | 1.5 |
| DLB | Inc-KCNT1-6:1 | SEQ2699 | 1.E-03 | 1.4 | DLB | PIK3CD-AS1:1 | SEQ5793 | 6.E-05 | 1.6 |
| DLB | Inc-KDM3A-1:4 | SEQ0104 | 2.E-03 | 1.4 | DLB | PIK3CD-AS2:10 | SEQ5127 | 6.E-04 | 1.6 |
| DLB | Inc-KDM4C-18:1 | SEQ5474 | 2.E-04 | 0.5 | MCI | PIK3CD-AS2:8 | SEQ5460 | 2.E-04 | 2.0 |
| MCI | Inc-KIAA0319L-1:4 | SEQ4874 | 9.E-04 | 1.8 | DLB | PIK3CD-AS2:8 | SEQ5460 | 3.E-04 | 2.0 |
| All AD | Inc-KIAA0355-5:1 | SEQ4993 | 3.E-02 | 0.8 | DLB | PIK3IP1-AS1:12 | SEQ5029 | 7.E-04 | 1.5 |
| FTD | Inc-KIAA0391-1:1 | SEQ3817 | 3.E-04 | 0.7 | All AD | PIK3IP1-AS1:12 | SEQ5029 | 4.E-02 | 1.2 |
| All AD | Inc-KIAA0391-1:1 | SEQ3817 | 3.E-02 | 0.8 | All AD | PIK3IP1-AS1:3 | SEQ5973 | 2.E-02 | 1.3 |
| msAD | Inc-KIAA0391-1:1 | SEQ3817 | 4.E-02 | 0.8 | MCI | PINK1-AS:2 | SEQ5500 | 2.E-04 | 2.3 |
| FTD | Inc-KIAA0825-1:2 | SEQ4996 | 5.E-08 | 0.5 | All AD | PLBD1-AS1:6 | SEQ3960 | 8.E-03 | 0.7 |
| MCI | Inc-KIAA0825-1:2 | SEQ4996 | 5.E-04 | 0.7 | miAD | PLBD1-AS1:6 | SEQ3960 | 1.E-02 | 0.8 |
| All AD | Inc-KIAA0825-1:2 | SEQ4996 | 2.E-02 | 0.7 | msAD | PLBD1-AS1:6 | SEQ3960 | 2.E-02 | 0.7 |
| MCI | Inc-KIAA1109-1:1 | SEQ5381 | 2.E-04 | 1.6 | miAD | PLBD1-AS1:7 | SEQ4173 | 2.E-04 | 0.8 |
| DLB | Inc-KIAA1109-1:1 | SEQ5381 | 3.E-04 | 1.6 | All AD | PLBD1-AS1:7 | SEQ4173 | 7.E-04 | 0.8 |
| DLB | Inc-KIAA1549L-1:1 | SEQ5427 | 2.E-04 | 1.3 | msAD | PLBD1-AS1:7 | SEQ4173 | 1.E-02 | 0.8 |
| MCI | Inc-KIAA1549L-1:1 | SEQ5427 | 3.E-04 | 1.4 | FTD | POC1B-AS1:8 | SEQ5156 | 5.E-04 | 0.6 |
| All AD | Inc-KIAA1551-13:1 | SEQ3715 | 3.E-02 | 0.8 | FTD | PPP3CB-AS1:4 | SEQ5981 | 2.E-06 | 0.4 |
| msAD | Inc-KIAA1551-13:1 | SEQ3715 | 4.E-02 | 0.8 | DLB | PRKAG2-AS1:1 | SEQ5488 | 1.E-04 | 1.7 |
| All AD | Inc-KIAA1551-2:1 | SEQ4066 | 9.E-03 | 0.7 | MCI | PRKAG2-AS1:1 | SEQ5488 | 2.E-04 | 1.6 |
| msAD | Inc-KIAA1551-2:1 | SEQ4066 | 2.E-02 | 0.7 | FTD | PRKCQ-AS1:11 | SEQ4800 | 1.E-03 | 0.5 |
| FTD | Inc-KIAA1551-4:1 | SEQ4359 | 3.E-06 | 0.5 | MCI | PRKCQ-AS1:3 | SEQ4536 | 3.E-06 | 4.4 |
| All AD | Inc-KIAA1551-4:1 | SEQ4359 | 9.E-04 | 0.6 | FTD | PRKCQ-AS1:3 | SEQ4536 | 1.E-03 | 2.1 |
| msAD | Inc-KIAA1551-4:1 | SEQ4359 | 1.E-03 | 0.7 | DLB | PRKCQ-AS1:3 | SEQ4536 | 2.E-03 | 2.2 |
| miAD | Inc-KIAA1551-4:1 | SEQ4359 | 4.E-03 | 0.6 | MCI | PRKCQ-AS1:36 | SEQ4537 | 3.E-06 | 4.4 |
| msAD | Inc-KIAA1551-6:1 | SEQ3988 | 2.E-02 | 0.8 | FTD | PRKCQ-AS1:36 | SEQ4537 | 1.E-03 | 2.1 |
| All AD | Inc-KIAA1551-6:1 | SEQ3988 | 3.E-02 | 0.8 | DLB | PRKCQ-AS1:36 | SEQ4537 | 2.E-03 | 2.2 |
| FTD | Inc-KIF14-1:2 | SEQ3885 | 1.E-06 | 0.5 | MCI | PRKCQ-AS1:38 | SEQ4661 | 1.E-03 | 2.1 |
| miAD | Inc-KIF14-1:2 | SEQ3885 | 4.E-04 | 0.6 | All AD | PRKCQ-AS1:45 | SEQ5977 | 3.E-02 | 1.4 |
| All AD | Inc-KIF14-1:2 | SEQ3885 | 2.E-03 | 0.6 | MCI | PRKCQ-AS1:53 | SEQ5501 | 2.E-04 | 2.3 |
| msAD | Inc-KIF14-1:2 | SEQ3885 | 3.E-02 | 0.6 | MCI | PRR7-AS1:7 | SEQ5332 | 2.E-04 | 1.7 |
| DLB | Inc-KIRREL1-1:3 | SEQ4531 | 2.E-03 | 1.8 | DLB | PRR7-AS1:7 | SEQ5332 | 4.E-04 | 1.7 |
| msAD | Inc-KLF11-1:4 | SEQ4039 | 2.E-02 | 1.5 | DLB | PRR7-AS1:8 | SEQ4420 | 1.E-03 | 1.4 |
| FTD | Inc-KLF12-4:1 | SEQ0938 | 2.E-05 | 0.5 | MCI | PRR7-AS1:8 | SEQ4420 | 2.E-03 | 1.4 |
| FTD | Inc-KLF12-5:1 | SEQ5149 | 5.E-04 | 0.6 | All AD | PSMA3-AS1:12 | SEQ5979 | 4.E-02 | 0.8 |
| FTD | Inc-KLF5-12:1 | SEQ5008 | 5.E-07 | 0.4 | DLB | PSMA3-AS1:16 | SEQ4675 | 1.E-03 | 1.3 |
| All AD | Inc-KLFS-12:1 | SEQ5008 | 4.E-02 | 0.7 | DLB | PSMA3-AS1:27 | SEQ3498 | 1.E-03 | 1.6 |
| DLB | Inc-KLHDC9-5:1 | SEQ4478 | 2.E-03 | 1.3 | DLB | PSMA3-AS1:28 | SEQ5075 | 2.E-04 | 2.5 |
| DLB | Inc-KLHDC9-5:3 | SEQ4458 | 2.E-03 | 2.2 | FTD | PSMA3-AS1:28 | SEQ5075 | 7.E-04 | 2.2 |
| DLB | Inc-KLHDC9-5:4 | SEQ3431 | 5.E-05 | 1.5 | DLB | PSMA3-AS1:36 | SEQ5190 | 5.E-04 | 1.5 |
| MCI | Inc-KLHDC9-5:4 | SEQ3431 | 2.E-03 | 1.3 | FTD | PSMA3-AS1:40 | SEQ3628 | 6.E-06 | 0.5 |
| DLB | Inc-KLHL10-2:1 | SEQ2459 | 2.E-04 | 1.4 | FTD | PSMA3-AS1:41 | SEQ5915 | 2.E-05 | 0.6 |
| DLB | Inc-KLHL25-1:4 | SEQ4938 | 7.E-04 | 1.4 | msAD | PSMB8-AS1:11 | SEQ3937 | 3.E-02 | 0.8 |
| MCI | Inc-KLHL25-1:4 | SEQ4938 | 8.E-04 | 1.4 | MCI | PSMB8-AS1:14 | SEQ5844 | 1.E-05 | 1.6 |
| DLB | Inc-KLHL28-3:5 | SEQ2642 | 5. E-04 | 1.5 | FTD | PSMB8-AS1:14 | SEQ5844 | 1.E-05 | 1.5 |
| DLB | Inc-KLH L36-4: 1 | SEQ5382 | 3.E-06 | 1.8 | DLB | PSMB8-AS1:14 | SEQ5844 | 4.E-05 | 1.5 |
| FTD | Inc-KLH L36-4: 1 | SEQ5382 | 2.E-04 | 1.5 | MCI | PSMB8-AS1:15 | SEQ5888 | 2.E-05 | 0.2 |
| MCI | Inc-KLH L36-4: 1 | SEQ5382 | 3.E-04 | 1.6 | MCI | PSMB8-AS1:16 | SEQ5453 | 3.E-04 | 1.8 |
| FTD | Inc-KLHL8-2:1 | SEQ4998 | 8.E-04 | 0.7 | DLB | PTOV1-AS1:8 | SEQ5212 | 5.E-04 | 1.9 |
| All AD | Inc-KLHL8-2:1 | SEQ4998 | 2.E-02 | 0.8 | DLB | PTOV1-AS2:1 | SEQ4744 | 1.E-03 | 1.4 |
| DLB | Inc-KLHL9-1:1 | SEQ5014 | 4.E-06 | 1.9 | MCI | PVT1:31 | SEQ4534 | 2.E-03 | 2.0 |
| MCI | Inc-KLHL9-1:1 | SEQ5014 | 2.E-04 | 1.6 | DLB | PXN-AS1:15 | SEQ3573 | 9.E-04 | 1.5 |
| miAD | Inc-KLHL9-1:1 | SEQ5014 | 7.E-04 | 1.4 | MCI | PXN-AS1:22 | SEQ4576 | 1.E-04 | 1.7 |
| All AD | Inc-KLHL9-1:1 | SEQ5014 | 4.E-03 | 1.3 | DLB | PXN-AS1:22 | SEQ4576 | 2.E-03 | 1.5 |
| All AD | Inc-KLLN-1:1 | SEQ5017 | 5.E-02 | 0.8 | MCI | PYCARD-AS1:1 | SEQ5028 | 1.E-04 | 1.6 |
| msAD | Inc-KLRD1-1:1 | SEQ3801 | 4.E-02 | 0.7 | FTD | PYCARD-AS1:1 | SEQ5028 | 1.E-04 | 1.5 |
| All AD | Inc-KLRD1-1:1 | SEQ3801 | 4.E-02 | 0.7 | DLB | PYCARD-AS1:1 | SEQ5028 | 7.E-04 | 1.5 |
| FTD | Inc-KMT2E-6:1 | SEQ3952 | 3.E-06 | 0.5 | DLB | RAB11B-AS1:7 | SEQ5538 | 2.E-04 | 1.8 |
| All AD | Inc-KMT2E-6:1 | SEQ3952 | 1.E-02 | 0.6 | DLB | RAD51-AS1:! | SEQ5132 | 6.E-04 | 1.6 |
| msAD | Inc-KMT2E-6:1 | SEQ3952 | 3.E-02 | 0.7 | DLB | RALY-AS1:13 | SEQ5553 | 2.E-05 | 1.7 |
| All AD | Inc-KPNA2-6:1 | SEQ5021 | 4.E-02 | 1.2 | MCI | RALY-AS1:13 | SEQ5553 | 6.E-05 | 1.6 |
| DLB | Inc-KPNA2-6:13 | SEQ4978 | 9.E-05 | 2.2 | FTD | RALY-AS1:13 | SEQ5553 | 2.E-04 | 1.4 |
| MCI | Inc-KPNA2-6:13 | SEQ4978 | 8.E-04 | 1.8 | All AD | RALY-AS1:13 | SEQ5553 | 4.E-02 | 1.2 |
| FTD | Inc-KPNA2-6:16 | SEQ5883 | 3.E-05 | 0.5 | miAD | RARA-AS1:3 | SEQ4223 | 1.E-02 | 0.8 |
| DLB | Inc-KPNA2-6:25 | SEQ5284 | 5.E-04 | 2.4 | All AD | RARA-AS1:3 | SEQ4223 | 3.E-02 | 0.8 |
| DLB | Inc-KRT80-3:1 | SEQ3433 | 3.E-04 | 1.5 | DLB | RASSF1-AS1:2 | SEQ4746 | 6.E-04 | 1.6 |
| DLB | Inc-KRTAP5-6-3:1 | SEQ4942 | 8.E-04 | 1.5 | FTD | RASSF1-AS1:2 | SEQ4746 | 1.E-03 | 1.3 |
| miAD | Inc-KRTDAP-1:2 | SEQ4276 | 8.E-03 | 0.7 | MCI | RASSF1-AS1:2 | SEQ4746 | 1.E-03 | 1.4 |
| All AD | Inc-KRTDAP-1:2 | SEQ4276 | 1.E-02 | 0.7 | MCI | RBM26-AS1:1 | SEQ4319 | 4.E-06 | 2.0 |
| All AD | Inc-KRTDAP-1:6 | SEQ3868 | 1.E-02 | 0.7 | DLB | RBM26-AS1:1 | SEQ4319 | 1.E-05 | 2.1 |
| msAD | Inc-KRTDAP-1:6 | SEQ3868 | 3.E-02 | 0.7 | FTD | RBM26-AS1:1 | SEQ4319 | 4.E-05 | 1.7 |
| miAD | Inc-KY-4:1 | SEQ0123 | 1.E-02 | 0.6 | All AD | RBM26-AS1:1 | SEQ4319 | 2.E-03 | 1.3 |
| All AD | Inc-KY-4:1 | SEQ0123 | 2.E-02 | 0.7 | msAD | RBM26-AS1:1 | SEQ4319 | 5.E-03 | 1.3 |
| DLB | Inc-LAMA5-1:4 | SEQ3434 | 1.E-04 | 1.5 | miAD | RBM26-AS1:1 | SEQ4319 | 6.E-03 | 1.3 |
| MCI | Inc-LAMA5-1:4 | SEQ3434 | 2.E-03 | 1.3 | DLB | RBM5-AS1:! | SEQ4691 | 1.E-03 | 1.4 |
| DLB | Inc-LAMA5-4:1 | SEQ3435 | 1.E-03 | 1.4 | DLB | RBMS-AS1:2 | SEQ4489 | 2.E-03 | 1.4 |
| All AD | Inc-LASP1-5:1 | SEQ2438 | 2.E-03 | 0.7 | All AD | RC3H1-IT1:1 | SEQ5983 | 2.E-02 | 0.7 |
| msAD | Inc-LASP1-5:1 | SEQ2438 | 3.E-03 | 0.7 | MCI | RNF219-AS1:16 | SEQ4990 | 3.E-04 | 0.6 |
| miAD | Inc-LASP1-5:1 | SEQ2438 | 8.E-03 | 0.7 | FTD | RNF219-AS1:16 | SEQ4990 | 8.E-04 | 0.6 |
| DLB | Inc-LCMT2-1:1 | SEQ4769 | 1.E-03 | 1.5 | MCI | RPARP-AS1:29 | SEQ4873 | 4.E-05 | 1.8 |
| miAD | Inc-LCOR-4:3 | SEQ3289 | 1.E-02 | 0.8 | DLB | RPARP-AS1:29 | SEQ4873 | 9.E-04 | 1.8 |
| All AD | Inc-LCOR-4:3 | SEQ3289 | 1.E-02 | 0.8 | DLB | RPARP-AS1:31 | SEQ5023 | 7.E-04 | 1.5 |
| All AD | Inc-LCOR-4:4 | SEQ5033 | 4.E-02 | 0.8 | All AD | RPS6KB2-AS1:2 | SEQ5986 | 4.E-02 | 1.2 |
| FTD | Inc-LCP2-3:1 | SEQ5766 | 8.E-05 | 0.7 | DLB | RUSC1-AS1:8 | SEQ5658 | 1.E-04 | 1.5 |
| DLB | Inc-LDAH-4:1 | SEQ4557 | 2.E-03 | 1.3 | DLB | SATB1-AS1:2 | SEQ3794 | 2.E-03 | 1.9 |
| MCI | Inc-LDHAL6A-1:2 | SEQ4830 | 9.E-04 | 1.3 | miAD | SATB1-AS1:2 | SEQ3794 | 7.E-03 | 1.5 |
| DLB | Inc-LEAP2-2:1 | SEQ2749 | 2.E-03 | 1.3 | All AD | SATB1-AS1:2 | SEQ3794 | 8.E-03 | 1.4 |
| FTD | Inc-LEKR1-7:1 | SEQ3831 | 3.E-04 | 0.6 | msAD | SATB1-AS1:2 | SEQ3794 | 4.E-02 | 1.4 |
| msAD | Inc-LEKR1-7:1 | SEQ3831 | 3.E-02 | 0.8 | All AD | SATB1-AS1:90 | SEQ5987 | 4.E-02 | 1.5 |
| All AD | Inc-LEKR1-7:1 | SEQ3831 | 4.E-02 | 0.8 | miAD | SBF2-AS1:7 | SEQ4385 | 3.E-03 | 0.7 |
| FTD | Inc-LEO1-2:1 | SEQ6013 | 1.E-10 | 0.3 | FTD | SDCBP2-AS1:4 | SEQ3711 | 3.E-04 | 2.2 |
| All AD | Inc-LEPROTL1-5:1 | SEQ4388 | 5.E-04 | 1.8 | MCI | SDCBP2-AS1:4 | SEQ3711 | 3.E-04 | 2.4 |
| msAD | Inc-LEPROTL1-5:1 | SEQ4388 | 1.E-03 | 1.8 | DLB | SDCBP2-AS1:4 | SEQ3711 | 5.E-04 | 2.3 |
| miAD | Inc-LEPROTL1-5:1 | SEQ4388 | 2.E-03 | 1.7 | msAD | SDCBP2-AS1:4 | SEQ3711 | 5.E-02 | 1.3 |
| DLB | Inc-LEPROTL1-5:2 | SEQ4449 | 2.E-03 | 1.6 | MCI | SGMS1-AS1:10 | SEQ5536 | 2.E-04 | 1.8 |
| DLB | Inc-LGALS2-1:3 | SEQ5164 | 5.E-05 | 1.6 | All AD | SGMS1-AS1:10 | SEQ5536 | 2.E-02 | 1.3 |
| FTD | Inc-LGALS2-1:3 | SEQ5164 | 5.E-04 | 1.4 | MCI | SLC25A25-AS1:1 | SEQ5910 | 2.E-05 | 2.8 |
| DLB | Inc-LGALS2-5:1 | SEQ4413 | 1.E-03 | 1.4 | DLB | SLC25A25-AS1:14 | SEQ4871 | 6.E-04 | 1.6 |
| MCI | Inc-LGALS2-5:1 | SEQ4413 | 2.E-03 | 1.4 | MCI | SLC25A25-AS1:14 | SEQ4871 | 9.E-04 | 1.7 |
| DLB | Inc-LGALS7B-1:5 | SEQ5114 | 6.E-04 | 1.4 | DLB | SLC25A25-AS1:8 | SEQ5861 | 4.E-05 | 1.8 |
| DLB | Inc-LGALSL-2:9 | SEQ4391 | 2.E-03 | 2.E-02 | DLB | SLC2A1-AS1:4 | SEQ3874 | 1.E-03 | 1.3 |
| MCI | Inc-LHFPL3-4:1 | SEQ5301 | 2.E-05 | 1.7 | miAD | SLC2A1-AS1:4 | SEQ3874 | 5.E-03 | 1.2 |
| DLB | Inc-LHFPL3-4:1 | SEQ5301 | 5.E-05 | 1.6 | All AD | SLC2A1-AS1:4 | SEQ3874 | 6.E-03 | 1.2 |
| FTD | Inc-LHFPL3-4:1 | SEQ5301 | 4.E-04 | 1.3 | msAD | SLC2A1-AS1:4 | SEQ3874 | 3.E-02 | 1.1 |
| DLB | Inc-LHPP-6:5 | SEQ5439 | 3.E-04 | 1.5 | msAD | SLC2A1-AS1:5 | SEQ3691 | 5.E-02 | 1.4 |
| DLB | Inc-LHPP-6:6 | SEQ3436 | 2.E-04 | 1.6 | FTD | SLC8A1-AS1:30 | SEQ4131 | 6.E-05 | 0.5 |
| All AD | Inc-LHPP-8:1 | SEQ3637 | 3.E-02 | 0.8 | miAD | SLC8A1-AS1:30 | SEQ4131 | 1.E-02 | 0.6 |
| msAD | Inc-LHPP-8:1 | SEQ3637 | 5.E-02 | 0.8 | All AD | SLC8A1-AS1:30 | SEQ4131 | 3.E-02 | 0.6 |
| All AD | Inc-LIN7A-3:1 | SEQ3437 | 4.E-02 | 0.7 | FTD | SMARCAS-AS1:2 | SEQ5505 | 7.E-06 | 2.6 |
| DLB | Inc-LINC00094-2:1 | SEQ5204 | 8.E-05 | 1.9 | MCI | SMARCAS-AS1:2 | SEQ5505 | 2.E-05 | 2.6 |
| MCI | Inc-LINC00094-2:1 | SEQ5204 | 5.E-04 | 1.7 | DLB | SMARCAS-AS1:2 | SEQ5505 | 9.E-05 | 2.2 |
| DLB | Inc-LINC00672-1:1 | SEQ5376 | 3.E-04 | 1.5 | msAD | SMARCAS-AS1:2 | SEQ5505 | 2.E-04 | 1.7 |
| DLB | Inc-LINC00694-4:1 | SEQ5521 | 2.E-04 | 1.4 | All AD | SMARCAS-AS1:2 | SEQ5505 | 9.E-04 | 1.5 |
| FTD | Inc-LINC02210-CRHR1-2:4 | SEQ5641 | 1.E-04 | 0.5 | DLB | SMCR5:1 | SEQ3037 | 2.E-04 | 2.0 |
| FTD | Inc-LINC02210-CRHR1-3:1 | SEQ5938 | 1.E-05 | 0.4 | DLB | SNAI3-AS1:12 | SEQ5634 | 9.E-05 | 1.9 |
| All AD | Inc-LINS1-3:1 | SEQ5047 | 2.E-02 | 1.2 | MCI | SNAI3-AS1:12 | SEQ5634 | 2.E-04 | 1.9 |
| FTD | Inc-LIPC-2:1 | SEQ4921 | 5.E-05 | 0.4 | FTD | SND1-IT1:4 | SEQ3933 | 1.E-04 | 0.5 |
| MCI | Inc-LIPC-2:1 | SEQ4921 | 8.E-04 | 0.7 | miAD | SND1-IT1:4 | SEQ3933 | 3.E-03 | 0.6 |
| All AD | Inc-LIPC-2:1 | SEQ4921 | 2.E-02 | 0.7 | All AD | SND1-IT1:4 | SEQ3933 | 4.E-03 | 0.6 |
| FTD | Inc-LIPC-3:1 | SEQ4070 | 3.E-05 | 0.4 | msAD | SND1-IT1:4 | SEQ3933 | 3.E-02 | 0.7 |
| MCI | Inc-LIPC-3:1 | SEQ4070 | 6.E-04 | 0.6 | DLB | SND1-IT1:5 | SEQ5050 | 7.E-04 | 1.9 |
| miAD | Inc-LIPC-3:1 | SEQ4070 | 1.E-02 | 0.6 | MCI | SNHG1:1 | SEQ5463 | 3.E-04 | 2.7 |
| All AD | Inc-LIPC-3:1 | SEQ4070 | 3.E-02 | 0.7 | FTD | SNHG1:54 | SEQ5502 | 6.E-10 | 9.7 |
| FTD | Inc-LIPC-4:1 | SEQ5054 | 3.E-06 | 0.4 | MCI | SNHG1:54 | SEQ5502 | 2.E-04 | 2.6 |
| MCI | Inc-LIPC-4:1 | SEQ5054 | 9.E-05 | 0.5 | DLB | SNHG12:16 | SEQ4759 | 1.E-03 | 1.5 |
| All AD | Inc-LIPC-4:1 | SEQ5054 | 2.E-02 | 0.6 | MCI | SNHG12:17 | SEQ5303 | 4.E-05 | 1.8 |
| FTD | Inc-LIPC-5:1 | SEQ4920 | 3.E-05 | 0.4 | DLB | SNHG12:17 | SEQ5303 | 4.E-04 | 1.8 |
| MCI | Inc-LIPC-5:1 | SEQ4920 | 8.E-04 | 0.5 | FTD | SNHG12:17 | SEQ5303 | 4.E-04 | 1.7 |
| All AD | Inc-LIPC-5:1 | SEQ4920 | 4.E-02 | 0.8 | All AD | SNHG12:17 | SEQ5303 | 4.E-02 | 1.2 |
| FTD | Inc-LIPC-6:1 | SEQ3678 | 1.E-07 | 0.3 | All AD | SNHG16:31 | SEQ5992 | 4.E-02 | 0.8 |
| MCI | Inc-LIPC-6:1 | SEQ3678 | 1.E-05 | 0.3 | DLB | SNHG16:4 | SEQ4727 | 1.E-03 | 2.3 |
| DLB | Inc-LIPC-6:1 | SEQ3678 | 6.E-04 | 0.3 | All AD | SNHG16:42 | SEQ5994 | 4.E-02 | 0.8 |
| miAD | Inc-LIPC-6:1 | SEQ3678 | 1.E-02 | 0.6 | DLB | SNHG17:23 | SEQ5489 | 2.E-04 | 1.6 |
| All AD | Inc-LIPC-6:1 | SEQ3678 | 1.E-02 | 0.6 | DLB | SNHG21:11 | SEQ4768 | 1.E-03 | 1.5 |
| msAD | Inc-LIPC-6:1 | SEQ3678 | 5.E-02 | 0.6 | MCI | SNHG22:3 | SEQ5497 | 1.E-04 | 1.8 |
| FTD | Inc-LIX1-1:1 | SEQ5413 | 3.E-04 | 0.6 | DLB | SNHG22:3 | SEQ5497 | 2.E-04 | 1.9 |
| MCI | Inc-LKAAEAR1-2:1 | SEQ5026 | 5.E-04 | 1.5 | DLB | SNHG25:1 | SEQ4827 | 9.E-04 | 1.2 |
| DLB | Inc-LKAAEAR1-2:1 | SEQ5026 | 7.E-04 | 1.5 | MCI | SNHG5:12 | SEQ5542 | 1.E-04 | 0.3 |
| miAD | Inc-LMLN-4:1 | SEQ3734 | 7.E-03 | 0.5 | FTD | SNHG5:12 | SEQ5542 | 2.E-04 | 0.3 |
| All AD | Inc-LMLN-4:1 | SEQ3734 | 9.E-03 | 0.6 | All AD | SNHG6:12 | SEQ5995 | 5.E-02 | 0.8 |
| msAD | Inc-LMLN-4:1 | SEQ3734 | 4.E-02 | 0.7 | DLB | SNHG7:12 | SEQ4121 | 1.E-05 | 1.8 |
| FTD | Inc-LONP2-8:1 | SEQ4310 | 8.E-06 | 0.5 | MCI | SNHG7:12 | SEQ4121 | 2.E-04 | 1.6 |
| miAD | Inc-LONP2-8:1 | SEQ4310 | 7.E-03 | 0.6 | FTD | SNHG7:12 | SEQ4121 | 7.E-04 | 1.4 |
| All AD | Inc-LONP2-8:1 | SEQ4310 | 1.E-02 | 0.7 | All AD | SNHG7:12 | SEQ4121 | 8.E-03 | 1.2 |
| DLB | Inc-LPAR6-4:1 | SEQ5491 | 2.E-04 | 1.6 | msAD | SNHG7:12 | SEQ4121 | 1.E-02 | 1.3 |
| FTD | Inc-LPCAT3-1:1 | SEQ2652 | 5.E-06 | 1.8 | FTD | SNRK-AS1:1 | SEQ5965 | 1.E-08 | 0.5 |
| MCI | Inc-LPCAT3-1:1 | SEQ2652 | 5.E-04 | 1.9 | MCI | SNRK-AS1:1 | SEQ5965 | 4.E-06 | 0.5 |
| DLB | Inc-LPCAT3-1:1 | SEQ2652 | 2.E-03 | 1.8 | FTD | SNRK-AS1:4 | SEQ4006 | 4.E-07 | 2.3 |
| All AD | Inc-LPCAT3-1:1 | SEQ2652 | 2.E-02 | 1.2 | MCI | SNRK-AS1:4 | SEQ4006 | 1.E-06 | 2.7 |
| FTD | Inc-LPIN1-5:1 | SEQ5066 | 1.E-05 | 1.6 | DLB | SNRK-AS1:4 | SEQ4006 | 2.E-05 | 2.3 |
| MCI | Inc-LPIN1-5:1 | SEQ5066 | 2.E-04 | 1.5 | All AD | SNRK-AS1:4 | SEQ4006 | 7.E-03 | 1.3 |
| All AD | Inc-LPIN1-5:1 | SEQ5066 | 4.E-02 | 1.2 | miAD | SNRK-AS1:4 | SEQ4006 | 9.E-03 | 1.3 |
| MCI | Inc-LPIN1-7:2 | SEQ5581 | 4.E-05 | 2.2 | msAD | SNRK-AS1:4 | SEQ4006 | 2.E-02 | 1.3 |
| DLB | Inc-LPIN1-7:2 | SEQ5581 | 2.E-04 | 2.0 | FTD | SOS1-IT1:2 | SEQ4034 | 2.E-07 | 0.4 |
| DLB | Inc-LPIN3-1:1 | SEQ3438 | 3.E-04 | 1.5 | MCI | SOS1-IT1:2 | SEQ4034 | 8.E-05 | 0.5 |
| FTD | Inc-LPP-2:1 | SEQ5070 | 7.E-06 | 0.7 | All AD | SOS1-IT1:2 | SEQ4034 | 1.E-02 | 0.7 |
| All AD | Inc-LPP-2:1 | SEQ5070 | 4.E-02 | 0.8 | msAD | SOS1-IT1:2 | SEQ4034 | 2.E-02 | 0.7 |
| FTD | Inc-LPP-2:2 | SEQ5072 | 3.E-05 | 0.7 | DLB | SPAG5-AS1:8 | SEQ5843 | 4.E-05 | 1.4 |
| All AD | Inc-LPP-2:2 | SEQ5072 | 5.E-02 | 0.8 | DLB | SPAG5-AS1:9 | SEQ4632 | 1.E-03 | 1.5 |
| miAD | Inc-LRFN5-10:1 | SEQ4213 | 1.E-02 | 1.2 | DLB | SSBP3-AS1:2 | SEQ5270 | 5.E-04 | 1.6 |
| All AD | Inc-LRFN5-10:1 | SEQ4213 | 1.E-02 | 1.2 | MCI | STARD7-AS1:5 | SEQ4797 | 1.E-06 | 2.2 |
| DLB | Inc-LRP1-1:1 | SEQ4508 | 2.E-03 | 1.5 | DLB | STARD7-AS1:5 | SEQ4797 | 6.E-06 | 2.2 |
| DLB | Inc-LRPSL-14:1 | SEQ5248 | 5.E-04 | 1.5 | FTD | STARD7-AS1:5 | SEQ4797 | 3.E-05 | 2.0 |
| miAD | Inc-LRRC10-1:1 | SEQ3290 | 2.E-03 | 0.7 | All AD | STARD7-AS1:5 | SEQ4797 | 1.E-04 | 1.5 |
| All AD | Inc-LRRC10-1:1 | SEQ3290 | 5.E-03 | 0.7 | miAD | STARD7-AS1:5 | SEQ4797 | 1.E-04 | 1.5 |
| msAD | Inc-LRRC10-1:1 | SEQ3290 | 4.E-02 | 0.7 | msAD | STARD7-AS1:5 | SEQ4797 | 1.E-03 | 1.4 |
| FTD | Inc-LRRC10-1:2 | SEQ3441 | 3.E-04 | 0.5 | MCI | STXBP5-AS1:16 | SEQ5579 | 2.E-04 | 1.9 |
| miAD | Inc-LRRC10-1:2 | SEQ3441 | 1.E-02 | 0.7 | FTD | SVIL-AS1:37 | SEQ5868 | 4.E-05 | 0.6 |
| All AD | Inc-LRRC10-1:2 | SEQ3441 | 1.E-02 | 0.7 | DLB | TBC1D22A-AS1:1 | SEQ4592 | 2.E-03 | 1.7 |
| msAD | Inc-LRRC10-1:2 | SEQ3441 | 4.E-02 | 0.7 | DLB | TBX2-AS1:17 | SEQ4493 | 2.E-03 | 1.4 |
| FTD | Inc-LRRC31-2:1 | SEQ5058 | 7.E-04 | 0.5 | MCI | TCONS_0000022 4 | SEQ1048 | 2.E-05 | 0.5 |
| FTD | Inc-LRRC37A3-5:15 | SEQ5603 | 2.E-04 | 0.6 | DLB | TCONS_0000022 4 | SEQ1048 | 9.E-04 | 0.6 |
| DLB | Inc-LRRC3C-2:1 | SEQ5388 | 1.E-04 | 1.8 | FTD | TCONS_0000041 6 | SEQ1091 | 6.E-04 | 0.6 |
| MCI | Inc-LRRC3C-2:1 | SEQ5388 | 3.E-04 | 1.7 | FTD | TCONS_0000055 6 | SEQ1026 | 1.E-04 | 0.6 |
| FTD | Inc-LRRC57-1:1 | SEQ2537 | 5.E-07 | 0.5 | All AD | TCONS_0000055 6 | SEQ1026 | 3.E-02 | 0.8 |
| MCI | Inc-LRRC57-1:1 | SEQ2537 | 2.E-04 | 0.6 | All AD | TCONS_0000065 5 | SEQ1050 | 4.E-02 | 0.7 |
| All AD | Inc-LRRC57-1:1 | SEQ2537 | 5.E-02 | 0.7 | miAD | TCONS_0000077 3 | SEQ1086 | 1.E-02 | 0.6 |
| FTD | Inc-LRRC72-10:2 | SEQ4806 | 1.E-03 | 0.6 | All AD | TCONS_0000077 3 | SEQ1086 | 3.E-02 | 0.7 |
| All AD | Inc-LRRC72-10:2 | SEQ4806 | 4.E-02 | 0.7 | FTD | TCONS_0000503 7 | SEQ1136 | 1.E-04 | 0.6 |
| miAD | Inc-LRRC72-10:4 | SEQ4336 | 5.E-03 | 0.5 | msAD | TCONS_0000506 0 | SEQ1140 | 5.E-02 | 1.2 |
| FTD | Inc-LRRC7-5:3 | SEQ4376 | 4.E-07 | 0.4 | FTD | TCONS_0000526 4 | SEQ1123 | 3.E-08 | 0.5 |
| MCI | Inc-LRRC7-5:3 | SEQ4376 | 1.E-03 | 0.6 | FTD | TCONS_0000885 6 | SEQ1166 | 3.E-04 | 0.5 |
| miAD | Inc-LRRC7-5:3 | SEQ4376 | 3.E-03 | 0.6 | miAD | TCONS_00008879 | SEQ1170 | 1.E-02 | 0.5 |
| All AD | Inc-LRRC7-5:3 | SEQ4376 | 8.E-03 | 0.6 | All AD | TCONS-00008879 | SEQ1170 | 2.E-02 | 0.5 |
| DLB | Inc-LRRC75A-2:1 | SEQ5568 | 2.E-04 | 1.6 | All AD | TCONS_00008889 | SEQ1172 | 2.E-02 | 0.6 |
| MCI | Inc-LRRK1-3:4 | SEQ0242 | 9.E-05 | 2.0 | msAD | TCONS_0000888 9 | SEQ1172 | 3.E-02 | 0.7 |
| DLB | Inc-LRRK1-3:4 | SEQ0242 | 8.E-04 | 1.7 | DLB | TCONS_0001197 2 | SEQ1217 | 5.E-04 | 1.3 |
| msAD | Inc-LRRK1-3:4 | SEQ0242 | 2.E-02 | 1.3 | DLB | TCONS_0001199 4 | SEQ1222 | 6.E-06 | 1.7 |
| All AD | Inc-LRRK1-3:4 | SEQ0242 | 4.E-02 | 1.2 | FTD | TCONS_0001206 2 | SEQ1202 | 4.E-05 | 0.6 |
| DLB | Inc-LSM11-1:1 | SEQ5011 | 7.E-04 | 1.3 | MCI | TCONS_0001206 2 | SEQ1202 | 2.E-03 | 0.6 |
| FTD | Inc-LSM14A-2:5 | SEQ5293 | 4.E-04 | 0.6 | FTD | TCONS_0001206 3 | SEQ1203 | 2.E-07 | 0.3 |
| DLB | Inc-LTBP2-2:1 | SEQ5625 | 2.E-04 | 1.7 | MCI | TCONS_0001206 3 | SEQ1203 | 2.E-04 | 0.4 |
| DLB | Inc-LTBP3-2:14 | SEQ4404 | 2.E-03 | 1.3 | All AD | TCONS_0001206 3 | SEQ1203 | 5.E-02 | 0.6 |
| MCI | Inc-LTBP3-2:5 | SEQ4364 | 6.E-06 | 0.2 | FTD | TCONS_0001414 1 | SEQ1224 | 5.E-08 | 0.4 |
| FTD | Inc-LTBP3-2:5 | SEQ4364 | 6.E-06 | 0.2 | MCI | TCONS_0001414 1 | SEQ1224 | 2.E-04 | 0.5 |
| miAD | Inc-LTBP3-2:5 | SEQ4364 | 4.E-03 | 0.3 | miAD | TCONS_0001414 1 | SEQ1224 | 3.E-03 | 0.5 |
| All AD | Inc-LTBP3-2:5 | SEQ4364 | 2.E-02 | 0.4 | FTD | TCONS_0001416 5 | SEQ1227 | 3.E-04 | 0.7 |
| MCI | Inc-LTC4S-1:1 | SEQ2941 | 2.E-04 | 1.6 | MCI | TCONS_0001421 1 | SEQ1239 | 1.E-03 | 1.8 |
| FTD | Inc-LUC7L3-6:1 | SEQ4810 | 1.E-03 | 0.7 | DLB | TCONS_0001421 1 | SEQ1239 | 2.E-03 | 1.8 |
| FTD | Inc-LXN-1:6 | SEQ5400 | 3.E-04 | 0.7 | All AD | TCONS_0001445 3 | SEQ1256 | 4.E-02 | 0.7 |
| All AD | Inc-LY86-4:8 | SEQ5094 | 4.E-02 | 0.7 | FTD | TCONS_0001737 2 | SEQ1273 | 6.E-09 | 4.4 |
| All AD | Inc-LYN-2:1 | SEQ5095 | 4.E-02 | 0.8 | MCI | TCONS_0001737 2 | SEQ1273 | 4.E-05 | 4.2 |
| DLB | Inc-LYPD6-2:1 | SEQ4429 | 8.E-05 | 1.8 | FTD | TCONS_0001737 5 | SEQ1276 | 2.E-04 | 3.E-08 |
| MCI | Inc-LYPD6-2:1 | SEQ4429 | 2.E-03 | 1.5 | DLB | TCONS_0001737 5 | SEQ1276 | 2.E-04 | 2.E-08 |
| FTD | Inc-LYPD8-4:1 | SEQ4365 | 1.E-05 | 0.4 | MCI | TCONS_0001737 5 | SEQ1276 | 7.E-04 | 2.E-08 |
| miAD | Inc-LYPD8-4:1 | SEQ4365 | 4.E-03 | 0.6 | DLB | TCONS_0001737 6 | SEQ1277 | 2.E-04 | 0.9 |
| All AD | Inc-LYPD8-4:1 | SEQ4365 | 9.E-03 | 0.6 | MCI | TCONS_0001737 6 | SEQ1277 | 5.E-04 | 0.9 |
| MCI | Inc-LYZL2-5:1 | SEQ4185 | 2.E-03 | 0.7 | msAD | TCONS_0001737 6 | SEQ1277 | 4.E-02 | 0.9 |
| miAD | Inc-LYZL2-5:1 | SEQ4185 | 1.E-02 | 0.7 | All AD | TCONS_0001737 6 | SEQ1277 | 5.E-02 | 0.9 |
| All AD | Inc-LYZL2-5:1 | SEQ4185 | 2.E-02 | 0.6 | FTD | TCONS_0001977 2 | SEQ1301 | 4.E-04 | 0.6 |
| DLB | Inc-M1AP-1:1 | SEQ4974 | 8.E-04 | 1.7 | DLB | TCONS_0001984 0 | SEQ1292 | 2.E-03 | 1.3 |
| DLB | Inc-M1AP-6:2 | SEQ5390 | 8.E-05 | 1.8 | FTD | TCONS_00019918 | SEQ1308 | 3.E-05 | 0.6 |
| MCI | Inc-M1AP-6:2 | SEQ5390 | 3.E-04 | 1.8 | DLB | TCONS_0002148 1 | SEQ1342 | 4.E-04 | 1.7 |
| DLB | Inc-M6PR-4:1 | SEQ3969 | 2.E-04 | 1.6 | MCI | TCONS_0002148 1 | SEQ1342 | 5.E-04 | 1.6 |
| MCI | Inc-M6PR-4:1 | SEQ3969 | 2.E-04 | 1.4 | MCI | TCONS_0002311 5 | SEQ1352 | 3.E-04 | 0.4 |
| All AD | Inc-M6PR-4:1 | SEQ3969 | 1.E-02 | 1.2 | FTD | TCONS_0002319 0 | SEQ1369 | 5.E-04 | 0.6 |
| msAD | Inc-M6PR-4:1 | SEQ3969 | 2.E-02 | 1.2 | miAD | TCONS_0002319 0 | SEQ1369 | 3.E-03 | 0.6 |
| MCI | Inc-MAD2L2-4:1 | SEQ5633 | 2.E-04 | 1.9 | All AD | TCONS_0002319 0 | SEQ1369 | 9.E-03 | 0.7 |
| MCI | Inc-MAFF-10:1 | SEQ4924 | 8.E-04 | 1.3 | FTD | TCONS_0002337 2 | SEQ1374 | 9.E-04 | 0.6 |
| DLB | Inc-MAFF-2:1 | SEQ3013 | 9.E-04 | 1.6 | DLB | TCONS_0002655 8 | SEQ1414 | 9.E-04 | 1.4 |
| MCI | Inc-MAFF-2:1 | SEQ3013 | 2.E-03 | 1.7 | DLB | TCONS_0003364 9 | SEQ1546 | 1.E-05 | 2.3 |
| MCI | Inc-MAFK-4:3 | SEQ3644 | 2.E-06 | 1.7 | FTD | TCONS_0003364 9 | SEQ1546 | 4.E-04 | 1.6 |
| DLB | Inc-MAFK-4:3 | SEQ3644 | 6.E-06 | 1.7 | DLB | TCONS_0003502 2 | SEQ1567 | 2.E-03 | 16.3 |
| FTD | Inc-MAFK-4:3 | SEQ3644 | 3.E-04 | 1.3 | DLB | TCONS_0003502 3 | SEQ1568 | 2.E-03 | 16.3 |
| msAD | Inc-MAFK-4:3 | SEQ3644 | 5.E-02 | 1.1 | MCI | TCONS_0003502 4 | SEQ1571 | 4.E-07 | 2.5 |
| All AD | Inc-MAGEB1-2:1 | SEQ3902 | 1.E-02 | 0.7 | DLB | TCONS_0003502 4 | SEQ1571 | 3.E-06 | 2.4 |
| msAD | Inc-MAGEB1-2:1 | SEQ3902 | 3.E-02 | 0.7 | FTD | TCONS_0003502 4 | SEQ1571 | 5.E-05 | 1.9 |
| FTD | Inc-MAGEE2-2:1 | SEQ3291 | 4.E-07 | 0.4 | All AD | TCONS_0003502 4 | SEQ1571 | 4.E-02 | 1.2 |
| MCI | Inc-MAGEE2-2:1 | SEQ3291 | 2.E-03 | 0.5 | All AD | TCONS_0003502 5 | SEQ1572 | 3.E-02 | 1.2 |
| FTD | Inc-MALRD1-11:1 | SEQ3815 | 4.E-06 | 0.6 | msAD | TCONS_0003502 7 | SEQ1575 | 2.E-02 | 1.4 |
| All AD | Inc-MALRD1-11:1 | SEQ3815 | 1.E-02 | 0.7 | FTD | TCONS_0003503 6 | SEQ1586 | 7.E-04 | 0.6 |
| miAD | Inc-MALRD1-11:1 | SEQ3815 | 1.E-02 | 0.7 | MCI | TCONS_0003509 1 | SEQ1565 | 2.E-06 | 1.8 |
| msAD | Inc-MALRD1-11:1 | SEQ3815 | 4.E-02 | 0.8 | DLB | TCONS_0003509 1 | SEQ1565 | 6.E-05 | 1.7 |
| msAD | Inc-MAN2B1-1:1 | SEQ3906 | 3.E-02 | 1.3 | FTD | TCONS_0003509 1 | SEQ1565 | 2.E-04 | 1.5 |
| All AD | Inc-MAN2B1-1:1 | SEQ3906 | 4.E-02 | 1.3 | DLB | TCONS_0003509 2 | SEQ1569 | 7.E-06 | 2.6 |
| All AD | Inc-MAN2C1-2:3 | SEQ5106 | 3.E-02 | 0.8 | MCI | TCONS_0003509 2 | SEQ1569 | 9.E-06 | 2.6 |
| FTD | Inc-MANBA-2:9 | SEQ3443 | 3.E-10 | 0.4 | FTD | TCONS_0003509 2 | SEQ1569 | 1.E-05 | 2.3 |
| MCI | Inc-MANBA-2:9 | SEQ3443 | 8.E-05 | 0.6 | All AD | TCONS_0003509 2 | SEQ1569 | 1.E-02 | 1.4 |
| miAD | Inc-MANBA-2:9 | SEQ3443 | 6.E-03 | 0.6 | miAD | TCONS_0003509 2 | SEQ1569 | 1.E-02 | 1.5 |
| All AD | Inc-MANBA-2:9 | SEQ3443 | 7.E-03 | 0.6 | msAD | TCONS_0003509 2 | SEQ1569 | 3.E-02 | 1.4 |
| msAD | Inc-MANBA-2:9 | SEQ3443 | 4.E-02 | 0.7 | MCI | TCONS_0003509 3 | SEQ1570 | 6.E-10 | 1.7 |
| FTD | Inc-MANSC4-2:2 | SEQ5685 | 1.E-04 | 0.6 | FTD | TCONS_0003509 3 | SEQ1570 | 6.E-06 | 1.4 |
| MCI | Inc-MAP1LC38-1:10 | SEQ5574 | 2.E-04 | 1.6 | DLB | TCONS_0003509 3 | SEQ1570 | 3.E-05 | 1.5 |
| DLB | Inc-MAP1LC3B-1:9 | SEQ4514 | 2.E-03 | 1.5 | miAD | TCONS_0003509 3 | SEQ1570 | 1.E-02 | 1.1 |
| All AD | Inc-MAP1LC3B2-14:13 | SEQ5112 | 4.E-02 | 0.7 | All AD | TCONS_0003509 3 | SEQ1570 | 4.E-02 | 1.1 |
| All AD | Inc-MAP1LC3B2-14:16 | SEQ4227 | 3.E-03 | 0.6 | MCI | TCONS_0003509 4 | SEQ1573 | 2.E-07 | 2.6 |
| miAD | Inc-MAP1LC3B2-14:16 | SEQ4227 | 6.E-03 | 0.5 | FTD | TCONS_0003509 4 | SEQ1573 | 5.E-07 | 2.4 |
| msAD | Inc-MAP1LC3B2-14:16 | SEQ4227 | 1.E-02 | 0.6 | DLB | TCONS_0003509 4 | SEQ1573 | 9.E-06 | 2.3 |
| DLB | Inc-MAP1LC3B2-15:1 | SEQ5337 | 4.E-04 | 1.8 | All AD | TCONS_0003509 4 | SEQ1573 | 1.E-02 | 1.3 |
| DLB | Inc-MAP2K2-2:3 | SEQ5736 | 9.E-05 | 1.7 | msAD | TCONS_0003509 4 | SEQ1573 | 4.E-02 | 1.3 |
| MCI | Inc-MAP3K3-2:1 | SEQ3807 | 6.E-07 | 2.5 | FTD | TCONS_0003703 1 | SEQ1676 | 3.E-04 | 0.5 |
| DLB | Inc-MAP3K3-2:1 | SEQ3807 | 7.E-07 | 2.4 | DLB | TCONS_0003711 0 | SEQ1641 | 7.E-06 | 1.6 |
| FTD | Inc-MAP3K3-2:1 | SEQ3807 | 2.E-06 | 2.3 | DLB | TCONS_0003719 8 | SEQ1659 | 6.E-05 | 2.6 |
| All AD | Inc-MAP3K3-2:1 | SEQ3807 | 1.E-02 | 1.3 | msAD | TCONS_0003719 8 | SEQ1659 | 4.E-02 | 1.4 |
| msAD | Inc-MAP3K3-2:1 | SEQ3807 | 4.E-02 | 1.3 | MCI | TCONS_0003721 6 | SEQ1662 | 6.E-05 | 0.4 |
| DLB | Inc-MAP3K3-2:5 | SEQ5175 | 5.E-04 | 1.4 | All AD | TCONS_0004080 3 | SEQ1719 | 4.E-02 | 0.7 |
| DLB | Inc-MAP3K6-1:1 | SEQ4742 | 3.E-04 | 1.5 | DLB | TCONS_0004099 4 | SEQ1713 | 2.E-05 | 1.8 |
| MCI | Inc-MAP3K6-1:1 | SEQ4742 | 1.E-03 | 1.4 | All AD | TCONS_0004099 4 | SEQ1713 | 4.E-02 | 1.1 |
| All AD | Inc-MAP3K7-3:3 | SEQ4255 | 8.E-03 | 1.6 | msAD | TCONS_0004099 4 | SEQ1713 | 5.E-02 | 1.1 |
| msAD | Inc-MAP3K7-3:3 | SEQ4255 | 9.E-03 | 1.7 | DLB | TCONS_0004493 2 | SEQ1735 | 1.E-06 | 2.0 |
| FTD | Inc-MAP3K8-20:1 | SEQ5064 | 7.E-04 | 0.7 | FTD | TCONS_0004504 1 | SEQ1769 | 5.E-05 | 0.5 |
| FTD | Inc-MAP3K9-10:2 | SEQ5122 | 7.E-06 | 0.6 | MCI | TCONS_0004504 1 | SEQ1769 | 9.E-04 | 0.6 |
| All AD | Inc-MAP3K9-10:2 | SEQ5122 | 5.E-02 | 0.8 | FTD | TCONS_0004512 5 | SEQ1790 | 2.E-07 | 0.6 |
| FTD | Inc-MAP3K9-13:1 | SEQ4811 | 1.E-03 | 0.7 | MCI | TCONS_0004512 5 | SEQ1790 | 1.E-04 | 0.6 |
| All AD | Inc-MAP3K9-13:1 | SEQ4811 | 3.E-02 | 0.8 | All AD | TCONS_0004512 5 | SEQ1790 | 3.E-02 | 0.7 |
| All AD | Inc-MAP6-4:10 | SEQ4008 | 8.E-03 | 0.7 | msAD | TCONS_0004512 5 | SEQ1790 | 4.E-02 | 0.7 |
| miAD | Inc-MAP6-4:10 | SEQ4008 | 1.E-02 | 0.7 | All AD | TCONS_00050017 | SEQ1804 | 3.E-02 | 1.2 |
| msAD | Inc-MAP6-4:10 | SEQ4008 | 2.E-02 | 0.7 | FTD | TCONS_0005022 4 | SEQ1854 | 8.E-05 | 0.6 |
| miAD | Inc-MAPK13-1:1 | SEQ4156 | 1.E-02 | 0.7 | miAD | TCONS_0005022 4 | SEQ1854 | 9.E-03 | 0.7 |
| All AD | Inc-MAPK13-1:1 | SEQ4156 | 2.E-02 | 0.8 | All AD | TCONS_0005022 4 | SEQ1854 | 1.E-02 | 0.7 |
| DLB | Inc-MAPK6-8:3 | SEQ4947 | 8.E-04 | 1.5 | MCI | TCONS_0005568 1 | SEQ1887 | 3.E-06 | 0.5 |
| All AD | Inc-MARC2-2:1 | SEQ3917 | 8.E-03 | 0.9 | FTD | TCONS_00059297 | SEQ1957 | 1.E-03 | 0.6 |
| miAD | Inc-MARC2-2:1 | SEQ3917 | 9.E-03 | 0.9 | MCI | TCONS_00059378 | SEQ1928 | 4.E-05 | 1.5 |
| msAD | Inc-MARC2-2:1 | SEQ3917 | 3.E-02 | 0.9 | DLB | TCONS_00059378 | SEQ1928 | 2.E-03 | 1.5 |
| DLB | Inc-MARCH10-8:2 | SEQ5669 | 1.E-04 | 1.7 | FTD | TCONS_0005940 5 | SEQ1944 | 2.E-06 | 0.5 |
| FTD | Inc-MARCH7-1:6 | SEQ4074 | 5.E-05 | 0.7 | FTD | TCONS_0005942 7 | SEQ1949 | 5.E-05 | 0.5 |
| miAD | Inc-MARCH7-1:6 | SEQ4074 | 1.E-02 | 0.7 | DLB | TCONS_0005949 2 | SEQ1962 | 3.E-04 | 1.6 |
| All AD | Inc-MARCH7-1:6 | SEQ4074 | 2.E-02 | 0.8 | MCI | TCONS_0005949 2 | SEQ1962 | 2.E-03 | 1.5 |
| FTD | Inc-MARCH7-3:1 | SEQ3444 | 2.E-07 | 0.5 | MCI | TCONS_0006231 3 | SEQ1998 | 2.E-04 | 0.5 |
| MCI | Inc-MARCH7-3:1 | SEQ3444 | 2.E-04 | 0.6 | miAD | TCONS_0006232 | SEQ2005 | 7.E-03 | 0.6 |
| All AD | Inc-MARCH7-3:1 | SEQ3444 | 3.E-02 | 0.7 | All AD | TCONS_0006232 7 | SEQ2005 | 2.E-02 | 0.7 |
| FTD | Inc-MARCH7-4:1 | SEQ3895 | 6.E-05 | 0.6 | All AD | TCONS_0006234 5 | SEQ2020 | 2.E-02 | 0.7 |
| miAD | Inc-MARCH7-4:1 | SEQ3895 | 7.E-03 | 0.7 | MCI | TCONS_00062359 | SEQ2028 | 3.E-05 | 1.9 |
| All AD | Inc-MARCH7-4:1 | SEQ3895 | 7.E-03 | 0.7 | miAD | TCONS_00062359 | SEQ2028 | 8.E-03 | 1.4 |
| msAD | Inc-MARCH7-4:1 | SEQ3895 | 3.E-02 | 0.7 | All AD | TCONS_00062359 | SEQ2028 | 8.E-03 | 1.3 |
| MCI | Inc-MASTL-6:1 | SEQ2430 | 2.E-04 | 0.6 | msAD | TCONS_00062359 | SEQ2028 | 3.E-02 | 1.3 |
| FTD | Inc-MASTL-7:1 | SEQ4246 | 4.E-06 | 0.6 | msAD | TCONS_0006242 6 | SEQ2041 | 2.E-03 | 0.5 |
| miAD | Inc-MASTL-7:1 | SEQ4246 | 9.E-03 | 0.7 | All AD | TCONS_0006242 6 | SEQ2041 | 4.E-03 | 0.6 |
| All AD | Inc-MASTL-7:1 | SEQ4246 | 2.E-02 | 0.7 | FTD | TCONS_00062427 | SEQ2042 | 3.E-07 | 0.2 |
| FTD | Inc-MAVS-3:1 | SEQ2534 | 3.E-08 | 0.5 | DLB | TCONS_0006242 7 | SEQ2042 | 1.E-05 | 0.2 |
| MCI | Inc-MAVS-3:1 | SEQ2534 | 7.E-06 | 0.5 | FTD | TCONS_0006245 4 | SEQ2047 | 1.E-04 | 0.6 |
| All AD | Inc-MAVS-3:1 | SEQ2534 | 4.E-03 | 0.7 | All AD | TCONS_0006245 4 | SEQ2047 | 5.E-02 | 0.7 |
| msAD | Inc-MAVS-3:1 | SEQ2534 | 9.E-03 | 0.7 | DLB | TCONS_00062559 | SEQ1995 | 1.E-03 | 1.4 |
| miAD | Inc-MAVS-3:1 | SEQ2534 | 1.E-02 | 0.7 | DLB | TCONS_0006270 0 | SEQ2045 | 4.E-04 | 1.3 |
| DLB | Inc-MB-9:1 | SEQ5524 | 2.E-04 | 1.5 | MCI | TCONS_0006631 2 | SEQ2053 | 2.E-03 | 0.8 |
| msAD | Inc-MBD3L2B-3:1 | SEQ4042 | 2.E-02 | 2.1 | DLB | TCONS_0006632 7 | SEQ2060 | 1.E-03 | 1.4 |
| All AD | Inc-MBD3L2B-3:1 | SEQ4042 | 4.E-02 | 1.8 | FTD | TCONS_0006635 7 | SEQ2067 | 1.E-05 | 0.5 |
| FTD | Inc-MBNL1-3:1 | SEQ5764 | 8.E-05 | 0.6 | FTD | TCONS_0006654 4 | SEQ2098 | 1.E-03 | 0.7 |
| FTD | Inc-MBOAT1-11:1 | SEQ5280 | 7.E-05 | 1.8 | All AD | TCONS_0006654 4 | SEQ2098 | 3.E-02 | 0.8 |
| MCI | Inc-MBOAT1-11:1 | SEQ5280 | 5.E-04 | 1.8 | FTD | TET2-AS1:5 | SEQ4311 | 2.E-04 | 0.6 |
| All AD | Inc-MBOAT2-2:1 | SEQ4338 | 2.E-03 | 0.7 | miAD | TET2-AS1:5 | SEQ4311 | 7.E-03 | 0.7 |
| miAD | Inc-MBOAT2-2:1 | SEQ4338 | 3.E-03 | 0.6 | All AD | TET2-AS1:5 | SEQ4311 | 2.E-02 | 0.7 |
| msAD | Inc-MBOAT2-2:1 | SEQ4338 | 5.E-03 | 0.7 | miAD | THAP9-AS1:27 | SEQ2178 | 1.E-02 | 0.6 |
| FTD | Inc-MCF2L-2:1 | SEQ5231 | 5.E-04 | 0.7 | All AD | THAP9-AS1:27 | SEQ2178 | 2.E-02 | 0.6 |
| DLB | Inc-MCHR1-1:1 | SEQ5182 | 9.E-05 | 1.5 | msAD | THRIL:2 | SEQ3970 | 2.E-02 | 1.4 |
| MCI | Inc-MCHR1-1:1 | SEQ5182 | 5.E-04 | 1.5 | All AD | THRIL:2 | SEQ3970 | 3.E-02 | 1.3 |
| All AD | Inc-MCL1-1:1 | SEQ3947 | 1.E-02 | 0.8 | FTD | THUMPD3-AS1:16 | SEQ5771 | 7.E-05 | 0.4 |
| msAD | Inc-MCL1-1:1 | SEQ3947 | 3.E-02 | 0.8 | DLB | THUMPD3-AS1:51 | SEQ5758 | 8.E-05 | 2.2 |
| All AD | Inc-MCL1-2:1 | SEQ5138 | 3.E-02 | 0.8 | MCI | TMEM161B-AS1:51 | SEQ4098 | 6.E-06 | 2.5 |
| All AD | Inc-MCL1-3:1 | SEQ5139 | 4.E-02 | 0.7 | FTD | TMEM161B-AS1:51 | SEQ4098 | 6.E-06 | 2.6 |
| FTD | Inc-MCTP2-1:1 | SEQ5748 | 9.E-05 | 0.7 | DLB | TMEM161B-AS1:51 | SEQ4098 | 2.E-05 | 2.5 |
| MCI | Inc-MCTP2-7:4 | SEQ3446 | 3.E-04 | 1.7 | miAD | TMEM161B-AS1:51 | SEQ4098 | 2.E-03 | 1.6 |
| DLB | Inc-MCTP2-7:4 | SEQ3446 | 5.E-04 | 1.8 | All AD | TMEM161B-AS1:51 | SEQ4098 | 2.E-03 | 1.5 |
| msAD | Inc-MCTP2-7:4 | SEQ3446 | 2.E-02 | 1.3 | msAD | TMEM161B-AS1:51 | SEQ4098 | 1.E-02 | 1.4 |
| All AD | Inc-MCTP2-7:4 | SEQ3446 | 2.E-02 | 1.3 | FTD | TMEM161B-AS1:52 | SEQ4308 | 1.E-09 | 0.3 |
| DLB | Inc-MCTS1-2:1 | SEQ2602 | 9.E-04 | 1.3 | MCI | TMEM161B-AS1:52 | SEQ4308 | 4.E-04 | 0.5 |
| All AD | Inc-M D M 1-5:1 | SEQ3748 | 1.E-02 | 0.6 | DLB | TMEM161B-AS1:52 | SEQ4308 | 2.E-03 | 0.5 |
| msAD | Inc-MDM1-5:1 | SEQ3748 | 4.E-02 | 0.6 | miAD | TMEM161B-AS1:52 | SEQ4308 | 7.E-03 | 0.5 |
| FTD | Inc-MDM4-8:1 | SEQ0816 | 2.E-04 | 0.7 | All AD | TMEM161B-AS1:52 | SEQ4308 | 2.E-02 | 0.6 |
| All AD | Inc-MED1-4:1 | SEQ5145 | 5.E-02 | 1.1 | MCI | TMEM202-AS1:3 | SEQ4015 | 1.E-05 | 1.7 |
| DLB | Inc-MED26-1:2 | SEQ4633 | 5.E-05 | 1.8 | DLB | TMEM202-AS1:3 | SEQ4015 | 2.E-05 | 1.7 |
| MCI | Inc-MED26-1:2 | SEQ4633 | 1.E-03 | 1.5 | FTD | TMEM202-AS1:3 | SEQ4015 | 9.E-05 | 1.5 |
| DLB | Inc-MED26-1:3 | SEQ4970 | 8.E-04 | 1.6 | All AD | TMEM202-AS1:3 | SEQ4015 | 7.E-03 | 1.3 |
| DLB | Inc-MED9-1:2 | SEQ2833 | 2.E-04 | 1.9 | miAD | TMEM202-AS1:3 | SEQ4015 | 1.E-02 | 1.3 |
| FTD | Inc-MEMO1-4:1 | SEQ4312 | 3.E-08 | 0.5 | msAD | TMEM202-AS1:3 | SEQ4015 | 2.E-02 | 1.3 |
| MCI | Inc-MEMO1-4:1 | SEQ4312 | 2.E-04 | 0.6 | DLB | TMEM9B-AS1:10 | SEQ4722 | 4.E-05 | 2.2 |
| miAD | Inc-MEMO1-4:1 | SEQ4312 | 7.E-03 | 0.7 | MCI | TMEM9B-AS1:10 | SEQ4722 | 1.E-03 | 1.9 |
| All AD | Inc-MEMO1-4:1 | SEQ4312 | 1.E-02 | 0.7 | DLB | TMEM9B-AS1:7 | SEQ5539 | 2.E-04 | 2.0 |
| DLB | Inc-MEP1B-2:1 | SEQ3447 | 1.E-04 | 2.1 | All AD | TMLHE-AS1:2 | SEQ6006 | 3.E-02 | 0.8 |
| MCI | Inc-MEP1B-2:1 | SEQ3447 | 5.E-04 | 1.8 | DLB | TMPO-AS1:5 | SEQ4339 | 3.E-06 | 1.8 |
| DLB | Inc-MEPCE-1:2 | SEQ5812 | 5.E-05 | 1.4 | MCI | TMPO-AS1:5 | SEQ4339 | 6.E-06 | 1.6 |
| DLB | Inc-MEPE-1:1 | SEQ4345 | 9.E-05 | 1.5 | FTD | TMPO-AS1-.5 | SEQ4339 | 7.E-06 | 1.6 |
| All AD | Inc-MEPE-1:1 | SEQ4345 | 2.E-03 | 1.3 | All AD | TMPO-AS1:5 | SEQ4339 | 1.E-03 | 1.3 |
| MCI | Inc-MEPE-1:1 | SEQ4345 | 2.E-03 | 1.3 | miAD | TMPO-AS1:5 | SEQ4339 | 2.E-03 | 1.3 |
| msAD | Inc-MEPE-1:1 | SEQ4345 | 3.E-03 | 1.3 | msAD | TMPO-AS1:5 | SEQ4339 | 5.E-03 | 1.3 |
| miAD | Inc-MEPE-1:1 | SEQ4345 | 5.E-03 | 1.3 | DLB | TNK2-AS1:1 | SEQ5728 | 9.E-05 | 1.5 |
| FTD | Inc-MEST-6:1 | SEQ0124 | 1.E-07 | 0.5 | MCI | TNRC6C-AS1:1 | SEQ3072 | 6.E-06 | 1.8 |
| MCI | Inc-MEST-6:1 | SEQ0124 | 2.E-04 | 0.6 | DLB | TNRC6C-AS1:1 | SEQ3072 | 8.E-05 | 1.8 |
| miAD | Inc-MEST-6:1 | SEQ0124 | 2.E-03 | 0.6 | MCI | TNRC6C-AS1:2 | SEQ3143 | 2.E-05 | 1.6 |
| All AD | Inc-MEST-6:1 | SEQ0124 | 3.E-03 | 0.7 | DLB | TNRC6C-AS1:2 | SEQ3143 | 5.E-04 | 1.6 |
| msAD | Inc-MEST-6:1 | SEQ0124 | 2.E-02 | 0.7 | MCI | TNRC6C-AS1:5 | SEQ3142 | 2.E-05 | 1.7 |
| All AD | Inc-METTL14-3:13 | SEQ5152 | 3.E-02 | 0.8 | DLB | TNRC6C-AS1:5 | SEQ3142 | 2.E-04 | 1.6 |
| DLB | Inc-METTL26-1:2 | SEQ5891 | 2.E-05 | 1.6 | DLB | TPT1-AS1:54 | SEQ2870 | 3.E-05 | 1.8 |
| FTD | Inc-METTL2B-3:1 | SEQ3710 | 9.E-04 | 1.7 | All AD | TRAF3IP2-AS1:35 | SEQ3829 | 4.E-02 | 1.3 |
| MCI | Inc-METTL2B-3:1 | SEQ3710 | 9.E-04 | 1.7 | msAD | TRAF3IP2-AS1:35 | SEQ3829 | 4.E-02 | 1.3 |
| All AD | Inc-METTL2B-3:1 | SEQ3710 | 4.E-02 | 1.3 | FTD | TRG-AS1:1 | SEQ3894 | 7.E-04 | 0.6 |
| msAD | Inc-METTL2B-3:1 | SEQ3710 | 5.E-02 | 1.3 | All AD | TRG-AS1:1 | SEQ3894 | 8.E-03 | 0.6 |
| DLB | Inc-METTL6-5:1 | SEQ5436 | 3.E-04 | 1.5 | miAD | TRG-AS1:1 | SEQ3894 | 9.E-03 | 0.6 |
| All AD | Inc-METTL8-3:1 | SEQ5157 | 3.E-02 | 1.2 | msAD | TRG-AS1:1 | SEQ3894 | 3.E-02 | 0.7 |
| FTD | Inc-MFN1-1:2 | SEQ4058 | 3.E-07 | 0.5 | All AD | TRPM2-AS:7 | SEQ6007 | 3.E-02 | 0.7 |
| MCI | Inc-MFN1-1:2 | SEQ4058 | 2.E-05 | 0.6 | DLB | TSPOAP1-AS1:2 | SEQ5852 | 3.E-05 | 23.4 |
| miAD | Inc-MFN1-1:2 | SEQ4058 | 2.E-04 | 0.6 | MCI | TSPOAP1-AS1:2 | SEQ5852 | 4.E-05 | 13.9 |
| All AD | Inc-MFN1-1:2 | SEQ4058 | 8.E-04 | 0.7 | msAD | TSPOAP1-AS1:9 | SEQ3939 | 3.E-02 | 1.1 |
| msAD | Inc-MFN1-1:2 | SEQ4058 | 2.E-02 | 0.7 | All AD | TSPOAP1-AS1:9 | SEQ3939 | 3.E-02 | 1.1 |
| DLB | Inc-MFNG-1:2 | SEQ5035 | 7.E-04 | 1.5 | FTD | TTC21B-AS1:1 | SEQ4910 | 9.E-04 | 0.7 |
| DLB | Inc-MFSD11-1:2 | SEQ5483 | 2.E-04 | 1.5 | FTD | TTC21B-AS1:2 | SEQ4735 | 8.E-05 | 0.6 |
| DLB | Inc-MFSD13A-2:1 | SEQ3448 | 2.E-04 | 1.3 | All AD | TTC21B-AS1:2 | SEQ4735 | 1.E-04 | 0.6 |
| DLB | Inc-MFSD3-2:1 | SEQ5576 | 2.E-04 | 1.7 | miAD | TTC21B-AS1:2 | SEQ4735 | 3.E-04 | 0.6 |
| FTD | Inc-MFSD4A-1:1 | SEQ2625 | 3.E-04 | 0.6 | msAD | TTC21B-AS1:2 | SEQ4735 | 6.E-04 | 0.6 |
| FTD | Inc-MGARP-3:1 | SEQ5165 | 2.E-04 | 0.7 | MCI | TTC21B-AS1:2 | SEQ4735 | 1.E-03 | 0.6 |
| All AD | Inc-MGARP-3:1 | SEQ5165 | 3.E-02 | 0.8 | All AD | TTC21B-AS1:33 | SEQ4252 | 3.E-03 | 0.7 |
| msAD | Inc-MGAT1-4:4 | SEQ3705 | 5.E-02 | 1.2 | miAD | TTC21B-AS1:33 | SEQ4252 | 6.E-03 | 0.6 |
| DLB | Inc-MGATS-3:1 | SEQ5166 | 5.E-04 | 1.5 | msAD | TTC21B-AS1:33 | SEQ4252 | 9.E-03 | 0.7 |
| All AD | Inc-MGATS-3:1 | SEQ5166 | 4.E-02 | 1.2 | All AD | TTN-AS1:4 | SEQ3692 | 4.E-02 | 1.4 |
| DLB | Inc-MGLL-5:1 | SEQ4517 | 2.E-05 | 1.6 | msAD | TTN-AS1:4 | SEQ3692 | 5.E-02 | 1.4 |
| MCI | Inc-MGLL-5:1 | SEQ4517 | 2.E-03 | 1.5 | FTD | TTN-AS1:58 | SEQ5991 | 1.E-06 | 0.5 |
| MCI | Inc-MICA-9:2 | SEQ4955 | 6.E-04 | 1.5 | All AD | TTN-AS1:6 | SEQ4123 | 1.E-02 | 0.6 |
| DLB | Inc-MICA-9:2 | SEQ4955 | 8.E-04 | 1.5 | msAD | TTN-AS1:6 | SEQ4123 | 1.E-02 | 0.6 |
| DLB | Inc-MIEF2-2:1 | SEQ5243 | 5.E-04 | 1.4 | MCI | TUG1:33 | SEQ4063 | 6.E-04 | 1.5 |
| DLB | Inc-MIER3-2:1 | SEQ4681 | 1.E-03 | 1.4 | All AD | TUG1:33 | SEQ4063 | 6.E-03 | 1.3 |
| All AD | Inc-MIER3-2:1 | SEQ4681 | 5.E-02 | 1.1 | miAD | TUG1:33 | SEQ4063 | 1.E-02 | 1.3 |
| FTD | Inc-MIOS-5:2 | SEQ3764 | 8.E-06 | 0.6 | msAD | TUG1:33 | SEQ4063 | 2.E-02 | 1.3 |
| All AD | Inc-MIOS-5:2 | SEQ3764 | 1.E-02 | 0.7 | DLB | TUG1:39 | SEQ5735 | 9.E-05 | 1.6 |
| msAD | Inc-MIOS-5:2 | SEQ3764 | 4.E-02 | 0.7 | DLB | TUG1:40 | SEQ5895 | 2.E-05 | 1.8 |
| DLB | Inc-MIS12-4:1 | SEQ4840 | 9.E-04 | 1.4 | FTD | TUG1:45 | SEQ5840 | 2.E-06 | 0.5 |
| DLB | Inc-MISP3-1:7 | SEQ4619 | 1.E-03 | 1.4 | MCI | TUG1:45 | SEQ5840 | 4.E-05 | 0.5 |
| All AD | Inc-MIXL1-7:1 | SEQ5170 | 5.E-02 | 0.8 | FTD | TUG1:7 | SEQ4607 | 1.E-06 | 0.3 |
| All AD | Inc-MKI67-4:1 | SEQ5171 | 5.E-02 | 0.8 | MCI | TUG1:7 | SEQ4607 | 1.E-06 | 0.3 |
| All AD | Inc-MKI67-8:2 | SEQ5172 | 4.E-02 | 0.7 | DLB | TUG1:7 | SEQ4607 | 1.E-03 | 0.4 |
| MCI | Inc-MKLN1-1:12 | SEQ5004 | 9.E-06 | 2.0 | FTD | UBRS-AS1:10 | SEQ5412 | 3.E-04 | 0.6 |
| DLB | Inc-MKLN1-1:12 | SEQ5004 | 2.E-05 | 1.9 | MCI | UBR5-AS1:4 | SEQ5078 | 9.E-05 | 0.3 |
| FTD | Inc-MKLN1-1:12 | SEQ5004 | 8.E-04 | 1.5 | DLB | UBRS-AS1:4 | SEQ5078 | 1.E-04 | 0.3 |
| FTD | Inc-MLH1-1:1 | SEQ2597 | 8.E-06 | 0.7 | FTD | UBRS-AS1:4 | SEQ5078 | 6.E-04 | 0.3 |
| MCI | Inc-MLH1-1:1 | SEQ2597 | 2.E-04 | 0.8 | All AD | USP3-AS1:11 | SEQ6010 | 3.E-02 | 0.8 |
| All AD | Inc-MLH1-1:1 | SEQ2597 | 7.E-03 | 0.8 | FTD | USP3-AS1:9 | SEQ4289 | 9.E-10 | 0.1 |
| msAD | Inc-MLH1-1:1 | SEQ2597 | 9.E-03 | 0.8 | MCI | USP3-AS1:9 | SEQ4289 | 4.E-04 | 0.2 |
| MCI | Inc-MLLT1-2:1 | SEQ3450 | 5.E-04 | 1.4 | miAD | USP3-AS1:9 | SEQ4289 | 7.E-03 | 0.2 |
| DLB | Inc-MLLT1-2:1 | SEQ3450 | 8.E-04 | 1.4 | DLB | VAC14-AS1:3 | SEQ5572 | 2.E-04 | 1.6 |
| DLB | Inc-MLX-3:1 | SEQ3451 | 4.E-05 | 1.6 | DLB | VASH1-AS1:7 | SEQ4786 | 1.E-03 | 1.7 |
| MCI | Inc-MLX-3:1 | SEQ3451 | 2.E-03 | 1.5 | DLB | VIM-AS1:1 | SEQ3832 | 5.E-05 | 1.5 |
| All AD | Inc-MLX-3:1 | SEQ3451 | 4.E-02 | 1.1 | MCI | VIM-AS1:1 | SEQ3832 | 8.E-05 | 1.5 |
| MCI | Inc-MMP23B-1:1 | SEQ4520 | 2.E-03 | 1.5 | FTD | VIM-AS1:1 | SEQ3832 | 2.E-04 | 1.3 |
| DLB | Inc-MMP23B-4:1 | SEQ4774 | 1.E-03 | 1.5 | All AD | VIM-AS1:1 | SEQ3832 | 2.E-02 | 1.2 |
| FTD | Inc-MOCS2-9:1 | SEQ5832 | 5.E-05 | 0.6 | msAD | VIM-AS1:1 | SEQ3832 | 3.E-02 | 1.2 |
| FTD | Inc-MOV10-2:1 | SEQ2519 | 4.E-04 | 0.5 | FTD | VIM-AS1:11 | SEQ3989 | 1.E-03 | 0.7 |
| All AD | Inc-MOV10-2:1 | SEQ2519 | 3.E-02 | 0.6 | All AD | VIM-AS1:11 | SEQ3989 | 1.E-02 | 0.8 |
| MCI | Inc-MPC1-3:1 | SEQ4672 | 1.E-03 | 0.8 | msAD | VIM-AS1:11 | SEQ3989 | 2.E-02 | 0.9 |
| MCI | Inc-M PLKIP-1:1 | SEQ5848 | 1.E-05 | 1.5 | All AD | VIM-AS1:7 | SEQ3576 | 7.E-03 | 1.2 |
| DLB | Inc-M PLKIP-1:1 | SEQ5848 | 4.E-05 | 1.5 | miAD | VIM-AS1:7 | SEQ3576 | 1.E-02 | 1.2 |
| DLB | Inc-MRNIP-4:1 | SEQ4511 | 2.E-05 | 1.8 | msAD | VIM-AS1:7 | SEQ3576 | 2.E-02 | 1.2 |
| MCI | Inc-MRNIP-4:1 | SEQ4511 | 2.E-03 | 1.5 | DLB | WAC-AS1:1 | SEQ5662 | 1.E-04 | 1.5 |
| DLB | Inc-MRPL11-1:11 | SEQ5481 | 2.E-04 | 1.4 | DLB | WAC-AS1:11 | SEQ4770 | 1.E-03 | 1.5 |
| FTD | Inc-MRPL16-1:1 | SEQ5189 | 4.E-04 | 0.7 | FTD | WAC-AS1:14 | SEQ4892 | 9.E-04 | 0.5 |
| All AD | Inc-MRPL16-1:1 | SEQ5189 | 5.E-02 | 0.8 | DLB | ZBED5-AS1:1 | SEQ4959 | 8.E-04 | 1.6 |
| DLB | Inc-MRPL41-1:1 | SEQ5528 | 2.E-04 | 1.6 | MCI | ZBTB11-AS1:2 | SEQ5006 | 1.E-04 | 2.4 |
| MCI | Inc-MRPL43-2:3 | SEQ3791 | 9.E-06 | 1.6 | DLB | ZBTB11-AS1:2 | SEQ5006 | 1.E-04 | 2.2 |
| DLB | Inc-MRPL43-2:3 | SEQ3791 | 2.E-05 | 1.7 | FTD | ZBTB11-AS1:2 | SEQ5006 | 8.E-04 | 2.1 |
| FTD | Inc-MRPL43-2:3 | SEQ3791 | 4.E-04 | 1.4 | All AD | ZBTB11-AS1:2 | SEQ5006 | 2.E-02 | 1.4 |
| msAD | Inc-MRPL43-2:3 | SEQ3791 | 4.E-02 | 1.2 | FTD | ZFAS1:30 | SEQ4911 | 9.E-04 | 0.7 |
| FTD | Inc-MRPL44-6:2 | SEQ5773 | 7.E-05 | 0.4 | DLB | ZFHX2-AS1:10 | SEQ4957 | 8.E-04 | 1.6 |
| MCI | Inc-MRPL48-1:2 | SEQ5193 | 2.E-07 | 2.1 | FTD | ZMIZ1-AS1:33 | SEQ4236 | 2.E-04 | 0.5 |
| FTD | Inc-MRPL48-1:2 | SEQ5193 | 3.E-04 | 1.6 | miAD | ZMIZ1-AS1:33 | SEQ4236 | 1.E-02 | 0.7 |
| DLB | Inc-MRPL48-1:2 | SEQ5193 | 4.E-04 | 1.7 | All AD | ZMIZ1-AS1:33 | SEQ4236 | 3.E-02 | 0.7 |
| All AD | Inc-MRPL48-1:2 | SEQ5193 | 4.E-02 | 1.1 | miAD | ZNF436-AS1:9 | SEQ4235 | 1.E-02 | 0.6 |
| DLB | Inc-MRPL48-1:3 | SEQ4932 | 8.E-04 | 1.4 | All AD | ZNF436-AS1:9 | SEQ4235 | 3.E-02 | 0.7 |
| DLB | Inc-MRPL58-2:2 | SEQ5615 | 2.E-04 | 1.4 | DLB | ZNF460-AS1:2 | SEQ3955 | 7.E-04 | 1.7 |
| FTD | Inc-MRPS14-1:6 | SEQ2452 | 2.E-04 | 0.7 | msAD | ZNF460-AS1:2 | SEQ3955 | 3.E-02 | 1.3 |
| DLB | Inc-MRPS25-5:2 | SEQ5123 | 6.E-04 | 1.5 | All AD | ZNF460-AS1:2 | SEQ3955 | 4.E-02 | 1.2 |
| MCI | Inc-MRPS5-2:2 | SEQ4304 | 9.E-05 | 2.1 | FTD | ZSCAN16-AS1:12 | SEQ4808 | 1.E-03 | 0.7 |

To further identify IncRNAs differentially expressed in AD patients, whole blood (Paxgene RNA tube) expression in AD patients were compared with non-AD subject groups. 136 IncRNAs were differentially expressed in whole blood (Paxgene RNA tube) of AD patient groups (mild AD + moderate-to-severe AD groups) when compared to non-AD subject groups (either DLB+ FTD groups or DLB+FTD+HC groups), with statistical significance (p value <0.05, Wilcoxon test) and a fold change FC≤0.80 or ≥1.20. The 136 IncRNAs are shown in Table 11.

**Table 11: 136 whole blood (Paxgene RNA tube) IncRNAs differentially expressed in AD patient groups (mild AD + moderate-to-severe AD groups) when compared to non-AD subject groups (either DLB+ FTD groups or DLB+FTD+HC groups).**

| **Group compared to AD** | **IncRNA** | **SEQ** | **p-value** | **FC** | **Group compared to AD** | **IncRNA** | **SEQ** | **p-value** | **FC** |
|---|---|---|---|---|---|---|---|---|---|
| NAD | ARRDC3-AS1:3 | SEQ6042 | 8E-04 | 0.8 | NAD+HC | Inc-LRRC37A3-5:8 | SEQ3442 | 4E-03 | 0.8 |
| NAD+HC | ARRDC3-AS1:3 | SEQ6042 | 3E-03 | 0.8 | NAD | Inc-LRRC47-4:4 | SEQ6118 | 4E-04 | 0.7 |
| NAD+HC | CARS-AS1:1 | SEQ6047 | 4E-03 | 0.8 | NAD | Inc-MARS-1:1 | SEQ6068 | 3E-04 | 0.8 |
| NAD | CELF2-AS1:2 | SEQ6134 | 2E-05 | 0.5 | NAD+HC | Inc-MARS-1:1 | SEQ6068 | 3E-04 | 0.8 |
| NAD+HC | CFLAR-AS1:21 | SEQ6090 | 2E-03 | 0.8 | NAD+HC | Inc-NDUFB3-2:1 | SEQ6043 | 2E-03 | 0.8 |
| NAD+HC | CFLAR-AS1:8 | SEQ6080 | 2E-03 | 0.8 | NAD | Inc-NPTX1-2:4 | SEQ6117 | 1E-04 | 0.6 |
| NAD+HC | CSNK1G2-AS1:2 | SEQ6094 | 2E-03 | 0.8 | NAD+HC | Inc-NPTX1-2:4 | SEQ6117 | 5E-04 | 0.7 |
| NAD | DLEU2:21 | SEQ6064 | 7E-05 | 0.7 | NAD | Inc-OR4F29-7:1 | SEQ6119 | 6E-04 | 0.6 |
| NAD+HC | DLEU2:21 | SEQ6064 | 1E-03 | 0.8 | NAD+HC | Inc-OR4F29-7:1 | SEQ6119 | 3E-03 | 0.7 |
| NAD | FAM157C:5 | SEQ6125 | 2E-04 | 0.6 | NAD | Inc-OTOL1-5:1 | SEQ6019 | 8E-05 | 2.3 |
| NAD+HC | FAM157C:5 | SEQ6125 | 3E-04 | 0.7 | NAD+HC | Inc-OTOL1-5:1 | SEQ6019 | 2E-04 | 1.9 |
| NAD | FAM198B-AS1:2 | SEQ6051 | 3E-05 | 0.7 | NAD | Inc-PBX2-1:1 | SEQ6081 | 3E-04 | 0.7 |
| NAD+HC | FAM198B-AS1:2 | SEQ6051 | 1E-03 | 0.8 | NAD+HC | Inc-PBX2-1:1 | SEQ6081 | 3E-04 | 0.8 |
| NAD+HC | ITGB2-AS1:6 | SEQ6097 | 4E-03 | 0.7 | NAD | Inc-PBX2-1:2 | SEQ6077 | 3E-04 | 0.7 |
| NAD | JPX:34 | SEQ6017 | 3E-04 | 2.7 | NAD+HC | Inc-PBX2-1:2 | SEQ6077 | 4E-04 | 0.8 |
| NAD+HC | LCMT1-AS1:9 | SEQ2883 | 4E-03 | 0.7 | NAD+HC | Inc-PGAM1-4:1 | SEQ6066 | 3E-03 | 0.8 |
| NAD | LINC00324:3 | SEQ6113 | 3E-04 | 0.7 | NAD+HC | Inc-PLCD3-4:8 | SEQ6112 | 3E-03 | 0.7 |
| NAD | LINC00861:2 | SEQ6132 | 7E-04 | 0.6 | NAD | Inc-PLCG2-6:1 | SEQ6095 | 7E-04 | 0.7 |
| NAD+HC | LINC01000:3 | SEQ6093 | 1E-03 | 0.8 | NAD+HC | Inc-PLEKHG3-1:1 | SEQ6056 | 3E-03 | 0.8 |
| NAD+HC | LINC01001:1 | SEQ6128 | 2E-03 | 0.6 | NAD | Inc-RMDN2-11:1 | SEQ6067 | 2E-04 | 0.7 |
| NAD+HC | LINC01814:2 | SEQ6071 | 2E-03 | 0.8 | NAD+HC | Inc-RMDN2-11:1 | SEQ6067 | 2E-03 | 0.8 |
| NAD | Inc-ALDH1A2-9:1 | SEQ6031 | 4E-04 | 1.5 | NAD | Inc-RNF19A-8:2 | SEQ6085 | 7E-04 | 0.8 |
| NAD | lnc-ANXA2R-1:25 | SEQ6121 | 3E-06 | 0.6 | NAD | lnc-ROM1-7:2 | SEQ6015 | 9E-06 | 3.8 |
| NAD+HC | lnc-ANXA2R-1:25 | SEQ6121 | 2E-05 | 0.7 | NAD+HC | lnc-ROM1-7:2 | SEQ6015 | 3E-04 | 2.9 |
| NAD | Inc-ARFGEF2-6:1 | SEQ6083 | 4E-04 | 0.7 | NAD | lnc-RPEL1-2:1 | SEQ6102 | 5E-04 | 0.7 |
| NAD+HC | lnc-ARFGEF2-6:1 | SEQ6083 | 2E-03 | 0.8 | NAD+HC | lnc-RPEL1-2:1 | SEQ6102 | 6E-04 | 0.7 |
| NAD | lnc-ATG2A-2:1 | SEQ6087 | 5E-04 | 0.8 | NAD+HC | lnc-RPLP0-4:4 | SEQ6060 | 2E-03 | 0.8 |
| NAD | lnc-ATP6V1G3-6:1 | SEQ6079 | 6E-04 | 0.8 | NAD | lnc-SAG-4:1 | SEQ6114 | 6E-04 | 0.7 |
| NAD+HC | lnc-B3GNTL1-2:2 | SEQ6040 | 4E-03 | 0.8 | NAD | lnc-SAMD11-12:2 | SEQ6021 | 2E-04 | 2.0 |
| NAD | lnc-BCL2L11-6:5 | SEQ6101 | 2E-04 | 0.7 | NAD+HC | lnc-SAMD11-12:2 | SEQ6021 | 1E-03 | 1.7 |
| NAD+HC | lnc-BCL2L11-6:5 | SEQ6101 | 2E-03 | 0.7 | NAD | lnc-SAMD11-15:1 | SEQ6030 | 4E-04 | 1.5 |
| NAD | Inc-BRD3-7:1 | SEQ6108 | 4E-05 | 0.6 | NAD+HC | lnc-SAMD11-15:1 | SEQ6030 | 9E-04 | 1.4 |
| NAD+HC | lnc-BRD3-7:1 | SEQ6108 | 2E-04 | 0.7 | NAD+HC | lnc-SBDS-19:2 | SEQ2509 | 3E-03 | 1.5 |
| NAD | lnc-C1QTNF3-AMACR-2:5 | SEQ6027 | 4E-04 | 1.6 | NAD+HC | lnc-SETD9-6:1 | SEQ6091 | 1E-03 | 0.8 |
| NAD | lnc-C5orf56-4:1 | SEQ6082 | 1E-04 | 0.7 | NAD | lnc-SETDB1-1:1 | SEQ6115 | 4E-04 | 0.7 |
| NAD+HC | lnc-C5orf56-4:1 | SEQ6082 | 3E-04 | 0.8 | NAD+HC | lnc-SETDB1-1:1 | SEQ6115 | 6E-04 | 0.7 |
| NAD | lnc-CACNA1B-1:2 | SEQ6120 | 3E-04 | 0.6 | NAD | lnc-SHC3-5:3 | SEQ3102 | 6E-04 | 1.9 |
| NAD+HC | lnc-CACNA1B-1:2 | SEQ6120 | 1E-03 | 0.7 | NAD | lnc-SLC29A4-1:2 | SEQ6052 | 5E-04 | 0.8 |
| NAD | lnc-CACNA1B-1:4 | SEQ6122 | 3E-04 | 0.6 | NAD+HC | lnc-SLC29A4-1:2 | SEQ6052 | 2E-03 | 0.8 |
| NAD+HC | lnc-CACNA1B-1:4 | SEQ6122 | 1E-03 | 0.7 | NAD | lnc-SLC35F5-3:9 | SEQ6022 | 7E-04 | 1.9 |
| NAD+HC | lnc-CAND2-2:5 | SEQ3083 | 2E-03 | 0.8 | NAD+HC | lnc-SLC35F5-3:9 | SEQ6022 | 1E-03 | 1.7 |
| NAD+HC | lnc-CAND2-2:6 | SEQ3084 | 2E-03 | 0.8 | NAD | lnc-SLC5A10-3:2 | SEQ6133 | 3E-04 | 0.5 |
| NAD | lnc-CATSPER3-2:10 | SEQ6104 | 5E-04 | 0.7 | NAD | lnc-SNX11-10:2 | SEQ6024 | 5E-04 | 1.8 |
| NAD | lnc-CDC42BPB-4:1 | SEQ3357 | 3E-04 | 0.8 | NAD+HC | lnc-SNX11-10:2 | SEQ6024 | 8E-04 | 1.7 |
| NAD | lnc-CDC42BPB-4:4 | SEQ6050 | 3E-04 | 0.8 | NAD | lnc-SPATA32-2:12 | SEQ6109 | 1E-04 | 0.7 |
| NAD+HC | lnc-CDC42BPB-4:4 | SEQ6050 | 2E-03 | 0.8 | NAD+HC | lnc-SPATA32-2:12 | SEQ6109 | 1E-04 | 0.7 |
| NAD | lnc-CDHR4-5:2 | SEQ6089 | 5E-04 | 0.8 | NAD+HC | lnc-STX4-1:4 | SEQ6075 | 3E-03 | 0.8 |
| NAD | lnc-CDIPT-1:1 | SEQ6048 | 5E-04 | 0.7 | NAD | lnc-SYNPO-6:1 | SEQ6123 | 1E-04 | 0.7 |
| NAD+HC | lnc-CDIPT-1:1 | SEQ6048 | 4E-03 | 0.8 | NAD | lnc-SYT2-4:1 | SEQ6107 | 2E-04 | 0.7 |
| NAD+HC | lnc-CDIPT-1:5 | SEQ6028 | 5E-04 | 1.5 | NAD | lnc-TARDBP-5:3 | SEQ6131 | 5E-04 | 0.6 |
| NAD | lnc-CDIPT-1:5 | SEQ6028 | 7E-04 | 1.6 | NAD | lnc-TCF19-1:103 | SEQ6130 | 6E-04 | 0.6 |
| NAD+HC | lnc-CEBPB-13:13 | SEQ6070 | 3E-03 | 0.8 | NAD | lnc-THTPA-2:29 | SEQ6016 | 8E-05 | 2.7 |
| NAD+HC | lnc-CEBPG-4:1 | SEQ6069 | 2E-03 | 0.8 | NAD+HC | lnc-THTPA-2:29 | SEQ6016 | 2E-04 | 2.2 |
| NAD | lnc-CEL-4:1 | SEQ6032 | 7E-04 | 1.5 | NAD+HC | lnc-TLE3-6:8 | SEQ6035 | 3E-03 | 1.3 |
| NAD+HC | lnc-CHIC1-2:1 | SEQ6026 | 1E-03 | 1.7 | NAD+HC | lnc-TMEM150A-4:2 | SEQ6046 | 4E-03 | 0.8 |
| NAD+HC | lnc-CLLU1-2:6 | SEQ3284 | 1E-04 | 12 | NAD+HC | lnc-TMEM181-1:1 | SEQ6084 | 2E-03 | 0.8 |
| NAD | lnc-CLLU1-2:6 | SEQ3284 | 2E-04 | 18 | NAD | lnc-TREM2-1:1 | SEQ6029 | 1E-04 | 1.5 |
| NAD+HC | lnc-CNTRL-6:31 | SEQ6023 | 3E-03 | 1.8 | NAD | lnc-TRMT61B-5:6 | SEQ6103 | 9E-05 | 0.7 |
| NAD+HC | lnc-CTXN1- | SEQ3096 | 3E-03 | 0.8 | NAD+HC | lnc-TRMT61B-5:6 | SEQ6103 | 2E-04 | 0.7 |
| NAD | lnc-DDX39A-4:1 | SEQ6045 | 3E-04 | 0.8 | NAD | lnc-UGT2B28-1:2 | SEQ1001 | 4E-04 | 1.8 |
| NAD+HC | lnc-DDX39A-4:1 | SEQ6045 | 3E-03 | 0.8 | NAD+HC | lnc-UGT2B28-1:2 | SEQ1001 | 2E-03 | 1.7 |
| NAD | lnc-DDX49-1:1 | SEQ2759 | 3E-04 | 0.8 | NAD | lnc-UNC93B1-1:8 | SEQ6092 | 6E-05 | 0.8 |
| NAD+HC | lnc-DDX49-1:1 | SEQ2759 | 3E-03 | 0.9 | NAD+HC | lnc-WASHC3-2:3 | SEQ6073 | 3E-03 | 0.8 |
| NAD | lnc-DEPTOR-2:1 | SEQ6037 | 4E-04 | 1.3 | NAD | lnc-WDR81-6:1 | SEQ6055 | 8E-04 | 0.7 |
| NAD | lnc-DGKE-7:1 | SEQ6062 | 3E-04 | 0.7 | NAD+HC | lnc-WDR81-6:1 | SEQ6055 | 3E-03 | 0.8 |
| NAD+HC | lnc-DGKE-7:1 | SEQ6062 | 4E-03 | 0.8 | NAD+HC | lnc-WSB1-1:1 | SEQ6054 | 2E-03 | 0.8 |
| NAD+HC | lnc-DHRS7B-1:10 | SEQ6041 | 1E-03 | 0.8 | NAD+HC | lnc-ZFP57-21:6 | SEQ6099 | 3E-03 | 0.7 |
| NAD | lnc-EIF4H-1:1 | SEQ6053 | 1E-04 | 0.7 | NAD | lnc-ZNF705B-1:6 | SEQ6086 | 7E-04 | 0.7 |
| NAD+HC | lnc-EIF4H-1:1 | SEQ6053 | 8E-04 | 0.8 | NAD+HC | lnc-ZNF705B-1:6 | SEQ6086 | 3E-03 | 0.8 |
| NAD | lnc-EPHB1-1:1 | SEQ6129 | 1E-04 | 0.6 | NAD | lnc-ZRANB1-7:1 | SEQ6039 | 6E-04 | 0.8 |
| NAD+HC | lnc-FANCI-5:3 | SEQ6044 | 3E-03 | 0.8 | NAD+HC | lnc-ZRANB1-7:1 | SEQ6039 | 2E-03 | 0.8 |
| NAD | lnc-FKBP6-4:1 | SEQ3394 | 8E-04 | 0.8 | NAD+HC | MALAT1:17 | SEQ6076 | 2E-03 | 0.8 |
| NAD+HC | lnc-FKBP6-4:1 | SEQ3394 | 3E-03 | 0.8 | NAD+HC | MIR22HG:36 | SEQ6038 | 2E-03 | 0.9 |
| NAD+HC | lnc-GABBR1-1:1 | SEQ6036 | 2E-03 | 1.3 | NAD+HC | MIR4435-2HG:29 | SEQ6074 | 4E-03 | 0.8 |
| NAD | lnc-GINS2- | SEQ6096 | 2E-04 | 0.7 | NAD | MME-AS1:1 | SEQ6106 | 1E-04 | 0.6 |
| NAD+HC | lnc-GINS2- | SEQ6096 | 4E-03 | 0.7 | NAD+HC | MME-AS1:1 | SEQ6106 | 2E-03 | 0.7 |
| NAD+HC | lnc-GPR20-3:6 | SEQ6105 | 3E-03 | 0.7 | NAD | NALT1:14 | SEQ6127 | 7E-04 | 0.7 |
| NAD+HC | lnc-GPR68-1:1 | SEQ6088 | 2E-03 | 0.8 | NAD | NUTM2A-AS1:16 | SEQ6110 | 2E-04 | 0.6 |
| NAD | lnc-GTPBP1-4:1 | SEQ6072 | 3E-04 | 0.7 | NAD+HC | NUTM2A-AS1:16 | SEQ6110 | 1E-03 | 0.7 |
| NAD+HC | lnc-GTPBP1-4:1 | SEQ6072 | 6E-04 | 0.8 | NAD+HC | PCBP1-AS1:170 | SEQ6098 | 4E-03 | 0.7 |
| NAD | lnc-HLA-B-2:12 | SEQ6116 | 4E-06 | 0.6 | NAD+HC | PCBP1-AS1:27 | SEQ6100 | 3E-03 | 0.7 |
| NAD+HC | lnc-HLA-B-2:12 | SEQ6116 | 9E-05 | 0.7 | NAD+HC | RARA-AS1:2 | SEQ6049 | 2E-03 | 0.8 |
| NAD+HC | lnc-HYAL3-1:1 | SEQ6065 | 8E-04 | 0.8 | NAD | SNHG10:11 | SEQ6078 | 3E-04 | 0.8 |
| NAD | lnc-IL10-1:1 | SEQ6126 | 3E-04 | 0.7 | NAD | SNHG22:6 | SEQ6034 | 7E-04 | 1.4 |
| NAD | lnc-IL31RA- | SEQ2557 | 8E-04 | 0.7 | NAD+HC | SNHG7:16 | SEQ6124 | 4E-04 | 0.7 |
| NAD | lnc-INSM2-8:1 | SEQ6033 | 4E-04 | 1.4 | NAD | SNHG7:16 | SEQ6124 | 4E-04 | 0.6 |
| NAD+HC | lnc-IRF1-2:5 | SEQ6058 | 4E-03 | 0.8 | NAD+HC | TCONS_00011974 | SEQ1218 | 3E-03 | 0.8 |
| NAD | lnc-ISYNA1-2:1 | SEQ6063 | 3E-04 | 0.8 | NAD | TCONS_00017370 | SEQ1271 | 4E-05 | 103 |
| NAD+HC | lnc-ISYNA1-2:1 | SEQ6063 | 2E-03 | 0.8 | NAD+HC | TCONS_00017370 | SEQ1271 | 3E-03 | 66 |
| NAD+HC | lnc-KIAA1551-3:1 | SEQ6061 | 2E-03 | 0.8 | NAD+HC | TCONS_00017373 | SEQ1274 | 2E-03 | 1.5 |
| NAD | lnc-KLHDC7B-5:2 | SEQ6111 | 7E-04 | 0.7 | NAD | XIST:27 | SEQ6020 | 4E-04 | 2.1 |
| NAD+HC | lnc-LAX1-4:1 | SEQ6059 | 4E-03 | 0.8 | NAD+HC | XIST:27 | SEQ6020 | 1E-03 | 1.8 |
| NAD | lnc-LGALS2-1:2 | SEQ6057 | 3E-04 | 0.8 | NAD+HC | XIST:47 | SEQ6025 | 3E-03 | 1.7 |
| NAD+HC | lnc-LGALS2-1:2 | SEQ6057 | 2E-03 | 0.8 | NAD | ZNF529-AS1:19 | SEQ6018 | 3E-05 | 2.4 |

Brain-enriched IncRNAs: Out of the 10122 IncRNAs sequenced by the invention in the AD and control brains and having an expression level > 5 CPM (median), 860 IncRNAs showed an expression level 2-fold higher in the brain as compared to peripheral organs lung, ovary, colon, prostate, breast, liver, bladder, kidney, skin, heart, muscle. The 860 IncRNAs are shown in Table 12.

**Table 12: 860 brain-enriched IncRNAs with an expression level in the brain at least 2-fold higher than that in 12 peripheral organs.**

| **IncRNA** | **SEQ** | **Ratio Br/org** | **IncRNA** | **SEQ** | **Ratio Br/Org** |
|---|---|---|---|---|---|
| TCONS_00005325 | SEQ1141 | 7.7E+01 | TCONS_00062327 | SEQ2005 | 7.4E+23 |
| TCONS_00035021 | SEQ1566 | 4.0E+00 | TCONS_00062311 | SEQ1996 | 1.9E+05 |
| TCONS_00035024 | SEQ1571 | 1.9E+01 | TCONS_00033704 | SEQ1562 | 2.8E+03 |
| TCONS_00035022 | SEQ1567 | 4.6E+32 | TCONS_00036143 | SEQ1611 | 9.6E+01 |
| TCONS_00012059 | SEQ1200 | 1.2E+03 | TCONS_00045398 | SEQ1789 | 7.7E+01 |
| TCONS_00045364 | SEQ1785 | 3.7E+00 | TCONS_00033666 | SEQ1553 | 3.9E+01 |
| TCONS_00050012 | SEQ1801 | 6.7E+02 | TCONS_00021559 | SEQ1333 | 3.3E+01 |
| TCONS_00035023 | SEQ1568 | 6.9E+04 | TCONS_00026547 | SEQ1410 | 2.6E+01 |
| TCONS_00012060 | SEQ1201 | 5.6E+02 | TCONS_00055516 | SEQ1902 | 2.4E+01 |
| TCONS_00035090 | SEQ1564 | 4.8E+00 | TCONS_00062422 | SEQ2040 | 2.4E+01 |
| TCONS_00035091 | SEQ1565 | 1.3E+01 | TCONS_00024800 | SEQ1391 | 2.0E+01 |
| TCONS_00050014 | SEQ1803 | 1.2E+03 | TCONS_00021416 | SEQ1328 | 1.9E+01 |
| TCONS_00035094 | SEQ1573 | 2.7E+01 | TCONS_00014326 | SEQ1225 | 1.3E+01 |
| TCONS_00035092 | SEQ1569 | 3.4E+02 | TCONS_00045346 | SEQ1781 | 9.0E+00 |
| TCONS_00035093 | SEQ1570 | 4.5E+01 | TCONS_00021544 | SEQ1320 | 9.0E+00 |
| TCONS_00012009 | SEQ1185 | 3.2E+01 | TCONS_00023115 | SEQ1352 | 7.6E+00 |
| TCONS_00045125 | SEQ1790 | 1.1E+01 | TCONS_00014261 | SEQ1249 | 6.3E+00 |
| TCONS_00024864 | SEQ1388 | 5.0E+01 | lnc-NKX1-2-2:1 | SEQ6510 | 3.2 E+00 |
| lnc-SP110-8:2 | SEQ6135 | 9.6E+02 | lnc-HSDL1-1:1 | SEQ6511 | 3.2 E+00 |
| MEG3:21 | SEQ6136 | 2.0E+02 | lnc-FAM115C-1:1 | SEQ6512 | 3.2 E+00 |
| lnc-CCDC150-6:1 | SEQ6137 | 1.8E+02 | Inc-AARSD1-2:7 | SEQ6513 | 3.2 E+00 |
| lnc-ZNF45-2:1 | SEQ6138 | 1.7E+02 | LINC00969:38 | SEQ6514 | 3.2E+00 |
| NUTM2B-AS1:31 | SEQ6139 | 1.5E+02 | lnc-VPREB1-7:13 | SEQ6515 | 3.2 E+00 |
| lnc-HMBS-1:1 | SEQ6140 | 1.2E+02 | lnc-MYO15A-1:1 | SEQ6516 | 3.2 E+00 |
| TMEM5-AS1:2 | SEQ6141 | 9.4E+01 | lnc-SNURF-1:22 | SEQ6517 | 3.2 E+00 |
| Inc-ZNF583-4:2 | SEQ6142 | 9.2E+01 | lnc-ZNF415-2:9 | SEQ6518 | 3.2E+00 |
| lnc-LYRM2-2:1 | SEQ6143 | 6.5E+01 | lnc-KNG1-2:5 | SEQ6519 | 3.2E+00 |
| lnc-TBX2-4:2 | SEQ6144 | 6.5E+01 | LINC00641:4 | SEQ6520 | 3.1E+00 |
| lnc-NAA50-3:1 | SEQ2266 | 6.1E+01 | lnc-KCND3-3:1 | SEQ6521 | 3.1E+00 |
| lnc-HINFP-1:1 | SEQ6145 | 5.9E+01 | lnc-XPOT-2:1 | SEQ6522 | 3.1E+00 |
| MIR4458HG:7 | SEQ6146 | 4.8E+01 | lnc-ARIH2-2:6 | SEQ6523 | 3.1E+00 |
| lnc-SLC25A44-1:2 | SEQ6147 | 4.4E+01 | lnc-GNGT1-1:3 | SEQ6524 | 3.1E+00 |
| lnc-SEPT5-1:4 | SEQ6148 | 4.2E+01 | lnc-CEPT1-1:6 | SEQ6525 | 3.1E+00 |
| lnc-UBE4A-1:1 | SEQ6149 | 4.1E+01 | lnc-SLC25A4-1:1 | SEQ6526 | 3.1E+00 |
| lnc-RP11-334E6.3.1-2:1 | SEQ6150 | 4.0E+01 | lnc-AGAP9-2:2 | SEQ6527 | 3.1E+00 |
| lnc-GBP5-2:6 | SEQ6151 | 4.0E+01 | lnc-DLG5-4:2 | SEQ6528 | 3.1E+00 |
| lnc-FAM49B-5:1 | SEQ6152 | 4.0E+01 | lnc-PPHLN1-1:2 | SEQ6529 | 3.1E+00 |
| STXBP5-AS1:2 | SEQ6153 | 4.0E+01 | lnc-RBM25-2:1 | SEQ6530 | 3.1E+00 |
| lnc-RO8O3-2:2 | SEQ6154 | 3.9E+01 | lnc-USH2A-4:1 | SEQ6531 | 3.1E+00 |
| lnc-ZNF91-4:4 | SEQ6155 | 3.9E+01 | lnc-ENC1-5:1 | SEQ2244 | 3.1E+00 |
| lnc-ATRNL1-1:1 | SEQ6156 | 3.7E+01 | lnc-FAM106A-2:18 | SEQ6532 | 3.1E+00 |
| lnc-MAPK8IP1-3:1 | SEQ6157 | 3.5E+01 | lnc-METTL17-1:2 | SEQ6533 | 3.1E+00 |
| LINC01024:12 | SEQ6158 | 3.4E+01 | lnc-TMEM178-1:19 | SEQ6534 | 3.1E+00 |
| lnc-CSNK2A2-2:2 | SEQ6159 | 3.2E+01 | lnc-CHRNAS-4:7 | SEQ6535 | 3.1E+00 |
| lnc-LPL-4:2 | SEQ6160 | 3.1E+01 | lnc-C1orf200-9:1 | SEQ6536 | 3.1E+00 |
| lnc-TBL1XR1-17:1 | SEQ2374 | 3.1E+01 | lnc-HEXA-3:1 | SEQ6537 | 3.1E+00 |
| lnc-POM121L2-2:1 | SEQ2133 | 2.8E+01 | lnc-PKHD1L1-2:7 | SEQ6538 | 3.1E+00 |
| lnc-MBOAT2-5:1 | SEQ6161 | 2.8E+01 | lnc-GALNT5-4:1 | SEQ6539 | 3.0E+00 |
| lnc-CDC27-7:1 | SEQ6162 | 2.7E+01 | lnc-TOB1-4:1 | SEQ5647 | 3.0E+00 |
| lnc-RP11-345P4.5.1-1:1 | SEQ6163 | 2.7E+01 | lnc-ZNF238-7:1 | SEQ6540 | 3.0E+00 |
| lnc-PNPLA8-2:8 | SEQ6164 | 2.5E+01 | lnc-KCNMB4-4:3 | SEQ6541 | 3.0E+00 |
| lnc-CYP20A1-1:3 | SEQ6165 | 2.5E+01 | lnc-BRWD1-1:1 | SEQ6542 | 3.0E+00 |
| lnc-CTD-2117L12.1.1-4:4 | SEQ6166 | 2.5E+01 | lnc-DYNLL1-1:1 | SEQ6543 | 3.0E+00 |
| lnc-KNG1-2:3 | SEQ6167 | 2.4E+01 | lnc-TMF1-2:1 | SEQ6544 | 3.0E+00 |
| lnc-SNAP25-3:1 | SEQ6168 | 2.4E+01 | lnc-HSPBP1-1:1 | SEQ6545 | 3.0E+00 |
| UBA6-AS1:10 | SEQ6169 | 2.3E+01 | TTC28-AS1:33 | SEQ6546 | 3.0E+00 |
| EIF3J-AS1:2 | SEQ6170 | 2.3E+01 | lnc-SLC8A1-1:1 | SEQ6547 | 3.0E+00 |
| SLC25A25-AS1:16 | SEQ6171 | 2.2E+01 | lnc-HMGA1-2:4 | SEQ3415 | 3.0E+00 |
| lnc-UQCRH-1:3 | SEQ6172 | 2.1E+01 | lnc-TH1L-5:6 | SEQ6548 | 3.0E+00 |
| lnc-DLX1-2:5 | SEQ6173 | 2.0E+01 | lnc-RGS9-1:5 | SEQ6549 | 3.0E+00 |
| SUCLG2-AS1:5 | SEQ6174 | 2.0E+01 | lnc-SERINC1-3:1 | SEQ5720 | 3.0E+00 |
| lnc-ARSG-1:1 | SEQ6175 | 1.8E+01 | lnc-SMARCA5-6:4 | SEQ6550 | 3.0E+00 |
| lnc-COX17-2:2 | SEQ6176 | 1.8E+01 | lnc-RGS9-1:1 | SEQ6551 | 3.0E+00 |
| lnc-TRIM26-2:8 | SEQ6177 | 1.8E+01 | LINC01420:2 | SEQ6552 | 3.0E+00 |
| lnc-RAB27A-1:4 | SEQ6178 | 1.8E+01 | lnc-AC007401.2.1-2:1 | SEQ6553 | 3.0E+00 |
| TP73-AS1:23 | SEQ6179 | 1.8E+01 | lnc-BX255923.1-8:3 | SEQ6554 | 3.0E+00 |
| lnc-VPREB1-7:6 | SEQ6180 | 1.7E+01 | NR2F1-AS1:15 | SEQ6555 | 3.0E+00 |
| RNF219-AS1:4 | SEQ6181 | 1.7E+01 | lnc-AC138969.4.1-1:1 | SEQ6556 | 3.0E+00 |
| lnc-BRAF-2:1 | SEQ6182 | 1.7E+01 | lnc-CYP26C1-1:1 | SEQ6557 | 3.0E+00 |
| lnc-TPTE2-4:1 | SEQ2431 | 1.5E+01 | lnc-TCEA1-3:1 | SEQ6558 | 3.0E+00 |
| MYLK-AS1:1 | SEQ6183 | 1.5E+01 | lnc-CSNK1D-2:6 | SEQ6559 | 2.9 E+00 |
| lnc-UQCRH-1:1 | SEQ6184 | 1.5E+01 | lnc-DNAL1-1:1 | SEQ2480 | 2.9 E+00 |
| lnc-GPR39-9:1 | SEQ2704 | 1.4E+01 | lnc-MLNR-2:2 | SEQ6560 | 2.9 E+00 |
| lnc-TRIM26-2:24 | SEQ6185 | 1.4E+01 | lnc-THEMIS-6:1 | SEQ6561 | 2.9 E+00 |
| lnc-EPHA4-4:1 | SEQ6186 | 1.4E+01 | lnc-DAPL1-3:1 | SEQ6562 | 2.9 E+00 |
| lnc-LSM5-2:1 | SEQ6187 | 1.4E+01 | lnc-ARL15-7:1 | SEQ6563 | 2.9 E+00 |
| lnc-MLST8-2:6 | SEQ6188 | 1.4E+01 | lnc-CDKL2-2:1 | SEQ6564 | 2.9 E+00 |
| NCBP2-AS2:1 | SEQ6189 | 1.3E+01 | lnc-UBLCP1-3:3 | SEQ6565 | 2.9 E+00 |
| LlMD1-AS1:3 | SEQ6190 | 1.3E+01 | lnc-VEZF1-1:6 | SEQ6566 | 2.9 E+00 |
| PINK1-AS:1 | SEQ2712 | 1.3E+01 | SNHG4:19 | SEQ6567 | 2.9 E+00 |
| lnc-BRWD1-2:2 | SEQ6191 | 1.3E+01 | lnc-TANK-3:1 | SEQ6568 | 2.9 E+00 |
| lnc-FAM200B-1:5 | SEQ6192 | 1.3E+01 | ATP6VOE2-AS1:1 | SEQ4643 | 2.9 E+00 |
| lnc-ARL6IP4-1:10 | SEQ6193 | 1.3E+01 | lnc-SP3-10:1 | SEQ6569 | 2.9 E+00 |
| lnc-TMEM189-UBE2V1-4:5 | SEQ6194 | 1.3E+01 | lnc-SNURF-1:8 | SEQ6570 | 2.9 E+00 |
| lnc-ARL16-2:2 | SEQ6195 | 1.3E+01 | lnc-AZIN1-1:5 | SEQ6571 | 2.9 E+00 |
| lnc-NAGA-2:1 | SEQ6196 | 1.3E+01 | lnc-CHMP1A-4:6 | SEQ6572 | 2.9 E+00 |
| lnc-LPCAT1-3:23 | SEQ6197 | 1.3E+01 | GAS5:40 | SEQ6573 | 2.9 E+00 |
| lnc-NEDD8-MDP1-1:1 | SEQ6198 | 1.2E+01 | lnc-BCO2-2:3 | SEQ6574 | 2.9 E+00 |
| LIFR-AS1:3 | SEQ6199 | 1.2E+01 | lnc-POC1B-GALNT4-3:1 | SEQ6575 | 2.9 E+00 |
| lnc-NEK3-1:8 | SEQ6200 | 1.2E+01 | lnc-TPD52-3:12 | SEQ6576 | 2.9 E+00 |
| lnc-KCNE4-7:1 | SEQ2466 | 1.2E+01 | lnc-FAM27B-12:3 | SEQ6577 | 2.9 E+00 |
| lnc-FAM200B-1:11 | SEQ6201 | 1.2E+01 | lnc-STARD9-1:1 | SEQ6578 | 2.9 E+00 |
| Inc-CDC42SE2-1:10 | SEQ2613 | 1.2E+01 | lnc-IKBIP-1:1 | SEQ4407 | 2.9 E+00 |
| lnc-TM9SF2-8:1 | SEQ6202 | 1.2E+01 | lnc-MBOAT4-2:1 | SEQ2592 | 2.9 E+00 |
| MIR4458HG:6 | SEQ6203 | 1.2E+01 | lnc-ATP6V1E1-2:2 | SEQ6579 | 2.9 E+00 |
| lnc-DCTN3-1:3 | SEQ6204 | 1.1E+01 | lnc-SPINK9-2:2 | SEQ6580 | 2.9 E+00 |
| lnc-PAPLN-3:1 | SEQ6205 | 1.1E+01 | lnc-SHC4-3:1 | SEQ6581 | 2.9 E+00 |
| lnc-RP11-1035H133.1-1:3 | SEQ6206 | 1.1E+01 | lnc-GATSL1-4:4 | SEQ6582 | 2.8E+00 |
| lnc-NDUFS1-2:2 | SEQ6207 | 1.1E+01 | lnc-DNAJC16-1:2 | SEQ4625 | 2.8E+00 |
| LINC00630:8 | SEQ6208 | 1.1E+01 | lnc-SLC39A6-3:1 | SEQ6583 | 2.8E+00 |
| lnc-ZNF449-3:5 | SEQ2420 | 1.1E+01 | lnc-POU5F1-10:2 | SEQ6584 | 2.8E+00 |
| lnc-ZNF449-3:4 | SEQ2421 | 1.1E+01 | lnc-NMD3-1:1 | SEQ2364 | 2.8E+00 |
| PEG3-AS1:1 | SEQ2398 | 1.1E+01 | lnc-FGD4-5:1 | SEQ6585 | 2.8E+00 |
| lnc-XRRA1-1:3 | SEQ6209 | 1.0E+01 | lnc-RAD54B-2:3 | SEQ6586 | 2.8E+00 |
| lnc-USP13-5:1 | SEQ6210 | 1.0E+01 | LINC01355:4 | SEQ6587 | 2.8E+00 |
| lnc-FAM104A-2:1 | SEQ2542 | 1.0E+01 | lnc-IMPDH1-3:3 | SEQ6588 | 2.8E+00 |
| lnc-CPO-2:1 | SEQ6211 | 1.0E+01 | lnc-TMEM88B-3:8 | SEQ6589 | 2.8E+00 |
| lnc-KBTBD6-2:1 | SEQ2349 | 1.0E+01 | EIF3J-AS1:4 | SEQ6590 | 2.8E+00 |
| lnc-GPC2-2:7 | SEQ6212 | 9.7 E+00 | lnc-PRR11-3:1 | SEQ6591 | 2.8E+00 |
| lnc-POLK-3:1 | SEQ6213 | 9.6E+00 | lnc-ZNF880-1:1 | SEQ6592 | 2.8E+00 |
| lnc-INO80B-1:2 | SEQ6214 | 9.6E+00 | lnc-SLC4A5-3:1 | SEQ6593 | 2.8E+00 |
| lnc-ZSCAN10-3:1 | SEQ6215 | 9.4E+00 | NR2F1-AS1:27 | SEQ6594 | 2.8E+00 |
| lnc-RASA1-13:5 | SEQ6216 | 9.4E+00 | lnc-STARD4-4:2 | SEQ6595 | 2.8E+00 |
| lnc-PRMT5-1:2 | SEQ6217 | 9.4E+00 | lnc-GUSB-15:1 | SEQ6596 | 2.8E+00 |
| lnc-CPOX-1:1 | SEQ6218 | 9.4E+00 | SNHG21:15 | SEQ6597 | 2.8E+00 |
| lnc-VEPH1-2:1 | SEQ6219 | 9.3 E+00 | lnc-CSNK2A2-2:1 | SEQ6598 | 2.8E+00 |
| lnc-RPAIN-2:1 | SEQ6220 | 9.3 E+00 | lnc-PON3-2:1 | SEQ6599 | 2.8E+00 |
| lnc-SNX20-5:3 | SEQ2643 | 9.3 E+00 | lnc-C14orf132-1:5 | SEQ6600 | 2.8E+00 |
| RNF219-AS1:16 | SEQ4990 | 9.2 E+00 | lnc-RNASEK-1:4 | SEQ6601 | 2.7 E+00 |
| lnc-C9orf123-8:2 | SEQ6221 | 9.1E+00 | lnc-NR2C2-1:1 | SEQ2751 | 2.7E+00 |
| lnc-SLC5A2-1:4 | SEQ6222 | 9.1E+00 | lnc-PNP-1:1 | SEQ6602 | 2.7 E+00 |
| lnc-GDPD4-7:1 | SEQ6223 | 9.0E+00 | lnc-CWH43-3:4 | SEQ6603 | 2.7 E+00 |
| lnc-PRKD2-2:1 | SEQ6224 | 9.0E+00 | lnc-CLSTN2-1:1 | SEQ6604 | 2.7 E+00 |
| lnc-C18orf54-3:4 | SEQ6225 | 9.0E+00 | lnc-SLC9A5-2:1 | SEQ6605 | 2.7E+00 |
| lnc-AGPHD1-1:6 | SEQ6226 | 9.0E+00 | lnc-C1orf185-5:1 | SEQ6606 | 2.7 E+00 |
| lnc-NPR3-3:5 | SEQ6227 | 8.9E+00 | lnc-TMEM167A-4:5 | SEQ6607 | 2.7 E+00 |
| lnc-CBWD5-2:8 | SEQ6228 | 8.9E+00 | lnc-TSC22D2-1:2 | SEQ6608 | 2.7 E+00 |
| lnc-NUDT3-1:2 | SEQ6229 | 8.8E+00 | lnc-TULP4-1:1 | SEQ6609 | 2.7 E+00 |
| lnc-VSTM5-1:13 | SEQ2419 | 8.7E+00 | lnc-SYNE2-4:1 | SEQ2308 | 2.7 E+00 |
| LINC00630:12 | SEQ6230 | 8.7E+00 | lnc-RPRML-3:20 | SEQ6610 | 2.7 E+00 |
| lnc-SLMO2-3:1 | SEQ6231 | 8.7E+00 | PSMD5-AS1:5 | SEQ6611 | 2.7E+00 |
| lnc-DPH5-2:7 | SEQ6232 | 8.6E+00 | lnc-R3HDM1-1:2 | SEQ6612 | 2.7E+00 |
| lnc-SCN4B-2:3 | SEQ6233 | 8.5E+00 | PCBP1-AS1:183 | SEQ6613 | 2.7E+00 |
| lnc-NDUFA6-1:1 | SEQ6234 | 8.5E+00 | lnc-PHLDB2-2:3 | SEQ6614 | 2.7 E+00 |
| RAB11B-AS1:1 | SEQ6235 | 8.5E+00 | LINC00339:21 | SEQ6615 | 2.7E+00 |
| lnc-LRRC37A2-2:3 | SEQ6236 | 8.4E+00 | lnc-POLR1B-2:1 | SEQ6616 | 2.7E+00 |
| lnc-PAQR7-1:1 | SEQ6237 | 8.3E+00 | lnc-MOCOS-3:10 | SEQ6617 | 2.7 E+00 |
| lnc-POLR2I-1:2 | SEQ6238 | 8.3E+00 | lnc-CSTF3-2:1 | SEQ6618 | 2.7E+00 |
| lnc-H2AFZ-1:4 | SEQ6239 | 8.2E+00 | lnc-SEPT5-1:2 | SEQ6619 | 2.7 E+00 |
| SVIL-AS1:4 | SEQ6240 | 8.1E+00 | NFYC-AS1:2 | SEQ6620 | 2.7 E+00 |
| lnc-DCUN1D3-1:5 | SEQ6241 | 8.1E+00 | lnc-PABPC1L-2:1 | SEQ6621 | 2.7 E+00 |
| lnc-PRR4-1:2 | SEQ6242 | 8.0E+00 | lnc-DUS4L-2:4 | SEQ6622 | 2.7 E+00 |
| MAGI2-AS3:13 | SEQ6243 | 8.0E+00 | CKMT2-AS1:6 | SEQ6623 | 2.7 E+00 |
| lnc-FANCM-8:3 | SEQ2291 | 7.9 E+00 | MIF-AS1:7 | SEQ3858 | 2.7E+00 |
| lnc-CRLF1-2:1 | SEQ6244 | 7.8E+00 | lnc-TBR1-1:1 | SEQ6624 | 2.7 E+00 |
| lnc-PPP4C-1:1 | SEQ6245 | 7.7 E+00 | lnc-NFAT5-2:1 | SEQ6625 | 2.7 E+00 |
| lnc-RNF187-2:2 | SEQ6246 | 7.6E+00 | lnc-NLRP3-1:5 | SEQ6626 | 2.7E+00 |
| lnc-ATAT1-4:1 | SEQ6247 | 7.5 E+00 | lnc-SLC25A39-2:1 | SEQ5393 | 2.7E+00 |
| lnc-AP2M1-2:1 | SEQ6248 | 7.5 E+00 | lnc-PPHLN1-1:1 | SEQ6627 | 2.7E+00 |
| lnc-MARCH7-1:3 | SEQ6249 | 7.5 E+00 | lnc-CTC-554D6.1.1-2:1 | SEQ6628 | 2.7E+00 |
| lnc-ZNF518B-2:4 | SEQ5239 | 7.5 E+00 | lnc-DDIT3-2:1 | SEQ6629 | 2.7E+00 |
| lnc-OTP-4:1 | SEQ6250 | 7.4E+00 | lnc-ABCD2-4:1 | SEQ2216 | 2.6E+00 |
| EPB41L4A-AS1:4 | SEQ6251 | 7.4E+00 | lnc-BMF-3:4 | SEQ6630 | 2.6E+00 |
| lnc-ATAD5-3:22 | SEQ6252 | 7.4E+00 | lnc-CXXC11-8:6 | SEQ6631 | 2.6E+00 |
| NDUFA6-AS1:4 | SEQ6253 | 7.4E+00 | lnc-PSMC1-2:2 | SEQ6632 | 2.6E+00 |
| lnc-RGPD1-6:1 | SEQ6254 | 7.4E+00 | lnc-AC008394.1-7:1 | SEQ6633 | 2.6E+00 |
| lnc-DLG2-3:2 | SEQ6255 | 7.3 E+00 | lnc-TNFRSF14-5:3 | SEQ6634 | 2.6E+00 |
| lnc-PSMA3-1:5 | SEQ6256 | 7.3 E+00 | lnc-FER-8:1 | SEQ6635 | 2.6E+00 |
| lnc-GRIN2A-1:2 | SEQ6257 | 7.0E+00 | lnc-ELN-2:2 | SEQ6636 | 2.6E+00 |
| lnc-KNG1-2:6 | SEQ6258 | 7.0E+00 | lnc-PSMA7-1:1 | SEQ5951 | 2.6E+00 |
| lnc-AMN1-1:2 | SEQ6259 | 7.0E+00 | lnc-TTYH2-1:4 | SEQ6637 | 2.6E+00 |
| lnc-MTMR4-2:1 | SEQ6260 | 7.0E+00 | lnc-RPL36A-1:4 | SEQ6638 | 2.6E+00 |
| lnc-PRTFDC1-3:1 | SEQ6261 | 6.9E+00 | lnc-WSB1-5:1 | SEQ6639 | 2.6E+00 |
| lnc-PRKAR1A-2:1 | SEQ6262 | 6.9E+00 | lnc-SLC25A38-2:1 | SEQ6640 | 2.6E+00 |
| lnc-ANKRD56-5:1 | SEQ6263 | 6.9E+00 | lnc-LEPREL4-1:3 | SEQ6641 | 2.6E+00 |
| lnc-GJC2-2:2 | SEQ6264 | 6.9E+00 | lnc-B4GALT2-1:2 | SEQ6642 | 2.6E+00 |
| lnc-CDKL3-3:1 | SEQ6265 | 6.9E+00 | lnc-AL049840.1-3:1 | SEQ6643 | 2.6E+00 |
| MYCBP2-AS1:10 | SEQ6266 | 6.8E+00 | lnc-SUMF2-10:1 | SEQ6644 | 2.6E+00 |
| lnc-POM121L2-2:8 | SEQ6267 | 6.8E+00 | lnc-EIF1AD-1:1 | SEQ2653 | 2.6E+00 |
| lnc-IQUB-2:6 | SEQ6268 | 6.7E+00 | lnc-NUDT16L1-2:1 | SEQ6645 | 2.6E+00 |
| lnc-ARMC9-2:2 | SEQ6269 | 6.7E+00 | lnc-TMEM180-2:1 | SEQ6646 | 2.6E+00 |
| lnc-DBF4-3:1 | SEQ6270 | 6.7E+00 | lnc-PPIL2-1:2 | SEQ6647 | 2.6E+00 |
| lnc-SOCS6-10:1 | SEQ2214 | 6.6E+00 | lnc-ERAS-4:1 | SEQ6648 | 2.6E+00 |
| lnc-AF131216.6.1-2:2 | SEQ6271 | 6.5E+00 | lnc-USP34-1:10 | SEQ3127 | 2.6E+00 |
| lnc-CLEC2D-8:7 | SEQ3369 | 6.5E+00 | lnc-EIF4G1-1:1 | SEQ6649 | 2.6E+00 |
| lnc-UROS-2:4 | SEQ6272 | 6.5E+00 | lnc-GPRIN2-2:1 | SEQ2453 | 2.6E+00 |
| lnc-EDC4-1:2 | SEQ6273 | 6.5E+00 | DICER1-AS1:9 | SEQ6650 | 2.6E+00 |
| lnc-ESCO2-1:2 | SEQ6274 | 6.5E+00 | lnc-C10orf40-5:1 | SEQ6651 | 2.6E+00 |
| OTUD6B-AS1:3 | SEQ6275 | 6.4E+00 | lnc-BCAT1-2:1 | SEQ6652 | 2.6E+00 |
| lnc-SREBF1-1:1 | SEQ6276 | 6.4E+00 | lnc-RP11-96O20.4.1-1:4 | SEQ6653 | 2.5 E+00 |
| lnc-DLG5-4:1 | SEQ6277 | 6.4E+00 | lnc-RP11-1012A1.4.1-1:5 | SEQ6654 | 2.5 E+00 |
| lnc-GNGT1-4:1 | SEQ6278 | 6.4E+00 | lnc-RP11-632K20.1.1-1:1 | SEQ6655 | 2.5 E+00 |
| lnc-PRDX2-1:1 | SEQ6279 | 6.4E+00 | lnc-BDH2-2:1 | SEQ6656 | 2.5 E+00 |
| lnc-MBOAT4-5:1 | SEQ6280 | 6.3E+00 | lnc-CDO1-1:7 | SEQ6657 | 2.5 E+00 |
| lnc-RBM25-5:2 | SEQ6281 | 6.2E+00 | lnc-GDI1-1:1 | SEQ4743 | 2.5 E+00 |
| LINC-PINT:9 | SEQ6282 | 6.2E+00 | lnc-SSBP2-5:9 | SEQ6658 | 2.5 E+00 |
| lnc-RP11-478C19.2.1-2:7 | SEQ6283 | 6.2E+00 | CERS6-AS1:3 | SEQ6659 | 2.5 E+00 |
| lnc-APOPT1-1:1 | SEQ6284 | 6.1E+00 | lnc-ABCA3-2:1 | SEQ6660 | 2.5 E+00 |
| lnc-ZBTB17-4:1 | SEQ6285 | 6.1E+00 | SNHG1:25 | SEQ6661 | 2.5 E+00 |
| lnc-PRKAB2-5:1 | SEQ6286 | 6.1E+00 | lnc-MLLT6-1:1 | SEQ6662 | 2.5 E+00 |
| lnc-TMEM154-4:2 | SEQ6287 | 6.1E+00 | lnc-OTOA-2:1 | SEQ6663 | 2.5 E+00 |
| GLIDR:2 | SEQ6288 | 6.1E+00 | lnc-CDK2AP1-1:1 | SEQ6664 | 2.5 E+00 |
| lnc-ZNF664-2:16 | SEQ6289 | 6.0E+00 | lnc-CDK2AP1-1:7 | SEQ6665 | 2.5 E+00 |
| lnc-MLL3-1:2 | SEQ6290 | 6.0E+00 | LINC00963:8 | SEQ6666 | 2.5 E+00 |
| lnc-MRPL48-4:1 | SEQ6291 | 6.0E+00 | lnc-C3orf25-2:7 | SEQ6667 | 2.5 E+00 |
| lnc-TMEM232-7:1 | SEQ6292 | 6.0E+00 | LINC00894:9 | SEQ6668 | 2.5 E+00 |
| lnc-BRCC3-4:1 | SEQ6293 | 6.0E+00 | lnc-CCDC68-4:1 | SEQ6669 | 2.5 E+00 |
| lnc-PKD2L1-6:1 | SEQ6294 | 5.9 E+00 | lnc-S100A13-2:7 | SEQ6670 | 2.5 E+00 |
| UBA6-AS1:2 | SEQ6295 | 5.9 E+00 | lnc-EFHB-5:1 | SEQ6671 | 2.5 E+00 |
| lnc-SESN3-2:2 | SEQ6296 | 5.9 E+00 | lnc-AC073263.1-3:1 | SEQ6672 | 2.5 E+00 |
| lnc-SGIP1-3:1 | SEQ6297 | 5.9 E+00 | LINC00623:8 | SEQ6673 | 2.5 E+00 |
| SNHG3:2 | SEQ6298 | 5.8E+00 | lnc-ZNF33B-4:11 | SEQ6674 | 2.5 E+00 |
| lnc-GABBR1-1:1 | SEQ6036 | 5.7E+00 | LINC01278:2 | SEQ6675 | 2.5 E+00 |
| lnc-EXD1-1:1 | SEQ6299 | 5.7E+00 | lnc-PLAT-4:1 | SEQ6676 | 2.5 E+00 |
| lnc-VPREB1-7:1 | SEQ6300 | 5.7 E+00 | lnc-STXBP3-1:2 | SEQ6677 | 2.5 E+00 |
| lnc-PPFIA4-1:1 | SEQ2325 | 5.7E+00 | CERS6-AS1:4 | SEQ6678 | 2.5 E+00 |
| lnc-C2orf63-3:1 | SEQ6301 | 5.7 E+00 | CERS6-AS1:5 | SEQ6679 | 2.5 E+00 |
| TP73-AS1:21 | SEQ6302 | 5.7 E+00 | TMEM254-AS1:1 | SEQ6680 | 2.5 E+00 |
| lnc-FAM200B-1:3 | SEQ6303 | 5.6E+00 | lnc-EPB41L3-3:1 | SEQ6681 | 2.5 E+00 |
| lnc-FAM210A-1:1 | SEQ6304 | 5.6E+00 | lnc-XRCC5-1:3 | SEQ6682 | 2.5 E+00 |
| lnc-FAM153A-3:4 | SEQ6305 | 5.6E+00 | lnc-CCAR1-4:1 | SEQ6683 | 2.5 E+00 |
| lnc-PCGF5-5:4 | SEQ6306 | 5.6E+00 | lnc-SNX2-1:2 | SEQ4824 | 2.5 E+00 |
| BCDIN3D-AS1:1 | SEQ6307 | 5.5 E+00 | lnc-GALNTL1-5:5 | SEQ6684 | 2.5 E+00 |
| lnc-KIAA1704-3:1 | SEQ6308 | 5.5 E+00 | lnc-FAM200B-1:16 | SEQ6685 | 2.4E+00 |
| lnc-C5orf13-1:3 | SEQ6309 | 5.5 E+00 | EAF1-AS1:14 | SEQ6686 | 2.4E+00 |
| lnc-RGR-3:4 | SEQ6310 | 5.5 E+00 | lnc-SLC16A9-6:2 | SEQ6687 | 2.4E+00 |
| lnc-RPGRIP1L-1:2 | SEQ6311 | 5.5 E+00 | lnc-GNA13-2:9 | SEQ6688 | 2.4E+00 |
| lnc-FAM120AOS-3:6 | SEQ6312 | 5.5 E+00 | lnc-GALT-1:9 | SEQ6689 | 2.4E+00 |
| lnc-DYNC1LI2-2:2 | SEQ6313 | 5.5 E+00 | lnc-ATP6V0A1-1:2 | SEQ6690 | 2.4E+00 |
| lnc-GPS2-1:1 | SEQ6314 | 5.5 E+00 | lnc-CCNB1IP1-1:1 | SEQ6691 | 2.4E+00 |
| PRKCQ-AS1:11 | SEQ4800 | 5.4E+00 | lnc-C11orf58-2:3 | SEQ6692 | 2.4E+00 |
| lnc-ZAR1L-2:1 | SEQ6315 | 5.4E+00 | lnc-ST7L-3:10 | SEQ6693 | 2.4E+00 |
| lnc-TRIOBP-1:3 | SEQ6316 | 5.4E+00 | lnc-DHX33-2:1 | SEQ6694 | 2.4E+00 |
| lnc-RNPEP-3:1 | SEQ6317 | 5.4E+00 | lnc-AC011551.1-1:1 | SEQ6695 | 2.4E+00 |
| lnc-FOXD4L6-2:6 | SEQ6318 | 5.4E+00 | lnc-SP9-4:2 | SEQ6696 | 2.4E+00 |
| lnc-FAM150B-5:3 | SEQ6319 | 5.4E+00 | lnc-C11orf31-1:1 | SEQ6697 | 2.4E+00 |
| LINC00662:21 | SEQ6320 | 5.4E+00 | lnc-AL669831.1-11:3 | SEQ6698 | 2.4E+00 |
| lnc-PNLIPRP1-1:2 | SEQ2540 | 5.3 E+00 | lnc-NINL-2:1 | SEQ6699 | 2.4E+00 |
| CARS-AS1:2 | SEQ6321 | 5.3 E+00 | OIP5-AS1:25 | SEQ5168 | 2.4E+00 |
| lnc-FBXO3-3:2 | SEQ6322 | 5.2 E+00 | lnc-KIAA1383-7:2 | SEQ6700 | 2.4E+00 |
| lnc-XRCC5-3:1 | SEQ2326 | 5.2E+00 | lnc-APBA1-1:2 | SEQ6701 | 2.4E+00 |
| lnc-AIPL1-6:1 | SEQ6323 | 5.2 E+00 | NIPBL-AS1:3 | SEQ6702 | 2.4E+00 |
| lnc-AF131215.2.1-1:3 | SEQ6324 | 5.1E+00 | lnc-MRPL30-2:4 | SEQ6703 | 2.4E+00 |
| lnc-MC5R-6:1 | SEQ6325 | 5.1E+00 | lnc-RWDD2B-4:2 | SEQ6704 | 2.4E+00 |
| lnc-DNAJC17-1:1 | SEQ6326 | 5.1E+00 | lnc-RASL11B-9:1 | SEQ6705 | 2.4E+00 |
| HEIH:11 | SEQ6327 | 5.1E+00 | lnc-TBC1D5-2:1 | SEQ6706 | 2.4E+00 |
| MIR100HG:17 | SEQ6328 | 5.1E+00 | lnc-CEP152-1:1 | SEQ6707 | 2.4E+00 |
| lnc-TMEM232-4:6 | SEQ6329 | 5.1E+00 | lnc-CCDC144NL-7:1 | SEQ6708 | 2.4E+00 |
| lnc-TMEM99-5:3 | SEQ6330 | 5.0E+00 | lnc-PLCE1-3:1 | SEQ6709 | 2.4E+00 |
| lnc-AC005609.1-3:7 | SEQ6331 | 5.0E+00 | lnc-CENPV-2:1 | SEQ2516 | 2.4E+00 |
| lnc-SPAG16-5:1 | SEQ6332 | 5.0E+00 | lnc-ZNF397-4:1 | SEQ4775 | 2.4E+00 |
| lnc-CYP19A1-2:1 | SEQ6333 | 4.9E+00 | lnc-WDR41-1:1 | SEQ6710 | 2.4E+00 |
| lnc-TYSND1-1:6 | SEQ6334 | 4.9E+00 | lnc-AC112715.2.1-7:1 | SEQ6711 | 2.4E+00 |
| lnc-CDT1-1:1 | SEQ2708 | 4.9E+00 | lnc-PHAX-2:1 | SEQ6712 | 2.4E+00 |
| lnc-C16orf61-3:7 | SEQ6335 | 4.9E+00 | lnc-BMI1-3:3 | SEQ6713 | 2.4E+00 |
| lnc-IFI27L2-3:2 | SEQ6336 | 4.9E+00 | lnc-NAV3-5:5 | SEQ6714 | 2.4E+00 |
| lnc-CLDN25-5:1 | SEQ6337 | 4.9E+00 | lnc-FAM156A-2:1 | SEQ3387 | 2.4E+00 |
| lnc-UNC80-1:1 | SEQ2219 | 4.9E+00 | lnc-CAND1-3:2 | SEQ6715 | 2.4E+00 |
| lnc-PRKAA1-2:1 | SEQ6338 | 4.8E+00 | lnc-DAPL1-6:2 | SEQ6716 | 2.4E+00 |
| lnc-RPRML-3:24 | SEQ6339 | 4.8E+00 | lnc-ENAH-2:3 | SEQ6717 | 2.4E+00 |
| SNHG16:11 | SEQ6340 | 4.8E+00 | lnc-PLSCR3-1:1 | SEQ6718 | 2.4E+00 |
| lnc-MRFAP1-2:1 | SEQ6341 | 4.8E+00 | lnc-HMBS-1:4 | SEQ6719 | 2.4E+00 |
| lnc-CERK-2:1 | SEQ6342 | 4.8E+00 | lnc-RAPSN-1:1 | SEQ6720 | 2.4E+00 |
| lnc-RAB11A-2:1 | SEQ6343 | 4.8E+00 | lnc-MATR3-3:2 | SEQ6721 | 2.4E+00 |
| lnc-NETO2-1:1 | SEQ6344 | 4.8E+00 | lnc-GLB1L-1:1 | SEQ6722 | 2.4E+00 |
| lnc-LYN-9:4 | SEQ6345 | 4.8E+00 | lnc-CLEC18B-7:5 | SEQ2353 | 2.4E+00 |
| lnc-GATC.1-2:1 | SEQ6346 | 4.8E+00 | LINC-PINT:2 | SEQ6723 | 2.4E+00 |
| lnc-PTMS-1:3 | SEQ6347 | 4.7E+00 | lnc-MRPS36-2:1 | SEQ6724 | 2.4E+00 |
| lnc-SOD1-4:4 | SEQ6348 | 4.7E+00 | lnc-RNF187-2:1 | SEQ6725 | 2.3E+00 |
| lnc-HMGA1-2:9 | SEQ6349 | 4.7E+00 | lnc-PEX19-4:1 | SEQ6726 | 2.3E+00 |
| lnc-ZNF518B-2:3 | SEQ6350 | 4.7E+00 | lnc-SNURF-1:44 | SEQ6727 | 2.3E+00 |
| lnc-RAP1GAP2-5:1 | SEQ6351 | 4.7E+00 | lnc-RNF43-2:2 | SEQ6728 | 2.3E+00 |
| lnc-CCNB2-2:1 | SEQ6352 | 4.6E+00 | lnc-AF131216.6.1-2:1 | SEQ6729 | 2.3E+00 |
| lnc-LIN7A-3:1 | SEQ3437 | 4.6E+00 | lnc-SCFD2-4:1 | SEQ6730 | 2.3E+00 |
| lnc-UBE2B-2:1 | SEQ2253 | 4.6E+00 | CD27-AS1:4 | SEQ6731 | 2.3E+00 |
| lnc-MBP-13:10 | SEQ6353 | 4.6E+00 | lnc-ENSA-1:1 | SEQ6732 | 2.3E+00 |
| lnc-RAP1GAP2-6:2 | SEQ6354 | 4.6E+00 | lnc-RP11-439E19.8.1-2:1 | SEQ6733 | 2.3E+00 |
| lnc-NEDD8-MDP1-1:3 | SEQ6355 | 4.6E+00 | lnc-OGFRL1-4:1 | SEQ6734 | 2.3E+00 |
| lnc-MFSD4-3:3 | SEQ6356 | 4.6E+00 | lnc-C12orf10-1:3 | SEQ6735 | 2.3E+00 |
| lnc-PKIA-4:1 | SEQ6357 | 4.5E+00 | lnc-HIBADH-5:1 | SEQ3962 | 2.3E+00 |
| lnc-NRBP2-2:1 | SEQ6358 | 4.5E+00 | CRHR1-IT1:8 | SEQ6736 | 2.3 E+00 |
| TP53TG1:8 | SEQ6359 | 4.5E+00 | lnc-BRAF-3:2 | SEQ6737 | 2.3 E+00 |
| BCDIN3D-AS1:6 | SEQ6360 | 4.5E+00 | lnc-SIRT2-1:2 | SEQ6738 | 2.3 E+00 |
| BCDIN3D-AS1:2 | SEQ6361 | 4.5E+00 | lnc-RAB27A-1:7 | SEQ6739 | 2.3E+00 |
| lnc-MMADHC-11:1 | SEQ6362 | 4.5E+00 | lnc-SNRNP35-1:2 | SEQ6740 | 2.3 E+00 |
| lnc-RNF123-1:1 | SEQ4013 | 4.5E+00 | MSC-AS1:2 | SEQ6741 | 2.3 E+00 |
| lnc-PRPF18-1:1 | SEQ6363 | 4.5E+00 | lnc-WSB2-1:1 | SEQ6742 | 2.3E+00 |
| lnc-ZNF91-4:19 | SEQ6364 | 4.5E+00 | lnc-ISCU-2:1 | SEQ6743 | 2.3E+00 |
| lnc-SATB1-8:6 | SEQ6365 | 4.4E+00 | lnc-KIAA0564-2:1 | SEQ6744 | 2.3 E+00 |
| lnc-FGD4-4:1 | SEQ6366 | 4.4E+00 | CRHR1-IT1:9 | SEQ6745 | 2.3 E+00 |
| lnc-PALLD-7:1 | SEQ6367 | 4.4E+00 | lnc-DRD5-23:2 | SEQ5314 | 2.3 E+00 |
| lnc-KIAA1841-3:1 | SEQ6368 | 4.4E+00 | lnc-CHST10-2:1 | SEQ6746 | 2.3E+00 |
| lnc-AARSD1-2:6 | SEQ6369 | 4.4E+00 | lnc-GJC2-2:9 | SEQ6747 | 2.3 E+00 |
| lnc-TRIM26-2:6 | SEQ6370 | 4.4E+00 | LINC01578:14 | SEQ6748 | 2.3 E+00 |
| OTUD6B-AS1:8 | SEQ6371 | 4.4E+00 | lnc-ERN1-3:1 | SEQ6749 | 2.3 E+00 |
| lnc-TRIM26-2:13 | SEQ6372 | 4.4E+00 | lnc-GRIA4-2:1 | SEQ6750 | 2.3 E+00 |
| lnc-RGPD3-5:2 | SEQ6373 | 4.4E+00 | lnc-GMNN-3:1 | SEQ6751 | 2.3 E+00 |
| lnc-ENSA-1:2 | SEQ6374 | 4.4E+00 | PRKCQ-AS1:9 | SEQ6752 | 2.3E+00 |
| LINC01278:3 | SEQ6375 | 4.4E+00 | lnc-VOPP1-5:1 | SEQ6753 | 2.3E+00 |
| lnc-SUGP1-1:1 | SEQ6376 | 4.3E+00 | lnc-FAM53B-1:1 | SEQ6754 | 2.3E+00 |
| lnc-ORMDL2-1:17 | SEQ6377 | 4.3E+00 | lnc-C12orf10-1:2 | SEQ6755 | 2.3 E+00 |
| lnc-ZNF397-7:1 | SEQ6378 | 4.3E+00 | lnc-ERICH1-19:8 | SEQ6756 | 2.3E+00 |
| lnc-TUBA1C-1:3 | SEQ6379 | 4.3E+00 | lnc-PALLD-8:1 | SEQ2633 | 2.3E+00 |
| lnc-RERE-4:2 | SEQ6380 | 4.3E+00 | lnc-POLD3-1:1 | SEQ6757 | 2.3 E+00 |
| lnc-PDE7A-3:1 | SEQ6381 | 4.3E+00 | lnc-ELAC1-1:1 | SEQ6758 | 2.3 E+00 |
| lnc-UROS-2:3 | SEQ6382 | 4.2E+00 | lnc-RAB4A-2:2 | SEQ6759 | 2.3E+00 |
| lnc-SRP19-1:4 | SEQ6383 | 4.2E+00 | lnc-VEZT-2:2 | SEQ6760 | 2.3 E+00 |
| ZNF571-AS1:12 | SEQ6384 | 4.2E+00 | lnc-C6orf228-4:1 | SEQ6761 | 2.3 E+00 |
| ZNF571-AS1:13 | SEQ6385 | 4.2E+00 | LINC00294:2 | SEQ6762 | 2.3E+00 |
| lnc-MOB4-1:1 | SEQ6386 | 4.2E+00 | lnc-LRRC8E-2:1 | SEQ6763 | 2.3E+00 |
| lnc-FMR1NB-4:3 | SEQ6387 | 4.2E+00 | RNF139-AS1:1 | SEQ6764 | 2.2 E+00 |
| lnc-NOL11-3:1 | SEQ6388 | 4.2E+00 | lnc-PRSS1-6:3 | SEQ6765 | 2.2E+00 |
| lnc-SMN1-1:2 | SEQ6389 | 4.2E+00 | lnc-SLC9A7-1:2 | SEQ6766 | 2.2 E+00 |
| lnc-POLR3GL-3:2 | SEQ6390 | 4.2E+00 | lnc-UBXN6-1:3 | SEQ6767 | 2.2 E+00 |
| lnc-PRPF39-2:1 | SEQ6391 | 4.2E+00 | lnc-ALG1-2:1 | SEQ6768 | 2.2E+00 |
| PAXIP1-AS1:7 | SEQ6392 | 4.2E+00 | lnc-AES-1:1 | SEQ6769 | 2.2E+00 |
| lnc-ACOT12-8:2 | SEQ6393 | 4.1E+00 | lnc-PLAGL2-4:1 | SEQ6770 | 2.2 E+00 |
| lnc-ZNF582-3:2 | SEQ6394 | 4.1E+00 | GMDS-AS1:44 | SEQ6771 | 2.2 E+00 |
| lnc-CPVL-2:1 | SEQ6395 | 4.1E+00 | lnc-PABPC4-1:3 | SEQ6772 | 2.2E+00 |
| lnc-TARSL2-2:1 | SEQ6396 | 4.1E+00 | LINC00294:1 | SEQ6773 | 2.2E+00 |
| lnc-THRAP3-1:1 | SEQ6397 | 4.1E+00 | USP46-AS1:4 | SEQ6774 | 2.2 E+00 |
| lnc-CCNB1-1:2 | SEQ6398 | 4.1E+00 | lnc-GLTPD1-1:1 | SEQ6775 | 2.2E+00 |
| lnc-TIRAP-2:3 | SEQ6399 | 4.1E+00 | lnc-HEBP2-1:1 | SEQ6776 | 2.2E+00 |
| lnc-ZNF583-4:3 | SEQ6400 | 4.1E+00 | lnc-VSTM5-1:12 | SEQ6777 | 2.2 E+00 |
| lnc-FAM200B-1:9 | SEQ6401 | 4.0E+00 | lnc-ZNF595-1:1 | SEQ6778 | 2.2E+00 |
| lnc-ANKUB1-2:2 | SEQ6402 | 4.0E+00 | lnc-URGCP-2:8 | SEQ5108 | 2.2E+00 |
| lnc-NKAIN3-3:4 | SEQ6403 | 4.0E+00 | lnc-SERGEF-3:2 | SEQ6779 | 2.2 E+00 |
| lnc-SNURF-1:10 | SEQ6404 | 4.0E+00 | lnc-APBB2-2:4 | SEQ6780 | 2.2 E+00 |
| MEG3:11 | SEQ6405 | 4.0E+00 | lnc-C19orf71-1:1 | SEQ6781 | 2.2E+00 |
| lnc-GSPT2-4:1 | SEQ6406 | 4.0E+00 | lnc-VEZF1-1:3 | SEQ6782 | 2.2 E+00 |
| PTOV1-AS1:4 | SEQ6407 | 3.9 E+00 | lnc-COMMD1-1:1 | SEQ2294 | 2.2E+00 |
| lnc-ZZZ3-1:1 | SEQ6408 | 3.9 E+00 | lnc-SECISBP2L-3:1 | SEQ6783 | 2.2E+00 |
| lnc-CHRNA5-4:2 | SEQ6409 | 3.9 E+00 | lnc-TAOK3-1:5 | SEQ6784 | 2.2 E+00 |
| LINC01024:6 | SEQ6410 | 3.9 E+00 | lnc-RWDD1-1:1 | SEQ6785 | 2.2 E+00 |
| PAXIP1-AS1:1 | SEQ6411 | 3.9 E+00 | lnc-TDRKH-1:1 | SEQ6786 | 2.2 E+00 |
| lnc-CTDSP2-2:11 | SEQ6412 | 3.9 E+00 | lnc-SNAPIN-2:2 | SEQ6787 | 2.2E+00 |
| lnc-ITGAL-2:2 | SEQ6413 | 3.9 E+00 | lnc-C1orf132-1:7 | SEQ6788 | 2.2E+00 |
| lnc-MACROD2-2:1 | SEQ6414 | 3.9 E+00 | lnc-ARVCF-4:8 | SEQ6789 | 2.2E+00 |
| lnc-RABGAP1L-1:1 | SEQ6415 | 3.9 E+00 | lnc-COLEC10-1:4 | SEQ6790 | 2.2 E+00 |
| lnc-CEP192-2:3 | SEQ6416 | 3.9 E+00 | lnc-CCNL2-3:1 | SEQ6791 | 2.2 E+00 |
| lnc-PRR18-1:1 | SEQ6417 | 3.9 E+00 | lnc-BCL7B-1:5 | SEQ6792 | 2.2E+00 |
| lnc-CTD-2228K2.5.1-1:2 | SEQ6418 | 3.9 E+00 | lnc-METTL2B-3:16 | SEQ6793 | 2.2E+00 |
| lnc-ARSG-1:2 | SEQ6419 | 3.8E+00 | lnc-FAM72B-12:1 | SEQ6794 | 2.2E+00 |
| SNHG8:11 | SEQ6420 | 3.8E+00 | lnc-SLC39A3-2:1 | SEQ6795 | 2.2 E+00 |
| lnc-AL592284.1-1:19 | SEQ6421 | 3.8E+00 | lnc-MSH3-2:1 | SEQ6796 | 2.2E+00 |
| lnc-C1QL3-1:1 | SEQ6422 | 3.8E+00 | lnc-OTP-4:2 | SEQ6797 | 2.2 E+00 |
| lnc-HELLS-2:1 | SEQ6423 | 3.8E+00 | lnc-SNURF-9:1 | SEQ6798 | 2.2E+00 |
| lnc-STXBP4-1:1 | SEQ6424 | 3.8E+00 | lnc-DCHS2-1:1 | SEQ6799 | 2.1E+00 |
| lnc-DOM3Z-3:6 | SEQ6425 | 3.8E+00 | EIF3J-AS1:3 | SEQ6800 | 2.1E+00 |
| lnc-EML6-5:3 | SEQ6426 | 3.8E+00 | lnc-VPREB1-7:14 | SEQ6801 | 2.1E+00 |
| lnc-CLK4-2:2 | SEQ6427 | 3.8E+00 | lnc-CLASP2-3:1 | SEQ6802 | 2.1E+00 |
| lnc-AC084851.1-9:1 | SEQ6428 | 3.8E+00 | lnc-BICD1-1:1 | SEQ2413 | 2.1E+00 |
| lnc-POM121L2-2:2 | SEQ2341 | 3.8E+00 | lnc-NIPA1-1:3 | SEQ6803 | 2.1E+00 |
| PAXBP1-AS1:4 | SEQ6429 | 3.8E+00 | lnc-MBTPS1-1:1 | SEQ6804 | 2.1E+00 |
| PAXBP1-AS1:3 | SEQ6430 | 3.8E+00 | lnc-SLC35G3-1:4 | SEQ6805 | 2.1E+00 |
| TRAF3IP2-AS1:4 | SEQ6431 | 3.8E+00 | lnc-STK16-4:1 | SEQ6806 | 2.1E+00 |
| lnc-KAZALD1-2:3 | SEQ6432 | 3.8E+00 | TTC28-AS1:2 | SEQ6807 | 2.1E+00 |
| lnc-AL669831.1-3:33 | SEQ6433 | 3.8E+00 | lnc-ECE1-2:2 | SEQ6808 | 2.1E+00 |
| lnc-SLC6A18-2:2 | SEQ6434 | 3.8E+00 | lnc-DNA2-1:1 | SEQ6809 | 2.1E+00 |
| lnc-VPS72-2:2 | SEQ6435 | 3.8E+00 | lnc-TCF3-2:2 | SEQ6810 | 2.1E+00 |
| LINC01278:1 | SEQ6436 | 3.7 E+00 | lnc-POLR2I-1:1 | SEQ6811 | 2.1E+00 |
| lnc-BDH1-5:3 | SEQ6437 | 3.7 E+00 | lnc-GRM1-1:4 | SEQ6812 | 2.1E+00 |
| NPTN-IT1:2 | SEQ6438 | 3.7 E+00 | lnc-ADAMTS18-1:1 | SEQ6813 | 2.1E+00 |
| lnc-DAXX-2:1 | SEQ6439 | 3.7E+00 | lnc-CCDC56-2:1 | SEQ6814 | 2.1E+00 |
| lnc-UBE2E3-2:12 | SEQ6440 | 3.7E+00 | lnc-SLC30A10-7:1 | SEQ6815 | 2.1E+00 |
| lnc-MINOS1-2:2 | SEQ6441 | 3.7 E+00 | lnc-AL627171.1-1:1 | SEQ6816 | 2.1E+00 |
| lnc-POM121C-1:3 | SEQ6442 | 3.7E+00 | lnc-ARL6IP4-1:6 | SEQ6817 | 2.1E+00 |
| WAC-AS1:2 | SEQ6443 | 3.7 E+00 | lnc-DNAJC5-1:3 | SEQ5372 | 2.1E+00 |
| lnc-RGPD8-1:1 | SEQ6444 | 3.7 E+00 | lnc-SMYD5-1:4 | SEQ6818 | 2.1E+00 |
| lnc-SLC4A5-5:4 | SEQ6445 | 3.7 E+00 | lnc-NAP1L5-3:1 | SEQ6819 | 2.1E+00 |
| lnc-GABBR1-1:2 | SEQ2290 | 3.7 E+00 | lnc-APBA2-1:8 | SEQ6820 | 2.1E+00 |
| lnc-AC006465.3.1-1:13 | SEQ6446 | 3.7E+00 | lnc-WTAP-2:2 | SEQ6821 | 2.1E+00 |
| lnc-TRAPPC11-4:1 | SEQ6447 | 3.7 E+00 | MIF-AS1:8 | SEQ2383 | 2.1E+00 |
| lnc-UROS-2:1 | SEQ6448 | 3.7 E+00 | lnc-NCAPD3-2:1 | SEQ6822 | 2.1E+00 |
| CD27-AS1:5 | SEQ6449 | 3.7 E+00 | lnc-ZBTB8OS-3:1 | SEQ6823 | 2.1E+00 |
| lnc-LXN-1:3 | SEQ6450 | 3.7 E+00 | lnc-EXTL3-6:7 | SEQ6824 | 2.1E+00 |
| lnc-RASL11B-10:1 | SEQ6451 | 3.6E+00 | lnc-MKKS-4:1 | SEQ6825 | 2.1E+00 |
| lnc-DLG2-3:1 | SEQ6452 | 3.6E+00 | lnc-CWH43-3:1 | SEQ6826 | 2.1E+00 |
| lnc-NECAP1-1:2 | SEQ6453 | 3.6E+00 | lnc-FAM75A4-3:3 | SEQ6827 | 2.1E+00 |
| lnc-EDEM1-1:1 | SEQ6454 | 3.6E+00 | lnc-PKHD1L1-2:11 | SEQ6828 | 2.1E+00 |
| lnc-STX2-8:1 | SEQ6455 | 3.6E+00 | lnc-TUBB3-3:1 | SEQ6829 | 2.1E+00 |
| lnc-SLC35F5-14:11 | SEQ6456 | 3.6E+00 | lnc-ARHGAP15-2:3 | SEQ2483 | 2.1E+00 |
| lnc-EPB41L5-1:1 | SEQ6457 | 3.6E+00 | lnc-NAB1-2:2 | SEQ6830 | 2.1E+00 |
| lnc-LAMTOR1-1:1 | SEQ6458 | 3.6E+00 | lnc-KCNT1-6:1 | SEQ2699 | 2.1E+00 |
| lnc-KBTBD2-2:2 | SEQ6459 | 3.5 E+00 | lnc-GRSF1-1:1 | SEQ4817 | 2.1E+00 |
| lnc-ARC-1:5 | SEQ6460 | 3.5 E+00 | lnc-LYRM7-3:1 | SEQ6831 | 2.1E+00 |
| lnc-SON-3:2 | SEQ6461 | 3.5 E+00 | lnc-RNF111-2:1 | SEQ6832 | 2.1E+00 |
| lnc-CCNL2-3:2 | SEQ6462 | 3.5 E+00 | lnc-DCTN5-1:2 | SEQ6833 | 2.1E+00 |
| lnc-RP11-1035H13.3.1-1:2 | SEQ6463 | 3.5 E+00 | lnc-C3orf38-3:3 | SEQ6834 | 2.1E+00 |
| lnc-LPCAT1-3:13 | SEQ6464 | 3.5 E+00 | lnc-PXDC1-12:3 | SEQ4641 | 2.1E+00 |
| lnc-RP11-96O20.4.1-1:1 | SEQ6465 | 3.5 E+00 | lnc-EGLN2-1:1 | SEQ6835 | 2.1E+00 |
| lnc-THAP4-3:2 | SEQ6466 | 3.5 E+00 | lnc-GRID2IP-1:2 | SEQ6836 | 2.1E+00 |
| lnc-SFXN5-1:4 | SEQ6467 | 3.5 E+00 | lnc-RP11-977G19.10.1-1:3 | SEQ6837 | 2.1E+00 |
| lnc-GOLT1B-1:3 | SEQ6468 | 3.5 E+00 | lnc-THAP4-4:1 | SEQ6838 | 2.1E+00 |
| lnc-SUGT1-3:1 | SEQ0131 | 3.5 E+00 | lnc-AL139333.1-1:1 | SEQ6839 | 2.1E+00 |
| ATP6VOE2-AS1:5 | SEQ6469 | 3.5 E+00 | lnc-UXS1-4:7 | SEQ6840 | 2.1E+00 |
| lnc-FAM71E1-1:1 | SEQ6470 | 3.4E+00 | lnc-ZNF746-3:4 | SEQ6841 | 2.1E+00 |
| lnc-ZNF8-4:3 | SEQ6471 | 3.4E+00 | lnc-FAM213A-1:1 | SEQ6842 | 2.1E+00 |
| LINC00936:2 | SEQ6472 | 3.4E+00 | lnc-RP11-219B4.5.1-2:1 | SEQ6843 | 2.1E+00 |
| DANCR:2 | SEQ6473 | 3.4E+00 | lnc-PRR3-1:2 | SEQ6844 | 2.1E+00 |
| lnc-DDX31-3:1 | SEQ6474 | 3.4E+00 | lnc-IL17RC-3:3 | SEQ6845 | 2.1E+00 |
| lnc-TKT-1:9 | SEQ6475 | 3.4E+00 | lnc-C11orf48-1:2 | SEQ6846 | 2.1E+00 |
| lnc-EFCAB8-1:2 | SEQ6476 | 3.4E+00 | lnc-FRG1B-2:3 | SEQ6847 | 2.1E+00 |
| lnc-RSPH10B2-1:3 | SEQ2580 | 3.4E+00 | lnc-SNAP47-2:1 | SEQ5143 | 2.1E+00 |
| lnc-NTRK1-4:1 | SEQ6477 | 3.4E+00 | lnc-AKT3-6:1 | SEQ6848 | 2.1E+00 |
| lnc-RPL3-2:3 | SEQ6478 | 3.4E+00 | lnc-ITGB3BP-5:5 | SEQ6849 | 2.1E+00 |
| lnc-CCL28-2:1 | SEQ6479 | 3.4E+00 | lnc-AL049840.1-2:1 | SEQ6850 | 2.1E+00 |
| lnc-ARHGEF35-2:1 | SEQ6480 | 3.4E+00 | lnc-HIGD1C-1:7 | SEQ6851 | 2.0E+00 |
| lnc-IL12RB2-3:1 | SEQ6481 | 3.4E+00 | lnc-CNP-1:1 | SEQ3058 | 2.0E+00 |
| lnc-BDH1-5:2 | SEQ6482 | 3.4E+00 | lnc-PRPF31-2:1 | SEQ6852 | 2.0E+00 |
| lnc-TRIM38-1:1 | SEQ6483 | 3.4E+00 | lnc-BX255923.1-5:1 | SEQ6853 | 2.0E+00 |
| lnc-ATPAF2-2:5 | SEQ6484 | 3.4E+00 | lnc-GUCY1A2-1:1 | SEQ6854 | 2.0E+00 |
| lnc-HIGD1C-1:5 | SEQ6485 | 3.4E+00 | LINC00674:12 | SEQ6855 | 2.0E+00 |
| lnc-RP11-645C24.1.1-3:8 | SEQ6486 | 3.3E+00 | lnc-ARMCX6-3:1 | SEQ6856 | 2.0E+00 |
| lnc-TCTA-1:1 | SEQ4966 | 3.3E+00 | lnc-ODF4-4:1 | SEQ6857 | 2.0E+00 |
| MAPKAPK5-AS1:8 | SEQ6487 | 3.3 E+00 | lnc-DDX19B-2:1 | SEQ6858 | 2.0E+00 |
| lnc-TMSB10-1:1 | SEQ6488 | 3.3 E+00 | lnc-CLNS1A-3:1 | SEQ6859 | 2.0E+00 |
| lnc-AES-1:3 | SEQ6489 | 3.3 E+00 | lnc-PPIL1-2:2 | SEQ6860 | 2.0E+00 |
| lnc-GNAI2-1:12 | SEQ6490 | 3.3 E+00 | lnc-PSMA3-1:1 | SEQ6861 | 2.0E+00 |
| lnc-ATP6V1C1-9:4 | SEQ3802 | 3.3E+00 | lnc-TCEANC2-2:2 | SEQ6862 | 2.0E+00 |
| lnc-GLTSCR2-2:6 | SEQ6491 | 3.3 E+00 | LINC01123:5 | SEQ6863 | 2.0E+00 |
| lnc-IL9R-1:6 | SEQ6492 | 3.3 E+00 | lnc-CLPB-1:1 | SEQ6864 | 2.0E+00 |
| lnc-ZEB2-1:9 | SEQ6493 | 3.3E+00 | lnc-RNMT-1:1 | SEQ6865 | 2.0E+00 |
| lnc-AL669831.1-3:10 | SEQ6494 | 3.3 E+00 | lnc-CUEDC1-2:1 | SEQ6866 | 2.0E+00 |
| lnc-GRIK2-5:1 | SEQ6495 | 3.3E+00 | lnc-NAB1-2:3 | SEQ6867 | 2.0E+00 |
| lnc-OTP-5:1 | SEQ6496 | 3.3E+00 | lnc-MUC5B-1:1 | SEQ5724 | 2.0E+00 |
| lnc-ST6GALNAC6-1:4 | SEQ6497 | 3.3 E+00 | lnc-BDH1-5:9 | SEQ6868 | 2.0E+00 |
| lnc-BLZF1-2:4 | SEQ6498 | 3.3 E+00 | lnc-CTDSP2-2:6 | SEQ6869 | 2.0E+00 |
| lnc-AL669831.1-3:4 | SEQ6499 | 3.2E+00 | lnc-LRRC40-3:1 | SEQ6870 | 2.0E+00 |
| lnc-CNR2-1:7 | SEQ6500 | 3.2 E+00 | lnc-C5orf32-3:1 | SEQ6871 | 2.0E+00 |
| lnc-SRPRB-1:1 | SEQ2427 | 3.2 E+00 | lnc-RPP38-5:1 | SEQ5046 | 2.0E+00 |
| lnc-NETO2-4:1 | SEQ6501 | 3.2E+00 | lnc-JAG1-6:1 | SEQ2547 | 2.0E+00 |
| lnc-RP11-796G6.2.1-4:7 | SEQ6502 | 3.2 E+00 | lnc-FGF14-1:1 | SEQ6872 | 2.0E+00 |
| lnc-SLC25A16-1:1 | SEQ6503 | 3.2 E+00 | lnc-ZNF8-4:1 | SEQ6873 | 2.0E+00 |
| lnc-FAM165B-1:2 | SEQ6504 | 3.2 E+00 | lnc-CPLX1-2:11 | SEQ6874 | 2.0E+00 |
| lnc-NUTF2-1:1 | SEQ6505 | 3.2 E+00 | lnc-KRT80-3:1 | SEQ3433 | 2.0E+00 |
| lnc-CTDSP2-2:9 | SEQ6506 | 3.2 E+00 | lnc-CPSF3-2:1 | SEQ6875 | 2.0E+00 |
| lnc-HNRNPA1L2-2:4 | SEQ6507 | 3.2E+00 | lnc-PROKR2-1:1 | SEQ6876 | 2.0E+00 |
| lnc-NDUFA7-1:2 | SEQ6508 | 3.2 E+00 | lnc-POLD1-2:1 | SEQ6877 | 2.0E+00 |
| lnc-TRAPPC6B-3:1 | SEQ6509 | 3.2 E+00 | lnc-C16orf52-1:1 | SEQ6878 | 2.0E+00 |

The correlation between 31 IncRNAs identified in the invention and neurocognitive MMSE (Mini-Mental State Examination) test or cerebrospinal fluid CSF biomarkers Abeta and Tau or phosphorylated tau or age were examined. The result is shown in Table 13.

**Table 13. Correlation between blood IncRNAs and neurocognitive MMSE test or cerebrospinal fluid CSF biomarkers Abeta and Tau or phosphorylated tau or age.**

| | ab42 | tau | ptau | MMSE | age | |
|---|---|---|---|---|---|---|
| n | 70 | 70 | 70 | 84 | 137 | |
| ab42 | r | 1.000 | -0.207 | -.322** | 0.067 | -0.044 |
| tau | r | -0.207 | 1.000 | .853^{**} | 0.028 | 0.117 |
| FTX_19 | r | .266^{*} | 0.000 | -0.076 | -0.032 | -0.093 |
| LEF1_AS1_1 | r | -0.121 | 0.013 | -0.016 | 0.089 | -.364** |
| lnc_ADAD1_3_1 | r | .302^{*} | 0.031 | -0.050 | -0.089 | -0.124 |
| lnc_ANKRD36_1_4 | r | -0.053 | -0.191 | -0.138 | 0.035 | -.222** |
| lnc_ATP6AP2_13_1 | r | -0.218 | 0.113 | 0.196 | -.216* | 0.151 |
| lnc_CA6_8_1 | r | 0.003 | -0.067 | -0.030 | .220^{*} | -0.133 |
| lnc_CA6_8_2 | r | 0.080 | 0.035 | 0.047 | .351^{**} | -0.082 |
| lnc_CDIPT_2_1 | r | -0.058 | -0.086 | 0.051 | .255^{*} | -0.073 |
| lnc_CGREF1_2_1 | r | .494^{**} | -0.089 | -0.177 | -0.099 | -0.156 |
| lnc_EVX1_15_1 | r | .255^{*} | 0.005 | -0.027 | 0.193 | -0.078 |
| lnc_IL31RA_1_1 | r | 0.231 | -0.053 | -0.077 | 0.164 | -.360** |
| lnc_KIF14_1_2 | r | .259^{*} | 0.075 | -0.021 | 0.035 | -0.015 |
| lnc_NEMF_1_4 | r | -.325** | 0.089 | 0.168 | 0.096 | .213^{*} |
| lnc_NID1_4_4 | r | .305^{*} | 0.118 | 0.053 | 0.038 | -0.130 |
| lnc_OCM_3_4 | r | 0.088 | -0.087 | -0.128 | 0.206 | -.308** |
| lnc_PCP4L1_3_2 | r | .342^{**} | 0.072 | -0.035 | -0.015 | -0.167 |
| lnc_PDGFA_6_2 | r | -.290* | 0.130 | 0.204 | 0.103 | .179^{*} |
| lnc_SLC35E3_8_2 | r | .386^{**} | 0.002 | -0.069 | -0.084 | -0.106 |
| lnc_SOCS6_10_1 | r | .321^{**} | -0.135 | -0.209 | -0.160 | -0.069 |
| Inc_STX10_2_1 | r | -.276* | 0.141 | 0.218 | 0.079 | -0.002 |
| lnc_UQCC3_2_1 | r | -.356** | -0.003 | 0.086 | 0.161 | 0.114 |
| lnc_USP47_2_1 | r | .237^{*} | -0.047 | -0.042 | 0.210 | -0.080 |
| lnc_ZCCHC13_4_1 | r | .329^{**} | 0.081 | -0.019 | -0.043 | -0.120 |
| lnc_ZNF516_14_1 | r | .270^{*} | 0.025 | -0.060 | -0.003 | -0.097 |
| MYLK_AS1_13 | r | .419^{**} | -0.031 | -0.183 | 0.031 | -.247** |
| OIP5_AS1_36 | r | .316^{**} | -0.075 | -0.110 | 0.079 | -.227** |
| PCBP1_AS1_302 | r | .251^{*} | 0.048 | -0.058 | -0.152 | 0.026 |
| TCONS_00017372 | r | -.306** | 0.083 | 0.118 | 0.038 | .182^{*} |
| TTC21B_AS1_2 | r | 0.193 | 0.005 | 0.027 | -0.001 | .194^{*} |
| lnc-FOXD4L5-16:1 | r | -.341** | 0.120 | 0.174 | 0.100 | 0.059 |
| lnc-ZC3H12B-11:1 | r | .302^{*} | -0.094 | -0.227 | -0.045 | -.235** |

In all IncRNAs examined, except ANKRD36-1-4, the correlation with age still remained significant for subject groups after adjusting with age by generalized linear model, indicating that the differential expression of selected IncRNAs when comparing subject groups is not due simply to an age effect but to the disease effect, highlighting their relevance for diagnosis and/or therapeutic applications for the respective dementia types.

To further select the best candidates of IncRNAs, fold change (FC) and AUC were calculated for each IncRNA and for each tested condition. IncRNA candidates were selected when differential expression was determined based on an appropriate fold-change (FC) such as a FC > 1.2 (or < 0.8) and/or an appropriate AUC such as AUC > 0.65 (or <0.35) when comparing two groups. Random Forest algorithm (Breimann 2001, Breiman and Cutler 2001) was used to build the model and also to select the top ranked IncRNAs. A predictive model based on the combination of the list of top ranked candidates comprising at least 2 IncRNAs enables to predict the disease with an accuracy of ≥ 65% and up to 100%.

The predictive modelling based on the random forest algorithm to discriminate between mild cognitive impairment (MCI) patient and healthy control populations when using the total of the 844 IncRNAs measurable in whole blood samples and having an AUC ≥ 0.8 (or <0.2) described in the Table 11 enabled to show that the AUC in function of the number of IncRNAs reached a plateau with the following 2 IncRNAs. These 2 IncRNAs were used to construct the model. The results show that this IncRNA signature enabled a discrimination between the 2 populations (Mild cognitive impairment patient and healthy control populations) with an AUC value = 1, an accuracy = 100%, sensitivity = 100% and specificity = 100%.

| IncRNA | Rank |
|---|---|
| TCONS_00035093 | 1 |
| Inc-OCM-3:4 | 2 |

The predictive modelling based on the random forest algorithm to discriminate between mild AD patient and healthy control populations when using the total of the 500 IncRNAs measurable in whole blood and having an AUC ≥ 0.7 (or ≤0.3) described in the Table 11 , enabled to show that the AUC in function of the number of IncRNAs reached a plateau with the following 7 IncRNAs. These 7 IncRNAs were used to construct the model. The results show that this IncRNA signature enabled a discrimination between the 2 populations (mild AD patient and healthy control populations) with an AUC value = 0.954, an accuracy = 91%, sensitivity = 90% and specificity = 91%.

| IncRNA | Rank |
|---|---|
| Inc-KIF14-1:2 | 1 |
| PCBP1-AS1:302 | 2 |
| Inc-CA6-8:1 | 3 |
| NEAT1:22 | 4 |
| Inc-CA6-8:2 | 5 |
| STARD7-AS1:5 | 6 |
| Inc-IL31RA-1:1 | 7 |

The predictive modelling based on the random forest algorithm to discriminate between moderate-to-severe AD patient and healthy control populations when using the total of the 605 IncRNAs measurable in whole blood and having an AUC ≥ 0.65 (or ≤0.35) described in the Table 11, enabled to show that the AUC in function of the number of IncRNAs reached a plateau with the following 6 IncRNAs. These 6 IncRNAs were used to construct the model. The results show that this IncRNA signature enabled a discrimination between the 2 populations (moderate to severe AD patient and healthy control populations) with an AUC value = 0.922, an accuracy = 83%, sensitivity = 70% and specificity = 91%.

| IncRNA | Rank |
|---|---|
| Inc-CA6-8:2 | 1 |
| Inc-ANKRD36-1:4 | 2 |
| Inc-ATP6AP2-13:1 | 3 |
| Inc-SOCS6-1 0:1 | 4 |
| Inc-CDIPT-2:1 | 5 |
| Inc-USP47-2:1 | 6 |

The predictive modelling based on the random forest algorithm to discriminate between all AD patient (mild+moderate-to-severe) and healthy control populations when using the total of the 1124 IncRNAs measurable in whole blood and having an AUC ≥ 0.65 (or ≤0.35) described in the Table 11, enabled to show that the AUC in function of the number of IncRNAs reached a plateau with the following 7 IncRNAs. These 7 IncRNAs were used to construct the model. The results show that this IncRNA signature enabled a discrimination between the 2 populations (AD patient and healthy control populations) with an AUC value = 0.954, an accuracy = 91%, sensitivity = 88% and specificity = 92%.

| IncRNA | Rank |
|---|---|
| Inc-CA6-8:2 | 1 |
| Inc-CA6-8:1 | 2 |
| RBM26-AS1:1 | 3 |
| PCBP1-AS1:302 | 4 |
| MIR181A2HG:1 | 5 |
| TTC21B-AS1:2 | 6 |
| Inc-SOCS6-10:1 | 7 |

The predictive modelling based on the random forest algorithm to discriminate between dementia with Levy bodies patient and healthy control populations when using the total of the 1352 IncRNAs measurable in whole blood and having an AUC ≥ 0.8 (or ≤ 0.2) described in the Table 11, enabled to show that the AUC in function of the number of IncRNA reached a plateau with the following 2 IncRNAs. These 2 IncRNAs were used to construct the model. The results show that this IncRNA signature enabled a discrimination between the 2 populations (Dementia with Levy bodies patient and healthy control populations) with an AUC value = 1, an accuracy = 100%, sensitivity = 100% and specificity = 100%.

| IncRNA | **Rank** |
|---|---|
| Inc-OCM-3:4 | 1 |
| Inc-SLC35E3-8:1 | 2 |

The predictive modelling based on the random forest algorithm to discriminate between frontotemporal dementia patient and healthy control populations when using the total of the 1041 IncRNAs measurable in whole blood and having an AUC ≥ 0.8 (or ≤ 0.2) described in the Table 4, enabled to show that the AUC in function of the number of IncRNA reached a plateau with the following 3 IncRNAs. These 3 IncRNAs were used to construct the model. The results show that this IncRNA signature enabled a discrimination between the 2 populations (Frontotemporal dementia patient and healthy control populations) with an AUC value = 1, an accuracy = 100%, sensitivity = 100% and specificity = 100%.

| IncRNA | Rank |
|---|---|
| Inc-ZCCHC13-4:1 | 1 |
| PCBP1-AS1:302 | 2 |
| Inc-NEMF-1:4 | 3 |

The predictive modelling based on the random forest algorithm to discriminate between All AD patients' population and NAD (FTD+DLB) patients populations when using the total of the 6 IncRNAs measurable in whole blood and having an AUC ≥ 0.65 (or ≤ 0.35) described in the Tables 11, 12, enabled to show that the AUC in function of the number of IncRNA reached a plateau with the following 6 IncRNAs. These X6 IncRNAs were used to construct the model. The results show that this IncRNA signature enabled a discrimination between the 2 populations (AD populations and population suffering other dementia types) with an AUC value = 0,804, an accuracy = 79%, sensitivity = 56% and specificity = 91%.

| IncRNA | rank |
|---|---|
| Inc-STAT3-1:2 | 1 |
| Inc-NEMF-1:4 | 2 |
| Inc-EPN2-3:2 | 3 |
| Inc-SLC25A39-2:1 | 4 |
| Inc-ARF6-1:1 | 5 |
| Inc-CCNYL1-2:1 | 6 |

The predictive modelling based on the random forest algorithm to discriminate between All AD patients' population and DLB, FTD patients + healthy control populations when using the total of the 5 IncRNAs measurable in whole blood and having an AUC ≥ 0.65 (or ≤ 0.35) described in the Table 4, enabled to show that the AUC in function of the number of IncRNAs reached a plateau with the following 5 IncRNAs. These 5 IncRNAs were used to construct the model. The results show that this IncRNA signature enabled a discrimination between the 2 populations (AD population and population either suffering other dementia type or having normal cognitive status) with an AUC value = 0,811, an accuracy = 72%, sensitivity = 61% and specificity = 81%.

| IncRNA | rank |
|---|---|
| ANKRD44-IT1:1 | 1 |
| APOBEC3B-AS1:2 | 2 |
| ADAMTSL4-AS1:2 | 3 |
| AGAP2-AS1:2 | 4 |
| A1BG-AS1:14 | 5 |

As for diagnosis, for differential diagnosis, several other IncRNAs panels were identified such as those listed below comprising at least 3 IncRNAs from Tables 11, 12 and enabling to detect each dementia type specifically:

| Panel Example 1 | Panel Example 2 | Panel Example 3 | Panel Example 4 |
|---|---|---|---|
| LEF1-AS1:1 | Inc-CGREF1-2:1 | TCONS_00011994 | OIP5-AS1:36 |
| PCBP1-AS1:302 | PSMB8-AS1:14 | Inc-EVX1-15:1 | MYLK-AS1:13 |
| Inc-CA6-8:2 | Inc-STX10-2:1 | Inc-REC8-2:1 | Inc-PCP4L1-3:2 |
| | Inc-TCFL5-6:1 | | |
| | ZC3H12B-11:1 | | |

The signature of the following 11 top ranked IncRNAs expressed in whole blood (Paxgene RNA samples) enabled an excellent discrimination between HC group and all other groups (MCI, all AD, DLB, FTD) with AUC=0.963, accuracy=93.4%, sensitivity=65% and specificity=98.3%.

| IncRNA | Rank |
|---|---|
| PCBP1-AS1:302 | 1 |
| STARD7-AS1:5 | 2 |
| Inc-NID1-4:4 | 3 |
| RBM26-AS1:1 | 4 |
| LEF1-AS1:1 | 5 |
| Inc-CA6-8:1 | 6 |
| Inc-CA6-8:2 | 7 |
| Inc-OCM-3:4 | 8 |
| Inc-AFG1L-7:1 | 9 |
| Inc-LASP1-5:1 | 10 |
| Inc-GCGR-1:2 | 11 |

The signature of the following 7 top ranked IncRNAs expressed in whole blood (Paxgene RNA samples) enabled an excellent discrimination between HC group and patients with mild AD, with with accuracy=90,7%, sensitivity=90% and specificity=91.2%.

| IncRNA | Rank |
|---|---|
| Inc-KIF14-1:2 | 1 |
| PCBP1-AS1:302 | 2 |
| Inc-CA6-8:1 | 3 |
| NEAT1:22 | 4 |
| Inc-CA6-8:2 | 5 |
| STARD7-AS1:5 | 6 |
| Inc-IL31RA-1:1 | 7 |

The signature of the following 7 top ranked IncRNAs expressed in whole blood (Paxgene RNA samples) enabled an excellent discrimination between HC group and patients with moderate to severe AD with accuracy=83.3%, sensitivity=70% and specificity=91.2%.

| | |
|---|---|
| Inc-CA6-8:2 | 1 |
| Inc-ANKRD36-1:4 | 2 |
| Inc-ATP6AP2-13:1 | 3 |
| Inc-SOCS6-1 0:1 | 4 |
| Inc-CDIPT-2:1 | 5 |
| Inc-USP47-2:1 | 6 |
| Inc-UXS1-3:1 | 7 |

The signature of the following 3 top ranked IncRNAs expressed in whole blood (Paxgene RNA samples) enabled an excellent discrimination between HC group and patients with dementia with Lewy bodies with accuracy=100%, sensitivity=100% and specificity=100%.

| | |
|---|---|
| Inc-OCM-3:4 | 1 |
| Inc-SLC35E3-8:1 | 2 |
| LINC-PINT:11 | 3 |

The signature of the following 2 top ranked IncRNAs expressed in whole blood (Paxgene RNA samples) enabled an excellent discrimination between HC group and patients with frontotemporal dementia with accuracy=100%, sensitivity=100% and specificity=100%.

| | |
|---|---|
| Inc-ZCCHC13-4:1 | 1 |
| PCBP1-AS1:302 | 2 |

The signature of the following 5 top ranked IncRNAs expressed in whole blood (Paxgene RNA samples) enabled an excellent discrimination between AD patients (with mild AD patients and moderate-to severe AD) and non-AD dementia group (FTD+DLB) with accuracy=76.5%, sensitivity=55.9% and specificity=86.8%.

| IncRNA | rank |
|---|---|
| Inc-JUNB-1:1 | 1 |
| Inc-RARB-4:1 | 2 |
| Inc-ZNF284-1:1 | 3 |
| TCONS_00035093 | 4 |
| Inc-TELO2-3:3 | 5 |

The signature of the following 5 top ranked IncRNAs expressed in whole blood (Paxgene RNA samples) enabled an excellent discrimination between AD patients (with mild AD patients and moderate-to severe AD) and non-AD dementia group (FTD+DLB) with accuracy=82.4%, sensitivity=61.8% and specificity=92.6%.

| IncRNA | Rank |
|---|---|
| Inc-CGREF1-2:1 | 1 |
| PSMB8-AS1:14 | 2 |
| Inc-STX10-2:1 | 3 |
| Inc-TCFL5-6:1 | 4 |
| Inc-ZC3H12B-11:1 | 5 |

### MATERIAL AND METHODS

To identify lncRNAs in brain, serum, plasma, whole blood or other samples of human subjects, total RNA was first extracted and sequencing libraries were prepared by removal of ribosomic RNA (RiboZero TruSeq library preparation kit, Illumina Inc. San Diego, USA) and sequenced on Illumina NextSeq500 with 2x75 bp read length.

IncRNAs were also measured by qPCR using specific primers. For this, total RNA was first extracted, reverse transcription and real time PCR using specific primers were performed. Using FiMAP (also called Quantamatrix QMAP platform and also a new fully automatized version QMX), a proprietary platform based on hybridization of PCR products on coated microdiscs with complementary oligonucleotide coupled to detection probes. Total RNA was extracted followed by reverse transcription and PCR step allowing multiplexing of several targets using specific primers to the IncRNAs.

To show the efficacy of FiMAP as a multiplexed platform capable to quantify IncRNAs, plasma samples have been tested using NGS, qPCR, Celemics and FiMAP. Most of the IncRNAs showed that they are well quantified since the dilutions of the input RNAs are concentration dependent manner measured on QMAP, and that for IncRNA 20321 very equivalent results were obtained when using the reference qPCR method. Quantification was therefore performed on the FiMAP platform using coded microdiscs specific of each IncRNA.

### Patient population and samples

Brain tissue biopsies: postmortem brain medio-temporal cortex tissue samples and the blood samples other than from the below-mentioned prospective studies, as well peripheral organ biopsies: lung, ovary, colon, prostate, breast, liver, bladder, kidney, skin, heart, muscle) were from diverse European hospitals and Firalis biobanks.

Body fluid samples: Body fluid samples were from healthy volunteers (HV), donors at the "Etablissement Français du Sang" (EFS) of Mulhouse, France, and from cognitively intact healthy control subjects and from patients with mild cognitive impairment or with different stages of Alzheimer's disease (AD) or with other dementia types recruited, according to the protocols of European hospitals accepted by country ethic committees, and in the Amoneta Diagnostics sponsored prospective studies registered to the Agence Nationale de Sécurité du Médicament et des Produits de Santé (ANSM) such as under the ID RCB: 2015-A00118-41 on Jan 22, 2015, the ID RCB: 2016-A200227-44 on Feb 4, 2016. Whole blood samples were collected in Paxgene RNA tubes. Serum or plasma samples were prepared from blood samples collected in lithium-heparin tubes or EDTA tubes and CSF, urine, saliva and tears were collected using polypropylene tubes.

Results shown in tables and drawings from the invention are those obtained in 10 postmortem brain samples (5AD, 5 HC), 24 serum samples (12 AD and 12 HC), 12 plasma samples (6 AD and 6 HC) and 139 samples from the following subject groups:

| Subject Group | Gender | | |
|---|---|---|---|
| | Male | Female | Total |
| HC | 6 | 14 | 20 |
| MCI | 8 | 7 | 15 |
| Mild AD | 15 | 19 | 34 |
| Mod-SevAD | 11 | 23 | 34 |
| DLB | 5 | 10 | 15 |
| FTD | 2 | 17 | 19 |
| Other diseases | 2 | 0 | 2 |
| Total | 49 | 90 | 139 |

### Method for IncRNAs

### Samples sequencing

Ribonucleic acid (RNA) extraction is performed starting from 1.5ml of serum, using Norgen Serum/plasma extraction and RNA Clean-Up and Concentration Micro-Elute Kits according to the manufacturer's instructions. Sequencing libraries are prepared from the total amount of extracted RNA, using the Illumina TruSeq stranded total RNA library preparation kit combined with the human/mouse/rat RiboZero rRNA removal kit (Illumina Inc. San Diego, USA, C). All steps are performed with the low-throughput protocol and according to the manufacturer's instructions, with no fragmentation step. Briefly, cytoplasmic ribosomal RNA (rRNA) are hybridized to biotinylated target-specific oligos and removed using streptavidin coated magnetic beads. rRNA depleted RNA samples are then reverse transcribed into complementary deoxyribonucleic acid (cDNA). To ensure strand specificity, single stranded cDNA is first synthetized using Super-Script II reverse transcriptase (Invitrogen) and random primers in the presence of Actinomycin D, and then converted to double stranded cDNA with the second strand marking mix that incorporates dUTP in place of dTTP. Resulting blunt ended cDNA are purified using AMPure XP magnetic beads. After a 3'end adenylation step, Illumina's adapters ligation is performed. So, obtained singled indexed libraries are washed twice using AMPure XP beads to remove excess adapters and enriched by PCR (15 cycles). PCR products are purified with a final AMPure XP beads wash and sequencing ready libraries are eluted in 30µl of resuspension buffer.

For quality control, 1µl of each library is run on the Agilent Technologies 2100 Bioanalyzer using a DNA 1000 chip according to the manufacturer's recommendations. Absence of adapter dimers is checked and the average library size is determined by a region table. Libraries are quantified on Qubit 2.0 using Qubit dsDNA High Sensitivity assay kit (Invitrogen). Library size previously determined on the Bioanalyzer is used to calculate molar concentrations from mass concentrations. All libraries are sequenced with the Illumina NextSeq500 (2x75bp).

### Bioinformatic analysis

### IncRNA based on the Homo sapiens hg38 genome

RNA-seq data analysis is performed using Partek Flow (Partek Inc., St Louis, MO, USA build 6). The pre-alignment QA/QC module of Partek Flow is used to visualize the read quality of the FASTQ files. All reads are examined. The raw FASTQ files are trimmed at the 3' end in function of their quality score (Phred score). The parameters used are an end minimum quality level of 30 and a minimum trimmed read length of 50. Unaligned reads are mapped using the Homo sapiens hg38 genome. This mapping is done using the software STAR version 2.5.3 (Dobin A. et al., 2013). STAR: ultrafast universal RNA-seq aligner. Bioinforma. Oxf. Engl. 29, 15-21). The default parameters are used. The post-alignment QC module of Partek Flow was used to visualize the average base quality score per position as well as the mapping quality per alignment. The mapped reads were quantified using the GTF file with the patented IncRNA annotation for quantification using the Partek Expectation/Maximization (E/M) algorithm (Xing Y. et al, 2006). An expectation-maximization algorithm for probabilistic reconstructions of full-length isoforms from splice graphs. Nucleic Acids Res. 34, 3150-3160). The default parameters are used. The transcripts where their medians are under the median read density in intergenic regions are discarded for the next steps of the analysis. The transcript counts were normalized by CPM (counts per million). Only transcripts showing high expression (CPM ≥ 5) in at least half the samples of one group are considered.

### Novel IncRNAs discovery

The novel discovery was performed by using UCIncR pipeline version 1.1.1 (Sun Z. et al., Scientific Reports, 7(1): 14196, 2017). In this pipeline, StringTie version 1.3.4d (Pertea M. et al., Nat Biotechnol, 33 (3): 290-295, 2015) was used for the reconstruction.

The "cuffcompare" program version 2.2.1 (C. Trapnell et al., 'Nat Protoc, 7 (3): 562-578, 2012) was used to compare the assembled transcripts to the reference annotated GTF file (Gencode 27 and LNCipedia 5) and to generate a new GTF file with all transcripts from 9 samples for further analysis.

Filtering was done on Transfrag class codes generated by cuffcompare, transcript length, number of exons and protein coding potential. Firstly, the transcripts with code 'i', 'j', '0', 'u', 'x' and '.' were extracted, all of which could potentially include novel IncRNAs. The 'i' category, for example, could contain the IncRNAs entirely within the intron of known genes. Similarly, the 'j' category could be long non-coding isoforms of known genes. The 'o' category could include novel IncRNAs having generic exonic overlap with known transcripts. The 'u' category could be long intergenic non-coding RNAs (IincRNAs). The 'x' category could contain novel IncRNAs on the opposite strand of reference genes. The `.' category may be sequences with multiple classifications. Following this, only the transcripts with a length of ≥ 200nt and with at least 2 exons were kept for the next step. CPAT (Coding-Potential Assessment Tool, L. Wang L., et al., Nucleic Acids Res, 41 (6): e74,2013.) is used for the evaluation the non-coding potential. The transcripts with CPAT score < 0.3 were considered as non-coding. A new GTF file was generated with the final list of selected novel IncRNAs.

Fastq files of GSE45326 were downloaded from ArrayExpress [9]. The dataset includes RNA-seq data of 12 normal human tissues. The paired-end sequencing was performed on ribominus total RNA library. Reads alignment was performed as described before. The quantification was done with the novel IncRNA annotation using the Partek E/M algorithm.

### Statistical analysis and predictive modelling

To determine differentially expressed IncRNA, a statistical analysis is performed using Wilcoxon Mann-Whitney parametric test to compare 2 groups of subjects. A IncRNA with a p value ≤ 0.05 is considered as differentially expressed. Anova test is used for comparison of more than 2 subject groups.

In order to build classification models for the 2 classifications, the Classification for MicroArrays (CMA) package of R (Slawski M, Daumer M, Boulesteix AL. CMA: a comprehensive Bioconductor package for supervised classification with high dimensional data. BMC Bioinformatics 2008, 9:439) with a leave-one-out cross-validation has been used.

The algorithms used for this predictive modelling are (a) random forest, (b) linear discriminant analysis and (c) naive Bayes (Breiman L. Random forests. Machine Learning, 45(1): 5-32, 2001).

The rank of the RNA candidate in a model was calculated by the mean rank of 10 candidates' selections (per CV-fold). Per model (and RNA selection method) the AUC was plotted as a function of the number of RNAs in the model. The optimal number of RNAs per model was determined graphically. ROC curves and confusion matrices were generated to assess the predictive performance of our models. The values of AUC, accuracy, sensitivity, specificity, positive and negative predictive values are reported.

### Additional method for quantification of IncRNA

### Total RNA extraction

Total RNA was extracted using Paxgene Blood RNA kit (Qiagen, France) and Serum/Plasma mini kit (Norgen Biotek, Canada) respectively, according to manufacturer's instructions. RNA quality was examined on an Agilent 2100 Bioanalyzer with the RNA 6000 Nano Kits (Agilent, France). RNA quantity was measured on Qubit 3.0 fluorometer using RNA High sensitivity kit (Fisher Scientific, France).

### cDNA synthesis

. Reverse transcription was performed using high capacity cDNA reverse transcription kit (Fisher scientific, France) according to the manufacturer instructions.

### qPCR quantification

IncRNA were preamplified before qPCR. Preamplification reactions were prepared using Applied Biosystems preamplification master mix with 0.1X (100nM) of each of the primers pairs corresponding to the IncRNAs. 16 preamplification cycles were performed as preconized by the furnisher (50°C 2 minutes, 96°C 10 minutes, 40 cycles at 95°C for 15 seconds and 60°C for 1 minute). Quantitative PCR of biological samples was done in 10 µl total volume with 1 µl of preamplification product diluted 1/20 in TE buffer, 5 µl of 2x Soadvanced SYBR green PCR master mix (Biorad, USA) and 250 nM of each primer. Cycling conditions were 95°C for 5-10 min followed by 40 cycles of 95°C for 10-30 sec and 60°C 30-60 sec. A melting curve analysis (60°C to 99°C) was performed after the thermal profile to ensure specificity in the amplification. Relative expression level was determined against a standard curve realized on a 5 log scale using CFX maestro Software (Biorad, USA).

### QUANTAMATRIX FIMAP quantification

IncRNAs of interest were also quantified using the FIMAP/QMAP platform developed by Quantamatrix (South Korea). Briefly, IncRNAs were amplified by PCR using primers with the same sequences as for qPCR that were chemically modified. Forward primers were phosphorylated in 5' and Reverse primers biotinylated in 5'. Multiplexed PCR reactions were performed on Biorad T100 thermocycler in 20µl reactions containing 2µl of IncRNA cDNA, 250 nM of each primers and 10µl of 2× One Taq Hot Start 2X master Mix (New England Biolabs, USA) with the following conditions: 30 seconds at 94°C, 25 cycles of 30s at 94°C, 1 min at 60°C and 1 min at 68°C, followed by a final extension at 68°C for 5 minutes. PCR products were then digested with 25U of lambda exonuclease for 30 minutes at 37°C to eliminate the phosphorylated strand and keep only the biotinylated ones. So digested products were quantified on QMAP using coded silica microdisks coated with oligos complementary to the RNAs of interest (one code per target oligo). Briefly, biotinylated PCR products were incubated with the coated microdisks in a 96 well plate, and hybridized products revealed by addition of a fluorescent streptavidin conjugate, SAPE (Prozyme, Denmark) after washing steps. Plates were imaged on QMAP that takes 2 images per spot: one dark image to quantify the fluorescence and 1 white light image to read the microdisks codes. Each target relative expression is then calculated by QMAP software by assigning fluorescence signal to each target according to the associated microdisk code.

IncRNAs were also measured by qPCR using specific primers. For this, total RNA was first extracted, reverse transcription and real time PCR using specific primers were performed. Using FiMAP (also called Quantamatrix QMAP platform and also a new fully automatized version QMX), a proprietary platform based on hybridization of PCR products on coated microdiscs with complementary oligonucleotide coupled to detection probes. Total RNA was extracted followed by reverse transcription and PCR step allowing multiplexing of several targets using specific primers to the IncRNAs.

To show the efficacy of FiMAP as a multiplexed platform capable to quantify IncRNAs, plasma samples have been tested using NGS, qPCR, Celemics and FiMAP. Most of the IncRNAs showed that they are well quantified on the QMAP and quantitative with dilutions linearity in function of the input RNAs concentration on a dose dependent manner measured on QMAP with very equivalent results obtained when using the reference qPCR method as for the known reference IncRNA 20321.

Quantification performed on the FiMAP platform using coded microdiscs specific of each IncRNA was appropriate for multiplexing testing platform.

### Celemics description

Celemics is based on a targeted sequencing approach using capture probes of 120 nucleotides that hybridize with the selected IncRNAs with magnetics beads. The capture step is performed following library preparation describe above. Briefly, 100 ng of library DNA is used in a volume of 3.4µl mixed with 5.6µl of Block Mix. Then, 7 µL of the Capture Library and 13 µL of the Hybridization Buffer is mixed with 9 µl of the DNA library preparation and incubated 24h at 65°C. After the incubation, 50 µl of Dynabeads Myone Steptavidin T1 are mixed with 200 µl of wash buffer #1 and added to the hybridization mixture. After removal of supernatant under magnetic condition, 500 µl of wash buffer #2 is added and removed using separator and wash 3 to 6 time with wash buffer #3 and then add 30 µl of nuclease free water for elution. Library is then amplified using PCR mixing on beads Captured DNA (15 µl), PCR post capture primer (5 µl), KAPA Library Amplification Mix (25 µl) and nuclease free water (5 µl) for 16 cycles and sample are purified of using AMPure XP beads prior sequencing. All libraries are sequenced with the Illumina NextSeq500 (2x75bp).

### Results

Profiling 127,802 transcripts based on LNCipedia v5.2 was performed on serum samples from patients suffering of Alzheimer disease and cognitively intact healthy subjects using total-RNAseq technology. Above 127,802 transcripts, 19867 IncRNAs were identified with expression level of over 10 CPM in at least half the samples of one group.

Out of 19867 serum IncRNAs, a list of 1008 IncRNAs useful for diagnosis of Alzheimer disease was selected based on the statistical significance (p value <0.05, Wilcoxon test). Random Forest algorithm was used for the classification model. From this, several sets of 2-20 IncRNAs with high predictive value were identified. The sets of selected 2-20 IncRNAs provide an AUC ranged from 0.7 and up to 1 with an accuracy, sensitivity and specificity ranged from 0.7 to 1.

A total of 1091 novel IncRNAs were further identified using transcript reconstruction in the human postmortem mediotemporal brain tissue of 5 AD cases and 10 HC cases from deep-sequencing experiments. Profiling these novel 1091 novel IncRNAs was performed on same brain area, the mediotemporal cortex from the group of human AD brains and the group of healthy control brains using total-RNAseq technology. Out of these 1091 novel brain transcripts, 26 showed deficient expression and 16 showed overexpression level in AD brains as compared to HC brains (P<0,05).

Profiling of a total of 128 893 comprising the 1091 novel IncRNAs and 127,802 transcripts based on LNCipedia v5.2 was performed using total-RNAseq technology on brain cortex (mediotemporal) from 5 AD cases and 5 healthy controls, and on plasma samples from 12 subjects, 6 patients suffering Alzheimer and 6 healthy controls as well as on whole blood (Paxgene RNA tubes) samples from 137 subjects included in 5 groups: patients suffering of MCI, mild AD, moderate-to-severe AD, DLB or, FTD and cognitively intact healthy control subjects.

Out of the 128 893 transcripts sequenced in each biological sample type from all patient and HC groups, IncRNAs were identified based on their threshold expression level (median over 5 count per million (CPM) in at least half the samples of at least one subject group studied). 10122 brain IncRNAs, 3774 plasma IncRNAs and 9367 whole blood IncRNAs were retained for statistical analysis. The results show:
1- **Brain IncRNAs panels:** Out of the 10122 brain IncRNAs sequenced in the brains and having an expression level > 5 CPM:
   - We confirmed on all brains tested, the expression in the brain of the 1091 novel IncRNAs (Table 7) never described before, which represent novel therapeutic and diagnostic targets for brain disorders including cognitive disorders.
   - We identified 860 brain-enriched IncRNAs (Table 12) that exhibit at least 2-fold higher expression level in the brain as compared to all other peripheral organs studied (lung, ovary, colon, prostate, breast, liver, bladder, kidney, skin, heart, muscle). These 860 IncRNAs represent novel therapeutic and/or diagnosis targets for brain disorders including cognitive disorders.
   - We identified 1202 IncRNAs (Table 8) comprising 42 novel IncRNAs, showing a statistically significant differential expression when comparing AD brains to HC brains: These 1202 IncRNAs represent therapeutic and/or diagnosis candidates specifically for cognitive disorders in particular Alzheimer
**2-Plasma IncRNAs panels:** Out of the retained 3714 plasma IncRNAs, we identified 410 plasma IncRNAs (Table 9) that showed a statistically significant differential expression (p<0.05, Wilcoxon test) when comparing AD plasma samples to HC plasma samples: These 410 IncRNAs represent novel panel of biomarkers useful for diagnosis and for therapeutic response monitoring applications of cognitive disorders in particular MCI and Alzheimer.
**3-Blood IncRNA panels:** Out of the retained blood 9367 IncRNAs, we identified 2982 blood IncRNAs (Tables 10, 11) that showed statistically significant differential expression in at least one of the subject groups studied (HC, MCI, mild AD, Moderate-to-severe AD, all AD, DLB, FTD) (p value< 0.05 using Anova test comparing all groups and a p value <0.05 when using the Wilcoxon test to compare the HC group and one of the patient population groups studied). Further, we applied additional criteria such as fold change <0.8 or >1.2 and AUC >0.65 or < 0.35 to select the 2847 IncRNAs and 136 IncRNAs listed in Tables 10 and 11.

These 2982 blood IncRNAs represent novel biomarker(s) and biomarker signature(s) useful for diagnosis, differential diagnosis and for therapeutic response monitoring applications of cognitive disorders in particular MCI and Alzheimer as MCI, mild AD, moderate-to-severe AD, DLB and FTD and other cognitive disorders.

**'-Circulating IncRNAs** panels (common-to brain and present in peripheral body fluids): Out of the 10122 IncRNAs sequenced in the brains, the following numbers were identified as circulating IncRNAs in body fluids:
- 2096 circulating IncRNAs in plasma : Out of these 2096 IncRNAs, a panel of 46 IncRNAs showed a differential expression in plasma samples of AD patients group as compared to HC subject group with statistical significance (p<0.05, Wilcoxon test) and having all an AUC value >0.8 or <0.2. Out of these 46 differentially expressed circulating IncRNAs, 17 have an AUC value >0.9, 4 candidates have AUC value=1 thus fully discriminating AD patients from healthy control subjects.
- 4902 IncRNAs detected in blood samples (Pax RNA tube) and thus identified as a novel panel of IncRNAs measurable in the peripheral body fluid. Out of these 4902 IncRNAs, a panel of 310 IncRNAs showed a differential expression in blood samples of at least one of the 5 patients groups studies (MCI, mild AD, moderate-to-severe AD, DLB or FTD patients) as compared to HC subject group with statistical significance (p<0.05, Wilcoxon test) and represent good candidates for diagnosis and therapeutic applications for cognitive disorders. - 5054 IncRNAs circulating IncRNAs in serum . Out of these 5054 IncRNAs, 43 IncRNAs are differentially expressed in serum of AD group as compared to healthy group with statistical significance (p<0.05, Wilcoxon test). Thus, the invention also relates to any common brain/plasma and/or common brain/blood and/or brain/serum lncRNAs and/or brain-enriched IncRNA circulating in plasma, blood, serum or in any other non-invasive peripheral body fluid detected using the methods of the invention for use for therapeutic and diagnosis applications of any brain disorders where such circulating IncRNAs are altered.

In particular the disclosure relates to the panels of lncRNAs listed in tables 1-12 for use for therapeutic and diagnosis applications of brain disorders in particular cognitive disorders.

The sequences of IncRNAs disclosed in this disclosure are shown in the sequence listing included in this application.

## Claims

1. A method for the diagnosis of a cognitive disorder including but not limited to Alzheimer disease in a subject at risk of having or developing a cognitive disorder, comprising:
(a) detecting a level of expression of lncRNAs in a biological sample from said subject;
(b) comparing the level of expression of lncRNAs in the sample to a level of expression in a reference, wherein an increased or decreased level of expression of lncRNAs in the sample compared to the level in the reference identifies the subject having a cognitive disorder or being at risk of developing a cognitive disorder,
wherein the biological sample is selected from blood, plasma, serum, saliva, tear and urine, and
wherein the lncRNAs comprise 2 lncRNAs: TCONS_00035093, Inc-OCM-3:4.

2. The method according to claim 1, wherein in step (a) the expression level of lncRNAs is detected in combination with another biomarker suitable for detection of a cognitive disorder such as neurocognitive tests, neuroimaging methods and/or CSF biomarkers.

3. A method for designing a therapeutic treatment of a cognitive disorder in a subject suffering from a loss or impairment of cognitive function or dementia, said method comprising steps (a), (b) according to claims 1 or 2, and
(c) designing the therapeutic treatment according to the said identified cognitive disorder.

4. A method for adapting therapeutic strategy to a subject suffering from a progressive impairment or loss of cognitive functions or dementia, the said method comprising the following steps:
(a) diagnosing or monitoring the progressive impairment or loss of cognitive function or dementia in said patient using the method according to any of claims 1 to 2 and
(b) modifying consequently the therapeutic strategy of the patient.

5. The method according to any preceding claim, wherein the expression level of lncRNAs is determined by FIMAP quantification.

6. A kit for diagnosing and/or monitoring a cognitive disorder including but not limited to AD, comprising at least one reagent for the determination of an expression profile of lncRNAs,
wherein the lncRNAs comprise 2 lncRNAs :TCONS_00035093, Inc-OCM-3:4, and wherein the at least one reagent comprises specific oligonucleotide probes complementary to the lncRNAs.

7. The kit according to claim 6, wherein the kit further comprises a reagent for purifying probe-IncRNA complexes.

8. The kit according to claim 7, wherein the oligonucleotide probes are biotinylated and the reagent for purifying the probe-IncRNA complexes is a streptavidin-coated substrate, e.g., a streptavidin-coated magnetic particle.

9. A targeted sequencing panel for next generation sequencing, comprising specific oligonucleotides complementary to lncRNAs, wherein the lnc RNAs comprise 2 lncRNAs: TCONS_00035093, Inc-OCM-3:4.

## Patentansprüche

1. Verfahren zur Diagnose einer kognitiven Störung, einschließlich, doch nicht beschränkt auf Morbus Alzheimer, bei einem Individuum, bei dem die Gefahr einer kognitiven Störung oder der Entwicklung davon besteht, umfassend:
(a) Nachweisen von IncRNA-Expressionsniveaus in einer biologischen Probe von dem Individuum;
(b) Vergleichen der IncRNA-Expressionsniveaus in der Probe mit einem Expressionsniveau in einer Referenz, wobei durch eine Zunahme oder Abnahme von IncRNA-Expressionsniveaus in der Probe im Vergleich zum Niveau in der Referenz eine kognitive Störung oder die Gefahr der Entwicklung einer kognitiven Störung bei dem Individuum identifiziert wird, wobei die biologische Probe aus Blut, Plasma, Serum, Speichel, Tränenflüssigkeit und Urin ausgewählt ist und wobei die IncRNAs 2 IncRNAs umfassen: TCONS_00035093, Inc-OCM-3:4.

2. Verfahren nach Anspruch 1, wobei in Schritt (a) die IncRNA-Expressionsniveaus zusammen mit einem weiteren zum Nachweis einer kognitiven Störung geeigneten Biomarker wie neurokognitiven Tests, Neurobildgebungsverfahren und/oder CSF-Biomarkern nachgewiesen werden.

3. Verfahren zur Gestaltung einer therapeutischen Behandlung einer kognitiven Störung bei einem an einem Verlust bzw. einer Beeinträchtigung kognitiver Funktion oder Demenz leidenden Individuum, wobei das Verfahren die Schritte (a), (b) gemäß Anspruch 1 oder 2 und
(c) Gestalten der therapeutischen Behandlung entsprechend der identifizierten kognitiven Störung umfasst.

4. Verfahren zum Anpassen einer Therapiestrategie an ein an einer fortschreitenden Beeinträchtigung bzw. einem fortschreitenden Verlust kognitiver Funktionen oder Demenz leidenden Individuum, wobei das Verfahren die folgenden Schritte umfasst:
(a) Diagnostizieren oder Überwachen der fortschreitenden Beeinträchtigung bzw. des fortschreitenden Verlusts kognitiver Funktionen bzw. der Demenz bei dem Patienten unter Verwendung des Verfahrens nach einem der Ansprüche 1 bis 2 und
(b) sich daraus ergebendes Modifizieren der Therapiestrategie für den Patienten.

5. Verfahren nach einem vorhergehenden Anspruch, wobei die IncRNA-Expressionsniveaus über FIMAP-Quantifizierung bestimmt werden.

6. Kit zur Diagnose und/oder Überwachung einer kognitiven Störung, einschließlich, doch nicht beschränkt auf AD, umfassend wenigstens ein Reagens zur Bestimmung eines Expressionsprofils von IncRNAs, wobei die IncRNAs 2 IncRNAs umfassen: TCONS_00035093, Inc-OCM-3:4, und wobei das wenigstens eine Reagens spezifische Oligonukleotidsonden umfasst, die zu den IncRNAs komplementär sind.

7. Kit nach Anspruch 6, wobei das Kit ferner ein Reagens zur Aufreinigung von Sonde-IncRNA-Komplexen umfasst.

8. Kit nach Anspruch 7, wobei die Oligonukleotidsonden biotinyliert sind und es sich bei dem Reagens zur Aufreinigung der Sonde-lncRNA-Komplexe um ein mit Streptavidin beschichtetes Substrat, z. B. ein mit Streptavidin beschichtetes magnetisches Partikel, handelt.

9. Gezielte Sequenzierpalette für Next-Generation-Sequencing, umfassend spezifische Oligonukleotide, die zu IncRNAs komplementär sind, wobei die IncRNAs 2 IncRNAs umfassen: TCONS_00035093, Inc-OCM-3:4.

## Revendications

1. Procédé de diagnostic d'un trouble cognitif incluant, sans s'y limiter, la maladie d'Alzheimer chez un sujet à risque d'avoir ou de développer un trouble cognitif, ledit procédé comprenant les étapes suivantes :
(a) détecter un niveau d'expression d'IncRNA dans un échantillon biologique provenant dudit sujet ;
(b) comparer le niveau d'expression des IncRNA dans l'échantillon à un niveau d'expression dans une référence, un niveau d'expression accru ou diminué des IncRNA dans l'échantillon par rapport au niveau dans la référence identifiant le sujet souffrant d'un trouble cognitif ou à risque de développer un trouble cognitif, l'échantillon biologique étant choisi parmi le sang, le plasma, le sérum, la salive, les larmes et l'urine, et les IncRNA comprenant 2 IncRNA : TCONS_00035093, Inc-OCM-3:4.

2. Procédé selon la revendication 1, **caractérisé en ce qu'**à l'étape (a) le niveau d'expression des IncRNA est détecté en combinaison avec un autre biomarqueur adapté à la détection d'un trouble cognitif tel que des tests neurocognitifs, des procédés de neuroimagerie et/ou des biomarqueurs du LCS.

3. Procédé de conception d'un traitement thérapeutique d'un trouble cognitif chez un sujet souffrant d'une perte ou d'une altération des fonctions cognitives ou d'une démence, ledit procédé comprenant les étapes (a), (b) selon les revendications 1 ou 2, et l'étape suivante
(c) concevoir le traitement thérapeutique en fonction dudit trouble cognitif identifié.

4. Procédé d'adaptation d'une stratégie thérapeutique à un sujet souffrant d'une altération ou d'une perte progressive de la fonction cognitive ou d'une démence, ledit procédé comprenant les étapes suivantes :
(a) diagnostiquer ou surveiller l'altération ou la perte progressive de la fonction cognitive ou la démence chez ledit patient à l'aide du procédé selon l'une quelconque des revendications 1 à 2 et
(b) modifier en conséquence la stratégie thérapeutique du patient.

5. Procédé selon l'une quelconque des revendications précédentes, le niveau d'expression des IncRNA étant déterminé par quantification FIMAP.

6. Kit de diagnostic et/ou de surveillance d'un trouble cognitif incluant, sans s'y limiter, la MA, ledit kit comprenant au moins un réactif destiné à la détermination d'un profil d'expression des IncRNA, les IncRNA comprenant 2 IncRNA : TCONS_00035093, Inc-OCM-3:4, et l'au moins un réactif comprenant des sondes oligonucléotidiques spécifiques complémentaires des IncRNA.

7. Kit selon la revendication 6, le kit comprenant en outre un réactif destiné à purifier les complexes sonde-IncRNA.

8. Kit selon la revendication 7, les sondes oligonucléotidiques étant biotinylées et le réactif destiné à purifier les complexes sonde-IncRNA étant un substrat enrobé de streptavidine, par exemple une particule magnétique enrobée de streptavidine.

9. Panel de séquençage ciblé destiné au séquençage de nouvelle génération, ledit panel comprenant des oligonucléotides spécifiques complémentaires des IncRNA, les IncRNA comprenant 2 IncRNA : TCONS_00035093, Inc-OCM-3:4.
